(19) Europäisches Patentamt
European Patent Office
Office européen des brevets

(11) EP 4 667 467 A1

(12) **EUROPEAN PATENT APPLICATION**

(43) Date of publication:
**24.12.2025 Bulletin 2025/52**

(21) Application number: **24183693.1**

(22) Date of filing: **21.06.2024**

(51) International Patent Classification (IPC):
***C07D 471/04*** *(2006.01)* ***A61P 35/00*** *(2006.01)*
***A61K 47/55*** *(2017.01)*

(52) Cooperative Patent Classification (CPC):
**C07D 471/04; A61K 47/55; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Dark Blue Therapeutics Ltd**
**Oxford, Oxfordshire OX4 4GB (GB)**

(72) Inventor: **The designation of the inventor has not yet been filed**

(74) Representative: **J A Kemp LLP**
**80 Turnmill Street**
**London EC1M 5QU (GB)**

(54) **PROTAC DEGRADERS OF MLLT1 AND/OR MLLT3**

(57) The invention relates to a compound which is a Proteolysis Targeting Chimera (PROTAC) or a pharmaceutically acceptable salt thereof, wherein the PROTAC has the structure:

**M-LINK-U**

wherein U is an E3 ubiquitin ligase binding moiety, LINK is a moiety that covalently links M and U, and M is an MLLT1 and/or MLLT3 binder of formula (I):

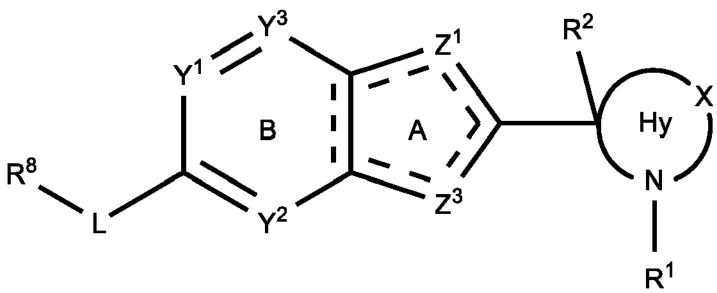

(I)

wherein:
wherein $Z^1$, $Z^2$, $Y^1$, $Y^2$, $Y^3$, $R^1$, $R^2$, $R^8$, X, L and Hy are as defined herein, and either $R^8$ is a bond to LINK, or M is bonded to LINK via a C or N atom within group $R^8$ or ring Hy such that a hydrogen atom on the C or N atom within group $R^8$ or ring Hy is replaced with a bond to LINK. The compounds are useful in the treatment of cancer.

EP 4 667 467 A1

## Description

### Field of the Invention

The present invention relates to compounds that find use in the treatment of cancer by inducing selective degradation of MLLT1 and/or MLLT3. The invention also provides such compounds *per se,* pharmaceutical compositions comprising such compounds, and methods of treating cancer by administering such compounds.

### Background

The processes controlling gene transcription are highly regulated during development and normal homeostasis. Dysregulation of gene transcription is a common driver of cancer with somatic defects in proteins that control gene transcription a frequent occurrence (Cell, 2013, 153, 17; Cell, 2017, 168, 629). Transcriptional elongation is a central step in gene transcription, carried out by the RNA polymerase II (PolII), which itself is kept under tight control by regulatory protein complexes. During development PolII is recruited to sites of the genome proximal to the transcription start site for target genes and is kept in a paused state. Such target genes are often immediate response genes such as heat-shock genes and key developmental genes. Productive elongation is initiated by the coordinated interplay of regulatory protein complexes including the super elongation complex (SEC). The SEC includes multiple proteins with diverse functions including protein phosphorylation, histone reader, histone modification and regulatory activities. Critical to the recruitment of the SEC to target genes are the histone reader proteins MLLT1 (mixed lineage leukaemia translocated to 1, also known as eleven-nineteen leukaemia, ENL) and MLLT3 (mixed lineage leukaemia translocated to 3, also known as AF-9). MLLT1 and 3 contain essential YEATS domains that bind acetylated histones. Mutations in the YEATS domains reduce loading of PolII on to SEC target genes and suppression of gene transcription. (Cell Mol Life Sci 2018, 75, 3931; Nat Rev Mol Cell Biol 2012, 13, 543).

Gain-of-function mutations in the MLLT1 Yeats domain have been causally associated with Wilm's tumor (also known as nephroblastoma) a kidney cancer most commonly observed in children (Nature 2020, 577, 121). Additionally, it has been shown that for certain acute leukaemias MLLT1 represents a critical dependency (Nature 2017, 543, 270). Such leukaemias include mixed-lineage leukaemia (MLL) rearranged leukaemia. MLL rearrangements arise from in frame fusions of the MLL gene with more than 80 different partner genes, many of which are involved in the regulation of transcription elongation including components of the SEC (Front. Pediatr. 5:4. doi: 10.3389/fped.2017.00004). MLL rearrangements are observed in approximately 10% of all acute leukaemias including a high frequency in infantile ALL where it accounts for 70-80% of all cases (Front. Pediatr. 5:4. doi: 10.3389/fped.2017.00004). Notably it is reported that patients with MLL rearranged leukaemias have an especially poor prognosis (New Engl J Med 2016, 374, 2209). Target genes for MLL-SEC complexes include potent oncogenes such as BCL-2, Myc and CDK6 along with many other genes implicated in maintaining cancer cell self-renewal, growth and survival, such as the HOX family genes and MEIS1 (Front. Pediatr. 5:4. doi: 10.3389/fped.2017.00004). Consistent with a role in the regulation of multiple oncogenes, it has been demonstrated that the SEC can play a critical role in the transcriptional addiction of both hematopoietic and solid cancers. For example, data supports potential in non-MLL rearranged leukaemia (Cancer Disc 2022, 12, 2684) and it is reported that with some breast cancer cells, growth and survival is dependent on a SEC mediated transcription of the Myc oncogene (Cell Rep. 2021 Feb 16;34(7): 108749. doi: 10.1016/j.celrep.2021.108749. PMID: 33596420; PMCID: PMC8006859).

Taken together the evidence supports MLLT1 as an attractive therapeutic target across acute leukaemias and solid cancers.

Proteolysis targeting chimeras (PROTACs) have been proposed as a small molecule-based platform technology capable of inducing proteolysis of a target protein in the body. The PROTAC is a bifunctional compound in which a molecule that binds to a disease-related target protein and an E3 ubiquitin ligase binding moiety are linked by a chemical linker. Theoretically, the PROTAC compound is capable of inducing degradation of the target protein by placing the disease-related target protein near the E3 ubiquitin ligase.

Drug discovery efforts have resulted in agents that either bind the YEATS domain to block the MLLT1 and/or MLLT3 protein to histone interaction or that result in degradation of the MLLT1 and/or MLLT3 protein (using a PROTAC approach). In all cases however, the reported activity has been weak (ACS Cent Sci 2021, 7, 815; Cancer Disc 2022, 12, 2684; Angew. Chem. Int. Ed. 2018, 57, 16302). Accordingly, there remains a high need to identify potent modulators of MLLT cell function.

### Summary of the Invention

The inventors have discovered a series of compounds that induce selective degradation of MLLT1 and/or MLLT3. Accordingly, the invention provides a compound which is a Proteolysis Targeting Chimera (PROTAC) or a pharmaceutically acceptable salt thereof, wherein the PROTAC has the structure:

**M - LINK - U**

wherein U is an E3 ubiquitin ligase binding moiety, LINK is a moiety that covalently links M and U, and M is an MLLT1 and/or MLLT3 binder of formula (I):

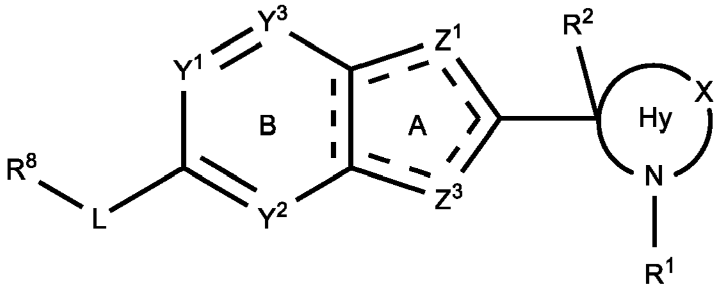

(I)

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;

Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;

L is -C(O)N(H)-, wherein the C atom of L is bonded to $R^8$, and the N atom of L is bonded to ring B;

$R^1$ is H, $C_{1-4}$ cycloalkyl, or $C_{1-4}$ alkyl which is itself unsubstituted or substituted with one $C_{1-4}$ alkoxy or one, two or three halo;

$R^2$ is H or methyl;

each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;

$R^4$ and $R^6$ are independently selected from H, halo, CN, $C_{1-4}$ alkoxy, and $C_{1-4}$ alkyl which is itself unsubstituted or substituted by one, two or three halo;

$R^5$ is selected from H, halo, $C_{1-4}$ alkoxy, $C_{3-5}$ cycloalkyl and $C_{1-4}$ alkyl which is itself unsubstituted or substituted by one, two or three halo;

$R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two R9;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and

each $R^{11}$ is independently selected from H, $C_{1-4}$ alkyl, and $C_{1-4}$ cycloalkyl;

wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

In a preferred embodiment, M is of formula (II):

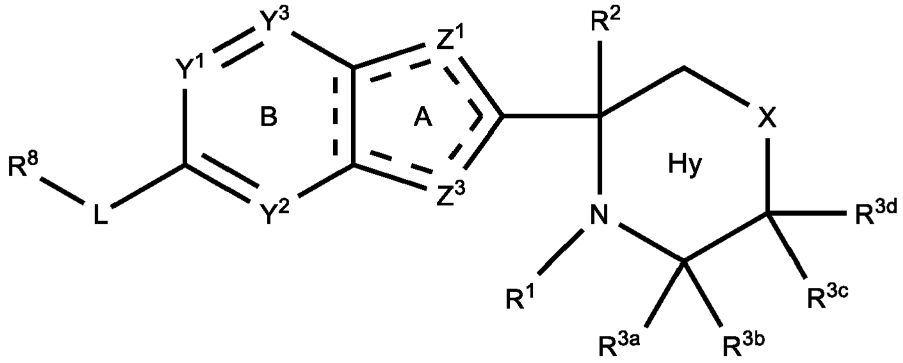

(II)

wherein:

$Z^1$, $Z^3$, $Y^1$, $Y^2$, $Y^3$, $R^1$, $R^2$, $R^8$, X and L are as defined herein;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -S(O)$_2$- or -S(O)(NR$^{11}$)-, then neither $R^{3c}$ nor $R^{3d}$ are halo;
wherein at least two of $R^{3a}$, $R^{3b}$, $R^{3c}$ and $R^{3d}$ are H; and
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

In a more preferred embodiment, M is of formula (III):

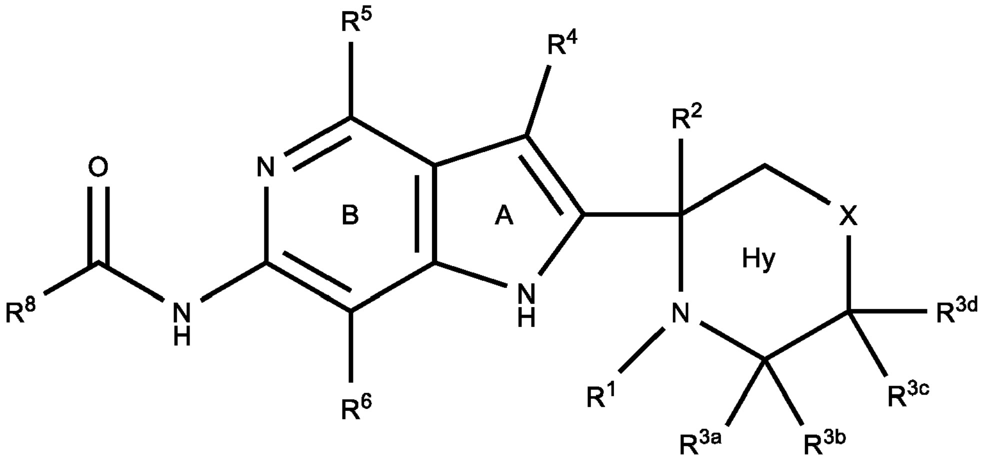

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring,

with the proviso that when X is -N(Me)- or O, then neither $R^{3c}$ nor $R^{3d}$ are halo; wherein at least two of $R^{3a}$, $R^{3b}$, $R^{3c}$ and $R^{3d}$ are H;
X is a bond, -N(Me)-, O or $-CH_2-$;
$R^4$ is selected from H, CN, halo, $C_{1-4}$ alkoxy, and $C_{1-4}$ alkyl which is itself unsubstituted or substituted by one, two or three halo;
$R^5$ and $R^6$ are independently selected from H, halo, $C_{1-4}$ alkoxy, and $C_{1-4}$ alkyl which is itself unsubstituted or substituted by one, two or three halo;
$R^8$ is (i) a bond to LINK or (ii) a group selected from a 6-membered aryl ring, a 5- to 6-membered heteroaryl ring, and a 5- to 6-membered heterocyclyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

In a particularly preferred embodiment, M is of formula (IV):

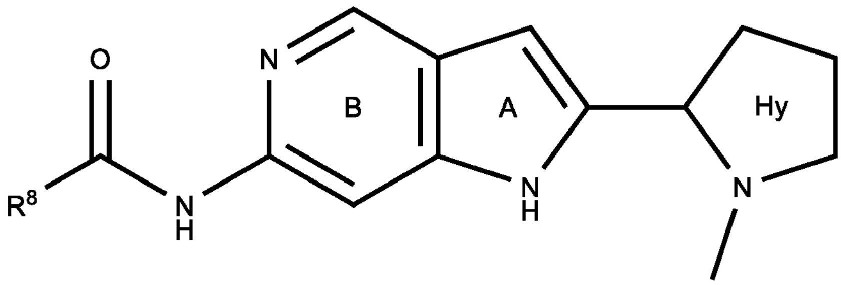

(IV)

wherein $R^8$ is (i) a bond to LINK or (ii) a group selected from a 6-membered aryl ring and a 5- to 6-membered heteroaryl ring, the group $R^8$ being unsubstituted or substituted by one, two or three substituents independently selected from halo, $C_{1-4}$ alkyl and $R^{10}$; $R^{10}$ is a 3-membered cycloalkyl ring;
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK. As discussed elsewhere herein, the stereochemistry of the compounds of the invention at the ring Hy is preferably such that the bond from ring Hy to the core ring A is in the "up" position and the bond from ring Hy to $R^2$ is the down position. Thus, formula (IV) preferably has the stereochemistry depicted below:

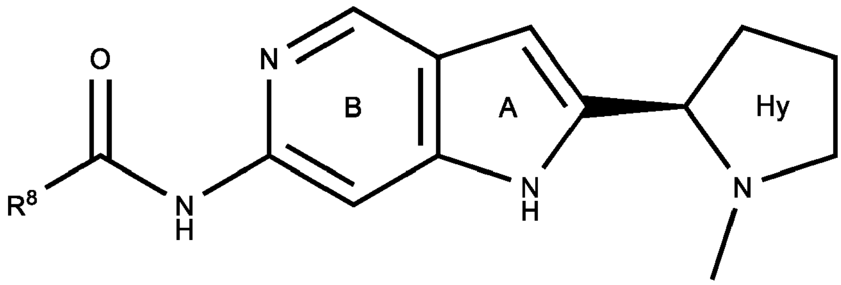

(IV)

The present invention also provides a compound as described herein for use in a method of treating cancer in a subject in need thereof. Also provided is a method for treating cancer in a subject, which method comprises administering to said subject an effective amount of a compound as described herein. Further provided is the use of a compound as described herein in the manufacture of a medicament for use in treating cancer in a subj ect.

<u>**Detailed Description of the Invention**</u>

*Definitions*

As used herein, a $C_{1-4}$ alkyl group is a linear or branched alkyl group containing from 1 to 4 carbon atoms. A $C_{1-4}$ alkyl group is often a $C_{1-3}$ alkyl group. Examples of $C_{1-4}$ alkyl groups include methyl, ethyl, n-propyl, iso-propyl, n-butyl, sec-butyl, and tert-butyl. A $C_{1-3}$ alkyl group is typically a $C_{1-2}$ alkyl group. A $C_{1-2}$ alkyl group is methyl or ethyl, typically methyl. For the avoidance of doubt, where multiple alkyl groups are present, the alkyl groups may be the same or different.

As used herein, a $C_{1-4}$ alkoxy group is typically a said $C_{1-4}$ alkyl group which is joined to the rest of the molecule via an oxygen atom. Typically, a $C_{1-4}$ alkoxy group is a $C_{1-3}$ alkoxy group. Examples of $C_{1-4}$ alkoxy groups include methoxy, ethoxy, propoxy and butoxy. Typically, a $C_{1-3}$ alkoxy group is a $C_{1-2}$ alkoxy group such as a methoxy or ethoxy group. For the avoidance of doubt, where two alkoxy groups are present, the alkoxy groups may be the same or different.

As used herein, a $C_{1-30}$ alkylene group is a linear or branched divalent alkyl group that contains from 1 to 30 carbon atoms. A $C_{1-30}$ alkylene group is sometimes a $C_{1-20}$ alkylene group, and often a $C_{1-10}$ alkylene group. $C_{1-30}$ alkylene groups, $C_{1-20}$ alkylene groups and $C_{1-10}$ alkylene groups are preferably linear.

A $C_{1-30}$ alkylene group is sometimes a $C_{1-6}$ alkylene group, typically a $C_{1-4}$ alkylene group or a $C_{1-3}$ alkylene group. Examples of $C_{1-4}$ alkylene groups include methylene, ethylene, n-propylene, iso-propylene, n-butylene, sec-butylene, and tert-butylene. A $C_{1-3}$ alkylene group is typically a $C_{1-2}$ alkylene group. A $C_{1-2}$ alkylene group is methylene or ethylene, typically methylene. For the avoidance of doubt, where multiple alkylene groups are present, the alkylene groups may be the same or different.

As used herein, a $C_{2-6}$ alkenylene group is a linear or branched divalent alkenyl group containing from 2 to 6 carbon atoms and having one or more, e.g. one or two, typically one double bonds. Typically, a $C_{2-6}$ alkenylene group is a $C_{2-4}$ alkenylene group. Examples of $C_{2-4}$ alkenylene groups include divalent ethenylene, propenylene and butenylene. For the avoidance of doubt, where multiple alkenylene groups are present, the alkenylene groups may be the same or different.

An alkyl, alkoxy, alkylene or alkenylene group as used herein may be unsubstituted or substituted. Unless otherwise stated, substituted alkyl, alkoxy, alkylene or alkenylene groups typically carry one or more, e.g. one, two or three e.g. one, or two, e.g. one substituent selected from halo, OH, and unsubstituted $C_{1-4}$ alkoxy. Preferred substituents are halo and $C_{1-4}$ alkoxy unless otherwise stated. The substituents on a substituted alkyl, alkoxy, alkylene or alkenylene group are typically themselves unsubstituted. Where more than one substituent is present, these may be the same or different.

As used herein, a halo typically refers to chlorine, fluorine, bromine or iodine, preferably chlorine, bromine or fluorine, more preferably chorine or fluorine, most preferably fluorine unless otherwise stated.

A $C_{3-8}$ cycloalkyl ring is a cyclic hydrocarbon containing from 3 to 8 carbon atoms. A cycloalkyl ring may be saturated or partially unsaturated, but is typically saturated. A $C_{3-8}$ cycloalkyl ring is typically a $C_{3-6}$ cycloalkyl ring. A partially unsaturated cycloalkyl ring is a cyclic hydrocarbon containing 1 or 2, e.g. 1 double bond. $C_{3-6}$ cycloalkyl and $C_{5-6}$ cycloalkyl rings may also be referred to herein as 3- to 6-membered cycloalkyl rings and 5- to 6-membered cycloalkyl rings respectively.

A $C_{3-6}$ cycloalkyl ring may be a saturated $C_{3-6}$ cycloalkyl ring. A $C_{3-6}$ cycloalkyl ring may be a $C_{5-6}$ cycloalkyl ring, in particular a saturated $C_{5-6}$ cycloalkyl ring. Examples of $C_{3-6}$ cycloalkyl rings are cyclopropyl, cyclobutyl cyclopentyl and cyclohexyl groups.

A $C_{3-8}$ cycloalkyl ring may be a $C_{7-8}$ cycloalkyl ring, in particular a saturated $C_{7-8}$ cycloalkyl ring. Examples of $C_{7-8}$ cycloalkyl rings are cycloheptanly, cyclooctanyl, bicyclo[2.2.1]heptanyl and bicyclo[2.2.2]octanyl groups.

A 4- to 7-membered heterocyclyl ring is a cyclic group containing from 4 to 7 atoms selected from C, O, N and S in the ring, including at least one heteroatom, and typically one or two heteroatoms unless otherwise stated. The heteroatom or heteroatoms are typically selected from O, N, and S, most typically from S and N, especially N. For example, where the heterocyclyl ring is denoted a nitrogen-containing heterocyclyl group, it contains one nitrogen atom and optionally a further heteroatom selected from O, N and S. A heterocyclyl ring may be saturated or partially unsaturated, but is typically saturated. A 4- to 7- membered partially unsaturated heterocyclyl ring is a cyclic group containing from 4 to 7 atoms selected from C, O, N and S in the ring and containing 1 or 2, e.g. 1 double bond.

A 4- to 7- membered heterocyclyl ring may sometimes be a 5- to 6-membered ring. A 4- to 7- membered heterocyclyl ring is typically a monocyclic ring and may be a monocyclic 5- or 6- membered heterocyclyl ring. In some compounds described herein, a 4- to 7- membered heterocyclyl group is a 4- to 7- membered nitrogen-containing heterocyclyl ring which is unsubstituted or is substituted as described herein. Preferred 4- to 7- membered nitrogen-containing heterocyclyl rings include morpholine, pyrrolidine, piperidine and piperazine.

Examples of 5- and 6- membered saturated heterocyclyl rings include piperazine, piperidine, morpholine, diazinane and pyrrolidine. Diazinane is typically 1,4-diazinane.

As used herein, a 6- to 10-membered aryl ring is a substituted or unsubstituted, monocyclic or fused polycyclic aromatic group containing from 6 to 10 carbon atoms in the ring portion. Examples include monocyclic groups such as phenyl and fused bicyclic groups such as naphthyl and indenyl. Phenyl (benzene) is preferred.

As used herein, a 5- to 10- membered heteroaryl ring is a substituted or unsubstituted monocyclic or fused

polycyclic aromatic group containing from 5 to 10 atoms in the ring portion, including at least one heteroatom, for example 1, 2 or 3 heteroatoms, typically selected from O, S and N. A heteroaryl ring is typically a 5- or 6-membered heteroaryl ring or an 8- to 10- membered heteroaryl ring. Preferably, the heteroaryl ring comprises 1, 2 or 3, preferably 1 or 2 nitrogen atoms.

Examples of 5- and 6- membered heteroaryl rings include thiazole, pyrazole, pyrimidine, triazole, 1,2,4-oxadiazole and pyrazine.

Examples of 8-, 9- and 10- membered heteroaryl rings include indazole, thieno[2,3-c]pyrazole, imidazo[4,5-b]pyridine, pyrazolo[3,4-b]pyridine, furo[2,3-c]pyridine, indole, benzoxazole, benzothiazole, [1,2,4]triazolo[4,3-a]pyridine, thieno[2,3-d]pyrimidine, 1,2,3-benzotriazole, imidazo[1,5-a]pyridine, imidazo[1,2-a]pyrazine, oxazolo[5,4-b]pyridine, quinoline, naphthyridine, isoquinoline, quinazoline, and quinoxaline. 8-, 9- and 10- membered heteroaryl rings as used herein are typically fused bicyclic groups.

For the avoidance of doubt, references to a heteroaryl ring also include fused polycyclic ring systems, including for instance fused bicyclic systems in which a heteroaryl ring is fused to an aryl group. When the heteroaryl ring is such a fused heteroaryl group, preferred examples are fused ring systems wherein a 5- to 6-membered heteroaryl group is fused to a phenyl group. Indazole is preferred.

As used herein, a fused bicyclic group is a group comprising two cyclic moieties sharing a common bond between two atoms.

When $R^8$ is selected from a 6-membered aryl ring, a 5- to 6-membered heteroaryl ring, a 5- to 6-membered heterocyclyl ring and a 5- to 6-membered cycloalkyl ring, it is to be understood that $R^8$ is a monocyclic ring.

A cycloalkyl, heterocyclyl, aryl or heteroaryl ring may be unsubstituted or substituted as described herein unless otherwise stated. For example, a cycloalkyl, heterocyclyl, aryl or heteroaryl ring may be unsubstituted or substituted with 1, 2 or 3, typically 1 or 2 such as e.g. 1 substituent. Suitable substituents include halo, $C_{1-4}$ alkoxy, $R^{10}$, -($C_{1-4}$ alkylene)-$R^{10}$, =O, -CN, and $C_{1-4}$ alkyl, wherein $R^{10}$ is as defined herein). The substituents on a substituted cycloalkyl, heterocyclyl, aryl or heteroaryl ring are typically themselves unsubstituted, unless otherwise stated.

The compounds described herein comprise at least one heterocyclyl ring comprising at least one nitrogen atom. Said nitrogen atom(s) are independently selected from secondary, tertiary and quaternary nitrogen atom(s). A quaternary nitrogen atom is present when the compound comprises a quaternised derivative of one or more monocyclic groups or fused bicyclic groups. As used herein, a quaternised derivative of a moiety such as a cyclic moiety is formed by bonding an additional alkyl group to a nitrogen atom in the moiety such that the valency of the said nitrogen atom increases from 3 to 4 and the nitrogen atom is positively charged.

The compounds described herein comprise a heterocyclyl ring identified as ring Hy, which is a 4- to 7-membered heterocyclyl ring. Ring Hy contains X and at least one N atom in the ring portion, wherein X is a bond, -N($R^{11}$)-, O, S, -$S(O)_2$-, -S(O)($NR^{11}$)-, or-$C(R^{11})_2$-, and $R^{11}$ is as defined herein. Ring Hy is linked to ring A (as identified in formula (I) above) via a C atom within ring Hy, said C atom also being linked to $R^2$ which is as defined herein. A N atom within ring Hy is substituted by $R^1$ which is as defined herein.

The rest of ring Hy is unsubstituted or substituted by one or two $R^3$. Each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring, as defined herein. In option (ii), the skilled person would understand that the C atom to which the two $R^3$ are attached is a spiro atom (i.e. the common atom that connects the two rings of a spiro compound).

As used herein, the terms "monovalent" or "monovalent moiety" are used to describe a chemical group obtainable by removing a hydrogen atom from the corresponding compound. As used herein, the terms "divalent" or "divalent moiety" are used to describe a chemical group obtainable by removing a hydrogen atom from the corresponding monovalent moiety. Thus, as used herein, the terms "divalent" and "divalent moiety" are used to describe a chemical group obtainable by removing two hydrogen atoms from the corresponding compound.

It is to be understood that each individual atom present in the formulae depicted herein may be present in the form of any of its naturally occurring isotopes, with the most abundant isotope(s) being preferred. Thus, by way of example, each individual hydrogen atom present in the formulae depicted herein may be present as a 1H, 2H (deuterium) or 3H (tritium) atom, preferably 1H. Similarly, by way of example, each individual carbon atom present in the formulae depicted herein may be present as a 12C, 13C or 14C atom, preferably 12C.

In the compounds of the invention, the stereochemistry is not limited. In particular, where moiety M of formula (I) and/or moiety U contains one or more chiral centre, the compounds may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60%, 75%, 90% or 95% of a compound which is enantiomerically or diasteriomerically pure. Thus, the compound is preferably substantially optically pure.

The moiety M of formula (I) typically contains at least one chiral centre at the carbon atom of ring Hy which is linked to $R^2$ and to ring A. Moiety M may be provided in the form of the R-enantiomer at said carbon atom, in the form of the S-enantiomer at said carbon atom, or in the form of a mixture of the two enantiomers. The substance or agent described herein may contain at least 50%, preferably at least 60, 75%, 90% or 95% of a compound which is enantiomerically or diasteriomerically pure at moiety M. A pure enantiomeric form of either the R- or the S-enantiomer may be preferred. In some embodiments, the R-enantiomer at said carbon atom is preferred, and in particular when $R^2$ is H then the R-enantiomer at said carbon atom is preferred. The preferred stereochemistry at said carbon atom is also depicted by the following illustrative structure of formula (I) where the bond from ring Hy to the core ring A is in the "up" position and the bond from ring Hy to $R^2$ is the down position:

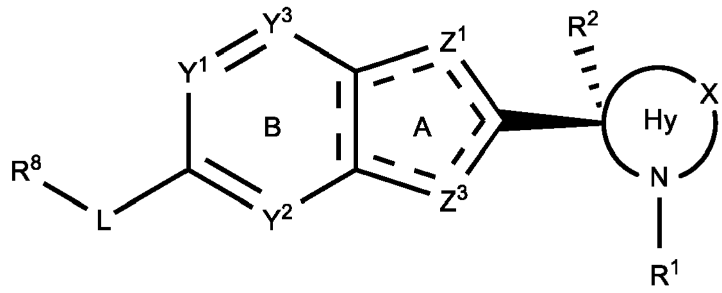

(I)

This steroechemsitry at the carbon atom of ring Hy which is linked to $R^2$ and to ring A, where the bond from ring Hy to the core ring A is in the "up" position and the bond from ring Hy to $R^2$ is the down position (as depicted above), is also preferred for the moiety M of formulae (II), (III), (IV), and for the compounds of formulae (I'), (II') and (III').

As discussed further herein, where the compounds of the invention contain a chiral centre, and in particular where moiety M and/or moiety U contains a chiral centre, the individual stereoisomers may be obtained by chiral synthesis, or by separation of stereoisomers. Stereoisomers may be separated, for example, using chiral chromatography. The individual stereoisomers may be identified using the IUPAC labels R and S as appropriate. Alternatively, the individual stereoisomers may be identified by the order in which they elute, for example the first, second, third or fourth stereisomer respectively, as obtained by separation using chiral chromatography. Where two or more stereocentres are present in a compound or moiety, a combination of IUPAC nomenclature for stereocentres where absolute stereochemistry has been defined, and rate of elution, may be used.

For example, where two or more individual stereoisomers are unidentified by IUPAC nomenclature, these stereoisomers may be identified as the first- and second-eluting isomers, as obtained by separation using chiral chromatography. Therefore, the stereoisomers at a chiral carbon atom of ring Hy which is linked to $R^2$ and to ring A may be defined as the first-eluting and second-eluting stereoisomers, as obtained by separation using chiral chromatography. Chiral chromatography is typically reverse phase HPLC.

The compounds of the present invention may exist in unsolvated as well as solvated forms with pharmaceutically acceptable solvents such as water, ethanol, and the like, and it is intended that the invention embrace both solvated and unsolvated forms.

A compound of the present invention can be converted into a pharmaceutically acceptable salt thereof, and a salt can be converted into the free compound, by conventional methods. For instance, a compound of the present invention can be contacted with a pharmaceutically acceptable acid to form a pharmaceutically acceptable salt. A pharmaceutically acceptable salt is a salt with a pharmaceutically acceptable acid or base.

Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic orp-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts.

*Proteolysis Targeting Chimera (PROTAC)s*

The compounds of the invention comprise a binder of MLLT1 and/or MLLT3 (M) that is covalently attached via a linker moiety (LINK) to an E3 ubiquitin ligase binding moiety (U). The inventors have surpisingly discovered that such compounds may have substantially enhanced efficacy compared with a corresponding MLLT1 and/or MLLT3 inhibitor that is not attached to an E3 ubiquitin ligase binding moiety. For example, the antiproliferative effects of the compounds may be substantially enhanced by covalent attachment to the E3 ubiquitin ligase binding moiety.

Thus, the invention provides a compound which is a Proteolysis Targeting Chimera (PROTAC) or a pharmaceutically acceptable salt thereof, wherein the PROTAC has the structure:

**M - LINK - U**

wherein U is an E3 ubiquitin ligase binding moiety, LINK is a moiety that covalently links M and U, and M is an MLLT1 and/or MLLT3 binder of formula (I) as defined herein.

In the compounds of the present invention, the MLLT 1 and/or MLLT3 binder is covalently attached to the E3 ubiquitin ligase binding moiety via the moiety labelled LINK. LINK is either a single bond or a chemical group that covalently attaches the MLLT1 and/or MLLT3 binder to the E3 ubiquitin ligase binding moiety.

The MLLT1 and/or MLLT3 binder is attached to LINK via (i) a single bond at $R^8$, or (ii) a C or N atom within group $R^8$ or ring Hy (as described herein) such that a hydrogen atom on the C or N atom within group $R^8$ or ring Hy is replaced with a bond to LINK. In case (ii), a hydrogen atom on the C or N atom within group $R^8$ or ring Hy is replaced by either a direct single bond to the E3 ubiquitin ligase binding moiety or by a chemical group that covalently attaches the MLLT1 and/or MLLT3 binder to the E3 ubiquitin ligase binding moiety.

The description herein discusses the substitution of group $R^8$ and ring Hy by various chemical groups aside from LINK. For completeness, the skilled person would readily appreciate that where group $R^8$ and/or ring Hy are defined as being unsubstituted, this is intended as a reference to the absence of substituents other than a bond to LINK. Therefore, such a definition retains the option that group $R^8$ or ring Hy is attached to LINK via a C or N atom within group $R^8$ or ring Hy such that a hydrogen atom on the C or N atom within group $R^8$ or ring Hy is replaced with a bond to LINK. Furthermore, the skilled person would readily appreciate that where group $R^8$ and/or ring Hy are defined as being substituted by one or more chemical groups (suitable such chemical groups are defined herein), this is intended as a reference to substituents other than a bond to LINK. Therefore, such a definition includes group $R^8$ or ring Hy being attached to LINK via a C or N atom within group $R^8$ or ring Hy such that a hydrogen atom on the C or N atom within group $R^8$ or ring Hy is replaced with a bond to LINK. Therefore, where group $R^8$ and/or ring Hy are defined as being unsubstituted or substituted by one or more chemical groups (suitable such chemical groups are defined herein), then one of group $R^8$ or ring Hy may also be attached to LINK via a C or N atom within group $R^8$ or ring Hy such that a hydrogen atom on the C or N atom within group $R^8$ or ring Hy is replaced with a bond to LINK. A bond to LINK may optionally be positioned on a carbon atom which carries a further substituent (where chemically possible). Typically, a carbon or nitrogen atom which is bonded to LINK does not carry a further substituent.

For the avoidance of doubt, where group $R^8$ and/or ring Hy are defined as being substituted by a hydrogen atom (e.g. where $R^1$, $R^2$, $R^{11}$, $R^{3a}$, $R^{3b}$, $R^{3c}$ and $R^{3d}$ are defined as being a hydrogen atom), then a reference herein to a hydrogen atom within group $R^8$ or ring Hy being replaced with a bond to LINK, includes the option that said hydrogen atom (e.g. a hydrogen atom at $R^1$, $R^2$, $R^{11}$, $R^{3a}$, $R^{3b}$, $R^{3c}$ and $R^{3d}$) is replaced with a bond to LINK. Therefore, where $R^1$, $R^2$, $R^{11}$, $R^{3a}$, $R^{3b}$, $R^{3c}$ and $R^{3d}$ are defined as being a hydrogen atom, then said hydrogen atom may be replaced with a bond to LINK.

For the avoidance of doubt, where ring Hy or group $R^8$ is substituted, a C or N atom which is bonded to LINK may be within the aryl, heteroaryl, heterocyclyl, or cycloalkyl ring of ring Hy or group $R^8$, or it may be a C or N atom of a substituent on ring Hy or group $R^8$. Preferably, a C or N atom which is bonded to LINK is a C or N atom on the heterocyclyl ring of ring Hy (i.e. directly to the ring and not via a substituent), a C or N atom of the heteroaryl ring of group $R^8$, or the heteroaryl, cycloalkyl, heterocyclyl or phenyl ring of $R^{10}$ in the case that a group $R^{10}$ is present as, or as part of, a substituent on $R^8$.

As the skilled person would readily appreciate, methods for preparation of compounds (in which two discrete chemical entities contribute discrete biological functions to the overall compound) are very well known in the art. A hugely diverse range of techniques for covalently attaching the respective chemical entities, via a vast number of chemical linker moieties, is well established. Such is the ubiquity of these methodologies, that standard text books devoted entirely to this topic have long been available. One such textbook is "Bioconjugate Techniques" (Greg T. Hermanson, Academic Press Inc., 1996), the content of which is herein incorporated by reference in its entirety.

*MLLT1 and/or MLLT3 binder group M*

In the compounds of the invention, the MLLT1 and/or MLLT3 binder M is a monovalent moiety of formula (I) as defined herein.

In the description that follows, the skilled person will readily understand that M is a monovalent moiety that is bonded to LINK via (i) a single bond at $R^8$, or (ii) a C or N atom within group $R^8$ or ring Hy (as described herein) such that a hydrogen atom on the C or N atom within group $R^8$ or ring Hy is replaced with a bond to LINK. Thus, in the description that follows, any of the hydrogen atoms on any of the C or N atoms within group $R^8$ or ring Hy may be removed to form the point of attachment of the monovalent moiety M to LINK.

Typically, ring Hy is a 5- to 6- membered heterocyclyl ring containing X and at least one N atom, for instance one or

two N atoms. X is typically a bond, $-N(R^{11})-$, O or $-C(R^{11})_2-$. Preferably, X is a bond, -N(Me)-, O or $-CH_2-$, most preferably X is a bond. In particular, when ring Hy is a 4- or 5-membered heterocyclyl ring, X is most preferably a bond. For example, ring Hy may be selected from a pyrrolidinyl, piperidinyl, morpholinyl or diazinanyl ring. Preferably, ring Hy is a 5-membered heterocyclyl ring containing X and at least one N atom, for instance one N atom. For example, ring Hy may be a pyrrolidinyl ring.

Ring Hy is linked to ring A via a C atom within ring Hy, said C atom also being linked to $R^2$. A N atom within ring Hy is substituted by $R^1$. Typically, the N atom of ring Hy which is substituted by $R^1$ is adjacent to the C atom of ring Hy that is bonded to ring A.

A C or N atom within ring Hy may be bonded to LINK by a hydrogen atom on that C or N atom being replaced by a bond to LINK. Typically, where ring Hy is bonded to LINK, this is via a C atom on Hy. Preferably, the bond to LINK is via replacement of a hydrogen atom on a C or N within group $R^8$, i.e. the bond to LINK is preferably not via ring Hy.

Typically, $R^1$ is H, $C_{1-4}$ cycloalkyl, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy, or $R^1$ may be H or $C_{1-2}$ alkyl which is unsubstituted or substituted with one $C_{1-2}$ alkoxy. Preferably, $R^1$ is H, methyl, ethyl or methoxyethyl, more preferably methyl or ethyl. Most preferably, $R^1$ is methyl.

$R^2$ is H or methyl. Preferably, $R^2$ is H.

Typically, each $R^{11}$ is independently selected from H and methyl. When X is $-N(R^{11})-$ or $-S(O)(NR^{11})-$, $R^{11}$ is preferably methyl. When X is $-C(R^{11})_2-$, optionally one $R^{11}$ is replaced with a single bond to LINK and the other $R^{11}$ is H. When X is $-C(R^{11})_2-$, each $R^{11}$ is preferably H.

Aside from groups $R^1$ and $R^2$, ring Hy may further be unsubstituted or substituted by one or two $R^3$. Typically, each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring. More typically, each $R^3$ is independently selected from methyl, ethyl, t-butyl methoxy and fluoro, or (i) two $R^3$ linked to adjacent C atoms in ring Hy form, together with the C atoms to which they are attached, a cyclohexyl ring, or (ii) two $R^3$ linked to the same C atom in ring Hy form, together with the C atom to which they are attached, a cyclopentyl ring. Preferably, ring Hy is substituted by no $R^3$ groups.

Typically, therefore, ring Hy is a 5- to 6- membered heterocyclyl ring containing X and at least one N atom, for example a pyrrolidinyl, piperidinyl, morpholinyl or diazinanyl ring; the N atom of ring Hy which is substituted by $R^1$ is adjacent to the C atom of ring Hy that is bonded to ring A; $R^1$ is H or $C_{1-2}$ alkyl which is unsubstituted or substituted with one $C_{1-2}$ alkoxy or one, two or three halo, preferably with one $C_{1-2}$ alkoxy; $R^2$ is H or methyl; X is a bond, $-N(R^{11})-$, O or $-C(R^{11})_2-$; $R^{11}$ is H or methyl; ring Hy is further unsubstituted or substituted by one or two $R^3$; and each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring.

Preferably, ring Hy is a pyrrolidinyl ring; the N atom of ring Hy which is substituted by $R^1$ is adjacent to the C atom of ring Hy that is bonded to ring A; $R^1$ is methyl or ethyl, more preferably methyl; $R^2$ is H; and ring Hy is substituted by no $R^3$ groups, i.e. ring Hy is not further substituted and carries substituent $R^1$ only. In this embodiment, ring Hy may be referred to as being an unsubstituted pyrrolidine ring, wherein the skilled person would understand that ring Hy still carries substituent $R^1$.

Typically, no more than two of $Y^1$, $Y^2$ and $Y^3$ are N. Preferably $Y^1$ is N, $Y^2$ is $-C(R^6)-$, and $Y^3$ is $-C(R^5)-$.

Typically, $Z^1$ is $-C(R^4)-$, $Z^3$ is -N(H)-, and $Y^1$ is N. Thus, the bicyclic structure formed by rings A and B has the structure:

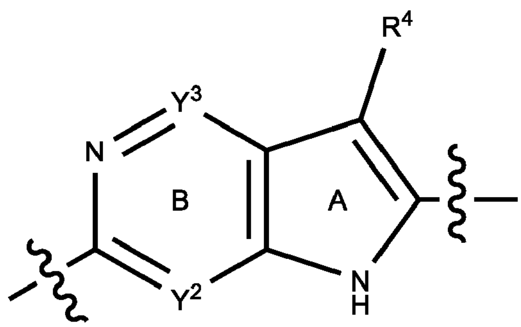

In one preferred embodiment, $Z^1$ is $-C(R^4)-$, $Z^3$ is -N(H)-, $Y^1$ is N, $Y^2$ is $-C(R^6)-$, and $Y^3$ is $-C(R^5)-$, such that the bicyclic structure formed by rings A and B has the structure:

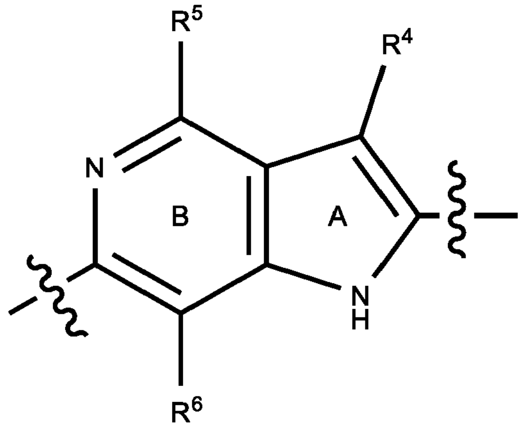

Typically, $R^4$ is selected from H, CN, halo, $C_{1-4}$ alkoxy, and $C_{1-4}$ alkyl which is itself unsubstituted or substituted by one, two or three halo; and $R^5$ and $R^6$ are independently selected from H, halo, $C_{1-4}$ alkoxy, and $C_{1-4}$ alkyl which is itself unsubstituted or substituted by one, two or three halo. For instance, $R^4$ is selected from H, halo (e.g. fluoro), methoxy, and methyl; and $R^5$ and $R^6$ are independently selected from H, halo (e.g. fluoro), methoxy, and methyl. Preferably, either $R^4$, $R^5$ and $R^6$ are H, or one of $R^4$, $R^5$ and $R^6$ is not H. More preferably, $R^4$, $R^5$ and $R^6$ are H.

In one preferred embodiment, $R^5$ and $R^6$ are as defined herein and $R^4$ is selected from H, halo, CN, $C_{1-4}$ alkoxy, and $C_{1-4}$ alkyl which is itself unsubstituted or substituted by one, two or three halo. More preferably, $R^5$ and $R^6$ are as defined herein and $R^4$ is selected from H, halo, CN, $C_{1-4}$ alkyl and $CF_3$. Most preferably, $R^5$ and $R^6$ are as defined herein and $R^4$ is selected from H, F, Cl, CN, Me and $CF_3$.

In one preferred embodiment, $R^5$ is selected from H, cyclopropyl, $C_{1-4}$ alkoxy, and $C_{1-4}$ alkyl, for example from H, $C_{1-4}$ alkoxy, and $C_{1-4}$ alkyl. More preferably, $R^5$ is selected from H, cyclopropyl, methoxy and methyl, for example H, methoxy and methyl.

In one preferred embodiment, $R^6$ is H.

Typically, therefore, in formula (I):

- $R^4$ is selected from H, halo, CN, $C_{1-4}$ alkoxy, and $C_{1-4}$ alkyl which is itself unsubstituted or substituted by one, two or three halo, and $R^5$ and $R^6$ when present are H; or
- $R^5$ is selected from H, cyclopropyl, $C_{1-4}$ alkoxy, and $C_{1-4}$ alkyl, typically from H, $C_{1-4}$ alkoxy, and $C_{1-4}$ alkyl, and $R^4$ and $R^6$ when present are H; or
- $R^4$, $R^5$ and $R^6$ are H.

Preferably, in formula (I):

- $R^4$ is selected from H, F, Cl, Me, CN and $CF_3$, and $R^5$ and $R^6$ when present are H;
- $R^5$ is selected from H, cyclopropyl, methoxy and methyl, typically from H, methoxy and methyl, and $R^4$ and $R^6$ when present are H; or
- $R^4$, $R^5$ and $R^6$ are H.

L is -C(O)N(H)-, wherein the C atom of L is bonded to $R^8$, and the N atom of L is bonded to ring B.

In one embodiment, $R^8$ is a bond to LINK.

In another embodiment, $R^8$ is a group selected from a 6-membered aryl ring, a 5- to 6-membered heteroaryl ring, a 5- to 6-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$. $R^9$ and $R^{10}$ are as defined herein.

Typically, $R^8$ is a group selected from a 6-membered aryl ring, a 5- to 6-membered heteroaryl ring, and a 5- to 6-membered heterocyclyl ring. The aryl, heteroaryl and heterocyclyl rings are as defined herein. For instance, $R^8$ may be selected from phenyl, thiazolyl, pyrazolyl, pyrimidinyl, pyridinyl, triazolyl, 1,2,4-oxadiazolyl, and pyrazinyl. Preferably, $R^8$ is phenyl or a 5- to 6-membered heteroaryl ring preferably containing one, two or three N atoms. More preferably, $R^8$ is a phenyl group, a pyridinyl, a pyrimidinyl or a pyrazolyl group.

A C or N atom within group $R^8$ may be bonded to LINK by a hydrogen atom on that C or N atom being replaced by a bond to LINK. Typically, where group $R^8$ is bonded to LINK, this is via a C atom on $R^8$. Preferably, a C or N atom within group $R^8$ is bonded to LINK such that a hydrogen atom on that C or N atom is replaced by a bond to LINK. Preferably, the C or N atom that is bonded to LINK is a C or N atom of the aryl, heteroaryl, heterocyclyl or cycloalkyl ring of $R^8$ or a C or N atom of

the heteroaryl, cycloalkyl, heterocyclyl or phenyl ring of $R^{10}$. More preferably, the C or N atom that is bonded to LINK is a C or N atom of the aryl, heteroaryl, heterocyclyl or cycloalkyl ring of $R^8$.

In addition to any bond to LINK (where present) $R^8$ is unsubstituted or substituted by one, two or three substituents, for instance one or two substituents. Typically, each substituent is independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy. Preferably, each substituent is independently selected from fluoro, $R^{10}$ and methyl. Most preferably, each substituent is independently selected from fluoro and methyl. $R^{10}$ is as defined herein.

Typically, $R^{10}$ is a group selected from a 5- to 6-membered heteroaryl ring, a 3- to 6-membered cycloalkyl ring, a 4- to 5-membered heterocyclyl ring, and a phenyl ring. The cycloalkyl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl.

Typically, in addition to any bond to LINK (where present), $R^{10}$ is unsubstituted or substituted by one or two substituents independently selected from fluoro, methyl and methoxy. Preferably, $R^{10}$ is unsubstituted.

Typically, therefore, $R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring; the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; wherein $R^{10}$ is a group selected from a 5- to 6-membered heteroaryl ring, a 3- to 6-membered cycloalkyl ring, a 4- to 5-membered heterocyclyl ring, and a phenyl ring; and $R^{10}$ is unsubstituted or substituted by one or two substituents independently selected from fluoro, methyl and methoxy. Typically, where a C or N atom within group $R^8$ is bonded to LINK, the C or N atom that is bonded to LINK is a C or N atom of the aryl, heteroaryl, heterocyclyl or cycloalkyl ring of $R^8$ or a C or N atom of the heteroaryl, cycloalkyl, heterocyclyl or phenyl ring of $R^{10}$.

Preferably, $R^8$ is phenyl or a 5- to 10-membered heteroaryl ring containing one, two or three N atoms, for example a phenyl group or a pyrazolyl group, a pyrimidinyl group or a pyridinyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted.

Preferably, a C or N atom within group $R^8$ is bonded to LINK, wherein the C or N atom that is bonded to LINK is a C or N atom of the aryl, heteroaryl, heterocyclyl or cycloalkyl ring of $R^8$.

In a typical embodiment of formula (I), the bicyclic structure formed by rings A and B has the structure:

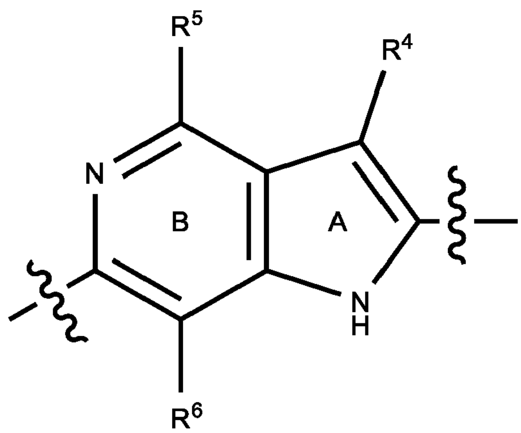

and:

ring Hy is a 5- to 6- membered heterocyclyl ring containing X and at least one N atom, for example a pyrrolidinyl, piperidinyl, morpholinyl or diazinanyl ring; wherein the N atom of ring Hy which is substituted by $R^1$ is adjacent to the C atom of ring Hy that is bonded to ring A;

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)-, O or $-CH_2-$;

ring Hy is further unsubstituted or substituted by one or two $R^3$; wherein each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring;

$R^4$ is selected from H, CN, halo (e.g. fluoro or chloro), methoxy, methyl and trifluoromethyl;

$R^5$ and $R^6$ are independently selected from H, halo (e.g. fluoro or chloro), methoxy, methyl and trifluoromethyl;

the C atom of L is bonded to $R^8$, and the N atom of L is bonded to ring B;

$R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring; wherein the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from halo and $C_{1-2}$ alkoxy; and

$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a preferred embodiment of formula (I), the bicyclic structure formed by rings A and B has the structure:

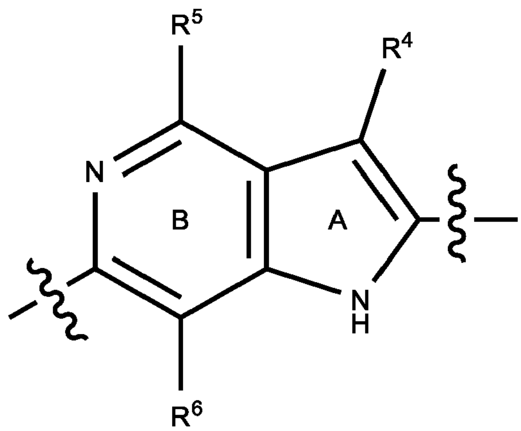

and:

ring Hy is a pyrrolidinyl ring; wherein the N atom of ring Hy which is substituted by $R^1$ is adjacent to the C atom of ring Hy that is bonded to ring A; $R^1$ is H, methyl, ethyl or methoxyethyl, more preferably methyl; $R^2$ is H; and ring Hy is substituted by no $R^3$ groups;

either $R^4$, $R^5$ and $R^6$ are H, or one of $R^4$, $R^5$ and $R^6$ is defined as described above except that it is not H, and the rest are H;

the C atom of L is bonded to $R^8$, and the N atom of L is bonded to ring B;

$R^8$ is phenyl or a 5- to 6-membered heteroaryl ring containing one, two or three N atoms; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a particular embodiment of the compounds of the invention, the MLLT1 and/or MLLT3 binder group M is a monovalent moiety of formula (II):

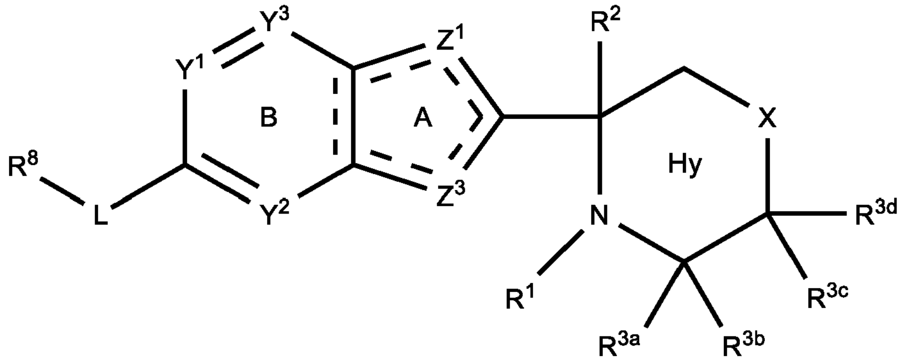

(II)

wherein:

$Z^1$, $Z^3$, $Y^1$, $Y^2$, $Y^3$, $R^1$, $R^2$, $R^8$, X and L are as defined herein;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -S(O)$_2$- or -S(O)(N$R^{11}$)-, then neither $R^{3c}$ nor $R^{3d}$ are halo;
wherein at least two of $R^{3a}$, $R^{3b}$, $R^{3c}$ and $R^{3d}$ are H; and
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

Typically, in formula (II): $R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is -N($R^{11}$)-, O, S, -S(O)$_2$- or-S(O)(N$R^{11}$)-, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.
Optionally, one of $R^{3a}$, $R^{3b}$, $R^{3c}$ and $R^{3d}$ is replaced with a single bond to LINK.
Preferably, in formula (II), $R^{3a}$, $R^{3b}$, $R^{3c}$ and $R^{3d}$ are all H.
In a typical embodiment of formula (II), the bicyclic structure formed by rings A and B has the structure:

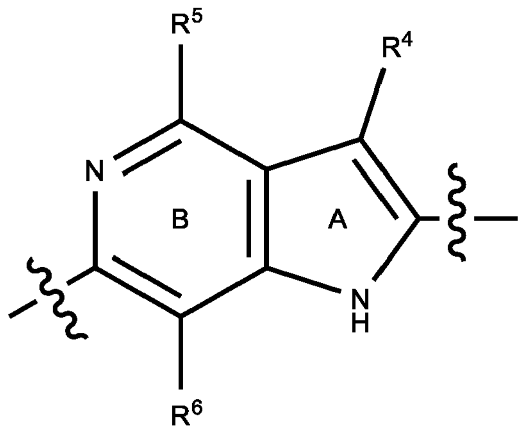

and:

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)-, O or $-CH_2-$;

$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;

$R^4$ is selected from H, CN, halo (e.g. fluoro or chloro), methoxy, methyl and trifluoromethyl;

$R^5$ and $R^6$ are independently selected from H, halo (e.g. fluoro or chloro), methoxy, methyl and trifluoromethyl;

the C atom of L is bonded to $R^8$, and the N atom of L is bonded to ring B;

$R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring; wherein the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; and

$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a preferred embodiment of formula (II), the bicyclic structure formed by rings A and B has the structure:

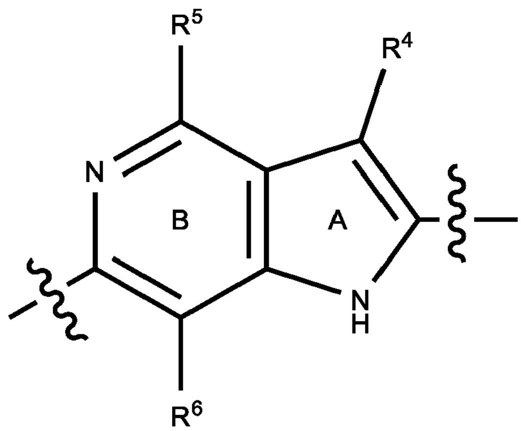

and:

X is absent;

$R^1$ is H, methyl, ethyl or methoxyethyl, more preferably methyl;

$R^2$ is H;

$R^{3a}$, $R^{3b}$ $R^{3c}$ and $R^{3d}$ are all H;

either $R^4$, $R^5$, and $R^6$, are H, or one of $R^4$, $R^5$, and $R^6$ is selected from CN, fluoro, methoxy, methyl and trifluoromethyl, and the rest are H, with the proviso that $R^5$, and $R^6$ cannot be CN;

the C atom of L is bonded to $R^8$, and the N atom of L is bonded to ring B;

$R^8$ is phenyl or a 5- to 6-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted; and

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a particularly preferred embodiment of the compounds of the invention, the MLLT1 and/or MLLT3 binder M is a monovalent moiety of formula (III):

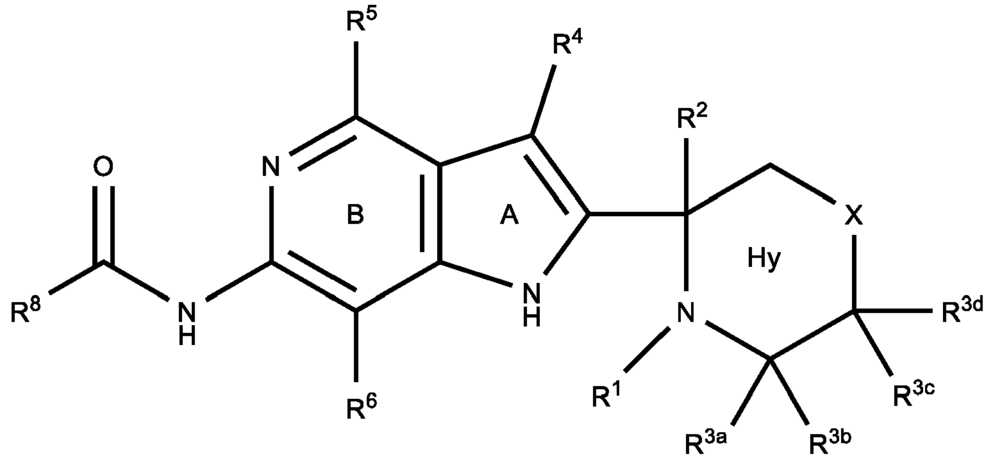

(III)

wherein $R^1$, $R^2$, $R^{3a}$, $R^{3b}$, $R^{3c}$, $R^{3d}$, X, $R^4$, $R^5$, $R^6$ and $R^8$ are as defined herein.

Typically, in the compounds of formula (III):

$R^1$ is H or $C_{1-4}$ alkyl which is itself unsubstituted or substituted with $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N(Me)- or O, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from H, halo $C_{1-4}$ alkoxy, and $C_{1-4}$ alkyl which is itself unsubstituted or substituted by one, two or three halo;
$R^5$ and $R^6$ are independently selected from H, halo $C_{1-4}$ alkoxy, and $C_{1-4}$ alkyl which is itself unsubstituted or substituted by one, two or three halo;
$R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two R9;
each $R^9$ is independently selected from halo and $C_{1-4}$ alkoxy; and
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
wherein either $R^8$ is (i) a bond to LINK, or $R^8$ is a group (ii) and M is bonded to LINK via a C or N atom within group $R^8$ or ring Hy (as described herein) such that a hydrogen atom on the C or N atom within group $R^8$ or ring Hy is replaced with a bond to LINK.

Typically, in formula (III), $R^1$ is H, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy. Preferably, $R^1$ is H, methyl, ethyl or methoxyethyl. Most preferably, $R^1$ is methyl.

In formula (III), $R^2$ is H or methyl. Preferably, $R^2$ is H.

Typically, in formula (III), X is a bond, $-N(R^{11})-$, O or $-C(R^{11})_2-$. Preferably, X is a bond, -N(Me)-, O or $-CH_2-$, most preferably X is a bond.

Typically, in formula (III), each $R^{11}$ is independently selected from H and methyl. When X is $-N(R^{11})-$, $R^{11}$ is preferably methyl. When X is $-C(R^{11})_2-$, one $R^{11}$ is optionally replaced with a single bond to LINK and the other $R^{11}$ is H. When X is$-C(R^{11})_2-$, each $R^{11}$ is preferably H.

Typically, in formula (III), $R^{3a}$ and $R^{3b}$ are independently selected from H, $C_{1-4}$ alkyl, halo and $C_{1-4}$ alkoxy, in particular from H and methyl; $R^{3c}$ and $R^{3d}$ are independently selected from H, methyl, ethyl, fluoro, methoxy and t-butyl, or

$R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.

Preferably, in formula (III), $R^{3a}$, $R^{3b}$, $R^{3c}$ and $R^{3d}$ are all H.

Typically, in formula (III), $R^4$ is selected from H, CN, halo, $C_{1-4}$ alkoxy, and $C_{1-4}$ alkyl which is itself unsubstituted or substituted by one, two or three halo. Preferably, $R^4$ is selected from H, CN, F, Cl, Me and $CF_3$.

Typically, in formula (III), $R^5$ is selected from Hand $C_{1-4}$ alkyl. Preferably, $R^5$ is selected from H and methyl, more preferably H.

Typically, in formula (III), $R^6$ is H.

Typically, therefore, in formula (III):

- $R^4$ is selected from H, CN, halo, $C_{1-4}$ alkoxy, and $C_{1-4}$ alkyl which is itself unsubstituted or substituted by one, two or three halo, and $R^5$ and $R^6$ are H; or
- $R^5$ is selected from H and $C_{1-4}$ alkyl, and $R^4$ and $R^6$ when present are H; or
- $R^4$, $R^5$, and $R^6$ when present are H.

Preferably, in in formula (III):

- $R^4$ is selected from selected from H, CN, F, Cl, Me and $CF_3$, and $R^5$ and $R^6$ are H; or
- $R^5$ is selected from H and methyl, and $R^4$ and $R^6$ when present are H; or
- $R^4$, $R^5$, and $R^6$ when present are H.

In one preferred embodiment of formula (III), $R^8$ is a group selected from phenyl, pyridinyl, thiazolyl, pyrazolyl, pyrimidinyl, triazolyl, 1,2,4-oxadiazolyl, and pyrazinyl. Preferably, $R^8$ is phenyl or a 5- to 6-membered heteroaryl ring containing one, two or three N atoms.

Typically, in formula (III), $R^8$ is unsubstituted or substituted by one, two or three substituents, for instance one or two substituents, independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy.

Preferably, each substituent is independently selected from fluoro, $R^{10}$ and methyl. Most preferably, each substituent is independently selected from fluoro and methyl. $R^{10}$ is as defined herein.

Typically, in formula (III), $R^{10}$ is a group selected from a 5- to 6-membered heteroaryl ring, a 3- to 6-membered cycloalkyl ring, a 4- to 5-membered heterocyclyl ring, and a phenyl ring. The aryl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl.

Typically, in in formula (III), $R^{10}$ is unsubstituted or substituted by one or two substituents independently selected from fluoro, methyl and methoxy. Preferably, $R^{10}$ is unsubstituted.

In typical embodiments of formula (III):

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)- O or $-C(H)_2-$;

$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;

one of the following options applies: $R^4$ is selected from H, CN, halo, $C_{1-4}$ alkoxy, and $C_{1-4}$ alkyl which is itself unsubstituted or substituted by one, two or three halo, and $R^5$ and $R^6$ are H; or $R^5$ is selected from H and $C_{1-4}$ alkyl, and $R^4$ and $R^6$ when present are H; or

$R^4$, $R^5$, and $R^6$ when present are H;

the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself

unsubstituted or substituted by one or two groups selected from selected from halo and $C_{1-2}$ alkoxy; and $R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy; and $R^8$ is a a 6-membered aryl ring, or a 5- to 6-membered heteroaryl ring;

wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In preferred embodiments of formula (III),

X is absent;
$R^1$ is H, methyl, ethyl or methoxyethyl, more preferably methyl;
$R^2$ is H;
$R^{3a}$, $R^{3b}$, $R^{3c}$ and $R^{3d}$ are all H;
$R^5$ and $R^6$ are H, and $R^4$ is selected from H, CN, F, Cl, Me and $CF_3$, preferably H, CN or Cl, more preferably H;
$R^8$ is phenyl or a 5- to 6-membered heteroaryl ring containing one, two or three N atoms; the group $R^8$ is unsubstituted or substituted by one or two substituents, each independently selected from fluoro, $R^{10}$ and methyl;
$R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, preferably pyrazolyl; and $R^{10}$ is unsubstituted;
wherein either $R^8$ is (i) a bond to LINK, or $R^8$ is a group (ii) and M is bonded to LINK via a C or N atom within group $R^8$ or ring Hy such that a hydrogen atom on the C or N atom within group $R^8$ or ring Hy is replaced with a bond to LINK.

In a most preferred embodiment of the compounds of the invention, the MLLT1 and/or MLLT3 binder M is a monovalent moiety of formula (IV):

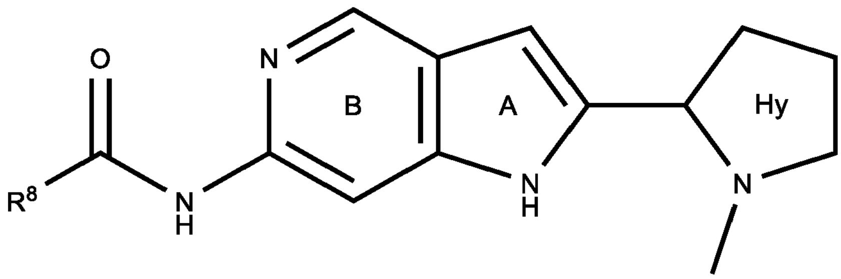

(IV)

wherein $R^8$ is as defined herein. In particular in formula (IV), the group $R^8$ may be unsubstituted or substituted by one, two or three substituents independently selected from $R^{10}$, halo and $C_{1-4}$ alkyl;
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

Typically, in formula (IV), $R^8$ is a group selected from phenyl, thiazolyl, pyrazolyl, pyrimidinyl, triazolyl, 1,2,4-oxadiazolyl, pyridinyl and pyrazinyl.

Typically, in formula (IV), $R^8$ is unsubstituted or substituted by one, two or three substituents, for instance one or two substituents, independently selected from halo and $C_{1-4}$ alkyl and/or by one group $R^{10}$, wherein $R^{10}$ is preferably pyrazolyl; preferably $R^8$ is unsubstituted or substituted by one, two or three substituents, for instance one or two substituents, independently selected from halo and $C_{1-4}$ alkyl. Preferably, each substituent is independently selected from fluoro and methyl.

In a particularly preferred embodiment, in formula (IV), $R^8$ is selected from one of the following structures:

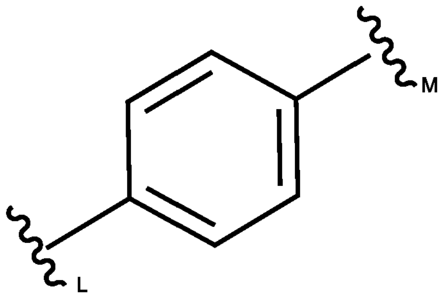

A

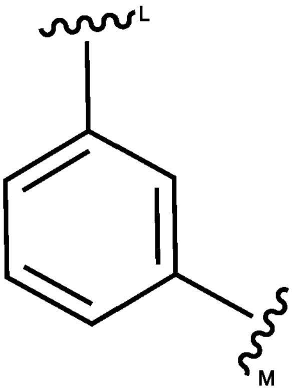

B

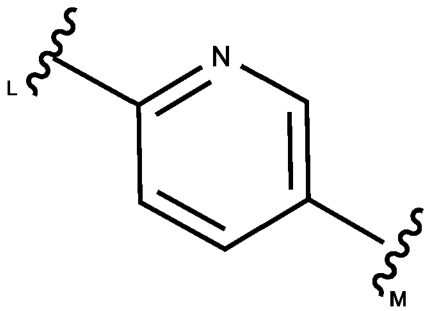

C

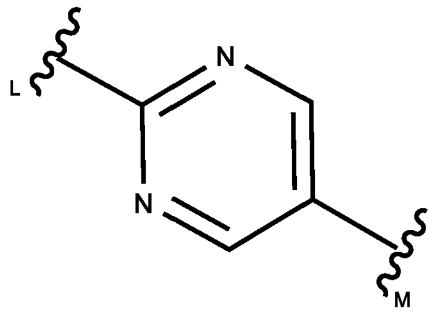

D

wherein:

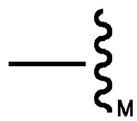

represents the point of attachment of $R^8$ to the rest of moiety M; and

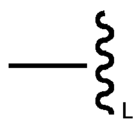

represents the point of attachment of $R^8$ to LINK or $R^{10}$.

When

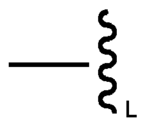

represents the point of attachment to $R^{10}$, then $R^{10}$ is attached to LINK.

For the avoidance of doubt, the group $R^8$ is further unsubstituted or substituted as described above. Preferably, the group $R^8$ is further unsubstituted or substituted by one or two substituents independently selected from fluorine and chlorine.

For the avoidance of doubt, $R^8$ may be selected from one of structures A, B, C and D in any of formulae (I), (II) and (III).

*E3 ubiquitin ligase binding moieties*

The presence of the E3 ubiquitin ligase binding moiety U in the compound of the invention means that the compound is a so-called "PROTAC".

The term PROTAC is an acronym for proteolysis targeting chimera. In general, PROTACs are, as is known in the art, heterobifunctional molecules that comprise two active moieties attached covalently by a linker group. In a PROTAC, the first active moiety (the MLLT1 and/or MLLT3 binder M in the compound of the present invention) binds to a target protein that is intended for degradation (target proteins for the compound of the present invention are MLLT1 and/or MLLT3). The second active moiety (U in the compound of the present invention) is capable of binding to an E3 ubiquitin ligase, thereby inducing selective intracellular proteolysis. Recruitment of the E3 ligase to the target protein results in ubiquitination and subsequent degradation of the target protein by the proteasome.

E3 ubiquitin ligase moities are known in the art. Substantially any such moiety can be used. The sole limitation on the moiety is that it be capable of binding to an E3 ubiquitin ligase. Those skilled in the art would appreciate that entirely routine laboratory methods can be used to determine whether a given substance binds to an E3 ubiquitin ligase (including but not limited to any of those disclosed specifically herein). Thus, those skilled in the art would have no difficulty in identifying E3 ubiquitin ligase moieties, nor in establishing whether any existing chemical moiety falls within the bounds of this definition. In certain embodiments, the moiety shows activity or binds to the E3 ubiquitin ligase with an $IC_{50}$ of less than about 200 mM. The $IC_{50}$ can be determined according to any method known in the art, e.g., a fluorescent polarization assay.

Merely by way of example of the extensive disclosure in the field concerning PROTACs, and hence E3 ubiquitin ligase binding moieties, reference can be made to Gu et al. (BioEssays 2018, 40, 1700247), Sun et al. (Signal Transduction and Targeted Therapy (2019) 4:64), WO 2020/041331, and WO 2019/140003, the contents of all of which are herein incorporated by reference in their entireties. Any of the numerous E3 ubiquitin ligase binding moieties disclosed in these documents can be used as an E3 ubiquitin ligase binding moiety in the compounds of the present invention. For the avoidance of doubt, Gu et al. refer to such moieties as ligands to recruit E3 ubiquitin ligase, Sun et al. refer to such moieties as E3 ubiquitin ligase (E3) recruiting ligands, in WO 2020/041331 such moieties are referred to as a "ULM" or (small molecule) E3 ubiquitin ligase binding moiety (that binds an E3 ubiquitin ligase) (and noting that the term "ULM" includes each of "ILM", "CLM", "VLM" and "MLM", any of which can be used in the present compounds), and in WO 2019/140003 such moieties (labelled "B" in WO 2019/140003's formula (I)) are referred to as a ubiquitin ligase ligand/binder.

Further examples of documents disclosing suitable moieties that can be used in the compounds of the present invention are WO2013/106643, US2016/0045607, WO2014187777, US20140356322 and US 9,249, 153, US2016/0058872, US2015/0291562 and Winter et al (Science, June 19, 2015, p. 1376), the contents of all of which are herein incorporated by reference in their entireties. Thalidomide, lenalidomide, pomalidomide and analogs thereof are still further examples of suitable moieties.

Examples of E3 ubiquitin ligases include von Hippel-Lindau (VHL) and cereblon (CRBN).

Preferably the E3 ubiquitin ligase binding moiety (U) is capable of binding to CRBN or VHL. Such moieties are referred to herein as CRBN or VHL E3 ubiquitin ligase binding moieties.

In one embodiment, U is a CRBN E3 ubiquitin ligase binding moiety. In another embodiment, U is a VHL E3 ubiquitin ligase binding moiety.

In some embodiments, the E3 ubiquitin ligase binding moiety (U) is:

(a) a CRBN E3 ubiquitin ligase binding moiety of formula (U1):

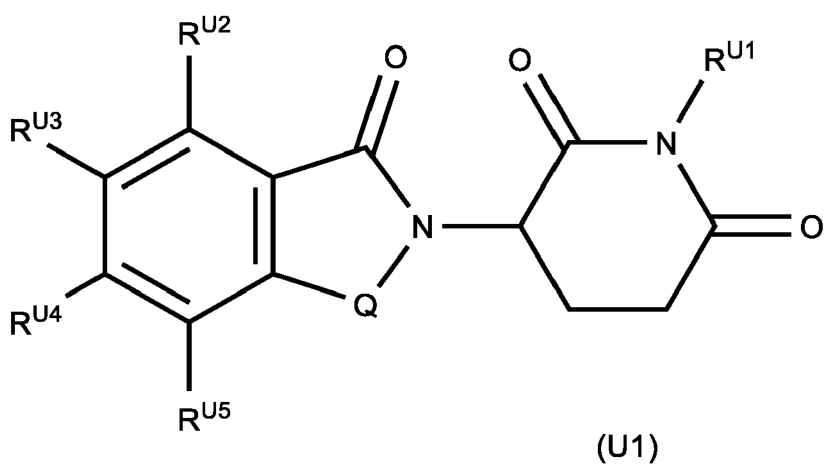

(U1)

wherein:

$R^{U1}$ is H;
Q is selected from $-CH(R^{U6})-$, $-N(R^{U6})-$, -O-, -C(O)-, $-NH-CH(R^{U6})-$, $-N=C(R^{U6})-$, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

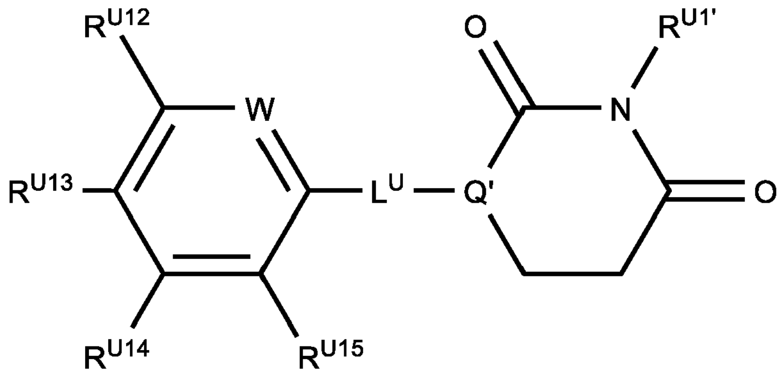

(U4)

wherein:

$R^{U1'}$ is H;
W is N or $CR^{U16}$;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK; wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

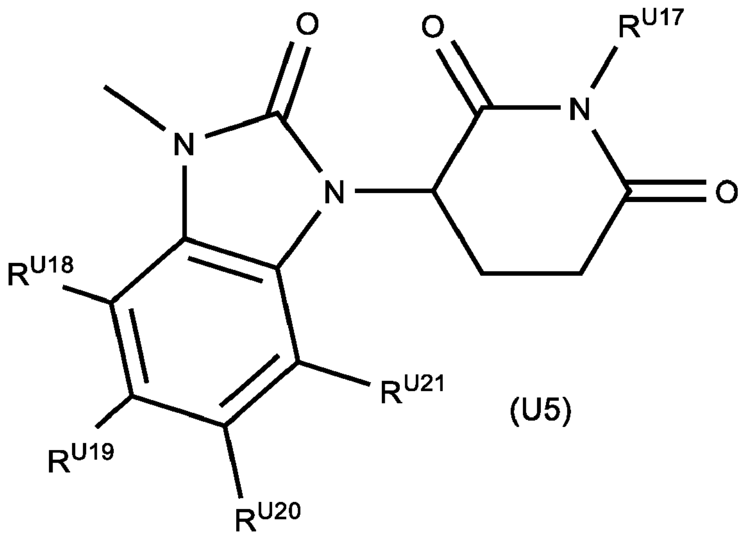

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;
wherein one and only one of $R^{U18}$, $R^{U19}$, $R^{U20}$ and $R^{U21}$ is a single bond to LINK;
or (d) a VHL E3 ubiquitin ligase binding moiety of formula (U2):

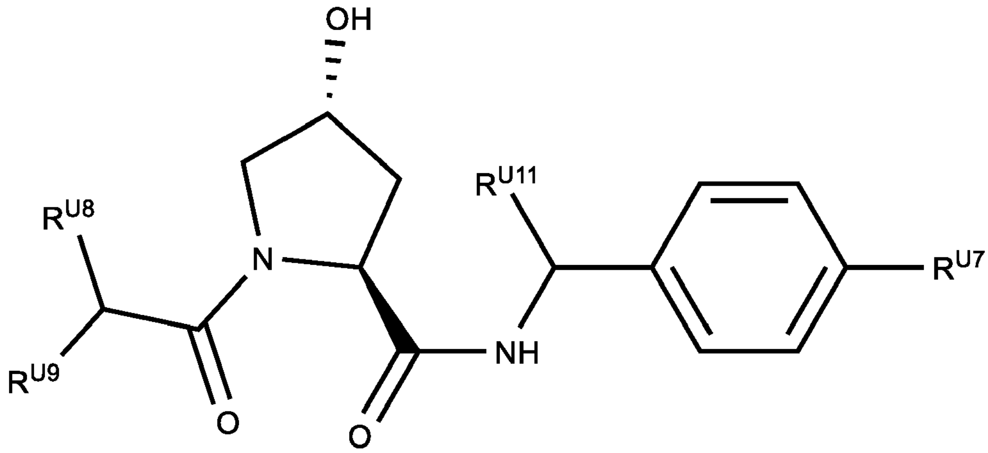

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

For the avoidance of doubt, in formula (U1), when Q is -NH-CH($R^{U6}$)- or -N=C($R^{U6}$)-, the N atom of Q is bonded to the phenyl ring in U, and the C atom of Q (not including any C atom that may be in $R^{U6}$) is bonded to the N atom in U that is adjacent to group Q.

Typically, in formula (U1), Q is selected from -CH($R^{U6}$)- and -C(O)-. Preferably, Q is selected from $-CH_2-$ and -C(O)-.

Typically, in formula (U1), $R^{U6}$ is H or methyl. Preferably, $R^{U6}$ is H.

Typically, in formula (U1), $R^{U2}$ and $R^{U5}$ are each independently selected from H, fluoro, methyl, $NH_2$, $NO_2$, OH, COOH, CN, $CF_3$, and a single bond to LINK. Preferably, $R^{U2}$ and $R^{U5}$ are each independently selected from H and a single bond to LINK.

Typically, in formula (U1), $R^{U3}$ and $R^{U4}$ are each independently selected from H, fluoro, methyl, $NH_2$, $NO_2$, OH, COOH, CN, $CF_3$ and a single bond to LINK. Preferably, $R^{U3}$ and $R^{U4}$ are each independently selected from H and a single bond to LINK.

Typically, in formula (U1), two or three of $R^{U2}$, $R^{U3}$, $R^{U4}$ and $R^{U5}$ are H. Preferably, three of $R^{U2}$, $R^{U3}$, $R^{U4}$ and $R^{U5}$ are H. More preferably, $R^{U2}$ and $R^{U3}$ are H, one of $R^{U4}$ and $R^{U5}$ is a single bond to LINK, and the other of $R^{U4}$ and $R^{U5}$ is H.

Typically, therefore, in formula (U1):

Q is selected from -CH($R^{U6}$)- and -C(O)-;
$R^{U6}$ is H or methyl;
$R^{U2}$ and $R^{U5}$ are each independently selected from H, fluoro, methyl, $NH_2$, $NO_2$, OH, COOH, CN, $CF_3$, and a single bond to LINK;
$R^{U3}$ and $R^{U4}$ are each independently selected from H, fluoro, methyl, $NH_2$, $NO_2$, OH, COOH, CN, $CF_3$ and a single bond to LINK; and
two or three of $R^{U2}$, $R^{U3}$, $R^{U4}$ and $R^{U5}$ are H.

Preferably, in formula (U1):

Q is selected from $-CH_2-$ and -C(O)-;
$R^{U6}$ is H;
one of $R^{U2}$, $R^{U3}$, $R^{U4}$ and $R^{U5}$ is a single bond to LINK, and the rest are H, for instance wherein $R^{U2}$ and $R^{U3}$ are H, one of $R^{U4}$ and $R^{U5}$ is a single bond to LINK, and the other of $R^{U4}$ and $R^{U5}$ is H.

Typically, in formula (U4), Q' is N and $L^U$ is a single bond, or Q' is CH and $L^U$ is - C(O)N(H)-. Preferably, Q' is N and $L^U$ is a single bond.

Typically, in formula (U4), when $L^U$ is -C(O)N(H)-, then the C atom of $L^U$ is bonded to phenyl, and the N atom of $L^U$ is bonded to Q'.

Typically, in formula (U4), $R^{U12}$, $R^{U13}$ and $R^{U14}$ are each independently selected from H, fluoro, methyl, $NH_2$, $NO_2$, OH, COOH, CN, $CF_3$, and a single bond to LINK (wherein one and only one of $R^{U12}$, $R^{U13}$ and $R^{U14}$ is a single bond to LINK). Preferably, one of $R^{U12}$, $R^{U13}$ and $R^{U14}$ is a single bond to LINK, one of $R^{U12}$, $R^{U13}$ and $R^{U14}$ is H and the other of $R^{U12}$, $R^{U13}$ and $R^{U14}$ is selected from H, fluoro, methyl, $NH_2$, $NO_2$, OH, COOH, CN, $CF_3$, preferably from H, fluoro and methyl. More preferably, one of $R^{U12}$, $R^{U13}$ and $R^{U14}$ is a single bond to LINK and the other two are H, for instance wherein $R^{U12}$ and $R^{U14}$ are H, and $R^{U13}$ is a single bond to LINK.

Typically, in formula (U4), $R^{U15}$ and $R^{U16}$ (when present) are each independently selected from H, fluoro, methyl, $NH_2$, $NO_2$, OH, COOH, CN and $CF_3$. Preferably, $R^{U15}$ and $R^{U16}$ (when present) are each independently selected from H, F and Me.

In one embodiment of formula (U4), $R^{U15}$ and $R^{U16}$ (when present) are H, and two of $R^{U12}$, $R^{U13}$ and $R^{U14}$ are H. Preferably, $R^{U13}$, $R^{U14}$ and $R^{U15}$ are H, $R^{U12}$ is a single bond to LINK, and $R^{U16}$ (when present) is selected from H, F and Me.

Typically, therefore, in formula (U4):

Q' is N and $L^U$ is a single bond, or Q' is CH and $L^U$ is -C(O)N(H)-;
$R^{U12}$, $R^{U13}$ and $R^{U14}$ are each independently selected from H, fluoro, methyl, $NH_2$, $NO_2$, OH, COOH, CN, $CF_3$, and a single bond to LINK; and
$R^{U15}$ and $R^{U16}$ (when present) are each independently selected from H, fluoro, methyl, $NH_2$, $NO_2$, OH, COOH, CN and $CF_3$;
wherein one and only one of $R^{U12}$, $R^{U13}$ and $R^{U14}$ is a single bond to LINK.

Preferably, in formula (U4):

Q' is N and $L^U$ is a single bond;
one of $R^{U12}$, $R^{U13}$ and $R^{U14}$ is a single bond to LINK and the other two are H, for instance wherein $R^{U12}$ and $R^{U14}$ are H, and $R^{U13}$ is a single bond to LINK, or $R^{U13}$, $R^{U14}$
and $R^{U15}$ are H, $R^{U12}$ is a single bond to LINK, and $R^{U16}$ (when present) is selected from H, F and Me.

Typically, in formula (U5), $R^{U18}$, $R^{U19}$, $R^{U20}$ and $R^{U21}$ are each independently selected from H, fluoro, methyl, $NH_2$, $NO_2$, OH, COOH, CN, $CF_3$, and a single bond to LINK (wherein one and only one of $R^{U18}$, $R^{U19}$, $R^{U20}$ and $R^{U21}$ is a single bond to LINK). Preferably, one of $R^{U18}$, $R^{U19}$, $R^{U20}$ and $R^{U21}$ is a single bond to LINK, one or two, preferably two of $R^{U18}$, $R^{U19}$, $R^{U20}$ and $R^{U21}$ are H and the other one or two, preferably one of $R^{U18}$, $R^{U19}$, $R^{U20}$ and $R^{U21}$ are selected from H, fluoro, methyl, $NH_2$, $NO_2$, OH, COOH, CN, $CF_3$, preferably from H, fluoro and methyl. More preferably, one of $R^{U18}$, $R^{U19}$, $R^{U20}$ and $R^{U21}$ is a single bond to LINK and the other three are H, for instance wherein $R^{U19}$, $R^{U20}$ and $R^{U21}$ are H, and $R^{U18}$ is a single bond to LINK.

Typically, in formula (U2), $R^{U7}$ is a 5- to 6-membered heteroaryl ring. Preferably, $R^{U7}$ is a 5-membered heteroaryl ring containing one S atom and optionally one N atom.

Typically, in formula (U2), $R^{U7}$ is unsubstituted or substituted by $C_{1-4}$ alkyl. Preferably, $R^{U7}$ is unsubstituted or substituted by methyl.

Typically, in formula (U2), $R^{U11}$ is H or methyl. Preferably, $R^{U11}$ is H.

Typically, in formula (U2), $R^{U8}$ is $C_{1-4}$ alkyl or phenyl. Preferably, $R^{U8}$ is t-butyl or phenyl, more preferably t-butyl.

Typically, therefore, in formula (U2), $R^{U7}$ is a 5- to 6-membered heteroaryl ring, the group $R^{U7}$ being unsubstituted or substituted by $C_{1-4}$ alkyl, $R^{U11}$ is H or methyl, and $R^{U8}$ is $C_{1-4}$ alkyl or phenyl.

Preferably, in formula (U2), $R^{U7}$ is a 5-membered heteroaryl ring containing one S atom and optionally one N atom, the group $R^{U7}$ being unsubstituted or substituted by methyl, $R^{U11}$ is H, and $R^{U8}$ is t-butyl or phenyl, more preferably t-butyl.

In a preferred embodiment, the E3 ubiquitin ligase binding moiety (U) is a VHL E3 ubiquitin ligase binding moiety

of formula (U3):

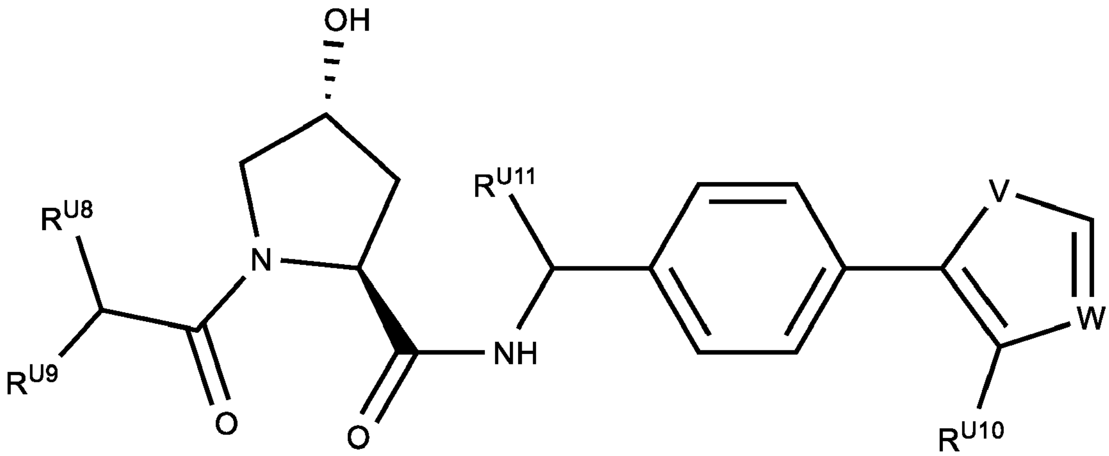

(U3)

wherein:

V is S or O;
W is N or CH;
$R^{U10}$ is H or $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

Typically, in formula (U3), V is S.

Typically, in formula (U3), W is N or CH. Preferably, W is N.

Typically, in formula (U3), $R^{U10}$ is H or methyl. Preferably, $R^{U10}$ is methyl.

Typically, in formula (U3), $R^{U11}$ is H or methyl. Preferably, $R^{U11}$ is H.

Typically, in formula (U3), $R^{U8}$ is $C_{1-4}$ alkyl or phenyl. Preferably, $R^{U8}$ is t-butyl or phenyl, more preferably *t*-butyl.

Typically, therefore, in formula (U3), V is S, W is N or CH, $R^{U10}$ is H or methyl, $R^{U11}$ is H or methyl, and $R^{U8}$ is $C_{1-4}$ alkyl or phenyl. Preferably, V is S, W is N, $R^{U10}$ is methyl, $R^{U11}$ is H, $R^{U8}$ is *t*-butyl or phenyl, more preferably *t*-butyl.

The moieties U of formulae (U1), (U4) when Q' is CH and (U5) typically contain at least one chiral centre at the carbon atoms marked with an asterisk as depicted below:

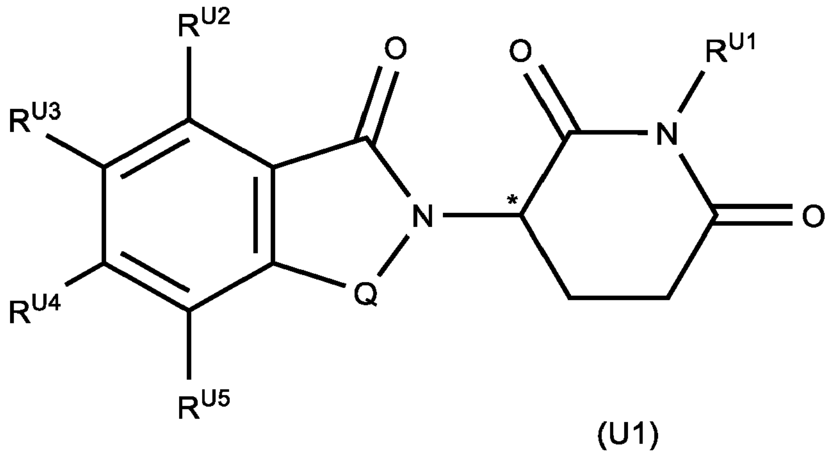

(U1) ;

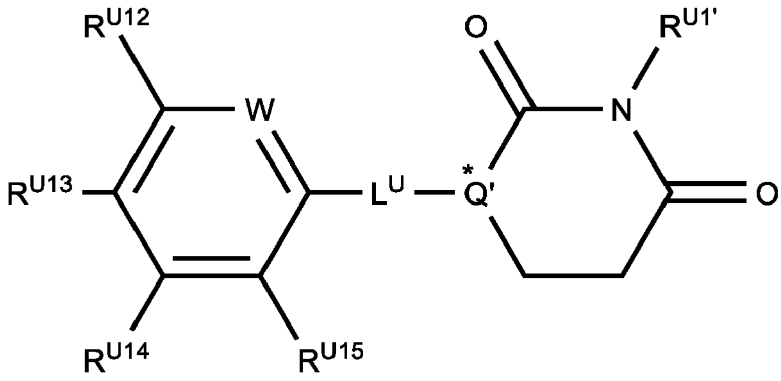

(U4)

;

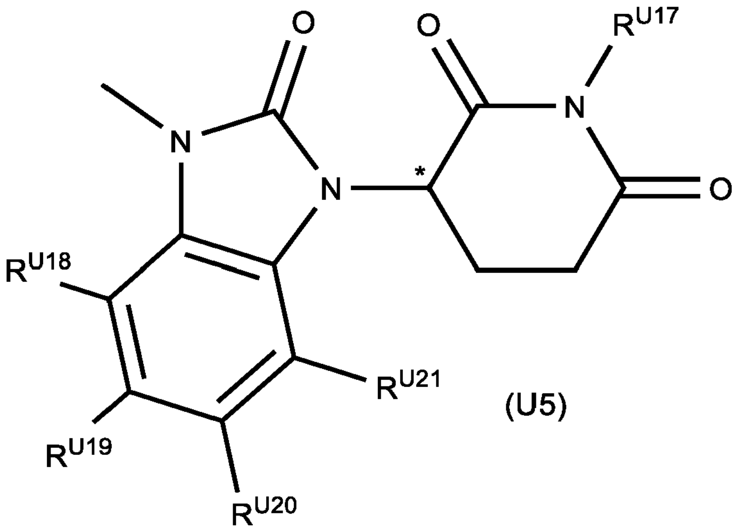

(U5)

.

Moiety U may therefore be provided in the form of the R-enantiomer at said carbon atom, in the form of the S-enantiomer at said carbon atom, or in the form of a mixture (for example a 1:1 mixture) of the two enantiomers. A pure enantiomeric form of either the R- or the S-enantiomer may be preferred. In some embodiments, the S-enantiomer at said carbon atom is preferred. In other embodiments, the R-enantiomer at said carbon atom is preferred. The substance or agent described herein may contain at least 50%, preferably at least 60, 75%, 90% or 95% of a compound which is enantiomerically or diasteriomerically pure at moiety U. Unless otherwise stated, the moiety U may typically be provided in the form of a 1:1 mixture of the two enantiomers.

*Linkers*

As discussed herein, in the compounds of the invention, the binder of MLLT1 and/or MLLT3 (M) is covalently attached to an E3 ubiquitin ligase binding moiety (U). This covalent attachment is labelled LINK and may be a direct single bond or a divalent chemical linker group that forms covalent bonds both to the binder of MLLT1 and/or MLLT3 (M), and to the E3 ubiquitin ligase binding moiety (U). Typically, LINK is a divalent chemical linker group that forms covalent bonds both to the binder of MLLT1 and/or MLLT3 (M), and to the E3 ubiquitin ligase binding moiety (U).

There is no particular limitation on the nature of LINK in the compounds of the present invention (beyond that the respective active moieties, e.g., U and M, are able to exert their desired function and LINK is capable of covalently attaching them together). Those skilled in the art would recognise that chemical linker groups are routinely used in the construction of bifunctional molecules and would be able routinely to provide appropriate chemical linker groups for attaching particular U and M moieties together. Typically, a chemical linker group for use in the present invention is an organic group.

When LINK is a divalent chemical linker group, it may be represented by formula (L):

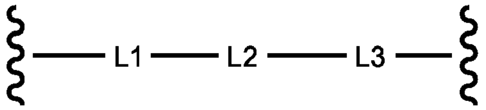

(L)

wherein:

LINK is attached to M via L1;
LINK is attached to U via L3;
L1 and L3 are each independently selected from a single bond, -N(R')-, -C(O)N(R')-, -O-, -N(R')C(O)-, -C(O)-, -S($O_2$)N(R')-, -N(R')S($O_2$)-, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

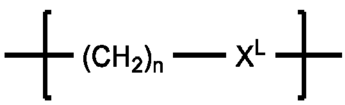
or
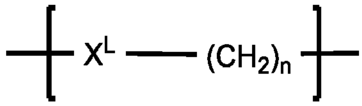
;

each $X^L$ is independently selected from a single bond, -N(R')-, -O-, -C(O)-, -S-, -SO-, - $SO_2$-, -C(H)=C(H)- and -C(H)=C(H)-;
n is selected from 1 to 4;
m is selected from 1 to 30; and
each R' is independently selected from H and $C_{1-4}$ alkyl.

In one embodiment, L1 and L3 are each independently selected from a single bond, - N(R')-, -C(O)N(R')-, -O-, -N(R')C(O)-, -C(O)-, -S($O_2$)N(R')-, -N(R')S($O_2$)-, -($C_{1-6}$ alkylene)-, -($C_{2-6}$ alkenylene)-, ethynyl, a divalent 3- to 6-membered cycloalkyl ring and a divalent 4- to 7-membered heterocyclyl ring;

L2 is represented by the formula $-(L4)_m-$; and
each L4 is independently selected from a divalent 3- to 6-membered cycloalkyl ring, a divalent 4- to 7-membered heterocyclyl ring and a unit of formula:

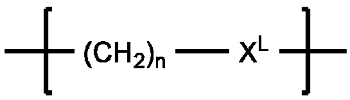
.

When m is not 1 (i.e. when there are two or more L4 groups present), then the $X^L$ group of any L4 group is not directly bonded to the $X^L$ group of any other L4 group that is present.

Typically, in formula (L), L1 is selected from a single bond, branched or straight-chain - ($C_{1-6}$ alkylene)-, -C(O)N(R')-, -N(R')C(O)-, -O-, -S($O_2$)N(R')-, -N(R')S($O_2$)-, and a divalent 4- to 7-membered heterocyclyl ring. When present, the divalent 4- to 7-membered heterocyclyl ring of L1 typically contains at least one N atom, for instance one or two N atoms. Preferably, when L1 is a divalent 4- to 7-membered heterocyclyl ring, it is a divalent 5- to 6-membered heterocyclyl ring, more preferably a saturated divalent 5- to 6-membered heterocyclyl ring, for instance a divalent moiety of piperidine or diazinane. Preferably, in formula (L), L1 is selected from a single bond, branched or straight-chain -($C_{1-6}$ alkylene)-, -C(O)N(H)-, -N(H)C(O)-, and a divalent moiety of piperidine or diazinane.

Typically, in formula (L), L3 is selected from a single bond, -N(R')-, -C(O)N(R')-, -O-, -N(R')C(O)-, -S($O_2$)N(R')-, -N(R')S($O_2$)-, ethynyl and a divalent 4- to 7-membered heterocyclyl ring. When present, the divalent 4- to 7-membered heterocyclyl ring of L3 typically contains at least one N atom, for instance one or two N atoms. Preferably, when L3 is a divalent 4- to 7-membered heterocyclyl ring, it is a divalent 5- to 6-membered heterocyclyl ring, more preferably a saturated divalent 5- to 6-membered heterocyclyl ring, for instance a divalent moiety of piperidine or diazinane, preferably 1,4-diazinane. Preferably, in formula (L), L3 is selected from a single bond, -N(H)-, - C(O)N(H)-, -O-, -N(H)C(O)-, ethynyl and a divalent moiety of piperidine or diazinane.

L2 is represented by the formula $-(L4)_m-$. Typically, in formula (L), each L4 is independently selected from a

divalent 4- to 7-membered heterocyclyl ring and a unit of formula

$$\left[ (CH_2)_n - X^L \right] \quad \text{or} \quad \left[ X^L - (CH_2)_n \right]$$

as described above.

Typically, each $X^L$ is independently selected from a single bond, -O-, -S-, -C(H)=C(H)- and -C(H)=C(H)-, for example a single bond, -O- and -S-, and n is selected from 1 or 2. In one embodiment, each L4 is the same. Alternatively, L2 may comprise two or more blocks, wherein in each block, each L4 is the same. For instance, L2 may be represented by the formula $-(L4')_p-(L4'')_q-(L4''')_r-$, wherein L4', L4" and L4‴ are selected from those moieties described above for L4, and wherein each L4' is the same, each L4" is the same and each L4‴ is the same. Preferably, L4', L4" and L4‴ are selected from $-CH_2-$, $-CH_2CH_2O-$, $-OCH_2CH_2-$, $-CH_2CH_2S-$ and $-SCH_2CH_2-$. More preferably, L4', L4" and L4‴ are selected from $-CH_2-$, $-CH_2CH_2O-$ and $-OCH_2CH_2-$. p, q and r are integers of from 0 to 30, wherein p+q+r=m.

In one embodiment, each L4 unit is the same. For instance, each L4 in formula (L) may be $-CH_2-$, each L4 in formula (L) may be $-CH_2CH_2O-$, each L4 in formula (L) may be - $OCH_2CH_2-$, each L4 in formula (L) may be $-CH_2CH_2S-$, or each L4 in formula (L) may be $-SCH_2CH_2-$. More preferably, each L4 in formula (L) is $-CH_2-$, each L4 in formula (L) is $-CH_2CH_2O-$ or each L4 in formula (L) is $-OCH_2CH_2-$. Most preferably, each L4 in formula (L) is $-CH_2-$.

Typically, in formula (L), m is selected from 1 to 10.

Typically, in formula (L), each R' is independently selected from H and methyl. Preferably, each R' is H.

When one or more L4 is a divalent 4- to 7-membered heterocyclyl ring, typically only one or two, e.g. one L4 is a divalent 4- to 7-membered heterocyclyl ring. Typically, therefore, none, one or two L4 is a divalent 4- to 7-membered heterocyclyl ring, for example none or one L4 is a divalent 4- to 7-membered heterocyclyl ring.

Typically, therefore, in formula (L):

L1 is selected from a single bond, branched or straight-chain -($C_{1-6}$ alkylene)-, - C(O)N(R')-, -N(R')C(O)-, -O-, $-S(O_2)N(R')-$, $-N(R')S(O_2)-$, and a divalent 4- to 7-membered heterocyclyl ring typically containing at least one N atom, for instance one or two N atoms;
L3 is selected from a single bond, -N(R')-, -C(O)N(R')-, -O-, -N(R')C(O)-, $-S(O_2)N(R')-$, $-N(R')S(O_2)-$, ethynyl and a divalent 4- to 7-membered heterocyclyl ring typically containing at least one N atom, for instance one or two N atoms;
each L4 is independently selected from $-CH_2-$, $-CH_2CH_2O-$, $-OCH_2CH_2-$, $-CH_2CH_2S-$, $-SCH_2CH_2-$, and a divalent 4- to 7-membered heterocyclyl ring, wherein when one or more L4 is a divalent 4- to 7-membered heterocyclyl ring, only one or two, e.g. one L4 is a divalent 4- to 7-membered heterocyclyl ring;
m is selected from 1 to 10; and
each R' is independently selected from H and methyl.

Preferably, in formula (L):

L1 is selected from a single bond, branched or straight-chain -($C_{1-6}$ alkylene)-, - C(O)N(H)-, -N(H)C(O)-, and a divalent moiety of piperidine or diazinane;
L3 is selected from a single bond, -N(H)-, -C(O)N(H)-, -O-, -N(H)C(O)-, ethynyl and a divalent moiety of piperidine or diazinane;
each L4 in formula (L) is $-CH_2-$, each L4 in formula (L) is $-CH_2CH_2O-$, each L4 in formula (L) is $-OCH_2CH_2-$, each L4 in formula (L) is $-CH_2CH_2S-$, or each L4 in formula (L) is $-SCH_2CH_2-$, with each L4 preferably being $-CH_2-$;
m is selected from 1 to 10; and
each R' is H.

In a typical embodiment, L2 is -($C_{1-10}$ alkylene)- or -($C_{1-10}$ alkylene)-C(O)-, preferably - ($C_{1-10}$ alkylene)-. In this embodiment, L1 is preferably selected from a single bond, - C(O)N(H)-, -N(H)C(O)-, and a divalent moiety of piperidine or diazinane. In this embodiment, L3 is preferably selected from a single bond, -N(H)-, -C(O)N(H)-, -O-, - N(H)C(O)-, ethynyl and a divalent moiety of piperidine or diazinane. More preferably, L1 and L3 are selected from the following options:

- L1 is a single bond and L3 is -N(H)-;
- L1 is a divalent moiety of piperidine and L3 is -N(H)-;
- L1 is -C(O)N(H)- or -N(H)C(O)-, and L3 is -C(O)N(H)- or -N(H)C(O)-;
- L1 is a divalent moiety of piperidine and L3 is a divalent moiety of diazinane;
- L1 is -C(O)N(H)- or -N(H)C(O)-, and L3 is a divalent moiety of diazinane;

- L1 is a single bond and L3 is a divalent moiety of diazinane;
- L1 is a single bond and L3 is a divalent moiety of piperidine;
- L1 is a single bond and L3 is -O-;
- L1 is a single bond and L3 is ethynyl;
- L1 is a single bond and L3 is a single bond; and
- L1 is -C(O)N(H)- or -N(H)C(O)-, and L3 is -O-.

For the avoidance of doubt, the skilled person would readily appreciate that each of the definitions of moieties U, M and LINK provided herein may be taken together in any combination to arrive at a definition of the compound of the present invention. As examples that are in no way limiting, definitions of moieties U, M and LINK labelled "typically" may be combined to arrive at a definition of a typical compound of the present invention, and definitions of moieties U, M and LINK labelled "preferably" may be combined to arrive at a definition of a preferred compound of the present invention. However, the skilled person would appreciate that a definition labelled "typically" of one moiety may be combined with a definition labelled "preferably" of another moiety to arrive at a definition of a compound of the present invention.

In preferred compounds of the present invention, M is of formula (III):

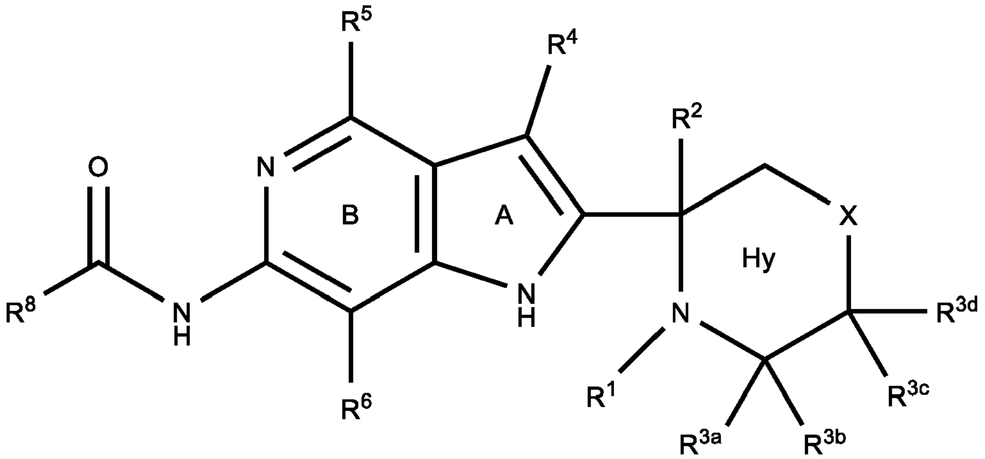

(III)

wherein $R^1$, $R^2$, $R^{3a}$, $R^{3b}$, $R^{3c}$, $R^{3d}$, X, $R^4$, $R^5$, $R^6$ and $R^8$ are as defined herein.

Typically, in such preferred compounds:

$R^1$ is H or $C_{1-4}$ alkyl which is itself unsubstituted or substituted with $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is O or -N(Me)-, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from H, halo $C_{1-4}$ alkoxy, and $C_{1-4}$ alkyl which is itself unsubstituted or substituted by one, two or three halo;
$R^5$ and $R^6$ are independently selected from H, halo $C_{1-4}$ alkoxy, and $C_{1-4}$ alkyl which is itself unsubstituted or substituted by one, two or three halo;
$R^8$ is phenyl or a 5- to 6-membered heteroaryl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;
each $R^9$ is independently selected from halo and $C_{1-4}$ alkoxy; and $R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
wherein either $R^8$ is (i) a bond to LINK, or $R^8$ is a group (ii) and M is bonded to LINK via a C or N atom within group $R^8$ or

ring Hy (as described herein) such that a hydrogen atom on the C or N atom within group $R^8$ or ring Hy is replaced with a bond to LINK; and

LINK is (a) a single bond, or (b) a chemical linker group represented by formula (L):

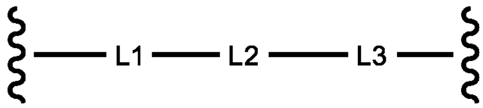

(L)

wherein:

LINK is attached to M via L1;

LINK is attached to U via L3;

L1 and L3 are each independently selected from a single bond, branched or straight-chain -($C_{1-6}$ alkylene)-, -N(R')-, -C(O)N(R')-, -O-, -N(R')C(O)-, -C(O)-, -S($O_2$)N(R')-, -N(R')S($O_2$)-, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;

L2 is represented by the formula $-(L4)_m-$;

each L4 is independently selected from a divalent 3- to 6-membered cycloalkyl ring, a divalent 4- to 7-membered heterocyclyl ring and a unit of formula:

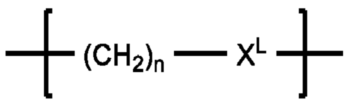 or 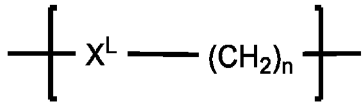 ;

each $X^L$ is independently selected from a single bond, -N(R')-, -O-, -C(O)-, -S-, -SO-, - $SO_2$-, -C(H)=C(H)- and -C(H)=C(H)-;

n is selected from 1 to 4;

m is selected from 1 to 30;

each R' is independently selected from H and $C_{1-4}$ alkyl; and

U is:

(a) a CRBN E3 ubiquitin ligase binding moiety of formula (U1):

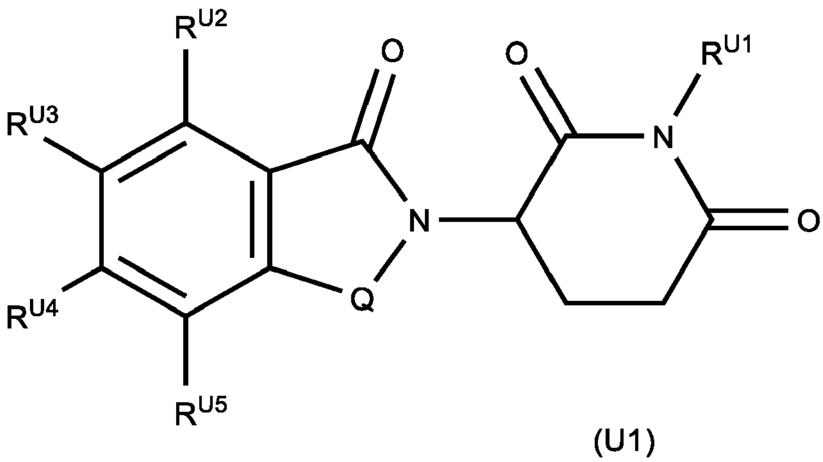

(U1)

wherein:

$R^{U1}$ is H;

Q is selected from -CH($R^{U6}$)-, -N($R^{U6}$)-, -O-, -C(O)-, -NH-CH($R^{U6}$)-, -N=C($R^{U6}$)-, or -N=N-;

$R^{U6}$ is H or $C_{1-4}$ alkyl;

$R^{U2}$ and $R^{U5}$ are each independently selected from H, halogen, $C_{1-4}$ alkyl, $NH_2$, $NO_2$, OH, COOH, CN, $CF_3$, and a single bond to LINK;

$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

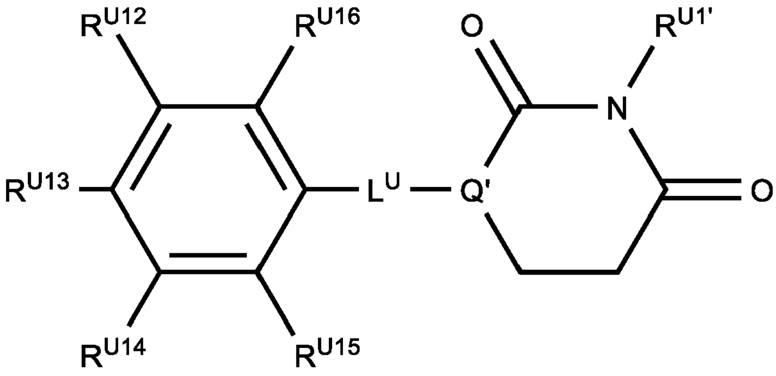

(U4)

wherein:

$R^{U1'}$ is H;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

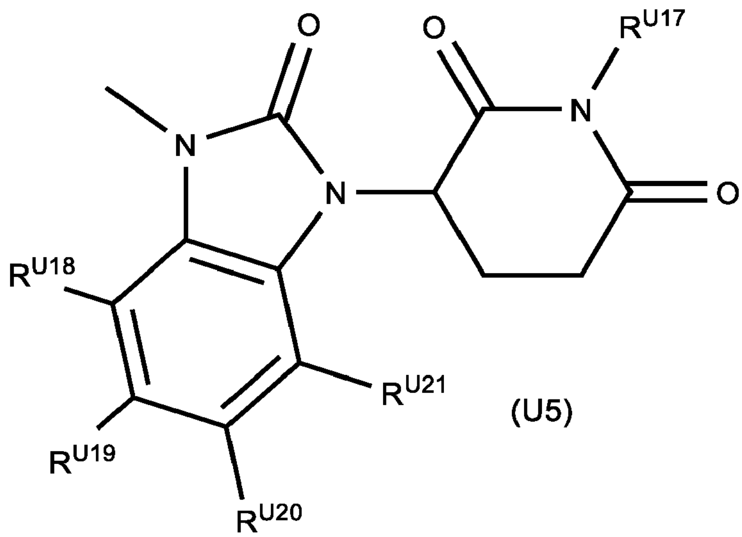

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;

wherein one and only one of $R^{U18}$, $R^{U19}$, $R^{U20}$ and $R^{U21}$ is a single bond to LINK;
or (d) a VHL E3 ubiquitin ligase binding moiety of formula (U2):

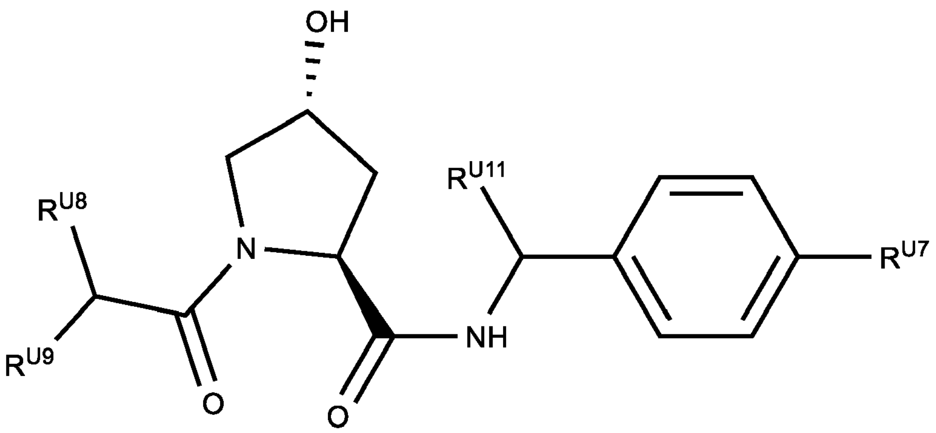

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

In one preferred aspect of this embodiment, M is of formula (IV):

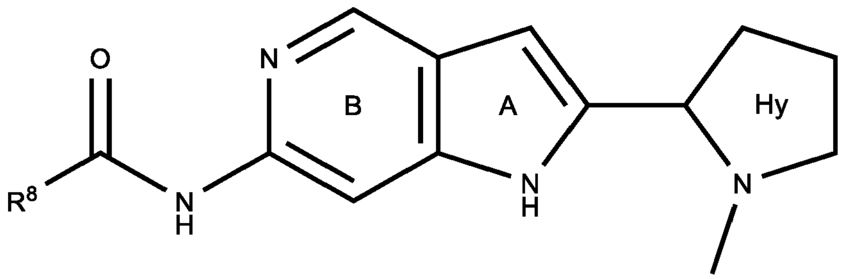

(IV)

wherein $R^8$ is as described herein, the group $R^8$ being unsubstituted or substituted by one, two or three substituents independently selected from $R^{10}$, halo and $C_{1-4}$ alkyl; wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.
In this embodiment, $R^8$ is preferably selected from one of the following structures:

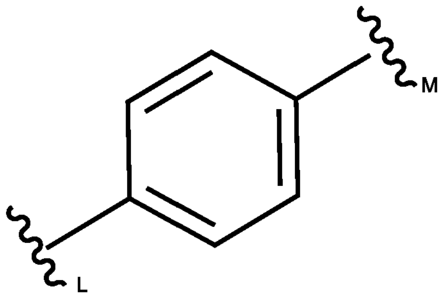

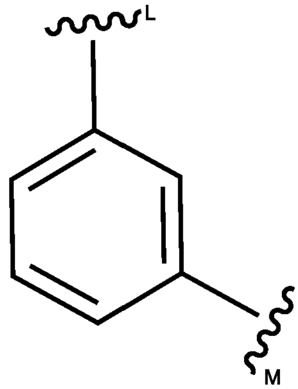

A

B

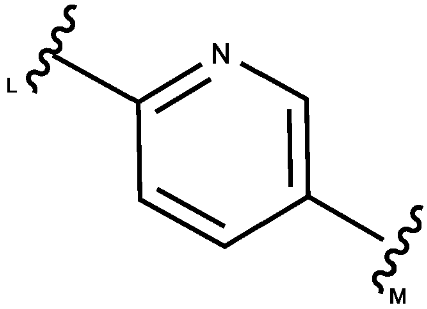

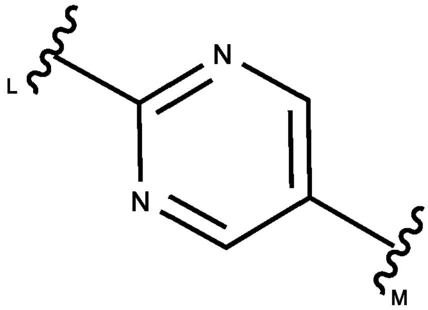

C

D

wherein:

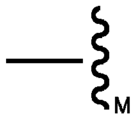

represents the point of attachment of $R^8$ to the rest of moiety M;

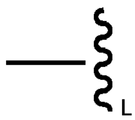

represents the point of attachment of $R^8$ to LINK or $R^{10}$; and the group $R^8$ is further unsubstituted or substituted by one or two substituents independently selected from $R^{10}$, fluorine and methyl.

In this embodiment, L2 is preferably selected from $-(CH_2)_m-$, $-(CH_2CH_2O)_m-$, - $(OCH_2CH_2)_m-$, $-(CH_2CH_2S)_m-$, $-(SCH_2CH_2)_m-$, and a divalent 4- to 7-membered heterocyclyl ring, preferably from $-(CH2)_m$ and a divalent 4- to 7-membered heterocyclyl ring.

In this embodiment, preferably L1 and L3 are each independently selected from a single bond, branched or straight-chain $-(C_{1-6}$ alkylene)-, -N(R')-, -C(O)N(R')-, -N(R')C(O)-, - O-, ethynyl and a divalent 4- to 7-membered heterocyclyl ring, preferably L1 and L3 are each independently selected from a single bond, branched or straight-chain -(C1-6 alkylene)-, -N(R')-, -C(O)N(R')-, -N(R')C(O)-, ethynyl, piperidinyl and diazinanyl. Preferably in this embodiment, R' is H and the divalent 4- to 7-membered heterocyclyl ring is piperidine or diazinane.

In this embodiment, U is preferably (i) of formula (U1) wherein preferably Q is selected from $-CH_2-$ and -C(O)-, $R^{U6}$ is H, one of $R^{U2}$, $R^{U3}$, $R^{U4}$ and $R^{U5}$ is a single bond to LINK, and the rest are H, for instance wherein $R^{U2}$ and $R^{U3}$ are H, one of $R^{U4}$ and $R^{U5}$ is a single bond to LINK, and the other of $R^{U4}$ and $R^{U5}$ is H, or (ii) of formula (U3) wherein preferably V is S, W is N, $R^{U10}$ is methyl, $R^{U11}$ is H, and $R^{U8}$ is t-butyl or phenyl, more preferably t-butyl.

Particularly preferred compounds of the invention may be selected from:

4-(4-(2-(2,6-dioxopiperidin-3-yl)-1-oxoisoindolin-4-yl)but-3-yn-1-yl)-N-(2-((R)-1-methylpyrrolidin-2-yl)-1H-pyrrolo[3,2-c]pyridin-6-yl)piperidine-1-carboxamide;
6-((3-(2-(2,6-dioxopiperidin-3-yl)-1-oxoisoindolin-4-yl)prop-2-yn-1-yl)oxy)-N-(2-((R)-1-methylpyrrolidin-2-yl)-1H-pyrrolo[3,2-c]pyridin-6-yl)nicotinamide;
4-((1-(2-(2,6-dioxopiperidin-3-yl)-1,3-dioxoisoindolin-4-yl)piperidin-4-yl)methyl)-N-(2-((R)-1-methylpyrrolidin-2-yl)-1H-pyrrolo[3,2-c]pyridin-6-yl)benzamide;
4-(2-(1-(2-(2,6-dioxopiperidin-3-yl)-1,3-dioxoisoindolin-4-yl)piperidin-4-yl)ethyl)-N-(2-((R)-1-methylpyrrolidin-2-yl)-1H-pyrrolo[3,2-c]pyridin-6-yl)benzamide;
6-(4-((1-(2-(2,6-dioxopiperidin-3-yl)-1,3-dioxoisoindolin-4-yl)piperidin-4-yl)methyl)piperazin-1-yl)-N-(2-((R)-1-methylpyrrolidin-2-yl)-1H-pyrrolo[3,2-c]pyridin-6-yl)nicotinamide;
6-(4-(3-(2-(2,6-dioxopiperidin-3-yl)-1-oxoisoindolin-4-yl)prop-2-yn-1-yl)piperazin-1-yl)-N-(2-((R)-1-methylpyrrolidin-2-yl)-1H-pyrrolo[3,2-c]pyridin-6-yl)nicotinamide;
N-((S)-2,6-dioxopiperidin-3-yl)-6-(6-(4-((2-((R)-1-methylpyrrolidin-2-yl)-1H-pyrrolo[3,2-c]pyridin-6-yl)carbamoyl)phenyl)hex-1-yn-1-yl)picolinamide;
4-(6-(2-(2, 6-dioxopiperidin-3-yl)-1-oxoisoindolin-4-yl)hex-5-yn-1-yl)-N-(2-((R)-1-methylpyrrolidin-2-yl)-1H-pyrrolo[3,2-c]pyridin-6-yl)benzamide;
(R)-4-(6-(6-(2,4-dioxotetrahydropyrimidin-1(2H)-yl)pyridin-2-yl)hex-5-yn-1-yl)-N-(2-(1-methylpyrrolidin-2-yl)-1H-pyrrolo[3,2-c]pyridin-6-yl)benzamide;
6-(6-(2-(2, 6-dioxopiperidin-3-yl)-1-oxoisoindolin-4-yl) hex-5-yn-1-yl)-N-(2-((R)-1-methylpyrrolidin-2-yl)-1H-pyrrolo[3, 2-c] pyridin-6-yl) nicotinamide;
N-((S)-2,6-dioxopiperidin-3-yl)-5-(5-(4-((2-((R)-1-methylpyrrolidin-2-yl)-1H-pyrrolo[3,2-c]pyridin-6-yl)carbamoyl)phenyl)pent-1-yn-1-yl)picolinamide;
6-(5-(2-(2,6-dioxopiperidin-3-yl)-1-oxoisoindolin-4-yl)pent-4-yn-1-yl)-N-(2-((R)-1-methylpyrrolidin-2-yl)-1H-pyrrolo[3,2-c]pyridin-6-yl)nicotinamide;
N-(3-chloro-2-((R)-1-methylpyrrolidin-2-yl)-1H-pyrrolo [3, 2-c] pyridin-6-yl)-9-(2-(2, 6-dioxopiperidin-3-yl)-1-oxoisoindolin-4-yl) non-8-ynamide;
3-(1-(2-(2,6-dioxopiperidin-3-yl)-1,3-dioxoisoindolin-4-yl)piperidin-4-yl)-N-(2-((R)-1-methylpyrrolidin-2-yl)-1H-pyrrolo[3,2-c]pyridin-6-yl)benzamide;
4-(1-(2-(2,6-dioxopiperidin-3-yl)-1,3-dioxoisoindolin-4-yl)piperidin-4-yl)-N-(2-((R)-1-methylpyrrolidin-2-yl)-1H-pyrrolo[3,2-c]pyridin-6-yl)benzamide;
6-(4-(3-(2-(2,6-dioxopiperidin-3-yl)-1-oxoisoindolin-4-yl)prop-2-yn-1-yl)piperidin-1-yl)-N-(2-((R)-1-methylpyrrolidin-2-yl)-1H-pyrrolo[3,2-c]pyridin-6-yl)nicotinamide;
6-(4-((2-(2,6-dioxopiperidin-3-yl)-1-oxoisoindolin-4-yl)ethynyl)piperidin-1-yl)-N-(2-((R)-1-methylpyrrolidin-2-yl)-1H-pyrrolo[3,2-c]pyridin-6-yl)nicotinamide;
6-(4-((1-(2-(2,6-dioxopiperidin-3-yl)-1,3-dioxoisoindolin-4-yl)piperidin-4-yl)methoxy)piperidin-1-yl)-N-(2-((R)-1-methylpyrrolidin-2-yl)-1H-pyrrolo[3,2-c]pyridin-6-yl)nicotinamide;
4-((4-(2-(2,6-dioxopiperidin-3-yl)-1-oxoisoindolin-4-yl)but-3-yn-1-yl)oxy)-N-(2-((R)-1-methylpyrrolidin-2-yl)-1H-pyrrolo[3,2-c]pyridin-6-yl)benzamide;
4-(1-(5-(2-(2,6-dioxopiperidin-3-yl)-1-oxoisoindolin-4-yl)pent-4-yn-1-yl)-1H-pyrazol-3-yl)-N-(2-((R)-1-methylpyrrolidin-2-yl)-1H-pyrrolo[3,2-c]pyridin-6-yl)benzamide;
4-(1-(5-(2-(2,6-dioxopiperidin-3-yl)-1-oxoisoindolin-4-yl)pent-4-yn-1-yl)-1H-pyrazol-4-yl)-N-(2-((R)-1-methylpyrrolidin-2-yl)-1H-pyrrolo[3,2-c]pyridin-6-yl)benzamide;
4-(1-(5-(2-(2,6-dioxopiperidin-3-yl)-1-oxoisoindolin-5-yl)pent-4-yn-1-yl)-1H-pyrazol-4-yl)-N-(2-((R)-1-methylpyrrolidin-2-yl)-1H-pyrrolo[3,2-c]pyridin-6-yl)benzamide;
3-chloro-4-((3-(2-(2,6-dioxopiperidin-3-yl)-1-oxoisoindolin-4-yl)prop-2-yn-1-yl)oxy)-N-(2-((R)-1-methylpyrrolidin-2-yl)-1H-pyrrolo[3,2-c]pyridin-6-yl)benzamide;
4-(((2S)-4-(2-(2,6-dioxopiperidin-3-yl)-1-oxoisoindolin-4-yl)but-3-yn-2-yl)oxy)-N-(2-((R)-1-methylpyrrolidin-2-yl)-1H-pyrrolo[3,2-c]pyridin-6-yl)benzamide;
4-(((2R)-4-(2-(2,6-dioxopiperidin-3-yl)-1-oxoisoindolin-4-yl)but-3-yn-2-yl)oxy)-N-(2-((R)-1-methylpyrrolidin-2-yl)-1H-pyrrolo[3,2-c]pyridin-6-yl)benzamide;
3 -((9-(2-(2, 6-diox opip eri din-3 -yl)-1, 3 -di ox oi soindolin-4-yl)non-8-yn-1-yl) oxy)-N-(2-((R)-1-methylpyrrolidin-2-yl)-1H-pyrrolo[3,2-c]pyridin-6-yl)benzamide;
7-{[2-(2,6-dioxopiperidin-3-yl)-1,3-dioxoisoindol-5-yl]amino}-N-{2-[(2R)-1-methylpyrrolidin-2-yl]-1H-pyrrolo[3,2-c]pyridin-6-yl}heptanamide;
7-{[2-(2,6-dioxopiperidin-3-yl)-1,3-dioxoisoindol-5-yl]amino}-N-{2-[(2S)-1-methylpyrrolidin-2-yl]-1H-pyrrolo[3,2-c]pyridin-6-yl}heptanamide;
7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}heptanamide;

9-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}nonanamide;
12-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}dodecanamide;
12-{[2-(2,6-dioxopiperidin-3-yl)-1,3-dioxoisoindol-4-yl]amino}-N-{2-[(2S)-1-methylpyrrolidin-2-yl]-1H-pyrrolo[3,2-c]pyridin-6-yl}dodecanamide;
N-{3-chloro-2-[(2R)-1-methylpyrrolidin-2-yl]-1H-pyrrolo[3,2-c]pyridin-6-yl}-7-{[2-(2,6-dioxopiperidin-3-yl)-1,3-dioxoisoindol-4-yl]amino}heptanamide;
4-[1-(3-{1-[2-(2,6-dioxopiperidin-3-yl)-1,3-dioxoisoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide;
4-[1-(3-{1-[2-(2,6-dioxopiperidin-3-yl)-1-oxo-3H-isoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide;
4-(3-{[2-(2,6-dioxopiperidin-3-yl)-1,3-dioxoisoindol-4-yl]amino}propyl)-N-{2-[(2R)-1-methylpyrrolidin-2-yl]-1H-pyrrolo[3,2-c]pyridin-6-yl}benzamide;
4-(3-{[2-(2,6-dioxopiperidin-3-yl)-1,3-dioxoisoindol-4-yl]amino}propyl)-N-{2-[(2S)-1-methylpyrrolidin-2-yl]-1H-pyrrolo[3,2-c]pyridin-6-yl}benzamide;
1-(3-{-[2-(2,6-dioxopiperidin-3-yl)-1,3-dioxoisoindol-4-yl]piperidin-4-yl}propyl)-N-{2-[(2R)-1-methylpyrrolidin-2-yl]-1H-pyrrolo[3,2-c]pyridin-6-yl}pyrazole-4-carboxamide;
2-(((1r,4R)-4-((2-(2,6-dioxopiperidin-3-yl)-1,3-dioxoisoindolin-4-yl)amino)cyclohexyl)oxy)-N-(2-((R)-1-methylpyrrolidin-2-yl)-1H-pyrrolo[3,2-c]pyridin-6-yl)acetamide;
2-(4-((2-(2,6-dioxopiperidin-3-yl)-1,3-dioxoisoindolin-4-yl)amino)butoxy)-N-(2-((R)-1-methylpyrrolidin-2-yl)-1H-pyrrolo[3,2-c]pyridin-6-yl)acetamide;
4-(1-(2-(2,6-dioxopiperidin-3-yl)-1,3-dioxoisoindolin-4-yl)piperidin-4-yl)-N-(2-((R)-1-methylpyrrolidin-2-yl)-1H-pyrrolo[3,2-c]pyridin-6-yl)butanamide;
5-(1-(2-(2,6-Dioxopiperidin-3-yl)-1,3-dioxoisoindolin-4-yl)piperidin-4-yl)-N-(2-((R)-1-methylpyrrolidin-2-yl)-1H-pyrrolo[3,2-c]pyridin-6-yl)pentanamide;
6-(1-(2-(2,6-dioxopiperidin-3-yl)-1,3-dioxoisoindolin-4-yl)piperidin-4-yl)-N-(2-((R)-1-methylpyrrolidin-2-yl)-1H-pyrrolo[3,2-c]pyridin-6-yl)hexanamide;
4-(1-(2-(2,6-dioxopiperidin-3-yl)-1,3-dioxoisoindolin-4-yl)piperidin-3-yl)-N-(2-((R)-1-methylpyrrolidin-2-yl)-1H-pyrrolo[3,2-c]pyridin-6-yl)butanamide;
3-(3-((2-(2,6-dioxopiperidin-3-yl)-1,3-dioxoisoindolin-4-yl)aminoamino)propyl)-N-(2-((R)-1-methylpyrrolidin-2-yl)-1H-pyrrolo[3,2-c]pyridin-6-yl)benzamide;
3-(((1-(2-(2,6-dioxopiperidin-3-yl)-1,3-dioxoisoindolin-4-yl)piperidin-4-yl)methyl)amino)-N-(2-((R)-1-methylpyrrolidin-2-yl)-1H-pyrrolo[3,2-c]pyridin-6-yl)benzamide;
3-(1-(3-(1-(2-(2,6-dioxopiperidin-3-yl)-1,3-dioxoisoindolin-4-yl)piperidin-4-yl)propyl)-1H-pyrazol-4-yl)-N-(2-((R)-1-methylpyrrolidin-2-yl)-1H-pyrrolo[3,2-c]pyridin-6-yl)benzamide;
4-(1-(2-(2,6-dioxopiperidin-3-yl)-1,3-dioxoisoindolin-4-yl)azetidin-3-yl)-N-(2-((R)-1-methylpyrrolidin-2-yl)-1H-pyrrolo[3,2-c]pyridin-6-yl)butanamide;
5-(4-(2-(2,6-dioxopiperidin-3-yl)-1-oxoisoindolin-4-yl)but-3-yn-1-yl)-N-(2-((R)-1-methylpyrrolidin-2-yl)-1H-pyrrolo[3,2-c]pyridin-6-yl)picolinamide;
5-[3-[2-(2,6-dioxo-3-piperidyl)-1-oxo-isoindolin-4-yl]prop-2-ynoxy]-N-[2-(1-methylpyrrolidin-2-yl)-1H-pyrrolo[3,2-c]pyridin-6-yl]pyridine-2-carboxamide;
4-((3-(2-(2,6-dioxopiperidin-3-yl)-1-oxoisoindolin-4-yl)prop-2-yn-1-yl)oxy)-N-(2-((R)-1-methylpyrrolidin-2-yl)-1H-pyrrolo[3,2-c]pyridin-6-yl)benzamide;
4-(4-(2-(2,6-dioxopiperidin-3-yl)-1-oxoisoindolin-4-yl)but-3-yn-1-yl)-N-(2-((R)-1-methylpyrrolidin-2-yl)-1H-pyrrolo[3,2-c]pyridin-6-yl)benzamide;
4-(4-(1-(2,6-dioxopiperidin-3-yl)-3-methyl-2-oxo-2,3-dihydro-1H-benzo[d]imidazol-4-yl)but-3-yn-1-yl)-N-(2-((R)-1-methylpyrrolidin-2-yl)-1H-pyrrolo[3,2-c]pyridin-6-yl)benzamide;
4-(4-(2-(2, 6-dioxopiperidin-3-yl)-1-oxoisoindolin-4-yl) but-3-yn-1-yl)-2-fluoro-N-(2-((R)-1-methylpyrrolidin-2-yl)-1H-pyrrolo [3,2-c] pyridin-6-yl) benzamide;
4-(5-(2-(2,6-dioxopiperidin-3-yl)-1-oxoisoindolin-4-yl)pent-4-yn-1-yl)-N-(2-((R)-1-methylpyrrolidin-2-yl)-1H-pyrrolo[3,2-c]pyridin-6-yl)benzamide;
4-((5-(2-(2,6-dioxopiperidin-3-yl)-1-oxoisoindolin-4-yl)pent-4-yn-1-yl)oxy)-N-(2-((R)-1-methylpyrrolidin-2-yl)-1H-pyrrolo[3,2-c]pyridin-6-yl)benzamide;
4-((3-(2-(2,6-dioxopiperidin-3-yl)-1-oxoisoindolin-4-yl)prop-2-yn-1-yl)oxy)-2-fluoro-N-(2-((R)-1-methylpyrrolidin-2-yl)-1H-pyrrolo[3,2-c]pyridin-6-yl)benzamide;
3-((3-(2-(2,6-dioxopiperidin-3-yl)-1-oxoisoindolin-4-yl)prop-2-yn-1-yl)oxy)-N-(2-((R)-1-methylpyrrolidin-2-yl)-1H-pyrrolo[3,2-c]pyridin-6-yl)benzamide;

4-(1-(6-(2-(2,6-dioxopiperidin-3-yl)-1-oxoisoindolin-4-yl)hex-5-yn-1-yl)-1H-pyrazol-4-yl)-N-(2-((R)-1-methylpyrrolidin-2-yl)-1H-pyrrolo[3,2-c]pyridin-6-yl)benzamide;
N-((S)-2,6-dioxopiperidin-3-yl)-6-(4-(3-(4-(4-((2-((R)-1-methylpyrrolidin-2-yl)-1H-pyrrolo[3,2-c]pyridin-6-yl)carbamoyl)phenyl)-1H-pyrazol-1-yl)propyl)piperidin-1-yl)picolinamide;
N-((S)-2,6-dioxopiperidin-3-yl)-6-(4-(2-(4-(4-((2-((R)-1-methylpyrrolidin-2-yl)-1H-pyrrolo[3,2-c]pyridin-6-yl)carbamoyl)phenyl)-1H-pyrazol-1-yl)ethoxy)piperidin-1-yl)picolinamide;
N-((S)-2,6-dioxopiperidin-3-yl)-6-(5-(4-(4-((2-((R)-1-methylpyrrolidin-2-yl)-1H-pyrrolo[3,2-c]pyridin-6-yl)carbamoyl)phenyl)-1H-pyrazol-1-yl)pent-1-yn-1-yl) picolinamide;
(R)-4-(5-(6-(2,4-dioxotetrahydropyrimidin-1(2H)-yl)pyridin-2-yl)pent-4-yn-1-yl)-N-(2-(1-methylpyrrolidin-2-yl)-1H-pyrrolo[3,2-c]pyridin-6-yl)benzamide;
6-(4-(4-(2-(2,6-dioxopiperidin-3-yl)-1,3-dioxoisoindolin-4-yl)piperazin-1-yl)piperidin-1-yl)-N-(2-((R)-1-methylpyrrolidin-2-yl)-1H-pyrrolo[3,2-c]pyridin-6-yl)nicotinamide;
2-(4-((2-(2,6-dioxopiperidin-3-yl)-1-oxoisoindolin-4-yl)ethynyl)piperidin-1-yl)-N-(2-((R)-1-methylpyrrolidin-2-yl)-1H-pyrrolo[3,2-c]pyridin-6-yl)pyrimidine-5-carboxamide;
4-((3-(1-(2,6-dioxopiperidin-3-yl)-3-methyl-2-oxo-2,3-dihydro-1H-benzo[d]imidazol-4-yl)prop-2-yn-1-yl)oxy)-N-(2-((R)-1-methylpyrrolidin-2-yl)-1H-pyrrolo[3,2-c]pyridin-6-yl)benzamide;
6-(4-(4-(2-(2,6-dioxopiperidin-3-yl)-1-oxoisoindolin-4-yl)phenyl)piperidin-1-yl)-N-(2-((R)-1-methylpyrrolidin-2-yl)-1H-pyrrolo[3,2-c]pyridin-6-yl)nicotinamide;
7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}heptanamide;
(2S,4R)-1-[(2S)-3,3-dimethyl-2-(11-{[4-({2-[(2R)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
(2S,4R)-1-[(2S)-3,3-dimethyl-2-(11-{[4-({2-[(2S)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
N-(3-chloro-2-((R)-1-methylpyrrolidin-2-yl)-1H-pyrrolo[3,2-c]pyridin-6-yl)-6-(6-(2-(2,6-dioxopiperidin-3-yl)-1-oxoisoindolin-4-yl)hex-5-yn-1-yl)nicotinamide;
N-(3-chloro-2-((R)-1-methylpyrrolidin-2-yl)-1H-pyrrolo[3,2-c]pyridin-6-yl)-6-(4-(3-(2-(2,6-dioxopiperidin-3-yl)-1-oxoisoindolin-4-yl)prop-2-yn-1-yl)piperazin-1-yl)nicotinamide;
6-(4-(3-(2-(2,6-dioxopiperidin-3-yl)-1-oxoisoindolin-4-yl)prop-2-yn-1-yl)piperidin-1-yl)-N-(2-((R)-1-ethylpyrrolidin-2-yl)-1H-pyrrolo[3,2-c]pyridin-6-yl)nicotinamide;
6-(6-(2-(2, 6-di ox opip eri din-3 -yl)-1-oxoi s oindolin-4-yl)hex-5 -yn-1-yl)-N-(2-((R)-1-ethylpyrrolidin-2-yl)-1H-pyrrolo[3,2-c]pyridin-6-yl)nicotinamide; and
6-(1'-(2-(2,6-dioxopiperidin-3-yl)-1-oxoisoindolin-4-yl)-[4,4'-bipiperidin]-1-yl)-N-(2-((R)-1-methylpyrrolidin-2-yl)-1H-pyrrolo[3,2-c]pyridin-6-yl)nicotinamide; and the pharmaceutically acceptable salts thereof.

*Therapeutic Efficacy*

The compounds of the present invention are therapeutically useful. The present invention therefore provides compounds as described herein, for use in medicine. The present invention provides compounds as described herein, for use in treating the human or animal body. For the avoidance of doubt, the agent may comprise a compound of the invention in the form of a solvate. Also provided is a pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.

The composition of the invention may be provided as a kit comprising instructions to enable the kit to be used in the methods described herein or details regarding which subjects the method may be used for.

As explained above, the compounds of the invention are useful in treating or preventing various disorders. Disorders for treatment using the compounds of the invention may include cancer. In particular, the compounds of the invention are useful for inducing selective degradation of MLLT1 and/or MLLT3, or inhibiting MLLT1 and/or MLLT3.

Cancer, e.g. acute lymphoblastic leukemia, acute myeloid leukemia, adrenocortical carcinoma, aids-related lymphoma, primary CNS lymphoma, anal cancer, astrocytomas, brain cancer, basal cell carcinoma, bile duct cancer, bladder cancer, bone cancer (e.g. ewing sarcoma, osteosarcoma and malignant fibrous histiocytoma), breast cancer, bronchial tumors, medulloblastoma and other CNS embryonal tumors, cervical cancer, chronic lymphocytic leukemia, chronic myelogenous leukemia, chronic myeloproliferative neoplasms, colorectal cancer, craniopharyngioma, endometrial cancer, ependymoma, esophageal cancer, esthesioneuroblastoma, ewing sarcoma, extragonadal germ cell tumor, intraocular melanoma, retinoblastoma, fallopian tube cancer, gallbladder cancer, gastric cancer, gastrointestinal carcinoid tumor, gastrointestinal stromal tumors (gist), germ cell tumors, extragonadal germ cell tumors, ovarian germ cell tumors, testicular cancer, gestational trophoblastic disease, hairy cell leukemia, hepatocellular cancer, histiocytosis, langerhans cell, hodgkin lymphoma, hypopharyngeal cancer, intraocular melanoma, islet cell tumors, pancreatic neuroendocrine tumors, kaposi sarcoma, kidney (renal cell) cancer, langerhans cell histiocytosis, laryngeal cancer, leukemia, liver cancer, lung cancer (non-small cell, small cell, pleuropulmonary blastoma, and tracheobronchial tumor), lymphoma, malignant fibrous histiocytoma of bone and osteosarcoma, merkel cell carcinoma, mesothelioma, mouth cancer, multiple endocrine neoplasia syndromes, multiple myeloma/plasma cell neoplasms, mycosis fungoides, myelodysplastic syndromes, myelodysplastic/ myeloproliferative neoplasms, myelogenous leukemia, neuroblastoma, non-hodgkin lymphoma, oropharyngeal cancer, osteosarcoma and undifferentiated pleomorphic sarcoma, pancreatic cancer, pancreatic neuroendocrine tumors (islet cell tumors), papillomatosis, paraganglioma, parathyroid cancer, penile cancer, pharyngeal cancer, pheochromocytoma, pituitary tumor, prostate cancer, rectal cancer, retinoblastoma, rhabdomyosarcoma, t-cell lymphoma, testicular cancer, throat cancer, thymoma and thymic carcinoma, thyroid cancer, tracheobronchial tumors and the like is particularly suitable to being treated with the compounds of the invention provided herein.

Typically, the compounds of the invention are for use in treating cancers which are transcriptionally addicted cancers, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma and sarcoma (Cell Rep, 2021, 16, 1087).

Preferably, the compounds of the invention are for use in treating acute myeloid leukaemia (AML) or acute lymphoblastic leukaemia (ALL), such as mixed-lineage leukaemia (MLL) rearranged acute leukemia (AML and ALL) and NPM1 mutant leukemia.

The compounds of the invention may be used as standalone therapeutic agents. Alternatively, they may be used in combination with other active agents such as chemotherapeutic agents, targeted precision medicines or immune oncology agents. For example, they may be used in combination with a Bcl2 targeted drug (for instance venetoclax), FLT3 inhibitor (for instance sorafenib, quizartinib or gilteritinib), EGFR inhibitor (for instance erlotinib, gefitinib, lapatinib or osimertinib), CDK4/6 inhibitor (for instance abemaciclib, palbociclib or ribociclib), a chemotherapy (for instance cytarabine, doxorubicin, gemcitabine, cisplatin or paclitaxel) or an immune checkpoint inhibitor (for instance pembrolizumab, nivolumab, durvalumab or cemiplimab).

When used to treat a cancer, the compounds of the invention may be used in alleviating, ameliorating or preventing aggravation of the symptoms of the cancer. Typically, treating a cancer may comprise reducing progression of the cancer, e.g. increasing progression free survival (PFS) and/or increasing survival e.g. increasing overall survival (OS). Treating a cancer may comprise preventing or inhibiting growth of a tumour associated with the cancer. Treating a cancer may comprise preventing metastasis of the cancer. Preferably, treating a cancer may comprise reducing the size of a tumour associated with the cancer. As such, the treatment may cause tumour regression in the cancer. Treating a cancer may comprise reducing the number of tumours or lesions present in the patient. When the treatment reduces the size of a tumour associated with the cancer, the size of the tumour is typically reduced from base line by at least 10%. Base line is the size of the tumour at the date treatment with the compound is first started. The size of the tumour is typically as measured in accordance with version 1.1 of the RECIST criteria (for instance as described in Eisenhauer et al, European Journal of Cancer 45 (2009) 228-247).

The response to the treatment with the compound may be complete response, partial response or stable disease, in accordance with version 1.1 of the RECIST criteria. Preferably, the response is partial response or complete response. The treatment may achieve progression free survival for at least 60 days, at least 120 days or at least 180 days.

The reduction in tumour size may be greater 20%, greater than 30% or greater than 50% reduction relative to base line. The reduction in tumour size may be observed after 30 days of treatment or after 60 days of treatment.

In haematological cancers, treating a cancer preferentially may lead to complete remission (CR) of the cancer or it may lead to complete remission with incomplete haematological recovery (CRh) or complete remission but with incomplete platelet recovery (CRp). Additionally, treatment may lead to an increase in duration of remission (DoR), an increase in minimal residual disease (MRD), or an increase in relapse free survival (RFS) (Blood 2007 109 1815 and Leukemia Res 2018, 68, 32).

As explained here, the compounds of the invention are useful in treating or preventing various disorders. The present invention therefore provides a compound of the invention for use in medicine. The invention also provides the use of a compound of the invention in the manufacture of a medicament. The invention also provides compositions and products comprising the compounds of the invention. Such compositions and products are also useful in treating or preventing disorders. The present invention therefore provides a composition or product as defined herein for use in

medicine. The invention also provides the use of a composition or product of the invention in the manufacture of a medicament. Also provided is a method of treating a subject in need of such treatment, said method comprising administering to the subject a compound of the invention. In some embodiments the subject suffers from or is at risk of suffering from one of the disorders disclosed herein.

In one aspect, the subject is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

A subject is typically a human patient. The patient may be male or female. The age of the patient is typically at least 18 years, for instance from 30 to 70 years or from 40 to 60 years. Alternatively, the patient may be a child, for instance the patient may be under 18 years of age, or under 12 years of age. In one embodiment, the patient is a child under ten years of age or an infant under two years of age. In one embodiment the invention provides a compound as defined herein for use in treating cancer in paediatric patients.

A compound or composition of the invention can be administered to the subject in order to treat one or more symptoms of the disorder. In this embodiment, the subject is typically symptomatic. A therapeutically effective amount of the agent or formulation is administered to such a subject. A therapeutically effective amount is an amount effective to ameliorate one or more symptoms of the disorder.

The compound or composition of the invention may be administered in a variety of dosage forms. Thus, it can be administered orally, for example as tablets, troches, lozenges, aqueous or oily suspensions, dispersible powders or granules. The compound or composition of the invention may also be administered parenterally, whether subcutaneously, intravenously, intramuscularly, intrasternally, transdermally or by infusion techniques. The compound or composition may also be administered as a suppository. Preferably, the compound, composition or combination may be administered orally.

The compound or composition of the invention is typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

A therapeutically effective amount of the compound or composition of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific compound, the age, weight and conditions of the subject to be treated, the type and severity of the disease and the frequency and route of administration. Preferably, daily dosage levels are from 5 mg to 2 g.

When the compound or composition of the invention is administered to a subject in combination with another active agent, the dose of the other active agent can be determined as described above. The dose may be determined according to various parameters, especially according to the agent used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific agent, the age, weight and conditions of the subject to be treated, the type and severity of the disease and the frequency and route of administration. Preferably, daily dosage levels are from 5 mg to 2 g.

*Synthesis*

Compounds of the invention can be prepared by the synthetic methods described in the Examples that follow, or by analogy with such methods using appropriate starting materials and methodologies familiar to the skilled chemist.

The following examples illustrate the invention. They do not however limit the invention in any way. In this regard, it is important to understand that the particular assay used in the Examples section is designed only to provide an indication of biological activity. There are many assays available to determine biological activity, and a negative result in any one particular assay is therefore not determinative. The invention is defined according to the claims.

## Examples

In the examples which follow below, compounds may be produced in racemic form. Compounds in racemic form are indicated by use of the term rac- in advance of the IUPAC name. In some instances, as indicated, enantiomers and/or diastereomers are separated. In general, the enantiomers and/or diastereomers of the invention can be separated using chiral chromatography. The separated enantiomers and/or diastereomers may be identified in the order in which they elute, for example the first, second, third or fourth eluting isomer respectively, as obtained by separation using chiral chromatography. The isomers may alternatively or additionally be identified using IUPAC naming conventions. Thus, where enantiomers and/or diastereomers have been assigned an absolute stereochemistry, this is indicated as R or S stereochemistry, as appropriate, and by use of a wedge-shaped bond (e.g. 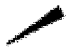 )in the chemical formula.

Where enantiomers have been separated but have undefined absolute stereochemistry the term "rel-R" or "rel-S" has been used. In these examples it is understood that the absolute configuration is unknown and the "rel-R" and "rel-S" labels are used merely to distinguish the two enantiomers form one another. The absolute stereochemistry of an enantiomer labelled as "rel-R" may be either R or S. In either case, the corresponding "rel-S" enantiomer has the opposite configuration to the "rel-R" enantiomer. In these examples the use of a rectangular bond (e.g. 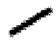 ) has been used to define the "rel" configuration.

Alternatively, where diastereomers have been separated and the absolute configuration is unknown they may be assigned a relative stereochemistry, which denotes the stereochemistry of a first chiral centre with respect to a second chiral centre. Where a relative stereochemistry has been assigned, this is indicated by the term "rel" in the IUPAC name, and by use of a rectangular-shaped bond (e.g. 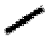 ). Where diastereomers have been separated and the relative and absolute configuration is unknown, the compounds have been labelled by the order in which they elute, for example the first, second, third or fourth eluting isomer respectively, as obtained by separation using chiral chromatography.

The moieties U of formulae (U1), (U4) when Q' is CH and (U5) typically contain at least one chiral centre at the carbon atoms marked with an asterisk as depicted below:

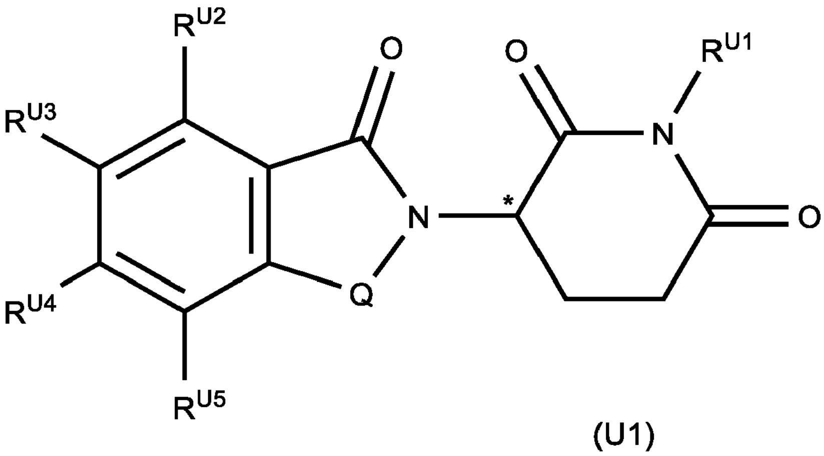

(U1) ;

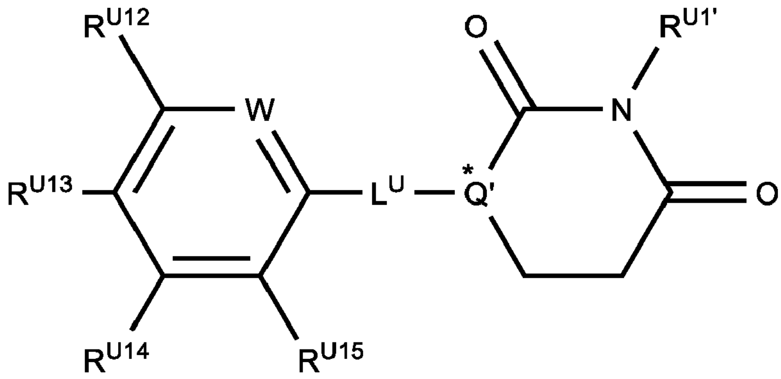

(U4) ;

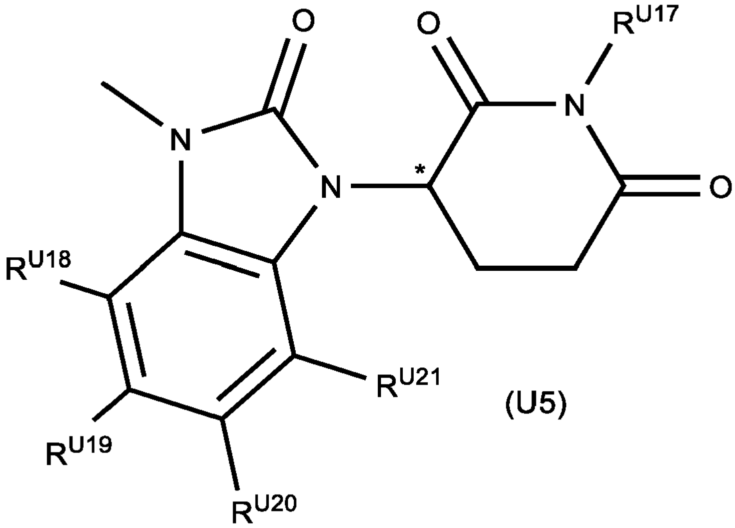

(U5) .

Specifically in relation to the examples that follow, it is understood that the moiety U is typically in the form of a 1:1 mixture of the R- and S-enantiomers at said carbon atom, indicated specifically in the examples that follow by the use of a simple line-shaped bond (e.g. 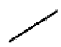 ).

Temperatures are given in degrees Celsius (°C). The reactants used in the examples below may be obtained from commercial sources or they may be prepared from commercially available starting materials as described herein or by methods known in the art. All the compounds of the invention are synthesized according to the Examples described herein. The progress of the reactions described herein were followed as appropriate by e.g. LC, GC or TLC, and as the skilled person will readily realize, reaction times and temperatures may be adjusted accordingly.

**NMR spectroscopy**:

Solids/oils were solubilized in DMSO-$d_6$ or MeOH-$d_4$, vortexed vigorously until the solution was clear and transferred to an NMR tube for data acquisition.

Liquid-state NMR experiments were recorded using:

- 600 MHz (14.1 Tesla) Bruker Avance III NMR spectrometer (600 MHz for $^1$H, 151 MHz for $^{13}$C) using a triple-resonance $^1$H,$^{15}$N,$^{13}$C CP-TCI 5 mm cryoprobe (Bruker Biospin, Germany)
- 500 MHz (11.75 Tesla) Bruker Avance I NMR spectrometer (500 MHz for $^1$H, 125 MHz for $^{13}$C) using a Dual Resonance BBI 5 mm probe (Bruker Biospin, Germany)
- 400 MHz (9.4 Tesla) Bruker Avance NEO NMR spectrometer (400 MHz for $^1$H, 100 MHz for $^{13}$C) using a SEI 5 mm probe (Bruker Biospin, Germany)
- 400 MHz (9.4 Tesla) Bruker Avance NEO NMR spectrometer using a PI HR-BBO400S1-BBF/H/D-5.0-Z SP probe (Bruker Biospin, Germany)
- 300 MHz Bruker AVANCE III HD NMR spectrometer using a PA BBO 300S1 BBF-H-D-05 Z probe (Bruker Biospin,

Germany)

- 300 MHz Bruker Avance NEO NMR spectrometer using a PA BBO BBF-H-D-05 Z (Bruker Biospin, Germany)

All the experiments used for the resonance assignment procedure and the elucidation of the products structure (1D $^1H$, 2D $^1H$-$^1H$-COSY, 2D $^1H$-$^1H$-ROESY, 2D $^1H$-$^{13}C$-HSQC, 2D $^1H$-$^{13}C$-HMBC) were recorded at 300 K. $^1H$ chemical shifts are reported in $\delta$ ppm as s (singlet), d (doublet), t (triplet), q (quartet), dd (double doublet), m (multiplet) or br s (broad singlet).

**UPLC-MS chromatography:**

UPLC-MS chromatography analysis were recorded using the following apparatus using:

- Waters UPLC: Acquity, UV: Acquity PDA, MS: Qda, ELSD
- Waters UPLC: Acquity, UV: Acquity TUV, MS: Qda
- Waters UPLC: Acquity, UV: Acquity PDA, MS: Qda

The apparatus was tested using a CSH C18 Waters column (50 $\times$ 2.1 mm), 1.7 $\mu$m. It used a combination of the following eluents: $H_2O$ + 0.05% TFA (v/v) (solvent A) and

MeCN + 0.035% TFA (v/v) (solvent B) and a positive electrospray ES+ as ionization mode. The UV detection was set at 220 and 254 nm. The methods used were the following:

- Polar method (1.7 min run): gradient t=0 2% B, t=0.5 min 2% B, t=1.5 min 98% B, t=1.52 min 2% B, t=1.7 min 2% B
- Polar method (3.5 min run): gradient t=0 2% B, t=1.0 min 2% B, t=2.4 min 98% B, t=3.00 min 98% B, t=3.03 min 2% B, t=3.5 min 2% B
- Normal method (1.7 min run): gradient t=0 2% B, t=1.0 min 98% B, t=1.5 min 98% B, t=1.52 min 2% B, t=1.7 min 2% B
- Normal method (3.5 min run): gradient t=0 2% B, t=2.4 min 98% B, t=3.0 min 98% B, t=3.03 min 2% B, t=3.5 min 2% B

Where it is observed mass spectra analysis is reported as $[M+H]^+$ for the molecular ion; Someone skilled in the art will also appreciate that isotope patterns may be reported where evident, for example $[M+H+2]^+$ represents the isotopic peak of Br if it is present in the molecule; Additionally, some fragmentation ions may be included in the analysis, for example [M+H-*t*Bu]$^+$, $[M+H-Boc]^+$ and $[M-Cl+MeOH]^+$.

**Preparative HPLC**

Where Example purification has been performed by preparative HPLC the conditions are included in the reaction Step where the purification was performed or one of the general Methods below was used:

**Method A**:

Gilson system used for preparative HPLC purification purposes. The system is equipped with a 333 pump A and a 334 pump B, Gilson Verity 1741 detector and GX-271 liquid handler fraction collector. The reverse phase purification was carried out by using a preparative column X-BRIDGE C18 (250 $\times$ 30 mm), 5 $\mu$M. Gradient elution was done using 10 mmol ammonium bicarbonate in water (as Phase A) and 100% Acetonitrile (as Phase B) with a gradient Program (B%): 10% B at 0 min hold until 3 min, 35% B at 10.0 min, 65% B at 35 min, 99% B at 35.1 min hold till 40.0 min, 10% B at 40.1 min hold until 45 min. Flow rate: 25 mL/min.

**Method B:**

Gilson system used for preparative HPLC purification purposes. The system is equipped with a 333 pump A and a 334 pump B, Gilson Verity 1741 detector and GX-271 liquid handler Fraction collector. The reverse phase purification is carried out by using preparative column YMC-ACTUS C18 (250*20mm) 5 $\mu$M. Gradient elution is done with 0.1% Formic acid in water (as Phase A) and 100% Acetonitrile (Phase B) with a gradient Program (B%): 5% B at 0 min hold till 5min,15% B at 200.0 min, 35% B at 35 min, 99% B at 35.1min hold till 40.0 min, 5% B at 40.1min hold till 45 min. Flow rate: 18 mL/min.

**Abbreviations:**

In addition to the definitions above, the following abbreviations are used in the Example experimental procedures below. If an abbreviation used herein is not defined, it has its generally accepted meaning:

| | |
|---|---|
| Ac | Acetyl |
| AcOH | Acetic acid |
| AIBN | 2,2'-Azobis(2-methylpropionitrile) |
| BINAP | 2,2'-Bis(diphenylphosphino)-1,1'-binaphthalene |
| Boc | *tert*-butyloxycarbonyl |
| $Boc_2O$ | Di-*tert*butyl decarbonate |
| BrettPhos | 2-(Dicyclohexylphosphino)3,6-dimethoxy-2',4',6'-triisopropyl-1,1'-biphenyl |
| BrettPhos Pd G3 | [(2-Di-cyclohexylphosphino-3,6-dimethoxy-2',4',6'- triisopropyl-1,1'-biphenyl)-2-(2'-amino-1,1'-biphenyl)]palladium(II) methanesulfonate methanesulfonate |
| $CBr_4$ | tetrabromomethane |
| $Cs_2CO_3$ | Cesium carbonate |
| CuI | Copper (I) iodide |
| CyJohnPhos | 2-(Dicyclohexylphosphino)biphenyl |
| DBU | 1,8-Diazabicyclo[5.4.0]undec-7-ene |
| DCE | 1,2-dichloroethane |
| DCM | Dichloromethane |
| DHP | 3,4-Dihydro-2H-pyran |
| DIAD | Diisopropyl azodicarboxylate |
| DIPEA | *N*,*N*-Diisopropylethylamine |
| Dioxane | 1,4-dioxane |
| DMAP | 4-Dimethylaminopyridine |
| DMEDA | *N,N'*-dimethylethylenediamine |
| DMF | Dimethylformamide |
| DMP | 1,1,1-Tris(acetyloxy)-1,1-dihydro-1,2-benziodoxol-3-(1H)-one (Dess-Martin Periodinane) |
| DMSO | Dimethylsulfoxide |
| DMSO-$d_6$ | Hexadeuterodimethylsulfoxide |
| ee | Enantiomeric excess |
| Et | Ethyl |
| EtOAc | Ethyl acetate |
| $Et_3N$ | Triethylamine |
| EtOH | Ethanol |
| $Et_2O$ | Diethylether |
| EPhos Pd G4 | Methanesulfonato{Dicyclohexyl[3-(1-methylethoxy)-2',4',6'-tris(1-methylethyl)[1,1'-biphenyl]-2-yl]phosphine}(2'-methylamino-1,1'-biphenyl-2-yl)palladium(II) |
| $FeCl_3$ | Iron(III) chloride |
| GC | Gas chromatography |
| GPhos | (3-(tert-Butoxy)-2',6'-diisopropyl-6-methoxy-[1,1'-biphenyl]-2-yl)dicyclohexylphosphane |
| Gphos Pd $G_6$ TES | GPhos OAC precatalyst TES |
| h | hour |
| $H_2O$ | water |
| HATU | 1-[Bis(dimethylamino)methylene]-1H-1,2,3-triazolo[4,5-b]pyridinium 3-oxid hexafluorophosphate |
| HCl | Hydrochloric acid |
| Het | Heteroaromatic |
| HPLC | High performance liquid chromatography |
| $K_2CO_3$ | Potassium carbonate |
| KI | Potassium iodide |
| LC | Liquid chromatography |
| LDA | Lithium diisopropylamide |
| $LiBH_4$ | Lithium borohydride |
| LiHMDS | Lithium bis(trimethylsilyl)amide |
| LiOH | Lithium hydroxide |
| min | Minutes |
| Me | Methyl |
| MeCN | Acetonitrile |
| MeOH | Methanol |
| MeTHF | 2-Methyltetrahydrofuran |
| $MgSO_4$ | Magnesium sulfate |

| | |
|---|---|
| MS | Mass spectrometry |
| MsCl | Methanesulfonyl chloride |
| MsOH | Methanesulfonic acid |
| MTBE | Methyl tert-butyl ether |
| MW | Microwave |
| $N_2$ | Nitrogen |
| NaH | Sodium hydride |
| NaOCN | Sodium cyanate |
| NaOH | Sodium hydroxide |
| $Na_2SO_4$ | Sodium sulfate |
| $NaBH_3CN$ | Sodium cyanoborohydride |
| NBS | N-bromosuccinimide |
| NCS | N-chlorosuccinimide |
| $NH_4Cl$ | Ammonium chloride |
| NIS | N-iodosuccinimide |
| NMP | N-methyl-2-pyrrolidone |
| Pd/C | Palladium on carbon |
| Pd(dppf)$Cl_2$ | [1,1'-Bis(diphenylphosphino)ferrocene]dichloropalladium(II) |
| Pd-PEPPSI-IHept-Cl | Palladium) 1,3-bis[2,6-bis(heptan-4-yl)phenyl]-4,5-dichloro-1,2-didehydro-1$\lambda^5$-imidazole 3-chloropyridine dichloride |
| $Pd(PPh_3)_2Cl_2$ | Bis(triphenylphosphine)palladium(II) dichloride |
| PE | Petroleum ether |
| $PPh_3$ | Triphenylphosphine |
| PMB-Cl | 4-methoxybenzyl chloride |
| rt | room temperature (18 to 22 °C) |
| Selectfluor | 1-(Chloromethyl)-4-fluoro-1,4-diazabicyclo[2.2.2]octane-1,4-diium ditetrafluoroborate |
| SEM-Cl | 2-(Trimethylsilyl)ethoxymethyl chloride |
| TBS-Cl | tert-Butyldimethylsilyl chloride |
| tBuOK | Potassium tert-butoxide |
| TFA | Trifluoroacetic acid |
| TfOH | Trifluoromethanesulfonic acid |
| TLC | Thin layer chromatography |
| THF | Tetrahydrofuran |
| TsCl | p-Toluenesulfonyl chloride |
| TsOH | p-Toluenesulfonic acid monohydrate |
| UPLC | Ultra Performance Liquid Chromatography |
| Xantphos | 4,5-Bis(diphenylphosphino)-9,9-dimethylxanthene |

**Example 1 was synthesised following Scheme 1**

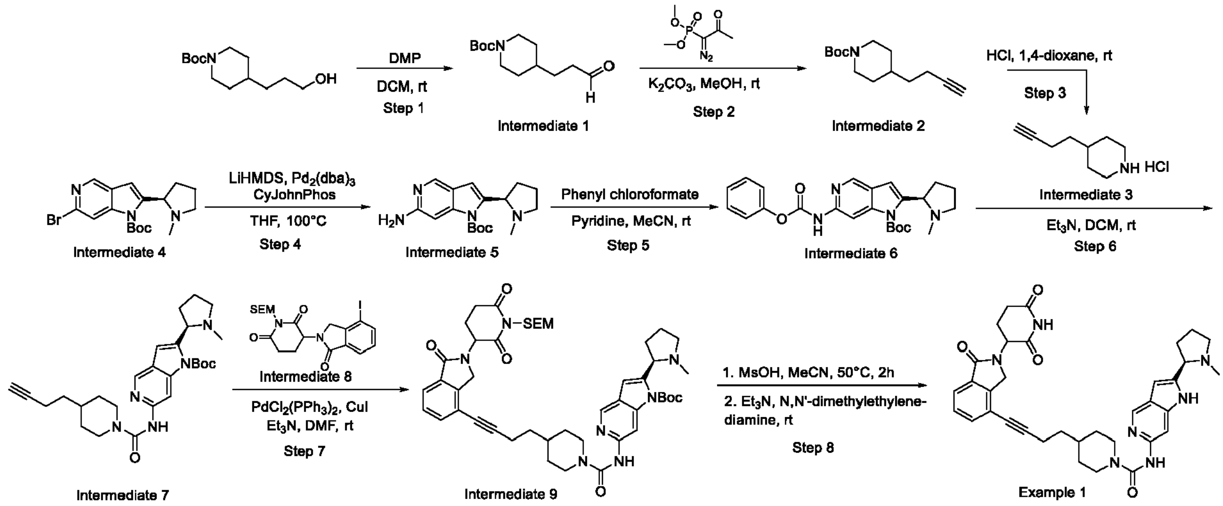


## Scheme 1

**Step 1**

**Intermediate 1: *tert*-butyl 4-(3-oxopropyl)piperidine-1-carboxylate**

To a solution of *tert*-butyl 4-(3-hydroxypropyl)piperidine-1-carboxylate (CAS No: 156185-63-6, 5.00 g, 20.55 mmol) in dichloromethane (100 mL) at 0°C was added Dess-Martin periodinane (13.48 g, 30.83 mmol) and the reaction stirred at room temperature for 6h. The reaction mixture was diluted with a mixture of sodium bicarbonate (200 mL) and sodium thiosulphate (200 mL) and extracted with dichloromethane (3 × 150 mL). The combined organic phases were dried over anhydrous $Na_2SO_4$ and concentrated *in vacuo.* The crude material was purified by combi-flash column chromatography by eluting with 20% ethyl acetate in heptane to afford *tert*-butyl 4-(3-oxopropyl)piperidine-1-carboxylate **(Intermediate 1**, 3.60 g, 73%) as a yellow liquid. $^1$H NMR (400 MHz, DMSO-$d_6$) δ ppm 0.88-1.00 (m, 2H), 1.38 (s, 9H), 1.41-1.50 (m, 3H), 1.58-1.64 (m, 2H), 2.42-2.47 (m, 2H), 2.60-2.70 (m, 2H), 3.88-3.94 (m, 2H), 9.67 (s, 1H). Mass spec: m/z: Mass spec: m/z: 240.0 $[M-H]^-$.

**Step 2**

**Intermediate 2: tert-butyl 4-(but-3-yn-1-yl) piperidine-1-carboxylate**

To a solution of *tert*-butyl 4-(3-oxopropyl) piperidine-1-carboxylate **(Intermediate 1,** 3.60 g, 15 mmol) in methanol (50 mL) was added potassium carbonate (4.10 g, 30 mmol) followed by dimethyl (1-diazo-2-oxopropyl)phosphonate (3.20 g, 16 mmol) and the reaction mixture was stirred at room temperature for 3h. The reaction mixture was diluted with water (200 mL) and extracted with ethyl acetate (3 × 200 mL). The combined organic phases were dried over anhydrous $Na_2SO_4$, filtered, and concentrated *in vacuo.* The crude material was purified by combi-flash column chromatography by eluting with 20% ethyl acetate in heptane to afford tert-butyl 4-(but-3-yn-1-yl)piperidine-1-carboxylate (**Intermediate 2**, 2.70 g, 69%d) as a yellow liquid. $^1$H NMR (400 MHz, DMSO-$d_6$) δ ppm 0.89-1.00 (m, 2H), 1.38 (s, 9H), 1.41-1.55 (m, 3H), 1.58-1.64 (m, 2H), 2.11-2.23 (m, 2H), 2.58-2.71 (m, 2H), 2.73(s, 1H), 3.86-3.94 (m, 2H).

**Step 3**

**Intermediate 3: 4-(but-3-yn-1-yl)piperidine hydrochloride**

To a solution of *tert*-butyl 4-(but-3-yn-1-yl) piperidine-1-carboxylate **(Intermediate 2,** 2.70 g, 11 mmol) in 1, 4-dioxane (30 mL) was added 4M hydrochloric acid in 1,4-dioxane (30 mL) at 0°C and the reaction mixture was stirred at room temperature for 4h. The reaction mixture was concentrated *in vacuo* to afford 4-(but-3-yn-1-yl)piperidine hydrochloride **(Intermediate 3**, 1.60 g) as a white solid, which was used for the next step without further purification. $^1$H NMR (400 MHz, DMSO-$d_6$) δ ppm 1.21-1.35 (m, 2H), 1.38-1.44 (m, 2H), 1.58-1.64 (m, 1H), 1.76-1.82 (m, 2H), 2.12-2.26 (m, 2H), 2.73-2.88 (m, 3H), 3.17-3.27 (m, 2H), 8.72 (br s, 2H).

**Step 4**

**Intermediate 5: *tert-butyl* (*R*)-6-amino-2-(1-methylpyrrolidin-2-yl)-1*H*-pyrrolo[3,2-*c*]pyridine-1-carboxylate**

To a solution of *tert*-butyl (*R*)-6-bromo-2-(1-methylpyrrolidin-2-yl)-1*H*-pyrrolo[3,2-c]pyridine-1-carboxylate **(Intermediate 4**, 1.00 g, 2.63 mmol) in tetrahydrofuran (20 mL) was added 2-(dicyclohexylphosphino)biphenyl (CyJohnPhos, 0.19 g, 0.52 mmol) followed by $Pd_2(dba)_3$ (0.25 g, 0.26 mmol) at room temperature and the reaction mixture was purged with argon for 15 min. LiHMDS (1M in THF, 7.89 mL, 7.89 mmol) was then added and the reaction mixture was further purged with argon for 10 min and then stirred at 100°C for 2h. The reaction mixture was quenched with saturated aqueous $NH_4Cl$ solution (50 mL) and extracted with ethyl acetate (2 × 50 mL). The combined organic phases were dried over anhydrous $Na_2SO_4$, filtered, and concentrated *in vacuo.* The crude material was purified by combi-flash chromatography by eluting with 90% ethyl acetate in heptane to afford *tert*-butyl (*R*)-6-amino-2-(1-methylpyrrolidin-2-yl)-1*H*-pyrrolo [3, 2-c]pyridine-1-carboxylate **(Intermediate 5**, 0.80 g, 96%) as a yellow solid. $^1$H NMR (400 MHz, DMSO-$d_6$) δ ppm 1.52-1.58 (m, 1H), 1.63 (s, 9H), 1.68-1.75 (m, 2H), 2.25-2.28 (m, 1H), 2.31 (s, 3H), 2.34-2.36 (m, 1H), 3.06-3.13 (m, 1H), 3.76-3.82 (m, 1H), 5.69 (br s, 2H), 6.49 (s, 1H), 7.01 (s, 1H), 8.12 (s, 1H). Mass spec: m/z: 317 $[M+H]^+$.

**Step 5**

**Intermediate 6: *tert*-butyl (*R*)-2-(1-methylpyrrolidin-2-yl)-6-((phenoxycarbonyl) amino)-1*H*-pyrrolo[3, 2-*c*]pyridine-1-carboxylate**

To a solution of *tert*-butyl (*R*)-6-amino-2-(1-methylpyrrolidin-2-yl)-1*H*-pyrrolo[3, 2-*c*]pyridine-1-carboxylate (**Intermediate 5**, 1.00 g, 3.161 mmol) in acetonitrile (15 mL) was added pyridine (0.52 mL, 6.32 mmol) at room temperature and stirred for 15 min. Phenyl chloroformate (0.54 g, 3.48 mmol) was added and the reaction stirred at room temperature for 16h. The reaction mixture was quenched with ice cold water (50 mL) and extracted with ethyl acetate (2 × 50 mL). The combined organic phases were dried over anhydrous $Na_2SO_4$, filtered and concentrated *in vacuo* to afford *tert*-butyl (*R*)-2-(1-methylpyrrolidin-2-yl)-6-((phenoxycarbonyl)amino)-1*H*-pyrrolo[3, 2-*c*]pyridine-1-carboxylate **(Intermediate 6**, 0.65 g, 47%) as a white solid, which was used for the next step without further purification. $^1$H NMR (400 MHz, DMSO-$d_6$) δ ppm 1.52-1.58 (m, 1H), 1.61 (s, 9H), 1.69-1.79 (m, 2H), 2.25-2.29 (m, 1H), 2.34 (s, 3H), 2.38-2.42 (m, 1H), 3.09-3.14 (m, 1H), 3.86-3.92 (m, 1H), 6.72 (s, 1H), 7.19-7.30 (m, 3H), 7.40-7.47 (m, 2H), 8.48 (s, 1H), 8.53 (s, 1H), 10.62 (s, 1H). Mass spec: m/z: 437.3 $[M+H]^+$.

**Step 6**

**Intermediate 7: *tert*-butyl (*R*)-6-(4-(but-3-yn-1-yl)piperidine-1-carboxamido)-2-(1-methylpyrrolidin-2-yl)-1*H*-pyrrolo[3, 2-*c*]pyridine-1-carboxylate**

To a solution of 4-(but-3-yn-1-yl)piperidine hydrochloride (**Intermediate 3**, 0.068 g, 0.39 mmol) in dichloromethane (2.0 mL) was added *tert*-butyl (*R*)-2-(1-methylpyrrolidin-2-yl)-6-((phenoxycarbonyl)amino)-1*H*-pyrrolo[3,2-*c*] pyridine-1-carboxylate (**Intermediate 6**, 0.17 g, 0.38 mmol) followed by triethylamine (0.28 mL, 2.0 mmol) and the reaction mixture was stirred at room temperature for 16h. The reaction mixture was diluted with water (50 mL) and extracted with ethyl acetate (3 × 50 mL). The combined organic phases were dried over anhydrous $Na_2SO_4$, filtered, and concentrated *in vacuo.* The crude material was purified by combi-flash column chromatography by eluting with 80% ethyl acetate in heptane to afford tert-butyl (R)-6-(4-(but-3-yn-1-yl) piperidine-1-carboxamido)-2-(1-methylpyrrolidin-2-yl)-1*H*-pyrrolo[3, 2-c]pyridine-1-carboxylate **(Intermediate 7**, 0.09g, 48%) as a yellow solid. $^1$H NMR (400 MHz, DMSO-$d_6$) δ ppm 0.98-1.11 (m, 2H), 1.36-1.44 (m, 2H), 1.56-1.60 (m, 2H), 1.65 (s, 9H), 1.68-1.78 (m, 4H), 2.18-2.22 (m, 2H), 2.27-2.31 (m, 1H), 2.33 (s, 3H), 2.36-2.38 (m, 1H), 2.71-2.80 (m, 3H), 3.10-3.16 (m, 1H), 3.84-3.90 (m, 1H), 4.14-4.18 (m, 2H), 6.66 (s, 1H), 8.42 (s, 1H), 8.50 (s, 1H), 8.90 (s, 1H). Mass spec: m/z: 480.1 $[M+H]^+$.

**Step 7**

**Intermediate 9: *tert*-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)piperidine-1-carboxamido)-2-((*R*)-1-methylpyrrolidin-2-yl)-1*H*-pyrrolo[3,2-*c*]pyridine-1-carboxylate**

To a solution of *tert*-butyl (*R*)-6-(4-(but-3-yn-1-yl) piperidine-1-carboxamido)-2-(1-methylpyrrolidin-2-yl)-1*H*-pyrrolo[3, 2-*c*]pyridine-1-carboxylate (**Intermediate 7**, 0.33 g, 0.69 mmol) in 1, 4-dioxane (7.0 mL) was added 3-(4-iodo-1-oxoisoindolin-2-yl)-1-((2-(trimethylsilyl)ethoxy)methyl)piperidine-2,6-dione **(Intermediate 8**, 0.34 g, 0.68 mmol)

followed by triethylamine (3.50 mL, 25.0 mmol). The reaction mixture was purged with argon for 15 min then $PdCl_2(PPh_3)_2$ (0.05 g, 0.070 mmol) and copper(I) iodide (0.014 g, 0.072 mmol) were added. The reaction mixture was further purged with argon for 10 min and then stirred at room temperature for 2h. The reaction mixture was diluted with water (100 mL) and extracted with ethyl acetate (2 × 100 mL). The combined organic phases were dried over anhydrous $Na_2SO_4$, filtered, and concentrated *in vacuo.* The crude material was purified by combi-flash column chromatography by eluting with 80% ethyl acetate in heptane to afford tert-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)piperidine-1-carboxamido)-2-((*R*)-1-methylpyrrolidin-2-yl)-1*H*-pyrrolo[3,2-*c*]pyridine-1-carboxylate **(Intermediate 9**, 0.29 g, 49%) as a yellow solid. $^1$H NMR (400 MHz, DMSO-$d_6$) δ ppm -0.07 (s, 9H), 0.76-0.82 (m, 2H), 1.05-1.10 (m, 3H), 1.46-1.56 (m, 3H), 1.62 (s, 9H), 1.63-1.65 (m, 1H), 1.67-1.78 (m, 4H), 2.00-2.09 (m, 1H), 2.26-2.28 (m, 2H), 2.31 (s, 3H), 2.36-2.38 (m, 1H), 2.69-2.83 (m, 3H), 2.97-3.12 (m, 2H), 3.33-3.40 (m, 1H), 3.46-3.52 (m, 2H), 3.82-3.86 (m, 1H), 4.14-4.18 (m, 2H), 4.23-4.27 (m, 1H), 4.43-4.48 (m, 1H), 4.95-5.08 (m, 2H), 5.23-5.28 (m, 1H), 6.63 (s, 1H), 7.48-7.52 (m, 1H), 7.63 (d, *J*=7.40 Hz, 1H), 7.69 (d, *J*=7.40 Hz, 1H), 8.39 (s, 1H), 8.48 (s, 1H), 8.88 (br s, 1H). Mass spec: m/z: 852.0 [M+H]$^+$.

**Step 8**

**Example 1: 4-(4-(2-(2,6-dioxopiperidin-3-yl)-1-oxoisoindolin-4-yl)but-3-yn-1-yl)-*N*-(2-((*R*)-1-methylpyrrolidin-2-yl)-1*H*-pyrrolo[3, 2-*c*]pyridin-6-yl)piperidine-1-carboxamide**

To a solution of *tert*-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)piperidine-1-carboxamido)-2-((*R*)-1-methylpyrrolidin-2-yl)-1*H*-pyrrolo[3,2-c]pyridine-1-carboxylate **(Intermediate 9**, 0.60 g, 0.70 mmol) in acetonitrile (10 mL) was added methanesulfonic acid (0.47 mL, 7.1 mmol) at room temperature and the reaction mixture was stirred at 50°C for 2h. *N,N'*-dimethylethylenediamine (0.43 mL, 3.6 mmol) and triethylamine (1.98 mL, 14.1 mmol) were added and the reaction stirred at room temperature for 3h. The reaction mixture was concentrated *in vacuo* to obtain crude compound. Water (50 mL) was added resulting in a precipitate which was collected by filtration and dried *in vacuo.* The crude material was purified by preparative HPLC (Method A) to afford 4-(4-(2-(2, 6-dioxopiperidin-3-yl)-1-oxoisoindolin-4-yl) but-3-yn-1-yl)-*N*-(2-((*R*)-1-methylpyrrolidin-2-yl)-1*H*-pyrrolo [3, 2-c] pyridin-6-yl) piperidine-1-carboxamide (**Example 1**, 0.15 g, 35%) as an off white solid. $^1$H NMR (400 MHz, DMSO-$d_6$) δ ppm 1.04-1.17 (m, 2H), 1.53-1.58 (m, 2H), 1.60-1.68 (m, 1H), 1.70-1.78 (m, 2H), 1.80-1.95 (m, 2H), 1.99-2.06 (m, 1H), 2.08-2.13 (m, 1H), 2.14 (s, 3H), 2.21-2.28 (m, 1H), 2.40-2.46 (m, 2H), 2.53-2.64 (m, 3H), 2.72-2.80 (m, 2H), 2.87-2.96 (m, 1H), 3.10-3.16 (m, 1H), 3.24-3.28 (m, 1H), 4.13-4.23 (m, 2H), 4.29-4.36 (m, 1H), 4.44-4.50 (m, 1H), 5.09-5.18 (m, 1H), 6.30 (s, 1H), 7.49-7.55 (m, 1H), 7.63-7.66 (m, 1H), 7.71-7.46 (m, 1H), 7.74 (s, 1H), 8.35 (s, 1H), 8.60 (s, 1H), 11.05 (s, 1H), 11.13 (s, 1H). Mass spec: m/z: 622.1 [M+H]$^+$.

**Intermediate 4 was synthesised following Scheme 2**

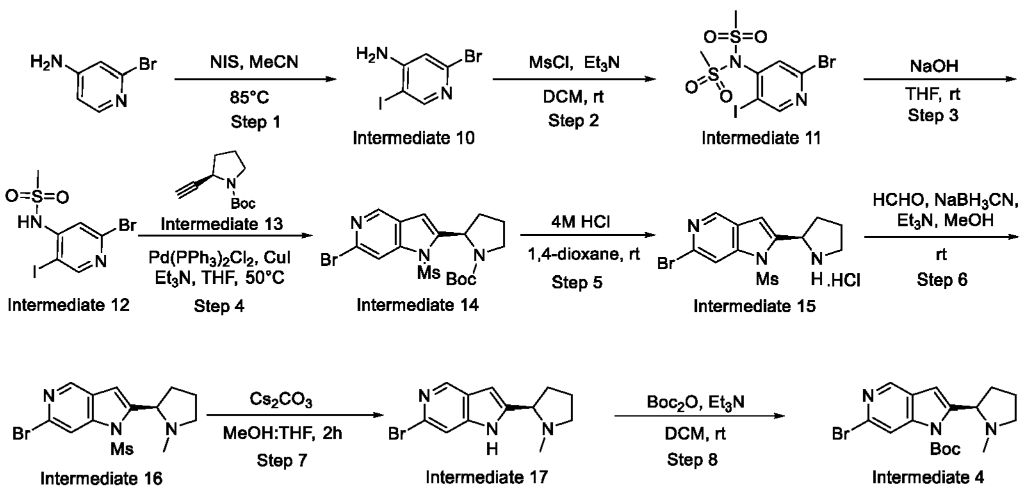


**Scheme 2**

**Step 1**

**Intermediate 10: 2-bromo-5-iodopyridin-4-amine**

To solution of 2-bromopyridin-4-amine (CAS No: 7598-35-8, 200 g, 1156.0 mmol) in acetonitrile (1 L) was added N-iodosuccinimide (297.23 g, 1294.7 mmol) and the reaction mixture was heated at 80°C for 16h. The reaction mixture was then cooled to room temperature and concentrated *in vacuo.* The obtained residue was diluted with aqueous saturated sodium thiosulphate solution (2 L) and extracted with ethyl acetate (3 × 2 L). The combined organic layers were dried over anhydrous $Na_2SO_4$ and concentrated *in vacuo* to obtain the crude compound. The crude material was purified by combi-flash chromatography, by eluting with 5% ethyl acetate in heptane, to afford 2-bromo-5-iodo-pyridin-4-amine **(Intermediate 10**, 115 g, 33%) as a yellow solid. $^1H$ NMR (400 MHz, DMSO-$d_6$) δ ppm 6.48 (br s, 2H), 6.77 (s, 1H), 8.16 (s, 1H). Mass spec: m/z: 300.8 $[M+H]^+$.

**Step 2**

**Intermediate 11: *N*-(2-bromo-5-iodopyridin-4-yl)-*N*-(methylsulfonyl)methanesulfonamide**

To a cooled (0°C) solution of 2-bromo-5-iodo-pyridin-4-amine **(Intermediate 10,** 100 g, 334.55 mmol) in dichloromethane (2.50 g, 29 mmol) was added triethylamine (234 mL, 1673 mmol) followed by methanesulfonyl chloride (106 mL, 1338.2 mmol) in dichloromethane (300 mL). After the addition was complete, the reaction mixture was stirred at room temperature for 3h. The reaction mixture was quenched with aqueous saturated $NaHCO_3$ solution (2 L) and extracted with dichloromethane (2 × 2 L). The combined organic layers were dried over anhydrous $Na_2SO_4$ and concentrated *in vacuo* to afford *N*-(2-bromo-5-iodopyridin-4-yl)-*N*-(methylsulfonyl)methanesulfonamide **(Intermediate 11**, 150 g, 98%) as a yellow liquid, which was used for next step without further purification. $^1H$ NMR (400 MHz, DMSO-$d_6$) δ 3.65 (s, 6H), 8.10 (s, 1H), 8.92 (s, 1H). Mass spec: m/z: 456 $[M+H]^+$.

**Step 3**

**Intermediate 12: *N*-(2-bromo-5-iodopyridin-4-yl)methanesulfonamide**

To a solution of *N*-(2-bromo-5-iodopyridin-4-yl)-*N*-(methylsulfonyl)methanesulfonamide **(Intermediate 11,** 150 g, 329.6 mmol) in tetrahydrofuran (2 L) and water (2 L) was added sodium hydroxide (79.9 g, 1978 mmol) and the reaction mixture was stirred at room temperature for 3h. The reaction mixture was poured into water (500 mL), extracted with ethyl acetate (3 × 1000 mL) and acidified with citric acid solution (1 L) to pH ~ 4. The resultant precipitate was collected by filtration, washed with water (100 mL) and dried *in vacuo* to afford *N*-(2-bromo-5-iodopyridin-4-yl) methanesulfonamide **(Intermediate 12,** 75 g, 60%) as a yellow solid, which was used for next step without further purification. $^1H$ NMR (400 MHz, DMSO-$d_6$) δ 3.28(s, 3H), 7.54 (s, 1H), 8.64 (s, 1H), 9.51 (s, 1H). Mass spec: m/z: 378.7 $[M+H]^+$.

**Step 4**

**Intermediate 14: *tert*-butyl (*R*)-2-(6-bromo-1-(methylsulfonyl)-1*H*-pyrrolo[3,2-c]pyridin-2-yl)pyrrolidine-1-carboxylate**

To a solution of *N*-(2-bromo-5-iodopyridin-4-yl)methanesulfonamide **(Intermediate 12,** 50.0 g, 132.63 mmol) and *tert*-butyl (*R*)-2-ethynylpyrrolidine-1-carboxylate **(Intermediate 13,** 25.89 g, 132.63 mmol) in THF (500 mL) was added *N*,*N*-diisopropylethylamine (138 g, 1061.0 mmol). The reaction mixture was purged with argon gas for 15 min then $PdCl_2(PPh_3)_2$ (9.60 g, 13.263 mmol) and copper(I) iodide (2.55 g, 13.26 mmol) were added. The reaction mixture was further purged with argon gas for 10 min and then heated at 60°C for 3h. The reaction mixture was diluted with water (500 mL) and extracted with ethyl acetate (3 × 300 mL). The combined organic layers were dried over anhydrous $Na_2SO_4$ and concentrated *in vacuo* to obtain crude compound. The crude material was purified by combi-flash chromatography, by eluting with 30% ethyl acetate in heptane, to afford *tert*-butyl (*R*)-2-(6-bromo-1-(methylsulfonyl)-1*H*-pyrrolo[3,2-c]pyridin-2-yl)pyrrolidine-1-carboxylate **(Intermediate 14,** 38.0 g, 62%) as a yellow solid. $^1H$ NMR (400 MHz, DMSO-$d_6$) δ 1.40 ppm (s, 9H), 1.85-1.91 (m, 4H), 3.18-3.21 (m, 1H), 3.37-3.41 (m, 1H), 3.62 (s, 3H), 5.23-5.31 (m, 1H), 6.72 (s, 1H), 7.97 (s, 1H), 8.69 (s, 1H). Mass spec: m/z: 445.9 $[M+H]^+$.

**Step 5**

**Intermediate 15: (*R*)-6-bromo-1-(methylsulfonyl)-2-(pyrrolidin-2-yl)-1*H*-pyrrolo[3,2-c]pyridine hydrochloride**

To a cooled (0°C) solution of *tert*-butyl (*R*)-2-(6-bromo-1-(methylsulfonyl)-1*H*-pyrrolo[3,2-c]pyridin-2-yl)pyrrolidine-1-carboxylate **(Intermediate 14**, 200 g, 450.1 mmol) in 1,4-dioxane (1 L) was added 4M hydrochloric acid in 1,4-dioxane (2 L) and the reaction mixture was stirred at room temperature for 6h. The reaction mixture was concentrated *in vacuo* to afford (*R*)-6-bromo-1-(methylsulfonyl)-2-(pyrrolidin-2-yl)-1*H*-pyrrolo[3,2-c]pyridine hydrochloride **(Intermediate 15**, 160 g) as a yellow solid, which was used for the next step without further purification. $^1$H NMR (400 MHz, DMSO-$d_6$) δ ppm 1.87-2.20 (m, 2H), 2.33-2.46 (m, 2H), 3.15-3.34 (m, 2H), 3.75 (s, 3H), 5.12-5.17 (m, 1H), 7.38 (s, 1H), 7.98 (s, 1H), 8.78 (s, 1H), 9.45 (s, 2H). Mass spec: m/z: 345.8 $[M+H]^+$.

**Step 6**

**Intermediate 16: (*R*)-6-bromo-2-(1-methylpyrrolidin-2-yl)-1-(methylsulfonyl)-1*H*-pyrrolo [3,2-c]pyridine**

To a cooled (0°C) solution of (*R*)-6-bromo-1-(methylsulfonyl)-2-(pyrrolidin-2-yl)-1*H*-pyrrolo[3,2-c]pyridine hydrochloride **(Intermediate 15,** 90.0 g, 236.4 mmol) in methanol (900 mL) was added triethylamine (192 g, 1891 mmol) followed by formaldehyde (60.83 g, 709.2 mmol). The reaction mixture was stirred for 5 min and then sodium cyanoborohydride (34.96 g, 472.8 mmol) was added at 0°C. The reaction mixture was then heated at 60°C for 16h. The reaction mixture was diluted with water (500 mL) and extracted with dichloromethane (3 × 500 mL). The combined organic layers were dried over anhydrous $Na_2SO_4$ and concentrated *in vacuo* to the obtain crude compound. The crude material was purified by combi-flash chromatography by eluting with 60% ethyl acetate in heptane, to afford (*R*)-6-bromo-2-(1-methylpyrrolidin-2-yl)-1-(methylsulfonyl)-1*H*-pyrrolo[3,2-c]pyridine **(Intermediate 16**, 60.0 g, 71%) as a yellow solid. $^1$H NMR (400 MHz, DMSO-$d_6$) δ ppm 1.70-1.78 (m, 3H), 2.33 (s, 3H), 2.36-2.40 (m, 2H), 3.01-3.22 (m, 1H), 3.56 (s, 3H), 3.76-3.81 (m, 1H), 6.89 (s, 1H), 7.97 (s, 1H), 8.68 (s, 1H). Mass spec: m/z: 359.8 $[M+H]^+$.

**Step 7**

**Intermediate 17: (*R*)-6-bromo-2-(1-methylpyrrolidin-2-yl)-1*H*-pyrrolo[3, 2-c]pyridine**

To a solution of (*R*)-6-bromo-2-(1-methylpyrrolidin-2-yl)-1-(methylsulfonyl)-1*H*-pyrrolo[3,2-c]pyridine **(Intermediate 16,** 110 g, 307.1 mmol) in methanol (600 mL) was added tetrahydrofuran (600 mL) and the mixture cooled to 0°C. Cesium carbonate (200 g, 614.2 mmol) was added and the reaction mixture was stirred at room temperature for 4h. The reaction mixture was quenched with water (2 L) and extracted with ethyl acetate (2 × 1 L). The combined organic layers were dried over anhydrous $Na_2SO_4$ and concentrated *in vacuo* to afford (R)-6-bromo-2-(1-methylpyrrolidin-2-yl)-1*H*-pyrrolo[3,2-c]pyridine (**Intermediate 17**, 60.0 g, 70%) as a yellow solid, which was used for the next step without further purification. $^1$H NMR (400 MHz, DMSO-$d_6$) δ ppm 1.75-1.91 (m, 3H), 2.14 (s, 3H), 2.21-2.29 (m, 1H), 3.10-3.15 (m, 1H), 3.33-3.36 (m, 2H), 6.45 (s, 1H), 7.43 (s, 1H), 8.48 (s, 1H), 11.57 (br s, 1H). Mass spec: m/z: 279.9 $[M+H]^+$.

**Step 8:**

**Intermediate 4: *tert*-butyl (*R*)-6-bromo-2-(1-methylpyrrolidin-2-yl)-1*H*-pyrrolo[3,2-c]pyridine-1-carboxylate**

To a solution of (*R*)-6-bromo-2-(1-methylpyrrolidin-2-yl)-1*H*-pyrrolo[3, 2-c]pyridine **(Intermediate 17,** 60.0 g, 214.16 mmol) in dichloromethane (600 mL) was added triethylamine (65.3 g, 642.49 mmol) followed by di-tert-butyldicarbonate (94.42 g, 428.33 mmol) and 4-(dimethylamino)pyridine (5.28 g, 42.833 mmol) and the reaction mixture was stirred at room temperature for 3h. The reaction mixture was diluted with water (500 mL) and extracted with diethyl ether (3 × 500 mL). The combined organic layers were dried over anhydrous $Na_2SO_4$ and concentrated *in vacuo* to obtain the crude compound. The crude material was purified by combi-flash chromatography, by eluting with 45% ethyl acetate in heptane, followed by SFC purification (Waters SFC Prep 150 Mgm equipped with 2489 PDA detector; Column: Chiralpak IC (30 × 250 mm, 5 μM); Solvent A) $CO_2$ 70 g/min; B) 0.1% Isopropyl amine in isopropyl alcohol and MeCN (50:50) 25 mL/min) to afford *tert*-butyl-(*R*)-6-bromo-2-(1-methylpyrrolidin-2-yl)-1*H*-pyrrolo[3,2-c]pyridine-1-carboxylate **(Intermediate 4**, 43 g, 53%) as an off white solid. Enantiomeric excess (ee) 98.4%. $^1$H NMR (400 MHz, DMSO-$d_6$) δ ppm 1.65 (s, 9H), 1.71-1.75 (m, 3H), 2.30-2.33 (m, 1H), 2.34 (s, 3H), 2.37-2.40 (m, 1H), 3.11-3.14 (m, 1H), 3.87-3.91 (m, 1H), 6.80 (s, 1H), 8.05 (s, 1H), 8.61 (s, 1H). Mass spec: m/z: 379.9 $[M+H]^+$.

**Intermediate 4 can also be synthesised following Scheme 3**

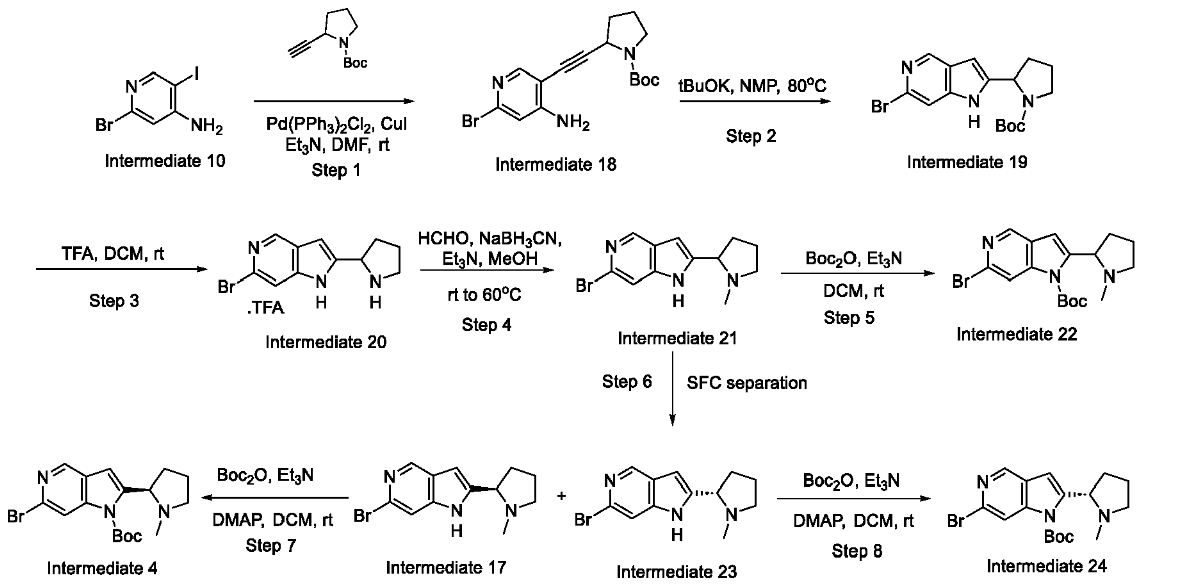


## Scheme 3

### Step 1

**Intermediate 18: rac-tert-butyl 2-[2-(4-amino-6-bromopyridin-3-yl)ethynyl] pyrrolidine- 1-carboxylate**

A solution of 2-bromo-5-iodopyridin-4-amine (**Intermediate 10,** 17 g, 54.2 mmol), rac-tert-butyl 2-ethynylpyrrolidine-1-carboxylate (CAS: 316141-37-4, 13.4 g, 65.04 mmol), $Pd(PPh_3)_2Cl_2$ (3.81 g, 5.42 mmol), CuI (1.03 g, 5.42 mmol) and $Et_3N$ (16.4 g, 162.6 mmol) in DMF (400 mL) was stirred for 2h at rt under nitrogen atmosphere. The reaction mixture was quenched with water and the resulting solution was extracted three times with EtOAc. The organic layers were combined, washed with brine, dried over anhydrous $Na_2SO_4$, filtered, and concentrated under reduced pressure. The crude material was purified by flash chromatography on silica gel using petroleum ether/EtOAc (7/3) as eluent to afford rac-tert-butyl 2-[2-(4-amino-6-bromopyridin-3-yl)ethynyl]pyrrolidine-1-carboxylate (**Intermediate 18,** 18 g, 83%) as a yellow oil. Mass spec: m/z 366 $[M+H]^+$.

### Step 2

**Intermediate 19: rac-tert-butyl 2-{6-bromo-1H-pyrrolo[3,2-c]pyridin-2-yl}pyrrolidine-1-carboxylate**

To a solution of rac-tert-butyl 2-[2-(4-amino-6-bromopyridin-3-yl)ethynyl]pyrrolidine-1-carboxylate (**Intermediate 18,** 8 g, 20.8 mmol) in NMP (40 mL) was added a solution of tBuOK (65.53 mL, 65.53 mmol, 1M in THF). The reaction mixture was stirred for 4h at 80°C. The reaction mixture was quenched with water and the resulting solution was extracted three times with EtOAc. The organic layers were combined, washed with brine, dried over anhydrous $Na_2SO_4$, filtered, and concentrated under reduced pressure. The crude material was purified by flash chromatography on silica gel using DCM/MeOH (95/5) as eluent to afford rac-tert-butyl 2-{6-bromo-1H-pyrrolo[3,2-c]pyridin-2-yl}pyrrolidine-1-carboxylate (**Intermediate 19,** 6 g, 71%) as a yellow oil. Mass spec: m/z 366 $[M+H]^+$.

### Step 3

**Intermediate 20: rac-2-{6-bromo-1H-pyrrolo[3,2-c]pyridin-2-yl}pyrrolidine trifluoroacetate**

To a solution of rac-tert-butyl 2-{6-bromo-1H-pyrrolo[3,2-c]pyridin-2-yl}pyrrolidine-1-carboxylate (**Intermediate 19,** 16 g, 41.5 mmol) in DCM (300 mL) was added TFA (30 mL). The reaction mixture was stirred for 2h at rt. The solution was concentrated under reduced pressure to afford rac-2-{6-bromo-1H-pyrrolo[3,2-c]pyridin-2-yl}pyrrolidine trifluoroacetate (**Intermediate 20,** 10 g, 77%) as a yellow oil which was used in Step 5 without further purification. Mass spec: m/z 266 $[M+H]^+$.

**Step 4**

**Intermediate 21: rac-6-bromo-2-(1-methylpyrrolidin-2-yl)-1H-pyrrolo[3,2-c]pyridine**

To a solution of rac-6-bromo-2-(pyrrolidin-2-yl)-1H-pyrrolo[3,2-c]pyridine trifluoroacetate (**Intermediate 20,** 10 g, 35.8 mmol) in MeOH (300 mL) were added $Et_3N$ (10.9 g, 108 mmol) and paraformaldehyde (5.37 g, 179 mmol). The reaction mixture was stirred for 1h at rt. $NaBH_3CN$ (6.77 g, 107.4 mmol) was added and the reaction mixture was stirred overnight at 60°C. The reaction mixture was quenched with water and the resulting solution was extracted three times with EtOAc. The organic layers were combined, washed with brine, dried over anhydrous $Na_2SO_4$, filtered, and concentrated under reduced pressure. The residue was chromatographed by reverse-phase flash chromatography (C18 aq) using $MeCN/H_2O$ (45/55) as eluent to afford rac-6-bromo-2-(1-methylpyrrolidin-2-yl)-1H-pyrrolo[3,2-c]pyridine (**Intermediate 21,** 8 g, 69%) as a yellow oil. Mass spec: m/z 280 $[M+H]^+$.

**Step 5**

**Intermediate 22: rac-tert-butyl 6-bromo-2-(1-methylpyrrolidin-2-yl)-1H-pyrrolo[3,2-c]pyridine-1-carboxylate**

To a solution of rac-6-bromo-2-(1-methylpyrrolidin-2-yl)-1H-pyrrolo[3,2-c]pyridine (**Intermediate 21,** 8 8 g, 25.8 mmol) in DCM (200 mL) were added $Boc_2O$ (8.44 g, 38.7 mmol), $Et_3N$ (7.82 g, 77.4 mmol) and DMAP (310 mg, 2.58 mmol). The reaction mixture was stirred overnight at rt and then quenched with water. The resulting solution was extracted three times with EtOAc and the organic layers were combined, washed with brine, dried over anhydrous $Na_2SO_4$, filtered, and concentrated under reduced pressure. The crude material was purified by flash chromatography on silica gel using EtOAc/petroleum ether (3/2) as eluent to afford rac-tert-butyl 6-bromo-2-(1-methylpyrrolidin-2-yl)-1H-pyrrolo[3,2-c]pyridine-1-carboxylate (**Intermediate 22,** 8.1 g, 71%) as a yellow oil. Mass spec: m/z 380 $[M+H]^+$.

**Step 6**

**Intermediate 17: (2R)-2-{6-bromo-1H-pyrrolo[3,2-c]pyridin-2-yl}-1-methylpyrrolidine and Intermediate 23: (2S)-2-{6-bromo-1H-pyrrolo[3,2-c]pyridin-2-yl}-1-methylpyrrolidine**

rac-6-bromo-2-(1-methylpyrrolidin-2-yl)-1H-pyrrolo[3,2-c]pyridine (**Intermediate 21,** 9.00 g, 30.5 mmol) was separated by SFC (Column: CHIRALPAK IH 3*25 cm, 5 μm; Mobile Phase A: $CO_2$, Mobile Phase B: MeOH (+1%-2M $NH_3$ in MeOH); Flow rate: 85 mL/min; Gradient: isocratic 20% B) to afford (2R)-2-{6-bromo-1H-pyrrolo[3,2-c]pyridin-2-yl}-1-methylpyrrolidine (**Intermediate 17,** 1st eluting peak, 3.5 g, 37%) as a white solid and (2S)-2-{6-bromo-1H-pyrrolo[3,2-c]pyridin-2-yl}-1-methylpyrrolidine (**Intermediate 23,** 2nd eluting peak, 4.0 g, 40%) as a white solid.

**Intermediate 17: (2R)-2-{6-bromo-1H-pyrrolo[3,2-c]pyridin-2-yl}-1-methylpyrrolidine**

1$^{st}$ eluting peak from SFC purification. Retention time: 4.35 mins. Enantiomeric excess (ee) 99.8%.
$^1$H NMR (400 MHz, DMSO-d6) δ ppm 11.56 (s, 1H), 8.49 (s, 1H), 7.43 (s, 1H), 6.45 (s, 1H), 3.34 (t, J = 9.0 Hz, 1H), 3.13 (t, J = 7.8 Hz, 1H), 2.24 - 2.30 (m, 1H), 2.15 (s, 4H), 1.87 - 1.90 (m, 1H), 1.75 - 1.84 (m, 2H).

**Intermediate 23: (2S)-2-{6-bromo-1H-pyrrolo[3,2-c]pyridin-2-yl}-1-methylpyrrolidine**

2nd eluting peak from SFC purification. Retention time: 4.87 mins.
Enantiomeric excess (ee) 99.8%.
$^1$H NMR (400 MHz, DMSO-d6) δ ppm 11.56 (s, 1H), 8.49 (s, 1H), 7.43 (s, 1H), 6.45 (s, 1H), 3.35 (t, J = 11.4 Hz, 1H), 3.14 (t, J = 8.0 Hz, 1H), 2.26 (t, J = 8.6 Hz, 1H), 2.15 (s, 4H), 1.88 (d, J = 8.0 Hz, 1H), 1.80 (d, J = 8.0 Hz, 2H).

**Step 7**

**Intermediate 4: *tert*-butyl (*R*)-6-bromo-2-(1-methylpyrrolidin-2-yl)-1*H*-pyrrolo[3,2-c]pyridine-1-carboxylate**

A 500 mL round-bottom flask was charged with (2R)-2-{6-bromo-1H-pyrrolo[3,2-c]pyridin-2-yl}-1-methylpyrrolidine (**Intermediate 17,** 500 mg, 1.68 mmol), DCM (10 mL), $Et_3N$ (541 mg, 5.35 mmol), DMAP (21.8 mg, 0.178 mmol), $(Boc)_2O$ (778 mg, 3.57 mmol) and the reaction was stirred overnight at room temperature. The reaction was quenched with water (200 mL). The resulting solution was extracted with ethyl acetate (3 × 200 mL) and the organic layers were combined, washed with brine (2 × 300 mL), dried over anhydrous sodium sulfate, filtered, and concentrated under

reduced pressure. The residue was chromatographed on a silica gel column, eluting with EtOAc/Petroleum ether (35/65) to afford tert-butyl (R)-6-bromo-2-(1-methylpyrrolidin-2-yl)-1H-pyrrolo[3,2-c]pyridine-1-carboxylate (**Intermediate 4, 600** mg, 92%) as an off-white solid. $^1$H NMR (400 MHz, DMSO-d6) δ ppm 1.65 (s, 9H), 1.71-1.75 (m, 3H), 2.30-2.33 (m, 1H), 2.34 (s, 3H), 2.37-2.40 (m, 1H), 3.11-3.14 (m, 1H), 3.87-3.91 (m, 1H), 6.80 (s, 1H), 8.05 (s, 1H), 8.61 (s, 1H). Mass spec: m/z: 379.9 $[M+H]^+$. Mass spec: m/z: 380.0 $[M+H]^+$.

**Step 8**

**Intermediate 24: *tert*-butyl (S)-6-bromo-2-(1-methylpyrrolidin-2-yl)-1H-pyrrolo[3,2-c]pyridine-1-carboxylate**

**Intermediate 24** was prepared in an analogous manner to **Intermediate 4** following **Step 7** of **Scheme 3** and exhibited the following data: $^1$H NMR (400 MHz, DMSO-d6) δ ppm 1.65 (s, 9H), 1.71-1.75 (m, 3H), 2.30-2.33 (m, 1H), 2.34 (s, 3H), 2.37-2.40 (m, 1H), 3.11-3.14 (m, 1H), 3.87-3.91 (m, 1H), 6.80 (s, 1H), 8.05 (s, 1H), 8.61 (s, 1H). Mass spec: m/z: 379.9 $[M+H]^+$. Mass spec: m/z: 380.0 $[M+H]^+$.

**Intermediate 13: *tert*-butyl (*R*)-2-ethynylpyrrolidine-1-carboxylate**

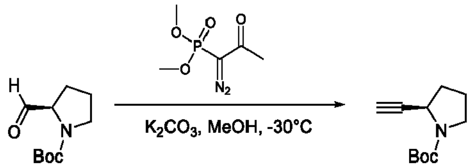


Intermediate 13

To a cooled (-30°C) solution of *tert*-butyl-(R)-2-formylpyrrolidine-1-carboxylate (CAS No: 73365-02-3, 50.0 g, 250.94 mmol) in methanol (500 mL) was added potassium carbonate (51.94 g, 376.41 mmol). Dimethyl(1-diazo-2-oxopropyl)phosphonate (CAS No: 90965-06-3, 57.85 g, 301.13 mmol) was then added dropwise over 20 min and the reaction mixture was stirred at -30°C for 3h. The reaction mixture was diluted with water (1000 mL) and extracted with diethyl ether (3 × 500 mL). The combined organic layers were dried over anhydrous $Na_2SO_4$ and concentrated *in vacuo* to obtain the crude compound. The crude material was purified by combi-flash chromatography, by eluting with 10% ethyl acetate in heptane, to afford tert-butyl (R)-2-ethynylpyrrolidine-1-carboxylate **(Intermediate 13,** 35.0 g, 71%) as a yellow liquid. $^1$H NMR (400 MHz, DMSO-$d_6$) δ ppm 1.40 (s, 9H), 1.81-1.89 (m, 3H), 1.99-2.12 (m, 1H), 3.08-3.25 (m, 2H), 3.26-3.35 (m, 1H), 4.32-4.38 (m, 1H).

**Intermediate 8 was synthesised following Scheme 4**

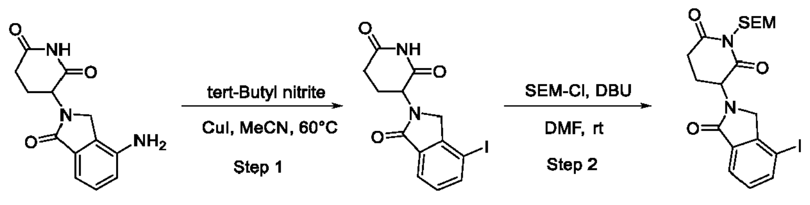


Intermediate 25

Intermediate 8

Scheme 4

**Step 1**

**Intermediate 25: 3-(4-iodo-1-oxoisoindolin-2-yl)piperidine-2,6-dione**

To a solution of 3-(4-amino-1-oxoisoindolin-2-yl)piperidine-2,6-dione (CAS No 191732-72-6, 25.0 g, 96.41 mmol) in acetonitrile (300 mL) was added copper(I) iodide (28.10 g, 144.6 mmol). The reaction mixture was purged with argon gas for 20 min then tert-butyl nitrite (16.57 g, 144.6 mmol) was added and the reaction mixture was heated at

60°C for 12h. The reaction mixture was poured then into water (300 mL), a solid precipitate formed, which was filtered and dried under vacuum to afford 3-(4-iodo-1-oxoisoindolin-2-yl) piperidine-2,6-dione (**Intermediate 25**, 45.2 g) as an off white solid, which was used for the next step without further purification. $^1$H NMR (400 MHz, DMSO-d6) δ ppm 1.98-2.04 (m, 2H), 2.82-2.98 (m, 2H), 4.11-4.32 (m, 2H), 5.13 (m, 1H), 7.31-7.42 (m, 1H), 7.71-7.80 (m, 1H), 7.98-8.06 (m, 1H), 10.99 (br s, 1H). Mass spec: m/z [M+H]$^+$371.0.

**Step 2**

**Intermediate 8: 3-(4-iodo-1-oxoisoindolin-2-yl)-1-((2-(trimethylsilyl) ethoxy)methyl) piperidine-2,6-dione**

To solution of 3-(4-iodo-1-oxoisoindolin-2-yl)piperidine-2,6-dione (**Intermediate 25,** 30.0 g, 81.06 mmol) in dimethylformamide (250 mL) was added DBU (44.07 g, 283.7 mmol) at 0°C, then 2-(trimethylsilyl)ethoxymethyl chloride (52 mL, 283.7 mmol) was added and the reaction mixture was stirred at room temperature for 16h under a nitrogen atmosphere. The reaction mixture was quenched with water (700 mL), diluted with ethyl acetate (500 mL) and filtered through a celite bed. The organic layer was separated, washed with water (500 mL), dried over anhydrous $Na_2SO_4$ and concentrated *in vacuo.* The crude material was purified by combi-flash chromatography, eluting with 50% ethyl acetate in heptane to afford 3-(4-iodo-1-oxoisoindolin-2-yl)-1-((2-(trimethylsilyl) ethoxy) methyl) piperidine-2,6-dione (**Intermediate 8,** 8.56 g, 21%) as an off-white solid. $^1$H NMR (400 MHz, DMSO-d6) δ ppm -0.02 (s, 9H), 0.78-0.90 (m, 2H), 2.03-2.11 (m, 1H), 2.76-2.82 (m, 1H), 3.02-3.12 (m, 1H), 3.48-3.60 (m, 3H), 4.08-4.13 (m, 1H), 4.28-4.33 (m, 1H), 5.02-5.10 (m, 2H), 5.26-5.31 (m, 1H), 7.31-7.37 (m, 1H), 7.79 (d, J=7.46 Hz, 1H), 8.05 (d, J=7.46 Hz, 1H). Mass spec: m/z [M-H]$^-$ 498.8.

**Example 2 was synthesised following Scheme 5**

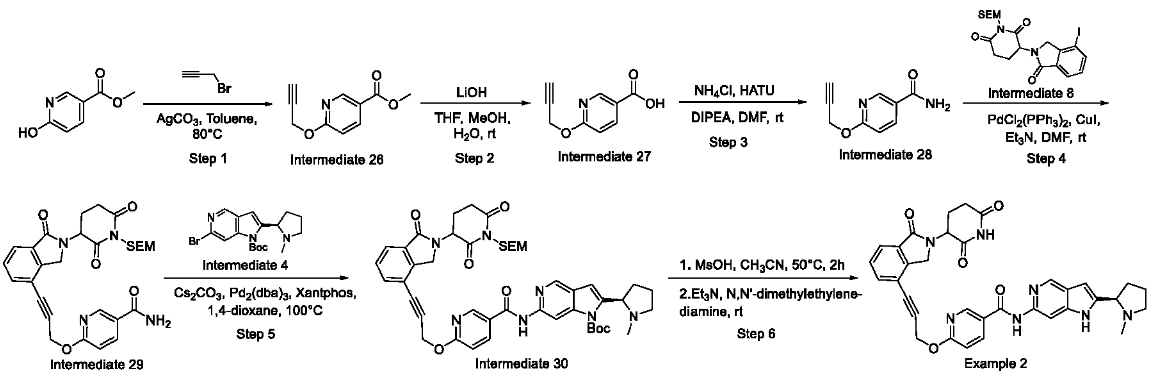


## Scheme 5

**Step 1**

**Intermediate 26: methyl 6-(prop-2-yn-1-yloxy)nicotinate**

To a solution of methyl 6-hydroxynicotinate (CAS No: 66171-50-4, 2.00 g, 13.060 mmol) in toluene (50 mL) was added 3-bromoprop-1-yne (CAS No: 106-96-7, 1.86 g, 15.67 mmol) followed by silver carbonate (9.47 g, 32.65 mmol) and the reaction mixture was heated at 80°C for 48h. The reaction mixture was diluted with water (500 mL) and extracted with ethyl acetate (3 × 200 mL). The combined organic phases were dried over anhydrous $Na_2SO_4$, filtered and concentrated *in vacuo.* The crude material was purified by combi-flash chromatography by eluting with 45% ethyl acetate in heptane to afford methyl 6-(prop-2-yn-1-yloxy)nicotinate (**Intermediate 26, 0.50** g, 20%) as an off-white solid. $^1$H NMR (400 MHz, $CDCl_3$) δ ppm 2.48 (s, 1H), 3.90 (s, 3H), 5.03 (s, 2H), 6.81 (d, *J*=8.80 Hz, 1H), 8.17 (dd, *J*=8.80, 2.40 Hz, 1H), 8.81 (d, *J*=2.40 Hz, 1H). Mass spec: m/z: 191.9 [M+H]$^+$.

**Step 2**

**Intermediate 27: 6-(prop-2-yn-1-yloxy) nicotinic acid**

To a solution of methyl 6-(prop-2-yn-1-yloxy)nicotinate (**Intermediate 26,** 1.00 g, 5.23 mmol) in tetrahydrofuran (10 mL), methanol (10 mL) and water (5 mL) was added lithium hydroxide (0.51 g, 20.92 mmol) and the reaction mixture was stirred at room temperature for 16h. The reaction mixture was diluted with water (5 mL), acidified to pH 4 with 0.5 N HCl and extracted with ethyl acetate (2 × 250 mL). The combined organic phases were dried over $Na_2SO_4$ and concentrated *in vacuo* to afford 6-(prop-2-yn-1-yloxy)nicotinic acid (**Intermediate 27, 0.75** g, 81%) as an off-white solid, which was used for the next step without further purification. $^1H$ NMR (400 MHz, $CDCl_3$) δ ppm 2.49 (s, 1H), 5.05 (s, 2H), 6.85 (d, *J*=8.80 Hz, 1H), 8.22 (dd, *J*=8.80, 2.0 Hz, 1H), 8.88 (d, *J*=2.0 Hz, 1H). Mass spec: m/z: 177.9 $[M+H]^+$.

**Step 3**

**Intermediate 28: 6-(prop-2-yn-1-yloxy)nicotinamide**

To a solution of 6-(prop-2-yn-1-yloxy)nicotinic acid (**Intermediate 27, 0.75** g, 4.23 mmol) in dimethylformamide (15 mL) was added HATU (2.54 g, 6.35 mmol) and *N, N*-diisopropylethylamine (1.73 g, 12.70 mmol) at room temperature and stirred for 10 min. Ammonium chloride (1.26 g, 21.16 mmol) was added at room temperature and the reaction mixture was stirred at room temperature for 16h. The reaction mixture was quenched with ice cold water (200 mL) and extracted with ethyl acetate (3 × 150 mL). The combined organic phases were dried over anhydrous $Na_2SO_4$ and concentrated *in vacuo.* The crude material was purified by combi-flash chromatography by eluting with 45% ethyl acetate in heptane to afford 6-(prop-2-yn-1-yloxy)nicotinamide (**Intermediate 28,** 0.25 g, 34%) as a white solid. $^1H$ NMR (400 MHz, $CDCl_3$) δ ppm 2.49 (s, 1H), 5.04 (s, 2H), 6.86 (d, *J*=8.40 Hz, 1H), 8.07 (d, *J*=8.40, 2.40 Hz, 1H), 8.62 (d, *J*=2.40 Hz, 1H). Mass spec: m/z: 177.0 $[M+H]^+$.

**Step 4**

**Intermediate 29: 6-((3-(2-(2, 6-dioxo-1-((2-(trimethylsilyl)ethoxy)methyl) piperidin-3-yl)-1-oxoisoindolin-4-yl) prop-2-yn-1-yl)oxy)nicotinamide**

To a solution of 6-(prop-2-yn-1-yloxy)nicotinamide (**Intermediate 28, 0.22** g, 1.25 mmol) in dimethylformamide (5 mL) was added 3-(4-iodo-1-oxoisoindolin-2-yl)-1-((2-(trimethylsilyl)ethoxy)methyl)piperidine-2,6-dione (**Intermediate 8,** 0.31 g, 0.62 mmol) followed by triethylamine (4.78 g, 44.95 mmol). The reaction mixture was purged with argon for 15 min then copper(I) iodide (0.024 g, 0.12 mmol) and $PdCl_2(PPh_3)_2$ (0.092 g, 0.12 mmol) were added. The reaction mixture was further purged with argon for 10 min and stirred at room temperature for 2h. The reaction mixture was diluted with water (100 mL) and extracted with ethyl acetate (3 × 150 mL). The combined organic phases were dried over anhydrous $Na_2SO_4$ and concentrated *in vacuo.* The crude material was purified by combi-flash chromatography by eluting with 90% ethyl acetate in heptane to afford 6-((3-(2-(2, 6-dioxo-1-((2-(trimethylsilyl)ethoxy)methyl)piperidin-3-yl)-1-oxoisoindolin-4-yl) prop-2-yn-1-yl)oxy)nicotinamide (**Intermediate 29,** 0.35 g, 51%) as a yellow solid. $^1H$ NMR (400 MHz, DMSO-$d_6$) δ ppm -0.03 (s, 9H), 0.80-0.88 (m, 2H), 2.02-2.11 (m, 1H), 2.30-2.41 (m, 1H), 2.76-2.82 (m, 1H), 3.01-3.12 (m, 1H), 3.49-3.57 (m, 2H), 4.18-4.24 (m, 1H), 4.38-4.43 (m, 1H), 5.02-5.09 (m, 2H), 5.24-5.28 (m, 1H), 5.32 (s, 2H), 6.97 (d, *J*=8.80 Hz, 1H), 7.42 (br s, 1H), 7.57 (t, *J*=7.60 Hz, 1H), 7.72 (d, *J*=7.60 Hz, 1H), 7.78 (d, *J*=7.60 Hz, 1H), 7.99 (br s, 1H), 8.19 (dd, *J*=8.80, 2.40 Hz, 1H), 8.71 (d, *J*=2.40 Hz, 1H). Mass spec: m/z: 546.9 $[M-H]^-$.

**Step 5**

**Intermediate 30: *tert*-butyl 6-(6-((3-(2-(2,6-dioxo-1-((2-(trimethylsilyl)ethoxy)methyl)piperidin-3-yl)-1-oxoisoindolin-4-yl)prop-2-yn-1-yl)oxy)nicotinamido)-2-((*R*)-1-methylpyrrolidin-2-yl)-1*H*-pyrrolo[3,2-c]pyridine-1-carboxylate**

To a solution of 6-((3-(2-(2, 6-dioxo-1-((2-(trimethylsilyl) ethoxy) methyl) piperidin-3-yl)-1-oxoisoindolin-4-yl) prop-2-yn-1-yl) oxy) nicotinamide (**Intermediate 29, 0.33** g, 0.60 mmol) in 1,4-dioxane (10 mL) was added *tert*-butyl (*R*)-6-bromo-2-(1-methylpyrrolidin-2-yl)-1*H*-pyrrolo[3,2-c]pyridine-1-carboxylate **(Intermediate 4,** 0.27 g, 0.72 mmol) followed by cesium carbonate (0.49 g, 1.50 mmol) at room temperature. The reaction mixture was purged with argon for 15 min then $Pd_2(dba)_3$ (0.085 g, 0.090 mmol) and Xantphos (0.11 g, 0.18 mmol) were added. The reaction mixture was further purged with argon for 10 min and then heated at 100°C for 2h. The reaction mixture was filtered through celite, washed with ethyl acetate (50 mL) and the filtrate was concentrated *in vacuo* to obtain crude compound. The crude material was purified by combi-flash chromatography by eluting with 90% ethyl acetate in heptane to afford *tert*-butyl 6-(6-((3-(2-(2,6-dioxo-1-((2-(trimethylsilyl)ethoxy)methyl)piperidin-3-yl)-1-oxoisoindolin-4-yl)prop-2-yn-1-yl)oxy)nicotinamido)-2-((*R*)-1-methylpyrrolidin-2-yl)-1*H*-pyrrolo[3,2-c]pyridine-1-carboxylate (**Intermediate 30,** 0.25 g, 49%) as yellow solid. $^1$H NMR (400 MHz, DMSO-$d_6$) δ ppm -0.05 (s, 9H), 0.78-0.84 (m, 2H), 1.58-1.65 (m, 2H), 1.69 (s, 9H), 1.73-1.79 (m, 2H), 2.01-2.09 (m, 1H), 2.29-2.33 (m, 1H), 2.36 (s, 3H), 2.37-2.43 (m, 1H), 2.72-2.81 (m, 1H), 2.99-3.18 (m, 2H), 3.45-3.52 (m, 2H), 3.88-3.96 (m, 1H), 4.20-4.24 (m, 1H), 4.40-4.44 (m, 1H), 4.97-5.06 (m, 2H), 5.23-5.27 (m, 1H), 5.36 (s, 2H), 6.76 (s, 1H), 7.02 (d, *J*=8.40 Hz, 1H), 7.54-7.61 (m, 1H), 7.74 (d, *J*=7.60 Hz, 1H), 7.79 (d, *J*=7.60 Hz, 1H), 8.36 (dd, *J*=8.40, 2.80 Hz, 1H), 8.59 (s, 1H), 8.90 (s, 1H), 8.88 (d, *J*=2.80 Hz, 1H), 8.92 (s, 1H), 10.84 (s, 1H). Mass spec: m/z: 847.9 [M+H]$^+$.

**Step 6**

**Example 2: 6-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)nicotinamide**

To a solution of *tert*-butyl 6-(6-((3-(2-(2,6-dioxo-1-((2-(trimethylsilyl)ethoxy)methyl)piperidin-3-yl)-1-oxoisoindolin-4-yl)prop-2-yn-1-yl)oxy)nicotinamido)-2-((*R*)-1-methylpyrrolidin-2-yl)-1*H*-pyrrolo[3,2-c]pyridine-1-carboxylate (**Intermediate 30, 0.25** g, 0.29 mmol) in acetonitrile (10 mL) was added methane sulfonic acid (0.19 mL, 2.95 mmol) at room temperature and the reaction mixture was heated at 50°C for 2h. To the reaction mixture was added *N,N'*-dimethylethylenediamine (0.17 mL, 1.47 mmol) followed by triethylamine (0.82 mL, 5.89 mmol) and the reaction stirred at room temperature for 3h. The reaction mixture was concentrated *in vacuo* and water (50 mL) added, resulting in a precipitate which was filtered and dried under vacuum to obtain crude compound. The crude material was purified by preparative HPLC (Method A) to afford 6-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)nicotinamide **(Example 2,** 0.045 g, 25%) as an off white solid. $^1$H NMR (400 MHz, DMSO-$d_6$) δ ppm 1.75-1.95 (m, 3H), 1.97-2.05 (m, 1H), 2.11-2.15 (m, 1H), 2.17 (s, 3H), 2.23-2.30 (m, 1H), 2.41-2.45 (m, 1H), 2.56-2.62 (m, 1H), 2.85-2.96 (m, 1H), 3.10-3.17 (m, 1H), 3.32-3.36 (m, 1H), 4.28-4.35 (m, 1H), 4.42-4.48 (m, 1H), 5.12-5.17 (m, 1H), 5.37 (s, 2H), 6.40 (s, 1H), 7.03 (d, *J*=8.40 Hz, 1H), 7.54-7.59 (m, 1H), 7.71-7.75 (m, 1H), 7.76-7.80 (m, 1H), 8.19 (s, 1H), 8.36 (dd, *J*=8.40, 2.40 Hz, 1H), 8.51 (s, 1H), 8.89 (d, *J*=2.40 Hz, 1H), 10.60 (s, 1H), 11.11 (br s, 1H), 11.39 (br s, 1H). Mass spec: m/z: 618.2 [M+H]$^+$.

**Example 3 was synthesised following Scheme 6**

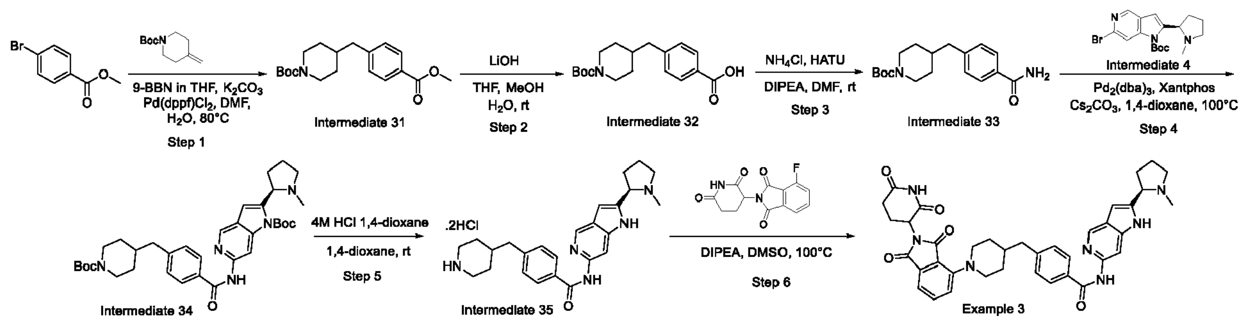


## Scheme 6

**Step** 1

**Intermediate 31: tert-butyl 4-(4-(methoxycarbonyl) benzyl)piperidine-1-carboxylate**

To a solution of methyl 4-bromobenzoate (CAS No: 619-42-1, 3.00 g, 13.95 mmol) in tetrahydrofuran (30 mL) was added 9-borabicyclo[3.3.1]nonane (4.26 g, 16.74 mmol) and the reaction mixture was heated at 60°C for 1h under a nitrogen atmosphere. *tert*-butyl 4-methylenepiperidine-1 -carboxylate (CAS No: 159635-49-1, 3.30 g, 16.74 mmol) in dimethylformamide (20 mL) followed by potassium carbonate (3.04 g, 20.93 mmol) and $PdCl_2$(dppf) (1.79 g, 2.09 mmol) were added at room temperature and the reaction mixture was stirred at 80°C for 2h. The reaction mixture was cooled to room temperature and diluted with water (100 mL) and extracted with ethyl acetate (3 × 100 mL). The combined organic phases were dried over anhydrous $Na_2SO_4$ and concentrated *in vacuo.* The crude material was purified by combi-flash chromatography by eluting with 40% ethyl acetate in heptane to afford *tert*-butyl 4-(4-(methoxycarbonyl) benzyl)

piperidine-1-carboxylate (**Intermediate 31,** 4.80 g, 100%) as a colorless liquid. $^1$H NMR (400 MHz, DMSO-$d_6$) δ ppm 1.37 (s, 9H), 1.49-1.62 (m, 2H), 1.66-1.82 (m, 3H), 2.55-2.68 (m, 4H), 3.83 (s, 3H), 3.88-3.91 (m, 2H), 7.31 (d, *J*=7.88 Hz, 2H), 7.87 (d, *J*=7.88 Hz, 2H). Mass spec: m/z: 234.2 [M-100+H]$^+$.

**Step 2**

**Intermediate 32: 4-((1-(tert-butoxycarbonyl) piperidin-4-yl) methyl)benzoic acid**

To a solution of *tert*-butyl 4-(4-(methoxycarbonyl) benzyl) piperidine-1-carboxylate (**Intermediate 31,** 4.80 g, 14.4 mmol) in tetrahydrofuran (20 mL), methanol (10 mL) and water (10 mL) was added lithium hydroxide (1.06 g, 43.2 mmol) and the reaction mixture was stirred at room temperature for 12h. The reaction mixture was concentrated *in vacuo* to obtain crude compound. The crude material was diluted with water (50 mL) and acidified to pH~4 with 1N HCl (20 mL), resulting in a solid precipitate which was collected by filtration and dried to afford 4-((1-(tert-butoxycarbonyl)piperidin-4-yl) methyl)benzoic acid (**Intermediate 32,** 4.00 g) as a white solid, which was used for the next step without further purification. $^1$H NMR (400 MHz, DMSO-$d_6$) δ ppm 0.98-1.04 (m, 2H), 1.37 (s, 9H), 1.50-1.54 (m, 2H), 1.67-1.70 (m, 1H), 2.57-2.62 (m, 4H), 3.87-3.91 (m, 2H), 7.28-7.30 (m, 2H), 7.84-7.86 (m, 2H), 12.78 (br s, 1H). Mass spec: m/z: 318.0 [M-H]$^-$.

**Step 3**

**Intermediate 33: tert-butyl 4-(4-carbamoylbenzyl) piperidine-1-carboxylate**

To a solution of 4-((1-(*tert*-butoxycarbonyl) piperidin-4-yl) methyl)benzoic acid (**Intermediate 32, 4.00** g, 12.52 mmol) in dimethylformamide (10 mL) was added HATU (7.36 g, 18.79 mmol) and the reaction mixture stirred at room temperature for 15 min. Ammonium chloride (2.68 g, 50.09 mmol) and *N, N*-diisopropylethylamine (8.75 mL, 50.09 mmol) were then added and the reaction mixture was stirred at room temperature for 16h. The reaction mixture was diluted with cold water (100 mL) and extracted with ethyl acetate (3 × 100 mL). The combined organic phases were dried over anhydrous $Na_2SO_4$ and concentrated *in vacuo.* The crude material was purified by combi-flash chromatography by eluting with 50% ethyl acetate in heptane to afford *tert*-butyl 4-(4-carbamoylbenzyl) piperidine-1-carboxylate (**Intermediate 33,** 3.00 g, 75%) as a white solid. $^1$H NMR (400 MHz, DMSO-$d_6$) δ ppm 0.99-1.06 (m, 2H), 1.38 (s, 9H), 1.48-1.54 (m, 2H), 1.66-1.72 (m, 1H), 2.52-2.58 (m, 4H), 3.86-3.92 (m, 2H), 7.22-7.24 (m, 3H), 7.77-7.79 (m, 2H), 7.88 (br s, 1H). Mass spec: m/z: 316.9 [M-H]$^-$.

**Step 4**

**Intermediate 34: *tert*-butyl (R)-6-(4-((1-(tert-butoxycarbonyl)piperidin-4-yl)methyl)benzamido)-2-(1-methylpyrrolidin-2-yl)-1*H*-pyrrolo [3, 2-c]pyridine-1-carboxylate**

To a solution of *tert*-butyl 4-(4-carbamoylbenzyl) piperidine-1-carboxylate **(Intermediate 33,** 0.42 g, 1.31 mmol) in 1, 4-dioxane (10 mL) was added *tert*-butyl (*R*)-6-bromo-2-(1-methylpyrrolidin-2-yl)-1*H*-pyrrolo[3,2-*c*]pyridine-1-carboxylate (**Intermediate 4,** 0.60 g, 1.64 mmol) followed by cesium carbonate (1.07 g, 3.27 mmol) at room temperature. The reaction mixture was purged with argon for 15 min then $Pd_2(dba)_3$ (0.15 g, 0.16 mmol) and Xantphos (0.29 g, 0.49 mmol) were added. The reaction mixture was further purged with argon for 10 min and stirred at 100°C for 2h. The reaction mixture was concentrated *in vacuo* to obtain crude compound. The crude material was purified by combi-flash chromatography by eluting with 80% ethyl acetate in heptane to afford *tert*-butyl (*R*)-6-(4-((1-(tert-butoxycarbonyl)piperidin-4-yl)methyl) benzamido)-2-(1-methylpyrrolidin-2-yl)-1*H*-pyrrolo[3,2-*c*]pyridine-1-carboxylate (**Intermediate 34,** 0.54 g, 53%) as a white solid. $^1$H NMR (400 MHz, DMSO-$d_6$) δ ppm 1.01-1.09 (m, 2H), 1.38 (s, 9H), 1.53-1.62 (m, 4H), 1.69 (s, 9H), 1.74-1.80 (m, 2H), 2.25-2.31 (m, 1H), 2.35 (s, 3H), 2.38-2.40 (m, 1H), 2.54-2.61 (m, 2H), 2.65-2.72 (m, 2H), 3.11-3.16 (m, 1H), 3.88-3.93 (m, 3H), 6.74 (s, 1H), 7.30 (d, *J*=7.60 Hz, 2H), 7.97 (d, *J*=7.20 Hz, 2H), 8.58 (s, 1H), 8.92 (s, 1H), 10.58 (br s, 1H). Mass spec: m/z: 618.1 [M+H]$^+$.

**Step 5**

**Intermediate 35: (*R*)-*N*-(2-(1-methylpyrrolidin-2-yl)-1*H*-pyrrolo[3, 2-*c*]pyridin-6-yl)-4-(piperidin-4-ylmethyl) benzamide dihydrochloride**

To a solution of *tert*-butyl (*R*)-6-(4-((1-(tert-butoxycarbonyl)piperidin-4-yl)methyl)benzamido)-2-(1-methylpyrrolidin-2-yl)-1*H*-pyrrolo[3,2-*c*]pyridine-1-carboxylate (**Intermediate 34, 0.52** g, 0.84 mmol) in 1, 4-dioxane (8.0 mL) was

added 4M hydrochloric acid in 1,4-dioxane (8.0 mL) at 0°C and the reaction mixture was stirred at room temperature for 4h. The reaction mixture was concentrated *in vacuo* to afford (*R*)-*N*-(2-(1-methylpyrrolidin-2-yl)-1*H*-pyrrolo[3, 2-c]pyridin-6-yl)-4-(piperidin-4-ylmethyl)benzamide dihydrochloride (**Intermediate 35,** 0.50 g) as a white solid which was used for next step without further purification. $^1$H NMR (400 MHz, DMSO-$d_6$) δ ppm 1.38-1.46 (m, 2H), 1.69-1.74 (m, 2H), 1.86-1.89 (m, 1H), 2.15-2.21 (m, 2H), 2.31-2.40 (m, 2H), 2.66-2.71 (m, 2H), 2.81 (s, 3H), 2.85-2.89 (m, 1H), 3.17-3.24 (m, 3H), 3.70-3.78 (m, 2H), 4.72-4.78 (m, 1H), 7.29 (s, 1H), 7.42-7.44 (m, 2H), 8.16-8.18 (m, 2H), 8.32 (s, 1H), 8.72-8.76 (m, 1H), 8.99 (br s, 1H), 9.10 (s, 1H), 11.55 (br s, 1H), 12.05 (br s, 1H), 13.33 (br s, 1H). Mass spec: m/z: 418.3 $[M+H]^+$.

Step 6

**Example 3: 4-((1-(2-(2,6-dioxopiperidin-3-yl)-1,3-dioxoisoindolin-4-yl)piperidin-4-yl)methyl)-*N*-(2-((*R*)-1-methylpyrrolidin-2-yl)-1*H*-pyrrolo[3,2-c]pyridin-6-yl)benzamide**

To a solution of (*R*)-*N*-(2-(1-methylpyrrolidin-2-yl)-1*H*-pyrrolo[3, 2-c]pyridin-6-yl)-4-(piperidin-4-ylmethyl) benzamide dihydrochloride (**Intermediate 35,** 0.40 g, 0.88 mmol) in dimethyl sulfoxide (5.0 mL) was added 2-(2,6-dioxopiperidin-3-yl)-4-fluoroisoindoline-1,3-dione (CAS No: 835616-60-9, 0.24 g, 0.88 mmol) followed by *N,N*-diisopropylethylamine (0.47 g, 3.52 mmol) at room temperature and the reaction mixture was heated at 100°C for 1h. The reaction mixture was diluted with water (50 mL), resulting in a solid precipitate which was collected by filtration and dried to obtain crude compound. The crude material was purified through reverse phase preparative HPLC (Method A) to afford 4-((1-(2-(2,6-dioxopiperidin-3-yl)-1,3-dioxoisoindolin-4-yl)piperidin-4-yl)methyl)-*N*-(2-((*R*)-1-methylpyrrolidin-2-yl)-1*H*-pyrrolo[3,2-*c*]pyridin-6-yl)benzamide (**Example 3,** 0.08 g, 14%) as a yellow solid. $^1$H NMR (400 MHz, DMSO-$d_6$) δ ppm 1.40-1.48 (m, 2H), 1.65-1.74 (m, 2H), 1.76-1.95 (m, 4H), 1.99-2.05 (m, 1H), 2.12-2.15 (m, 1H), 2.17 (s, 3H), 2.23-2.35 (m, 2H), 2.56-2.62 (m, 2H), 2.66-2.70 (m, 2H), 2.81-2.88 (m, 3H), 3.12-3.16 (m, 1H), 3.65-3.72 (m, 2H), 5.06-5.11 (m, 1H), 6.39 (s, 1H), 7.31-7.36 (m, 4H), 7.65-7.69 (m, 1H), 8.00 (d, J=8.26 Hz, 2H), 8.19 (s, 1H), 8.50 (s, 1H), 10.35 (br s, 1H), 10.94 (br s, 1H), 11.35 (br s, 1H). Mass spec: m/z: 674.1 $[M+H]^+$.

**Example 4 was synthesised following Scheme 7**

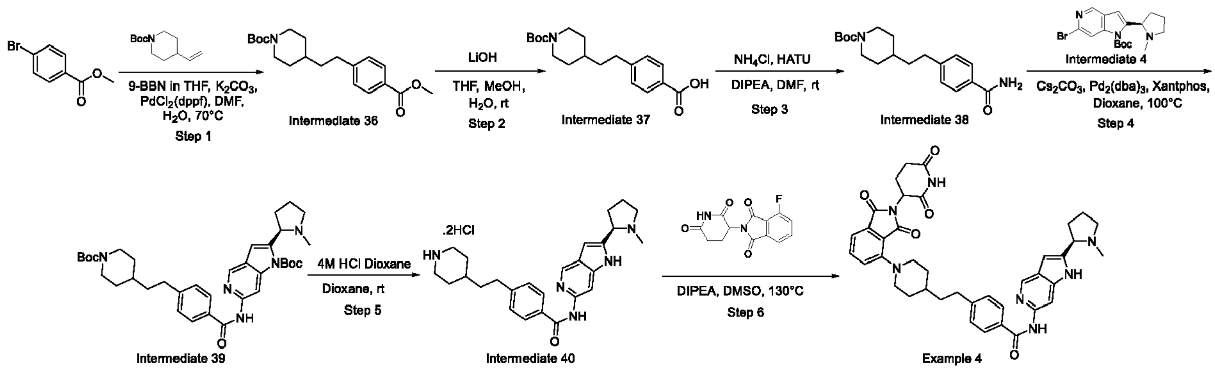


Scheme 7

**Step 1**

**Intermediate 36: tert-butyl 4-(4-(methoxycarbonyl) phenethyl) piperidine-1-carboxylate**

To a solution of *tert*-butyl 4-vinylpiperidine-1-carboxylate (CAS No: 180307-56-6, 3.00 g, 14 mmol) in tetrahydrofuran (50 mL) was added 9-borabicyclo[3.3.1]nonane (30.0 mL, 17 mmol) at 0°C. The reaction mixture was heated at 70°C for 2h. The reaction mixture was cooled to room temperature. To the reaction mixture was added methyl 4-bromobenzoate (CAS No: 619-42-1, 3.70 g, 17 mmol) followed by potassium carbonate (3.1 g, 21.0 mmol) and $PdCl_2$(dppf) (1.6 g, 1.8 mmol) in dimethylformamide (20 mL) and water (3 mL) at room temperature. The reaction mixture was

further heated at 70°C for 3h. Progress of the reaction was monitored by TLC. After completion, the reaction mixture was poured into water (100 mL) and extracted with ethyl acetate (3 × 100 mL). The combined organic phases were dried over anhydrous $Na_2SO_4$ and concentrated *in vacuo* to obtain crude compound. The crude material was purified by combi-flash chromatography by eluting with 20% ethyl acetate in heptane to afford *tert*-butyl 4-(4-(methoxycarbonyl) phenethyl) piperidine-1-carboxylate (**Intermediate 36,** 4.00 g, 82%) as a colorless solid. $^1$H NMR (400 MHz, DMSO-$d_6$) δ ppm 0.98-1.02 (m, 2H), 1.38 (s, 9H), 1.49-1.57 (m, 2H), 1.60-1.77 (m, 3H), 2.65-2.69 (m, 4H), 3.83 (s, 3H), 3.88-3.93 (m, 2H), 7.35 (d, *J*=7.60 Hz, 2H), 7.87 (d, *J*=7.60 Hz, 2H). Mass spec: m/z: 291.9 [M-56+H]$^+$.

**Step 2**

**Intermediate 37: 4-(2-(1-(*tert*-butoxycarbonyl)piperidin-4-yl) ethyl)benzoic acid**

To a solution of *tert*-butyl 4-(4-(methoxycarbonyl)phenethyl) piperidine-1-carboxylate (**Intermediate 36,** 4.00 g, 12 mmol) in tetrahydrofuran:methanol:water (1:1:1, 70 mL) was added lithium hydroxide (0.84 g, 95 mmol) at 0°C and the reaction mixture was stirred at room temperature for 16h. The reaction mixture was concentrated *in vacuo* and water (100 mL) added and the mixture acidified with 1N HCl, resulting in a precipitate which was filtered and dried to afford 4-(2-(1-(*tert*-butoxycarbonyl)piperidin-4-yl)ethyl)benzoic acid (**Intermediate 37,** 2.50 g) as an off white solid, which was used for the next step without further purification. $^1$H NMR (400 MHz, DMSO-$d_6$) δ ppm 0.98-1.02 (m, 2H), 1.38 (s, 9H), 1.46-1.48 (m, 1H), 1.49-1.55 (m, 2H), 1.65-1.70 (m, 2H), 2.62-2.70 (m, 4H), 3.88-3.94 (m, 2H), 7.32 (d, *J*=8.0 Hz, 2H), 7.85 (d, *J*=8.0 Hz, 2H), 12.78 (br s, 1H). Mass spec: m/z: 278.0 [M-56+H]$^+$.

**Step 3**

**Intermediate 38: tert-butyl 4-(4-carbamoylphenethyl)piperidine-1-carboxylate**

To a solution of 4-(2-(1-(*tert*-butoxycarbonyl) piperidin-4-yl)ethyl)benzoic acid **(Intermediate 37,** 2.50 g, 7.5 mmol) in dimethylformamide (30 mL) was added HATU (4.4 g, 11 mmol) and *N,N*-diisopropylethylamine (4.0 g, 30 mmol) at 0°C and the reaction stirred for 10 min. Ammonium chloride (1.6 g, 30.0 mmol) was then added at 0°C and the reaction mixture was stirred at room temperature for 16h. The reaction mixture was poured into water (100 mL), resulting in a precipitate, which was filtered and dried under vacuum to afford *tert*-butyl 4-(4-carbamoylphenethyl) piperidine-1-carboxylate (**Intermediate 38,** 1.80 g, 72%) as an off white solid, which was used for next step without further purification. $^1$H NMR (400 MHz, DMSO-$d_6$) δ ppm 0.94-1.06 (m, 2H), 1.39 (s, 9H), 1.46-1.48 (m, 1H), 1.49-1.54 (m, 2H), 1.66-1.69 (m, 2H), 2.61-2.68 (m, 4H), 3.88-3.94 (m, 2H), 7.23 (br s,1H), 7.27 (d, *J*=7.60 Hz, 2H), 7.78 (d, *J*=7.60 Hz, 2H), 7.86 (br s, 1H). Mass spec: m/z: 331.3 [M-H]$^-$.

**Step 4**

**Intermediate 39: *tert*-butyl (R)-6-(4-(2-(1-(*tert*-butoxycarbonyl) piperidin-4-yl) ethyl) benzamido)-2-(1-methylpyrrolidin-2-yl)-1*H*-pyrrolo [3, 2-c] pyridine-1-carboxylate**

To a solution of *tert*-butyl 4-(4-carbamoylphenethyl)piperidine-1-carboxylate **(Intermediate 38, 0.27** g, 0.84 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate **(Intermediate 4, 0.40** g, 1.05 mmol) followed by cesium carbonate (0.68 g, 2.10 mmol) at room temperature. The reaction mixture was purged with argon for 15 min followed by the addition of $Pd_2(dba)_3$ (0.15 g, 0.00157 mmol) and Xantphos (0.18 g, 0.31 mmol). The reaction mixture was further purged with argon for 10 min and then heated at 100°C for 2h. The reaction mixture was concentrated *in vacuo* to obtain crude compound which purified by combi-flash chromatography by eluting with 80% ethyl acetate in heptane to afford *tert*-butyl (R)-6-(4-(2-(1-(tert-butoxycarbonyl)piperidin-4-yl) ethyl)benzamido)-2-(1-methylpyrrolidin-2-yl)-1*H*-pyrrolo[3,2-c]pyridine-1-carboxylate (**Intermediate 39,** 0.45 g, 68%) as a yellow solid. $^1$H NMR (400 MHz, DMSO-$d_6$) δ ppm 1.35-1.38 (m, 2H), 1.40 (s, 9H), 1.41-1.45 (m, 2H), 1.52-1.68 (m, 4H), 1.70 (s, 9H), 1.72-1.78 (m, 2H), 2.31-2.34 (m, 2H), 2.36 (s, 3H), 2.64-2.69 (m, 4H), 3.12-3.16 (m, 1H), 3.90-3.96 (m, 3H), 6.75 (s, 1H), 7.31-7.34 (m, 2H), 7.96-7.98 (m, 2H), 8.59 (s, 1H), 8.93 (s, 1H), 10.59 (br s, 1H). Mass spec: m/z: 532.4 [M-100+H]$^+$.

**Step 5**

**Intermediate 40: (*R*)-*N*-(2-(1-methylpyrrolidin-2-yl)-1*H*-pyrrolo[3,2-c]pyridin-6-yl)-4-(2-(piperidin-4-yl)ethyl)benzamide dihydrochloride**

To a solution of *tert*-butyl (*R*)-6-(4-(2-(1-(tert-butoxycarbonyl)piperidin-4-yl)ethyl)benzamido)-2-(1-methylpyrrolidin-2-yl)-1*H*-pyrrolo[3,2-c]pyridine-1-carboxylate (**Intermediate 39,** 0.40 g, 0.63 mmol) in 1, 4-dioxane (5 mL) was added 4M hydrochloric acid in 1,4-dioxane (5 mL) at 0°C and the reaction mixture was stirred at room temperature for 16h. The reaction mixture was concentrated *in vacuo* to afford as (*R*)-*N*-(2-(1-methylpyrrolidin-2-yl)-1*H*-pyrrolo[3,2-c]pyridin-6-yl)-4-(2-(piperidin-4-yl)ethyl)benzamide dihydrochloride (**Intermediate 40,** 0.40 g) as a brown solid, which was used for next step without further purification. $^1$H NMR (400 MHz, DMSO-$d_6$) δ ppm 1.34-1.39 (m, 2H), 1.52-1.59 (m, 3H), 1.84-1.87 (m, 2H), 2.10-2.20 (m, 2H), 2.32-2.40 (m, 1H), 2.71-2.76 (m, 3H), 2.81 (s, 3H), 3.22-3.26 (m, 3H), 3.54-3.57 (m, 1H), 3.72-3.78 (m, 2H), 4.70-4.76 (m, 1H), 7.29 (s, 1H), 7.44-7.46 (m, 2H), 8.12-8.16 (m, 2H), 8.30 (s, 1H), 8.69-8.78 (m, 1H), 8.94 (s, 1H), 9.10 (s, 1H), 11.54 (br s, 1H), 12.04 (br s, 1H), 13.34 (br s, 1H). Mass spec: m/z: 432.0 $[M+H]^+$.

**Step 6**

**Example 4: 4-(2-(1-(2-(2,6-dioxopiperidin-3-yl)-1,3-dioxoisoindolin-4-yl)piperidin-4-yl)ethyl)-*N*-(2-((*R*)-1-methylpyrrolidin-2-yl)-1*H*-pyrrolo[3,2-c]pyridin-6-yl)benzamide**

To a solution of (*R*)-*N*-(2-(1-methylpyrrolidin-2-yl)-1*H*-pyrrolo [3,2-c] pyridin-6-yl)-4-(2-(piperidin-4-yl) ethyl) benzamide dihydrochloride (**Intermediate 40,** 0.38 g, 0.88 mmol) in dimethyl sulfoxide (5 mL) was added 2-(2,6-dioxopiperidin-3-yl)-4-fluoroisoindoline-1,3-dione (CAS No: 835616-60-9, 0.24 g, 0.88 mmol) followed by *N*,*N*-diisopropylethylamine (0.58 g, 4.40 mmol) at room temperature and the reaction mixture was heated at 130°C for 2h. The reaction mixture was poured into water (10 mL), resulting in a precipitate which was filtered and dried under vacuum to obtain the crude compound. The crude material was purified by preparative HPLC (Method A) to afford 4-(2-(1-(2-(2,6-dioxopiperidin-3-yl)-1,3-dioxoisoindolin-4-yl)piperidin-4-yl)ethyl)-*N*-(2-((*R*)-1-methylpyrrolidin-2-yl)-1*H*-pyrrolo[3,2-c]pyridin-6-yl) benzamide (**Example 4, 0.13** g, 22%) as a yellow solid. $^1$H NMR (400 MHz, DMSO-$d_6$) δ ppm 1.39-1.50 (m, 3H), 1.55-1.68 (m, 2H), 1.78-1.92 (m, 5H), 2.01-2.04 (m, 1H), 2.06-2.12 (m, 1H), 2.16 (s, 3H), 2.23-2.32 (m, 1H), 2.52-2.61 (m, 3H), 2.71-2.75 (m, 2H), 2.82-2.88 (m, 3H), 3.12-3.16 (m, 1H), 3.68-3.73 (m, 2H), 5.07-5.11 (m, 1H), 6.39 (s, 1H), 7.31-7.37 (m, 4H), 7.66-7.69 (m, 1H), 7.99 (d, *J*=8.31 Hz, 2H), 8.19 (s, 1H), 8.50 (s, 1H), 10.36 (s, 1H), 11.36 (s, 1H). Mass spec: m/z: 688.3 $[M+H]^+$.

**Example 5 was synthesised following Scheme 8**

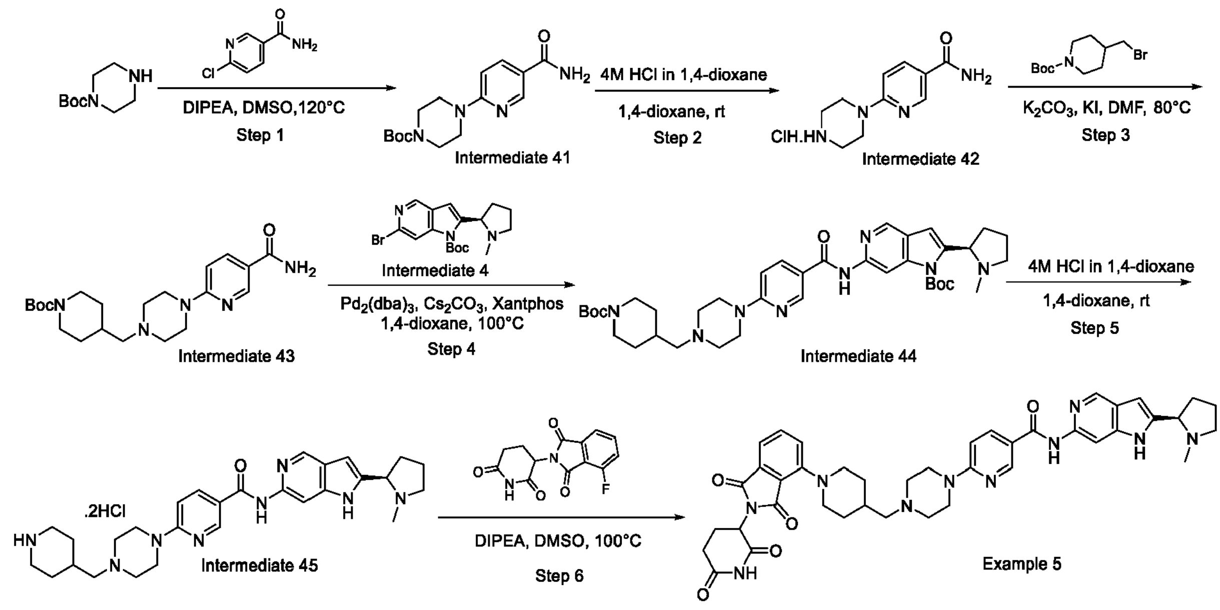


**Scheme 8**

**Step** 1

**Intermediate 41: *tert*-butyl 4-(5-carbamoylpyridin-2-yl) piperazine-1-carboxylate**

To a solution of *tert*-butyl piperazine-1-carboxylate (CAS No: 57260-71-6, 5.00 g, 26.8 mmol) in dimethyl sulfoxide (60 mL) was added 6-chloronicotinamide (CAS No: 6271-78-9, 4.20 g, 26.8 mmol) followed by *N*, *N*-diisopropylethylamine (14.1 mL, 80.5 mmol) and the reaction mixture was heated at 120°C for 16h. The reaction mixture was poured into ice cold water (500 mL), resulting in a precipitate, which was filtered, washed with cold water (2 × 150 mL), heptane (2 × 100 mL) and dried *in vacuo* to afford *tert*-butyl 4-(5-carbamoylpyridin-2-yl) piperazine-1-carboxylate **(Intermediate 41,** 4.00 g, 49%) as a yellow solid, which was used for the next step without further purification. $^1H$ NMR (400 MHz, DMSO-$d_6$) δ ppm 1.42 (s, 9H), 3.38-3.46 (m, 4H), 3.54-3.65 (m, 4H), 6.84 (d, *J*=9.20 Hz, 1H), 7.14 (br s, 1H), 7.77 (br s, 1H), 7.89 (d, *J*=9.20 Hz, 1H), 8.62 (s, 1H).

**Step 2**

**Intermediate 42: 6-(piperazin-1-yl)nicotinamide hydrochloride**

To a solution of tert-butyl 4-(5-carbamoylpyridin-2-yl) piperazine-1-carboxylate **(Intermediate 41,** 3.50 g, 11.0 mmol) in 1, 4-dioxane (60 mL) was added 4M hydrochloric acid in 1,4-dioxane (32 mL) at 0°C and the reaction mixture was stirred at room temperature for 4h. The reaction mixture was concentrated *in vacuo,* washed with ether (2 × 20 mL) and dried under vacuum to afford as 6-(piperazin-1-yl)nicotinamide hydrochloride (**Intermediate 42,** 2.80 g) as a white solid, which was used for the next step without further purification. $^1H$ NMR (400 MHz, DMSO-$d_6$) δ ppm 3.88-4.02 (m, 4H), 2.52-2.55 (m, 4H), 7.14 (d, *J*=9.20 Hz, 1H), 7.36 (br s, 1H), 8.02 (br s, 1H), 8.18 (d, *J*=9.20 Hz, 1H), 8.62 (s, 1H), 9.55 (br s, 1H), 9.58 (br s, 1H). Mass spec: m/z: 207.5 $[M+H]^+$.

**Step 3**

**Intermediate 43: *tert*-butyl 4-((4-(5-carbamoylpyridin-2-yl) piperazin-1-yl) methyl) piperidine-1-carboxylate**

To a solution 6-(piperazin-1-yl)nicotinamide hydrochloride (**Intermediate 42,** 2.80 g, 11.5 mmol) in dimethylformamide (60 mL) was added *tert*-butyl 4-(bromomethyl)piperidine-1-carboxylate (CAS No: 158407-04-6, 3.85 g, 13.8 mmol) followed by potassium iodide (0.19 g, 1.15 mmol) and potassium carbonate (7.97 g, 57.7 mmol) and the reaction mixture was heated at 80°C for 16h. The reaction mixture was poured into ice cold water (200 mL), resulting in a precipitate which filtered, washed with water (80 mL), n-pentane (80 mL) and then dried in under vacuum to afford *tert*-butyl 4-((4-(5-carbamoylpyridin-2-yl) piperazin-1-yl) methyl) piperidine-1-carboxylate (**Intermediate 43,** 0.80 g, 17%) as an off white solid, which was used for the next step without further purification. $^1H$ NMR (400 MHz, DMSO-$d_6$) δ ppm 0.99-1.14 (m, 3H) 1.38 (s, 9H), 1.66-1.76 (m, 4H), 2.60-2.81 (m, 4H), 3.50-3.63 (m, 4H), 3.86-4.02 (m, 4H), 6.81 (d, *J*=9.20 Hz, 1H), 7.11 (br s, 1H), 7.74 (br s, 1H), 7.94 (d, *J*=9.20 Hz, 1H), 8.59 (s, 1H). Mass spec: m/z: 404.3 $[M+H]^+$.

**Step 4**

**Intermediate 44: *tert*-butyl (*R*)-6-(6-(4-((1-(*tert*-butoxycarbonyl)piperidin-4-yl)methyl)piperazin-1-yl) nicotinamido)-2-(1-methylpyrrolidin-2-yl)-1*H*-pyrrolo[3, 2-c]pyridine-1-carboxylate**

To a solution of *tert*-butyl 4-((4-(5-carbamoylpyridin-2-yl)piperazin-1-yl)methyl)piperidine-1-carboxylate (**Intermediate 43,** 0.62 g, 1.53 mmol) in 1,4-dioxane (15 mL) were added *tert*-butyl (R)-6-bromo-2-(1-methylpyrrolidin-2-yl)-1*H*-pyrrolo[3,2-c]pyridine-1-carboxylate (**Intermediate 4,** 0.64 g, 1.69 mmol) and cesium carbonate (1.25 g, 3.84 mmol) at room temperature. The reaction mixture was purged with argon for 15 min, then $Pd_2(dba)_3$ (0.22 g, 0.23 mmol) and Xantphos (0.27 g, 0.46 mmol) were added. The reaction mixture was further purged with argon for 10 min and then heated at 100°C for 2h. The reaction mixture was filtered through celite, washed with ethyl acetate (100 mL) and the filtrate was concentrated *in vacuo* to obtain the crude compound. The crude material was purified by combi-flash chromatography by eluting with 5% MeOH in DCM to afford *tert*-butyl (*R*)-6-(6-(4-((1-(*tert*-butoxycarbonyl)piperidin-4-yl)methyl)piperazin-1-yl)nicotinamido)-2-(1-methylpyrrolidin-2-yl)-1*H*-pyrrolo[3, 2-c]pyridine-1-carboxylate (**Intermediate 44,** 0.32 g, 30%) as a yellow solid. $^1H$ NMR (400 MHz, DMSO-$d_6$) δ ppm 0.90-1.03 (m, 2H), 1.39 (s, 9H), 1.52-1.64 (m, 2H), 1.68 (s, 9H), 1.71-1.80 (m, 4H), 2.11-2.20 (m, 2H), 2.34 (s, 3H), 2.37-2.44 (m, 4H), 2.66-2.72 (m, 3H), 3.08-3.14 (m, 1H), 3.56-3.68 (m, 4H), 3.84-3.98 (m, 4H), 6.73 (s, 1H), 6.86 (d, *J*=9.20 Hz, 1H), 8.15 (d, *J*=9.20 Hz, 1H), 8.56 (s, 1H), 8.79 (s, 1H), 8.89 (s, 1H), 10.48 (br s, 1H). Mass spec: m/z: 703.1 $[M+H]^+$.

**Step 5**

**Intermediate 45: (*R*)-*N*-(2-(1-methylpyrrolidin-2-yl)-1*H*-pyrrolo [3, 2-c] pyridin-6-yl)-6-(4-(piperidin-4-ylmethyl) piperazin-1-yl) nicotinamide dihydrochloride**

To a solution of *tert*-butyl (*R*)-6-(6-(4-((1-(tert-butoxycarbonyl)piperidin-4-yl)methyl)piperazin-1-yl) nicotinamido)-2-(1-methylpyrrolidin-2-yl)-1*H*-pyrrolo[3,2-c]pyridine-1-carboxylate (**Intermediate 44,** 0.30 g, 0.42 mmol) in 1, 4-dioxane (10 mL) was added 4M hydrochloric acid in 1, 4-dioxane (5 mL) and the reaction mixture was stirred at room temperature for 4h. The reaction mixture was concentrated *in vacuo,* the resulting residue was washed with diethyl ether (2 × 50 mL) and dried under reduced pressure to afford (*R*)-*N*-(2-(1-methylpyrrolidin-2-yl)-1*H*-pyrrolo [3, 2-c] pyridin-6-yl)-6-(4-(piperidin-4-ylmethyl) piperazin-1-yl) nicotinamide dihydrochloride **(Intermediate 45,** 0.21 g) as an off-white solid, which was used for the next step without further purification. Mass spec: m/z: 503.34 $[M+H]^+$.

**Step 6**

**Example 5: 6-(4-((1-(2-(2,6-dioxopiperidin-3-yl)-1,3-dioxoisoindolin-4-yl)piperidin-4-yl)methyl)piperazin-1-yl)-*N*-(2-((*R*)-1-methylpyrrolidin-2-yl)-1*H*-pyrrolo[3,2-c]pyridin-6-yl)nicotinamide**

To a solution of (*R*)-*N*-(2-(1-methylpyrrolidin-2-yl)-1*H*-pyrrolo[3,2-c]pyridin-6-yl)-6-(4-(piperidin-4-ylmethyl)piperazin-1-yl)nicotinamide dihydrochloride **(Intermediate 45**, 0.21 g, 0.42 mmol) in dimethyl sulfoxide (10 mL) was added 2-(2,6-dioxopiperidin-3-yl)-4-fluoroisoindoline-1,3-dione (CAS No: 835616-60-9, 0.11 g, 0.42 mmol) followed by *N*, *N*-Diisopropylethylamine (0.36 mL, 2.08 mmol) and the reaction mixture was heated at 100°C for 4h. The reaction mixture was concentrated *in vacuo.* The obtained residue was poured into ice cold water (10 mL), resulting in a precipitate, which was filtered and dried under vacuum to obtain the crude compound. The crude material was purified by preparative HPLC (Method A) to afford 6-(4-((1-(2-(2,6-dioxopiperidin-3-yl)-1,3-dioxoisoindolin-4-yl)piperidin-4-yl)methyl)piperazin-1-yl)-*N*-(2-((*R*)-1-methylpyrrolidin-2-yl)-1*H*-pyrrolo[3,2-c]pyridin-6-yl)nicotinamide **(Example 5, 0.03** g, 9%) as a yellow solid. $^1$H NMR (400 MHz, DMSO-$d_6$) δ ppm 1.29-1.41 (m, 2H), 1.73-1.95 (m, 6H), 1.98-2.08 (m, 1H), 2.09-2.14 (m, 1H), 2.16 (s, 3H), 2.22-2.29 (m, 3H), 2.43-2.47 (m, 4H), 2.57-2.62 (m, 2H), 2.82-2.95 (m, 3H), 3.12-3.16 (m, 1H), 3.28-3.30 (m, 1H), 3.60-3.68 (m, 4H), 3.68-3.75 (m, 2H), 5.07-5.12 (m, 1H), 6.38 (s, 1H), 6.88 (d, *J*=9.20 Hz, 1H), 7.29-7.37 (m, 2H), 7.66-7.70 (m, 1H), 8.14-8.19 (m, 2H), 8.48 (s, 1H), 8.80 (s, 1H), 10.30 (s, 1H), 11.09 (br s, 1H), 11.35 (s, 1H). Mass spec: m/z: 759.2 $[M+H]^+$.

**Example 6 was synthesised following Scheme 9**

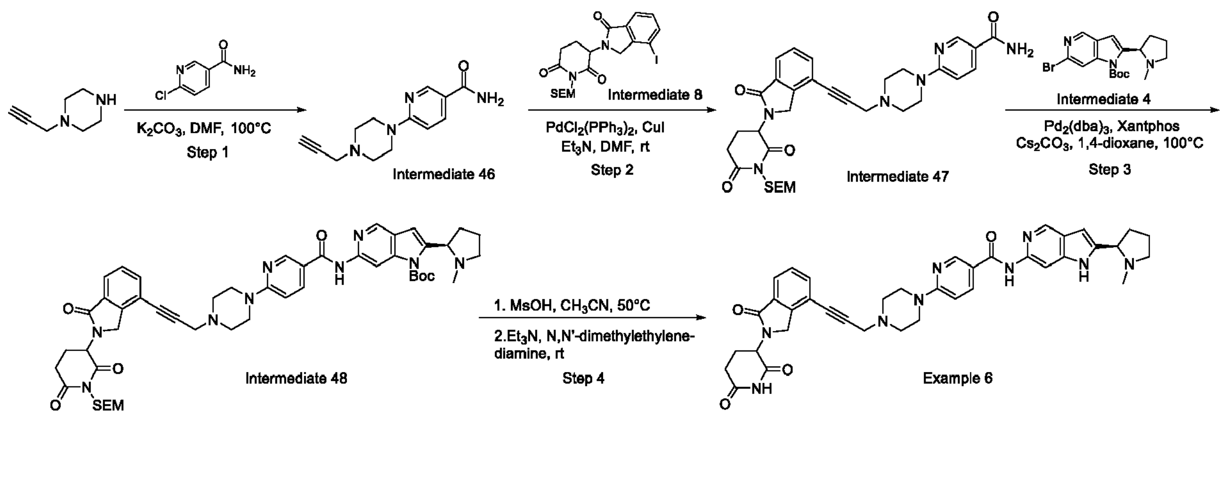


Scheme 9

**Step 1**

**Intermediate 46: 6-(4-(prop-2-yn-1-yl)piperazin-1-yl)nicotinamide**

To a solution of 1-(prop-2-yn-1-yl)piperazine (CAS No: 52070-67-4, 1.50 g, 12 mmol) in dimethylformamide (10 mL) was added 6-chloronicotinamide (CAS No: 6271-78-9, 2.26 g, 14.48 mmol) followed by potassium carbonate (6.00 g,

60 mmol) and the reaction mixture was stirred at 100°C for 12h. The reaction mixture was diluted with water (200 mL) and extracted with ethyl acetate (3 × 200 mL). The combined organic phases were dried over anhydrous $Na_2SO_4$ and concentrated *in vacuo.* The crude material was purified by combi-flash column chromatography by eluting with 22% ethyl acetate in heptane to afford 6-(4-(prop-2-yn-1-yl) piperazin-1-yl) nicotinamide **(Intermediate 46,** 0.60 g, 20%) as an off white solid. $^1$H NMR (400 MHz, DMSO-$d_6$) δ ppm 2.42-2.49 (m, 2H), 2.52-2.57 (m, 2H), 3.16 (s, 1H), 3.55-3.67 (m, 6H), 6.84 (d, *J*=9.0 Hz, 1H), 7.12 (br s, 1H), 7.75 (br s, 1H), 7.95 (d, *J*=9.0 Hz, 1H), 8.60 (s, 1H). Mass spec: m/z: 245.13 $[M+H]^+$.

**Step 2**

**Intermediate 47: 6-(4-(3-(2-(2,6-dioxo-1-((2-(trimethylsilyl)ethoxy)methyl)piperidin-3-yl)-1-oxoisoindolin-4-yl) prop-2-yn-1-yl) piperazin-1-yl)nicotinamide**

To a solution of 6-(4-(prop-2-yn-1-yl)piperazin-1-yl)nicotinamide (**Intermediate 46,** 0.60 g, 2 mmol) in dimethylformamide (10 mL) was added 3-(4-iodo-1-oxoisoindolin-2-yl)-1-((2-(trimethylsilyl)ethoxy)methyl)piperidine-2,6-dione **(Intermediate 8,** 1.00 g, 2 mmol) followed by triethylamine (10 mL, 90 mmol). The reaction mixture was purged with argon for 15 min and then copper(I) iodide (0.05 g, 0.2 mmol) and $PdCl_2(PPh_3)_2$ (0.20 g, 0.2 mmol) were added. The reaction mixture was further purged with argon for 10 min and stirred at room temperature for 3h. The reaction mixture was diluted with water (100 mL) and extracted with ethyl acetate (2 × 100 mL). The combined organic phases were dried over anhydrous $Na_2SO_4$, and concentrated *in vacuo.* The crude material was purified by combi-flash chromatography by eluting with 100% ethyl acetate to afford 6-(4-(3-(2-(2,6-dioxo-1-((2-(trimethylsilyl)ethoxy)methyl)piperidin-3-yl)-1-oxoisoindolin-4-yl) prop-2-yn-1-yl)piperazin-1-yl)nicotinamide **(Intermediate 47,** 0.84 g, 60%) as an yellow solid. $^1$H NMR (401 MHz, DMSO-$d_6$) δ ppm -0.02 (s, 9H), 0.74-0.89 (m, 2H), 2.01-2.10 (m, 1H), 2.38-2.47 (m, 1H), 2.58-2.68 (m, 4H), 2.73-2.83 (m, 1H), 3.00-3.12 (m, 1H), 3.49-3.57 (m, 2H), 3.62-3.68 (m, 6H), 4.28-4.32 (m, 1H), 4.47-4.51 (m, 1H), 5.01-5.08 (m, 2H), 5.23-5.28 (m, 1H), 6.85 (d, *J*=9.05 Hz, 1H), 7.16 (br s, 1H), 7.53-7.58 (m, 1H), 7.70-7.72 (m, 1H), 7.74-7.78 (m, 2H), 7.96 (dd, *J*=9.05, 2.32 Hz, 1H), 8.61 (d, J=2.32 Hz, 1H). Mass spec: m/z: 617.42 $[M+H]^+$.

**Step 3**

**Intermediate 48: *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperazin-1-yl)nicotinamido)-2-((*R*)-1-methylpyrrolidin-2-yl)-1*H*-pyrrolo[3,2-*c*]pyridine-1-carboxylate**

To a solution of 6-(4-(3-(2-(2, 6-dioxo-1-((2-(trimethylsilyl)ethoxy)methyl)piperidin-3-yl)-1-oxoisoindolin-4-yl) prop-2-yn-1-yl) piperazin-1-yl)nicotinamide **(Intermediate 47**, 0.40 g, 0.6 mmol) in 1,4-dioxane (10 mL) was added *tert*-butyl (*R*)-6-bromo-2-(1-methylpyrrolidin-2-yl)-1*H*-pyrrolo[3,2-c]pyridine-1-carboxylate **(Intermediate 4,** 0.20 g, 0.6 mmol) followed by cesium carbonate (0.4 g, 1 mmol) at room temperature. The reaction mixture was purged with argon for 15 min and then $Pd_2(dba)_3$ (0.06 g, 0.06 mmol) and Xantphos (0.1 g, 0.2 mmol) were added. The reaction mixture was further purged with argon for 10 min and stirred at 100°C for 2h. The reaction mixture was concentrated *in vacuo* to obtain the crude compound, which was purified by combi-flash chromatography by eluting with 90% ethyl acetate in heptane to afford *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperazin-1-yl)nicotinamido)-2-((*R*)-1-methylpyrrolidin-2-yl)-1*H*-pyrrolo[3,2-c]pyridine-1-carboxylate (**Intermediate 48,** 0.54 g, 90%) as a white solid. $^1$H NMR (401 MHz, DMSO-$d_6$) δ ppm -0.03 (s, 9H), 0.80-0.86 (m, 2H), 1.38-1.42 (m, 1H), 1.53-1.65 (m, 2H), 1.69 (s, 9H), 1.73-1.80 (m, 2H), 2.02-2.10 (m, 1H), 2.36 (s, 3H), 2.38-2.41 (m, 2H), 2.62-2.69 (m, 4H), 2.74-2.83 (m, 1H), 3.08-3.18 (m, 1H), 3.48-3.56 (m, 2H), 3.65-3.73 (m, 6H), 3.86-3.95 (m, 1H), 4.29-4.37 (m, 1H), 4.44-4.51 (m, 1H), 5.10-5.15 (m, 2H), 5.22-5.29 (m, 1H), 6.74 (s, 1H), 6.90 (d, *J*=9.20 Hz, 1H), 7.54-7.58 (m, 1H), 7.70-7.73 (m, 1H), 7.74-7.78 (m, 1H), 8.16 (dd, *J*=9.20, 2.45 Hz, 1H), 8.58 (s, 1H), 8.81 (d, *J*=2.45 Hz, 1H), 8.92 (s, 1H), 10.54 (s, 1H). Mass spec: m/z: 916.5 $[M+H]^+$.

**Step 4**

**Example 6: 6-(4-(3-(2-(2,6-dioxopiperidin-3-yl)-1-oxoisoindolin-4-yl)prop-2-yn-1-yl)piperazin-1-yl)-*N*-2-((*R*)-1-methylpyrrolidin-2-yl)-1*H*-pyrrolo[3,2-*c*]pyridin-6-yl)nicotinamide**

To a solution of *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperazin-1-yl)nicotinamido)-2-((*R*)-1-methylpyrrolidin-2-yl)-1*H*-pyrrolo[3,2-c]pyridine-1-carboxylate (**Intermediate 48,** 0.40 g, 0.4 mmol) in acetonitrile (10 mL) was added methanesulfonic acid (0.4 mL, 7 mmol) and the reaction mixture was stirred at 50°C for 2h. *N*,*N'*-dimethylethylenediamine (0.3 mL, 3 mmol) followed by triethylamine (0.9 g, 9 mmol) were then added and the reaction mixture stirred at room temperature for 3h. The reaction mixture was

poured into ice cold water (100 mL), resulting in a precipitate, which was collected by filtration and dried under vacuum to obtain crude compound. The crude compound was purified by preparative HPLC (Method A) to afford 6-(4-(3-(2-(2,6-dioxopiperidin-3-yl)-1-oxoisoindolin-4-yl)prop-2-yn-1-yl)piperazin-1-yl)-*N*-(2-((*R*)-1-methylpyrrolidin-2-yl)-1*H*-pyrrolo [3,2-*c*]pyridin-6-yl)nicotinamide **(Example 6**, 0.19 g, 60%) as an off white solid. $^1$H NMR (400 MHz, DMSO-$d_6$) δ ppm 1.76-1.96 (m, 3H), 1.96-2.04 (m, 1H), 2.17 (s, 3H), 2.21-2.31 (m, 2H), 2.41-2.46 (m, 1H), 2.55-2.68 (m, 6H), 2.84-2.95 (m, 1H), 3.12-3.16 (m, 1H), 3.65-3.73 (m, 6H), 4.29-4.37 (m, 1H), 4.44-4.51 (m, 1H), 5.10-5.15 (m, 1H), 6.38 (s, 1H), 6.88 (d, *J*=9.20 Hz, 1H), 7.51-7.57 (m, 1H), 7.68-7.72 (m, 1H), 7.72-7.76 (m, 1H), 8.13-8.19 (m, 2H), 8.48 (s, 1H), 8.79-8.81 (m, 1H), 10.29 (br s, 1H), 11.00 (br s, 1H), 11.34 (br s, 1H). Mass spec: m/z: 686.0 [M+H]$^+$.

**Example 7 was synthesised following Scheme 10**

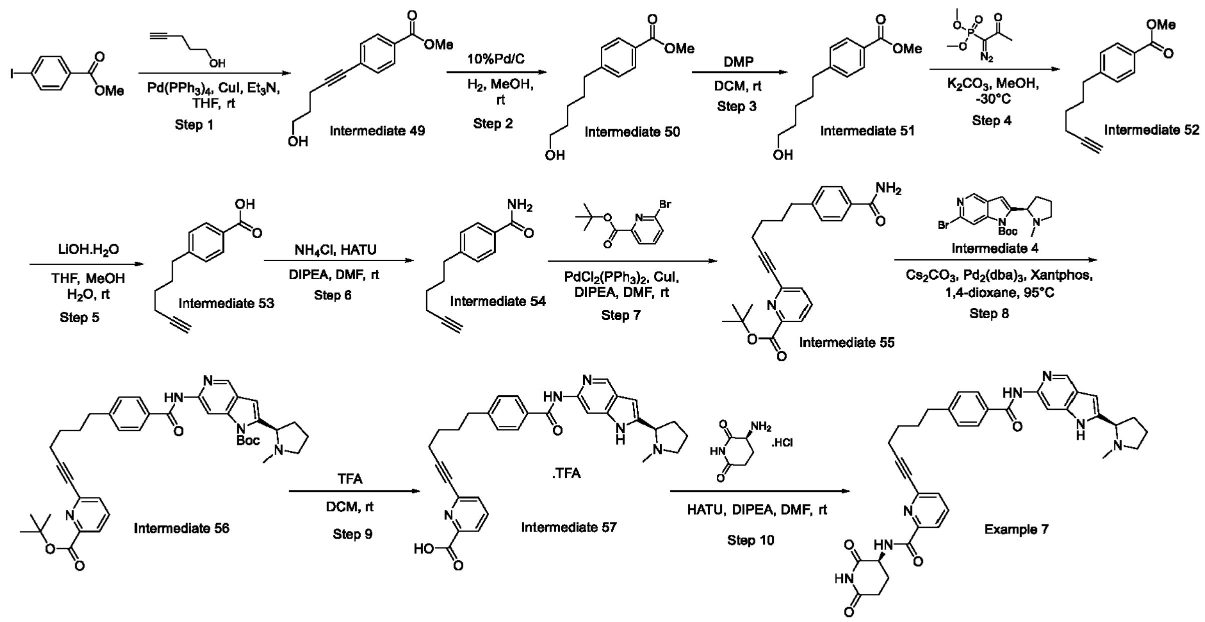


## Scheme 10

**Step 1**

**Intermediate 49: methyl 4-(5-hydroxypent-1-yn-1-yl)benzoate**

To a solution of methyl 4-iodobenzoate (CAS No: 619-44-3, 25.0 g, 95.4 mmol) in tetrahydrofuran (200 mL) was added pent-4-yn-1-ol (CAS No: 5390-04-5, 12.0 g, 143 mmol) followed by triethylamine (200 mL, 1430 mmol) and the reaction mixture was purged with argon gas for 15 min. $Pd(PPh_3)_4$ (5.51 g, 4.77 mmol) and copper(I) iodide (1.85 g, 9.54 mmol) were added and the reaction mixture was further purged with argon gas for 10 min and then stirred at room temperature for 16h. The reaction mixture was diluted with water (400 mL) and extracted with ethyl acetate (3 × 500 mL). The combined organic phases were dried over anhydrous $Na_2SO_4$, filtered, and concentrated *in vacuo.* The crude material was purified by combi-flash column chromatography by eluting with 80% ethyl acetate in heptane to afford methyl 4-(5-hydroxypent-1-yn-1-yl)benzoate (**Intermediate 49,** 16.0 g, 77%) as a yellow solid. $^1$H NMR (400 MHz, DMSO-$d_6$) δ ppm 1.67-1.73 (m, 2H), 2.50 (t, *J*=6.40 Hz, 2H), 3.50-3.55 (m, 2H), 3.84 (s, 3H), 4.55 (t, *J*=5.20 Hz, 1H), 7.49 (d, *J*=8.30 Hz, 2H), 7.89 (d, *J*=8.40 Hz, 2H). Mass spec: m/z: 219.4 [M+H]$^+$.

**Step 2**

**Intermediate 50: methyl 4-(5-hydroxypentyl)benzoate**

To a solution of methyl 4-(5-hydroxypent-1-yn-1-yl) benzoate (**Intermediate 49,** 16.0 g, 73.3 mmol) in methanol (200 mL) was added 10% Pd/C (5.86 g, 55.0 mmol) and the reaction mixture was stirred at room temperature for 16h under $H_2$ (110 psi). The reaction mixture was filtered through celite, washed with methanol (500 mL) and the filtrate was

concentrated *in vacuo* to afford methyl 4-(5-hydroxypentyl)benzoate **(Intermediate 50,** 14.0 g, 86%) as a yellow solid, which was used for the next step without further purification. $^1$H NMR (400 MHz, DMSO-$d_6$) δ ppm 1.24-1.35 (m, 2H), 1.39-1.48 (m, 2H), 1.54-1.62 (m, 2H), 2.64 (t, *J*=7.60 Hz, 2H), 3.34-3.41 (m, 2H), 3.83 (s, 3H), 4.33 (t, *J*=5.20 Hz, 1H), 7.33 (d, *J*=8.40 Hz, 2H), 7.86 (d, *J*=8.40 Hz, 2H). Mass spec: m/z: 223.16 $[M+H]^+$.

**Step 3**

**Intermediate 51: methyl 4-(5-oxopentyl)benzoate**

To a solution of methyl 4-(5-hydroxypentyl)benzoate **(Intermediate 50,** 14.0 g, 62.98 mmol) in dichloromethane (200 mL) was added Dess-Martin periodinane (40.07 g, 94.47 mmol) at 0°C. The reaction mixture was then stirred at room temperature for 2h. The reaction mixture was filtered through celite, filtrates were quenched with saturated $NaHCO_3$ solution (200 mL) and extracted with ethyl acetate (3 × 200 mL). The combined organic phases were dried over anhydrous $Na_2SO_4$, filtered and concentrated *in vacuo.* The crude material was purified by combi-flash chromatography by eluting with 30% ethyl acetate in heptane to afford methyl 4-(5-oxopentyl)benzoate **(Intermediate 51, 8.00** g, 58%) as yellow solid. $^1$H NMR (400 MHz, DMSO-$d_6$) δ ppm 1.47-1.64 (m, 4H), 2.21-2.25 (m, 1H), 2.43-2.48 (m, 1H), 2.61-2.69 (m, 2H), 3.83 (s, 3H), 7.34 (d, *J*=8.20 Hz, 2H), 7.87 (d, *J*=8.20 Hz, 2H), 9.65 (s, 1H).

**Step 4**

**Intermediate 52: methyl 4-(hex-5-yn-1-yl)benzoate**

To a solution of methyl 4-(5-oxopentyl)benzoate **(Intermediate 51,** 8.00 g, 36.3 mmol) in methanol (200 mL) was added potassium carbonate (6.01 g, 43.6 mmol) at 0°C. Dimethyl (1-diazo-2-oxopropyl)phosphonate (9.08 mL, 54.5 mmol) was added dropwise to the reaction mixture, at 0°C, over 20 min and the reaction mixture was stirred at -30°C for 3h. The reaction mixture was diluted with water (50 mL) and extracted with ethyl acetate (3 × 50 mL). The combined organic phases were dried over anhydrous $Na_2SO_4$ and concentrated *in vacuo* to obtain crude compound. The crude material was purified by combi-flash chromatography by eluting with 10% ethyl acetate in heptane to afford methyl 4-(hex-5-yn-1-yl) benzoate (**Intermediate 52,** 4.00 g, 51%) as yellow liquid. $^1$H NMR (400 MHz, DMSO-$d_6$) δ ppm 1.39-1.51 (m, 2H), 1.61-1.72 (m, 2H), 2.15-2.20 (m, 2H), 2.61-2.77 (m, 3H),

**Step 5**

**Intermediate 53: 4-(hex-5-yn-1-yl)benzoic acid**

To a solution of methyl 4-(hex-5-yn-1-yl)benzoate (**Intermediate 52,** 4.00 g, 18.5 mmol) in tetrahydrofuran (50 mL), methanol (100 mL) and water (50 mL) was added lithium hydroxide (0.90 g, 37 mmol) and the reaction mixture was stirred at room temperature for 5h. The reaction mixture was concentrated *in vacuo* and diluted with water (50 mL) and the aqueous phase was extracted with ethyl acetate (1 × 100 mL). The aqueous layer was then acidified up to pH~4 with 1N HCl and extracted with ethyl acetate (3 × 300 mL). The combined organic phases were dried over $Na_2SO_4$, and concentrated *in vacuo* to afford 4-(hex-5-yn-1-yl) benzoic acid (**Intermediate 53, 3.3** g) as a white solid, which was used for the next step without further purification. $^1$H NMR (400 MHz, DMSO-$d_6$) δ ppm 1.42-1.50 (m, 2H), 1.63-1.72 (m, 2H), 2.12-2.23 (m, 2H), 2.65 (t, *J*=7.60 Hz, 2H), 2.74 (s, 1H), 7.31 (d, *J*=8.0 Hz, 2H), 7.85 (d, *J*=8.0 Hz, 2H), 12.77 (br s, 1H). Mass spec: m/z: 201.5 $[M-H]^-$.

**Step 6**

**Intermediate 54: 4-(hex-5-yn-1-yl)benzamide**

To a solution of 4-(hex-5-yn-1-yl)benzoic acid (**Intermediate 53, 3.3** g, 16 mmol) in dimethylformamide (60 mL) was added HATU (9.6 g, 24 mmol) and *N, N*-diisopropylethylamine (8.5 mL, 49 mmol) and the reaction was stirred at room temperature for 10 min. Ammonium chloride (4.4 g, 82 mmol) was added at 0°C and the reaction mixture was stirred at room temperature for 16h. The reaction mixture was diluted with ice cold water (200 mL), resulting in a precipitate which was filtered, washed with water (100 mL) and dried under vacuum to afford 4-(hex-5-yn-1-yl)benzamide (**Intermediate 54,** 2.70 g, 82%) as a white solid, which was used for next step without further purification. $^1$H NMR (400 MHz, DMSO-$d_6$) δ ppm 1.41-1.50 (m, 2H), 1.61-1.70 (m, 2H), 2.13-2.22 (m, 2H), 2.63 (d, *J*=7.60 Hz, 2H), 2.74 (s, 1H), 7.24 (br s, 1H), 7.26 (d, *J*=7.60 Hz, 2H), 7.78 (d, *J*=7.60 Hz, 2H), 7.88 (br s, 1H). Mass spec: m/z: 202.5 $[M+H]^+$.

**Step 7**

**Intermediate 55: *tert-butyl* 6-(6-(4-carbamoylphenyl) hex-1-yn-1-yl)picolinate**

To a solution of 4-(hex-5-yn-1-yl)benzamide (**Intermediate 54, 0.25** g, 1.24 mmol) in dimethylformamide (3 mL) was added *tert*-butyl 6-bromopicolinate (CAS No: 910044-07-4, 0.42 g, 1.61 mmol) followed by *N*, *N*-diisopropylethylamine (0.7 mL, 3.73 mmol). The reaction mixture was purged with argon for 15 min followed by the addition of copper(I) iodide (0.025 g, 0.12 mmol) and $PdCl_2(PPh_3)_2$ (0.14 g, 0.18 mmol). The reaction mixture was further purged with argon for 10 min and then stirred at room temperature for 1h. The reaction mixture was quenched with ice cold water (50 mL) and extracted with ethyl acetate (2 $\times$ 100 mL). The combined organic phases were dried over anhydrous $Na_2SO_4$ and concentrated *in vacuo.* The crude material was purified by combi-flash chromatography by eluting with 70% ethyl acetate in heptane to afford *tert*-butyl 6-(6-(4-carbamoylphenyl)hex-1-yn-1-yl)picolinate (**Intermediate 55,** 0.2 g, 42%) as a yellow solid. $^1$H NMR (400 MHz, DMSO-$d_6$) $\delta$ ppm 1.53 (s, 9H), 1.68-1.77 (m, 2H), 2.64-2.73 (m, 4H), 2.84-2.88 (m, 2H), 7.24 (br s, 1H), 7.26-7.28 (m, 2H), 7.61 (d, *J*=7.60 Hz, 1H), 7.77 (d, *J*=7.60 Hz, 2H), 7.86 (br s, 1H), 7.88-7.93 (m, 2H). Mass spec: m/z: 379.3 $[M+H]^+$.

**Step 8**

**Intermediate 56: *tert*-butyl (*R*)-6-(4-(6-(6-(*tert*-butoxycarbonyl) pyridin-2-yl)hex-5-yn-1-yl)benzamido)-2-(1-methylpyrrolidin-2-yl)-1*H*-pyrrolo[3, 2-c]pyridine-1-carboxylate**

To a solution of *tert*-butyl 6-(6-(4-carbamoylphenyl)hex-1-yn-1-yl) picolinate **(Intermediate 55,** 0.16 g, 0.42 mmol) in 1,4-dioxane (3 mL) was added *tert*-butyl (*R*)-6-bromo-2-(1-methylpyrrolidin-2-yl)-1*H*-pyrrolo[3,2-c]pyridine-1-carboxylate **(Intermediate 4,** 0.2 g, 0.52 mmol) followed by cesium carbonate (0.54 g, 1.57 mmol) at room temperature. The reaction mixture was purged with argon for 15 min followed by the addition of $Pd_2(dba)_3$ (0.067 g, 0.078 mmol) and Xantphos (0.096 g, 0.16 mmol). The reaction mixture was further purged with argon for 10 min and then heated at 95°C for 5h. The reaction mixture was concentrated *in vacuo* to obtain crude compound, which was purified by combi-flash chromatography by eluting with 7% MeOH in DCM to afford tert-butyl (*R*)-6-(4-(6-(6-(*tert*-butoxycarbonyl)pyridin-2-yl) hex-5-yn-1-yl)benzamido)-2-(1-methylpyrrolidin-2-yl)-1*H*-pyrrolo[3,2-c]pyridine-1-carboxylate **(Intermediate 56,** 0.2 g, 34%) as a yellow solid. $^1$H NMR (400 MHz, DMSO-$d_6$) $\delta$ ppm 1.55 (s, 9H), 1.57-1.64 (m, 4H), 1.69 (s, 9H), 1.74-1.80 (m, 4H), 2.27-2.33 (m, 1H), 2.35 (s, 3H), 2.38-2.40 (m, 1H), 2.65-2.77 (m, 3H), 3.12-3.16 (m, 1H), 3.87-3.97 (m, 1H), 6.74 (s, 1H), 7.34-7.36 (m, 1H), 7.59-7.67 (m, 1H), 7.77-7.80 (m, 1H), 7.88-8.01 (m, 4H), 8.58 (s, 1H), 8.92 (s, 1H), 10.59 (br s, 1H). Mass spec: m/z: 678.4 $[M+H]^+$.

**Step 9**

**Intermediate 57: (*R*)-6-(6-(4-((2-(1-methylpyrrolidin-2-yl)-1*H*-pyrrolo[3,2-c]pyridin-6-yl)carbamoyl) phenyl) hex-1-yn-1-yl)picolinic acid trifluoroacetate**

To a solution of *tert*-butyl (*R*)-6-(4-(6-(6-(*tert*-butoxycarbonyl)pyridin-2-yl)hex-5-yn-1-yl)benzamido)-2-(1-methylpyrrolidin-2-yl)-1*H*-pyrrolo[3,2-c]pyridine-1-carboxylate **(Intermediate 56,** 0.2 g, 0.29 mmol) in dichloromethane (2 mL) was added trifluoroacetic acid (0.57 mL, 7.37 mmol) at 0°C. The reaction mixture was then stirred at room temperature for 16h. The reaction mixture was concentrated *in vacuo* to obtain crude compound, which was washed with diethyl ether (5 $\times$ 2 mL) and dried *in vacuo* to afford *(R)*-6-(6-(4-((2-(1-methylpyrrolidin-2-yl)-1*H*-pyrrolo[3,2-c]pyridin-6-yl) carbamoyl)phenyl)hex-1-yn-1-yl)picolinic acid trifluoroacetate **(Intermediate 57,** 0.2 g, 65%) as a red gum, which was used in the next step without further purification. Mass spec: m/z: 522.1 $[M+H]^+$.

**Step 10**

**Example 7: *N*-((S)-2,6-dioxopiperidin-3-yl)-6-(6-(4-((2-((*R*)-1-methylpyrrolidin-2-yl)-1*H*-pyrrolo[3,2-c]pyridin-6-yl)carbamoyl)phenyl)hex-1-yn-1-yl)picolinamide**

To a solution of (*R*)-6-(6-(4-((2-(1-methylpyrrolidin-2-yl)-1*H*-pyrrolo [3, 2-c] pyridin-6-yl) carbamoyl) phenyl) hex-1-yn-1-yl) picolinic acid trifluoroacetate **(Intermediate 57,** 0.19 g, 0.29 mmol) in dimethylformamide (3 mL) was added HATU (0.23 g, 0.59 mmol) and *N*, *N*-diisopropylethylamine (0.2 mL, 0.89 mmol) at 0°C and the reaction stirred for 10 min. (S)-3-aminopiperidine-2,6-dione hydrochloride (CAS No: 25181-50-4, 0.074 g, 0.45 mmol) was added at 0°C and the reaction mixture was stirred at room temperature for 3h. The reaction mixture was poured into ice cold water (50 mL), resulting in a precipitate which was filtered and dried under vacuum to obtain the crude compound. The crude material was

purified by preparative HPLC (Method A) to afford *N*-((S)-2,6-dioxopiperidin-3-yl)-6-(6-(4-((2-((*R*)-1-methylpyrrolidin-2-yl)-1*H*-pyrrolo[3,2-c]pyridin-6-yl)carbamoyl)phenyl)hex-1-yn-1-yl)picolinamide **(Example 7,** 0.025 g, 13%d) as an off white solid. $^1$H NMR (400 MHz, DMSO-$d_6$) δ ppm 1.58-1.66 (m, 2H), 1.75-2.03 (m, 6H), 2.11-2.15 (m, 1H), 2.17 (s, 3H), 2.21-2.30 (m, 2H), 2.53-2.57 (m, 3H), 2.69-2.85 (m, 3H), 3.08-3.19 (m, 1H), 3.28-3.30 (m, 1H), 4.75-4.85 (m, 1H), 6.39 (s, 1H), 7.34-7.36 (m, 2H), 7.66-7.70 (m, 1H), 7.95-8.01 (m, 4H), 8.19 (s, 1H), 8.50 (s, 1H), 8.94-8.98 (m, 1H), 10.33 (br s, 1H), 10.89 (br s, 1H), 11.35 (br s, 1H). Mass spec: m/z: 632.1 [M+H]$^+$.

**Example 8 was synthesised following Scheme 11**

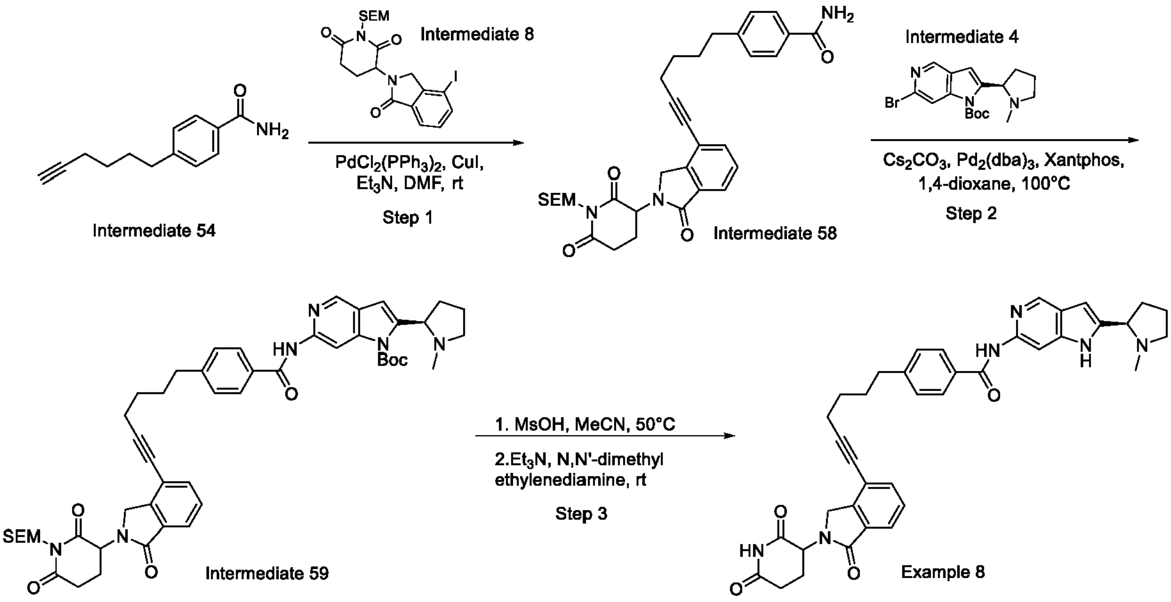


## Scheme 11

**Step** 1

**Intermediate 58: 4-(6-(2-(2,6-dioxo-1-((2-(trimethylsilyl) ethoxy) methyl) piperidin-3-yl)-1-oxoisoindolin-4-yl) hex-5-yn-1-yl)benzamide**

To a solution of 4-(hex-5-yn-1-yl)benzamide **(Intermediate 54**, 0.60 g, 2.98 mmol) in dimethylformamide (2 mL) was added 3-(4-iodo-1-oxoisoindolin-2-yl)-1-((2-(trimethylsilyl)ethoxy)methyl)piperidine-2,6-dione **(Intermediate 8,** 1.49 g, 2.98 mmol) followed by triethylamine (14.6 mL, 104.3 mmol). The reaction mixture was purged with argon for 15 min, then copper (I) iodide (0.057 g, 0.29 mmol) and $PdCl_2(PPh_3)_2$ (0.22 g, 0.29 mmol) were added. The reaction mixture was further purged with argon for 10 min and stirred at room temperature for 2h. The reaction mixture was then diluted with water (100 mL) and extracted with ethyl acetate (3 × 100 mL). The combined organic layers were dried over anhydrous $Na_2SO_4$, and concentrated *in vacuo.* The crude material was purified by combi-flash chromatography, by eluting with 90% ethyl acetate in heptane, to afford 4-(6-(2-(2,6-dioxo-1-((2-(trimethylsilyl)ethoxy)methyl)piperidin-3-yl)-1-oxoisoindolin-4-yl)hex-5-yn-1-yl)benzamide (**Intermediate 58,** 1.00 g, 58%) as an off- white solid. $^1$H NMR (400 MHz, DMSO-$d_6$) δ ppm -0.06 (s, 9H), 0.76-0.87 (m, 2H), 1.52-1.62 (m, 2H), 1.68-1.78 (m, 2H), 1.99-2.08 (m, 1H), 2.33-2.38 (m, 1H), 2.42-2.46 (m, 2H), 2.64-2.68 (m, 2H), 2.75-2.80 (m, 1H), 2.99-3.10 (m, 1H), 3.46-3.54 (m, 2H), 4.20-4.26 (m, 1H), 4.40-4.44 (m, 1H), 4.98-5.08 (m, 2H), 5.21-5.26 (m, 1H), 7.22 (br s, 1H), 7.25 (d, *J*=8.0 Hz, 2H), 7.50 (t, *J*=7.60 Hz, 1H), 7.62 (d, *J*=7.60 Hz, 1H), 7.69 (d, *J*=7.60 Hz, 1H), 7.76 (d, *J*=8.0 Hz, 2H), 7.84 (br s, 1H). Mass spec m/z 572.52 [M+H]$^+$.

**Step 2**

**Intermediate 59: *tert*-butyl-6-(4-(6-(2-(2,6-dioxo-1-((2-(trimethylsilyl)ethoxy)methyl)piperidin-3-yl)-1-oxoisoindolin-4-yl)hex-5-yn-1-yl)benzamido)-2-((*R*)-1-methylpyrrolidin-2-yl)-1*H*-pyrrolo[3,2-c]pyridine-1-carboxylate**

To a solution of 4-(6-(2-(2,6-dioxo-1-((2-(trimethylsilyl)ethoxy)methyl)piperidin-3-yl)-1-oxoisoindolin-4-yl)hex-5-yn-1-yl) benzamide (**Intermediate 58, 0.40** g, 0.69 mmol) in 1,4-dioxane (10 mL) was added *tert*-butyl (*R*)-6-bromo-2-(1-methylpyrrolidin-2-yl)-1*H*-pyrrolo[3,2-c]pyridine-1-carboxylate (**Intermediate 4, 0.26** g, 0.69 mmol) followed by cesium carbonate (0.46 g, 1.39 mmol). The reaction mixture was purged with argon for 15 min, then $Pd_2(dba)_3$ (0.098 g, 0.10 mmol) and Xantphos (0.12 g, 0.21 mmol) were added. The reaction mixture was further purged with argon for 10 min and then heated at 100°C for 2h. The reaction mixture was concentrated *in vacuo* and the crude material was purified by combi-flash chromatography, by eluting with 90% ethyl acetate in heptane, to afford *tert*-butyl-6-(4-(6-(2-(2,6-dioxo-1-((2-(trimethylsilyl)ethoxy)methyl)piperidin-3-yl)-1-oxoisoindolin-4-yl)hex-5-yn-1-yl)benzamido)-2-((*R*)-1-methylpyrrolidin-2-yl)-1*H*-pyrrolo[3,2-c]pyridine-1-carboxylate (**Intermediate 59,** 0.45 g, 74%) as a white solid. $^1$H NMR (400 MHz, DMSO-$d_6$) δ ppm -0.05 (s, 9H), 0.76-0.86 (m, 2H), 1.58-1.66 (m, 4H), 1.69 (s, 9H), 1.73-1.82 (m, 4H), 2.02-2.10 (m, 1H), 2.28-2.33 (m, 2H), 2.35 (s, 3H), 2.37-2.42 (m, 1H), 2.66-2.78 (m, 4H), 3.01-3.17 (m, 2H), 3.47-3.54 (m, 2H), 3.88-3.94 (m, 1H), 4.24-4.28 (m, 1H), 4.43-4.50 (m, 1H), 4.98-5.08 (m, 2H), 5.24-5.30 (m, 1H), 6.75 (s, 1H), 7.34 (d, *J*=8.0 Hz, 2H), 7.53 (t, *J*=7.20 Hz, 1H), 7.65 (d, *J*=8.0 Hz, 1H), 7.72 (d, *J*=7.20 Hz, 1H), 7.97 (d, *J*=7.20 Hz, 2H), 8.58 (s, 1H), 8.93 (s, 1H), 10.60 (s, 1H). Mass spec m/z 873.57 $[M+H]^+$.

**Step 3**

**Example 8: 4-(6-(2-(2, 6-dioxopiperidin-3-yl)-1-oxoisoindolin-4-yl)hex-5-yn-1-yl)-*N*-(2-((*R*)-1-methylpyrrolidin-2-yl)-1*H*-pyrrolo[3,2-c]pyridin-6-yl)benzamide**

To a solution of *tert*-butyl-6-(4-(6-(2-(2,6-dioxo-1-((2-(trimethylsilyl)ethoxy)methyl)piperidin-3-yl)-1-oxoisoindolin-4-yl)hex-5-yn-1-yl)benzamido)-2-((*R*)-1-methylpyrrolidin-2-yl)-1*H*-pyrrolo[3,2-c]pyridine-1-carboxylate (**Intermediate 59,** 0.40 g, 0.54 mmol) in acetonitrile (5 mL) was added methanesulfonic acid (0.53 mL, 8.07 mmol) at room temperature and the reaction mixture was heated at 50°C for 2h. After cooling to room temperature, *N, N'*-dimethylethylenediamine (0.39 mL, 3.23 mmol) followed by triethylamine (1.10 g, 10.77 mmol were added the reaction mixture was stirred at room temperature for 3h. The reaction mixture was diluted with water (100 mL), the resultant precipitate was collected by filtration and dried *in vacuo.* The crude material was purified by preparative HPLC (Method A) to afford 4-(6-(2-(2,6-dioxopiperidin-3-yl)-1-oxoisoindolin-4-yl)hex-5-yn-1-yl)-*N*-(2-((*R*)-1-methylpyrrolidin-2-yl)-1*H*-pyrrolo[3,2-c]pyridin-6-yl)benzamide **(Example 8,** 0.15 g, 44%) as an off white solid. $^1$H NMR (400 MHz, DMSO-$d_6$) δ 1.58-1.66 (m, 2H), 1.74-1.95 (m, 5H), 1.97-2.05 (m, 1H), 2.10-2.15 (m, 1H), 2.17 (s, 3H), 2.23-2.30 (m, 1H), 2.40-2.45 (m, 1H), 2.53-2.58 (m, 2H), 2.59-2.64 (m, 1H), 2.70-2.76 (m, 2H), 2.85-2.96 (m, 1H), 3.10-3.17 (m, 1H), 3.32-3.36 (m, 1H), 4.28-4.36 (m, 1H), 4.40-4.48 (m, 1H), 5.11-5.16 (m, 1H), 6.39 (s, 1H), 7.34 (d, *J*=8.0 Hz, 2H), 7.52 (t, *J*=8.0 Hz, 1H), 7.63 (d, *J*=7.60 Hz, 1H), 7.70 (d, *J*=7.60 Hz, 1H), 7.98 (d, *J*=8.0 Hz, 2H), 8.18 (s, 1H), 8.49 (s, 1H), 10.33 (s, 1H), 11.01 (br s, 1H), 11.35 (s, 1H). Mass spec m/z 643.5 $[M+H]^+$.

**Example 9 was synthesised following Scheme 12**

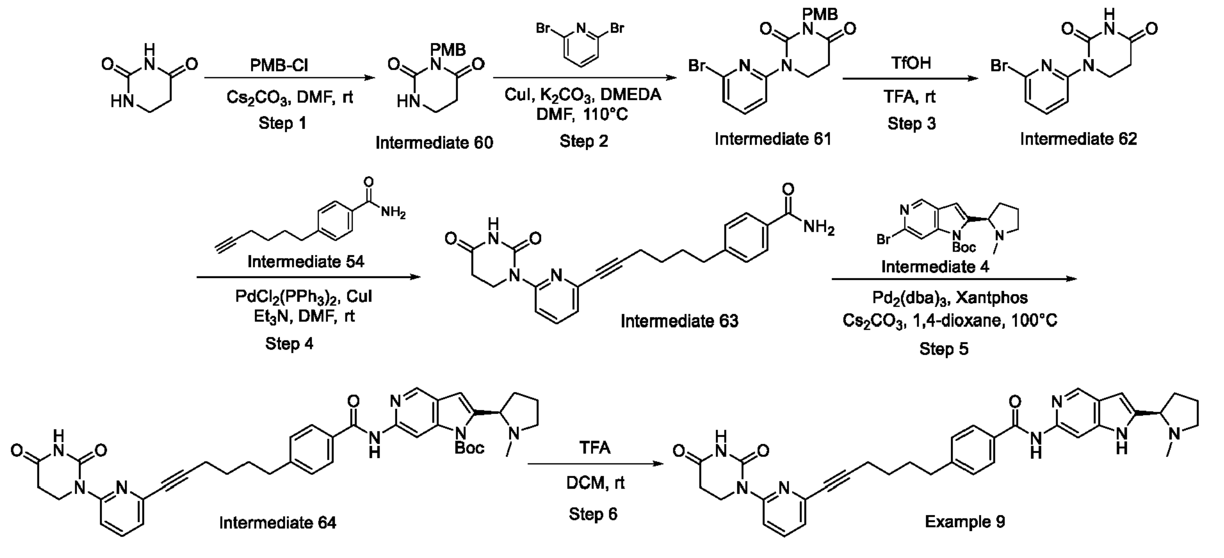


## Scheme 12

### Step 1

**Intermediate 60: 3-(4-methoxybenzyl) dihydropyrimidine-2,4(1*H*, 3*H*)-dione**

To a solution of dihydropyrimidine-2,4(1*H*,3*H*)-dione (CAS No: 504-07-4, 5.00 g, 43.82 mmol) in dimethylformamide (150 mL) was added cesium carbonate (28.56 g, 87.64 mmol) followed by 4-methoxybenzyl chloride (4.06 mL, 28.48 mmol) and the reaction mixture was stirred at room temperature for 16h. The reaction mixture was quenched with ice cold water (1500 mL) and stirred for 30 min, resulting in a precipitate, which was filtered and dried *in vacuo* to afford 3-(4-methoxybenzyl)dihydropyrimidine-2,4(1*H*,3*H*)-dione (**Intermediate 60,** 4.50 g, 44%) as an off white solid, which was used for the next step without further purification. $^1H$ NMR (400 MHz, DMSO-$d_6$) δ ppm 2.62 (t, *J*=6.80 Hz, 2H), 3.19-3.23 (m, 2H), 3.71 (s, 3H), 4.71 (s, 2H), 6.83-6.85 (m, 2H), 7.16-7.18 (m, 2H), 7.80 (br s, 1H).

### Step 2

**Intermediate 61: 1-(6-bromopyridin-2-yl)-3-(4-methoxybenzyl) dihydropyrimidine-2,4(1*H*, 3*H*)-dione**

To a solution of 2,6-dibromopyridine (CAS No: 626-05-1, 7.00 g, 29.5 mmol) in dimethylformamide (10 mL) was added 3-(4-methoxybenzyl)dihydropyrimidine-2,4(1*H*, *3*H)-dione (**Intermediate 60, 4.15** g, 17.7 mmol) followed by $K_2CO_3$ (12.3 g, 88.6 mmol). The reaction mixture was purged with argon gas for 15 min then copper(I) iodide (0.56 g, 2.95 mmol) and *N*, *N'*-dimethylethylenediamine (0.53 g, 5.91 mmol) were added and the reaction mixture was heated at 110°C for 16h. The reaction mixture was filtered and washed with ethyl acetate (100 mL). the organic phases was washed with ice cold water (100 mL), dried over $Na_2SO_4$, and concentrated *in vacuo.* The crude material was purified by combi-flash column chromatography by eluting with 20% ethyl acetate in heptane to afford 1-(6-bromopyridin-2-yl)-3-(4-methoxybenzyl)dihydropyrimidine-2,4(1*H*,3*H*)-dione (**Intermediate 61,** 3.4 g, 29%) as an off white solid. $^1H$ NMR (400 MHz, DMSO-$d_6$) δ ppm 2.88 (t, *J*=6.80 Hz, 2H), 3.72 (s, 3H), 4.02 (t, *J*=6.80 Hz, 2H), 4.83 (s, 2H), 6.84-6.86 (m, 2H), 7.22-7.25 (m, 2H), 7.42-7.47 (m, 1H), 7.76-.7.79 (m, 2H).

### Step 3

**Intermediate 62: 1-(6-bromopyridin-2-yl) dihydropyrimidine-2, 4(1*H*, 3*H*)-dione**

To a solution of 1-(6-bromopyridin-2-yl)-3-(4-methoxybenzyl)dihydropyrimidine-2, 4(1*H*, 3*H*)-dione (**Intermediate 61,** 4.00 g, 10.3 mmol) in trifluoroacetic acid (40 mL) was added trifluoromethanesulfonic acid (10.00 mL) and the reaction was stirred at room temperature for 16h. The reaction mixture was concentrated at a lower temperature. The obtained residue was quenched with saturated $NaHCO_3$, to pH-8, resulting in a precipitate which was collected by filtration, washed with saturated $NaHCO_3$ solution (50 mL) and water (50 mL) and dried *in vacuo* to afford 1-(6-bromopyridin-2-yl)

dihydropyrimidine-2,4(1*H*, 3H)-dione (**Intermediate 62,** 3.50 g) as a brown solid, which was used for the next step without further purification. $^1$H NMR (400 MHz, DMSO-$d_6$) δ ppm 2.66-2.70 (m, 2H), 4.01-4.04 (m, 2H), 7.40-7.44 (m, 1H), 7.72-7.79 (m, 1H), 7.80-7.86 (m, 1H), 10.63 (br s, 1H). Mass spec: m/z: 270.2 [M+H]$^+$.

**Step 4**

**Intermediate 63: 4-(6-(6-(2, 4-dioxotetrahydropyrimidin-1(2*H*)-yl) pyridin-2-yl)hex-5-yn-1-yl)benzamide**

To a solution of 4-(hex-5-yn-1-yl)benzamide (**Intermediate 54,** 0.65 g, 3.23 mmol) in dimethylformamide (15 mL) was added 1-(6-bromopyridin-2-yl)dihydropyrimidine-2,4(1*H*, 3*H*)-dione (**Intermediate 62,** 1.04 g, 3.87 mmol) followed by triethylamine (15.8 mL, 113.0 mmol). The reaction mixture was purged with argon for 15 min and then copper(I) iodide (0.064 g, 0.32 mmol) and $PdCl_2(PPh_3)_2$ (0.23 g, 0.32 mmol) were added. The reaction mixture was further purged with argon for 10 min and stirred at room temperature for 2h. The reaction mixture was quenched with ice cold water (80 mL) and extracted with ethyl acetate (2 × 150 mL). The combined organic phases were dried over anhydrous $Na_2SO_4$ and concentrated *in vacuo.* The crude material was purified by combi-flash chromatography by eluting with 100% ethyl acetate to afford 4-(6-(6-(2, 4-dioxotetrahydropyrimidin-1(2*H*)-yl) pyridin-2-yl) hex-5-yn-1-yl)benzamide **(Intermediate 63,** 0.80 g, 63%) as a yellow solid. $^1$H NMR (400 MHz, DMSO-$d_6$) δ ppm 1.51-1.60 (m, 2H), 1.70-1.80 (m, 2H), 2.44-2.47 (m, 2H), 2.63-2.71 (m, 4H), 3.98-4.04 (m, 2H), 7.21-7.29 (m, 4H), 7.68-7.80 (m, 4H), 7.88 (br s, 1H), 10.54 (s, 1H). Mass spec: m/z: 391.2 [M+H]$^+$.

**Step 5**

**Intermediate 64: tert-butyl (R)-6-(4-(6-(6-(2, 4-dioxotetrahydropyrimidin-1(2*H*)-yl)pyridin-2-yl)hex-5-yn-1-yl)benzamido)-2-(1-methylpyrrolidin-2-yl)-1*H*-pyrrolo[3, 2-c]pyridine-1-carboxylate**

To a solution of 4-(6-(6-(2,4-dioxotetrahydropyrimidin-1(2*H*)-yl) pyridin-2-yl) hex-5-yn-1-yl)benzamide (**Intermediate 63, 0.50** g, 1.28 mmol) in 1,4-dioxane (20 mL) was added tert-butyl (*R*)-6-bromo-2-(1-methylpyrrolidin-2-yl)-1*H*-pyrrolo[3,2-c]pyridine-1-carboxylate **(Intermediate 4,** 0.58 g, 1.54 mmol) followed by cesium carbonate (1.25 g, 3.84 mmol) at room temperature. The reaction mixture was purged with argon for 15 min and then $Pd_2(dba)_3$ (0.18 g, 0.19 mmol) and Xantphos (0.23 g, 0.38 mmol) were added. The reaction mixture was further purged with argon for 10 min and stirred at 100°C for 2h. The reaction mixture was filtered through celite, washed with ethyl acetate (100 mL) and filtrate was concentrated *in vacuo* to obtain crude compound. The crude material was purified by combi-flash column chromatography by eluting with 5% MeOH in DCM to afford *tert*-butyl (*R*)-6-(4-(6-(6-(2, 4-dioxotetrahydropyrimidin-1(2*H*)-yl)pyridin-2-yl) hex-5-yn-1-yl)benzamido)-2-(1-methylpyrrolidin-2-yl)-1*H*-pyrrolo[3, 2-c]pyridine-1-carboxylate (**Intermediate 64,** 0.50 g, 57%) as a yellow solid. $^1$H NMR (400 MHz, DMSO-$d_6$) δ ppm 1.55-1.66 (m, 4H), 1.69 (s, 9H), 1.72-1.81 (m, 4H), 2.29-2.32 (m, 1H), 2.35 (s, 3H), 2.37-2.42 (m, 2H), 2.64-2.77 (m, 4H), 3.07-3.18 (m, 1H), 3.88-3.95 (m, 1H), 3.98-4.06 (m, 2H), 6.75 (s, 1H), 7.22-7.26 (m, 1H), 7.35 (d, *J*= 8.20 Hz, 2H), 7.67-7.79 (m, 2H), 7.98 (d, *J*= 8.20 Hz, 2H), 8.59 (s, 1H), 8.92 (s, 1H), 10.55 (br s, 1H), 10.61 (br s, 1H). Mass spec: m/z: 690.0 [M+H]$^+$.

**Step 6**

**Example 9: (*R*)-4-(6-(6-(2,4-dioxotetrahydropyrimidin-1(2*H*)-yl)pyridin-2-yl)hex-5-yn-1-yl)-*N*(2-(1-methylpyrrolidin-2-yl)-1*H*-pyrrolo[3,2-c]pyridin-6-yl)benzamide**

To a solution of *tert*-butyl (*R*)-6-(4-(6-(6-(2,4-dioxotetrahydropyrimidin-1(2*H*)-yl) pyridin-2-yl)hex-5-yn-1-yl) benzamido)-2-(1-methylpyrrolidin-2-yl)-1*H*-pyrrolo [3, 2-c]pyridine-1-carboxylate (**Intermediate 64,** 0.40 g, 0.58 mmol) in dichloromethane (10 mL) was added trifluoroacetic acid (5 mL) at 0°C and the reaction mixture was stirred at room temperature for 1h. The volatiles were removed under reduced pressure and co-distilled with DCM to obtain the crude compound which was purified by preparative HPLC (Method A) to afford (*R*)-4-(6-(6-(2,4-dioxotetrahydropyrimidin-1(2*H*)-yl)pyridin-2-yl)hex-5-yn-1-yl)-*N*-(2-(1-methylpyrrolidin-2-yl)-1*H*-pyrrolo[3,2-c]pyridin-6-yl)benzamide **(Example 9,** 0.15 g, 44%) as a yellow solid. $^1$H NMR (400 MHz, DMSO-$d_6$) δ ppm 1.55-1.64 (m, 2H), 1.72-1.94 (m, 5H), 2.10-2.14 (m, 1H), 2.16 (s, 3H), 2.22-2.30 (m, 1H), 2.42-2.47 (m, 1H), 2.52-2.56 (m, 2H), 2.66-2.75 (m, 4H), 3.08-3.21 (m, 1H), 4.01-4.04 (m, 2H), 6.39 (s, 1H), 7.23-7.25 (m, 1H), 7.32-7.36 (m, 2H), 7.69-7.80 (m, 2H), 7.96-8.00 (m, 2H), 8.18 (s, 1H), 8.49 (s, 1H), 10.35 (br s, 1H), 10.55 (br s, 1H), 11.36 (br s, 1H). Mass spec: m/z: 590.2 [M+H]$^+$.

**Example 10 was synthesised following Scheme 13**

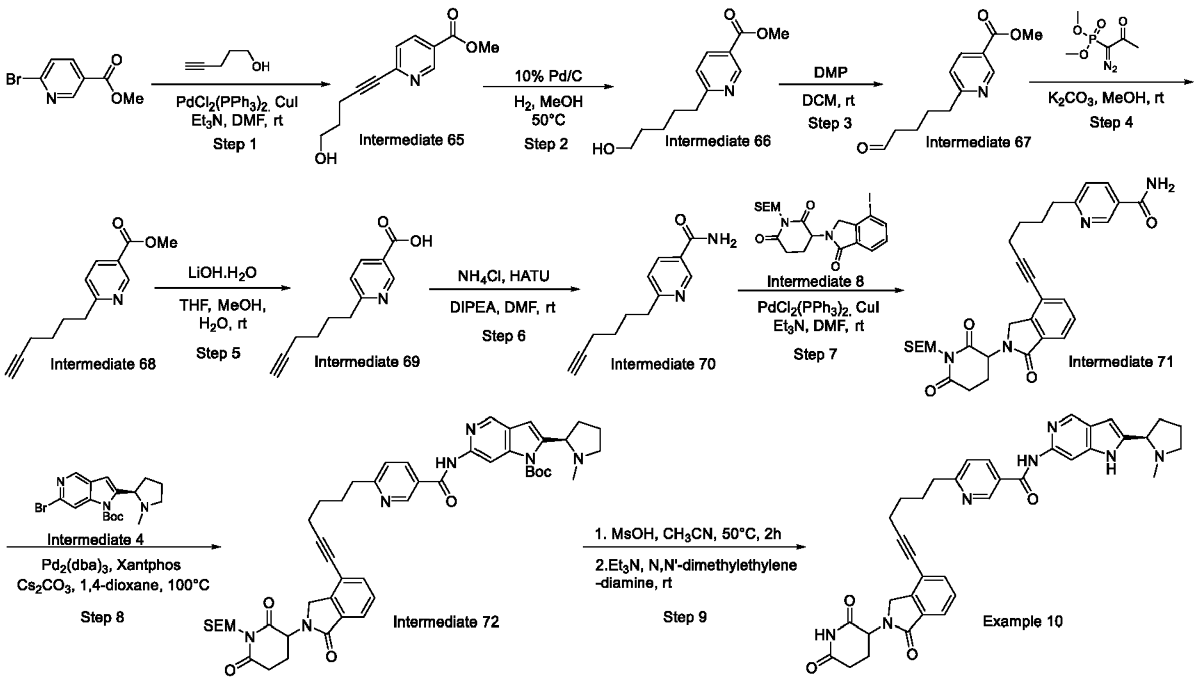


**Scheme 13**

**Step** 1

**Intermediate 65: methyl 6-(5-hydroxypent-1-yn-1-yl)nicotinate**

To a solution of methyl 6-bromonicotinate (CAS No: 26218-78-0 , 12.0 g, 54.4 mmol) in dimethylformamide (80 mL) was added pent-4-yn-1-ol (CAS No: 5390-04-5, 7.0 mL, 70.8 mmol) followed by triethylamine (229 mL, 1630 mmol). The reaction mixture was purged with argon gas for 15 min. $PdCl_2(PPh_3)_2$ (5.91 g, 8.17 mmol) and copper(I) iodide (1.64 g, 8.17 mmol) were then added and the reaction mixture was further purged with argon gas for 10 min and stirred at room temperature for 2h. The reaction mixture was diluted with cold water (200 mL) and extracted with ethyl acetate (3 × 100 mL). The combined organic phases were washed with brine solution (2 × 100 mL), dried over anhydrous $Na_2SO_4$, filtered, and concentrated *in vacuo.* The crude material was purified by combi-flash chromatography by eluting with 60% ethyl acetate in heptane to afford methyl 6-(5-hydroxypent-1-yn-1-yl) nicotinate (**Intermediate 65,** 10.0 g, 84%) as a liquid. $^1H$ NMR (400 MHz, DMSO-$d_6$) δ ppm 1.68-1.76 (m, 2H), 2.54 (t, *J*=7.20 Hz, 2H), 3.43 (t, *J*=6.0 Hz, 1H), 3.51-3.55 (m, 2H), 3.88 (s, 3H), 7.58 (d, *J*=8.0 Hz, 1H), 8.23 (d, *J*=8.0 Hz, 1H), 9.00 (s, 1H). Mass spec: m/z: 220.4 $[M+H]^+$.

**Step 2**

**Intermediate 66: methyl 6-(5-hydroxypentyl)nicotinate**

To a solution of methyl 6-(5-hydroxypent-1-yn-1-yl)nicotinate (**Intermediate 65,** 10.0 g, 45.61 mmol) in methanol (150 mL) was added 10% Pd/C (3.88 g, 36.49 mmol). The reaction mixture was heated at 50°C for 5h under an $H_2$ (140 psi). The reaction mixture was filtered through celite, washed with methanol (100 mL) and concentrated *in vacuo* to afford methyl 6-(5-hydroxypentyl)nicotinate (**Intermediate 66,** 8.00 g, 79%) as a yellow liquid, which was used for the next step without further purification. $^1H$ NMR (400 MHz, DMSO-$d_6$) δ ppm 1.25-1.33 (m, 2H), 1.38-1.46 (m, 2H), 1.63-1.70 (m, 2H), 2.78 (t, *J*=7.60 Hz, 2H), 3.32-3.38 (m, 2H), 3.85 (s, 3H), 4.31 (t, *J*=4.80 Hz, 1H), 7.40 (d, *J*=8.40 Hz, 1H), 8.17 (d, *J*=8.40 Hz, 1H), 8.97 (s, 1H).

**Step 3**

**Intermediate 67: methyl 6-(5-oxopentyl)nicotinate**

To a solution of methyl 6-(5-hydroxypentyl)nicotinate (**Intermediate 66,** 8.00 g, 36 mmol) in dichloromethane (100 mL) was added Dess-Martin periodinane (23.0 g, 54 mmol) at 0°C. The reaction mixture was then stirred at room

temperature for 2h. The reaction mixture was quenched with aqueous saturated $NaHCO_3$ solution (200 mL) followed by aqueous sodium thiosulfate (200 mL) solution and extracted with ethyl acetate (2 × 250 mL). The combined organic phases were dried over anhydrous $Na_2SO_4$, filtered, and concentrated *in vacuo.* The crude material was purified by combi-flash chromatography by eluting with 25% ethyl acetate in heptane to afford methyl 6-(5-oxopentyl) nicotinate **(Intermediate 67,** 4.00 g, 50%) as a yellow liquid. $^1H$ NMR (400 MHz, DMSO-$d_6$) δ ppm 1.48-1.56 (m, 2H), 1.64-1.72 (m, 2H), 2.41-2.46 (m, 2H), 2.80 (t, *J*=7.20 Hz, 2H), 3.85 (s, 3H), 7.41 (d, *J*=7.80 Hz, 1H), 8.17 (d, *J*=7.80 Hz, 1H), 8.97 (s, 1H), 9.64 (s, 1H). Mass spec: m/z: 222.5 $[M+H]^+$.

**Step 4**

**Intermediate 68: methyl 6-(hex-5-yn-1-yl)nicotinate**

To a solution of methyl 6-(5-oxopentyl)nicotinate **(Intermediate 67,** 4.00 g, 18.1 mmol) in methanol (100 mL) was added potassium carbonate (2.99 g, 21.7 mmol) at 0°C, then dimethyl (1-diazo-2-oxopropyl) phosphonate (Comp-6, 4.52 mL, 27.1 mmol) was added dropwise to the reaction mixture for over 5 min. The reaction mixture was then stirred at room temperature for 16h. The reaction mixture was concentrated and water (50 mL) was added and extracted with ethyl acetate (3 × 50 mL). The combined organic phases were dried over anhydrous $Na_2SO_4$, and concentrated *in vacuo* to obtain crude compound which was purified by combi-flash chromatography by eluting with 10% ethyl acetate in heptane to afford methyl 6-(hex-5-yn-1-yl) nicotinate **(Intermediate 68,** 2.90 g, 74%) as a colorless liquid. $^1H$ NMR (400 MHz, DMSO-$d_6$) δ ppm 1.42-1.52 (m, 2H), 1.71-1.82 (m, 2H), 2.15-2.23 (m, 2H), 2.74 (s, 1H), 2.82 (t, *J*=7.60 Hz, 2H), 3.87 (s, 3H), 7.42 (d, *J*=8.40 Hz, 1H), 8.19 (d, *J*=8.40 Hz, 1H), 8.99 (s, 1H). Mass spec: m/z: 218.1 $[M+H]^+$.

**Step 5**

**Intermediate 69: 6-(hex-5-yn-1-yl)nicotinic acid**

To a solution of methyl 6-(hex-5-yn-1-yl)nicotinate (**Intermediate 68,** 2.80 g, 12.9 mmol) in tetrahydrofuran (12 mL), methanol (12 mL) and water (12 mL) was added lithium hydroxide (0.94 g, 38.7 mmol) and the reaction mixture was stirred at room temperature for 16h. The reaction mixture was concentrated *in vacuo.* The crude residue was acidified by the addition of 1N HCl to pH~5 and then extracted with ethyl acetate (2 × 200 mL). The combined organic phases were dried over anhydrous $Na_2SO_4$ and concentrated *in vacuo* to afford 6-(hex-5-yn-1-yl)nicotinic acid (**Intermediate 69,** 2.2 g, 84%) as an off-white solid, which was used for the next step without further purification. $^1H$ NMR (400 MHz, DMSO-$d_6$) δ ppm 1.41-1.49 (m, 2H), 1.71-1.80 (m, 2H), 2.10-2.23 (m, 2H), 2.73 (s, 1H), 2.80 (t, J=7.60 Hz, 2H), 7.37 (d, *J*=8.00 Hz, 1H), 8.14 (d, *J*=8.00 Hz, 1H), 8.95 (s, 1H), 13.21 (br s, 1H). Mass spec: m/z: 204.5 $[M+H]^+$.

**Step 6**

**Intermediate 70: 6-(hex-5-yn-1-yl)nicotinamide**

To a solution of 6-(hex-5-yn-1-yl)nicotinic acid (**Intermediate 69, 2.00** g, 9.84 mmol) in dimethylformamide (20 mL) was added HATU (5.79 g, 14.8 mmol) and the reaction stirred at room temperature for 10 min. Ammonium chloride (2.64 g, 49.2 mmol) and *N, N*-diisopropylethylamine (5.15 mL, 29.5 mmol) were then added and the reaction mixture was stirred at room temperature for 16h. The reaction mixture was poured into ice cold water (100 mL), resulting in a precipitate, which was collected by filtration and washed with water (2 × 50 mL) and dried under reduced pressure to afford 6-(hex-5-yn-1-yl)nicotinamide **(Intermediate 70,** 1.20 g, 60%) as an off-white solid, which was used for next step without further purification. $^1H$ NMR (400 MHz, DMSO-$d_6$) δ ppm 1.41-1.51 (m, 2H), 1.71-1.81 (m, 2H), 2.15-2.22 (m, 2H), 2.75 (s, 1H), 2.79 (t, *J*=7.60 Hz, 2H), 7.34 (d, *J*=8.0 Hz, 1H), 7.48 (br s, 1H), 8.06 (br s, 1H), 8.09 (d, *J*=8.0 Hz, 1H), 8.92 (s, 1H). Mass spec: m/z: 203.5 $[M+H]^+$.

**Step 7**

**Intermediate 71: 6-(6-(2-(2, 6-dioxo-1-((2-(trimethylsilyl) ethoxy) methyl) piperidin-3-yl)-1-oxoisoindolin-4-yl) hex-5-yn-1-yl)nicotinamide**

To a solution of 6-(hex-5-yn-1-yl) nicotinamide **(Intermediate 70,** 0.30 g, 1.48 mmol) in dimethylformamide (5 mL) was added 3-(4-iodo-1-oxoisoindolin-2-yl)-1-((2-(trimethylsilyl)ethoxy)methyl)piperidine-2,6-dione **(Intermediate 8,** 0.81 g, 1.63 mmol) followed by triethylamine (7.27 mL, 51.91 mmol). The reaction mixture was purged with argon for 15 min then copper (I) iodide (0.029 g, 0.15 mmol) and $PdCl_2(PPh_3)_2$ (0.11 g, 0.15 mmol) were added. The reaction mixture was

further purged with argon for 10 min and stirred at room temperature for 2h. The reaction mixture was filtered through celite, washed with ethyl acetate (100 mL) and the filtrate was concentrated *in vacuo* to obtain crude compound. The crude material was purified by combi-flash chromatography by eluting with 2% MeOH in DCM to afford 6-(6-(2-(2, 6-dioxo-1-((2-(trimethylsilyl)ethoxy)methyl)piperidin-3-yl)-1-oxoisoindolin-4-yl)hex-5-yn-1-yl)nicotinamide **(Intermediate 71,** 0.45 g, 53%) as a yellow solid. $^1$H NMR (400 MHz, DMSO-$d_6$) δ ppm -0.04 (s, 9H), 0.77-0.89 (m, 2H), 1.56-1.66 (m, 2H), 1.79-1.93 (m, 3H), 2.02-2.11 (m, 1H), 2.31-2.43 (m, 2H), 2.75-2.88 (m, 3H), 3.01-3.12 (m, 1H), 3.47-3.57 (m, 2H), 4.24-4.28 (m, 1H), 4.43-4.48 (m, 1H), 4.99-5.10 (m, 2H), 5.24-5.28 (m, 1H), 7.35 (d, *J*=8.0 Hz, 1H), 7.50 (br s, 1H), 7.52-7.54 (m, 1H), 7.64 (d, *J*=7.46 Hz, 1H), 7.72 (d, *J*=7.46 Hz, 1H), 8.05 (br s, 1H), 8.10 (d, *J*=8.0 Hz, 1H), 8.92 (br s, 1H). Mass spec: m/z: 575.1 [M+H]$^+$.

**Step 8**

**Intermediate 72: *tert*-butyl 6-(6-(6-(2-(2,6-dioxo-1-((2-(trimethylsilyl)ethoxy)methyl)piperidin-3-yl)-1-oxoisoindolin-4-yl)hex-5-yn-1-yl)nicotinamido)-2-((*R*)-1-methylpyrrolidin-2-yl)-1*H*-pyrrolo[3,2-*c*]pyridine-1-carboxylate**

To a solution of 6-(6-(2-(2, 6-dioxo-1-((2-(trimethylsilyl) ethoxy) methyl) piperidin-3-yl)-1-oxoisoindolin-4-yl) hex-5-yn-1-yl) nicotinamide **(Intermediate 71,** 0.41 g, 0.71 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate **(Intermediate 4,** 0.32 g, 0.85 mmol) followed by cesium carbonate (0.69 g, 2.14 mmol) at room temperature. The reaction mixture was purged with argon for 15 min then $Pd_2(dba)_3$ (0.10 g, 0.11 mmol) and Xantphos (0.13 g, 0.21 mmol) were added. The reaction mixture was further purged with argon for 10 min and then stirred at 100°C for 2h. The reaction mixture was filtered through celite, washed with ethyl acetate (100 mL) and the filtrate was concentrated *in vacuo* to obtain the crude compound. The crude material was purified by combi-flash chromatography by eluting with 5% MeOH in DCM to afford tert-butyl 6-(6-(6-(2-(2,6-dioxo-1-((2-(trimethylsilyl)ethoxy)methyl)piperidin-3-yl)-1-oxoisoindolin-4-yl)hex-5-yn-1-yl)nicotinamido)-2-((*R*)-1-methylpyrrolidin-2-yl)-1*H*-pyrrolo[3,2-*c*]pyridine-1-carboxylate **(Intermediate 72,** 0.31 g, 50%) as a yellow solid. $^1$H NMR (400 MHz, DMSO-$d_6$) δ ppm -0.05 (s, 9H), 0.77-0.86 (m, 2H), 1.56-1.66 (m, 4H), 1.69 (s, 9H), 1.74-1.76 (m, 2H), 1.83-1.93 (m, 2H), 2.02-2.11 (m, 1H), 2.28-2.33 (m, 1H), 2.35 (s, 3H), 2.38-2.42 (m, 1H), 2.53-2.58 (m, 2H), 2.74-2.90 (m, 3H), 3.01-3.16 (m, 2H), 3.44-3.55 (m, 2H), 3.90-3.94 (m, 1H), 4.25-4.29 (m, 1H), 4.45-4.49 (m, 1H), 4.98-5.09 (m, 2H), 5.24-5.28 (m, 1H), 6.76 (s, 1H), 7.41 (d, *J*=8.0 Hz, 1H), 7.50-7.56 (m, 1H), 7.65 (d, *J*=7.60 Hz, 1H), 7.72 (d, *J*=7.60 Hz, 1H), 8.28 (d, *J*=80 Hz, 1H), 8.60 (s, 1H), 8.93 (s, 1H), 9.06 (s, 1H), 10.91 (br s, 1H). Mass spec: m/z: 873.8 [M+H]$^+$.

**Step 9**

**Example 10: 6-(6-(2-(2, 6-dioxopiperidin-3-yl)-1-oxoisoindolin-4-yl) hex-5-yn-1-yl)-*N*-(2-((*R*)-1-methylpyrrolidin-2-yl)-1*H*-pyrrolo [3, 2-*c*] pyridin-6-yl) nicotinamide**

To a solution of *tert*-butyl 6-(6-(6-(2-(2,6-dioxo-1-((2-(trimethylsilyl)ethoxy)methyl)piperidin-3-yl)-1-oxoisoindolin-4-yl)hex-5-yn-1-yl)nicotinamido)-2-((*R*)-1-methylpyrrolidin-2-yl)-1*H*-pyrrolo[3,2-*c*]pyridine-1-carboxylate **(Intermediate 72,** 0.30 g, 0.34 mmol) in acetonitrile (8 mL) was added methanesulfonic acid (0.22 mL, 3.43 mmol) and the reaction stirred at 50°C for 2h. *N*,*N'*-dimethylethylenediamine (0.41 mL, 3.43 mmol) and triethylamine (0.96 mL, 6.86 mmol) were added at room temperature and the reaction mixture was stirred at room temperature for 2h. The reaction mixture was poured into cold water (80 mL), resulting in a precipitate which was collected by filtration, washed with pentane (2 × 40 mL) and dried *in vacuo* to obtain the crude compound. The crude material was purified by preparative HPLC (Method A) to afford 6-(6-(2-(2, 6-dioxopiperidin-3-yl)-1-oxoisoindolin-4-yl)hex-5-yn-1-yl)-*N*-(2-((*R*)-1-methylpyrrolidin-2-yl)-1*H*-pyrrolo [3,2-c]pyridin-6-yl) nicotinamide **(Example 10,** 0.09 g, 38%) as an off white solid. $^1$H NMR (400 MHz, DMSO-$d_6$) δ ppm 1.60-1.68 (m, 2H), 1.76-1.95 (m, 5H), 1.97-2.06 (m, 1H), 2.12-2.14 (m, 1H), 2.17 (s, 3H), 2.24-2.30 (m, 1H), 2.41-2.46 (m, 1H), 2.56-2.62 (m, 3H), 2.84-2.92 (m, 3H), 3.10-3.19 (m, 1H), 3.34-3.41 (m, 1H), 4.28-4.36 (m, 1H), 4.43-4.50 (m, 1H), 5.11-5.16 (m, 1H), 6.40 (s, 1H), 7.38-7.42 (m, 1H), 7.50-7.55 (m, 1H), 7.64 (d, *J*=7.40 Hz, 1H), 7.71 (d, *J*=7.40 Hz, 1H), 8.19 (s, 1H), 8.27-8.30 (m, 1H), 8.51 (s, 1H), 9.08 (s, 1H), 10.68 (br s, 1H), 11.00 (br s, 1H), 11.40 (br s, 1H). Mass spec: m/z: 644.0 [M+H]$^+$.

**Example 11 was synthesised following Scheme 14**

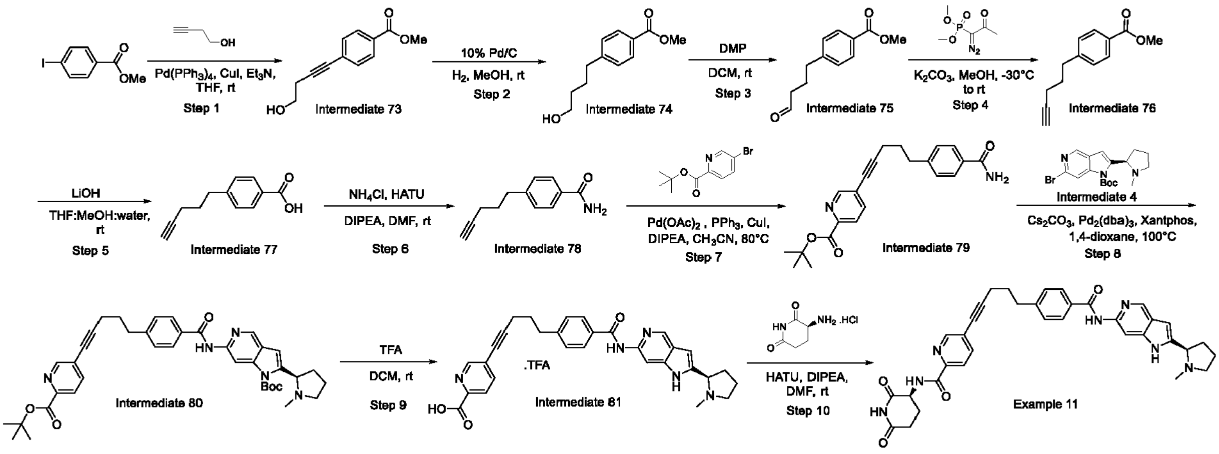


## Scheme 14

### Step 1

**Intermediate 73: methyl 4-(4-hydroxybut-1-yn-1-yl)benzoate**

To a solution of methyl 4-iodobenzoate (CAS No: 619-44-3, 20.0 g, 76.32 mmol) in tetrahydrofuran (150 mL) was added but-3-yn-1-ol (CAS No: 927-74-2, 8.02 g, 114.49 mmol) followed by triethylamine (150 mL, 1070.0 mmol). The reaction mixture was purged with argon for 15 min followed by the addition of copper(I) iodide (1.48 g, 7.63 mmol) and $Pd(PPh_3)_4$ (4.41 g, 3.81 mmol). The reaction mixture was further purged with argon for 10 min and stirred at room temperature for 16h. The reaction mixture was diluted with water (400 mL) and extracted with ethyl acetate (3 × 500 mL). The combined organic phases were dried over anhydrous $Na_2SO_4$ and concentrated *in vacuo.* The crude material was purified by combi-flash column chromatography by eluting with 80% ethyl acetate in heptane to afford methyl 4-(4-hydroxybut-1-yn-1-yl)benzoate **(Intermediate 73,** 14.00 g, 90%) as yellow solid. $^1H$ NMR (400 MHz, DMSO-$d_6$) δ ppm 2.59 (t, *J*=6.80 Hz, 2H), 3.56-3.62 (m, 2H), 3.85 (s, 3H), 4.93 (t, *J*=5.20 Hz, 1H), 7.52 (d, *J*=7.89 Hz, 2H), 7.91 (d, *J*=7.89 Hz, 2H). Mass spec: m/z: 205.4 $[M+H]^+$

### Step 2

**Intermediate 74:** methyl **4-(4-hydroxybutyl)benzoate**

To a solution of methyl 4-(4-hydroxybut-1-yn-1-yl)benzoate **(Intermediate 73,** 16.00 g, 78.35 mmol) in methanol (200 mL) was added 10% Pd/C (5.00 g) at room temperature. The reaction mixture was stirred at room temperature for 16h under $H_2$ (110 psi). The reaction mixture was filtered through celite, washed with MeOH (300 mL) and concentrated *in vacuo* to afford methyl 4-(4-hydroxybutyl) benzoate **(Intermediate 74,** 15.0 g, 92%) as yellow liquid, which was used for the next step without further purification. $^1H$ NMR (400 MHz, DMSO-$d_6$) δ ppm 1.37-1.48 (m, 2H), 1.55-1.66 (m, 2H), 2.65 (t, *J*=7.60 Hz, 2H), 3.37-3.43 (m, 2H), 3.83 (s, 3H), 4.37 (t, *J*=5.20 Hz, 1H), 7.33 (d, *J*=8.0 Hz, 2H), 7.85-7.89 (d, *J*=8.0 Hz, 2H). Mass spec: m/z: 209.5 $[M+H]^+$.

### Step 3

**Intermediate 75:** methyl **4-(4-oxobutyl)benzoate**

To a solution of methyl 4-(4-hydroxybutyl)benzoate **(Intermediate 74,** 14.00 g, 67.22 mmol) in dichloromethane (300 mL) was added Dess-Martin periodinane (44.09 g, 100.84 mmol) and the reaction mixture was stirred at room temperature for 4h. The reaction mixture was diluted with mixture of saturated sodium bicarbonate and sodium thiosulphate (1:1, 500 mL) solution and extracted with dichloromethane (3 × 300 mL). The combined organic phases were dried over anhydrous $Na_2SO_4$ and concentrated *in vacuo.* The crude material was purified by combi-flash chromatography by eluting with 20% ethyl acetate in heptane to afford methyl 4-(4-oxobutyl) benzoate **(Intermediate 75,** 11.00 g, 82%) as a yellow liquid. $^1H$ NMR (400 MHz, DMSO-$d_6$) δ ppm 1.78-1.91 (m, 2H), 2.47 (t, *J*=7.20 Hz, 2H), 2.66 (t, *J*=7.20 Hz, 2H), 3.84 (s, 3H), 7.35 (d, *J*=8.0 Hz, 2H), 7.88 (d, *J*=8.0 Hz, 2H), 9.66 (s, 1H).

**Step 4**

**Intermediate 76: methyl 4-(pent-4-yn-1-yl)benzoate**

To a solution of methyl 4-(4-oxobutyl)benzoate **(Intermediate 75,** 11.00 g, 53.33 mmol) in methanol (200 mL) was added potassium carbonate (14.72 g, 106.67 mmol) at -30°C. Dimethyl (1-diazo-2-oxopropyl)phosphonate (Comp-6, 15.37 g, 80.0 mmol) was added and the reaction mixture stirred from -30°C to room temperature and then at room temperature for 3h. The reaction mixture was diluted water (500 mL) and extracted with ethyl acetate (3 × 200 mL). The combined organic phases were dried over anhydrous $Na_2SO_4$ and concentrated *in vacuo.* The crude material was purified by combi-flash chromatography by eluting with 10% ethyl acetate in heptane to afford methyl 4-(pent-4-yn-1-yl)benzoate **(Intermediate 76,** 8.00 g, 74%) as a yellow liquid. $^1$H NMR (400 MHz, DMSO-$d_6$) δ ppm 1.73-1.91 (m, 2H), 2.14-2.18 (m, 2H), 2.73 (t, *J*=7.60 Hz, 2H), 2.82 (s, 1H), 3.83 (s, 3H), 7.35 (d, *J*=8.0 Hz, 2H), 7.88 (d, *J*=8.0 Hz, 2H).

**Step 5**

**Intermediate 77: 4-(pent-4-yn-1-yl)benzoic acid**

To a solution of methyl 4-(pent-4-yn-1-yl)benzoate **(Intermediate 76,** 8.00 g, 39.56 mmol) in tetrahydrofuran (40 mL), methanol (40 mL) and water (20 mL) was added lithium hydroxide (3.86 g, 158.3 mmol) in water (20 mL). The reaction mixture was then stirred at room temperature for 16h. The reaction mixture was then concentrated *in vacuo,* the crude was then diluted with water (30 mL) and acidified to pH~4 with 0.5 N HCl. The resulting precipitate was filtered and dried *in vacuo* to afford 4-(pent-4-yn-1-yl)benzoic acid **(Intermediate 77,** 6.30 g, 85%) as a white solid, which was used for the next step without further purification. $^1$H NMR (400 MHz, DMSO-$d_6$) δ ppm 1.72-1.80 (m, 2H), 2.12-2.20 (m, 2H), 2.73 (t, *J*=7.60 Hz, 2H), 2.82 (s, 1H), 7.32 (d, *J*=7.88 Hz, 2H), 7.86 (d, *J*=7.88 Hz, 2H), 12.80 (br s, 1H).

**Step 6**

**Intermediate 78: 4-(pent-4-yn-1-yl)benzamide**

To a solution of 4-(pent-4-yn-1-yl)benzoic acid **(Intermediate 77,** 6.30 g, 33.0 mmol) in dimethylformamide (80 mL) were added HATU (20 g, 50.0 mmol) and *N, N*-diisopropylethylamine (18 mL, 100.0 mmol). Ammonium chloride (7.2 g, 130.0 mmol) was then added and the reaction stirred at room temperature for 16h. The reaction mixture was diluted water (100 mL) and extracted with ethyl acetate (3 × 100 mL). The combined organic phases were dried over anhydrous $Na_2SO_4$ and concentrated *in vacuo.* The crude material was purified by combi-flash chromatography by eluting with 80% ethyl acetate in heptane to afford 4-(pent-4-yn-1-yl) benzamide **(Intermediate 78,** 5.60 g, 89%) as an off white solid. $^1$H NMR (400 MHz, DMSO-$d_6$) δ ppm 1.68-1.80 (m, 2H), 2.09-2.20 (m, 2H), 2.71 (t, *J*=7.60 Hz, 2H), 2.83 (s, 1H), 7.26 (br s, 1H), 7.27 (d, *J*=8.0 Hz, 2H), 7.79 (d, *J*=8.0 Hz, 2H), 7.89 (br s, 1H). Mass spec: 188.5 $[M+H]^+$.

**Step 7**

**Intermediate 79: *tert*-butyl 5-(5-(4-carbamoylphenyl)pent-1-yn-1-yl)picolinate**

To a solution of 4-(pent-4-yn-1-yl)benzamide **(Intermediate 78,** 1.00 g, 5.34 mmol) in acetonitrile (20 mL) was added *tert*-butyl 5-bromopicolinate (CAS No: 845306-08-3, 1.38 g, 5.34 mmol) followed by *N, N*-diisopropylethylamine (5 mL, 26.704 mmol). The reaction mixture was purged with argon for 15 min followed by the addition of copper(I) iodide (0.11 g, 0.53 mmol), $Pd(OAc)_2$ (0.13 g, 0.80 mmol) and $PPh_3$ (0.42 g, 1.60 mmol). The reaction mixture was further purged with argon for 10 min and heated at 80°C for 2h. The reaction mixture was diluted with water (100 mL) and extracted with ethyl acetate (3 × 50 mL). The combined organic phases were dried over anhydrous $Na_2SO_4$ and concentrated *in vacuo.* The crude material was purified by combi-flash chromatography by eluting with 2% MeOH in DCM to afford *tert*-butyl 5-(5-(4-carbamoylphenyl)pent-1-yn-1-yl)picolinate **(Intermediate 79,** 1.20 g, 62%) as a yellow solid. $^1$H NMR (400 MHz, DMSO-$d_6$) δ ppm 1.55 (s, 9H), 1.54-1.58 (m, 2H), 1.89 (t, *J*=7.60 Hz, 2H), 2.82 (t, *J*=7.60 Hz, 2H), 7.28 (br s, 1H), 7.31 (d, *J*=8.0 Hz, 2H), 7.80 (d, *J*=8.0 Hz, 2H), 7.87 (br s, 1H), 7.92-7.96 (m, 2H), 8.70 (s, 1H). Mass spec: m/z: 365.3 $[M+H]^+$.

**Step 8**

**Intermediate 80: *tert*-butyl (*R*)-6-(4-(5-(6-(tert-butoxycarbonyl) pyridin-3-yl)pent-4-yn-1-yl)benzamido)-2-(1-methylpyrrolidin-2-yl)-1*H*-pyrrolo [3, 2-c]pyridine-1-carboxylate**

To a solution of *tert*-butyl 5-(5-(4-carbamoylphenyl)pent-1-yn-1-yl)picolinate **(Intermediate 79,** 1.00 g, 2.7 mmol) in 1,4-dioxane (20 mL) was added *tert*-butyl (*R*)-6-bromo-2-(1-methylpyrrolidin-2-yl)-1*H*-pyrrolo[3,2-c]pyridine-1-carboxylate **(Intermediate 4,** 1.1 g, 3.0 mmol) followed by cesium carbonate (1.60 g, 8.2 mmol) at room temperature. The reaction mixture was purged with argon for 15 min followed by the addition of $Pd_2(dba)_3$ (0.39 g, 0.41 mmol) and Xantphos (0.48 g, 0.82 mmol). The reaction mixture was further purged with argon for 10 min and heated at 100°C for 2h. The reaction mixture was concentrated *in vacuo* and the crude material was purified by combi-flash chromatography by eluting with 70% ethyl acetate in heptane to afford *tert*-butyl (*R*)-6-(4-(5-(6-(*tert*-butoxycarbonyl)pyridin-3-yl)pent-4-yn-1-yl)benzamido)-2-(1-methylpyrrolidin-2-yl)-1*H*-pyrrolo [3,2-c] pyridine-1-carboxylate (**Intermediate 80,** 1.20 g, 66%) as a yellow solid. $^1$H NMR (400 MHz, DMSO-$d_6$) δ ppm 1.55 (s, 9H), 1.58-1.64 (m, 2H), 1.70 (s, 9H), 1.74-1.82 (m, 2H), 1.86-2.00 (m, 3H), 2.11-2.20 (m, 2H), 2.35 (s, 3H), 2.78-2.88 (m, 2H), 3.12-3.18 (m, 1H), 3.88-3.96 (m, 1H), 6.75 (s, 1H), 7.26-7.46 (m, 3H), 7.96-8.02 (m, 3H), 8.59 (s, 1H), 8.71 (s, 1H), 8.93 (s, 1H), 10.60 (br s, 1H). Mass spec: m/z: 664.1 $[M+H]^+$.

**Step 9**

**Intermediate 81: (*R*)-5-(5-(4-((2-(1-methylpyrrolidin-2-yl)-1*H*-pyrrolo[3,2-c]pyridin-6-yl)carbamoyl) phenyl) pent-1-yn-1-yl)picolinic acid trifluoroacetate**

To a solution of *tert*-butyl (*R*)-6-(4-(5-(6-(*tert*-butoxycarbonyl)pyridin-3-yl)pent-4-yn-1-yl)benzamido)-2-(1-methylpyrrolidin-2-yl)-1*H*-pyrrolo[3,2-c]pyridine-1-carboxylate **(Intermediate 80,** 0.50 g, 0.75 mmol) in dichloromethane (10 mL) was added trifluoroacetic acid (10 mL) at 0°C. The reaction mixture was then stirred at room temperature for 16h. The reaction mixture was concentrated *in vacuo,* the crude material was washed with diethyl ether (5 × 2 mL) and dried under reduced pressure to afford (*R*)-5-(5-(4-((2-(1-methylpyrrolidin-2-yl)-1*H*-pyrrolo[3,2-c]pyridin-6-yl)carbamoyl)phenyl)pent-1-yn-1-yl)picolinic acid trifluoroacetate (**Intermediate 81,** 0.50 g) as a gum, which was used for the next step without further purification. Mass spec: m/z: 508.2 $[M+H]^+$.

**Step 10**

**Example 11: *N*-((*S*)-2,6-dioxopiperidin-3-yl)-5-(5-(4-((2-((*R*)-1-methylpyrrolidin-2-yl)-1*H*-pyrrolo[3,2-c]pyridin-6-yl)carbamoyl)phenyl)pent-1-yn-1-yl)picolinamide**

To a solution of (*R*)-5-(5-(4-((2-(1-methylpyrrolidin-2-yl)-1*H*-pyrrolo[3,2-c]pyridin-6-yl)carbamoyl)phenyl)pent-1-yn-1-yl)picolinic acid trifluoroacetate (**Intermediate 81,** 0.40 g, 0.78 mmol) in dimethylformamide (10 mL) was added HATU (0.47 g, 1.18 mmol) and the reaction stirred at room temperature for 15 min. (S)-3-aminopiperidine-2,6-dione hydrochloride (CAS No: 25181-50-4, 0.25 g, 1.18 mmol) and *N*, *N*-diisopropylethylamine (0.55 mL, 3.15 mmol) were added and the reaction mixture was stirred at room temperature for 3h. The reaction mixture was quenched with ice cold water (100 mL) to afford a precipitate which was filtered and dried *in vacuo* to obtain crude compound. The crude material was purified by preparative HPLC (Method A) to afford *N*-((*S*)-2,6-dioxopiperidin-3-yl)-5-(5-(4-((2-((*R*)-1-methylpyrrolidin-2-yl)-1*H*-pyrrolo[3,2-c]pyridin-6-yl)carbamoyl)phenyl)pent-1-yn-1-yl)picolinamide **(Example 11,** 0.09 g, 20%) as a white solid. $^1$H NMR (400 MHz, DMSO-$d_6$) δ ppm 1.78-2.03 (m, 6H), 2.17 (s, 3H), 2.19-2.30 (m, 3H), 2.44-2.48 (m, 2H), 2.54-2.57 (m, 2H), 2.75-2.87 (m, 3H), 3.12-3.16 (m, 1H), 4.74-4.83 (m, 1H), 6.39 (s, 1H), 7.39 (d, *J*=8.0 Hz, 2H), 7.98-8.07 (m, 4H), 8.19 (s, 1H), 8.50 (s, 1H), 8.70 (s, 1H), 9.08 (d, *J*=8.0 Hz, 1H), 10.36 (br s, 1H), 10.86 (br s, 1H), 11.35 (br s, 1H). Mass spec: m/z: 618.2 $[M+H]^+$.

**Example 12 was synthesised following Scheme 15**

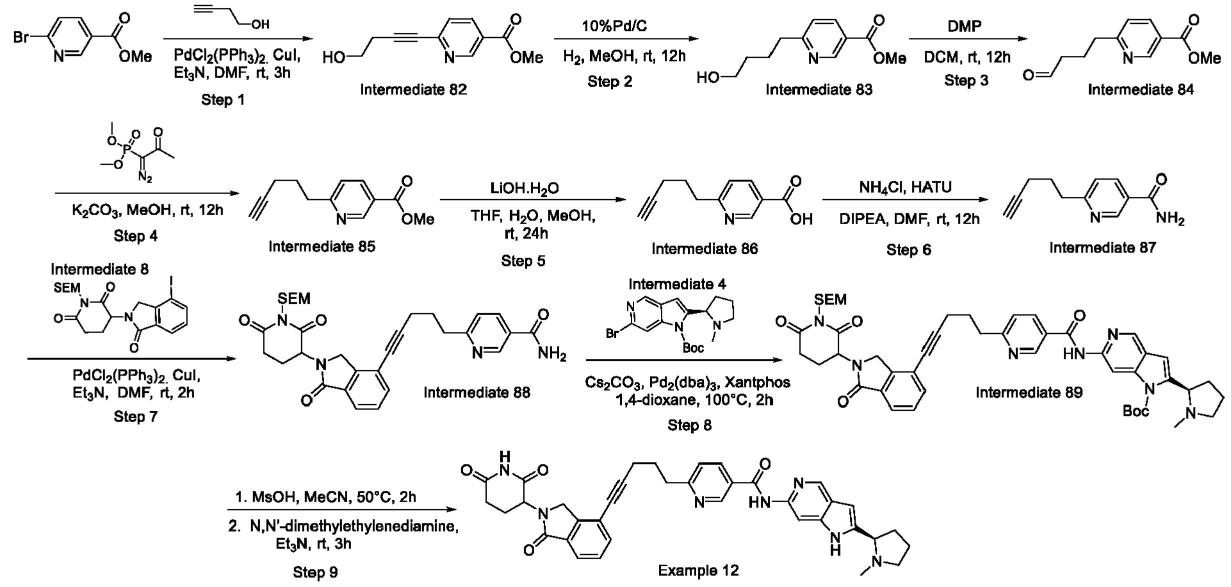


## Scheme 15

### Step 1

### Intermediate 82: methyl 6-(4-hydroxybut-1-yn-1-yl)nicotinate

To a solution of methyl 6-bromonicotinate (CAS No: 26218-78-0, 25.0 g, 115.7 mmol) in dimethylformamide (150 mL) was added but-3-yn-1-ol (CAS No: 927-74-2, 8.11 g, 115.7 mmol) followed by triethylamine (48.6 mL, 347.2 mmol). The reaction mixture was purged with argon for 15 min. Copper(I) iodide (2.23 g, 11.57 mmol) and $PdCl_2(PPh_3)_2$ (8.37 g, 11.57 mmol) were then added and the reaction mixture was further purged with argon for 10 min and stirred at room temperature for 3h. The reaction mixture was diluted with water (500 mL) and extracted with ethyl acetate (3 × 500 mL). The combined organic phases were dried over anhydrous $Na_2SO_4$ and concentrated *in vacuo.* The crude material was purified by combi-flash column chromatography by eluting with 100% ethyl acetate in heptane to afford methyl 6-(4-hydroxybut-1-yn-1-yl) nicotinate (**Intermediate 82,** 14.0 g, 59%) as an off white solid. $^1$H NMR (400 MHz, DMSO-$d_6$) δ ppm 2.63 (t, *J*=6.80 Hz, 2H), 3.59-3.64 (m, 2H), 3.88 (s, 3H), 4.98 (t, *J*=6.80 Hz, 1H), 7.60 (d, *J*=8.40 Hz, 1H), 8.24 (d, *J*=8.40 Hz, 1H), 9.01 (s, 1H). Mass spec: m/z: 206.01 [M+H]$^+$.

### Step 2

### Intermediate 83: methyl 6-(4-hydroxybutyl)nicotinate

To a solution of methyl 6-(4-hydroxybut-1-yn-1-yl)nicotinate (**Intermediate 82,** 13.0 g, 63.35 mmol) in methanol (150 mL) was added 10% Pd/C (4.5 g) and the mixture was stirred at room temperature for 12h under hydrogen (100 psi). After completion, the reaction mixture was filtered through a celite bed and concentrated *in vacuo* to afford methyl 6-(4-hydroxybutyl)nicotinate (**Intermediate 83,** 9.00 g, 70%) as yellow liquid, which was used for the next step without further purification. $^1$H NMR (400 MHz, DMSO-$d_6$) δ ppm 1.34-1.50 (m, 2H), 1.66-1.76 (m, 2H), 2.81 (t, *J*=7.60 Hz, 2H), 3.38-3.43 (m, 2H), 3.87 (s, 3H), 4.38 (t, *J*=5.20 Hz, 1H), 7.42 (d, *J*=8.40 Hz, 1H), 8.18 (d, *J*=8.40 Hz, 1H), 8.99 (s, 1H). Mass spec: m/z: 208.02 [M-H]$^-$.

### Step 3

### Intermediate 84: methyl 6-(4-oxobutyl)nicotinate

To a solution of methyl 6-(4-hydroxybutyl)nicotinate (**Intermediate 83,** 9.00 g, 43.01 mmol) in dichloromethane (90 mL) was added Dess-Martin Periodinane (22.11 g, 51.61 mmol) and the mixture was stirred at room temperature for 12h. The reaction mixture was then filtered through celite and concentrated *in vacuo* to afford methyl 6-(4-oxobutyl) nicotinate (**Intermediate 84,** 2.70 g, 30%) as a yellow liquid, which was used for the next step without further purification. $^1$H NMR (400 MHz, DMSO-$d_6$) δ ppm 1.84-2.00 (m, 2H), 2.82 (t, *J*=7.60, 2H), 3.32-3.35 (m, 2H), 3.87 (s, 3H), 7.37-7.48 (m,

1H), 8.08-8.24 (m, 1H), 9.00 (s, 1H), 9.69 (s, 1H).

**Step 4**

**Intermediate 85: methyl 6-(pent-4-yn-1-yl)nicotinate**

To a solution of methyl 6-(4-oxobutyl) nicotinate (**Intermediate 84,** 2.70 g, 13.0 mmol) in methanol (20 mL) was added potassium carbonate (1.90 g, 20.0 mmol). Dimethyl(1-diazo-2-oxopropyl)phosphonate (3.0 g, 16.0 mmol) was added and the reaction stirred at room temperature for 12h. The reaction mixture was concentrated *in vacuo* and the crude material was purified by combi-flash column chromatography by eluting with 25% ethyl acetate in heptane to afford methyl 6-(pent-4-yn-1-yl) nicotinate **(Intermediate 85,** 1.70 g, 64%) as colourless liquid. $^1$H NMR (400 MHz, DMSO-$d_6$) δ ppm 1.84-1.92 (m, 2H), 2.20 (t, *J*=7.60 Hz, 2H), 2.81 (s, 1H), 2.90 (t, *J*=7.60 Hz 2H), 3.87 (s, 3H), 7.43 (d, *J*=8.40 Hz, 1H), 8.19 (d, *J*=8.40 Hz, 1H), 9.00 (s, 1H). Mass spec: m/z 204.04 $[M+H]^+$.

**Step 5**

**Intermediate 86: 6-(pent-4-yn-1-yl)nicotinic acid**

To a solution of methyl 6-(pent-4-yn-1-yl)nicotinate (**Intermediate 85,** 1.70 g, 8.4 mmol) in tetrahydrofuran: methanol:water (3:1:1, 50 mL) was added lithium hydroxide (0.61 g, 25.0 mmol) in water (10 mL) at 0°C. The reaction mixture was then stirred at room temperature for 24h. The reaction mixture was concentrated *in vacuo.* The reaction mixture was diluted with water (50 mL), acidified to pH~4 with 1N HCl and extracted with ethyl acetate (3 × 100 mL). The combined organic phases were dried over anhydrous $Na_2SO_4$, and concentrated *in vacuo* to afford 6-(pent-4-yn-1-yl) nicotinic acid (**Intermediate 86,** 1.50 g, 95%) as an off white solid. $^1$H NMR (401 MHz, DMSO-$d_6$) δ ppm 1.84-1.92 (m, 2H), 2.18-2.24 (m, 2H), 2.84 (s, 1H), 2.90 (t, *J*=7.60 Hz, 2H), 7.41 (d, *J*=8.0 Hz, 1H), 8.17 (d, *J*=8.0 Hz, 1H), 8.98 (s, 1H), 13.28 (br s, 1H). Mass spec: m/z: 189.98 $[M+H]^+$.

**Step 6**

**Intermediate 87: 6-(pent-4-yn-1-yl)nicotinamide**

To a solution of 6-(pent-4-yn-1-yl)nicotinic acid (**Intermediate 86,** 1.50 g, 7.93 mmol) in dimethylformamide (10 mL) was added HATU (3.73 g, 9.51 mmol) and *N, N*-diisopropylethylamine (5.54 mL, 31.7 mmol). Ammonium chloride (1.70 g, 31.7 mmol) was then added and the reaction stirred at room temperature for 12h. The reaction mixture was diluted with water (100 mL) and extracted with ethyl acetate (3 × 200 mL). The combined organic phases were dried over anhydrous $Na_2SO_4$, and concentrated *in vacuo.* The crude material was purified by combi-flash chromatography by eluting with 100% ethyl acetate in heptane to afford 6-(pent-4-yn-1-yl)nicotinamide **(Intermediate 87,** 1.40 g, 94%) as an off white solid. Mass spec: m/z: 189.06 $[M+H]^+$.

**Step 7**

**Intermediate 88: 6-(5-(2-(2, 6-dioxo-1-((2-(trimethylsilyl)ethoxy)methyl)piperidin-3-yl)-1-oxoisoindolin-4-yl) pent-4-yn-1-yl)nicotinamide**

To a solution of 6-(pent-4-yn-1-yl) nicotinamide **(Intermediate 87,** 0.60 g, 3.18 mmol) in dimethylformamide (5 mL) was added 3-(4-iodo-1-oxoisoindolin-2-yl)-1-((2-(trimethylsilyl)ethoxy)methyl)piperidine-2,6-dione **(Intermediate 8,** 1.59 g, 3.18 mmol) followed by triethylamine (16.1 mL, 114.75 mmol). The reaction mixture was purged with argon for 15 min followed by the addition of copper(I) iodide (0.06 g, 0.31 mmol) and $PdCl_2(PPh_3)_2$ (0.23 g, 0.31 mmol). The reaction mixture was further purged with argon for 10 min and stirred at room temperature for 2h. The reaction mixture was diluted with water (100 mL) and extracted with ethyl acetate (3 × 100 mL). The combined organic phases were dried over anhydrous $Na_2SO_4$, and concentrated *in vacuo.* The crude material was purified by combi-flash chromatography by eluting with ethyl acetate to afford 6-(5-(2-(2, 6-dioxo-1-((2-(trimethylsilyl)ethoxy)methyl)piperidin-3-yl)-1-oxoisoindolin-4-yl)pent-4-yn-1-yl)nicotinamide (**Intermediate 88,** 0.25 g, 10%) as a yellow solid. $^1$H NMR (400 MHz, DMSO-$d_6$) δ ppm -0.05 (s, 9H), 0.75-0.89 (m, 2H), 1.94-2.11 (m, 3H), 2.36-2.44 (m, 2H), 2.52-2.56 (m, 2H), 2.90-2.96 (m, 2H), 3.02-3.12 (m, 1H), 3.44-3.56 (m, 2H), 4.26-4.30 (m, 1H), 4.47-4.52 (m, 1H), 4.99-5.10 (m, 2H), 5.25-5.30 (m, 1H), 7.38 (d, *J*=7.60 Hz, 1H), 7.51 (br s, 1H), 7.53 (d, *J*=7.60 Hz, 1H), 7.65 (d, *J*=7.60 Hz, 1H), 7.72 (d, *J*=7.60 Hz, 1H), 8.07 (br s, 1H), 8.11-8.14 (m, 1H), 8.94 (s, 1H). Mass spec: m/z: 561.3 $[M+H]^+$.

**Step 8**

**Intermediate 89: tert-butyl 6-(6-(5-(2-(2,6-dioxo-1-((2-(trimethylsilyl)ethoxy)methyl)piperidin-3-yl)-1-oxoisoindolin-4-yl)pent-4-yn-1-yl)nicotinamido)-2-((*R*)-1-methylpyrrolidin-2-yl)-1*H*-pyrrolo[3,2-c]pyridine-1-carboxylate**

To a solution of 6-(5-(2-(2,6-dioxo-1-((2-(trimethylsilyl)ethoxy)methyl)piperidin-3-yl)-1-oxoisoindolin-4-yl) pent-4-yn-1-yl)nicotinamide (**Intermediate 88,** 0.50 g, 0.9 mmol) in 1,4-dioxane (10 mL) was added *tert*-butyl (*R*)-6-bromo-2-(1-methylpyrrolidin-2-yl)-1*H*-pyrrolo[3,2-c]pyridine-1-carboxylate (**Intermediate 4,** 0.30 g, 0.9 mmol) followed by cesium carbonate (0.6 g, 2.0 mmol) at room temperature. The reaction mixture was purged with argon for 15 min followed by $Pd_2(dba)_3$ (0.1 g, 0.1 mmol) and Xantphos (0.2 g, 0.3 mmol). The reaction mixture was purged with argon for 10 min and heated at 100°C for 2h. Progress of the reaction was monitored by TLC. After completion, the reaction mixture was concentrated *in vacuo.* The crude material was purified by combi-flash chromatography by eluting with 90% ethyl acetate in heptane to afford tert-butyl 6-(6-(5-(2-(2,6-dioxo-1-((2-(trimethylsilyl)ethoxy)methyl)piperidin-3-yl)-1-oxoisoindolin-4-yl)pent-4-yn-1-yl)nicotinamido)-2-((*R*)-1-methylpyrrolidin-2-yl)-1*H*-pyrrolo[3,2-c]pyridine-1-carboxylate (**Intermediate 89,** 0.40 g, 50%) as white solid. $^1$H NMR (400 MHz, DMSO-$d_6$) δ ppm -0.06 (s, 9H), 0.75-0.87 (m, 2H), 1.69 (s, 9H), 1.72-1.79 (m, 3H), 2.01-2.08 (m, 3H), 2.35 (s, 3H), 2.38-2.43 (m, 1H), 2.52-2.58 (m, 4H), 2.92-3.08 (m, 3H), 3.09-3.17 (m, 2H), 3.47-3.55 (m, 2H), 3.89-3.95 (m, 1H), 4.27-4.32 (m, 1H), 4.48-4.54 (m, 1H), 4.99-5.10 (m, 2H), 5.25-5.30 (m, 1H), 6.76 (s, 1H), 7.45 (d, *J*=8.40 Hz, 1H), 7.51-7.56 (m, 1H), 7.66-7.68 (m, 1H), 7.72-7.74 (m, 1H), 8.28-8.30 (m, 1H), 8.60 (s, 1H), 8.92 (s, 1H), 9.08 (s, 1H), 10.92 (br s, 1H). Mass spec: m/z: 860.2 $[M+H]^+$.

**Step 9**

**Example 12: 6-(5-(2-(2,6-dioxopiperidin-3-yl)-1-oxoisoindolin-4-yl)pent-4-yn-1-yl)-*N*-(2-((*R*)-1-methylpyrrolidin-2-yl)-1*H*-pyrrolo[3,2-c]pyridin-6-yl)nicotinamide**

To a solution of *tert*-butyl 6-(6-(5-(2-(2,6-dioxo-1-((2-(trimethylsilyl)ethoxy)methyl)piperidin-3-yl)-1-oxoisoindolin-4-yl)pent-4-yn-1-yl)nicotinamido)-2-((*R*)-1-methylpyrrolidin-2-yl)-1*H*-pyrrolo[3,2-c]pyridine-1-carboxylate (**Intermediate 89,** 0.40 g, 0.5 mmol) in acetonitrile (4 mL) was added methane sulfonic acid (0.5 mL, 7.0 mmol) at room temperature and heated at 50°C for 2h. *N*$^1$, *N*$^2$-dimethylethane-1, 2-diamine (0.3 mL, 3.0 mmol) followed by triethylamine (0.9 g, 9.0 mmol) were added and the reaction was stirred at room temperature for 3h. The reaction mixture was diluted with water (100 mL), resulting in a precipitate which was filtered and dried *in vacuo* to obtain the crude compound. The crude material was purified by preparative HPLC (Method A) to afford 6-(5-(2-(2,6-dioxopiperidin-3-yl)-1-oxoisoindolin-4-yl) pent-4-yn-1-yl)-*N*-(2-((*R*)-1-methylpyrrolidin-2-yl)-1*H*-pyrrolo[3,2-c]pyridin-6-yl)nicotinamide (**Example 12**, 0.11 g, 40%) as an off white solid. $^1$H NMR (400 MHz, DMSO-$d_6$) δ ppm 1.76-1.95 (m, 3H), 1.97-2.09 (m, 3H), 2.17 (s, 3H), 2.22-2.31 (m, 2H), 2.42-2.46 (m, 1H), 2.53-2.63 (m, 4H), 2.85-3.03 (m, 3H), 3.09-3.19 (m, 1H), 4.31-4.39 (m, 1H), 4.45-4.53 (m, 1H), 5.12-5.16 (m, 1H), 6.40 (s, 1H), 7.44 (d, *J*=8.0 Hz, 1H), 7.50-7.56 (m, 1H), 7.64-7.67 (m, 1H), 7.68-7.74 (m, 1H), 8.19 (s, 1H), 8.29-8.32 (m, 1H), 8.51 (s, 1H), 9.10 (s, 1H), 10.68 (br s, 1H), 11.00 (br s, 1H), 11.39 (br s, 1H). Mass spec: m/z: 630.0 $[M+H]^+$.

**Example 13 was synthesized following Scheme 16**

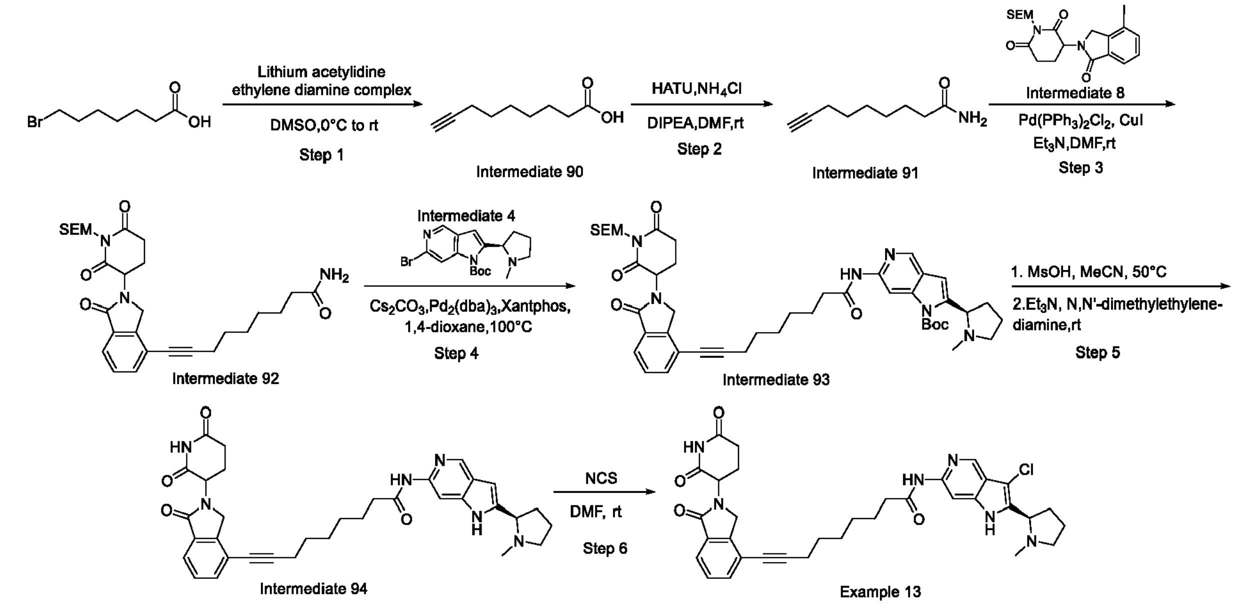


## Scheme 16

**Step 1**

**Intermediate 90: non-8-ynoic acid**

To solution of 7-bromoheptanoic acid (CAS No: 30515-28-7, 6.00 g, 28.69 mmol) in anhydrous DMSO (9 mL) was added dropwise over 30 min to a suspension of lithium acetylide ethylenediamine complex (11.74 g, 114.79 mmol) in anhydrous DMSO (90 mL) at 0°C and reaction mixture was stirred at 0°C for 1h. The reaction mixture was stirred then stirred at room temperature for 2h. The reaction mixture was quenched with 10% sulfuric acid solution (250 mL) at 0°C. The aqueous layer was extracted with hexane (4 × 100 mL) and the combined organic layer was dried over anhydrous $Na_2SO_4$, filtered, and concentrated *in vacuo* to afford non-8-ynoic acid (**Intermediate 90**, **3.**7 g, 84%) as a pink oil, which was used for the next step without further purification. $^1H$ NMR (400 MHz, $CDCl_3$) δ ppm 1.336-1.48 (m, 4H), 1.52-1.57 (m, 2H), 1.62-1.69 (m, 2H), 1.95 (s, 1H), 2.16-2.21 (m, 2H), 2.36 (t, *J*=7.45 Hz, 2H).

**Step 2**

**Intermediate 91: non-8-ynamide**

To solution of non-8-ynoic acid (**Intermediate 90,** 3.9 g, 25 mmol) in dimethylformamide (20 mL) was added HATU (15 g, 38 mmol) and *N,N*-diisopropylethylamine (18 mL, 100 mmol) and stirred for 10 min at room temperature. Ammonium chloride (4.4 mL, 130 mmol) was then added and the reaction mixture was stirred at room temperature for 16h. The reaction mixture was diluted with ice cold water (100 mL) and extracted with ethyl acetate (2 × 50 mL). The combined organic phases were dried over anhydrous $Na_2SO_4$, filtered, and concentrated *in vacuo* to obtain crude compound. The crude material was poured in water (50 mL) and stirred for 10 min, upon which time a solid precipitated, which was filtered and dried, to afford non-8-ynamide (**Intermediate 91,** 2.5 g, 65%) as an off white solid. $^1H$ NMR (400 MHz, DMSO-$d_6$) δ ppm 1.20-1.28 (m, 2H), 1.29-1.37 (m, 2H), 1.39-1.50 (m, 4H), 2.02 (t, *J*=7.45 Hz, 2H), 2.12-2.16 (m, 2H), 2.72 (s, 1H), 6.65 (br s, 1H), 7.20 (br s, 1H).

**Step 3**

**Intermediate 92: 9-(2-(2, 6-dioxo-1-((2-(trimethylsilyl)ethoxy)methyl) piperidin-3-yl)-1-oxoisoindolin-4-yl) non-8-ynamide**

To a solution of non-8-ynamide (**Intermediate 91,** 2.40 g, 15.7 mmol) in dimethylformamide (20 mL) was added 3-(4-iodo-1-oxoisoindolin-2-yl)-1-((2-(trimethylsilyl)ethoxy)methyl)piperidine-2,6-dione (**Intermediate 8,** 7.84 g, 15.7

mmol) followed by triethylamine (79 mL, 564 mmol) and the reaction mixture was purged with argon gas for 15 min. $PdCl_2(PPh_3)_2$(1.10 g, 1.57 mmol) and Copper(I) iodide (0.63 g, 3.13 mmol) were then added at room temperature and the reaction mixture was further purged with argon for 10 min and stirred at room temperature for 2h. The reaction mixture was quenched with ice cold water (100 mL) and extracted with ethyl acetate (2 × 100 mL). The combined organic phases were dried over anhydrous $Na_2SO_4$, filtered, and concentrated *in vacuo.* The crude material was purified by combi-flash column chromatography, by eluting with 11% of MeOH in DCM, to afford 9-(2-(2,6-dioxo-1-((2-(trimethylsilyl)ethoxy)methyl) piperidin-3-yl)-1-oxoisoindolin-4-yl)non-8-ynamide (**Intermediate 92,** 2.7 g, 33%) as light brown solid. $^1$H NMR (400 MHz, DMSO-$d_6$) δ ppm -0.02 (s, 9H), 0.78-0.90 (m, 2H), 1.27-1.32 (m, 2H), 1.40-1.60 (m, 6H), 1.99-2.10 (m, 3H), 2.41-2.46 (m, 3H), 2.78-2.82 (m, 1H), 3.02-3.11 (m, 1H), 3.51-3.56 (m, 2H), 4.25-4.29 (m, 1H), 4.44-4.49 (m, 1H), 5.01-5.09 (m, 2H), 5.24-5.29 (m, 1H), 6.64 (br s, 1H), 7.19 (br s, 1H), 7.48-7.57 (m, 1H), 7.64 (d, *J*=7.46 Hz, 1H), 7.72 (d, *J*=7.46 Hz, 1H). Mass spec: m/z: 523.9 $[M-H]^-$.

**Step 4**

**Intermediate 93: *tert*-butyl 6-(9-(2-(2,6-dioxo-1-((2-(trimethylsilyl)ethoxy)methyl)piperidin-3-yl)-1-oxoisoindolin-4-yl)non-8-ynamido)-2-((R)-1-methylpyrrolidin-2-yl)-1*H*-pyrrolo[3,2-c]pyridine-1-carboxylate**

To a solution of 9-(2-(2, 6-dioxo-1-((2-(trimethylsilyl) ethoxy) methyl) piperidin-3-yl)-1-oxoisoindolin-4-yl) non-8-ynamide (**Intermediate 92**, 2.45 g, 4.66 mmol) and *tert*-butyl (R)-6-bromo-2-(1-methylpyrrolidin-2-yl)-1*H*-pyrrolo[3,2-c] pyridine-1-carboxylate (**Intermediate 4**, 2.13 g, 5.59 mmol) in 1, 4-dioxane (40 mL) was added cesium carbonate (4.60 g, 14.0 mmol) and the reaction mixture was purged with argon for 15 min. $Pd_2(dba)_3$ (0.66 g, 0.69 mmol) and xantphos (0.83 g, 1.40 mmol) were added at room temperature and the reaction mixture was purged with argon gas for a further 10 min and then heated at 100°C for 2h. The reaction mixture was concentrated *in vacuo* to obtain crude compound. The crude material was purified by combi-flash column chromatography, by eluting with 7% MeOH in DCM, to afford *tert*-butyl 6-(9-(2-(2,6-dioxo-1-((2-(trimethylsilyl)ethoxy)methyl)piperidin-3-yl)-1-oxoisoindolin-4-yl)non-8-ynamido)-2-((R)-1-methylpyrrolidin-2-yl)-1*H*-pyrrolo[3,2-c]pyridine-1-carboxylate (**Intermediate 93,** 1.95 g, 51%) as a light brown solid. Mass spec: m/z: 825.1 $[M+H]^+$.

**Step 5**

**Intermediate 94: 9-(2-(2, 6-dioxopiperidin-3-yl)-1-oxoisoindolin-4-yl)-*N*-(2-((R)-1-methylpyrrolidin-2-yl)-1*H*-pyrrolo [3, 2-c] pyridin-6-yl) non-8-ynamide**

To a solution of *tert*-butyl 6-(9-(2-(2,6-dioxo-1-((2-(trimethylsilyl)ethoxy)methyl)piperidin-3-yl)-1-oxoisoindolin-4-yl)non-8-ynamido)-2-((R)-1-methylpyrrolidin-2-yl)-1*H*-pyrrolo[3,2-c]pyridine-1-carboxylate (**Intermediate 93,** 1.9 g, 2.3 mmol) in acetonitrile (25 mL) was added methanesulfonic acid (1.5 mL, 23 mmol) at room temperature and the reaction mixture was heated at 50°C for 2h. *N, N'*-dimethylethylenediamine (1.4 mL, 12 mmol) followed by triethylamine (6.5 mL, 46 mmol) were added and the reaction stirred at room temperature for 3h. The reaction mixture was quenched with water (100 mL), a solid precipitated which was filtered and washed with water (20 mL) and then dried to afford 9-(2-(2,6-dioxopiperidin-3-yl)-1-oxoisoindolin-4-yl)-*N*-(2-((R)-1-methylpyrrolidin-2-yl)-1*H*-pyrrolo[3,2-c]pyridin-6-yl) non-8-ynamide (**Intermediate 94,** 1.30 g, 95%) as an off white solid. $^1$H NMR (400 MHz, DMSO-$d_6$) δ ppm 1.32-1.38 (m, 2H), 1.42-1.48 (m, 2H), 1.55-1.70 (m, 5H), 1.76-1.90 (m, 3H), 1.99-2.05 (m, 1H), 2.08-2.11 (m, 1H), 2.14 (s, 3H), 2.21-2.28 (m, 1H), 2.35-2.39 (m, 3H), 2.54-2.67 (m, 2H), 2.84-2.96 (m, 2H), 3.12 (t, *J*=7.46 Hz, 1H), 4.29-4.36 (m, 1H), 4.43-4.48 (m, 1H), 5.11-5.16 (m, 1H), 6.33 (s, 1H), 7.48-7.52 (m, 1H), 7.62 (d, *J*=7.46 Hz, 1H), 7.69 (d, *J*=7.46 Hz, 1H), 8.07 (s, 1H), 8.40 (s, 1H), 10.08 (br s, 1H), 10.99 (br s, 1H), 11.23 (br s, 1H). Mass spec: 595.38 $[M+H]^+$.

**Step 6**

**Example 13: *N*-(3-chloro-2-((R)-1-methylpyrrolidin-2-yl)-1*H*-pyrrolo [3, 2-c] pyridin-6-yl)-9-(2-(2, 6-dioxopiperidin-3-yl)-1-oxoisoindolin-4-yl) non-8-ynamide**

To a solution of 9-(2-(2,6-dioxopiperidin-3-yl)-1-oxoisoindolin-4-yl)-*N*-(2-((R)-1-methylpyrrolidin-2-yl)-1*H*-pyrrolo[3,2-c]pyridin-6-yl)non-8-ynamide **(Intermediate 94**, 0.50 g, 0.84 mmol) in dimethylformamide (20 mL) was added *N*-chlorosuccinimide (0.17 g, 1.26 mmol) and the reaction mixture was stirred at room temperature for 16h. The reaction mixture was quenched with ice cold water (100 mL) and extracted with ethyl acetate (2 × 100mL). The combined organic phases were dried over anhydrous $Na_2SO_4$, filtered, and concentrated *in vacuo.* The crude material was purified by preparative HPLC (Method A) to afford *N*-(3-chloro-2-((R)-1-methylpyrrolidin-2-yl)-1*H*-pyrrolo[3,2-c]pyridin-6-yl)-9-(2-(2,6-dioxopiperidin-3-yl)-1-oxoisoindolin-4-yl)non-8-ynamide (**Example 13**, 0.06 g, 11%) as an off white solid.

$^1$H NMR (400 MHz, DMSO-$d_6$) δ ppm 1.33-1.40 (m, 2H), 1.43-1.52 (m, 2H), 1.55-1.65 (m, 4H), 1.80-2.02 (m, 4H), 2.14 (s, 3H), 2.27-2.34 (m, 1H), 2.36-2.41 (m, 3H), 2.57-2.60 (m, 1H), 2.85-2.93 (m, 1H), 3.13-3.18 (m, 2H), 3.45-3.52 (m, 2H), 4.29-4.34 (m, 2H), 4.43-4.48 (m, 1H), 5.11-5.16 (m, 1H), 7.50 (t, *J*=7.03 Hz, 1H), 7.63 (d, *J*=7.25 Hz, 1H), 7.70 (d, *J*=7.50 Hz, 1H), 8.13 (s, 1H), 8.40 (s, 1H), 10.26 (s, 1H), 10.98 (br s, 1H), 11.56 (br s, 1H). Mass spec: m/z: 629.2 [M+H]$^+$.

**Example 14 was synthesised following Scheme 17**

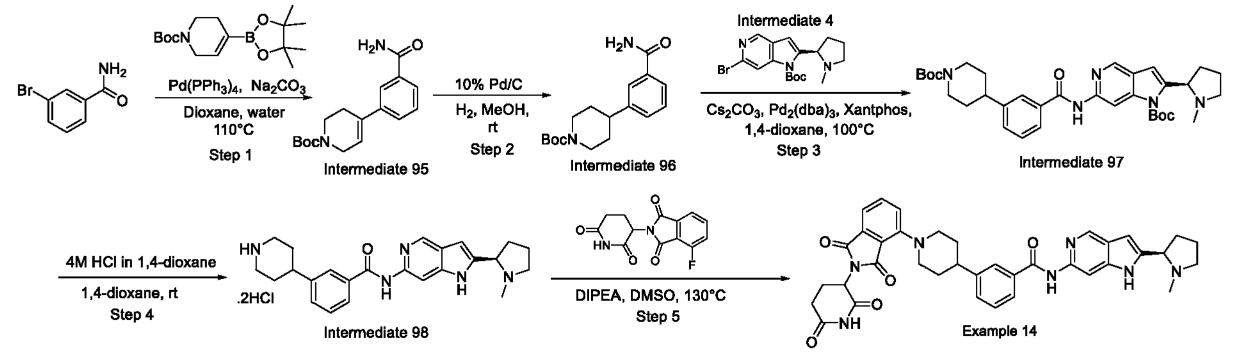


**Scheme 17**

**Step 1**

**Intermediate 95: *tert*-butyl 4-(3-carbamoylphenyl)-3,6-dihydropyridine-1(2*H*)-carboxylate**

To a solution of *tert*-butyl 4-(4,4,5,5-tetramethyl-1,3,2-dioxaborolan-2-yl)-3,6-dihydropyridine-1(2*H*)-carboxylate (CAS No: 286961-14-6, 2.80 g, 9.0 mmol) in 1,4-dioxane (16 mL) and water (4 mL) was added 3-bromobenzamide (CAS No: 22726-00-7, 1.50 g, 7.5 mmol) followed by $Na_2CO_3$ (1.7 g, 16 mmol) and the reaction mixture was purged with argon for 15 min. $Pd(PPh_3)_4$ (0.45 g, 0.37 mmol) was added and the reaction mixture was further purged with argon for 10 min and then heated at 110°C for 16h. The reaction mixture was concentrated *in vacuo* to obtain crude residue, which was purified by combi-flash chromatography, by eluting with 30% ethyl acetate in heptane, to afford tert-butyl 4-(3-carbamoylphenyl)-3, 6-dihydropyridine-1(2*H*)-carboxylate **(Intermediate 95**, 1.5 g, 66%) as an off white solid. $^1$H NMR (400 MHz, DMSO-$d_6$) δ ppm 1.43 (s, 9H), 3.29-3.36 (m, 2H), 3.52-3.56 (m, 2H), 3.95-4.10 (m, 2H), 6.21-6.24 (m, 1H), 7.37 (br s, 1H), 7.40-7.44 (m, 1H) 7.56-7.64 (m, 1H), 7.75-7.79 (m, 1H), 7.92 (s, 1H), 8.01 (br s, 1H). Mass spec: m/z: 203.03 [M+H]$^+$.

**Step 2**

**Intermediate 96: *tert*-butyl 4-(3-carbamoylphenyl) piperidine-1-carboxylate**

To a solution of *tert*-butyl 4-(3-carbamoylphenyl)-3,6-dihydropyridine-1(2*H*)-carboxylate (**Intermediate 95,** 1.5 g, 5.0 mmol) in methanol (30 mL) was added 10% Pd/C (0.4 g) and the reaction mixture was stirred at room temperature for 16h under 100 psi $H_2$ atmosphere. The reaction mixture was filtered through celite and washed with MeOH (3 × 30 mL). The filtrate was concentrated *in vacuo* to afford *tert*-butyl 4-(3-carbamoylphenyl) piperidine-1-carboxylate (**Intermediate 96,** 1.20 g, 79%) as an off white solid, which was used for the next step without further purification. $^1$H NMR (400 MHz, DMSO-$d_6$) δ ppm 1.42 (s, 9H), 1.49-1.58 (m, 2H), 1.70-1.80 (m, 2H), 2.66-2.90 (m, 3H), 4.05-4.10 (m, 2H), 7.31 (br s, 1H), 7.35-7.41 (m, 1H), 7.52-7.65 (m, 1H), 7.69-7.72 (m, 1H), 7.75 (s, 1H), 7.95 (br s, 1H).

**Step 3**

**Intermediate 97: *tert*-butyl (*R*)-6-(3-(1-(*tert*-butoxycarbonyl) piperidin-4-yl) benzamido)-2-(1-methylpyrrolidin-2-yl)-1*H*-pyrrolo [3, 2-c] pyridine-1-carboxylate**

To a solution of *tert*-butyl 4-(3-carbamoylphenyl) piperidine-1-carboxylate **(Intermediate 96**, 0.32 g, 1.05 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate **(Intermediate 4**, 0.50 g, 1.31 mmol) followed by cesium carbonate (1.29 g, 3.94 mmol) at room temperature and the reaction mixture was purged with argon for 15 min. $Pd_2(dba)_3$ (0.18 g, 0.19 mmol) and Xantphos (0.23 g, 0.39 mmol) were

added and the reaction mixture was further purged with argon for 10 min and then heated at 100°C for 2h. The reaction mixture was concentrated *in vacuo* to obtain crude residue which was purified by combi-flash chromatography, by eluting with 80% ethyl acetate in heptane, to afford *tert*-butyl (*R*)-6-(3-(1-(*tert*-butoxycarbonyl) piperidin-4-yl) benzamido)-2-(1-methylpyrrolidin-2-yl)-1*H*-pyrrolo [3, 2-c] pyridine-1-carboxylate (**Intermediate 97,** 0.38 g, 48%) as a yellow solid. $^1H$ NMR (400 MHz, DMSO-$d_6$) δ ppm 1.42 (s, 9H), 1.50-1.56 (m, 1H), 1.56-1.64 (m, 1H), 1.69 (s, 9H), 1.75-1.84 (m, 2H), 2.35 (s, 3H), 2.37-2.42 (m, 2H), 2.71-2.90 (m, 4H), 3.11-3.16 (m, 2H), 3.88-3.96 (m, 2H), 4.03-4.15 (m, 2H), ), 6.75 (s, 1H), 7.39-7.47 (m, 2H), 7.84-7.86 (m, 1H), 7.98 (s, 1H), 8.59 (s, 1H), 8.93 (s, 1H), 10.76 (br s, 1H). Mass spec: m/z: 604.49 $[M+H]^+$.

**Step 4**

**Intermediate 98: (*R*)-*N*-(2-(1-methylpyrrolidin-2-yl)-1*H*-pyrrolo[3,2-c]pyridin-6-yl)-3-(piperidin-4-yl)benzamide dihydrochloride**

To a solution of *tert*-butyl (*R*)-6-(3-(1-(*tert*-butoxycarbonyl)piperidin-4-yl)benzamido)-2-(1-methylpyrrolidin-2-yl)-1*H*-pyrrolo[3,2-c]pyridine-1-carboxylate (**Intermediate 97,** 0.35 g, 0.58 mmol) in 1,4-dioxane (5 mL) was added 4M HCl in 1,4-dioxane (10 mL) at 0°C and the reaction mixture was stirred at room temperature for 16h. The reaction mixture was concentrated *in vacuo* to afford as (*R*)-*N*-(2-(1-methylpyrrolidin-2-yl)-1H-pyrrolo[3,2-c]pyridin-6-yl)-3-(piperidin-4-yl) benzamide dihydrochloride (**Intermediate 98,** 0.35 g) as a brown solid, which was used for the next step without further purification. Mass spec: m/z: 404.32 $[M+H]^+$.

**Step 5**

**Example 14: 3-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide**

To a solution of (*R*)-*N*-(2-(1-methylpyrrolidin-2-yl)-1*H*-pyrrolo[3,2-c]pyridin-6-yl)-3-(piperidin-4-yl)benzamide dihydrochloride (**Intermediate 98,** 0.35 g, 0.86 mmol) in dimethyl sulfoxide (3 mL) was added 2-(2,6-dioxopiperidin-3-yl)-4-fluoroisoindoline-1,3-dione (CAS No: 835616-60-9, 0.24 g, 0.86 mmol) followed by *N,N*-diisopropylethylamine (0.76 mL, 4.33 mmol) and the reaction mixture was heated at 130°C for 2h. The reaction mixture was then poured into water (10 mL), resulting in a precipitate, which was filtered and dried to obtain crude compound. The crude material was purified by preparative HPLC (Method A) to afford 3-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide (**Example 14**, 0.19 g, 34%) as a yellow solid. $^1H$ NMR (400 MHz, DMSO-$d_6$) δ ppm 1.76-2.09 (m, 8H), 2.12-2.15 (m, 1H), 2.17 (s, 3H), 2.23-2.30 (m, 1H), 2.56-2.63 (m, 2H), 2.81-2.90 (m, 2H), 3.01-3.10 (m, 2H), 3.11-3.19 (m, 1H), 3.32-3.36 (m, 1H), 3.84-3.90 (m, 2H), 5.08-5.14 (m, 1H), 6.39 (s, 1H), 7.36 (d, *J*=7.20 Hz, 1H), 7.39-7.53 (m, 3H), 7.70-7.74 (m, 1H), 7.88 (d, *J*=7.23 Hz, 1H), 8.04 (s, 1H), 8.21 (s, 1H), 8.50 (s, 1H), 10.57 (s, 1H), 11.07 (s, 1H), 11.36 (s, 1H). Mass spec: m/z: 660.0 $[M+H]^+$

**Example 15 was synthesised following Scheme 18**

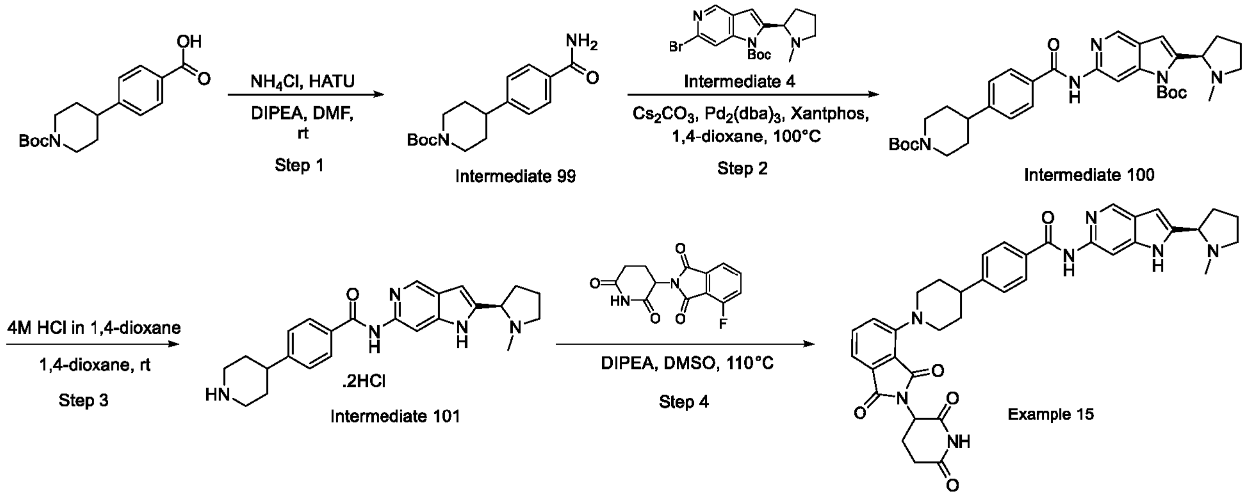


**Scheme 18**

**Step 1**

**Intermediate 99: *tert*-butyl 4-(4-carbamoylphenyl) piperidine-1-carboxylate**

To a solution of 4-(1-(*tert*-butoxycarbonyl)piperidin-4-yl)benzoic acid (CAS No: 149353-75-3, 1.5 g, 4.9 mmol) in dimethylformamide (30 mL) was added HATU (2.9 g, 7.4 mmol) and *N, N*-diisopropylethylamine (3.4 mL, 20 mmol) at room temperature and stirred for 10 min. Ammonium chloride (1.1 g, 20 mmol) was added at 0°C and the reaction mixture was stirred at room temperature for 16h. The reaction mixture was poured into water (100 mL) to obtain the solid precipitate which was filtered and dried under vacuum to afford *tert*-butyl 4-(4-carbamoylphenyl) piperidine-1-carboxylate **(Intermediate 99,** 1.30 g, 87%) as a white solid, which was used in the next step without further purification. Mass spec: m/z 205.08 [M-Boc]$^+$.

**Step 2**

**Intermediate 100: *tert*-butyl (*R*)-6-(4-(1-(*tert*-butoxycarbonyl)piperidin-4-yl)benzamido)-2-(1-methylpyrrolidin-2-yl)-1*H*-pyrrolo [3, 2-c] pyridine-1-carboxylate**

To a solution of *tert*-butyl 4-(4-carbamoylphenyl)piperidine-1-carboxylate **(Intermediate 99**, 0.32 g, 1.05 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate **(Intermediate 4**, 0.50 g, 1.31 mmol) followed by cesium carbonate (0.86 g, 2.63 mmol) at room temperature and the mixture was purged with argon for 15 min. $Pd_2(dba)_3$ (0.18 g, 0.19 mmol) and Xantphos (0.23 g, 0.39 mmol) were added and the reaction mixture was purged with argon for a further 10 min and then heated at 100°C for 2h. The reaction mixture was concentrated *in vacuo* to obtain the crude compound which was then was purified by combi-flash column chromatography, by eluting with 80% ethyl acetate in heptane, to afford *tert*-butyl (*R*)-6-(4-(1-(*tert*-butoxycarbonyl) piperidin-4-yl)benzamido)-2-(1-methylpyrrolidin-2-yl)-1*H*-pyrrolo[3,2-c]pyridine-1-carboxylate (**Intermediate 100,** 0.51 g, 64%) as a yellow solid. $^1$H NMR (400 MHz, DMSO-$d_6$) δ ppm 1.42 (s, 9H), 1.50-1.56 (m, 1H), 1.56-1.64 (m, 1H), 1.69 (s, 9H), 1.75-1.84 (m, 2H), 2.35 (s, 3H), 2.37-2.42 (m, 2H), 2.71-2.90 (m, 4H), 3.11-3.16 (m, 2H), 3.88-3.96 (m, 2H), 4.03-4.15 (m, 2H), 6.75 (s, 1H), 7.38 (d, *J*=7.88Hz, 2H), 7.98 (d, *J*=7.88 Hz, 2H), 8.58 (s, 1H), 8.92 (s, 1H), 10.61 (s, 1H). Mass spec: m/z: 604.57 [M+H]$^+$.

**Step 3**

**Intermediate 101: (R)-*N*-(2-(1-methylpyrrolidin-2-yl)-1*H*-pyrrolo [3, 2-c] pyridin-6-yl)-4-(piperidin-4-yl) benzamide dihydrochloride**

To a solution of tert-butyl (*R*)-6-(4-(1-(*tert*-butoxycarbonyl)piperidin-4-yl)benzamido)-2-(1-methylpyrrolidin-2-yl)-1*H*-pyrrolo[3, 2-c]pyridine-1-carboxylate (**Intermediate 100**, 0.4 g, 0.66 mmol) in 1,4-dioxane (3 mL) was added 4M HCl in 1,4-dioxane (5 mL) at 0°C and the reaction mixture was stirred at room temperature for 16h. The reaction mixture was concentrated *in vacuo* to afford (*R*)-*N*-(2-(1-methylpyrrolidin-2-yl)-1*H*-pyrrolo [3, 2-c] pyridin-6-yl)-4-(piperidin-4-yl)

benzamide dihydrochloride (**Intermediate 101,** 0.3 g) as an off white solid, which was used for the next step without further purification. $^1$H NMR (400 MHz, DMSO-$d_6$) δ ppm 1.86-2.02 (m, 3H), 2.12-2.26 (m, 2H), 2.33-2.44 (m, 1H), 2.53 (s, 3H), 2.74-2.86 (m, 2H), 2.90-3.08 (m, 2H), 3.22-3.26 (m, 1H), 3.34-3.50 (m, 3H), 3.68-3.80 (m, 2H), 7.26 (s, 1H), 7.46 (d, *J*=7.88 Hz, 2H), 8.18 (d, *J*=7.88 Hz, 1H), 8.27 (s, 1H), 8.92-9.08 (m, 2H), 11.41 (br s, 1H), 11.94 (br s, 1H), 13.20 (br s, 1H). $^1$H NMR (400 MHz, DMSO-$d_6$) δ ppm 1.86-2.02 (m, 3H), 2.12-2.26 (m, 2H), 2.33-2.44 (m, 1H), 2.53 (s, 3H), 2.74-2.86 (m, 2H), 2.90-3.08 (m, 2H), 3.22-3.26 (m, 1H), 3.34-3.50 (m, 3H), 3.68-3.80 (m, 2H), 7.26 (s, 1H), 7.46 (d, *J*=7.88 Hz, 2H), 8.18 (d, *J*=7.88 Hz, 1H), 8.27 (s, 1H), 8.92-9.08 (m, 2H), 11.41 (br s, 1H), 11.94 (br s, 1H), 13.20 (br s, 1H). Mass spec: m/z: 404.34 $[M+H]^+$.

**Step 4**

**Example 15: 4-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide**

To a solution of of (*R*)-N-(2-(1-methylpyrrolidin-2-yl)-1*H*-pyrrolo[3,2-c]pyridin-6-yl)-4-(piperidin-4-yl) benzamide dihydrochloride (**Intermediate 101,** 0.30 g, 0.74 mmol) in dimethyl sulfoxide (2 mL) was added 2-(2,6-dioxopiperidin-3-yl)-4-fluoroisoindoline-1,3-dione (CAS No: 835616-60-9, 0.20 g, 0.74 mmol) followed by DIPEA (0.65 mL, 3.72 mmol) and the reaction mixture was heated at 110°C for 2h. The reaction mixture was poured into water (10 mL), resulting in a precipitate which was filtered and dried to obtain crude compound. The crude material was purified by preparative HPLC (Method A) to afford 4-(1-(2-(2,6-dioxopiperidin-3-yl)-1,3-dioxoisoindolin-4-yl)piperidin-4-yl)-*N*-(2-((*R*)-1-methylpyrrolidin-2-yl)-1H-pyrrolo[3,2-c]pyridin-6-yl)benzamide (**Example 15**, 0.15 g, 31%) as a yellow solid. $^1$H NMR (400 MHz, DMSO-$d_6$) δ ppm 1.76-1.98 (m, 8H), 2.01-2.08 (m, 1H), 2.12-2.14 (m, 1H), 2.17 (s, 3H), 2.22-2.30 (m, 1H), 2.55-2.64 (m, 2H), 2.80-2.94 (m, 2H), 2.99-3.09 (m, 2H), 3.09-3.17 (m, 1H), 3.82-3.88 (m, 2H), 5.08-5.16 (m, 1H), 6.39 (s, 1H), 7.35-7.45 (m, 4H), 7.70-7.74 (m, 1H), 8.01-8.04 (m, 2H), 8.20 (s, 1H), 8.50 (s, 1H), 10.39 (s, 1H), 11.09 (s, 1H), 11.36 (s, 1H). Mass spec: m/z: 660.05$[M+H]^+$.

**Example 16 was synthesised following Scheme 19**

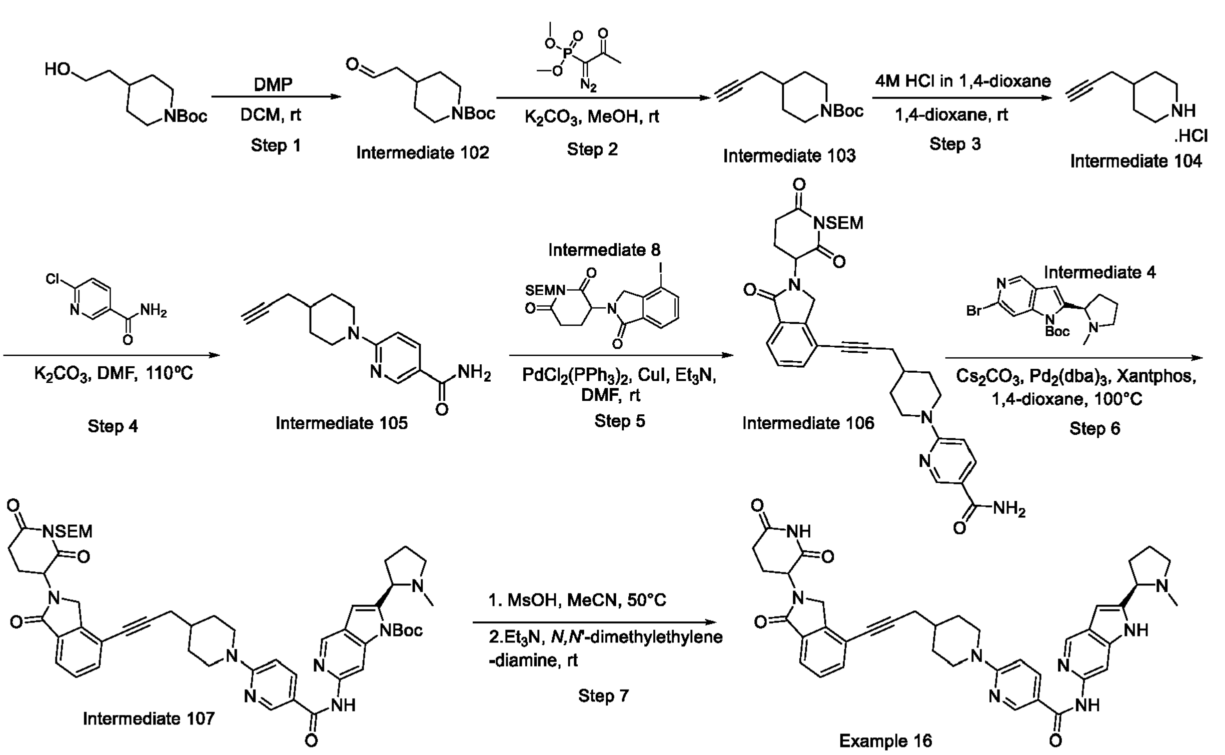


**Scheme 19**

**Step 1**

**Intermediate 102: *tert*-butyl 4-(2-oxoethyl) piperidine-1-carboxylate**

To a solution of *tert*-butyl 4-(2-hydroxyethyl)piperidine-1-carboxylate (CAS No: 89151-44-0, 10.0 g, 43.6 mmol) in dichloromethane (150 mL) was added Dess-Martin Periodinane (27.7 g, 65.4 mmol) at 0°C and the reaction mixture was stirred at room temperature for 16h under a nitrogen atmosphere. The reaction mixture was quenched with saturated $NH_4Cl$ solution (10 mL) and extracted with dichloromethane (3 × 200 mL). The combined organic layers were dried over anhydrous $Na_2SO_4$, filtered, and concentrated *in vacuo* to afford *tert*-butyl 4-(2-oxoethyl) piperidine-1-carboxylate **(Intermediate 102,** 7.0 g) as colourless oil, which was used for the next step without further purification. $^1H$ NMR (400 MHz, DMSO-$d_6$) δ ppm 0.92-1.10 (m, 2H), 1.35 (s, 9H), 1.52-1.62 (m, 2H), 1.90-2.00 (m, 1H), 2.30-2.38 (m, 2H), 2.62-2.78 (m, 2H), 3.82-3.96 (m, 2H), 9.63 (s, 1H).

**Step 2**

**Intermediate 103: *tert*-butyl 4-(prop-2-yn-1-yl) piperidine-1-carboxylate**

To a solution of *tert*-butyl 4-(2-oxoethyl) piperidine-1-carboxylate (**Intermediate 102,** 6.50 g, 29.0 mmol) in methanol (80 mL) was added potassium carbonate (5.9 g, 43.0 mmol) at 0°C followed by dimethyl (1-diazo-2-oxopropyl) phosphonate (5.5 g, 29.0 mmol) and the reaction mixture was stirred at 0°C for 3h. The reaction mixture was diluted with water (500 mL) and extracted with ethyl acetate (3 × 200 mL). The combined organic phases were dried over anhydrous $Na_2SO_4$, filtered, and concentrated *in vacuo.* The crude material was purified by combi-flash column chromatography, by eluting with 80% ethyl acetate in heptane, to afford *tert*-butyl 4-(prop-2-yn-1-yl) piperidine-1-carboxylate (**Intermediate 103,** 4.5 g, 70%) as a yellow liquid. $^1H$ NMR (400 MHz, DMSO-$d_6$) δ ppm 0.96-1.11 (m, 2H), 1.36 (s, 9H), 1.47-1.71 (m, 3H), 2.02-2.18 (m, 2H), 2.56-2.72 (m, 2H), 2.77 (s, 1H), 3.81-4.00 (m, 2H).

**Step 3**

**Intermediate 104: 4-(prop-2-yn-1-yl)piperidine hydrochloride**

To a solution of tert-butyl 4-(prop-2-yn-1-yl)piperidine-1-carboxylate (**Intermediate 103**, 4.5 g, 20.0 mmol) in 1, 4-dioxane (10 mL) was added 4M HCl in 1, 4-dioxane (40 mL) at 0°C and the reaction mixture was stirred at room temperature for 16h. The reaction mixture was concentrated *in vacuo* to afford 4-(prop-2-yn-1-yl)piperidine hydrochloride (**Intermediate 104,** 4.0 g) as brown solid, which was used for the next step without further purification. $^1H$ NMR (400 MHz, DMSO-$d_6$) δ ppm 1.33-1.48 (m, 2H), 1.43-71 (m, 1H), 1.79-1.85 (m, 2H), 2.15-2.18 (m, 2H), 2.82 (t, *J*=11.6 Hz, 2H), 2.89 (s, 1H), 3.21-3.25 (m, 2H), 8.62 (br s, 1H), 8.89 (br s, 1H).

**Step 4**

**Intermediate 105: 6-(4-(prop-2-yn-1-yl) piperidin-1-yl)nicotinamide**

To a solution of 4-(prop-2-yn-1-yl)piperidine (**Intermediate 104,** 2.50 g, 20.0 mmol) and 6-chloronicotinamide (CAS No: 6271-78-9, 3.2 g, 20.0 mmol) in dimethylformamide (25 mL) was added potassium carbonate (11.0 g, 81.0 mmol) and the reaction mixture was heated at 110°C for 16h. The reaction mixture was poured into ice cold water (200 mL), resulting in a precipitate, which was filtered and dried *in vacuo* to afford 6-(4-(prop-2-yn-1-yl) piperidin-1-yl) nicotinamide (**Intermediate 105,** 1.70 g, 34%) as an off white solid, which was used in the next step without further purification. $^1H$ NMR (400 MHz, DMSO-$d_6$) δ ppm 1.11-1.25 (m, 2H), 1.69-1.83 (m, 3H), 2.14-2.16 (m, 2H), 2.76-2.85 (m, 2H), 2.85 (s, 1H), 4.39-4.44 (m, 2H), 6.82 (d, *J*=9.20 Hz, 1H), 7.08 (br s, 1H), 7.71 (br s, 1H), 7.92 (d, *J*=8.80 Hz, 1H), 8.59 (s, 1H). Mass spec: m/z: 244.12 $[M+H]^+$.

**Step 5**

**Intermediate 106: 6-(4-(3-(2-(2,6-dioxo-1-((2-(trimethylsilyl)ethoxy)methyl)piperidin-3-yl)-1-oxoisoindolin-4-yl) prop-2-yn-1-yl) piperidin-1-yl) nicotinamide**

To a solution of 6-(4-(prop-2-yn-1-yl)piperidin-1-yl)nicotinamide (**Intermediate 105,** 0.5 g, 2.05 mmol) in dimethylformamide (5 mL) was added 3-(4-iodo-1-oxoisoindolin-2-yl)-1-((2-(trimethylsilyl)ethoxy)methyl)piperidine-2,6-dione (**Intermediate 8,** 1.02 g, 2.05 mmol) followed by triethylamine (10.7 mL, 76.04 mmol) and the reaction mixture was purged with argon gas for 15 min. $PdCl_2(PPh_3)_2$ (0.14 g, 0.20 mmol) and copper(I) iodide (0.03 g, 0.20 mmol) were added and the reaction mixture was further purged with argon gas for 10 min and stirred at room temperature for 3h.

The reaction mixture was then poured into water (60 mL) and extracted with ethyl acetate (3 × 100 mL). The combined organic phases were dried over anhydrous $Na_2SO_4$, filtered, and concentrated *in vacuo.* The crude material was purified by combi-flash column chromatography, by eluting with 100% ethyl acetate, to afford 6-(4-(3-(2-(2, 6-dioxo-1-((2-(trimethylsilyl)ethoxy) methyl) piperidin-3-yl)-1-oxoisoindolin-4-yl) prop-2-yn-1-yl) piperidin-1-yl)nicotinamide (**Intermediate 106,** 0.75 g, 59%) as a yellow solid. $^1$H NMR (400 MHz, DMSO-$d_6$) δ ppm -0.03 (s, 9H), 0.79-0.89 (m, 2H), 1.21-1.33 (m, 2H), 1.80-1.93 (m, 3H), 2.01-2.10 (m, 1H), 2.35-2.47 (m, 2H), 2.75-2.94 (m, 3H), 2.98-3.13 (m, 2H), 3.48-3.58 (m, 2H), 4.25-4.29 (m, 1H), 4.39-4.50 (m, 3H), 4.98-5.13 (m, 2H), 5.23-5.27 (m, 1H), 6.82 (d, *J*=9.20 Hz, 1H), 7.08 (br s, 1H), 7.52 (t, *J*=7.60 Hz, 1H), 7.63 (d, *J*=7.20 Hz, 1H), 7.71 (d, *J*=7.20 Hz, 2H), 7.91-7.94 (m,1H), 8.59 (s, 1H). Mass spec: m/z: 616.41 $[M+H]^+$.

**Step 6**

**Intermediate 107: *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperidin-1-yl)nicotinamido)-2-((*R*)-1-methylpyrrolidin-2-yl)-1*H*-pyrrolo[3,2-c]pyridine-1-carboxylate**

To a solution of 6-(4-(3-(2-(2, 6-dioxo-1-((2-(trimethylsilyl) ethoxy) methyl) piperidin-3-yl)-1-oxoisoindolin-4-yl) prop-2-yn-1-yl) piperidin-1-yl)nicotinamide (**Intermediate 106**, 0.51 g, 0.84 mmol) ) in 1,4-dioxane (15 mL) was added *tert*-butyl (R)-6-bromo-2-(1-methylpyrrolidin-2-yl)-1*H*-pyrrolo[3,2-c]pyridine-1-carboxylate (**Intermediate 4,** 0.4 g, 1.05 mmol) followed by cesium carbonate (0.68 g, 2.10 mmol) at room temperature and the reaction mixture was purged with argon for 15 min. $Pd_2(dba)_3$ (0.14 g, 0.15 mmol) and Xantphos (0.18 g, 0.31 mmol) were added and the reaction mixture was purged with argon for 10 min and then heated at 100°C for 2h. The reaction mixture was concentrated *in vacuo* and the crude material was purified by combi-flash column chromatography, by eluting with 80% ethyl acetate in heptane, to afford *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperidin-1-yl)nicotinamido)-2-((*R*)-1-methylpyrrolidin-2-yl)-1*H*-pyrrolo[3,2-c]pyridine-1-carboxylate (**Intermediate 107**, 0.41 g, 43%) as a yellow solid. $^1$H NMR (400 MHz, DMSO-$d_6$) δ ppm -0.03 (s, 9H), 0.78-0.88 (m, 2H), 1.23-1.35 (m, 2H), 1.54-1.64 (m, 1H), 1.69 (s, 9H), 1.72-1.80 (m, 2H), 1.86-1.92 (m, 4H), 2.01-2.10 (m, 1H), 2.28-2.32 (m, 1H), 2.35 (s, 3H), 2.38-2.45 (m, 2H), 2.76-2.82 (m, 1H), 2.90-2.96 (m, 2H), 3.00-3.19 (m, 3H), 3.46-3.59 (m, 2H), 3.89-3.92 (m, 1H), 4.26-4.30 (m, 1H), 4.41-4.55 (m, 3H), 5.01-5.10 (m, 2H), 5.23-5.28 (m, 1H), 6.74 (s, 1H), 6.88 (d, *J*=9.20 Hz, 1H), 7.49-7.57 (m, 1H), 7.65 (d, *J*=7.89 Hz, 1H), 7.72 (d, *J*=7.89 Hz, 1H), 8.14 (d, J=7.45 Hz, 1H), 8.57 (s, 1H), 8.79 (s, 1H), 8.91 (s, 1H), 10.47 (s, 1H). Mass spec: m/z: 815.5 $[M+H]^+$

**Step 7**

**Example 16: 6-(4-(3-(2-(2,6-dioxopiperidin-3-yl)-1-oxoisoindolin-4-yl)prop-2-yn-1-yl)piperidin-1-yl)-*N*-(2-((*R*)-1-methylpyrrolidin-2-yl)-1*H*-pyrrolo[3,2-c]pyridin-6-yl)nicotinamide**

To a solution of *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperidin-1-yl)nicotinamido)-2-((*R*)-1-methylpyrrolidin-2-yl)-1*H*-pyrrolo[3,2-c]pyridine-1-carboxylate (**Intermediate 107,** 0.40 g, 0.43 mmol) in acetonitrile (10 mL) was added methanesulfonic acid (0.43 mL, 6.55 mmol) at 0°C and the reaction heated at 50°C for 2h. *N,N'*-dimethylethylenediamine (0.31 mL, 2.62 mmol) and triethylamine (0.88 g, 8.74 mmol) were added at room temperature and the reaction stirred for 2h. The reaction mixture was concentrated *in vacuo* water (100 mL) was added, resulting in a precipitate which was filtered and dried *in vacuo.* The crude material was purified by preparative HPLC (Method A) to afford 6-(4-(3-(2-(2,6-dioxopiperidin-3-yl)-1-oxoisoindolin-4-yl)prop-2-yn-1-yl)piperidin-1-yl)-*N*-(2-((*R*)-1-methylpyrrolidin-2-yl)-1*H*-pyrrolo[3,2-c]pyridin-6-yl)nicotinamide (**Example 16,** 0.11 g, 37%). $^1$H NMR (400 MHz, DMSO-$d_6$) δ ppm 1.22-1.36 (m, 2H), 1.76-1.96 (m, 6H), 1.97-2.06 (m, 1H), 2.10-2.14 (m, 1H), 2.16 (s, 3H), 2.23-2.29 (m, 1H), 2.40-2.45 (m, 1H), 2.58-2.62 (m, 2H), 2.85-2.99 (m, 3H), 3.08-3.18 (m, 1H), 3.26-3.30 (m, 1H), 3.34-3.36 (m, 1H), 4.27-4.37 (m, 1H), 4.40-4.55 (m, 3H), 5.10-5.15 (m, 1H), 6.37 (s, 1H), 6.88 (d, *J*=9.13 Hz, 1H), 7.48-7.55 (m, 1H), 7.64-7.79 (m, 2H), 8.12-8.17 (m, 2H), 8.48 (s, 1H), 8.79 (m, 1H), 10.23 (s, 1H), 11.00 (s, 1H), 11.32 (s, 1H). Mass spec: m/z: 685.05 $[M+H]^+$.

**Example 17 was synthesised following Scheme 20**

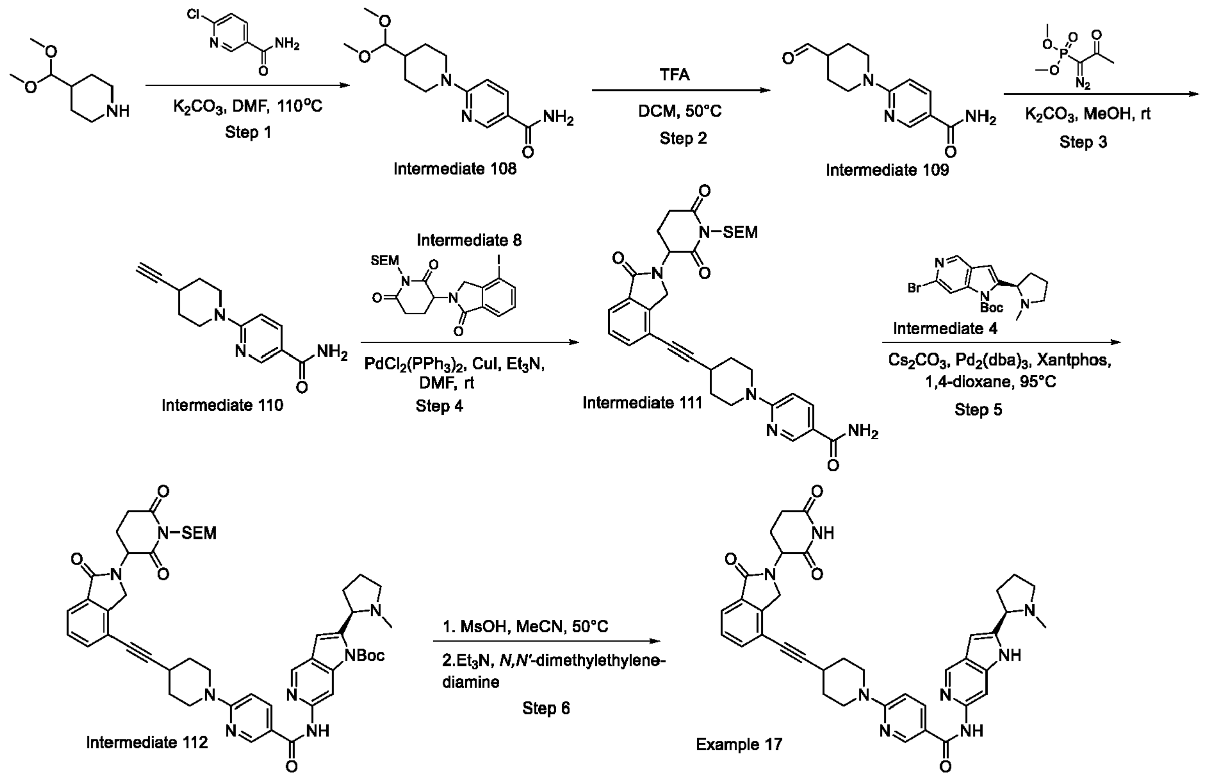


## Scheme 20

### Step 1

**Intermediate 108: 6-(4-(dimethoxymethyl)piperidin-1-yl)nicotinamide**

To a solution of 6-chloronicotinamide (CAS No: 6271-78-9, 4.00 g, 25.5 mmol) in dimethylformamide (50 mL) were added potassium carbonate (10.6 g, 76.6 mmol) and 4-(dimethoxymethyl)piperidine (CAS No: 188646-83-5, 4.47 g, 28.1 mmol) at room temperature and the reaction mixture was heated at 110°C for 16h. The reaction mixture was poured into ice cold water (400 mL), a solid precipitated, which was filtered and dried *in vacuo* to afford 6-(4-(dimethoxymethyl) piperidin-1-yl)nicotinamide (**Intermediate 108,** 5.5 g, 77%) as an off white solid, which was used for the next step without further purification. $^1$H NMR (400 MHz, DMSO-$d_6$) δ ppm 1.10-1.23 (m, 2H), 1.66-1.72 (m, 2H), 1.81-1.93 (m, 1H), 2.79-2.85 (m, 2H), 3.26 (s, 6H), 4.01-4.10 (m, 1H), 4.38-4.44 (m, 2H), 6.81 (d, *J*=9.12 Hz, 1H), 7.08 (br s, 1H), 7.71 (br s, 1H), 7.92 (d, *J*=9.12 Hz, 1H), 8.58 (s, 1H). Mass spec: m/z 280.19 [M+H]$^+$.

### Step 2

**Intermediate 109: 6-(4-formylpiperidin-1-yl)nicotinamide**

To a solution of 6-(4-(dimethoxymethyl)piperidin-1-yl)nicotinamide (**Intermediate 108,** 5.5 g, 20.0 mmol) in dichloromethane (60 mL) was added trifluoroacetic acid (15 mL) at 0°C and the reaction mixture was heated at 50°C for 2h. the reaction mixture was basified with saturated $NaHCO_3$ (200 mL) solution to pH~8 and extracted with ethyl acetate (3 × 150 mL). The combined organic layers were dried over anhydrous $Na_2SO_4$, and filtered and concentrated *in vacuo* to afford 6-(4-formylpiperidin-1-yl)nicotinamide (**Intermediate 109,** 4.5 g, 98%) as a gum, which was used for the next step without further purification. $^1$H NMR (400 MHz, DMSO-$d_6$) δ ppm 1.40-1.54 (m, 2H), 1.84-1.93 (m, 2H), 2.58-2.67 (m, 1H), 3.10-3.16 (m, 2H), 4.20-4.26 (m, 2H), 6.85 (d, *J*=8.77 Hz, 1H), 7.10 (br s, 1H), 7.73 (br s, 1H), 7.93 (d, *J*=8.77, 1H), 8.60 (s, 1H), 9.61 (s, 1H). Mass spec: m/z: 234.4 [M+H]$^+$.

### Step 3

**Intermediate 110: 6-(4-ethynylpiperidin-1-yl) nicotinamide**

To a solution of 6-(4-formylpiperidin-1-yl)nicotinamide (**Intermediate 109,** 4.5 g, 19.0 mmol) in methanol (60 mL) was added potassium carbonate (5.6 g, 39.0 mmol) at 0°C followed by dimethyl (1-diazo-2-oxopropyl)phosphonate (5.6 g, 29.0 mmol) and the reaction mixture was stirred at room temperature for 2h. The reaction mixture was diluted with water (120 mL) and extracted with ethyl acetate (3 × 150 mL). The combined organic layers were separated, dried over anhydrous $Na_2SO_4$, filtered, and concentrated *in vacuo.* The crude material was purified by combi-flash column chromatography, by eluting with 8% MeOH in DCM, to afford 6-(4-ethynylpiperidin-1-yl)nicotinamide (**Intermediate 110,** 3.0 g, 68%) as an off white solid. $^1H$ NMR (400 MHz, DMSO-$d_6$) δ ppm 1.43-1.56 (m, 2H), 1.78-1.84 (m, 2H), 2.65-2.74 (m, 1H), 2.95 (s, 1H), 3.32-3.36 (m, 2H), 3.92-4.04 (m, 2H), 6.84 (d, *J*=9.21 Hz, 1H), 7.10 (br s, 1H), 7.73 (br s, 1H), 7.93 (d, *J*=7.89 Hz, 1H), 8.60 (s, 1H). Mass spec: m/z: 230.11 $[M+H]^+$.

**Step 4**

**Intermediate 111: 6-(4-((2-(2, 6-dioxo-1-((2-(trimethylsilyl) ethoxy) methyl) piperidin-3-yl)-1-oxoisoindolin-4-yl) ethynyl) piperidin-1-yl)nicotinamide**

To a solution of 6-(4-ethynylpiperidin-1-yl)nicotinamide (**Intermediate 110,** 0.22 g, 0.99 mmol) and 3-(4-iodo-1-oxoisoindolin-2-yl)-1-((2-(trimethylsilyl)ethoxy)methyl)piperidine-2,6-dione (**Intermediate 8,** 0.5 g, 0.99 mmol) in dimethylformamide (2 mL) was added triethylamine (6 mL, 36.97 mmol) and the reaction mixture was purged with argon gas for 15 min. $Pd(PPh_3)_2Cl_2$ (0.11 g, 0.14 mmol) and copper (I) iodide (0.02 g, 0.09 mmol) were added and the reaction mixture was further purged with argon gas for 10 min and stirred at room temperature for 1h. The reaction mixture was diluted with water (50 mL) and extracted with ethyl acetate (2 × 100 mL). The combined organic layers were dried over anhydrous $Na_2SO_4$, filtered, and concentrated *in vacuo.* The crude material was purified by combi-flash column chromatography, by eluting with 8% MeOH in DCM, to afford 6-(4-((2-(2, 6-dioxo-1-((2-(trimethylsilyl) ethoxy) methyl) piperidin-3-yl)-1-oxoisoindolin-4-yl) ethynyl) piperidin-1-yl) nicotinamide (**Intermediate 111,** 0.30 g, 50%) as a yellow solid. $^1H$ NMR (400 MHz, DMSO-$d_6$) δ ppm -0.04 (s, 9H), 0.77-0.89 (m, 2H), 1.15-1.19 (m, 4H), 1.61-1.66 (m, 2H), 2.01-2.10 (m, 2H), 2.76-2.82 (m, 1H), 2.97-3.08 (m, 2H), 3.37-3.45 (m, 2H), 3.46-3.58 (m, 2H), 4.25-4.29 (m, 1H), 4.44-4.50 (m, 1H), 5.00-5.10 (m, 2H), 5.23-5.28 (m, 1H), 6.86 (d, *J*=8.77 Hz, 1H), 7.10 (br s, 1H), 7.50-7.57 (m, 1H), 7.65-7.67 (m, 1H), 7.71-7.73 (m, 1H), 7.94 (d, *J*=8.33 Hz, 1H), 8.60 (br s, 1H). Mass spec: m/z: 602.36 $[M+H]^+$.

**Step 5**

**Intermediate 112: *tert*-butyl 6-(6-(4-((2-(2,6-dioxo-1-((2-(trimethylsilyl)ethoxy)methyl)piperidin-3-yl)-1-oxoisoindolin-4-yl)ethynyl)piperidin-1-yl)nicotinamido)-2-((*R*)-1-methylpyrrolidin-2-yl)-1*H*-pyrrolo[3,2-c]pyridine-1-carboxylate**

To a mixture of 6-(4-((2-(2,6-dioxo-1-((2-(trimethylsilyl)ethoxy)methyl) piperidin-3-yl)-1-oxoisoindolin-4-yl) ethynyl)piperidin-1-yl)nicotinamide (**Intermediate 111,** 0.22 g, 0.57 mmol) and *tert*-butyl (*R*)-6-bromo-2-(1-methylpyrrolidin-2-yl)-1*H*-pyrrolo[3,2-c]pyridine-1-carboxylate (**Intermediate 4**, 0.27 g, 0.46 mmol) in 1,4-dioxane (3 mL) was added cesium carbonate (0.29 g, 0.86 mmol) at room temperature and the reaction mixture was purged with argon for 10 min followed. $Pd_2(dba)_3$ (0.07 g, 0.08 mmol) and Xantphos (0.10 g, 0.17 mmol) were added and the reaction mixture was further purged with argon for 10 min and then heated at 95°C for 1h. The reaction mixture was concentrated *in vacuo* and the crude material was purified by combi-flash column chromatography, by eluting with 8% MeOH in DCM, to afford *tert*-butyl 6-(6-(4-((2-(2,6-dioxo-1-((2-(trimethylsilyl)ethoxy)methyl)piperidin-3-yl)-1-oxoisoindolin-4-yl)ethynyl)piperidin-1-yl)nicotinamido)-2-((*R*)-1-methylpyrrolidin-2-yl)-1*H*-pyrrolo[3,2-c]pyridine-1-carboxylate (**Intermediate 112,** 0.35 g, 67%) as a yellow solid. $^1H$ NMR (400 MHz, DMSO-$d_6$) δ ppm -0.04 (s, 9H), 0.77-0.88 (m, 2H), 1.16-1.20 (m, 1H), 1.58-1.66 (m, 4H), 1.69 (s, 9H), 1.73-1.79 (m, 2H), 1.93-1.99 (m, 2H), 2.02-2.10 (m, 1H), 2.30-2.33 (m, 1H), 2.35 (s, 3H), 2.75-2.83 (m, 1H), 3.00-3.17 (m, 3H), 3.41-3.57 (m, 4H), 3.89-3.94 (m, 1H), 4.03-4.14 (m, 2H), 4.26-4.30 (m, 1H), 4.46-4.52 (m, 1H), 4.99-5.10 (m, 2H), 5.24-5.28 (m, 1H), 6.74 (s, 1H), 6.91 (d, J=9.29 Hz, 1H), 7.50-7.56 (m, 1H), 7.66-7.75 (m, 2H), 8.14-8.17 (m, 1H), 8.57 (s, 1H), 8.80 (s, 1H), 8.90 (s, 1H), 10.49 (s, 1H). Mass spec: m/z: 901.55 $[M+H]^+$.

**Step 6**

**Example 17: 6-(4-((2-(2,6-dioxopiperidin-3-yl)-1-oxoisoindolin-4-yl)ethynyl)piperidin-1-yl)-*N*-(2-((R)-1-methylpyrrolidin-2-yl)-1*H*-pyrrolo[3,2-c]pyridin-6-yl)nicotinamide**

To a solution of *tert*-butyl 6-(6-(4-((2-(2,6-dioxo-1-((2-(trimethylsilyl)ethoxy)methyl)piperidin-3-yl)-1-oxoisoindolin-4-yl)ethynyl)piperidin-1-yl)nicotinamido)-2-((*R*)-1-methylpyrrolidin-2-yl)-1*H*-pyrrolo[3,2-c]pyridine-1-carboxylate (**Intermediate 112,** 0.35 g, 0.38 mmol) in acetonitrile (3.5 mL) was added methanesulfonic acid (0.39 g, 3.88 mmol) at 0°C and then heated at 50°C for 2h. *N, N'*-dimethylethylenediamine (0.21 g, 2.33 mmol) and triethylamine (0.82 g, 7.76 mmol) were then added and the reaction mixture was stirred at room temperature for 2h. The reaction mixture was concentrated *in vacuo* and the resulting mixture was quenched with water (50 mL), affording a precipitate, which was filtered and dried *in vacuo*. The crude material was then purified by preparative HPLC (Method A) to afford 6-(4-((2-(2,6-dioxopiperidin-3-yl)-1-oxoisoindolin-4-yl)ethynyl)piperidin-1-yl)-*N*-(2-((R)-1-methylpyrrolidin-2-yl)-1*H*-pyrrolo[3,2-c]pyridin-6-yl)nicotinamide (**Example 17**, 0.11 g, 31%) as an off white solid. $^1H$ NMR (400 MHz, DMSO-$d_6$) δ ppm 1.61-1.73 (m, 2H), 1.75-1.92 (m, 3H), 1.93-2.05 (m, 3H), 2.11-2.14 (m, 1H), 2.16 (s, 3H), 2.21-2.30 (m, 1H), 2.43-2.47 (m, 1H), 2.56-2.63 (m, 1H), 2.84-2.96 (m, 1H), 3.03-3.09 (m, 1H), 3.11-3.19 (m, 1H), 3.24-3.28 (m, 1H), 3.40-3.52 (m, 2H), 4.04-4.15 (m, 2H), 4.28-4.38 (m, 1H), 4.40-4.51 (m, 1H), 5.10-5.15 (m, 1H), 6.38 (s, 1H), 6.91 (d, *J*=9.01 Hz, 1H), 7.48-7.56 (m, 1H), 7.63-7.75 (m, 2H), 8.13-8.20 (m, 2H), 8.48 (s, 1H), 8.80 (s, 1H), 10.26 (s, 1H), 10.97 (s, 1H), 11.33 (s, 1H). Mass spec: m/z: 671.05 $[M+H]^+$.

**Example 18 was synthesised following Scheme 21**

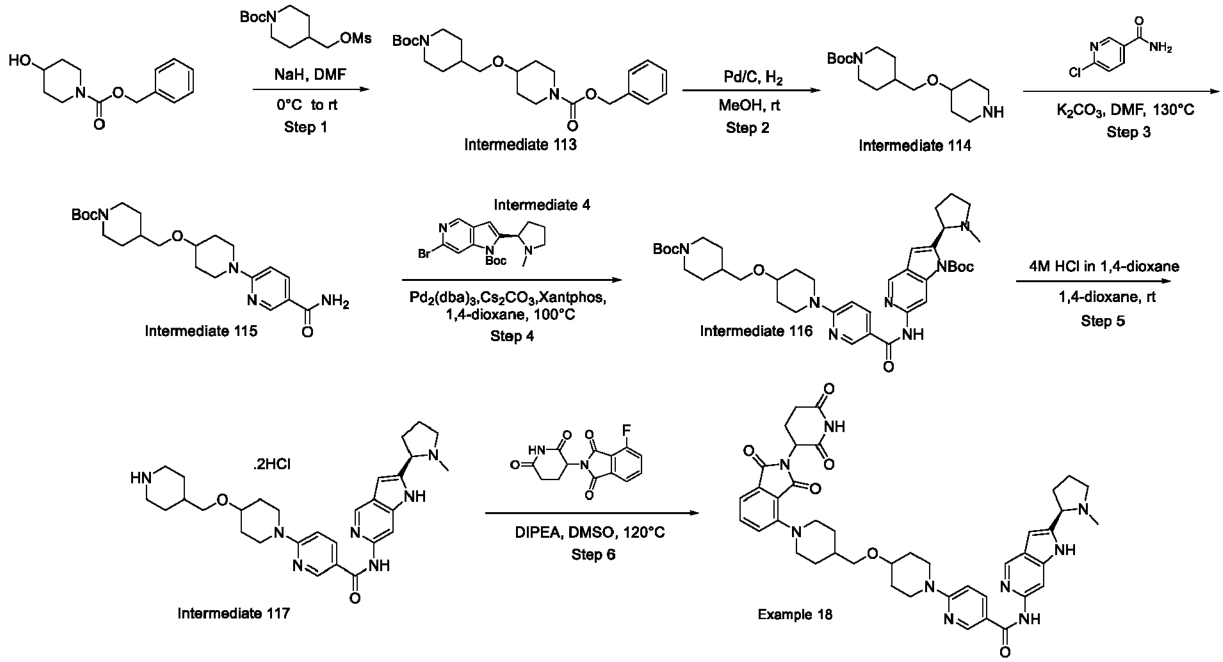


## Scheme 21

**Step 1**

**Intermediate 113: benzyl 4-((1-(*tert*-butoxycarbonyl)piperidin-4-yl)methoxy)piperidine-1-carboxylate**

To a solution of *tert*-butyl 4-(((methylsulfonyl)oxy)methyl)piperidine-1-carboxylate (CAS No: 161975-39-9, 17.0 g, 72.25 mmol) in dimethylformamide (210 mL) was added sodium hydride (4.33 g, 108.4 mmol) and the reaction stirred for 30 in at 0 °C. Benzyl 4-hydroxypiperidine-1-carboxylate (CAS No: 95798-23-5, 23.32 g, 79.47 mmol) in dimethylformamide (50 mL) was added dropwise and the reaction heated at 60°C for 16h. The reaction was quenched with water (1000 mL) at 0 °C and extracted with ethyl acetate (2 × 200 mL). The combined organic layers were dried over anhydrous $Na_2SO_4$, filtered, and concentrated *in vacuo*. The crude material was then purified by combi-flash column chromatography, by eluting with 30% ethyl acetate in heptane, to afford benzyl 4-((1-(*tert*-butoxycarbonyl) piperidin-4-yl) methoxy) piperidine-1-carboxylate (**Intermediate 113**, 6.5 g, 21%) as a colourless oil. $^1H$ NMR (400 MHz, $CDCl_3$) δ ppm 1.07-1.19 (m, 2H), 1.45 (s, 9H), 1.69-1.80 (m, 5H), 2.64-2.72 (m, 2H), 3.22-3.28 (m, 4H), 3.41-3.45 (m, 2H), 3.70-3.80 (m, 2H), 4.08-4.12 (m, 2H), 4.67-4.70 (m, 1H), 5.12 (s, 2H), 7.28-7.40 (m, 5H).

**Step 2**

**Intermediate 114: *tert*-butyl 4-((piperidin-4-yloxy)methyl)piperidine-1-carboxylate**

To a solution of benzyl 4-((1-(tert-butoxycarbonyl)piperidin-4-yl)methoxy)piperidine-1-carboxylate (**Intermediate 113**, 6.50 g, 15.0 mmol) in methanol (300 mL) was added 10% Pd/C (1.75 g) under a nitrogen atmosphere. The suspension was de-gassed under vacuum and purged with $H_2$ three times. The mixture was then stirred at room temperature for 16h under a $H_2$ (35 psi). The reaction mixture was filtered through celite and washed with MeOH (2 × 50 mL). The filtrate was concentrated *in vacuo* to afford *tert*-butyl 4-((piperidin-4-yloxy) methyl) piperidine-1-carboxylate (**Intermediate 114**, 4.5 g) as a colourless oil, which was used for the next step without further purification. $^1$H NMR (400 MHz, $CDCl_3$) δ ppm 1.00-1.19 (m, 2H), 1.45 (s, 9H), 1.68-1.74 (m, 5H), 1.89-2.04 (m, 2H), 2.66-2.74 (m, 2H), 2.82-2.93 (m, 2H), 3.09-3.19 (m, 2H), 3.22-3.31 (m, 2H), 3.42-3.48 (m, 1H), 4.04-4.12 (m, 2H), 4.70 (br s, 1H).

**Step 3**

**Intermediate 115: *tert*-butyl 4-(((1-(5-carbamoylpyridin-2-yl)piperidin-4-yl)oxy)methyl)piperidine-1-carboxylate**

To a solution of *tert*-butyl 4-((piperidin-4-yloxy)methyl)piperidine-1-carboxylate (**Intermediate 114**, 1.6 g, 5.4 mmol) and 6-chloronicotinamide (CAS No: 6271-78-9, 0.76 g, 4.8 mmol) in dimethylformamide (10 mL) was added potassium carbonate (2.2 g, 16.0 mmol) and the reaction mixture was heated at 130 °C for 16h. The reaction mixture was poured in ice cold water (100 mL), a precipitate formed which was filtered, washed with water (50 mL) and dried *in vacuo* to afford *tert*-butyl 4-(((1-(5-carbamoylpyridin-2-yl)piperidin-4-yl)oxy)methyl) piperidine-1-carboxylate (**Intermediate 115**, 1.30 g, 58%) as an off white solid. $^1$H NMR (400 MHz, DMSO-$d_6$) δ ppm 0.96-1.08 (m, 2H), 1.38 (s, 9H), 1.42-1.44 (m, 2H), 1.61-1.68 (m, 3H), 1.81-1.86 (m, 2H), 2.61-2.70 (m, 2H), 3.28-3.32 (m, 4H), 3.48-3.54 (m, 1H), 3.90-3.98 (m, 4H), 6.84 (d, *J*=8.77Hz, 1H), 7.10 (br s, 1H), 7.73 (br s, 1H), 7.93 (d, *J*=8.77 Hz, 1H), 8.59 (s, 1H). Mass spec: m/z 419.3 $[M+H]^+$.

**Step 4**

**Intermediate 116: *tert*-butyl (*R*)-6-(6-(4-((1-(tert-butoxycarbonyl)piperidin-4-yl)methoxy)piperidin-1-yl) nicotinamido)-2-(1-methylpyrrolidin-2-yl)-1*H*-pyrrolo[3, 2-c] pyridine-1-carboxylate**

To a mixture of *tert*-butyl 4-(((1-(5-carbamoylpyridin-2-yl)piperidin-4-yl)oxy)methyl)piperidine-1-carboxylate (**Intermediate 115**, 0.80 g, 1.91 mmol) and *tert*-butyl (*R*)-6-bromo-2-(1-methylpyrrolidin-2-yl)-1H-pyrrolo[3,2-c]pyridine-1-carboxylate (**Intermediate 4**, 0.79 g, 2.10 mmol) in 1,4-dioxane (16 mL) was added cesium carbonate (1.88 g, 5.73 mmol) at room temperature. The reaction mixture was purged with argon for 15 min followed by the addition of $Pd_2(dba)_3$ (0.28 g, 0.27 mmol) and Xantphos (0.34 g, 0.57 mmol). The reaction mixture was further purged with argon for 10 min and heated at 100°C for 2h. The reaction mixture was concentrated *in vacuo* and the crude material was purified by combi-flash column chromatography, by eluting with 3% MeOH in DCM, to afford *tert*-butyl (*R*)-6-(6-(4-((1-(*tert*-butoxycarbonyl)piperidin-4-yl)methoxy)piperidin-1-yl)nicotinamido)-2-(1-methylpyrrolidin-2-yl)-1*H*-pyrrolo[3, 2-c]pyridine-1-carboxylate (**Intermediate 116,** 1.1 g, 80%) as a brown solid. $^1$H NMR (400 MHz, DMSO-$d_6$) δ ppm 0.90-1.08 (m, 4H), 1.35 (s, 9H), 1.38-1.40 (m, 2H), 1.49-1.61 (m, 3H), 1.65 (s, 9H), 1.68-1.77 (m, 3H), 1.81-1.90 (m, 2H), 2.24-2.29 (m, 1H), 2.32 (s, 3H), 2.62-2.66 (m, 3H), 3.04-3.10 (m, 2H), 3.48-3.52 (m, 2H), 3.79-4.08 (m, 5H), 6.70 (s, 1H), 6.85 (d, *J*=8.71 Hz, 1H), 8.08-8.12 (m, 1H), 8.53 (s, 1H), 8.75 (s, 1H), 8.87 (s, 1H), 10.45 (br s, 1H). Mass spec: m/z 718.1 $[M+H]^+$.

**Step 5**

**Intermediate 117: (*R*)-*N*-(2-(1-methylpyrrolidin-2-yl)-1*H*-pyrrolo [3, 2-c] pyridin-6-yl)-6-(4-(piperidin-4-ylmethoxy) piperidin-1-yl)nicotinamide dihydrochloride**

To a solution of *tert*-butyl (*R*)-6-(6-(4-((1-(tert-butoxycarbonyl) piperidin-4-yl)methoxy)piperidin-1-yl) nicotinamido)-2-(1-methylpyrrolidin-2-yl)-1*H*-pyrrolo[3, 2-c]pyridine-1-carboxylate (**Intermediate 116**, 1.00 g, 1.39 mmol) in 1,4-dioxane (20 mL) was added 4M HCl in 1,4-dioxane (20 mL) at 0°C and the reaction mixture was stirred at room temperature for 16h. The reaction mixture was concentrated *in vacuo* and washed with diethyl ether (20 mL) to afford (*R*)-*N*-(2-(1-methylpyrrolidin-2-yl)-1*H*-pyrrolo[3, 2-c]pyridin-6-yl)-6-(4-(piperidin-4-ylmethoxy)piperidin-1-yl)nicotinamide dihydrochloride (**Intermediate 117**, 0.90 g) as an off white solid, which was used for the next step without further purification. Mass spec: m/z 518.5 $[M+H]^+$.

**Step 6**

**Example 18: 6-(4-((1-(2-(2,6-dioxopiperidin-3-yl)-1,3-dioxoisoindolin-4-yl)piperidin-4-yl)methoxy)piperidin-1-yl)-*N*-(2-((*R*)-1-methylpyrrolidin-2-yl)-1*H*-pyrrolo[3,2-c]pyridin-6-yl)nicotinamide**

To a solution of (*R*)-*N*-(2-(1-methylpyrrolidin-2-yl)-1*H*-pyrrolo[3, 2-c]pyridin-6-yl)-6-(4-(piperidin-4-ylmethoxy) piperidin-1-yl)nicotinamide dihydrochloride (**Intermediate 117, 0.50** g, 0.90 mmol) in dimethyl sulfoxide (10 mL) was added *N,N*-diisopropylethylamine (0.78 mL, 4.51 mmol) followed by 2-(2,6-dioxopiperidin-3-yl)-4-fluoroisoindoline-1,3-dione (CAS No: 835616-60-9, 0.24 g, 0.90 mmol) and the reaction mixture was heated at 120 °C for 2h. The reaction mixture was quenched with ice cold water (50 mL), the precipitate formed was filtered and dried *in vacuo.* The crude precipitate was purified by preparative HPLC (Method A) to afford 6-(4-((1-(2-(2,6-dioxopiperidin-3-yl)-1,3-dioxoisoindolin-4-yl)piperidin-4-yl)methoxy)piperidin-1-yl)-*N*-(2-((R)-1-methylpyrrolidin-2-yl)-1*H*-pyrrolo[3,2-c]pyridin-6-yl)nicotinamide (**Example 18**, 0.16 g, 24%) as a yellow solid. $^1$H NMR (400 MHz, DMSO-$d_6$) δ ppm 1.37-1.53 (m, 4H), 1.69-1.95 (m, 9H), 1.98-2.06 (m, 1H), 2.09-2.14 (m, 1H), 2.16 (s, 3H), 2.22-2.30 (m, 1H), 2.56-2.62 (m, 2H), 2.82-2.95 (m, 3H), 3.10-3.19 (m, 1H), 3.36-3.41 (m, 4H), 3.53-3.63 (m, 1H), 3.66-3.74 (m, 2H), 3.98-4.04 (m, 2H), 5.06-5.11 (m, 1H), 6.37 (s, 1H), 6.89 (d, *J*=9.20 Hz, 1H), 7.30-7.36 (m, 2H), 7.66-7.70 (m, 1H), 8.12-8.14 (m, 1H), 8.16 (s, 1H), 8.48 (s, 1H), 8.79 (s, 1H), 10.24 (s, 1H), 11.07 (s, 1H), 11.32 (s, 1H). Mass spec: m/z: 774.0 [M+H]$^+$.

**Example 19 was synthesised following Scheme 22**

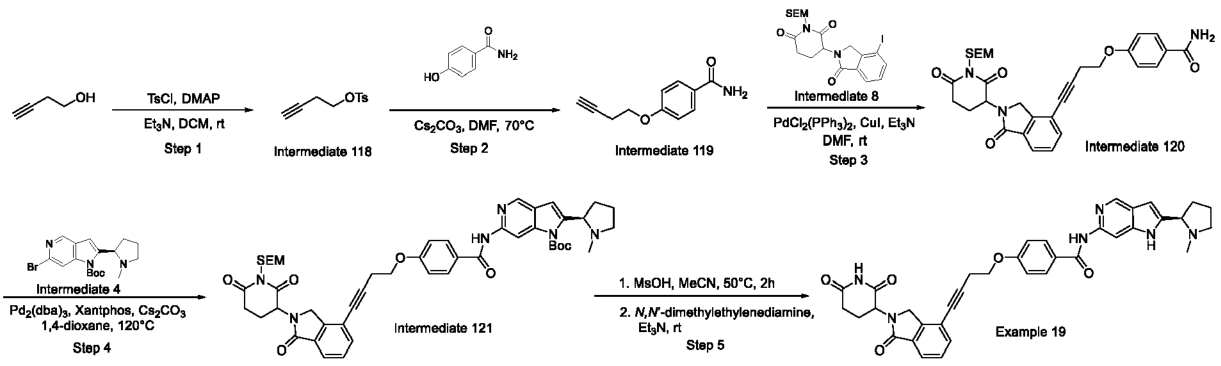


**Scheme 22**

**Step 1**

**Intermediate 118: but-3-yn-1-yl 4-methylbenzenesulfonate**

To a solution of but-3-yn-1-ol (CAS No: 927-74-2, 10.0 g, 142.7 mmol) in dichloromethane (240 mL) was added triethylamine (21.87 g, 214.0 mmol) and 4-dimethylaminopyridine (1.76 g, 14.27 mmol) and the reaction was stirre at 0°C for 15 min. p-Toluenesulfonyl chloride (33.3 g, 171.2 mmol) was added and the reaction was stirred for 16h at room temperature. The reaction mixture was diluted with 1N HCl (150 mL) and extracted with dichloromethane (2 × 200 mL). The combined organic layers were washed with $NaHCO_3$ solution (2 × 100 mL) and brine solution (2 × 100 mL), dried over anhydrous $Na_2SO_4$ and concentrated *in vacuo* to afford but-3-yn-1-yl 4-methylbenzenesulfonate (**Intermediate 118,** 25.0 g, 78%) as yellow colour oil, which was used for the next step without further purification. $^1$H NMR (400 MHz, DMSO-$d_6$) δ ppm 2.43 (t, *J*=2.60 Hz, 1H), 2.46 (s, 3H), 2.87 (t, *J*=2.60 Hz, 1H), 3.31 (t, *J*=7.0 Hz, 1H), 4.04 (t, *J*=7.0 Hz, 2H), 7.50 (d, *J*=8.0 Hz, 2H), 7.80 (d, *J*=8.0 Hz, 2H).

**Step 2**

**Intermediate 119: 4-(but-3-yn-1-yloxy)benzamide**

To a solution of but-3-yn-1-yl 4-methylbenzenesulfonate (**Intermediate 118,** 8.18 g, 36.5 mmol) in dimethylformamide (150 mL) was added 4-hydroxybenzamide (CAS No: 619-57-8, 2.00 g, 14.6 mmol) followed by cesium carbonate (14.3 g, 43.8 mmol) at room temperature. The reaction mixture was then heated at 70°C for 16h. Water (100 mL) was added and the aqueous phase was extracted with ethyl acetate (2 × 60 mL). The combined organic phases were washed with brine solution (2 × 60 mL), dried over anhydrous $Na_2SO_4$, filtered, and concentrated *in vacuo.* The crude material was purified by combi-flash chromatography, by eluting with 50% ethyl acetate in heptane, to afford 4-(but-3-yn-1-yloxy)benzamide (**Intermediate 119,** 0.38 g, 14%) as a white solid. $^1$H NMR (400 MHz, DMSO-$d_6$) δ ppm 2.65 (t, *J*=7.20 Hz, 2H) 2.89 (t, *J*=7.20 Hz, 1H) 4.11 (t, *J*=7.20 Hz, 2H) 6.97-6.99 (m, 2H) 7.17 (br s, 1H) 7.82-7.84 (m, 3H).

**Step 3**

**Intermediate 120: 4-((4-(2-(2,6-dioxo-1-((2-(trimethylsilyl)ethoxy)methyl)piperidin-3-yl)-1-oxoisoindolin-4-yl) but-3-yn-1-yl)oxy)benzamide**

To a solution of 3-(4-iodo-1-oxoisoindolin-2-yl)-1-((2-(trimethylsilyl)ethoxy)methyl)piperidine-2,6-dione (**Intermediate 119,** 0.97 g, 1.94 mmol) in dimethylformamide (3 mL) was added 4-(but-3-yn-1-yloxy)benzamide **(Intermediate 8**, 0.35 g, 1.84 mmol) followed by triethylamine (9.07 mL, 64.74 mmol). The reaction mixture was purged with argon gas for 15 min then $PdCl_2(PPh_3)_2$ (0.13 g, 0.18 mmol) and copper(I) iodide (0.03 g, 0.18 mmol) were added. The reaction mixture was further purged with argon gas for 10 min and stirred at room temperature for 3h. The reaction mixture was diluted with water (50 mL) and extracted with ethyl acetate (3 × 50 mL). The combined organic layers were washed with brine solution (2 × 30 mL), dried over anhydrous $Na_2SO_4$, filtered, and concentrated *in vacuo.* The crude material was purified by combi-flash chromatography, by eluting with ethyl acetate, to afford 4-((4-(2-(2,6-dioxo-1-((2-(trimethylsilyl)ethoxy)methyl) piperidin-3-yl)-1-oxoisoindolin-4-yl) but-3-yn-1-yl)oxy)benzamide (**Intermediate 120,** 0.35 g, 34%) as a white solid. $^1$H NMR (400 MHz, DMSO-$d_6$) δ ppm -0.03 (s, 9H), 0.76-0.92 (m, 2H), 1.97-2.12 (m, 1H), 2.27-2.41 (m, 1H), 2.66-2.85 (m, 2H), 2.93-3.14 (m, 3H), 3.46-3.59 (m, 2H), 4.20-4.32 (m, 2H), 4.41-4.46 (m, 1H), 4.99-5.12 (m, 2H), 5.23-5.27 (m, 1H), 7.02 (d, J=8.40 Hz, 2H), 7.17 (br s, 1H), 7.54 (t, *J*=7.20 Hz, 1H), 7.66 (d, *J*=7.20 Hz, 1H), 7.74 (d, *J*=7.21 Hz, 1H), 7.82-7.86 (m, 3H). Mass spec: m/z: 560.0 $[M-H]^-$.

**Step 4**

**Intermediate 121: *tert*-butyl 6-(4-((4-(2-(2,6-dioxo-1-((2-(trimethylsilyl)ethoxy)methyl)piperidin-3-yl)-1-oxoisoindolin-4-yl)but-3-yn-1-yl)oxy)benzamido)-2-((*R*)-1-methylpyrrolidin-2-yl)-1*H*-pyrrolo[3,2-c]pyridine-1-carboxylate**

To a solution of 4-((4-(2-(2, 6-dioxo-1-((2-(trimethylsilyl)ethoxy)methyl)piperidin-3-yl)-1-oxoisoindolin-4-yl) but-3-yn-1-yl)oxy)benzamide (**Intermediate 120,** 0.35 g, 0.62 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate (**Intermediate 4,** 0.28 g, 0.74 mmol) followed by cesium carbonate (0.61 g, 1.86 mmol) at room temperature. The reaction mixture was purged with argon for 15 min followed by $Pd_2(dba)_3$ (0.11 g, 0.12 mmol) and Xantphos (0.14 g, 0.24 mmol). The reaction mixture was purged with argon for 10 min and heated at 120°C for 90 min. The reaction mixture was diluted with water (50 mL) and extracted with ethyl acetate (2 × 100 mL). The organic layers were separated, dried over anhydrous $Na_2SO_4$, and concentrated *in vacuo.* The crude material was purified by combi-flash chromatography, by eluting with 2% MeOH in DCM, to afford *tert*-butyl 6-(4-((4-(2-(2,6-dioxo-1-((2-(trimethylsilyl)ethoxy)methyl)piperidin-3-yl)-1-oxoisoindolin-4-yl)but-3-yn-1-yl)oxy) benzamido)-2-((*R*)-1-methylpyrrolidin-2-yl)-1*H*-pyrrolo[3,2-c]pyridine-1-carboxylate (**Intermediate 121,** 0.31 g, 58%) as a brown solid. $^1$H NMR (400 MHz, DMSO-$d_6$) δ ppm 0.00 (s, 9H), 0.77-0.90 (m, 2H), 1.35-1.40 (m, 1H), 1.69 (s, 9H), 1.79- 1.98 (m, 4H), 2.35 (s, 3H), 2.37-2.39 (m, 3H), 2.75-2.79 (m, 1H), 3.00-3.13 (m, 3H), 3.50-3.52 (m, 2H), 3.88-3.94 (m, 1H), 4.26-4.38 (m, 3H), 4.48-4.53 (m, 1H), 5.00-5.06 (m, 2H), 5.25-4.27 (m, 1H), 6.75 (s, 1H), 7.04-7.12 (m, 2H), 7.25-7.40 (m, 2H), 7.62-7.66 (m, 1H), 8.02-8.13 (m, 2H), 8.60 (s, 1H), 8.92 (s, 1H), 10.78 (s, 1H).

**Step 5**

**Example 19: 4-((4-(2-(2,6-dioxopiperidin-3-yl)-1-oxoisoindolin-4-yl)but-3-yn-1-yl)oxy)-*N*-(2-((*R*)-1-methylpyrrolidin-2-yl)-1*H*-pyrrolo[3,2-c]pyridin-6-yl)benzamide**

To a solution of *tert*-butyl 6-(4-((4-(2-(2,6-dioxo-1-((2-(trimethylsilyl)ethoxy)methyl)piperidin-3-yl)-1-oxoisoindolin-4-yl)but-3-yn-1-yl)oxy)benzamido)-2-((*R*)-1-methylpyrrolidin-2-yl)-1*H*-pyrrolo[3,2-c]pyridine-1-carboxylate (**Intermediate 121,** 0.30 g, 0.34 mmol) in acetonitrile (10 mL) was added methanesulfonic acid (0.34 mL, 5.22 mmol) at room temperature and the reaction mixture heated at 50°C for 2h. After cooling to room temperature, *N,N'*-dimethylethylenediamine (0.25 mL, 2.09 mmol) followed by triethylamine (0.73 mL, 5.22 mmol) were added and the reaction stirred at room

temperature for 2h. The reaction mixture was then concentrated under reduced pressure, washed with ether (2 × 50 mL) and n-pentane (2 × 40 mL) and then dried to obtain crude compound. The crude material was purified by preparative HPLC (Method A) to afford 4-((4-(2-(2,6-dioxopiperidin-3-yl)-1-oxoisoindolin-4-yl)but-3-yn-1-yl)oxy)-*N*-(2-((*R*)-1-methylpyrrolidin-2-yl)-1*H*-pyrrolo[3,2-c]pyridin-6-yl)benzamide (**Example 19**, 0.01 g, 8%) as an off white solid. $^1$H NMR (400 MHz, DMSO-$d_6$) δ ppm 1.75-1.94 (m, 3H), 1.96-2.04 (m, 1H), 2.10-2.14 (m, 1H), 2.17 (s, 3H), 2.22-2.30 (m, 1H), 2.31-2.37 (m, 1H), 2.55-2.64 (m, 2H), 2.86-2.96 (m, 1H), 3.01-3.55 (m, 2H), 3.11-3.19 (m, 1H), 4.26-4.35 (m, 3H), 4.39-4.48 (m, 1H), 5.11-5.16 (m, 1H), 6.38 (s, 1H), 7.06-7.11 (m, 2H), 7.50-7.57 (m, 1H), 7.66-7.71 (m, 1H), 7.73-7.78 (m, 1H), 8.03-8.07 (m, 2H), 8.17 (s, 1H), 8.49 (s, 1H), 10.28 (br s, 1H), 11.02 (br s, 1H), 11.34 (br s, 1H). Mass spec: m/z: 631.3 $[M+H]^+$.

**Example 20 was synthesised following Scheme 23**

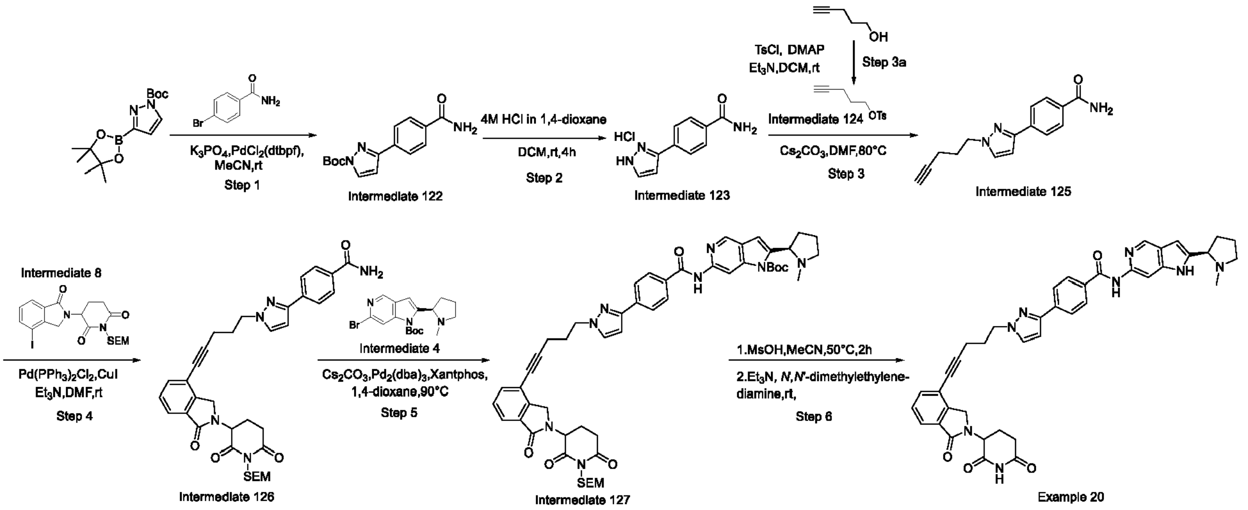


## Scheme 23

**Step 1**

**Intermediate 122: tert-butyl 3-(4-carbamoylphenyl)-1*H*-pyrazole-1-carboxylate**

A solution of 4-bromobenzamide (CAS No: 698-67-9, 3.0 g, 15 mmol), tert-butyl 3-(4,4,5,5-tetramethyl-1,3,2-dioxaborolan-2-yl)-1*H*-pyrazole-1-carboxylate (CAS No: 914672-66-5, 4.9 g, 16 mmol) and $K_3PO_4$ (9.8 g, 45 mmol) in acetonitrile (60 mL) at room temperature was purged with argon gas for 5 min. $PdCl_2$(dtbpf) (0.80 g, 1.2 mmol) was added at room temperature and stirred for 4h. The reaction mixture was poured into water (100 mL) and extracted with ethyl acetate (2 × 100 mL). The combined organic layers were washed with saturated $Na_2S_2O_3$ (150 mL), dried over anhydrous $Na_2SO_4$, filtered and concentrated *in vacuo* to obtain crude compound. The crude material was purified by combi-flash column chromatography, by eluting with 80% ethyl acetate in heptane, to afford *tert*-butyl 3-(4-carbamoylphenyl)-1*H*-pyrazole-1-carboxylate (**Intermediate 122,** 2.2 g, 51%) as a yellow gum. $^1$H NMR (400 MHz, DMSO-$d_6$) δ ppm 1.62 (s, 9H), 7.14 (d, *J*=2.07 Hz, 1H), 7.40 (br s, 1H), 7.94-8.05 (m, 4H), 8.03 (s, 1H), 8.36 (d, *J*=2.07 Hz, 1H). Mass spec: m/z: 288.06 $[M+H]^+$.

**Step 2**

**Intermediate 123: 4-(1*H*-pyrazol-3-yl)benzamide hydrochloride**

To a solution of *tert*-butyl 3-(4-carbamoylphenyl)-1*H*-pyrazole-1-carboxylate (**Intermediate 122**, 2.20 g, 7.7 mmol) in dichloromethane (20 mL) was added 4M hydrochloric acid in 1,4-dioxane (19 mL, 77 mmol) at 0°C. The reaction mixture was then stirred at room temperature for 4h. The reaction mixture was concentrated *in vacuo* and the resulting solid was washed with diethyl ether (20 mL) and dried under reduced pressure to afford 4-(1*H*-pyrazol-3-yl)benzamide hydrochloride (**Intermediate 123,** 1.50 g) as an off white solid, which was used in the next step without further purification. $^1$H NMR (400 MHz, DMSO-$d_6$) δ ppm 6.81 (s, 1H), 7.33 (br s, 1H), 7.76 (s, 1H), 7.86-7.92 (m, 4H), 7.95 (br s, 1H). Mass

spec: m/z: 188.02 $[M+H]^+$.

**Step 3a**

**Intermediate 124: pent-4-yn-1-yl 4-methylbenzenesulfonate**

To solution of pent-4-yn-1-ol (CAS No: 5390-04-5, 5.0 g, 59 mmol) in dichloromethane (100 mL) was added triethylamine (19 g, 180 mmol) at 0°C. *p*-toluenesulfonylchloride (18 g, 89 mmol) followed by 4-dimethylaminopyridine (0.76 g, 5.9 mmol) were added and the reaction mixture was stirred at room temperature for 16h. The reaction mixture was poured into ice cold water (100 mL) and extracted with ethyl acetate (2 × 150 mL). The combined organic phases were dried over anhydrous $Na_2SO_4$, filtered and concentrated *in vacuo* to afford pent-4-yn-1-yl 4-methylbenzenesulfonate **(Intermediate 124,** 7.00 g, 49%) as a gum. $^1H$ NMR (400 MHz, $CDCl_3$) δ ppm 1.83-1.89 (m, 3H), 2.24-2.28 (m, 2H), 2.45 (s, 3H), 4.15 (t, *J*=6.14 Hz, 2H), 7.34 (d, *J*=7.89 Hz, 2H), 7.80 (d, *J*=8.33 Hz, 2H). Mass spec: m/z: 239.0 $[M+H]^+$.

**Step 3**

**Intermediate 125: 4-(1-(pent-4-yn-1-yl)-1*H*-pyrazol-3-yl)benzamide**

A solution of pent-4-ynyl 4-methylbenzenesulfonate (**Intermediate 124**, 2.05 g, 8.58 mmol), 4-(1H-pyrazol-3-yl) benzamide hydrochloride (**Intermediate 123,** 1.28 g, 5.72 mmol) and cesium carbonate (5.89 g, 17.2 mmol) in 1,4-dioxane (25 mL) was heated at 80°C for 3h. The reaction mixture was poured into water (100 mL) and extracted with ethyl acetate (2 × 150 mL). The combined organic phases were dried over anhydrous $Na_2SO_4$, filtered, and concentrated *in vacuo* to obtain crude compound. The crude material was purified by combi-flash column chromatography, by eluting with 80% ethyl acetate in heptane, to afford 4-(1-(pent-4-yn-1-yl)-1*H*-pyrazol-3-yl) benzamide (**Intermediate 125,** 1.25 g, 86%) as an off white solid. $^1H$ NMR (400 MHz, DMSO-$d_6$) δ ppm ppm 1.98-2.02 (m, 2H), 2.16-2.18 (m, 2H), 2.86 (br s, 1H), 4.21-4.24 (m, 2H), 6.79 (s, 1H), 7.31 (br s, 1H), 7.76-7.91 (m, 5H), 7.96 (br s, 1H). Mass spec: m/z: 254.09 $[M+H]^+$.

**Step 4**

**Intermediate 126: 4-(1-(5-(2-(2,6-dioxo-1-((2-(trimethylsilyl)ethoxy)methyl)piperidin-3-yl)-1-oxoisoindolin-4-yl) pent-4-yn-1-yl)-1*H*-pyrazol-3-yl)benzamide**

A solution of 3-(4-iodo-1-oxo-isoindolin-2-yl)-1-(2-trimethylsilylethoxymethyl)piperidine-2,6-dione (**Intermediate 8,** 1.0 g, 2.0 mmol), 4-(1-pent-4-ynylpyrazol-3-yl)benzamide (**Intermediate 125,** 0.51 g, 2.0 mmol) and $Et_3N$ (8.3 g, 78 mmol) in dimethylformamide (1 mL) was purged with argon gas for 15 min. Copper(I) iodide (0.040 g, 0.20 mmol) and $PdCl_2(PPh_3)_2$ (0.15 g, 0.20 mmol) were added and the reaction was stirred at room temperature and stirred for 1h. The reaction mixture was poured into water (80 mL) and extracted with ethyl acetate (2 × 80 mL). The combined organic phases were dried over anhydrous $Na_2SO_4$, filtered, and concentrated *in vacuo* to obtain crude compound. The crude material was purified by combi-flash column chromatography, by eluting with 3% MeOH in DCM, to afford 4-(1-(5-(2-(2, 6-dioxo-1-((2-(trimethylsilyl) ethoxy)methyl)piperidin-3-yl)-1-oxoisoindolin-4-yl) pent-4-yn-1-yl)-1*H*-pyrazol-3-yl) benzamide (**Intermediate 126**, 1.0 g, 80%) as brown solid. $^1H$ NMR (400 MHz, DMSO-$d_6$) δ ppm -0.05 (s, 9H), 0.79-0.84 (m, 2H), 1.99-2.06 (m, 2H), 2.10-2.17 (m, 2H), 2.37-2.40 (m, 2H), 2.73-2.79 (m, 1H), 3.01-3.10 (m, 1H), 3.46-3.54 (m, 2H), 4.27-4.31 (m, 3H), 4.48-4.52 (m, 1H), 4.99-5.08 (m, 2H), 5.23-5.28 (m, 1H), 6.79 (s, 1H), 7.31 (s, 1H), 7.51-7.55 (m, 1H), 7.67 (d, *J*=7.46 Hz, 1H), 7.72 (d, *J*=7.88 Hz, 1H), 7.81-7.85 (m, 3H), 7.87-7.90 (m, 2H), 7.93-7.95 (m, 1H). Mass spec: m/z: 626.33 $[M+H]^+$.

**Step 5**

**Intermediate 127: *tert*-butyl 6-(4-(1-(5-(2-(2,6-dioxo-1-((2-(trimethylsilyl)ethoxy)methyl)piperidin-3-yl)-1-oxoisoindolin-4-yl)pent-4-yn-1-yl)-1*H*-pyrazol-3-yl)benzamido)-2-((*R*)-1-methylpyrrolidin-2-yl)-1*H*-pyrrolo[3,2-c]pyridine-1-carboxylate**

A solution of 4-(1-(5-(2-(2,6-dioxo-1-((2-(trimethylsilyl)ethoxy)methyl)piperidin-3-yl)-1-oxoisoindolin-4-yl) pent-4-yn-1-yl)-1*H*-pyrazol-3-yl)benzamide (**Intermediate 126,** 1.05 g, 1.683 mmol), *tert*-butyl (*R*)-6-bromo-2-(1-methylpyrrolidin-2-yl)-1*H*-pyrrolo[3, 2-c]pyridine-1-carboxylate (**Intermediate 4,** 0.8 g, 2.104 mmol) and $Cs_2CO_3$ (1.80 g, 5.26 mmol) in 1,4-dioxane (10 mL) at room temperature was purged with argon gas for 15 min. To the reaction mixture was added $Pd_2(dba)_3$ (0.27 g, 0.32 mmol) followed by Xantphos (0.38 g, 0.63 mmol) at room temperature and the reaction mixture was heated at 90°C for 1h. The reaction mixture was filtered through celite, washed with DCM (20 mL) and

concentrated *in vacuo* to obtain crude compound. The crude material was purified by combi-flash column chromatography, by eluting with 10% MeOH in DCM, to afford *tert*-butyl 6-(4-(1-(5-(2-(2,6-dioxo-1-((2-(trimethylsilyl)ethoxy)methyl) piperidin-3-yl)-1-oxoisoindolin-4-yl)pent-4-yn-1-yl)-1*H*-pyrazol-3-yl)benzamido)-2-((*R*)-1-methylpyrrolidin-2-yl)-1*H*-pyrrolo[3,2-c]pyridine-1-carboxylate (**Intermediate 127,** 0.40 g, 21%) as brown solid. $^1$H NMR (400 MHz, DMSO-$d_6$) δ ppm -0.05 (s, 9H), 0.79-0.81 (m, 2H), 1.70 (s, 9H), 1.75-1.89 (m, 3H), 2.05-2.17 (m, 4H), 2.36 (s, 3H), 2.37-2.39 (m, 3H), 2.75-2.79 (m, 1H), 3.00-3.13 (m, 3H), 3.50-3.52 (m, 2H), 3.90-3.92 (m, 1H), 4.28-4.32 (m, 3H), 4.48-4.53 (m, 1H), 5.00-5.06 (m, 2H), 5.25-4.27 (m, 1H), 6.76 (s, 1H), 6.84 (s, 1H), 7.53-7.55 (m, 1H), 7.67-7.74 (m, 2H), 7.87-7.92 (m, 3H), 8.08 (d, *J*=7.20 Hz, 2H), 8.60 (s, 1H), 8.95 (s, 1H), 10.68 (br s, 1H). Mass spec: m/z: 925.2 $[M+H]^+$.

**Step 6**

**Example 20: 4-(1-(5-(2-(2,6-dioxopiperidin-3-yl)-1-oxoisoindolin-4-yl)pent-4-yn-1-yl)-1*H*-pyrazol-3-yl)-*N*-(2-((*R*)-1-methylpyrrolidin-2-yl)-1*H*-pyrrolo[3,2-c]pyridin-6-yl)benzamide**

To a solution of *tert*-butyl 6-(4-(1-(5-(2-(2,6-dioxo-1-((2-(trimethylsilyl)ethoxy)methyl)piperidin-3-yl)-1-oxoisoindolin-4-yl)pent-4-yn-1-yl)-1*H*-pyrazol-3-yl)benzamido)-2-((*R*)-1-methylpyrrolidin-2-yl)-1*H*-pyrrolo[3,2-c]pyridine-1-carboxylate (**Intermediate 127,** 0.40 g, 0.43 mmol) in acetonitrile (10 mL) was added methanesulfonic acid (0.44 g, 4.32 mmol) at 0°C and the reaction mixture was heated to 50°C for 2h. *N*, *N'*-dimethylethylenediamine (0.20 g, 2.16 mmol) followed by triethylamine (0.92 g, 8.65 mmol) were added and the reaction stirred at room temperature and stirred for 16h. The reaction mixture was concentrated *in vacuo* and quenched with cold water (60 mL) to obtain solid precipitate, which was filtered and dried. The crude material was purified by preparative HPLC (Method A) to afford 4-(1-(5-(2-(2,6-dioxopiperidin-3-yl)-1-oxoisoindolin-4-yl)pent-4-yn-1-yl)-1*H*-pyrazol-3-yl)-*N*-(2-((*R*)-1-methylpyrrolidin-2-yl)-1*H*-pyrrolo [3,2-c]pyridin-6-yl)benzamide (**Example 20**, 0.12 g, 39%) as an off white solid. $^1$H NMR (400 MHz, DMSO-$d_6$) δ ppm 1.75-1.95 (m, 3H), 1.99-2.03 (m, 1H), 2.11-2.16 (m, 3H), 2.17 (s, 3H), 2.23-2.30 (m, 1H), 2.40-2.44 (m, 2H), 2.52-2.57 (m, 3H), 2.86-2.95 (m, 1H), 3.12-3.16 (m, 1H), 4.31-4.37 (m, 3H), 4.47-4.51 (m, 1H), 5.11-5.15 (m, 1H), 6.40 (s, 1H), 6.84 (d, *J*=2.25 Hz, 1H), 7.52 (t, *J*=7.63 Hz, 1H), 7.67 (d, *J*=6.88 Hz, 1H), 7.71 (d, *J*=7.63 Hz, 1H), 7.87 (d, *J*=2.40 Hz, 1H), 7.91 (d, *J*=8.38 Hz, 2H), 8.09 (d, *J*=8.40 Hz, 2H), 8.21 (s, 1H), 8.51 (s, 1H), 10.43 (s, 1H), 10.99 (s, 1H), 11.37 (s, 1H). Mass spec: m/z: 695.1 $[M+H]^+$.

**Example 21 was synthesised following Scheme 24**

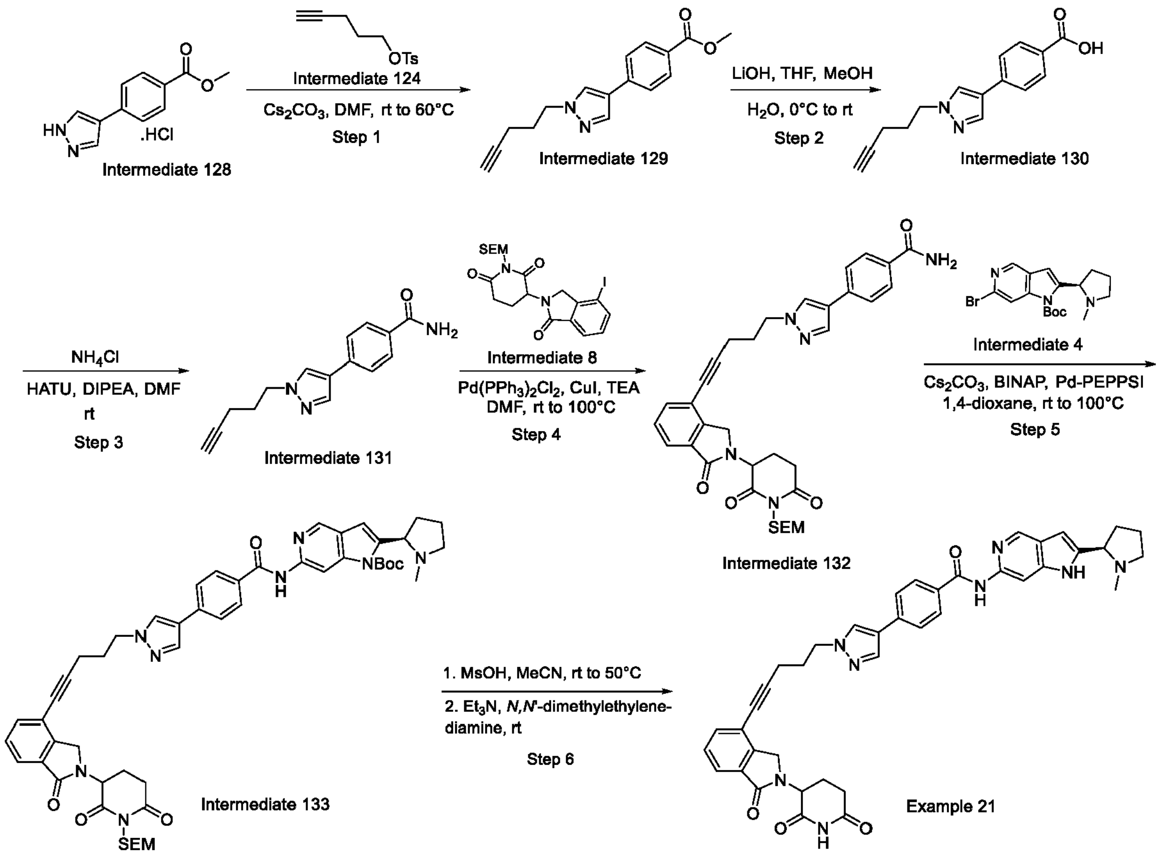


**Scheme 24**

**Step 1**

**Intermediate 129: methyl 4-(1-(pent-4-yn-1-yl)-1H-pyrazol-4-yl)benzoate**

To a solution of pent-4-yn-1-yl 4-methylbenzenesulfonate (**Intermediate 124,** 7 g, 85.22 mmol) in DMF (70 mL) was added cesium carbonate (83.4 g, 255.7 mmol) and methyl 4-(1H-pyrazol-4-yl)benzoate hydrochloride (**Intermediate 128,** 4.3 g, 85.22 mmol) at room temperature. The reaction mixture was heated to 60°C for 16h. Progress of the reaction was monitored by TLC and LCMS. After completion, the reaction mixture was cooled to room temperature, treated with water (100 mL) and extracted with EtOAc (2 × 200 mL). The organic layer was dried over $Na_2SO_4$ and concentrated *in vacuo.* The obtained residue was purified by normal phase MPLC (25-30% in EtOAc in heptane) to afford methyl 4-(1-(pent-4-yn-1-yl)-1H-pyrazol-4-yl)benzoate (**Intermediate 129,** 4.3 g, 19%) as an off-white solid. $^1$H NMR (400 MHz, DMSO-$d_6$) δ ppm 1.99-2.00 (m, 2H), 2.17-2.19 (m, 2H), 2.86 (s, 1H), 3.85 (s, 3H), 4.21-4.32 (m, 2H), 7.73 (d, *J*=6.85 Hz, 2H), 7.93 (d, *J*=6.85 Hz, 2H) 8.00(s, 1H), 8.34 (s, 1H). Mass spec: m/z 269.1 $[M+H]^+$.

**Step 2**

**Intermediate 130: 4-(1-(pent-4-yn-1-yl)-1H-pyrazol-4-yl)benzoic acid**

To a solution of methyl 4-(1-(pent-4-yn-1-yl)-1H-pyrazol-4-yl)benzoate **(Intermediate 129,** 5 g, 18.64 mmol) in tetrahydrofuran (20 mL) and methanol (20 mL) was added lithium hydroxide (1.366 g, 55.91 mmol) in water (10 mL) at 0°C. The reaction mixture was stirred at room temperature for 24h. Progress of the reaction was monitored by TLC. After completion, the reaction mixture was concentrated *in vacuo* and water (25 ml) was added to the residue and the pH adjusted to neutralize the solution with 1N HCl. A solid precipitated out, which was filtered, washed with excess of water and dried to obtain 4-(1-(pent-4-yn-1-yl)-1H-pyrazol-4-yl)benzoic acid (**Intermediate 130,** 3.5 g, 74%) as off-white solid. $^1$H NMR (400 MHz, DMSO-$d_6$) δ ppm 1.94-2.00 (m, 2H), 2.13-2.17 (m, 2H), 2.83 (s, 1H), 4.18 (t, *J*=6.84 Hz, 2H), 7.67 (d, *J*=7.90 Hz, 2H), 7.89 (d, *J*=7.90 Hz, 2H), 7.96 (s, 1H), 8.28 (s, 1H), 12.80 (br s, 1H). Mass spec: m/z 255.1 $[M+H]^+$.

**Step 3**

**Intermediate 131: 4-(1-(pent-4-yn-1-yl)-1H-pyrazol-4-yl)benzamide**

To a solution of 4-(1-(pent-4-yn-1-yl)-1H-pyrazol-4-yl)benzoic acid (**Intermediate 130,** 3.5 g, 14 mmol) in DMF (30 mL) was added diisopropylethylamine (7.3 g, 55 mmol) and HATU (8.1 g, 21 mmol) followed by ammonium chloride (3.0 g, 55 mmol) at room temperature and stirred for 12h. Progress of the reaction was monitored by TLC and LCMS. After completion, the reaction mixture was treated with water (100 mL) and extracted with EtOAc (3 × 100 mL). The organic layer was dried over anhydrous $Na_2SO_4$ and concentrated *in vacuo* to afford 4-(1-(pent-4-yn-1-yl)-1H-pyrazol-4-yl) benzamide (**Intermediate 131,** 2.0 g) as off-white solid. $^1H$ NMR (400 MHz, DMSO-$d_6$) δ ppm 1.97-2.02 (m, 2H), 2.14-2.21 (m, 2H), 2.86 (s, 1H), 4.20 (t, *J*=6.80 Hz, 2H), 7.28 (s, 1H) 7.65 (d, *J*=7.89 Hz, 2H), 7.86 (d, *J*=7.89 Hz, 2H), 7.92 (s, 1H), 7.97 (s, 1H), 8.28 (s, 1H). Mass spec: m/z 254.1 $[M+H]^+$.

**Step 4**

**Intermediate 132: 4-(1-(5-(2-(2,6-dioxo-1-((2-(trimethylsilyl)ethoxy)methyl)piperidin-3-yl)-1-oxoisoindolin-4-yl) pent-4-yn-1-yl)-1H-pyrazol-4-yl)benzamide (8)**

To a solution of 4-(1-(pent-4-yn-1-yl)-1H-pyrazol-4-yl)benzamide (**Intermediate 131,** 600 mg, 2.36 mmol) in DMF (2 mL) was added copper(I) iodide (45 mg, 0.23 mmol), bis(triphenylphosphine)palladium chloride (171 mg, 0.23 mmol) and 3-(4-iodo-1-oxoisoindolin-2-yl)-1-((2-(trimethylsilyl)ethoxy)methyl)piperidine-2,6-dione (**Intermediate 8,** 1.18 g, 2.36 mmol), followed by triethylamine (12.3 mL, 87.6 mmol). The reaction mixture was purged with argon for 10-15 min. The reaction mixture was stirred at RT for 12h. Progress of the reaction was monitored by TLC. After completion, water (100 mL) was added and extracted with EtOAc (3 × 10 mL). The organic layer was dried over anhydrous $Na_2SO_4$ and concentrated *in vacuo.* The obtained residue was purified by normal phase MPLC (0-10% EtOAc in heptane) to afford 4-(1-(5-(2-(2,6-dioxopiperidin-3-yl)-1-oxoisoindolin-4-yl)pent-4-yn-1-yl)-1H-pyrazol-4-yl) benzamide **(Intermediate 132,** 705 mg, 47%) as a white solid. $^1H$ NMR (400 MHz, DMSO-$d_6$) δ ppm -0.06 (s, 9H), 0.80-0.86 (m, 2H), 2.03-2.08 (m, 2H), 2.42-2.45 (m, 2H), 2.78-2.80 (m, 2H), 3.04-3.06 (m, 2H), 3.55-3.58 (m, 2H), 4.28(s, 2H), 4.48-4.53 (m, 2H), 5.04-5.07 (m, 2H), 5.26-5.28 (m,1H), 7.27 (s, 1H), 7.52-7.54 (m, 1H), 7.62- 7.73 (m, 4H), 7.84-7.90 (m, 3H), 7.96 (s, 1H), 8.32 (s, 1H).

**Step 5**

**Intermediate 133: *tert*-butyl 6-(4-(1-(5-(2-(2,6-dioxo-1-((2-(trimethylsilyl)ethoxy)methyl)piperidin-3-yl)-1-oxoisoindolin-4-yl)pent-4-yn-1-yl)-1H-pyrazol-4-yl)benzamido)-2-((R)-1-methylpyrrolidin-2-yl)-1H-pyrrolo[3,2-c] pyridine- 1-carboxylate**

To a solution of 4-(1-(5-(2-(2,6-dioxopiperidin-3-yl)-1-oxoisoindolin-4-yl)pent-4-yn-1-yl)-1H-pyrazol-4-yl)benzamide (**Intermediate 132,** 700 mg, 1.12 mmol) in 1,4-dioxane (5 mL) was added cesium carbonate (1.09 g, 3.36 mmol) followed by *tert*-butyl (R)-6-bromo-2-(1-methylpyrrolidin-2-yl)-1H-pyrrolo[3,2-c]pyridine-1-carboxylate (**Intermediate 4,** 425 mg, 1.12 mmol) at room temperature. The reaction mixture was purged with argon for 15 min. To this reaction mixture (*R*)-(+)-2,2'-bis(diphenylphosphino)-1,1'-binaphthyl (71 mg, 0.11mmol) and dichloro[1,3-bis(2,6-di-3-pentylphenyl)imidazol-2-ylidene](3-chloropyridyl)palladium(II) (93 mg, 0.11 mmol) were added and reaction mixture was again degassed for 5 minutes then heated to 100°C for 2h. Progress of the reaction was monitored by TLC and LCMS. After completion of reaction, water (200 mL) and extracted with EtOAc (3 × 200 mL). The organic layer was dried over anhydrous $Na_2SO_4$ and concentrated *in vacuo.* The obtained residue was purified by normal phase MPLC (0-50% EtOAc in heptane) to afford *tert*-butyl 6-(4-(1-(5-(2-(2,6-dioxo-1-((2-(trimethylsilyl)ethoxy)methyl)piperidin-3-yl)-1-oxoisoindolin-4-yl)pent-4-yn-1-yl)-1H-pyrazol-4-yl)benzamido)-2-((R)-1-methylpyrrolidin-2-yl)-1H-pyrrolo[3,2-c]pyridine-1-carboxylate (**Intermediate 133,** 700 mg, 67%) as an off-white solid.$^1H$ NMR (400 MHz, DMSO-$d_6$) δ ppm -0.06 (s, 9H), 0.74-0.86 (m, 2H), 1.69- 1.74 (m, 13H), 2.06 - 2.14 (m, 4H), 2.35 (s, 5H), 2.75-2.80 (m, 1H), 3.03-3.12 (m, 2H), 3.48-3.53 (m, 2H), 3.90-3.94 (m, 1H), 4.28-4.31 (m, 3H), 4.50-4.53 (m, 1H), 5.00-5.07(m, 2 H) 5.24- 5.28 (m, 1H), 6.75 (br s, 1 H) 7.50-7.53 (m, Hz, 1H), 7.66-7.74 (m, 4H), 8.01-8.06 (m, 3H), 8.38 (br s, 1H) 8.59 (br s, 1H) 8.94 (br s, 1H) 10.63 (br s, 1H). Mass spec: m/z 925.3 $[M+H]^+$.

**Step 6**

**Example 21: 4-(1-(5-(2-(2,6-dioxopiperidin-3-yl)-1-oxoisoindolin-4-yl)pent-4-yn-1-yl)-1H-pyrazol-4-yl)-N-(2-((R)-1-methylpyrrolidin-2-yl)-1H-pyrrolo[3,2-c]pyridin-6-yl)benzamide**

To a solution of tert-butyl 6-(4-(1-(5-(2-(2,6-dioxo-1-((2-(trimethylsilyl)ethoxy)methyl)piperidin-3-yl)-1-oxoisoindolin-4-yl)pent-4-yn-1-yl)-1H-pyrazol-4-yl)benzamido)-2-((R)-1-methylpyrrolidin-2-yl)-1H-pyrrolo[3,2-c]pyridine-1-carboxylate (**Intermediate 133,** 700 mg, 0.75 mmol) in acetonitrile (5 mL) was added methanesulfonic acid (1.08 g, 11.34

mmol) at room temperature and reaction mixture was heated at 50°C for 2h. The reaction mixture was cooled to room temperature. To this mixture, *N,N'*-dimethylethylenediamine (396 mg, 4.5 mmol) followed by triethylamine (1.14 g, 11.34 mmol) were added at room temperature and stirred at RT for 3h. Progress of the reaction was monitored by TLC and LCMS. After completion, the reaction mixture was treated with water (50 mL) and filtered. The crude compound was purified by reverse phase preparative HPLC (Method A) to afford 4-(1-(5-(2-(2,6-dioxopiperidin-3-yl)-1-oxoisoindolin-4-yl)pent-4-yn-1-yl)-1H-pyrazol-4-yl)-N-(2-((R)-1-methylpyrrolidin-2-yl)-1H-pyrrolo[3,2-c]pyridin-6-yl)benzamide (**Example 21**, 56 mg, 11%) as an off-white solid.$^1$H NMR (400 MHz, DMSO-$d_6$) δ ppm 1.76-1.97 (m, 3 H), 1.98-2.10 (m, 1 H), 2.12-2.16 (m, 3 H), 2.17 (s, 3 H), 2.21-2.32 (m, 1 H), 2.35-2.47 (m, 2 H), 2.52-2.63 (m, 3 H), 2.80-3.03 (m, 1 H), 3.08-3.24 (m, 1 H), 4.25-4.40 (m, 3 H), 4.44-4.55 (m, 1 H), 5.13-5.14 (m, 1 H), 6.39 (s, 1 H), 7.49-7.52 (m, 1 H), 7.64-7.74 (m, 4 H), 8.02 (s, 1 H), 8.05-8.06 (m, 2 H), 8.20 (s, 1 H), 8.38 (s, 1 H), 8.50 (s, 1 H), 10.38 (s, 1 H), 11.36 (s, 1 H). Mass spec: m/z 695.5 [M+H]$^+$.

**Intermediate 128 was synthesised following Scheme 25**

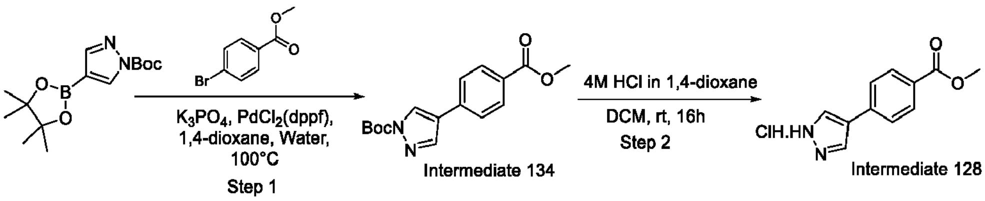


**Scheme 25**

**Step 1**

**Intermediate 134: *tert*-butyl 4-(4-(methoxycarbonyl) phenyl)-1*H*-pyrazole-1-carboxylate**

To a solution of *tert*-butyl 4-(4,4,5,5-tetramethyl-1,3,2-dioxaborolan-2-yl)-1*H*-pyrazole-1-carboxylate (CAS No: 552846-17-0, 52.67 g, 179.0 mmol) and methyl 4-bromobenzoate (CAS No: 619-42-1, 35.00 g, 162.8 mmol) in 1,4-dioxane (350 mL) was added potassium phosphate (86.26 g, 406.9 mmol) and water (100 mL). The reaction mixture was purged with argon gas for 15 min then [1,1'-bis(diphenylphosphino)ferrocene]dichloropalladium (II) (5.95 g, 8.14 mmol) was added at room temperature. The reaction mixture was further purged with argon gas for 10 min and then heated at 100°C for 16h. The reaction mixture was filtered through celite and the residue was poured into water (1000 mL) and extracted with ethyl acetate (3 × 500 mL). The combined organic phases were washed with brine solution (2 × 250 mL), dried over anhydrous $Na_2SO_4$, filtered, and concentrated *in vacuo.* The crude material was purified by combi-flash chromatography by eluting with 65% ethyl acetate in heptane to afford *tert*-butyl 4-(4-(methoxycarbonyl) phenyl)-1*H*-pyrazole-1-carboxylate **(Intermediate 134,** 37.0 g, 68.5% yield) as a pale yellow solid. $^1$H NMR (400 MHz, DMSO-$d_6$) δ ppm 1.61 (s, 9H), 3.86 (s, 3H), 7.89-8.04 (m, 4H), 8.40 (s, 1H), 8.88 (s, 1H).

**Step** 2

**Intermediate 128: methyl 4-(1*H*-pyrazol-4-yl)benzoate hydrochloride**

To a solution of *tert*-butyl 4-(4-(methoxycarbonyl)phenyl)-1*H*-pyrazole-1-carboxylate (**Intermediate 134,** 37.00 g, 122.4 mmol) in dichloromethane (100 mL) was added 4M HCl in 1,4-dioxane (50 mL) at 0°C under nitrogen and the reaction mixture was stirred at room temperature for 16h. The reaction mixture was concentrated *in vacuo* to obtain the crude compound. The crude material was triturated with ethyl acetate (2 × 100 mL) and pentane (2 × 100 mL) and dried *in vacuo* to afford methyl 4-(1*H*-pyrazol-4-yl)benzoate hydrochloride (**Intermediate 128,** 27 g, (93%) as a white solid. $^1$H NMR (400 MHz, DMSO-$d_6$) δ ppm 3.84 (s, 3H), 7.77 (d, *J*=8.31 Hz, 2H), 7.93 (d, *J*=7.82 Hz, 2H), 8.23 (s, 2H). Mass spec: m/z: 203.11 [M+H]$^+$.

**Example 22 was synthesised following Scheme 26**

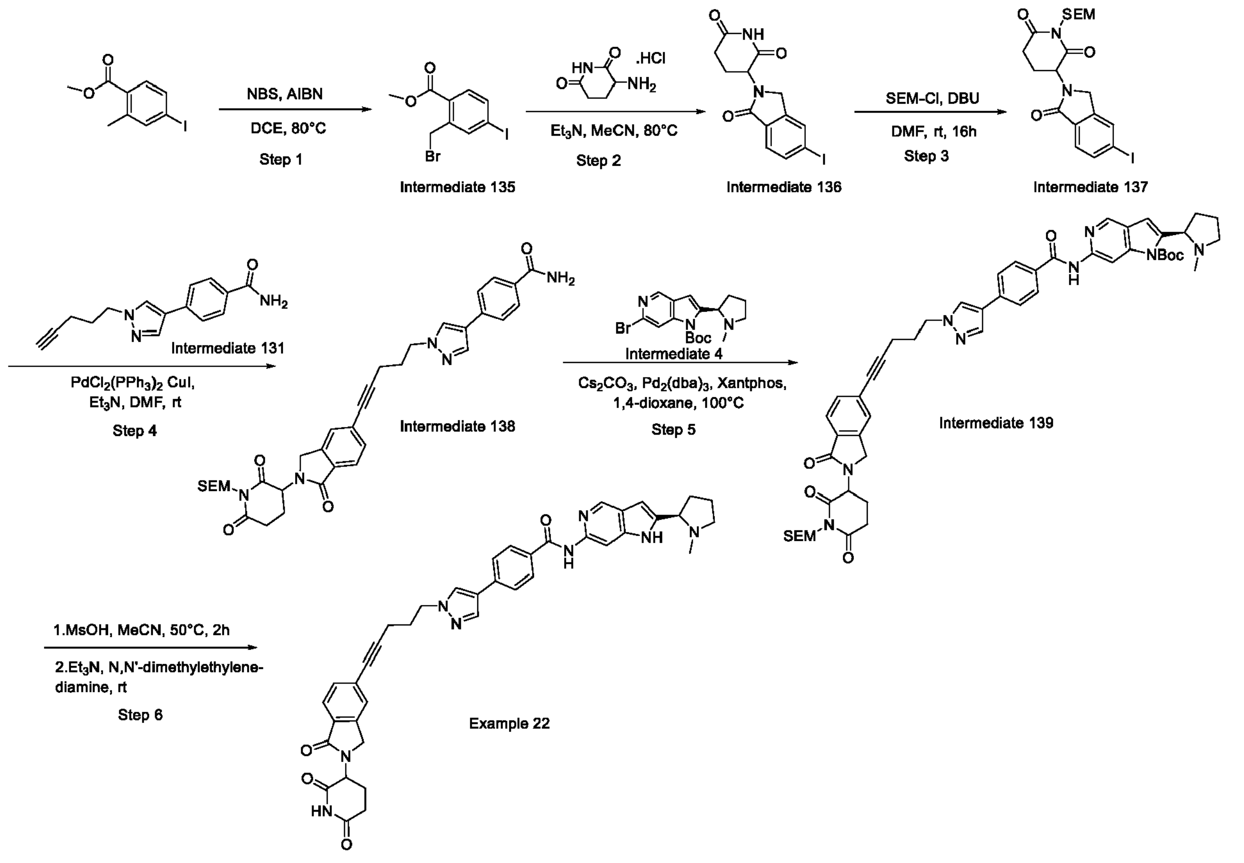


## Scheme 26

**Step 1**

**Intermediate 135: methyl 2-(bromomethyl)-4-iodobenzoate**

To solution of methyl 4-iodo-2-methylbenzoate (CAS No: 103440-53-5, 20 g, 72.45 mmol) in dichloroethane (200 mL) were added *N*-Bromosuccinimide (19.54 g, 108.7 mmol) and AIBN (9.71 g, 57.96 mmol) under nitrogen atmosphere and the reaction mixture was heated at 80°C for 14h. The reaction mixture was quenched with water (100 mL) and extracted with dichloromethane (2 × 100 mL). The organic layers were separated, washed with brine solution (3 × 200 mL), dried over anhydrous $Na_2SO_4$, and concentrated *in vacuo* to afford methyl 2-(bromomethyl)-4-iodobenzoate (**Intermediate 135,** 15.0 g, 58%) as a brown solid, which was used directly into the next step without further purification. $^1$H NMR (400 MHz, DMSO-$d_6$) δ ppm 3.92 (s, 3H), 4.99 (s, 2H), 7.59-7.68 (m, 1H), 7.80-7.87 (m, 1H), 8.01-8.05 (m, 1H).

**Step 2**

**Intermediate 136: 3-(5-iodo-1-oxoisoindolin-2-yl) piperidine-2, 6-dione**

To a solution of methyl 2-(bromomethyl)-4-iodobenzoate (**Intermediate 135,** 15 g, 42.25 mmol) in acetonitrile (230 mL) was added 3-aminopiperidine-2,6-dione hydrochloride (CAS No: 24666-56-6, 10.43 g, 63.38 mmol) followed by triethylamine (12.9 g, 126.77 mmol) at 0°C under a nitrogen atmosphere. The reaction mixture was slowly warmed to room temperature and heated at 80°C for 14h. The reaction mixture was quenched with water (100 mL) and extracted with ethyl acetate (2 × 400 mL). The combined organic phases were washed with brine solution (2 × 100 mL), dried over anhydrous $Na_2SO_4$, and concentrated *in vacuo* to obtain the crude compound which was purified by combi-flash chromatography, by eluting with 1-2% ethyl acetate, in heptane to afford 3-(5-iodo-1-oxoisoindolin-2-yl) piperidine-2, 6-dione (**Intermediate 136,** 12.0 g, 77%) as an off white solid. $^1$H NMR (400 MHz, DMSO-$d_6$) δ ppm 1.98-2.01 (m, 1H), 2.35-2.40 (m, 1H), 2.57-2.61 (m, 1H), 2.86-2.97 (m, 1H), 4.28-4.33 (m, 1H), 4.41-4.46 (m, 1H), 5.07-5.12 (m, 1H), 7.51 (d, *J*=7.60 Hz, 1H), 7.89 (d, *J*=7.60 Hz, 1H), 8.06 (s, 1H), 10.99 (br s, 1H). Mass spec: m/z 371.0 [M+H]$^+$.

**Step 3**

**Intermediate 137: 3-(5-iodo-1-oxoisoindolin-2-yl)-1-((2-(trimethylsilyl)ethoxy)methyl)piperidine-2, 6-dione**

To a solution of 3-(5-iodo-1-oxoisoindolin-2-yl)piperidine-2,6-dione (**Intermediate 136, 2.5** g, 6.8 mmol) in dimethylformamide (20 mL) was added DBU (2.1 g, 14 mmol) followed by 2-(trimethylsilyl)ethoxymethyl chloride (1.8 g, 10 mmol) at 0°C under a nitrogen atmosphere and stirred the reaction mixture at room temperature for 16h. The reaction mixture was quenched with water (50 mL) and extracted with ethyl acetate (2 × 100 mL). The organic layers were washed with brine solution (2 × 200 mL), separated, dried over anhydrous $Na_2SO_4$, and concentrated *in vacuo* to obtain crude compound. The crude material was purified by combi-flash chromatography, by eluting with 2% MeOH in DCM, to afford 3-(5-iodo-1-oxoisoindolin-2-yl)-1-((2-(trimethylsilyl)ethoxy) methyl)piperidine-2, 6-dione (**Intermediate 137,** 0.85 g, 25%) as a dark blue solid. $^1$H NMR (400 MHz, DMSO-$d_6$) δ ppm -0.02 (s, 9H), 0.81-0.85 (m, 2H), 2.04-2.06 (m, 1H), 2.33-2.40 (m, 1H), 2.76-2.81 (m, 1H), 3.02-3.09 (m, 1H), 3.51-3.53 (m, 2H), 4.26-4.31 (m, 1H), 4.44-4.48 (m, 1H), 5.01-5.08 (m, 2H), 5.19-5.23 (m, 1H), 7.53 (d, *J*=7.60 Hz, 1H), 7.90 (d, *J*=7.60 Hz, 1H), 8.07 (s, 1H).

**Step 4**

**Intermediate 138: 4-(1-(5-(2-(2,6-dioxo-1-((2-(trimethylsilyl)ethoxy)methyl)piperidin-3-yl)-1-oxoisoindolin-5-yl) pent-4-yn-1-yl)-1*H*-pyrazol-4-yl) benzamide**

To a solution of 4-(1-(pent-4-yn-1-yl)-1*H*-pyrazol-4-yl)benzamide (**Intermediate 131,** 0.45 g, 1.77 mmol) in dimethylformamide (10.0 mL) was added 3-(5-iodo-1-oxoisoindolin-2-yl)-1-((2-(trimethylsilyl) ethoxy)methyl)piperidine-2,6-dione (**Intermediate 137,** 0.88 g, 1.77 mmol) followed by triethylamine (6.32 g, 62.18 mmol). The reaction mixture was purged with argon gas for 15 min then copper(I) iodide (0.03 g, 0.17 mmol) followed by $PdCl_2(PPh_3)_2$ (0.13 g, 0.17 mmol) were added and the reaction was stirred for 2h at room temperature. The reaction mixture was diluted with water (100 mL) and extracted with ethyl acetate (3 × 100 mL). The organic layers were separated, dried over anhydrous $Na_2SO_4$, and concentrated *in vacuo* to obtain crude compound. The crude material was purified by combi-flash chromatography, by eluting with 100% ethyl acetate in heptane, to afford 4-(1-(5-(2-(2,6-dioxo-1-((2-(trimethylsilyl) ethoxy)methyl)piperidin-3-yl)-1-oxoisoindolin-5-yl)pent-4-yn-1-yl)-1*H*-pyrazol-4-yl)benzamide (**Intermediate 138,** 0.53 g, 59%) as a yellow solid. $^1$H NMR (400 MHz, DMSO-$d_6$) δ ppm -0.02 (s, 9H), 0.81-0.86 (m, 2H), 2.01-2.14 (m, 3H), 2.33-2.42 (m, 1H), 2.71-2.73 (m, 2H), 2.77-2.81 (m, 1H), 3.02-3.11 (m, 1H), 3.49-3.55 (m, 2H), 4.26-4.31 (m, 3H), 4.38-4.43 (m, 1H), 5.01-5.08 (m, 2H), 5.20-5.25 (m, 1H), 7.31 (br s, 1H), 7.53 (d, *J*=8.40 Hz, 1H), 7.62-7.69 (m, 4H), 7.86 (d, *J*=8.40 Hz, 2H), 7.93-7.95 (m, 1H), 8.00 (s, 1H), 8.35 (br s, 1H). Mass spec: m/z: 626.0 $[M+H]^+$.

**Step 5**

**Intermediate 139: *tert*-butyl 6-(4-(1-(5-(2-(2,6-dioxo-1-((2-(trimethylsilyl)ethoxy)methyl)piperidin-3-yl)-1-oxoisoindolin-5-yl)pent-4-yn-1-yl)-1*H*-pyrazol-4-yl)benzamido)-2-((*R*)-1-methylpyrrolidin-2-yl)-1*H*-pyrrolo[3,2-c]pyridine-1-carboxylate**

To a solution of 4-(1-(5-(2-(2,6-dioxo-1-((2-(trimethylsilyl)ethoxy)methyl)piperidin-3-yl)-1-oxoisoindolin-5-yl) pent-4-yn-1-yl)-1*H*-pyrazol-4-yl)benzamide (**Intermediate 138**, 0.30 g, 0.47 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate (**Intermediate 4,** 0.18 g, 0.47mmol) followed by cesium carbonate (0.31 g, 0.96 mmol) at room temperature. The reaction mixture was purged with argon gas for 15 min then $Pd_2(dba)_3$ (0.045 g, 0.047 mmol) and Xantphos (0.085 g, 0.14 mmol) were added. The reaction mixture was further purged with argon gas for 10 min and heated at 100°C for 2h. The reaction mixture was then concentrated *in vacuo* to obtain crude compound. The crude material purified by combi-flash chromatography, by eluting with 80% ethyl acetate in heptane, to afford *tert*-butyl 6-(4-(1-(5-(2-(2,6-dioxo-1-((2-(trimethylsilyl)ethoxy)methyl)piperidin-3-yl)-1-oxoisoindolin-5-yl)pent-4-yn-1-yl)-1*H*-pyrazol-4-yl)benzamido)-2-((*R*)-1-methylpyrrolidin-2-yl)-1*H*-pyrrolo [3,2-c]pyridine-1-carboxylate (**Intermediate 139**, 0.31 g, 70%) as a yellow solid. $^1$H NMR (400 MHz, DMSO-$d_6$) δ ppm -0.03 (s, 9H), 0.79-0.85 (m, 2H), 1.61-1.66 (m, 2H), 1.70 (s, 9H), 1.68-1.70 (m, 2H), 1.74-1.77 (m, 1H), 2.01-2.07 (m, 2H), 2.31-2.33 (m, 1H), 2.36 (s, 3H), 2.36-2.38 (m, 2H), 2.52-2.55 (m, 2H), 2.72-2.76 (m, 1H), 3.03-3.07 (m, 1H), 3.37-3.51 (m, 2H), 3.90-3.94 (m, 1H), 4.18-4.22 (m, 1H), 4.28-4.32 (m, 2H), 4.36-4.40 (m, 1H), 4.95-4.99 (m, 1H), 5.03-5.06 (m, 1H), 5.19-5.23 (m, 1H), 6.75 (s, 1H), 7.52 (d, *J*=7.64 Hz, 1H), 7.58 (s, 1H), 7.68 (d, *J*=7.64 Hz, 1H), 7.70-7.73 (m, 2H), 8.03-8.05 (m, 2H), 8.07 (s, 1H), 8.41 (s, 1H), 8.59 (s, 1H), 8.95 (s, 1H), 10.65 (s, 1H). Mass spec: m/z: 924.8 $[M-H]^-$.

**Step 6**

**Example 22: 4-(1-(5-(2-(2,6-dioxopiperidin-3-yl)-1-oxoisoindolin-5-yl)pent-4-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide**

To a solution of *tert*-butyl 6-(4-(1-(5-(2-(2,6-dioxo-1-((2-(trimethylsilyl)ethoxy)methyl)piperidin-3-yl)-1-oxoisoindolin-5-yl)pent-4-yn-1-yl)-1*H*-pyrazol-4-yl)benzamido)-2-((*R*)-1-methylpyrrolidin-2-yl)-1*H*-pyrrolo[3,2-c]pyridine-1-carboxylate (**Intermediate 139,** 0.40 g, 0.43 mmol) in acetonitrile (10 mL) was added methanesulfonic acid (0.28 mL, 4.32 mmol) at room temperature and the reaction mixture was heated at 50°C for 2h. After cooling to room temperature, *N*, *N'*-dimethylethylenediamine (0.21 g, 2.162 mmol) followed by triethylamine (0.87 g, 8.64 mmol) was added the reaction was stirred for 3h at room temperature. The reaction mixture was poured into ice cold water (20 mL), the resulting precipitate was filtered and dried under vacuum to obtain crude compound. The crude material was purified by preparative HPLC (Method A) to afford 4-(1-(5-(2-(2,6-dioxopiperidin-3-yl)-1-oxoisoindolin-5-yl)pent-4-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide (**Example 22**, 0.065 g, 22%) as an off white solid. $^1$H NMR (400 MHz, DMSO-$d_6$) δ ppm 1.78-1.95 (m, 3H), 1.97-2.03 (m, 1H), 2.11-2.16 (m, 3H), 2.17 (s, 3H), 2.23-2.30 (m, 1H), 2.36-2.41 (m, 1H), 2.52-2.54 (m, 2H), 2.57-2.62 (m, 1H), 2.85-2.94 (m, 2H), 3.12-3.16 (m, 1H), 4.27-4.32 (m, 3H), 4.37-4.41 (m, 1H), 5.08-5.13 (m, 1H), 6.40 (s, 1H), 7.52 (d, *J*=7.83 Hz, 1H), 7.61 (s, 1H), 7.65-7.72 (m, 3H), 8.01-8.08 (m, 3H), 8.20 (s, 1H), 8.39 (s, 1H), 8.51 (s, 1H), 10.38 (s, 1H), 11.06 (s, 1H), 11.36 (s, 1H). Mass spec: m/z: 694.8 [M+H]$^+$.

**Example 23 was synthesised following Scheme 27**

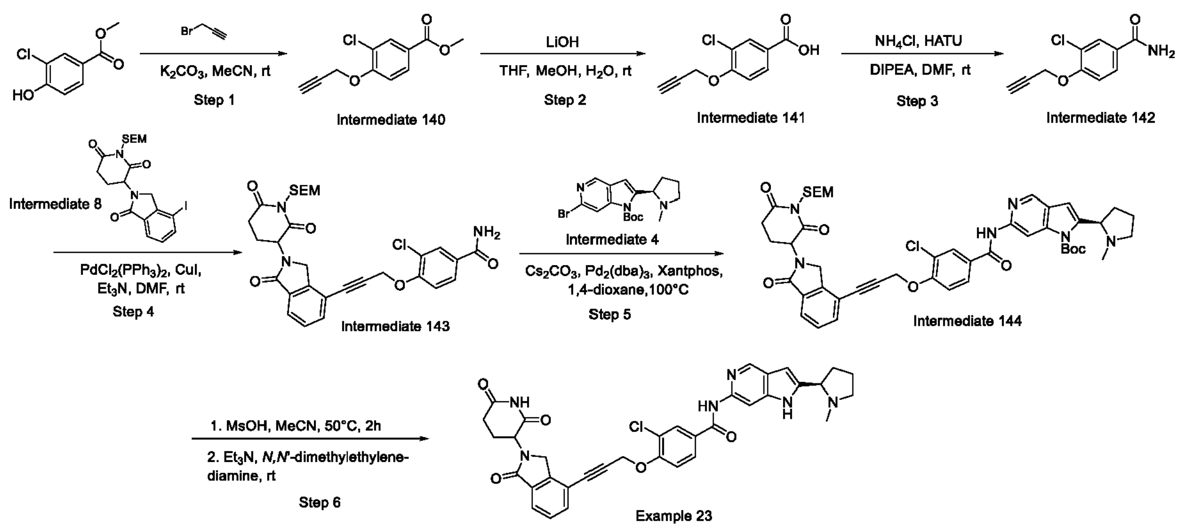


**Scheme 27**

**Step 1**

**Intermediate 140: methyl 3-chloro-4-(prop-2-yn-1-yloxy)benzoate**

To solution of methyl 3-chloro-4-hydroxy-benzoate (CAS No: 3964-57-6, 10 g, 53.59 mmol) in acetonitrile (100 mL) was added potassium carbonate (8.87 g, 64.31 mmol) followed by 3-bromoprop-1-yne (CAS No: 106-96-7, 7.65 g, 64.31 mmol) at room temperature and the reaction mixture was stirred for 6h. The reaction mixture was then diluted with water (1000 mL) and extracted with dichloromethane (3 × 500 mL). The organic layers were separated, dried over anhydrous $Na_2SO_4$, filtered, and concentrated *in vacuo.* The crude material was purified by combi-flash chromatography, by eluting with 90% ethyl acetate in heptane, to afford methyl 3-chloro-4-(prop-2-yn-1-yloxy) benzoate (**Intermediate 140,** 11 g, 83%) as a white solid. $^1$H NMR (400 MHz, DMSO-$d_6$) δ ppm 3.69 (s, 1H), 3.84 (s, 3H), 5.04 (s, 2H), 7.30-7.35 (m, 1H), 7.91-7.95 (m, 2H).

**Step 2**

**Intermediate 141: 3-chloro-4-(prop-2-yn-1-yloxy) benzoic acid**

To a solution of methyl 3-chloro-4-(prop-2-yn-1-yloxy)benzoate (**Intermediate 140,** 11 g, 48.96 mmol) in tetrahydrofuran (50 mL) was added MeOH (50 mL) and water (25 mL) followed by lithium hydroxide (4.78 g, 195.87 mmol) at 0°C and the reaction mixture was stirred at room temperature for 6h. The reaction mixture was then diluted with water (5 mL) and concentrated *in vacuo.* The pH of the aqueous layer was adjusted to pH~4 by dropwise addition 0.5 N HCl. The resulting suspension was extracted with ethyl acetate (2 × 250 mL). The organic layers were separated, dried over anhydrous $Na_2SO_4$, filtered, and concentrated under reduced pressure to afford 3-chloro-4-(prop-2-yn-1-yloxy) benzoic acid (**Intermediate 141,** 9.60 g, 93%) as an off-white solid, which was used for the next step without further purification. $^1$H NMR (400 MHz, DMSO-$d_6$) δ ppm 3.68 (s, 1H), 5.02 (s, 2H), 7.29-7.32 (m, 1H), 7.89-7.92 (m, 2H). Mass spec: m/z: 208.9 [M-H]$^-$.

**Step 3**

**Intermediate 142: 3-chloro-4-(prop-2-yn-1-yloxy)benzamide**

To a solution of 3-chloro-4-(prop-2-yn-1-yloxy)benzoic acid (**Intermediate 141,** 10.5 g, 49.9 mmol) in dimethylformamide (100 mL) was added HATU (29.9 g, 74.8 mmol) and stirred for 10 min at rt. To the reaction mixture was added ammonium chloride (8.72 mL, 249 mmol) followed by DIPEA (19.7 g, 150 mmol) at room temperature and the reaction mixture was stirred for 16h. The reaction mixture was then diluted with water (500 mL) and extracted with ethyl acetate (3 × 250 mL). The organic layers were separated, dried over anhydrous $Na_2SO_4$, filtered, and concentrated *in vacuo* to afford 3-chloro-4-(prop-2-yn-1-yloxy) benzamide (**Intermediate 142,** 8.00 g, 76%) as a white solid, which was used for the next step without further purification. $^1$H NMR (400 MHz, DMSO-$d_6$) δ ppm 3.70 (s, 1H), 5.00 (s, 2H), 7.25-7.28 (m, 1H), 7.36 (br s, 1H), 7.84-7.92 (m, 2H), 7.96 (br s, 1H). Mass spec: m/z 209.9 [M+H]$^+$.

**Step 4**

**Intermediate 143: 3-chloro-4-((3-(2-(2,6-dioxo-1-((2-(trimethylsilyl)ethoxy)methyl)piperidin-3-yl)-1-oxoisoindolin-4-yl)prop-2-yn-1-yl)oxy)benzamide:**

To a solution of 3-chloro-4-(prop-2-yn-1-yloxy) benzamide (**Intermediate 142,** 1 g, 4.77 mmol) and 3-(4-iodo-1-oxoisoindolin-2-yl)-1-(2-trimethylsilylethoxymethyl)piperidine-2,6-dione (**Intermediate 8**, 2.38 g, 4.77 mmol) in dimethylformamide (10 mL) was added triethylamine (24.1 mL, 171.73 mmol) at room temperature. The reaction mixture was purged with argon gas for 15 min then $PdCl_2(PPh_3)_2$ (0.34 g, 0.477 mmol) and copper (I) iodide (0.093 g, 0.477 mmol) were added at room temperature. The reaction mixture was further purged with argon gas for 10 min and stirred at room temperature for 2h. The reaction mixture was concentrated *in vacuo* to obtain crude compound. This crude material was purified by combi-flash chromatography, by eluting with 80% ethyl acetate in heptane, to afford 3-chloro-4-((3-(2-(2,6-dioxo-1-((2-(trimethylsilyl)ethoxy)methyl)piperidin-3-yl)-1-oxoisoindolin-4-yl) prop-2-yn-1-yl)oxy) benzamide (**Intermediate 143,** 1.6 g, 58%) as a yellow solid. $^1$H NMR (400 MHz, DMSO-$d_6$) δ ppm -0.03 (s, 9H), 0.81-0.88 (m, 2H), 2.03-2.09 (m, 1H), 2.27-236 (m, 1H), 2.79-2.83 (m, 3H), 3.03-3.12 (m, 1H), 3.50-3.55 (m, 2H), 4.13-4.18 (m, 1H), 4.32-4.36 (m, 1H), 5.03-5.09 (m, 1H), 5.31 (s, 2H), 7.35 (br s, 1H), 7.40-7.55 (m, 1H), 7.58-7.71 (m, 1H), 7.72-7.78 (m, 1H), 7.80-7.87 (m, 1H), 7.89-7.93 (m, 1H), 7.95-7.98 (m, 1H), 8.00 (br s, 1H). Mass spec: m/z 579.9 [M-H]$^-$.

**Step 5**

**Intermediate 144: *tert*-butyl 6-(3-chloro-4-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((*R*)-1-methylpyrrolidin-2-yl)-1*H*-pyrrolo[3,2-c]pyridine-1-carboxylate**

To a solution of 3-chloro-4-((3-(2-(2,6-dioxo-1-((2-(trimethylsilyl)ethoxy)methyl)piperidin-3-yl)-1-oxoisoindolin-4-yl) prop-2-yn-1-yl)oxy) benzamide (**Intermediate 143,** 1.22 g, 2.10 mmol) and *tert*-butyl (*R*)-6-bromo-2-(1-methylpyrrolidin-2-yl)-1*H*-pyrrolo[3,2-c]pyridine-1-carboxylate (**Intermediate 4,** 1.00 g, 2.63 mmol) in 1,4-dioxane (20 mL) was added cesium carbonate (2.57 g, 7.88 mmol). The reaction mixture was purged with argon gas for 15 min then Xantphos (0.47 g, 0.788 mmol) and $Pd_2(dba)_3$ (0.37 g, 0.39 mmol) were added at room temperature. The reaction mixture was further purged with argon gas for another 10 min and heated at 100°C for 2h. The reaction mixture was then concentrated *in vacuo.* The resulting crude material was purified by combi-flash chromatography, by eluting with 6% MeOH in DCM, to afford *tert*-butyl 6-(3-chloro-4-((3-(2-(2,6-dioxo-1-((2-(trimethylsilyl)ethoxy)methyl)piperidin-3-yl)-1-oxoisoindolin-4-yl) prop-2-yn-1-yl)oxy)benzamido)-2-((*R*)-1-methylpyrrolidin-2-yl)-1*H*-pyrrolo[3,2-c]pyridine-1-carboxylate (**Intermediate**

**144,** 0.34 g, 15%) as a yellow solid. $^1$H NMR (400 MHz, DMSO-$d_6$) δ ppm -0.08 (s, 9H), 0.71-0.87 (m, 2H), 1.07-1.11 (m, 2H), 1.51-1.59 (m, 2H), 1.70 (s, 9H), 2.23-2.32 (m, 2H), 2.35 (s, 3H), 2.37-2.42 (m, 2H), 3.01-3.17 (m, 2H), 3.90-3.94 (m, 2H), 4.14-4.16 (m, 1H), 4.33-4.36 (m, 2H), 4.96-5.02 (m, 2H), 5.21-5.26 (m, 1H), 5.35 (s, 2H), 6.75 (s, 1H), 7.42-7.48 (m, 1H), 7.54-7.60 (m, 1H), 7.74-7.77 (m, 1H), 7.79-7.85 (m, 1H), 8.08-8.10 (m, 1H), 8.15-8.20 (m, 1H), 8.59 (s, 1H), 8.91 (s, 1H), 10.73 (br s, 1H). Mass spec: m/z 881.0 [M+H]$^+$.

**Step 6**

**Example 23: 3-chloro-4-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)benzamide**

To a solution of *tert*-butyl 6-(3-chloro-4-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((*R*)-1-methylpyrrolidin-2-yl)-1*H*-pyrrolo[3,2-c]pyridine-1-carboxylate (**Intermediate 144,** 0.30 g, 0.34 mmol) in acetonitrile (25 mL) was added methanesulfonic acid (0.22 mL, 3.403 mmol) at room temperature and the reaction mixture was heated at 50°C for 2h. The reaction mixture was cooled to room temperature then *N*, *N'*-dimethylethylenediamine (0.20 mL, 1.70 mmol) followed by triethylamine (0.95 mL, 6.80 mmol) were added and the reaction stirred at room temperature and for 3h. The reaction mixture was concentrated *in vacuo* and with water (20 mL) added resulting in s solid precipitating. The solid was filtered and and purified by preparative HPLC (Method A) to afford 3-chloro-4-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)benzamide (**Example 23**, 0.046 g, 21%) as an off white solid. $^1$H NMR (400 MHz, DMSO-$d_6$) δ ppm 1.77-1.94 (m, 3H), 1.98-2.04 (m, 1H), 2.11-2.14 (m, 1H), 2.17 (s, 3H), 2.22-2.30 (m, 1H), 2.38-2.43 (m, 1H), 2.59-2.64 (m, 1H), 2.86-2.96 (m, 1H), 3.12-3.16 (m, 1H), 3.33-3.35 (m, 1H), 4.28-4.33 (m, 1H), 4.39-4.43 (m, 1H), 5.13-5.18 (m, 1H), 5.36 (s, 2H), 6.40 (s, 1H), 7.47- 7.51 (m, 1H), 7.54-7.59 (m, 1H), 7.72-7.76 (m, 1H), 7.77-7.80 (m, 1H), 8.08-8.12 (m, 1H), 8.18 (s, 2H), 8.50 (s, 1H), 10.47 (br s, 1H), 11.22 (br s, 1H), 11.39 (br s, 1H). Mass spec: m/z 651.0 [M+H]$^+$.

**Examples 24-25** in **Table 1** were synthesised in an analogous manner to **Example 23,** following **Step 6** from **Scheme 27** by deprotection of the appropriate **Intermediate 145** or **146**

**Table 1**

| Example | Example Structure and Name | Intermediate | $^1$H NMR (400 MHz, DMSO-d6) δ ppm | Mass spec m/z |
|---|---|---|---|---|
| **24** | 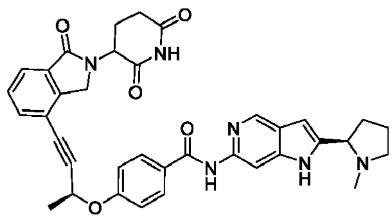 4-(((2S)-4-(2-(2,6-dioxopiperidin-3-yl)-1-oxoisoindolin-4-yl)but-3 -yn-2-yl) oxy)-N-(2-((R)-1-methylpyrrolidin-2-yl)-1H-pyrrolo[3,2-c]pyridin-6-yl)benzamide | **145** | 1.73 (d, J=6.40 Hz, 3H), 1.78-2.01 (m, 4H), 2.10-2.15 (m, 1H), 2.17 (s, 3H), 2.23-2.30 (m, 1H), 2.31-2.36 (m, 1H), 2.59-2.64 (m, 2H), 2.86-2.97 (m, 1H), 3.10-3.18 (m, 1H), 4.19-4.23 (m, 1H), 4.31-4.36 (m, 1H), 5.16-5.21 (m, 1H), 5.52-5.57 (m, 1H), 6.41 (s, 1H), 7.19 (d, J=8.80 Hz, 2H), 7.53 (m, 1H), 7.67 (d, J=6.80 Hz, 1H), 7.75 (d, J=6.80 Hz, 1H), 8.04 (d, J=8.80 Hz, 2H), 8.21 (s, 1H), 8.49 (s, 1H), 10.22 (s, 1H), 11.39 (s, 1H), 11.54 (s, 1H) | 631.0 [M+H]+ |

(continued)

| Example | Example Structure and Name | Intermediate | $^1$H NMR (400 MHz, DMSO-d6) $\delta$ ppm | Mass spec m/z |
|---|---|---|---|---|
| **25** | 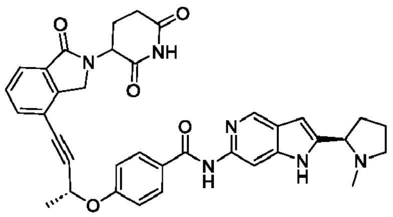 4-(((2R)-4-(2-(2,6-dioxopiperidin-3-yl)-1-oxoisoindolin-4-yl)but-3 -yn-2-yl) oxy)-N-(2-((R)-1-methylpyrrolidin-2-yl)-1H-pyrrolo[3,2-c]pyridin-6-yl)benzamide | **146** | 1.72 (d, J=5.60 Hz, 3H), 1.78-1.92 (m, 3H), 1.94-2.00 (m, 2H), 2.12-2.14 (m, 1H), 2.16 (s, 3H), 2.23-2.29 (m, 1H), 2.33-2.39 (m, 1H), 2.59-2.64 (m, 1H), 2.87-2.96 (m, 1H), 3.12-3.16 (m, 1H), 4.18-4.23 (m, 1H), 4.31-4.37 | 631.0 [M+H]+ |
| | | | (m, 1H), 5.16-5.21 (m, 1H), 5.52-5.57 (m, 1H), 6.41 (s, 1H), 7.19 (d, J=9.00 Hz, 2H), 7.53 (t, J=7.20 Hz, 1H), 7.67 (d, J=7.20 Hz, 1H), 7.75 (d, J=7.20 Hz, 1H), 8.04 (d, J=9.00 Hz, 2H), 8.21 (s, 1H), 8.49 (s, 1H), 10.23 (s, 1H), 11.40 (s, 1H), 11.57 (s, 1H) | |

**Intermediates 145-146** shown in **Table 2** were prepared in an analogous manner to **Intermediate 144,** following **Step 5** from **Scheme 27,** using **Intermediate 4** and the appropriate amide **Intermediates 147-148.**

**Table 2**

| Intermediate | Intermediate Structure and Name | Amide Intermediate | $^1$H NMR (400 MHz, DMSO-d6) $\delta$ ppm | Mass spec m/z |
|---|---|---|---|---|
| **145** | 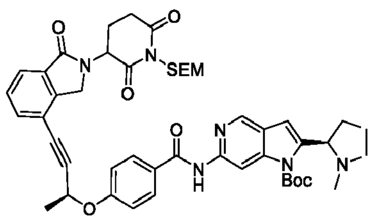 tert-butyl 6-(4-(((2S)-4-(2-(2,6-dioxo-1-((2-(trimethylsilyl)ethoxy)methyl)piperidin-3-yl)-1-oxoisoindolin-4-yl)but-3-yn-2-yl)oxy)benzamido)-2-((R)-1-methylpyrrolidin-2-yl)-1H-pyrrolo[3,2-c]pyridine-1-carboxylate | **147** | -0.07 (s, 9H), 0.72-0.79 (m, 2H), 1.57-1.67 (m, 4H), 1.71 (s, 9H), 1.73 (m, 3H), 1.98-2.07 (m, 1H), 2.23-2.32 (m, 3H), 2.36 (s, 3H), 2.71-2.81 (m, 1H), 2.98-3.07 (m, 1H), 3.41-3.46 (m, 2H), 3.91-3.97 (m, 1H), 4.01-4.09 (m, 1H), 4.17-4.25 (m, 1H), 4.92-5.01 (m, 2H), 5.16-5.23 (m, 1H), 5.55-5.60 (m, 1H), 6.75 (s, 1H), 7.17-7.20 (m, 2H), 7.53-7.57 (m, 1H), 7.68 (d, J=7.80 Hz, 1H), | 861.1 [M+H]$^+$ |
| | | | 7.76 (d, J=7.80 Hz, 1H), 8.06-8.09 (m, 2H), 8.58 (s, 1H), 8.94 (s, 1H), 10.53 (br s, 1H) | |

(continued)

| Intermediate | Intermediate Structure and Name | Amide Intermediate | $^1$H NMR (400 MHz, DMSO-d6) δ ppm | Mass spec m/z |
|---|---|---|---|---|
| **146** | 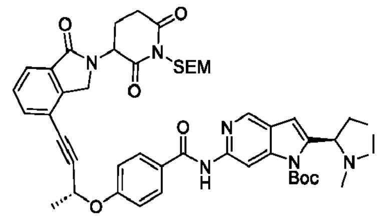 tert-butyl 6-(4-(((2R)-4-(2-(2,6-dioxo-1-((2-(trimethylsilyl)ethoxy)methyl)piperidin-3-yl)-1-oxoisoindolin-4-yl)but-3-yn-2-yl)oxy)benzamido)-2-((R)-1-methylpyrrolidin-2-yl)-1H-pyrrolo[3,2-c]pyridine-1-carboxylate | **148** | -0.07 (s, 9H), 0.74-0.77 (m, 2H), 1.57-1.71 (m, 1H), 1.70 (s, 9H), 1.72 (s, 3H), 1.98-2.07 (m, 2H), 2.27-2.32 (m, 3H), 2.35 (s, 3H), 2.70-2.78 (m, 1H), 2.97-3.06 (m, 1H), 3.11-3.13 (m, 1H), 3.41-3.46 (m, 2H), 3.92-4.09 (m, 3H), 4.17-4.25 (m, 1H), 4.95-4.99 (m, 2H), 5.17-5.20 (m, 1H), 5.55-5.57 (m, 1H), 6.74 (s, 1H), 7.18 (d, J=7.20 Hz, 2H), 7.54 (t, J=7.20 Hz, 1H), 7.67 (d, J=7.20 Hz, 1H), 7.75 (d, J=7.20 Hz, 1H), 8.07 (d, J=7.20 Hz, 2H), 8.57 (s, 1H), 8.92 (s, 1H), 10.52 (br s, 1H) | 861.7 [M+H]$^+$ |

**Intermediates 147-148** shown in **Table 3** were synthesised in an analogous manner to **Intermediate 143,** following **Step 4** from **Scheme 27,** using **Intermediate 8** and the appropriate alkyne **Intermediates 149-150.**

**Table 3**

| Intermediate | Intermediate Structure and Name | Alkyne Intermediate | $^1$H NMR (400 MHz, DMSO-d6) δ ppm | Mass spec m/z |
|---|---|---|---|---|
| **147** | 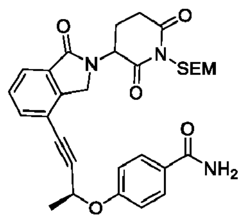 4-(((2S)-4-(2-(2,6-dioxo-1-((2-(trimethylsilyl)ethoxy)met hyl) piperidin-3-yl)-1-oxoisoindolin-4-yl) but-3-yn-2-yl)oxy)benzamide | **149** | -0.02 (s, 9H), 0.84-1.86 (m, 2H), 1.70 (d, J=5.81 Hz, 3H), 1.99-2.03 (m, 1H), 2.22-2.30 (m, 1H), 2.73-2.89 (m, 1H), 3.03-3.10 (m, 1H), 3.50-3.61 (m, 2H), 3.94-4.22 (m, 2H), 5.04-5.12 (m, 2H), 5.19-5.22 (m, 1H), 5.50-5.53 (m, 1H), 7.12 (d, J=8.00 Hz, 2H), 7.19 (br s, 1H), 7.52-7.56 (m, 1H), 7.65-7.67 (m, 1H), 7.75-7.77 (m, 1H), 7.82 (br s, 1H), 7.87 (d, J=8.00 Hz, 2H) | 560.0 [M-H]$^-$ |
| **148** | 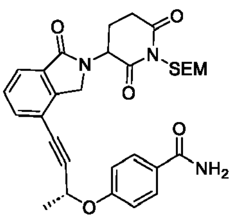 4-(((2R)-4-(2-(2,6-dioxo-1-((2-(trimethylsilyl)ethoxy)met hyl)piperidin-3-yl)-1-oxoisoindolin-4-yl)but-3-yn-2-yl)oxy)benzamide | **150** | -0.02 (s, 9H), 0.84-0.86 (m, 2H), 1.69 (d, J=6.00 Hz, 3H), 1.99-2.03 (m, 1H), 2.24-2.35 (m, 1H), 2.73-2.89 (m, 1H), 3.03-3.10 (m, 1H), 3.50-3.61 (m, 2H), 3.98-4.22 (m, 2H), 5.04-5.12 (m, 2H), 5.19-5.22 (m, 1H), 5.50-5.53 (m, 1H), 7.12 (d, J=7.60 Hz, 2H), 7.18 (br s, 1H), 7.54 (t, J=7.20 Hz, 1H), 7.66 (d, J=7.20 Hz, 1H), 7.75 (d, J=7.20 Hz, 1H), 7.81 (br s, 1H), 7.86 (d, J=7.60 Hz, 2H) | 560.0 [M-H]$^-$ |

**Intermediates 149-150** shown in **Table 4** were prepared in an analogous manner to **Intermediate 142,** following **Step 3** from **Scheme 27,** using the appropriate acid **Intermediates 151-152.**

**Table 4**

| Intermediate | Intermediate Structure and Name | Acid Intermediate | $^1$H NMR (400 MHz, DMSO-d6) δ ppm | Mass spec m/z |
|---|---|---|---|---|
| **149** | 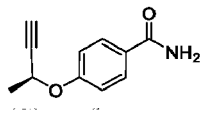 (S)-4-(but-3 -yn-2-yloxy) benzamide | **151** | 1.56 (d, J=6.00 Hz, 3H), 3.55 (s, 1H), 5.15-5.21 (m, 1H), 7.02 (d, J=8.00 Hz, 2H), 7.18 (br s, 1H), 7.82-7.85 (m, 3H) | 190.0 [M+H]$^+$ |
| **150** | 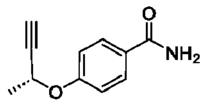 (R)-4-(but-3 -yn-2-yloxy) benzamide | **152** | 1.56 (d, J=6.00 Hz, 3H), 3.55 (s, 1H), 5.18-5.20 (m, 1H), 7.02 (d, J=7.20 Hz, 2H), 7.19 (br s, 2H), 7.82-7.85 (m, 3H) | 190.0 [M+H]$^+$ |

**Intermediates 151-152** shown in **Table 5** were prepared in an analogous manner to **Intermediate 141,** following **Step 2** from **Scheme 27,** using the appropriate ester **Intermediates 153-154.**

**Table 5**

| Intermediate | Intermediate Structure and Name | Ester Intermediate | $^1$H NMR (400 MHz, DMSO-d6) δ ppm | Mass spec m/z |
|---|---|---|---|---|
| **151** | 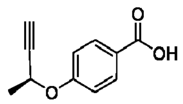 (S)-4-(but-3-yn-2-yloxy)benzoic acid | **153** | 1.57 (d, J=6.40 Hz, 3H), 3.57 (s, 1H), 5.18-5.23 (m, 1H), 7.07 (d, J=8.77 Hz, 2H), 7.97 (d, J=8.77 Hz, 2H), 12.65 (br s, 1H). | 191.0 [M+H]$^+$ |
| **152** | 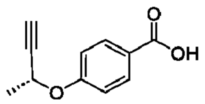 (R)-4-(but-3-yn-2-yloxy)benzoic acid | **154** | 1.57 (d, J=5.60 Hz, 3H), 3.57 (s, 1H), 5.17-5.22 (m, 1H), 7.06 (d, J=7.80 Hz, 2H), 7.89 (d, J=7.80 Hz, 2H), 12.65 (br s, 1H) | 191.0 [M+H]$^+$ |

**Intermediate 153: methyl (S)-4-(but-3-yn-2-yloxy)benzoate**

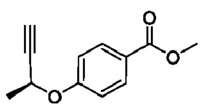

To solution of methyl 4-hydroxybenzoate (CAS No: 99-76-3, 10.0 g, 65.7 mmol), (R)-but-3-yn-2-ol (CAS No: 42969-65-3, 4.61 g, 65.7 mmol) and triphenylphosphine (17.2 g, 65.7 mmol) in tetrahydrofuran (100 mL) was added DIAD (13.3 g, 65.7 mmol) at 0°C and the reaction mixture was stirred at room temperature for 16h. The reaction mixture was poured into water (100 mL) and extracted with ethyl acetate (3 × 150 mL). The combined organic layers were separated, dried over anhydrous $Na_2SO_4$, filtered and concentrated *in vacuo.* The crude material was purified by combi-flash chromatography, by eluting with 30% ethyl acetate in heptane, to afford methyl (S)-4-(but-3-yn-2-yloxy)benzoate **(Intermediate 153,** 8.00 g, 60 %) as a yellow solid. $^1$H NMR (400 MHz, DMSO-*d6*) δ ppm 1.55 (d, J=6.80 Hz, 3H), 3.56 (s, 1H), 3.80 (s, 3H), 5.16-5.22 (m, 1H), 7.07 (d, J=8.60 Hz, 2H), 7.90 (d, J=8.60 Hz, 2H).

**Intermediates 154: methyl (R)-4-(but-3-yn-2-yloxy)benzoate**

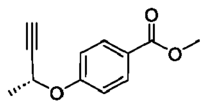

**Intermediate 154** was synthesised in an analogous manner to **Intermediate 153** using methyl 4-hydroxybenzoate (CAS No: 99-76-3) and (S)-but-3-yn-2-ol (CAS No: 2914-69-4) and exhibited: $^1$H NMR (400 MHz, DMSO-d6) δ ppm 1.57 (d, J=6.40 Hz, 3H), 3.58 (d, J=2.0 Hz, 1H), 3.81(s, 3H), 5.21-5.23 (m, 1H), 7.09 (d, J=8.60 Hz, 2H), 7.92 (d, J=8.60 Hz, 2H). Mass spec: m/z 205.0 [M+H]$^+$.

**Example 26 was synthesised following Scheme 28**

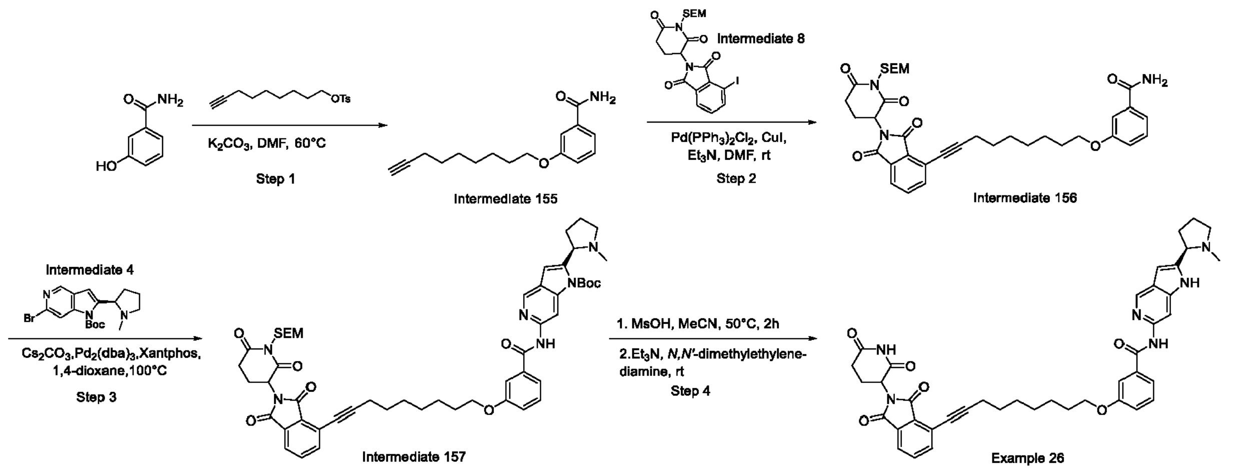


## Scheme 28

**Step 1**

**Intermediate 155: 3-(non-8-yn-1-yloxy) benzamide**

To solution of 3-hydroxybenzamide (CAS No: 618-49-5, 1.00 g, 7.29 mmol) in dimethylformamide (10 mL) was added potassium carbonate (1.53 g, 10.94 mmol) followed by non-8-yn-1-yl 4-methylbenzenesulfonate (CAS No: 87462-64-4, 2.15 g, 7.29 mmol) at room temperature and the reaction mixture was heated at 60°C for 16h. The reaction mixture was diluted with water (20 mL) and extracted with ethyl acetate (2 × 20 mL). The organic layer was separated, dried over anhydrous $Na_2SO_4$, filtered and concentrated *in vacuo.* The crude material was purified by combi-flash chromatography, by eluting with 60% ethyl acetate in heptane, to afford 3-(non-8-yn-1-yloxy) benzamide (**Intermediate 155,** 0.55 g, 29%) as an off white solid. $^1$H NMR (400 MHz, DMSO-$d_6$) δ ppm 1.31-1.49 (m, 8H), 1.70-1.72 (m, 2H), 2.13-2.15 (m, 2H), 2.72 (s, 1H), 3.99-4.02 (m, 2H), 7.03-7.06 (m, 1H), 7.28-7.37 (m, 2H), 7.40-7.47 (m, 2H), 7.92 (br s, 1H). Mass spec: m/z: 260.0 [M+H]$^+$.

**Step 2**

**Intermediate 156: 3-((9-(2-(2,6-dioxo-1-((2-(trimethylsilyl)ethoxy)methyl)piperidin-3-yl)-1,3-dioxoisoindolin-4-yl)non-8-yn-1-yl)oxy)benzamide**

To a solution of 3-(non-8-yn-1-yloxy) benzamide (**Intermediate 155,** 0.55 g, 3.86 mmol) in dimethylformamide (5 mL) was added 2-(2,6-dioxo-1-((2-(trimethylsilyl)ethoxy)methyl)piperidin-3-yl)-4-iodoisoindoline-1, 3-dione (**Intermediate 8,** 1.0 g, 1.9 mmol) followed by triethylamine (19.4 mL, 139 mmol). The reaction mixture was purged with argon gas for 20 min. then copper(I) iodide (0.074 g, 0.38 mmol) and $PdCl_2(PPh_3)_2$ (0.27 g, 0.38 mmol) were added, and the reaction mixture was stirred at room temperature for 2h. The reaction mixture was diluted with water (100 mL) and extracted with ethyl acetate (3 × 100 mL). The organic layer was separated, dried over anhydrous $Na_2SO_4$, filtered, and concentrated *in vacuo.* The crude material was purified by combi-flash chromatography, by eluting with 100% ethyl acetate in heptane, to afford 3-((9-(2-(2,6-dioxo-1-((2-(trimethylsilyl) ethoxy)methyl)piperidin-3-yl)-1,3-dioxoisoindolin-4-yl)non-8-yn-1-yl)oxy)benzamide (**Intermediate 156,** 1.3 g, 52%) as a yellow solid. $^1$H NMR (400 MHz, DMSO-$d_6$) δ ppm -0.02 (s, 9H), 0.82-0.86 (m, 2H), 1.40-1.50 (m, 6H), 1.59-1.61 (m, 2H), 1.72-1.74 (m, 2H), 2.07-2.09 (m, 1H), 2.52-2.57 (m, 2H), 2.76-2.81 (m, 1H), 3.29-3.32 (m, 2H), 3.48-3.58 (m, 2H), 3.98-4.01 (m, 2H), 5.06-5.09 (m, 2H), 5.23-5.29 (m, 1H), 7.02-7.07 (m, 1H), 7.27-7.42 (m, 3H), 7.44 (br s, 1H), 7.78-7.87 (m, 3H), 7.91 (br s, 1H). Mass spec: m/z: 644.0 $[M-H]^-$.

**Step 3**

**Intermediate 157: *tert*-butyl 6-(3-((9-(2-(2,6-dioxo-1-((2-(trimethylsilyl)ethoxy)methyl)piperidin-3-yl)-1,3-dioxoisoindolin-4-yl)non-8-yn-1-yl)oxy)benzamido)-2-((R)-1-methylpyrrolidin-2-yl)-1*H*-pyrrolo[3,2-c]pyridine-1-carboxylate**

To a solution of 3-((9-(2-(2,6-dioxo-1-((2-(trimethylsilyl)ethoxy)methyl)piperidin-3-yl)-1,3-dioxoisoindolin-4-yl) non-8-yn-1-yl)oxy)benzamide (**Intermediate 156**, 0.50 g, 0.77 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate (**Intermediate 4,** 0.29 g, 0.77 mmol) and cesium carbonate (0.51 g, 1.55 mmol. The reaction mixture was purged with argon gas for 10 min then $Pd_2(dba)_3$ (0.11 g, 0.12 mmol) and Xantphos (0.14 g, 0.23 mmol) were added at room temperature. The reaction mixture was further purged with argon gas for 15 min then heated at 100°C for 2h. The reaction mixture was then concentrated *in vacuo* and the residue was purified by combi-flash chromatography, by eluting with 80% ethyl acetate in heptane, to afford *tert*-butyl 6-(3-((9-(2-(2,6-dioxo-1-((2-(trimethylsilyl)ethoxy)methyl)piperidin-3-yl)-1,3-dioxoisoindolin-4-yl)non-8-yn-1-yl)oxy)benzamido)-2-((*R*)-1-methylpyrrolidin-2-yl)-1*H*-pyrrolo[3,2-c]pyridine-1-carboxylate (**Intermediate 157**, 0.60 g, 82%) as a yellow solid. $^1$H NMR (400 MHz, DMSO-$d_6$) δ ppm -0.06 (s, 9H), 0.77-0.83 (m, 2H), 1.38-1.49 (m, 6H), 1.58-1.63 (m, 4H), 1.67 (s, 9H), 1.70-1.77 (m, 4H), 2.02-2.10 (m, 1H), 2.29-2.31 (m, 1H), 2.33 (s, 3H), 2.35-2.39 (m, 1H), 2.51-2.56 (m, 2H), 2.74-2.78 (m, 1H), 2.95-3.04 (m, 1H), 3.09-3.12 (m, 1H), 3.45-3.52 (m, 2H), 3.87-3.92 (m, 1H), 4.01-4.05 (m, 2H), 5.05 (s, 2H), 5.22-5.26 (m, 1H), 6.73 (s, 1H), 7.08 (m, 1H), 7.34-7.38 (m, 1H), 7.57-7.62 (m, 2H), 7.78-7.85 (m, 3H), 8.57 (s, 1H), 8.89 (s, 1H), 10.65 (br s, 1H). Mass spec: m/z: 945.1 $[M+H]^+$

**Step 4**

**Example 26: 3-((9-(2-(2,6-dioxopiperidin-3-yl)-1,3-dioxoisoindolin-4-yl)non-8-yn-1-yl)oxy)-*N*-(2-((*R*)-1-methylpyrrolidin-2-yl)-1*H*-pyrrolo[3,2-c]pyridin-6-yl)benzamide**

To a solution of *tert*-butyl 6-(3-((9-(2-(2,6-dioxo-1-((2-(trimethylsilyl)ethoxy)methyl)piperidin-3-yl)-1,3-dioxoisoindolin-4-yl)non-8-yn-1-yl)oxy)benzamido)-2-((*R*)-1-methylpyrrolidin-2-yl)-1*H*-pyrrolo[3,2-c]pyridine-1-carboxylate (**Intermediate 157**, 0.30 g, 0.32 mmol) in acetonitrile (10 mL) was added methanesulfonic acid (0.21 mL, 3.17mmol) at room temperature and the reaction mixture was heated at 50°C for 2h. The reaction mixture was cooled to room temperature then *N*, *N*'-dimethylethylenediamine (0.19 mL, 1.58 mmol) followed by triethylamine (0.88 mL, 6.35 mmol) were added and the reactions stirred at room temperature for 3h. The reaction mixture was then concentrated *in vacuo* and water (20.0 mL) was added. The resulting precipitate was filtered, dried and the crude compound was purified by preparative HPLC (Method A) to afford 3-((9-(2-(2,6-dioxopiperidin-3-yl)-1,3-dioxoisoindolin-4-yl)non-8-yn-1-yl)oxy)-*N*-(2-((*R*)-1-methylpyrrolidin-2-yl)-1*H*-pyrrolo[3,2-c]pyridin-6-yl)benzamide (**Example 26**, 0.085 g, 37%) as an off white solid. $^1$H NMR (400 MHz, DMSO-$d_6$) δ ppm 1.39-1.56 (m, 6H), 1.58-1.67 (m, 2H), 1.73-1.94 (m, 5H), 2.01-2.09 (m, 1H), 2.12-2.15 (m, 1H), 2.16 (s, 3H), 2.22-2.30 (m, 1H), 2.52-2.58 (m, 4H), 2.60-2.66 (m, 1H), 2.82-2.92 (m, 1H), 3.11-3.14 (m, 1H), 4.03-4.07 (m, 2H), 5.10-5.15 (m, 1H), 6.39 (s, 1H), 7.08-7.11 (m, 1H), 7.36-7.38 (m, 1H), 7.57-7.61 (m, 2H), 7.79-7.86 (m, 3H), 8.18 (s, 1H), 8.50 (s, 1H), 10.45 (br s, 1H), 11.12 (br s, 1H), 11.37 (br s, 1H). Mass spec: m/z: 715.1 $[M+H]^+$.

**Example 27 was synthesised following Scheme 29**

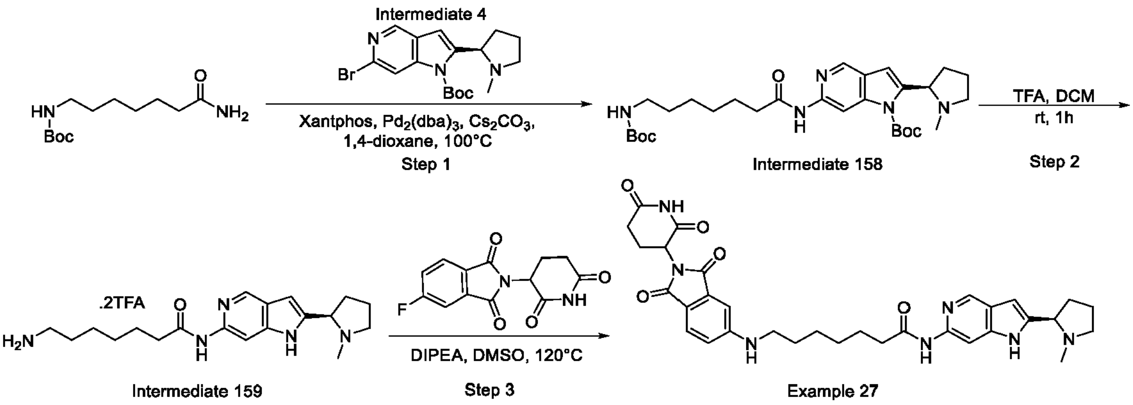


## Scheme 29

### Step 1

### Intermediate 158: *tert*-butyl 6-{7-[(tert-butoxycarbonyl)amino]heptanamido}-2-[(2R)-1-methylpyrrolidin-2-yl] pyrrolo[3,2-c]pyridine-1-carboxylate

A solution of *tert*-butyl N-(6-carbamoylhexyl)carbamate (CAS No: 51857-17-1, 0.3 g, 1.23 mmol), *tert*-butyl 6-bromo-2-[(2R)-1-methylpyrrolidin-2-yl]pyrrolo[3,2-c]pyridine-1-carboxylate (**Intermediate 4**, 0.42 g, 1.105 mmol), xantphos (0.28 g, 0.49 mmol), $Pd_2(dba)_3$ (0.22 g, 0.246 mmol) and $Cs_2CO_3$ (1.20 g, 3.684 mmol) in 1,4-dioxane (30 mL) was stirred for 4h at 100°C, under $N_2$. The reaction was quenched with water (50 mL) and the aqueous phase was extracted with EtOAc (3 × 50 mL). The combined organic layers were washed with brine, dried over anhydrous $Na_2SO_4$, filtered and concentrated under reduced pressure. The residue was then chromatographed on a silica gel column, eluting with $CH_2Cl_2$/MeOH (94/6), to afford *tert*-butyl 6-{7-[(tert-butoxycarbonyl)amino]heptanamido}-2-[(2R)-1-methylpyrrolidin-2-yl] pyrrolo[3,2-c]pyridine-1-carboxylate (**Intermediate 158**, 0.6 g, 100%) as a yellow solid. Mass spec: m/z: 544.7 $[M+H]^+$.

### Step 2

### Intermediate 159: 7-amino-N-{2-[(2R)-1-methylpyrrolidin-2-yl]-1H-pyrrolo[3,2-c]pyridin-6-yl}heptanamide ditrifluoroacetate

A solution of *tert*-butyl 6-{7-[(tert-butoxycarbonyl)amino]heptanamido}-2-[(2R)-1-methylpyrrolidin-2-yl] pyrrolo [3,2-c]pyridine-1-carboxylate (**Intermediate 158,** 600 mg, 1.10 mmol) and TFA (4 mL) in DCM (8 mL) was stirred at room temperature for 2h. The volatiles were then removed under reduced pressure to afford 7-amino -N-{2-[(2R)-1-methylpyrrolidin-2-yl]-1H-pyrrolo[3,2-c]pyridin-6-yl}heptanamide ditrifluoroacetate (**Intermediate 159,** 380 mg) as a yellow oil, which was used in the next step without further purification. Mass spec: m/z: 344.5 $[M+H]^+$.

### Step 3

### Example 27: 7-{[2-(2,6-dioxopiperidin-3-yl)-1,3-dioxoisoindol-5-yl]amino}-N-{2-[(2R)-1-methylpyrrolidin-2-yl]-1H-pyrrolo[3,2-c] pyridin-6-yl}heptanamide

A solution of 7-amino-N- { 2-[(2R)-1-methylpyrrolidin-2-yl]-1H-pyrrolo[3,2-c]pyridin-6-yl}heptanamide ditrifluoracetate (**Intermediate 159**, 200 mg, 0.58 mmol), 2-(2,6-dioxopiperidin-3-yl)-5-fluoroisoindole-1,3-dione (CAS No: 835616-61-0, 402 mg, 1.46 mmol) and DIPEA (753 mg, 5.820 mmol) in DMSO (5 mL) was stirred overnight at 120°C, under $N_2$. The resulting mixture was then purified directly by preparative HPLC (Column: YMC-Actus Triart C18ExRs, 30*150 mm, 5 μm; Mobile Phase A: Water (10 mmol/L $NH_4HCO_3$), Mobile Phase B: MeCN; Flow rate: 60 mL/min; Gradient: 25% B to 55% B in 10 min) to afford 7-{[2-(2,6-dioxopiperidin-3-yl)-1,3-dioxoisoindol-5-yl]amino}-N-{2-[(2R)-1-methylpyrrolidin-2-yl]-1H-pyrrolo[3,2-c]pyridin-6-yl}heptanamide (**Example 27**, 88.3 mg, 34%) as a yellow solid. $^1H$ NMR (400 MHz, DMSO-d6) δ ppm 11.25 (s, 1H), 11.05 (s, 1H),10.11 (s, 1H), 8.40 (s, 1H), 8.08 (s, 1H), 7.55 (d, *J* = 8.4 Hz, 1H), 7.10 (t, *J* = 5.4 Hz 1H), 6.94 (s, 1H), 6.82 - 6.85 (m, 1H), 6.33 (s, 1H), 5.00 - 5.05 (m, 1H), 3.26 - 3.30 (m, 1H), 3.13 - 3.18 (m, 3H), 2.84 - 2.90 (m, 1H), 2.55 - 2.59 (m, 2H), 2.37 (t, *J* = 7.4 Hz, 2H), 2.24 - 2.26 (m, 1H) 2.12 - 2.14 (m, 4H), 1.97 - 2.01 (m,

1H), 1.85 - 1.87 (m, 3H), 1.57 - 1.63 (m, 4H), 1.35 - 1.41 (m, 4H). Mass spec: m/z: 600.4 $[M+H]^+$.

**Example 28 was synthesised following Scheme 30**

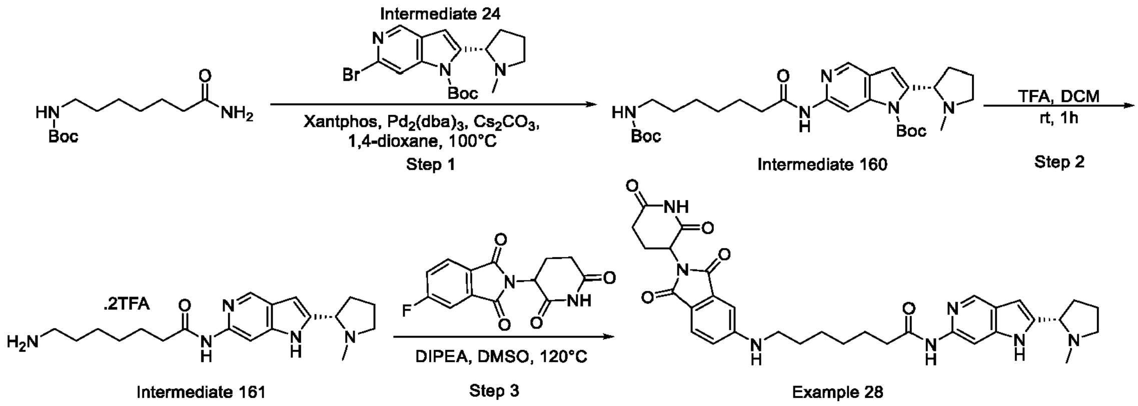


## Scheme 30

**Step 1**

**Intermediate 160: *tert*-butyl 6-{7-[(tert-butoxycarbonyl)amino]heptanamido}-2-[(2S)-1-methylpyrrolidin-2-yl]pyrrolo[3,2-c]pyridine-1-carboxylate**

**Intermediate 160** was synthesised in an analogous manner to **Intermediate 158**, following **Step 1** of **Scheme 29**, using *tert*-butyl N-(6-carbamoylhexyl)carbamate (CAS No: 51857-17-1) as the amide and *tert*-butyl 6-bromo-2-[(2S)-1-methylpyrrolidin-2-yl]pyrrolo[3,2-c]pyridine-1-carboxylate (**Intermediate 24**) as the aryl bromide, to afford *tert*-butyl 6-{7-[(tert-butoxycarbonyl)amino]heptanamido}-2-[(2S)-1-methylpyrrolidin-2-yl]pyrrolo[3,2-c]pyridine-1-carboxylate (**Intermediate 160**). Mass spec: m/z: 544.4 $[M+H]^+$.

**Step 2**

**Intermediate 161: 7-amino-N-{2-[(2S)-1-methylpyrrolidin-2-yl]-1H-pyrrolo[3,2-c]pyridin-6-yl}heptanamide ditrifluoracetate**

**Intermediate 161** was synthesised in an analogous manner to **Intermediate 159,** following **Step 2** of **Scheme 29,** using *tert*-butyl 6-{7-[(tert-butoxycarbonyl)amino]heptanamido}-2-[(2S)-1-methylpyrrolidin-2-yl]pyrrolo[3,2-c]pyridine-1-carboxylate (**Intermediate 160**) as the amide, to afford 7-amino-N-{2-[(2S)-1-methylpyrrolidin-2-yl]-1H-pyrrolo[3,2-c]pyridin-6-yl}heptanamide ditrifluoracetate (**Intermediate 161**). Mass spec: m/z: 344.2 $[M+H]^+$.

**Step 3**

**Example 28: 7-{[2-(2,6-dioxopiperidin-3-yl)-1,3-dioxoisoindol-5-yl]amino}-N-{2-[(2S)-1-methylpyrrolidin-2-yl]-1H-pyrrolo[3,2-c]pyridin-6-yl}heptanamide**

**Example 28** was synthesised in an analogous manner to **Example 27,** following **Step 3** of **Scheme 29,** using 7-amino-N-{2-[(2S)-1-methylpyrrolidin-2-yl]-1H-pyrrolo[3,2-c]pyridin-6-yl}heptanamide ditrifluoracetate (**Intermediate 161**) and 2-(2,6-dioxopiperidin-3-yl)-5-fluoroisoindole-1,3-dione (CAS No: 835616-61-0) as the aryl fluoride, to afford 7-{[2-(2,6-dioxopiperidin-3-yl)-1,3-dioxoisoindol-5-yl]amino}-N-{2-[(2S)-1-methylpyrrolidin-2-yl]-1H-pyrrolo[3,2-c]pyridin-6-yl}heptanamide (**Example 28**). $^1$H NMR (400 MHz, DMSO-d6) δ ppm 11.25 (s, 1H), 11.05 (s, 1H), 10.11 (s, 1H), 8.40

(s, 1H), 8.08 (s, 1H), 7.55 (d, *J* = 8.4 Hz, 1H), 7.10 - 7.12 (m, 1H), 7.09 (s, 1H), 6.84 (d, *J* = 8.4 Hz, 1H), 6.33 (s, 1H), 5.00 - 5.05(m, 1H), 3.28 - 3.33 (m, 2H), 3.13 - 3.16 (m, 3H), 2.91 - 2.92 (m, 1H), 2.86 - 2.88 (m, 1H), 2.35 - 2.41 (m, 2H), 2.35 - 2.39 (m, 1H), 2.14 - 2.20 (m, 4H), 1.90 - 1.99 (m, 1H), 1.84 - 1.89 (m, 3H), 1.65 - 1.71 (m, 4H), 1.45 - 1.55 (m, 4H). Mass spec: m/z: 600.4 $[M+H]^+$.

**Example 29: 7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c] pyridin-6-yl}heptanamide**

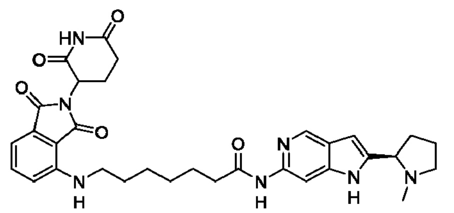

To a solution of 7-amino-N-{2-[(2R)-1-methylpyrrolidin-2-yl]-1H-pyrrolo[3,2-c]pyridin-6-yl}heptanamide ditrifluoroacetate (**Intermediate 159**, 190 mg, 0.498 mmol) in DMSO (4 mL) was added 2-(2,6-dioxopiperidin-3-yl)-4-fluoroisoindole-1,3-dione (CAS No: 835616-60-9, 275 mg, 0.996 mmol) and DIPEA (643 mg, 4.98 mmol) and the reaction mixture was stirred at 130 °C for 2h. Water (2 mL) was added and the resulting mixture was chromatographed on a C18 silica column, eluting with MeCN/$H_2O$ (+1-2 drops of formic acid) (24/76), to afford crude product. The crude product was then further purified by preparative HPLC (Column: XselectCSH Prep OBD C18 Column, 30*150 mm, 5μm; Mobile Phase A: Water (+0.1% formic acid), Mobile Phase B: MeCN; Flow rate: 60 mL/min mL/min; Gradient: 2% B to 30% B in 8 min), to afford 7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c] pyridin-6-yl}heptanamide (**Example 29**, 108 mg, 36%) as a yellow solid. $^1$H NMR (400 MHz, DMSO-d6) δ ppm 11.26 (s, 1H), 11.10 (s, 1H), 10.10 (s, 1H), 8.40 (s, 1H), 8.08 (s, 1H), 7.56 (t, *J* = 8.0 Hz, 1H), 7.09 (d, *J* = 8.8 Hz, 1H), 7.01 (d, *J* = 6.8 Hz, 1H), 6.54 (t, *J* = 6.0 Hz, 1H), 6.34 (s, 1H), 5.02 - 5.06 (m, 1H), 3.26 - 3.32 (m, 3H), 3.13 - 3.15 (m, 1H), 2.84 - 2.87 (m, 1H), 2.54 - 2.60 (m, 2H), 2.36 (t, *J* = 7.2 Hz, 2H), 2.24 - 2.26 (m, 1H). 2.12 - 2.14 (m, 4H), 2.00 - 2.04 (m, 1H), 1.80 - 1.89 (m, 3H), 1.57 - 1.62 (m, 4H), 1.36 (m, 4H). Mass spec: m/z: 600.30 $[M+H]^+$.

**Example 30: 9-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}nonanamide**

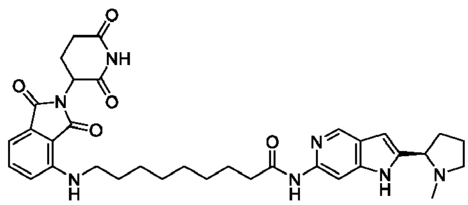

9-{[2-(2,6-dioxopiperidin-3-yl)-1,3-dioxoisoindol-4-yl]amino}-N-{2-[(2R)-1-methylpyrrolidin-2-yl]-1H-pyrrolo [3,2-c]pyridin-6-yl}nonanamide (**Example 30**) was prepared in an analogous manner to **Example 29,** using 9-amino-N-{2-[(2R)-1-methylpyrrolidin-2-yl]-1H-pyrrolo[3,2-c]pyridin-6-yl}nonanamide ditrifluoroacetate (**Intermediate 345**) as the amine. **Example 30** exhibited: $^1$H NMR (400 MHz, MeOH-d4) δ ppm as 11.26 (s, 1H), 11.10 (s, 1H), 10.10 (s, 1H), 8.40 (s, 1H), 8.08 (s, 1H), 7.57 (t, *J* = 7.6 Hz, 1H), 7.09 (d, *J* = 8.8 Hz, 1H), 7.02 (d, *J* = 6.8 Hz, 1H), 6.52 - 6.53 (m, 1H), 6.34 (s, 1H), 5.03 - 5.08 (m, 1H), 3.20 - 3.30 (m, 3H), 3.11 - 3.15 (m, 1H), 2.84 - 2.93 (m, 1H), 2.61 - 2.68 (m, 2H), 2.33 - 2.37 (m, 2H), 2.24 - 2.26 (m, 1H), 2.10 - 2.15 (m, 4H), 2.02 - 2.05 (m, 1H), 1.75 - 1.98 (m, 3H), 1.55 - 1.65 (m, 4H), 1.20 - 1.40 (m, 8H). Mass spec: m/z: 628.4 $[M+H]^+$.

**Example 31 was synthesised following Scheme 31**

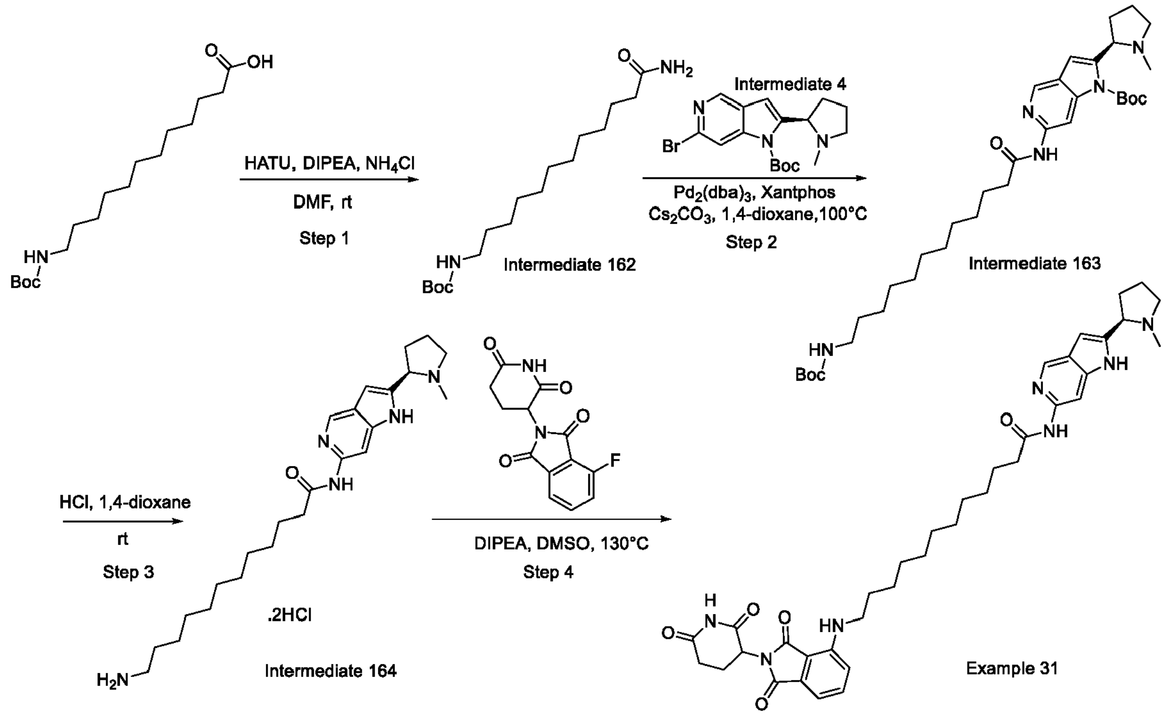


## Scheme 31

### Step 1

### Intermediate 162: *tert*-butyl N-(11-carbamoylundecyl)carbamate

To a solution of 12-[(*tert*-butoxycarbonyl)amino]dodecanoic acid (CAS No: 18934-81-1, 500 mg, 1.57 mmol), HATU (895 mg, 2.35 mmol), DIPEA (608 mg, 4.71 mmol) in anhydrous DMF (10 mL) was added $NH_4Cl$ (420 mg, 7.84 mmol) and the reaction was stirred at room temperature for 2h. The resulting mixture was chromatographed on a C18 silica column, eluting with $MeCN/H_2O$ (with 1-2 drops formic acid) (45/55), to afford *tert*-butyl N-(11-carbamoylundecyl) carbamate (**Intermediate 162,** 460 mg, 84%) as a white solid. Mass spec: m/z 315.45 $[M+H]^+$.

### Step 2

### Intermediate 163: *tert*-butyl 6-{12-[(tert-butoxycarbonyl)amino]dodecanamido}-2-[(2R)-1-methylpyrrolidin-2-yl] pyrrolo[3,2-c]pyridine-1-carboxylate

To a solution of *tert*-butyl N-(11-carbamoylundecyl)carbamate (**Intermediate 162,** 200 mg, 0.572 mmol) and *tert*-butyl 6-bromo-2-[(2R)-1-methylpyrrolidin-2-yl]pyrrolo[3,2-c]pyridine-1-carboxylate **(Intermediate 4,** 218 mg, 0.572 mmol) in 1,4-dioxane (4 mL) was added $Cs_2CO_3$ (373 mg, 1.144 mmol), XantPhos (66.24 mg, 0.114 mmol) and $Pd_2(dba)_3$ (52.42 mg, 0.057 mmol) under nitrogen and the mixture was stirred at 100°C for 3h. Water (5 mL) was added and the resulting solution was chromatographed on a C18 slicia column, eluting with $MeCN/H_2O$ (with 1-2 drops formic acid) (52/48) to afford *tert*-butyl 6-{12-[(*tert*-butoxycarbonyl)amino]dodecanamido}-2-[(2R)-1-methylpyrrolidin-2-yl]pyrrolo[3,2-c]pyridine-1-carboxylate (**Intermediate 163,** 280 mg, 76%) as a yellow solid. Mass spec: m/z: 614.45 $[M+H]^+$.

### Step 3

### Intermediate 164: 12-amino-N-{2-[(2R)-1-methylpyrrolidin-2-yl]-1H-pyrrolo[3,2-c]pyridin-6-yl}dodecanamide dihydrochloride

To a solution of *tert*-butyl 6-{ 12-[(tert-butoxycarbonyl)amino]dodecanamido}-2-[(2R)-1-methylpyrrolidin-2-yl] pyrrolo[3,2-c]pyridine-1-carboxylate (**Intermediate 163,** 280 mg, 0.433 mmol) in 1,4-dioxane (10 mL) was added HCl

(12M, 4 mL) dropwise at room temperature and the reaction mixture was stirred at room temperature for 3h. The volatiles were then removed under reduced pressure to afford 12-amino-N-{2-[(2R)-1-methylpyrrolidin-2-yl]-1H-pyrrolo[3,2-c]pyridin-6-yl}dodecanamide dihydrochloride **(Intermediate 164,** 180 mg) as a yellow solid, which was used in the next step without further purification. Mass spec: m/z: 414.20 $[M+H]^+$.

**Step 4**

**Example 31: 12-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}dodecanamide**

To a solution of 12-amino-N-{2-[(2R)-1-methylpyrrolidin-2-yl]-1H-pyrrolo[3,2-c]pyridin-6-yl}dodecanamide dihydrochloride (**Intermediate 164**, 188 mg, 0.364 mmol) in DMSO (4 mL) was added 2-(2,6-dioxopiperidin-3-yl)-4-fluoroisoindole-1,3-dione (CAS No: 835616-60-9, 201 mg, 0.728 mmol) and DIPEA (470 mg, 3.640 mmol) and the reaction was stirred at 130°C for 2h. Water (2 mL) was added the resulting mixture was chromatographed on a C18 silica column, eluting with MeCN/$H_2O$ (with 1-2 drops formic acid) (45/55), to give crude product. The crude product was further purified by preparative HPLC (Column: Xselect CSH Prep C18 Column, 30*150 mm, 5μm; Mobile Phase A: Water (+0.1% formic acid), Mobile Phase B: MeCN; Flow rate: 60 mL/min; Gradient: 12% B to 42 % B in 10 min) to afford 12-{[2-(2,6-dioxopiperidin-3-yl)-1,3-dioxoisoindol-4-yl]amino}-N- f 2-[(2R)-1-methylpyrrolidin-2-yl]-1H-pyrrolo[3,2-c]pyridin-6-yl}dodecanamide (**Example 31**, 72.6 mg, 29%) as a yellow solid. $^1H$ NMR (400 MHz, DMSO-d6) δ ppm 11.25 (s, 1H), 11.09 (s, 1H), 10.09 (s, 1H), 8.40 (s, 1H), 8.07 (s, 1H), 7.57 (t, *J* = 8.0 Hz, 1H), 7.08 (d, *J* = 8.8 Hz, 1H), 7.02 (d, *J* = 6.8 Hz, 1H), 6.52 (t, *J* = 6.0 Hz, 1H), 6.33 (s, 1H), 5.02 - 5.07 (m, 1H), 3.26 - 3.32 (m, 3H), 3.12 (t, *J* = 7.6 Hz, 1H), 2.85 - 2.88 (m, 1H), 2.54 - 2.60 (m, 2H), 2.34 (t, *J* = 7.2 Hz, 2H), 2.22 - 2.27 (m, 1H). 2.11 - 2.14 (m, 4H), 2.01 - 2.04 (m, 1H), 1.76 - 1.90 (m, 3H), 1.54 - 1.57 (m, 4H), 1.26 (br, 14H). Mass spec: m/z: 670.40 $[M+H]^+$.

**Example 32 was synthesised following Scheme 32**

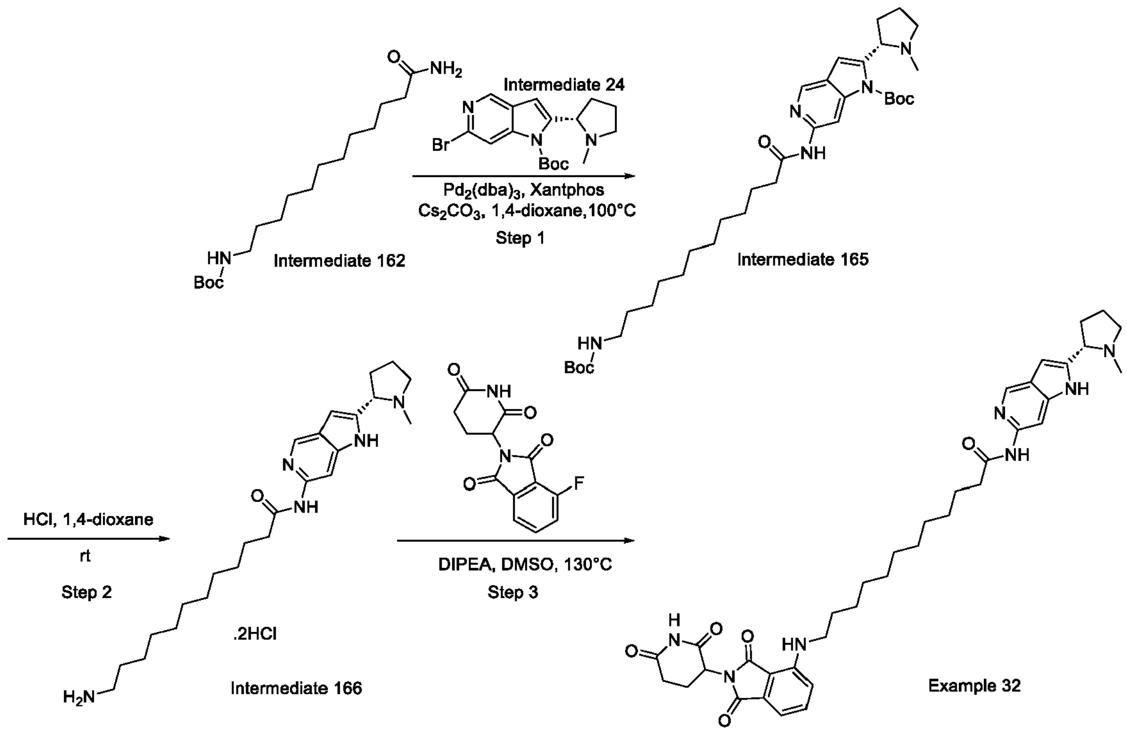


**Scheme 32**

**Step 1**

**Intermediate 165: *tert*-butyl N-[11-({2-[(2S)-1-methylpyrrolidin-2-yl]-1H-pyrrolo[3,2-c]pyridin-6-yl}carbamoyl) undecyl]carbamate**

**Intermediate 165** was synthesised in an analogous manner to **Intermediate 163**, by following **Step 2** shown in **Scheme 31,** using *tert*-butyl N-(11-carbamoylundecyl)carbamate (**Intermediate 162**) as the amide and *tert*-butyl 6-bromo-2-[(2S)-1-methylpyrrolidin-2-yl]pyrrolo[3,2-c]pyridine-1-carboxylate (**Intermediate 24)** as the aryl bromide, to afford *tert*-butyl N-[11-({2-[(2S)-1-methylpyrrolidin-2-yl]-1H-pyrrolo[3,2-c]pyridin-6-yl}carbamoyl)undecyl]carbamate (**Intermediate 165**). Mass spec: m/z: 514.40 $[M+H]^+$.

**Step 2**

**Intermediate 166: 12-amino-N-{2-[(2S)-1-methylpyrrolidin-2-yl]-1H-pyrrolo[3,2-c]pyridin-6-yl}dodecanamide dihydrochloride**

**Intermediate 166** was synthesised in an analogous manner to **Intermediate 164,** by following **Step 3** shown in **Scheme 31,** using *tert*-butyl N-[11-({2-[(2S)-1-methylpyrrolidin-2-yl]-1H-pyrrolo[3,2-c]pyridin-6-yl}carbamoyl)undecyl] carbamate (**Intermediate 165**) as the amide, to afford 12-amino-N-{2-[(2S)-1-methylpyrrolidin-2-yl]-1H-pyrrolo[3,2-c] pyridin-6-yl}dodecanamide dihydrochloride (**Intermediate 166**). Mass spec: m/z: 414.50 $[M+H]^+$.

**Step 3**

**Example 32: 12-{[2-(2,6-dioxopiperidin-3-yl)-1,3-dioxoisoindol-4-yl]amino}-N-{2-[(2S)-1-methylpyrrolidin-2-yl]-1H-pyrrolo[3,2-c]pyridin-6-yl}dodecanamide**

**Example 32** was synthesised from **Intermediate 166** in an analogous manner to **Example 31,** by following **Step 4** shown in **Scheme 31,** using the following preparative HPLC conditions for the purification (Column: XBridge Prep OBD C18 Column, 30*150 mm, 5μm; Mobile Phase A: Water (10mmol/L $NH_4HCO_3$), Mobile Phase B: MeCN; Flow rate: 60 mL/min; Gradient: 50% B to 80% B in 7 min), to afford 12-{[2-(2,6-dioxopiperidin-3-yl)-1,3-dioxoisoindol-4-yl]amino}-N-{2-[(2S)-1-methylpyrrolidin-2-yl]-1H-pyrrolo[3,2-c]pyridin-6-yl}dodecanamide (**Example 32**). $^1H$ NMR (400 MHz, MeOH-d4) δ ppm 8.45 (s, 1H), 8.05 (s, 1H), 7.55 (t, *J* = 8.0 Hz, 1H), 7.02 - 7.04 (m, 2H), 6.47 (s, 1H), 5.05 - 5.09 (m, 1H), 3.39 - 3.43 (m, 1H), 3.29 - 3.32 (m, 1H), 3.20 - 3.24 (m, 1H), 2.82 - 2.87 (m, 1H), 2.71 - 2.77 (m, 2H), 2.38 - 2.46 (m, 3H), 2.24 - 2.36 (m, 4H), 2.10 - 2.13 (m, 1H), 1.99 - 2.01 (m, 2H), 1.90 -1.92 (m, 1H), 1.62 -1.74 (m, 4H), 1.33 - 1.38 (m, 15H). Mass spec: m/z: 670.60 $[M+H]^+$.

**Example 33: N-{3-chloro-2-[(2R)-1-methylpyrrolidin-2-yl]-1H-pyrrolo[3,2-c]pyridin-6-yl}-7-{[2-(2,6-dioxopiperidin-3-yl)-1,3-dioxoisoindol-4-yl]amino}heptanamide**

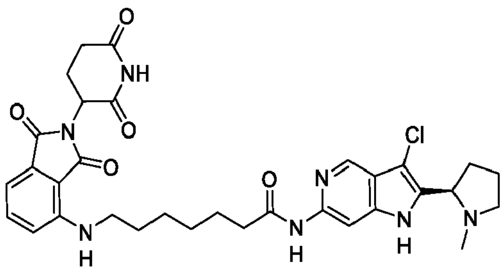

To a solution of 7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}heptanamide (**Example 29,** 50 mg, 0.083 mmol) in DMF (2.5 mL) was added NCS (14.5 mg, 0.108 mmol) and the reaction was stirred overnight at rt. The reaction was quenched by the addition of sat. aq. $NH_4Cl$ (2 mL) and filtered. The resulting crude was then purified by preparative HPLC (Column: XBridge Prep OBD C18 Column, 30*150 mm, 5μm; Mobile Phase A: Water (10 mmol/L $NH_4HCO_3$), Mobile Phase B: MeCN; Flow rate: 60 mL/min mL/min; Gradient: 40% B to 70% B in 7 min) to afford N-{3-chloro-2-[(2R)-1-methylpyrrolidin-2-yl]-1H-pyrrolo[3,2-c] pyridin-6-yl}-7-{[2-(2,6-dioxopiperidin-3-yl)-1,3-dioxoisoindol-4-yl]amino}heptanamide (**Example 33**, 15.1 mg, 28%) as a yellow solid. $^1H$ NMR (400 MHz, MeOH-d4) δ ppm 11.58 (s, 1H), 11.10 (s, 1H), 10.27 (s, 1H), 8.41 (s, 1H), 8.14 (s, 1H), 7.55 - 7.59 (m, 1H), 7.09 (d, *J* = 8.8 Hz, 1H), 7.02 (d, *J* = 6.4 Hz, 1H), 6.54 (s, 1H), 5.04 - 5.06 (m, 1H), 3.50 - 3.60 (m, 2H), 3.10 -

3.16 (m, 1H), 2.85 - 2.88 (m, 1H), 2.57 - 2.61 (m, 3H), 2.36 - 2.40 (m, 2H), 2.28 - 2.30 (m, 1H), 2.14 - 2.15 (m, 4H), 1.83 - 2.05 (m, 4H), 1.59 - 1.61 (m, 4H), 1.35 - 1.39 (m, 4H). Mass spec: m/z: 634.5 $[M+H]^+$.

**Example 34 was synthesised following Scheme 33**

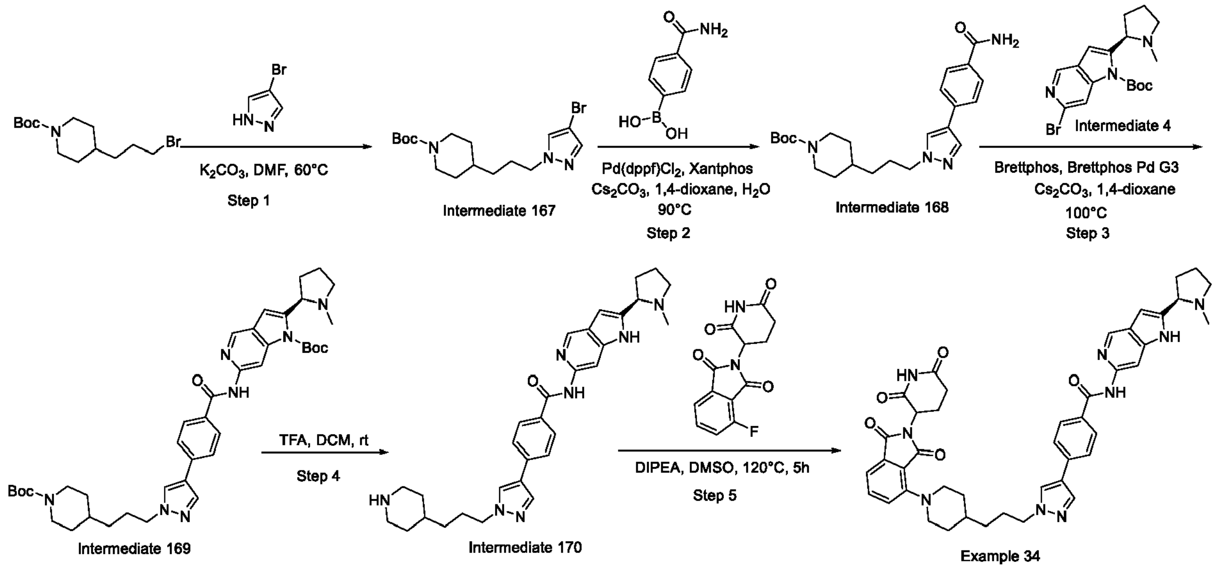


## Scheme 33

**Step 1**

**Intermediate 167: *tert*-butyl 4-[3-(4-bromopyrazol-1-yl)propyl]piperidine-1-carboxylate**

To a solution of *tert*-butyl 4-(3-bromopropyl)piperidine-1 -carboxylate (CAS No: 164149-27-3, 4.9 g, 16.0 mmol) and 4-bromopyrazole (CAS No: 2075-45-8, 2.35 g, 16.0 mmol) in DMF (100 mL) was added $K_2CO_3$ (6.63 g, 48.0 mmol) and the reaction was stirred at 60 °C overnight. Water (100mL) was added and the reaction mixture was extracted with EtOAc (3 × 200 mL). The combined organic layers was washed with brine (4 × 150 mL), dried over anhydrous sodium sulfate and concentrated under redcued pressure to afford *tert*-butyl 4-[3-(4-bromopyrazol-1-yl)propyl]piperidine-1-carboxylate (**Intermediate 167**, 6.00 g, 91%) as a colorless oil, which was used in the next step with out further purification. Mass spec: m/z: 372.1 $[M+H]^+$.

**Step 2**

**Intermediate 168: tert-butyl 4-{3-[4-(4-carbamoylphenyl)pyrazol-1-yl]propyl}piperidine-1-carboxylate**

To a solution of *tert*-butyl 4-[3-(4-bromopyrazol-1-yl)propyl]piperidine-1-carboxylate **(Intermediate 167**, 3.00 g, 7.25 mmol) and 4-carbamoylphenylboronic acid (CAS No: 123088-59-5, 1.33 g, 8.06 mmol) in dioxane (168 mL) and $H_2O$ (42 mL) was added Pd(dppf)$Cl_2$ (1.77 g, 2.42 mmol) and $Cs_2CO_3$ (3.94 g, 12.1mmol) and the reaction was stirred at 90 °C for 4 h under $N_2$. Water (200 mL) was then added and the mixture was extracted with EtOAc (3 × 150mL). The combined organic phases were washed with brine (200mL), dried over anhydrous sodium sulfate and concentrated under reduced pressure. The resulting residue was purified on a silica gel column, eluting with DCM/MeOH (97/3) to afford *tert*-butyl 4-{3-[4-(4-carbamoylphenyl)pyrazol-1-yl]propyl}piperidine-1-carboxylate (**Intermediate 168**, 900 mg, 29%) as a brown solid. Mass spec: m/z: 313.15 $[M-Boc+H]^+$.

**Step 3**

**Intermediate 169: *tert*-butyl 4-{3-[4-(4-{[1-(tert-butoxycarbonyl)-2-[(2R)-1-methylpyrrolidin-2-yl]pyrrolo[3,2-c]pyridin-6-yl]carbamoyl}phenyl)pyrazol-1-yl]propyl}piperidine-1-carboxylate**

To a solution of *tert*-butyl 4-{3-[4-(4-carbamoylphenyl)pyrazol-1-yl]propyl}piperidine-1-carboxylate (**Intermediate 168**, 250 mg, 0.606 mmol) and *tert*-butyl 6-bromo-2-[(2R)-1-methylpyrrolidin-2-yl]pyrrolo[3,2-c]pyridine-1-carboxylate (**Intermediate 4**, 276 mg, 0.727 mmol) in dioxane (10 mL) was added Brettphos (195 mg, 0.364 mmol), Brettphos Pd G3 (165 mg, 0.182 mmol), $Cs_2CO_3$ (494 mg, 1.52 mmol) and the reaction stirred overnight at 100°C. The reaction was cooled to room temperature and the residue chromatographed on a silica gel column, eluting with DCM/MeOH (95/5) to afford *tert*-butyl 4-{ 3-[4-(4-{ [1-(*tert*-butoxycarbonyl)-2-[(2R)-1-methylpyrrolidin-2-yl]pyrrolo[3,2-c]pyridin-6-yl]carbamoyl}phenyl)pyrazol-1-yl]propyl}piperidine-1-carboxylate (**Intermediate 169**, 250 mg, 54%) as a brown solid. Mass spec: m/z: 712.6 $[M+H]^+$.

**Step 4**

**Intermediate 170: N-{2-[(2R)-1-methylpyrrolidin-2-yl]-1H-pyrrolo[3,2-c]pyridin-6-yl}-4-{1-[3-(piperidin-4-yl)propyl]pyrazol-4-yl}benzamide ditrifluoroacetate**

To a solution of tert-butyl 4-{3-[4-(4-{[1-(*tert*-butoxycarbonyl)-2-[(2R)-1-methylpyrrolidin-2-yl]pyrrolo[3,2-c]pyridin-6-yl]carbamoyl}phenyl)pyrazol-1-yl]propyl}(piperidine-1-carboxylate (**Intermediate 169**, 250 mg, 0.316 mmol) in DCM (5 mL) was added TFA (2.5 mL). The reaction was stirred at rt for 2h and the volatiles were removed under reduced pressure to afford N-{2-[(2R)-1-methylpyrrolidin-2-yl]-1H-pyrrolo[3,2-c]pyridin-6-yl}-4-{1-[3-(piperidin-4-yl)propyl]pyrazol-4-yl}benzamide ditrifluoroacetate (**Intermediate 170**, 179 mg) as a brown oil, which was used in the next step without further purification. Mass spec: m/z: 512.4 $[M+H]^+$.

**Step 5**

**Example 34: 4-[1-(3-{1-[2-(2,6-dioxopiperidin-3-yl)-1,3-dioxoisoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide**

To a solution of N-{2-[(2R)-1-methylpyrrolidin-2-yl]-1H-pyrrolo[3,2-c]pyridin-6-yl}-4-{1-[3-(piperidin-4-yl)propyl]pyrazol-4-yl}benzamide ditrifluoroacetate (**Intermediate 170, 40.0** mg, 0.07 mmol) and 2-(2,6-dioxopiperidin-3-yl)-4-fluoroisoindole-1,3-dione (CAS No: 835616-60-9, 32.4 mg, 0.117 mmol) in DMSO (2 mL) was added DIPEA (101 mg, 0.780 mmol) and the reaction was stirred at 120 °C for 5h. The reaction mixture was then purfiied by preparative HPLC (Column: XBridge Prep OBD C18 Column, 30 *150 mm, 5μm; Mobile Phase A: Water (10 mmol/L $NH_4HCO_3$), Mobile Phase B: MeCN; Flow rate: 60 mL/min; Gradient: 38% B to 68% B in 13 min) to afford 4-[1-(3-{1-[2-(2,6-dioxopiperidin-3-yl)-1,3-dioxoisoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide (**Example 34**, 20.0 mg, 35%) as a yellow solid. $^1H$ NMR (400 MHz, MeOH-d4) δ ppm 11.38 (s, 1H), 11.08 (s, 1H), 10.40 (s, 1H), 8.51 (s, 1H), 8.36 (s, 1H), 8.20 (s, 1H), 8.06 (d, *J* = 8.4 Hz, 2H), 8.01 (s, 1H), 7.65 - 7.72 (m, 3H), 7.30 - 7.34 (m, 2H), 6.40 (s, 1H), 5.06 - 5.11 (m, 1H), 4.13 - 4.17 (m, 2H), 3.67 - 3.70 (m, 2H), 3.10 - 3.15 (m, 1H), 2.82 - 2.88 (m, 3H), 2.53 - 2.61 (m, 2H), 2.40 - 2.50 (m, 1H), 2.26 - 2.28 (m, 1H), 2.14 - 2.17 (m, 4H), 1.76 - 2.05 (m, 8H), 1.20 - 1.50 (m, 5H). Mass spec: m/z: 768.65 $[M+H]^+$.

**Example 35: 4-[1-(3-{1-[2-(2,6-dioxopiperidin-3-yl)-1-oxo-3H-isoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide**

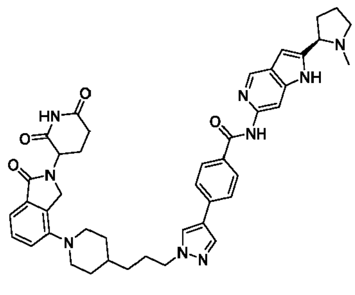

To a solution of N-{2-[(2R)-1-methylpyrrolidin-2-yl]-1H-pyrrolo[3,2-c]pyridin-6-yl}-4-{1-[3-(piperidin-4-yl)propyl]pyrazol-4-yl}benzamide (**Intermediate 170**, 100 mg, 0.195 mmol) and 3-(4-bromo-1-oxo-3H-isoindol-2-yl)piperidine-2,6-dione (CAS No: 2093387-36-9, 75.8 mg, 0.234 mmol) in 1,4-dioxane (5 mL) was added $Cs_2CO_3$ (191 mg, 0.585 mmol) and Pd-PEPPSI-IHept-Cl (CAS No:1814936-54-3, 24.6 mg, 0.0290 mmol) and the reaction was stirred overnight at 100 °C, under $N_2$. The reaction mixture was then purified by preparative HPLC (Column: YMC-Actus Triart C18ExRs, 30*150 mm, 5μm; Mobile Phase A: Water (with 10mmol/L $NH_4HCO_3$), Mobile Phase B: MeCN; Flow rate: 60 mL/min; Gradient: 33% B to 63% B in 10 min) to afford 4-[1-(3-f 1-[2-(2,6-dioxopiperidin-3-yl)-1-oxo-3H-isoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide (**Example 35**, 11.7 mg, 7.7%) as a white solid. $^1H$ NMR (400 MHz, DMSO-d6) δ ppm as 11.38 (s, 1H), 11.00 (s, 1H), 10.41 (s, 1H), 8.51 (s, 1H), 8.36 (s, 1H), 8.21 (s, 1H), 8.06 (d, *J* = 8.0 Hz, 2H), 8.02 (s, 1H), 7.72 (d, *J* = 8.4 Hz, 2H), 7.40 - 7.42 (m, 1H), 7.29 (d, *J* = 7.6 Hz, 1H), 7.16 (d, *J* = 8.0 Hz, 1H), 6.40 (s, 1H), 5.05 - 5.15 (m, 1H), 4.40 - 4.44 (m, 1H), 4.26 - 4.30 (m, 1H), 4.14 - 4.17 (m, 2H), 3.35 - 3.40 (m, 2H), 3.10 - 3.20 (m, 1H), 2.85 - 2.95 (m, 1H), 2.65 - 2.75 (m, 2H), 2.55 - 2.60 (m, 2H), 2.45 - 2.50 (m, 1H), 2.20 - 2.30 (m, 1H), 2.10 - 2.20 (m, 4H), 1.90 - 1.99 (m, 1H), 1.79 - 1.90 (m, 4H), 1.77 - 1.79 (m, 3H), 1.38 - 1.50 (m, 1H), 1.20 - 1.38 (m, 4H). Mass spec: m/z: 754.70 $[M+H]^+$.

**Example 36 was synthesised following Scheme 34**

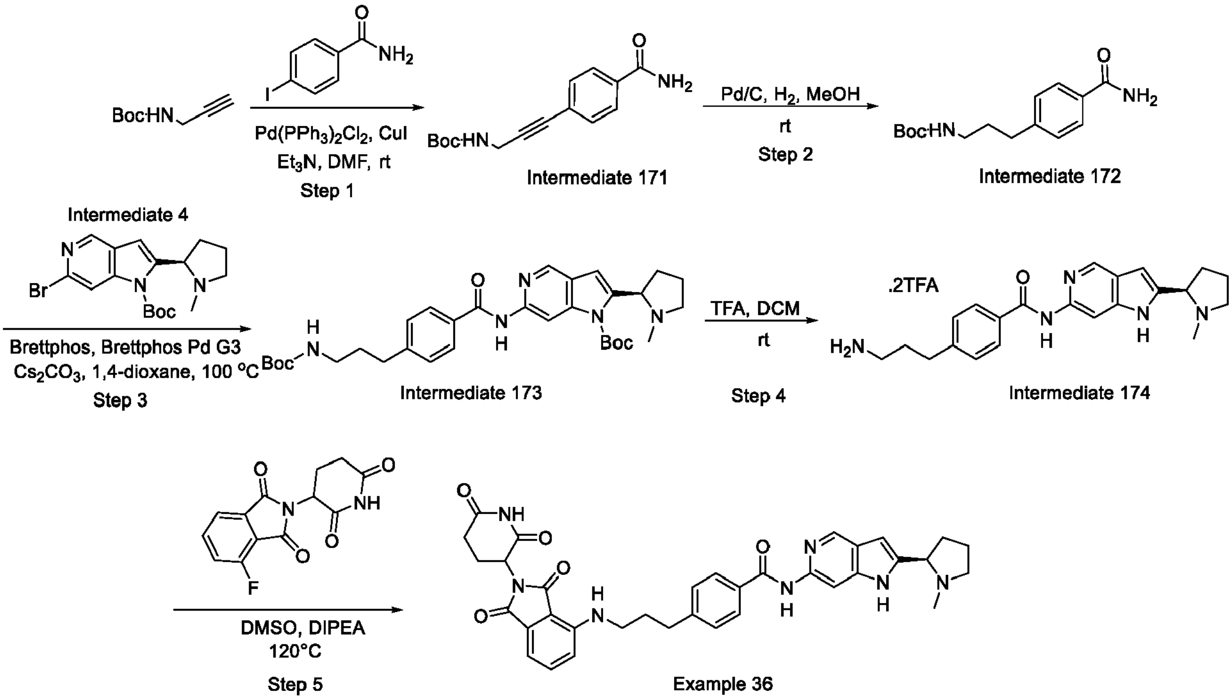


**Scheme 34**

**Step 1**

**Intermediate 171: *tert*-butyl N-[3-(4-carbamoylphenyl)prop-2-yn-1-yl]carbamate**

To a solution of 4-iodobenzamide (CAS No: 3956-07-8, 800 mg, 3.23 mmol) in DMF (10 mL) was added tert-butyl N-(prop-2-yn-1-yl)carbamate (CAS No: 92136-39-5, 603 mg, 3.88 mmol), $Pd(PPh_3)_2Cl_2$ (227 mg, 0.32 mmol), CuI (61.6 mg, 0.324 mmol) and $Et_3N$ (983 mg, 9.71 mmol) and the reaction was stirred at room temperature for 4h under $N_2$. The mixture was quenched with water (20 mL) and the aqueous phase was extracted with EtOAc (3 × 20 mL). The combined organic layers were washed with brine, dried over anhydrous $Na_2SO_4$ and concentrated under reduced pressure. The resulting mixture was purified on a silica gel column, eluting with MeOH /DCM (6/94), to afford *tert*-butyl N-[3-(4-carbamoylphenyl)prop-2-yn-1-yl]carbamate (**Intermediate 171**, 800 mg, 90%) as a yellow solid. Mas spec: m/z: 275.0 $[M+H]^+$.

**Step 2**

**Intermediate 172: *tert*-butyl N-[3-(4-carbamoylphenyl)propyl]carbamate**

To a solution of *tert*-butyl *N*-[3-(4-carbamoylphenyl)prop-2-yn-1-yl]carbamate **(Intermediate 171,** 600 mg, 2.18 mmol) in MeOH (10 mL) was added Pd/C (10% w/w, 300 mg, 2.81 mmol) and hydrogen (3 atm) was introduced and the reaction was stirred at room temperature for 2 h. The reaction was then filtered through a Celite pad washing with MeOH. The volatiles were then concentrated under reduced pressure to afford *tert*-butyl N-[3-(4-carbamoylphenyl)propyl] carbamate (**Intermediate 172**, 500 mg, 91%) as a yellow solid, which was used in the next step without further purification. Mass spec m/z: 279.0 $[M+H]^+$.

**Step 3**

**Intermediate 173: *tert*-butyl 6-(4-{3-[(*tert*-butoxycarbonyl)amino]propyl}benzamido)-2-[(2R)-1-methylpyrrolidin-2-yl]pyrrolo[3,2-c]pyridine-1-carboxylate**

To a solution of *tert*-butyl N-[3-(4-carbamoylphenyl)propyl]carbamate (**Intermediate 172**, 250 mg, 0.9 mmol) and *tert*-butyl 6-bromo-2-[(2R)-1-methylpyrrolidin-2-yl]pyrrolo[3,2-c]pyridine-1-carboxylate (**Intermediate 4**, 342 mg, 0.9 mmol) in 1,4-dioxane (10 mL) was added $Cs_2CO_3$ (585.2 mg, 1.79 mmol), Brettphos (289.2 mg, 0.539 mmol) and Brettphos Pd G3 (244.2 mg, 0.269 mmol) and the reaction was stirred overnight at 100 °C under the $N_2$. The volatiles were removed under reduced pressure and the resulting residue was chromatographed on a silica gel column, eluting with MeOH/DCM (6/94), to afford *tert*-butyl 6-(4-{3-[(*tert*-butoxycarbonyl)amino]propyl}benzamido)-2-[(2R)-1-methylpyrrolidin-2-yl]pyrrolo[3,2-c]pyridine-1-carboxylate (**Intermediate 173**, 190 mg, 36%) as a yellow solid. Mass spec: m/z: 578.0 $[M+H]^+$.

**Step 4**

**Intermediate 174: 4-(3-aminopropyl)-N-{2-[(2R)-1-methylpyrrolidin-2-yl]-1H-pyrrolo[3,2-c]pyridin-6-yl}benzamide ditrifluoroacetate**

To a solution of *tert*-butyl 6-(4-{3-[(*tert*-butoxycarbonyl)amino]propyl}benzamido)-2-[(2R)-1-methylpyrrolidin-2-yl]pyrrolo[3,2-c]pyridine-1-carboxylate (**Intermediate 173,** 190 mg, 0.329 mmol) in DCM (6 mL) was added TFA (2 mL). The reaction was stirred at room temperature for 2 h and concentrated under reduced pressure to get 4-(3-aminopropyl)-N-{2-[(2R)-1-methylpyrrolidin-2-yl]-1H-pyrrolo[3,2-c]pyridin-6-yl}benzamide ditrifluoroacetate (**Intermediate 174**, 120 mg, 97%) as a yellow solid, which was used in the next step without further purification. Mass spec: m/z: 378.0 $[M+H]^+$.

**Step 5**

**Example 36: 4-(3-{[2-(2,6-dioxopiperidin-3-yl)-1,3-dioxoisoindol-4-yl]amino}propyl)-N-{2-[(2R)-1-methylpyrrolidin-2-yl]-1H-pyrrolo[3,2-c]pyridin-6-yl}benzamide**

To a solution of 4-(3-aminopropyl)-N-{2-[(2R)-1-methylpyrrolidin-2-yl]-1H-pyrrolo[3,2-c]pyridin-6-yl}benzamide ditrifluoroacetate (**Intermediate 174**, 120 mg, 0.32 mmol) and 2-(2,6-dioxopiperidin-3-yl)-4-fluoroisoindole-1,3-dione (CAS No: 835616-60-9, 87.8 mg, 0.318 mmol) in DMSO (4 mL) was added DIPEA (411 mg, 3.18 mmol) and the reaction was stirred at 120°C for 4 h. The reaction mixture was cooled and and purified by preparative HPLC (Column: YMC-Actus Triart C18ExRs, 30*150 mm, 5μm; Mobile Phase A: Water (10 mmol/L $NH_4HCO_3$), Mobile Phase B: MeCN; Flow rate: 60 mL/min; Gradient: 33% B to 63% B in 7 min) to afford 4-(3-{[2-(2,6-dioxopiperidin-3-yl)-1,3-dioxoisoindol-4-yl]amino}propyl)-N-{2-[(2R)-1-methylpyrrolidin-2-yl]-1H-pyrrolo[3,2-c]pyridin-6-yl}benzamide (**Example 36**, 34.1 mg, 17%) as a yellow solid. $^1H$ NMR (400 MHz, DMSO-d6) δ ppm 11.36 (s, 1H), 11.10 (s, 1H), 10.36 (s, 1H), 8.50 (s, 1H), 8.19 (s, 1H), 7.99 (d, *J* = 8.0 Hz, 2H), 7.58 (t, *J* = 8.0 Hz, 1H), 7.36 (d, *J* = 8.4 Hz, 2H), 7.02 - 7.09 (m, 2H), 6.63 (t, *J* = 6.4 Hz, 1H), 6.39 (s, 1H), 5.04 - 5.08 (m, 1H), 3.32 - 3.36 (m, 2H), 3.26 - 3.28 (m, 1H), 3.12 - 3.16 (m, 1H), 2.85 - 2.89 (m, 1H), 2.74 - 2.78 (m, 2H), 2.51 - 2.55 (m, 3H), 2.25 - 2.33 (m, 1H), 2.14 - 2.19 (m, 4H), 2.00 - 2.05 (m, 1H), 1.98 - 1.99 (m, 3H), 1.89 - 1.96 (m, 1H). Mass spec: m/z: 634.3 $[M+H]^+$.

**Example 37 was synthesised following Scheme 35**

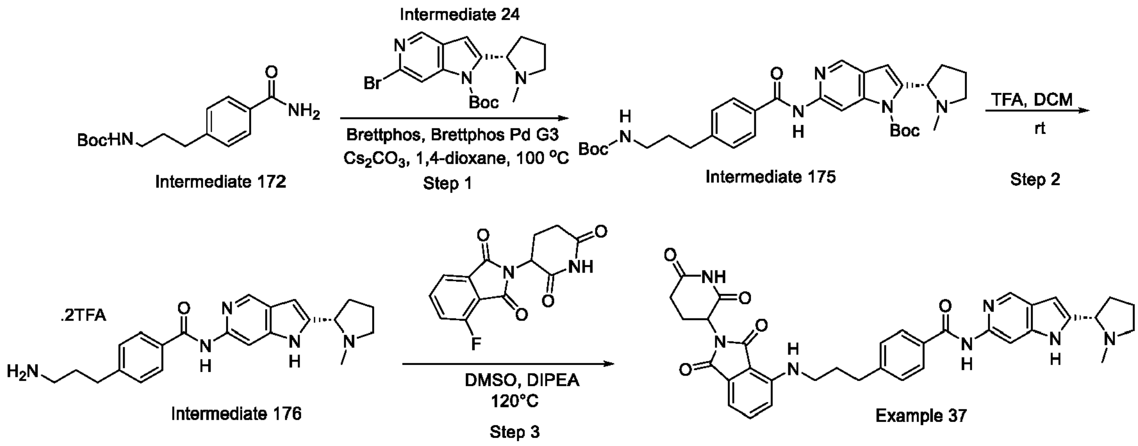


## Scheme 35

**Step 1**

**Intermediate 175: *tert*-butyl 6-(4-{3-[(tert-butoxycarbonyl)amino]propyl}benzamido)-2-[(2S)-1-methylpyrrolidin-2-yl]pyrrolo[3,2-c]pyridine-1-carboxylate**

**Intermediate 175** was synthesised in an analogous manner to **Intermediate 173** following **Step 3** in **Scheme 34,** using *tert*-butyl N-[3-(4-carbamoylphenyl)propyl]carbamate (**Intermediate 172**) as the amide and tert-butyl 6-bromo-2-[(2S)-1-methylpyrrolidin-2-yl]pyrrolo[3,2-c]pyridine-1-carboxylate (**Intermediate 24**) as the aryl bromide, to afford *tert*-butyl 6-(4-{3-[(*tert*-butoxycarbonyl)amino]propyl}benzamido)-2-[(2S)-1-methylpyrrolidin-2-yl]pyrrolo[3,2-c]pyridine-1-carboxylate (**Intermediate 175**). Mass spec: m/z: 578.3 $[M+H]^+$.

**Step 2**

**Intermediate 176: 4-(3-aminopropyl)-N-{2-[(2S)-1-methylpyrrolidin-2-yl]-1H-pyrrolo[3,2-c]pyridin-6-yl}benzamide**

**Intermediate 176** was synthesised in an analogous manner to **Intermediate 174** following **Step 4** in **Scheme 34,** using *tert*-butyl 6-(4-{3-[(*tert*-butoxycarbonyl)amino]propyl}benzamido)-2-[(2S)-1-methylpyrrolidin-2-yl]pyrrolo[3,2-c]pyridine-1-carboxylate (**Intermediate 175**) as the amide, to afford 4-(3-aminopropyl)-N-{2-[(2S)-1-methylpyrrolidin-2-yl]-1H-pyrrolo[3,2-c]pyridin-6-yl}benzamide ditrifluoroacetate (**Intermediate 176**). Mass spec: m/z: 378.2 $[M+H]^+$.

Step 3

**Example 37: 4-(3-{[2-(2,6-dioxopiperidin-3-yl)-1,3-dioxoisoindol-4-yl]amino}propyl)-N-{2-[(2S)-1-methylpyrrolidin-2-yl]-1H-pyrrolo[3,2-c]pyridin-6-yl}benzamide**

**Example 37** was synthesised in an analogous manner to **Example 36,** following **Step 5** in **Scheme 34,** using 4-(3-aminopropyl)-N-{2-[(2S)-1-methylpyrrolidin-2-yl]-1H-pyrrolo[3,2-c]pyridin-6-yl}benzamide ditrifluoroacetate (**Intermediate 176**) as the amine and 2-(2,6-dioxopiperidin-3-yl)-4-fluoroisoindole-1,3-dione (CAS No: 835616-60-9) as the aryl fluoride, to afford 4-(3-{[2-(2,6-dioxopiperidin-3-yl)-1,3-dioxoisoindol-4-yl]amino}propyl)-N-{2-[(2S)-1-methylpyrrolidin-2-yl]-1H-pyrrolo[3,2-c]pyridin-6-yl}benzamide (**Example 37**). $^1H$ NMR (400 MHz, MeOH-d4) δ ppm 8.55 (s, 1H), 8.19 (s, 1H), 7.92 (d, *J* = 8.0 Hz, 2H), 7.55(t, *J* = 7.2 Hz, 1H), 7.41 (d, *J* = 8.0 Hz, 2H), 7.00 - 7.06 (m, 2H), 6.52 (s, 1H), 5.03 - 5.08 (m, 1H), 3.40 - 3.47 (m, 3H), 3.24 - 3.26 (m, 1H), 2.80 - 2.89 (m, 3H), 2.70 - 2.74 (m, 2H), 2.40 - 2.42 (m, 1H), 2.29 - 2.33 (m, 4H), 2.02 - 2.13 (m, 5H), 1.92 - 1.94 (m, 1H). Mass spec: m/z: 634.5 $[M+H]^+$.

**Example 38 was synthesised following Scheme 36**

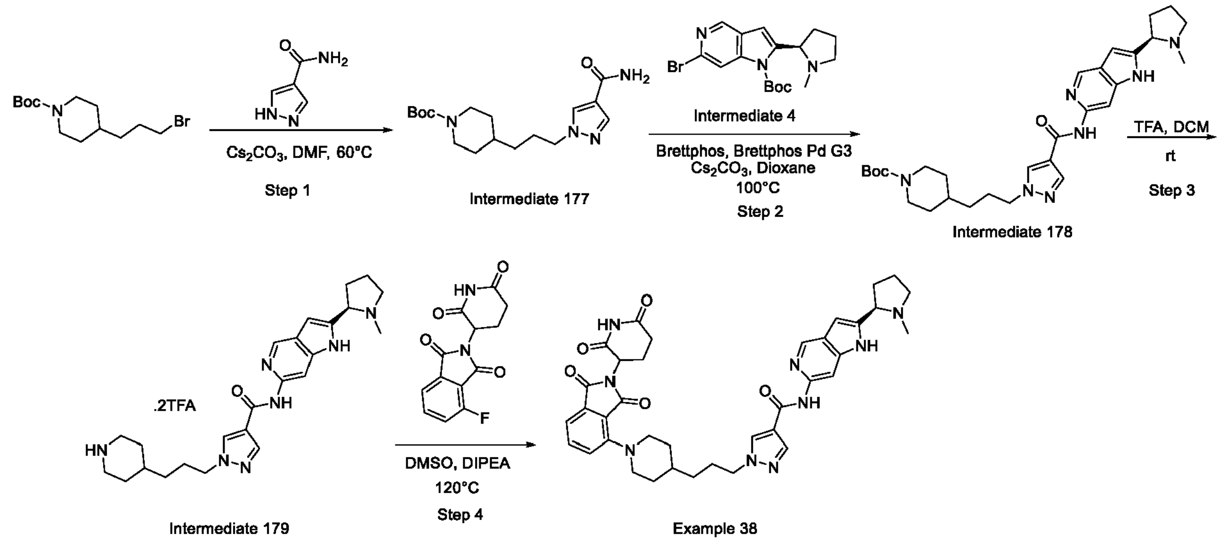


## Scheme 36

**Step 1**

**Intermediate 177: *tert*-butyl 4-[3-(4-carbamoylpyrazol-1-yl)propyl]piperidine-1-carboxylate**

To a solution of *tert*-butyl 4-(3-bromopropyl)piperidine-1 -carboxylate (CAS No: 164149-27-3, 600 mg, 1.95 mmol) in DMF (20 mL) was added 1H-pyrazole-4-carboxamide (CAS No: 437701-80-9, 217 mg, 1.95 mmol) and $Cs_2CO_3$ (1915 mg, 5.87 mmol) and the reaction was stirred overnight at 60 °C for 2h. The mixture was quenched with water (100 mL) and extracted with EtOAc (3 × 100 mL). The combined organic layers were washed with brine, dried over anhydrous $Na_2SO_4$ and the volatiles removed under reduced pressure. The resulting crude was chromatographed on a silical gel column with MeOH/DCM (5/95) to afford *tert*-butyl 4-[3-(4-carbamoylpyrazol-1-yl)propyl]piperidine-1-carboxylate (**Intermediate 177**, 650 mg, 91%) as a yellow solid. Mass spec: m/z: 337 $[M+H]^+$.

**Step 2**

**Intermediate 178: *tert*-butyl 4-{3-[4-({2-[(2R)-1-methylpyrrolidin-2-yl]-1H-pyrrolo[3,2-c]pyridin-6-yl}carbamoyl) pyrazol-1-yl]propyl}piperidine-1-carboxylate**

To a solution of *tert*-butyl 4-[3-(4-carbamoylpyrazol-1-yl)propyl]piperidine-1-carboxylate (**Intermediate 177**, 200 mg, 0.594 mmol) in 1,4-dioxane (5 mL) was added *tert*-butyl 6-bromo-2-[(2R)-1-methylpyrrolidin-2-yl]pyrrolo[3,2-c] pyridine-1-carboxylate (**Intermediate 4**, 226 mg, 0.594 mmol), Brettphos (191 mg, 0.356 mmol), Brettphos Pd G3 (161 mg, 0.178 mmol) and $Cs_2CO_3$ (387 mg, 1.18 mmol) and the reaction was stirred overnight at 100°C under $N_2$. The crude mixture was then purified on a silical gel column with MeOH / DCM (5/95) to afford *tert*-butyl 4-{3-[4-({2-[(2R)-1-methylpyrrolidin-2-yl]-1H-pyrrolo[3,2-c]pyridin-6-yl}carbamoyl)pyrazol-1-yl]propyl}piperidine-1-carboxylate (**Intermediate 178**, 300 mg, 82%) as a yellow solid. Mass spec: m/z: 536 $[M+H]^+$.

**Step 3**

**Intermediate 179: N-{2-[(2R)-1-methylpyrrolidin-2-yl]-1H-pyrrolo[3,2-c]pyridin-6-yl}-1-[3-(piperidin-4-yl)propyl] pyrazole-4-carboxamide ditrifluroacetate**

To a solution of *tert*-butyl 4-{3-[4-({2-[(2R)-1-methylpyrrolidin-2-yl]-1H-pyrrolo[3,2-c]pyridin-6-yl}carbamoyl)pyrazol-1-yl]propyl}piperidine-1-carboxylate (**Intermediate 178, 150** mg, 0.28 mmol) in DCM (2 mL) was added TFA (1 mL) and the reaction mixture was stirred at room temperature for 1h. The volatiles were then removed under reduced pressure to afford N-{2-[(2R)-1-methylpyrrolidin-2-yl]-1H-pyrrolo[3,2-c]pyridin-6-yl}-1-[3-(piperidin-4-yl)propyl]pyrazole-4-carboxamide ditrifluroacetate (**Intermediate 179**, 122 mg) as a yellow oil, which was used in the next step without further purification. Mass spec: m/z: 436 $[M+H]^+$.

**Step 4**

**Example 38: 1-(3-{1-[2-(2,6-dioxopiperidin-3-yl)-1,3-dioxoisoindol-4-yl]piperidin-4-yl}propyl)-N-{2-[(2R)-1-methylpyrrolidin-2-yl]-1H-pyrrolo[3,2-c]pyridin-6-yl}pyrazole-4-carboxamide**

To a solution of N-{2-[(2R)-1-methylpyrrolidin-2-yl]-1H-pyrrolo[3,2-c]pyridin-6-yl}-1-[3-(piperidin-4-yl)propyl]pyrazole-4-carboxamide ditrifluoroacetate (**Intermediate 179,** 100 mg, 0.23 mmol) in DMSO (2 mL) was added 2-(2,6-dioxopiperidin-3-yl)-4-fluoroisoindole-1,3-dione (CAS No: 835616-60-9, 126 mg, 0.460 mmol) and DIPEA (89.0 mg, 0.690 mmol) and the reaction was stirred at 120°C overnight. The resulting crude product was then purified by preparative HPLC (Column: WelFlash C18, Regular C18 20-40 μm, 120g; Mobile Phase A: Water(10 mmol/L $NH_4HCO_3$ + 0.05% $NH_3.H_2O$), Mobile Phase B: MeCN; Flow rate: 60 mL/min mL/min; Gradient: 30% B to 60% B in 30 min; Wave Length: 254nm/220nm nm) to afford 1-(3-{1-[2-(2,6-dioxopiperidin-3-yl)-1,3-dioxoisoindol-4-yl]piperidin-4-yl}propyl)-N-{2-[(2R)-1-methylpyrrolidin-2-yl]-1H-pyrrolo[3,2-c]pyridin-6-yl}pyrazole-4-carboxamide (**Example 38**, 38.0 mg, 23%) as a yellow solid. $^1H$ NMR (400 MHz, MeOH-d4) δ ppm 8.51 (s, 1H), 8.31 (s, 1H), 2.11 - 2.12 (m, 2H), 7.63 - 7.66 (m, 1H), 7.31 - 7.35 (m, 2H), 6.52 (s, 1H), 5.08 - 5.13 (m, 1H), 4.25 - 4.28 (m, 2H), 3.75 - 3.78 (m, 2H), 3.46 - 3.50 (m, 1H), 3.20 - 3.30 (m, 1H), 2.83 - 2.93 (m, 3H), 2.72 - 2.78 (m, 2H), 2.40 - 2.42 (m, 1H), 2.20 - 2.31 (m, 4H), 1.80 - 2.12 (m, 7H), 1.30 - 1.60 (m, 6H). Mass spec: m/z: 692.3 $[M+H]^+$.

**Example 39 was synthesised following Scheme 37**

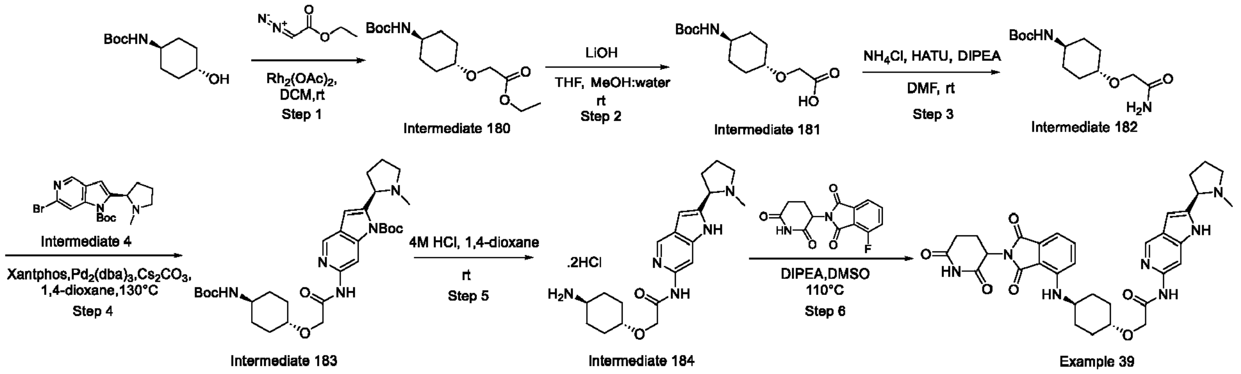


**Scheme 37**

**Step** 1

**Intermediate 180: Ethyl 2-(((1r, 4r)-4-((*tert*-butoxycarbonyl) amino)cyclohexyl)oxy)acetate**

To a solution of *tert*-butyl ((1r,4r)-4-hydroxycyclohexyl)carbamate (CAS No: 111300-06-2, 10.0 g, 46.4 mmol) in dichloromethane (50 mL) was added rhodium(II) acetate dimer (1.04 g, 2.32 mmol) at room temperature and the reaction mixture was stirred at room temperature for 10 min. Ethyl 2-diazoacetate (6.36 g, 55.7 mmol) was then added and the reaction stirred for 16h. The reaction mixture was poured into water (100 mL) and extracted with DCM (3 × 80 mL). The combined organic layers were dried over anhydrous $Na_2SO_4$ and concentrated *in vacuo*. The resulting residue was purified by combiflash column chromatography (20% EtOAc in heptane) to afford ethyl 2-(((1r, 4r)-4-((*tert*-butoxycarbonyl) amino)cyclohexyl)oxy)acetate (**Intermediate 180**, 8.0 g, 57%) as an off white solid. $^1H$ NMR (400 MHz, DMSO-$d_6$) δ ppm 1.11-1.17 (m, 3H), 1.18-1.21 (m, 4H), 1.37 (s, 9H), 1.73-1.77 (m, 2H), 1.91-1.98 (m, 2H), 3.16-3.27 (m, 2H), 4.07-4.13 (m, 2H), 4.19 (s, 2H), 6.68 (d, *J*=7.50 Hz, 1H).

**Step 2**

**Intermediate 181: 2-(((1r, 4r)-4-((*tert*-butoxycarbonyl) amino)cyclohexyl)oxy)acetic acid**

To a solution of ethyl 2-(((1r, 4r)-4-((*tert*-butoxycarbonyl) amino)cyclohexyl)oxy)acetate (**Intermediate 180**, 7.5 g, 25 mmol) in THF (40 mL), MeOH (20 mL) and water (30 mL) was added LiOH (1.8 g, 75 mmol) and the reaction was stirred at room temperature for 3h. The reaction mixture was then concentrated *in vacuo* and poured into water (200 mL), acidified to pH ~5 with dilute HCl and extracted with DCM (3 × 100 mL). The combined organic layers were dried over anhydrous $Na_2SO_4$ and concentrated *in vacuo* to afford 2-(((1r, 4r)-4-((*tert*-butoxycarbonyl)amino)cyclohexyl)oxy) acetic

acid (**Intermediate 181,** 5.1 g, 75%) as an off white solid. $^1$H NMR (400 MHz, $CDCl_3$) δ ppm 1.14-1.19 (m, 2H), 1.26-1.28 (m, 2H), 1.36-1.41 (m, 2H), 1.44 (s, 9H), 2.04-2.10 (m, 2H), 3.28-3.49 (m, 2H), 4.39 (s, 2H). Mass spec: m/z 271.9 $[M-H]^-$.

**Step 3**

**Intermediate 182: *tert*-butyl ((1r,4r)-4-(2-amino-2-oxoethoxy) cyclohexyl) carbamate**

To a solution of 2-(((1r,4r)-4-((*tert*-butoxycarbonyl) amino)cyclohexyl)oxy)acetic acid (**Intermediate 181,** 1.00 g, 3.659 mmol) in DMF (10 mL) was added HATU (2.15 g, 5.488 mmol) and ammonium chloride (0.78 g, 14.64 mmol) followed by *N,N*-diisopropylethylamine (1.45 g, 10.98 mmol) at room temperature. The reaction mixture was stirred at room temperature for 12h. Progress of the reaction was monitored by TLC. After completion, the reaction mixture was diluted with water (30 mL) and extracted with EtOAc (3 × 30 mL). The organic layer was separated, dried over anhydrous $Na_2SO_4$, and concentrated *in vacuo* to afford *tert*-butyl ((1r, 4r)-4-(2-amino-2-oxoethoxy)cyclohexyl)carbamate (**Intermediate 182**, 600 mg crude) as a yellow solid. $^1$H NMR (400 MHz, DMSO-$d_6$) δ ppm 1.08-1.28 (m, 4H), 1.37 (s, 9H), 1.74-1.76 (m, 2H), 1.94-1.97 (m, 2H), 3.14-3.25 (m, 2H), 3.78 (s, 2H), 6.70 (d, *J*=7.45 Hz, 1H), 7.01 (br s, 1H), 7.20 (br s, 1H).

**Step 4:**

**Intermediate 183: *tert*-butyl 6-(2-(((1r,4r)-4-((tert-butoxycarbonyl)amino)cyclohexyl)oxy)acetamido)-2-((R)-1-methylpyrrolidin-2-yl)-1*H*-pyrrolo[3, 2-c] pyridine-1-carboxylate**

To a solution of *tert*-butyl (R)-6-bromo-2-(1-methylpyrrolidin-2-yl)-1*H*-pyrrolo[3,2-c]pyridine-1-carboxylate (**Intermediate 182, 0.40** g, 1.052 mmol) and tert-butyl ((1r,4r)-4-(2-amino-2-oxoethoxy)cyclohexyl)carbamate (**Intermediate 4,** 0.22 g, 0.84 mmol) and in 1,4-dioxane (15.0 mL) was added Cesium carbonate (1.03 g, 3.15 mmol) and reaction mixture was degassed with Argon gas for 10 min. $Pd_2(dba)_3$ (0.14 g, 0.15 mmol) was added, followed by Xantphos (0.18 g, 0.315 mmol) and the mixture degassed with Argon for 5 min. The reaction mixture was then heated at 130°C for 2h. The reaction mixture was concentrated *in vacuo* and the resulting crude material was purified by combi-flash chromatography (50% EtOAc in heptane) to afford *tert*-butyl 6-(2-(((1r,4r)-4-((tert-butoxycarbonyl)amino)cyclohexyl)oxy)acetamido)-2-((R)-1-methylpyrrolidin-2-yl)-1*H*-pyrrolo [3, 2-c] pyridine-1-carboxylate (**Intermediate 183,** 0.32 g, 53%) as an off white solid. $^1$H NMR (400 MHz, DMSO-$d_6$) δ ppm 1.12-1.32 (m, 6H), 1.37 (s, 9H), 1.49-1.63 (m, 2H), 1.67 (s, 9H), 1.70-1.84 (m, 4H), 1.97-2.07 (m, 2H), 2.34 (s, 3H), 3.09-3.15 (m, 1H), 3.20-3.24 (m, 1H), 3.88-3.92 (m, 1H), 4.12 (s, 2H), 6.70 (s, 1H) 6.72 (s, 1H), 8.52 (s, 1H), 8.79 (s, 1H), 9.50 (s, 1H). Mass spec: m/z: 571.9 $[M+H]^+$.

**Step 5**

**Intermediate 184: 2-(((1r, 4r)-4-aminocyclohexyl) oxy)-*N*-(2-((R)-1-methylpyrrolidin-2-yl)-1*H*-pyrrolo [3, 2-c] pyridin-6-yl) acetamide dihydrochloride**

To a solution of *tert*-butyl 6-(2-(((1r,4R)-4-((tert-butoxycarbonyl)amino)cyclohexyl)oxy)acetamido)-2-((R)-1-methylpyrrolidin-2-yl)-1*H*-pyrrolo[3,2-c]pyridine-1-carboxylate (**Intermediate 183, 0.30** g, 0.52 mmol) in 1,4-dioxane (5.0 mL) was added HCl (4M in 1,4-dioxane, 5.0 mL) at 0°C. The reaction mixture was stirred at room temperature for 16h then concentrated *in vacuo* to afford 2-(((1r,4r)-4-aminocyclohexyl) oxy)-*N*-(2-((R)-1-methylpyrrolidin-2-yl)-1*H*-pyrrolo [3, 2-c] pyridin-6-yl) acetamide dihydrochloride (**Intermediate 184,** 0.25 g) as an off white solid, which was used in the next step without further purification. $^1$H NMR (400 MHz, DMSO-$d_6$) δ ppm 1.30-1.46 (m, 4H), 1.91-1.99 (m, 4H), 2.09-2.24 (m, 4H), 2.32-2.40 (m, 2H), 2.79 (s, 3H), 2.99-3.05 (m, 1H), 3.15-3.33 (m, 1H), 3.39-3.47 (m,1H), 4.26 (s, 2H), 7.21 (s, 1H), 8.08 (s, 2H), 8.16 (s, 1H), 8.99 (s, 1H), 11.03 (br s, 1H), 11.43 (br s, 1H). Mass spec: m/z 372 $[M+H]^+$.

**Step 6**

**Example 39: 2-(((1r,4r)-4-((2-(2,6-dioxopiperidin-3-yl)-1,3-dioxoisoindolin-4-yl)amino)cyclohexyl) oxy)-*N*-(2-((R)-1-methylpyrrolidin-2-yl)-1*H*-pyrrolo[3,2-c]pyridin-6-yl)acetamide**

To a solution of 2-(((1r,4r)-4-aminocyclohexyl)oxy)-*N*-(2-((R)-1-methylpyrrolidin-2-yl)-1*H*-pyrrolo[3,2-c] pyridin-6-yl) acetamide dihydrochloride (**Intermediate 184,** 0.25 g, 0.67 mmol) and 2-(2,6-dioxopiperidin-3-yl)-4-fluoroisoindoline-1,3-dione (CAS No: 835616-60-9, 0.18 g, 0.67 mmol) in DMSO (1 mL) was added diisopropylethylamine (0.44 g, 3.36 mmol) and the reaction mixture heated at 130°C for 2h. The reaction mixture was then poured into ice cold water, resulting a solid precipitate, which was filtered and dried under vacuum. The crude solid was purified by preparative HPLC

(Method A), pure fractions were collected and lyophilised to afford 2-(((1r,4r)-4-((2-(2,6-dioxopiperidin-3-yl)-1,3-dioxoisoindolin-4-yl)amino)cyclohexyl)oxy)-N (2-((R)-1-methylpyrrolidin-2-yl)-1*H*-pyrrolo[3,2-c]pyridin-6-yl)acetamide (**Example 39,** 0.042 g, 10%) as a yellow solid. $^1$H NMR (400 MHz, DMSO-$d_6$) δ ppm 1.32-1.40 (m, 2H), 1.44-1.53 (m, 2H), 1.77-1.89 (m, 3H), 2.02-2.09 (m, 6H), 2.15 (s, 3H), 2.22-2.28 (m, 1H), 2.52-2.54 (m, 1H) 2.84-2.92 (m, 1H), 3.11-3.14 (m, 1H), 3.28-3.32 (m, 2H), 3.47-3.54 (m, 1H), 3.60-3.63 (m, 1H), 4.13 (s, 2H), 5.01-5.06 (m, 1H), 6.20 (d, *J*=8.25 Hz, 1H), 6.37 (s, 1H), 7.04 (d, *J*=7.00 Hz, 1H), 7.20 (d, *J*=8.63 Hz, 1H), 7.59-7.60 (m, 1H), 8.08 (s, 1H), 8.43 (s, 1H), 9.31 (br s, 1H), 11.08 (br s, 1H), 11.34 (br s, 1H). Mass spec: m/z 628.4 [M+H]$^+$.

**Example 40 was synthesised following Scheme 38**

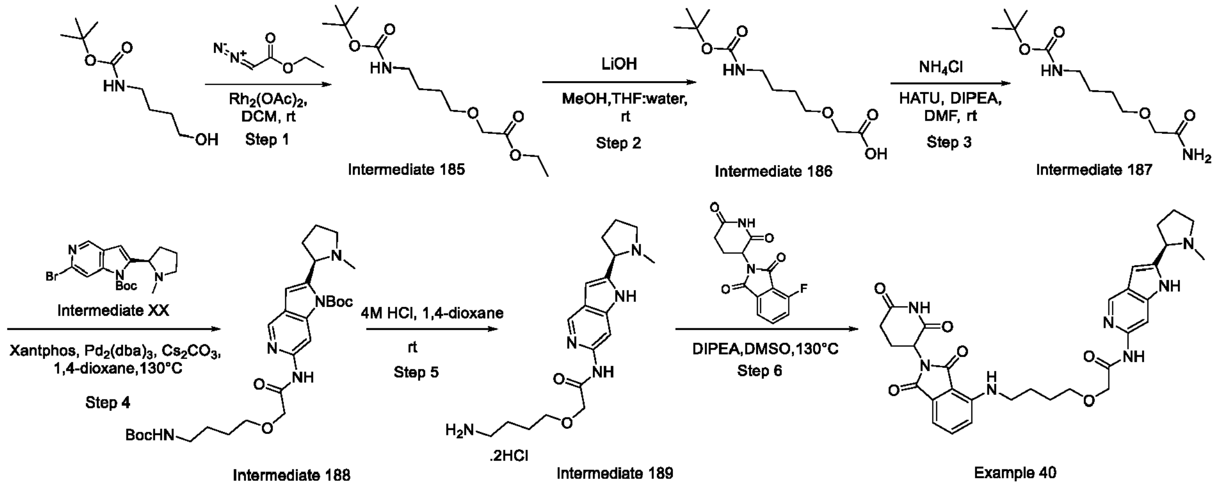


**Scheme 38**

**Step 1**

**Intermediate 185: ethyl 2-(4-((*tert*-butoxycarbonyl)amino)butoxy)acetate**

To a solution of tert-butyl (4-hydroxybutyl) carbamate (10.0 g, 52.84 mmol) in dichloromethane (100 mL) was added rhodium (ii) acetate dimer (1.18 g, 2.64 mmol) at room temperature. The reaction mixture was stirred for 10 min then ethyl 2-diazoacetate (7.23 g, 63.40 mmol) was added dropwise at room temperature. The reaction mixture was stirred at room temperature for 24h. Progress of the reaction was monitored by TLC. After completion, the reaction mixture was poured into water (100 mL) extracted with DCM (3 × 100 mL). The organic layer was separated, dried over anhydrous $Na_2SO_4$ and concentrated *in vacuo.* The crude material was purified by combi-flash column chromatography by eluting with 20% EtOAc in heptane to afford ethyl 2-(4-((*tert*-butoxycarbonyl) amino) butoxy) acetate (**Intermediate 185,** 8.0 g, 55%) as an off white solid. $^1$H NMR (400 MHz, DMSO-$d_6$) δ ppm 1.17-1.21 (m, 3H), 1.38 (s, 9H), 1.45-1.49 (m, 4H), 2.88-2.93 (m, 2H), 3.40-3.44 (m, 2H), 4.05 (s, 2H), 4.08-4.14 (m, 2H).

**Step 2**

**Intermediate 186: 2-(4-((tert-butoxycarbonyl)amino)butoxy) acetic acid**

To a solution of ethyl 2-(4-((*tert*-butoxycarbonyl)amino)butoxy)acetate (**Intermediate 185, 2.5** g, 0.10 mmol) in tetrahydrofuran (20 mL) and methanol (10 mL) was added lithium hydroxide (0.72 g, 0.03 mmol) in water (10 mL) at 0°C. The reaction mixture was stirred at room temperature for 24h. Progress of the reaction was monitored by TLC. After completion, the reaction mixture was concentrated *in vacuo,* and diluted with water (20 mL), pH was adjusted to 3-4 with saturated solution of citric acid (15 mL) and extracted with EtOAc (2 × 50 mL). The organic layer was separated, dried over anhydrous $Na_2SO_4$ and concentrated *in vacuo.* The crude material was purified by combi-flash column chromatography by eluting with 50% EtOAc in heptane to afford 2-(4-((*tert*-butoxycarbonyl)amino)butoxy) acetic acid (**Intermediate 186**, 2.2 g, 88%) as a yellow liquid. $^1$H NMR (400 MHz, DMSO-$d_6$) δ ppm 1.37 (s, 9H), 1.41-1.49 (m, 4H), 2.88-2.93 (m, 2H), 3.31-3.43 (m, 2H), 3.96 (s, 2H), 6.77 (br s, 1H), 12.46 (br s, 1H).

**Step 3**

**Intermediate 187: *tert*-butyl (4-(2-amino-2-oxoethoxy)butyl)carbamate**

To a solution of 2-(4-((*tert*-butoxycarbonyl)amino)butoxy)acetic acid (**Intermediate 186**, 2.2 g, 9.4 mmol) in DMF (2 mL) was added diisopropylethylamine (8.2 mL, 47.0 mmol) followed HATU (5.5 g, 14.0 mmol) and ammonium chloride (2.5 g, 47.0 mmol). The reaction mixture was stirred at room temperature for 12h. Progress of the reaction was monitored by TLC. After completion, the reaction mixture was diluted with water (50 mL) and extracted with EtOAc (3 × 50 mL). The organic layer was separated, dried over anhydrous $Na_2SO_4$ and concentrated *in vacuo*. The crude material was purified by combi-flash column chromatography by eluting with 80% EtOAc in heptane to afford *tert*-butyl (4-(2-amino-2-oxoethoxy) butyl)carbamate (**Intermediate 187**, 1.8 g, 82%) as a yellow solid. $^1$H NMR (400 MHz, DMSO-$d_6$) 1.35 (s, 9H), 1.44-1.50 (m, 4H), 2.91-2.93 (m, 2H), 3.36-3.42 (m, 2H), 4.02 (s, 2H) 6.68 (s, 1H), 7.40 (s, 1H), 7.60 (d, *J* = 8.32 Hz, 1H).

**Step 4**

**Intermediate 188: *tert*-butyl (R)-6-(2-(4-((*tert*-butoxycarbonyl)amino)butoxy)acetamido)-2-(1-methylpyrrolidin-2-yl)-1*H*-pyrrolo[3,2-c]pyridine-1-carboxylate**

To a solution of *tert*-butyl (R)-6-bromo-2-(1-methylpyrrolidin-2-yl)-1*H*-pyrrolo[3,2-c]pyridine-1-carboxylate (**Intermediate 4,** 0.40 g,1.05 mmol) in 1,4-dioxane was added tert-butyl (4-(2-amino-2-oxoethoxy) butyl) carbamate (**Intermediate 187**, 0.21 g, 0.80 mmol) (15 mL) followed by cesium carbonate (1.03 g, 3.15 mmol). The reaction mixture was purged with argon for 10 min then added $Pd_2(dba)_3$ (0.15 g, 0.15 mmol) and Xantphos (0.18 g, 0.31 mmol) at room temperature. The reaction mixture was again purged with argon gas and heated at 130°C for 2h. Progress of the reaction was monitored by TLC. After completion, the reaction mixture was concentrated *in vacuo*. The crude material was purified by combi-flash column chromatography by eluting with 95% EtOAc in heptane to *tert*-butyl (R)-6-(2-(4-((*tert*-butoxycarbonyl)amino)butoxy)acetamido)-2-(1-methylpyrrolidin-2-yl)-1*H*-pyrrolo [3, 2-c] pyridine-1-carboxylate (**Intermediate 188,** 0.3 g, 52%) as an off-white solid. Mass spec: m/z 546.4 [M+H]+.

**Step 5**

**Intermediate 189: (R)-2-(4-aminobutoxy)-N-(2-(1-methylpyrrolidin-2-yl)-1*H*-pyrrolo[3,2-c]pyridin-6-yl) acetamide dihydrochloride**

To a solution of solution of tert-butyl (R)-6-(2-(4-((tert-butoxycarbonyl) amino) butoxy) acetamido)-2-(1-methylpyrrolidin-2-yl)-1*H*-pyrrolo [3, 2-c] pyridine-1-carboxylate (**Intermediate 188,** 0.28 g, 0.51 mmol) in 1, 4-dioxane (5.0 mL) was added 4M HCl in 1,4-dioxane (5.0 mL) at 0°C. The reaction mixture was stirred at room temperature for 16h. Progress of the reaction was monitored by TLC. After completion, the reaction mixture was concentrated *in vacuo* to afford (R)-2-(4-aminobutoxy)-*N*-(2-(1-methylpyrrolidin-2-yl)-1*H*-pyrrolo[3,2-c]pyridin-6-yl) acetamide dihydrochloride (**Intermediate 189,** 0.22 g) as an off-white solid which was used in the next step without further purification.

**Step 6**

**Example 40: 2-(4-((2-(2,6-dioxopiperidin-3-yl)-1,3-dioxoisoindolin-4-yl)amino)butoxy)-*N*-(2-((R)-1-methylpyrrolidin-2-yl)-1*H*-pyrrolo[3,2-c]pyridin-6-yl)acetamide**

To a solution of (R)-2-(4-aminobutoxy)-*N*-(2-(1-methylpyrrolidin-2-yl)-1*H*-pyrrolo[3,2-c]pyridin-6-yl)acetamide dihydrochloride (**Intermediate 189,** 0.22 g, 0.22 mmol) and 2-(2,6-dioxo-3-piperidyl)-4-fluoro-isoindoline-1,3-dione (CAS No: 835616-60-9, 0.18 g, 0.64 mmol) in dimethyl sulfoxide (1.0 mL) was added *N*,*N*-Diisopropylethylamine (0.42 g, 3.20 mmol). The reaction mixture was heated at 130°C for 2h. Progress of reaction was monitored by TLC. After completion, reaction mixture was poured into ice cold water (15 mL), yellow solid precipitated, which was filtered, dried to obtain crude compound. The crude material was purified by preparative HPLC (Method A) to afford 2-(4-((2-(2,6-dioxopiperidin-3-yl)-1,3-dioxoisoindolin-4-yl)amino)butoxy)-*N*-(2-((R)-1-methylpyrrolidin-2-yl)-1*H*-pyrrolo[3,2-c]pyridin-6-yl)acetamide (**Example 40**, 0.02 g, 6%) as a yellow solid. $^1$H NMR (400 MHz, DMSO-$d_6$) $\delta$ ppm 1.64-1.72 (m, 4H), 1.75-1.94 (m, 3H), 1.98-2.06 (m, 1H), 2.10-2.12 (m, 1H), 2.15 (s, 3H), 2.21-2.26 (m, 1H), 2.54-2.62 (m, 2H), 2.83-2.92 (m, 1H), 3.10-3.14 (m, 1H), 3.25-3.28 (m, 1H), 3.30-3.38 (m, 2H), 3.56-3.60 (m, 2H), 4.09 (s, 2H), 5.02-5.07 (m, 1H), 6.36 (br s, 1H), 6.60 (t, *J*=5.75 Hz, 1H), 7.01 (d, *J*=7.00 Hz, 1H), 7.14 (d, *J*=8.50 Hz, 1H), 7.54-7.59 (m, 1H), 8.07 (s, 1H), 8.41 (s, 1H), 9.44 (br s, 1H), 11.08 (br s, 1H), 11.32 (br s, 1H). Mass spec: m/z 602.2 $[M+H]^+$.

**Example 41 was synthesised following Scheme 39**

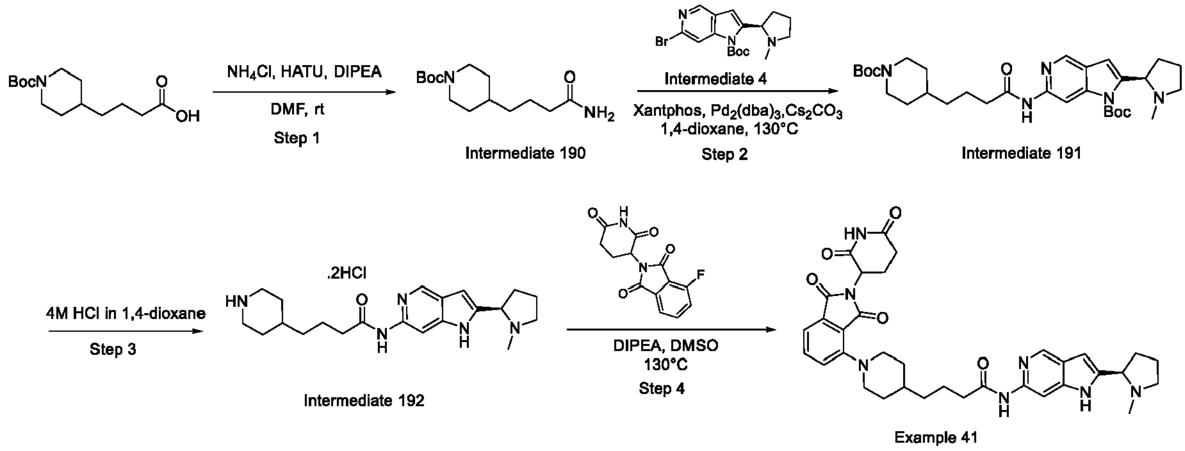


# Scheme 39

**Step** 1

**Intermediate 190: *tert*-butyl 4-(4-amino-4-oxobutyl) piperidine-1-carboxylate**

To a solution of 4-(1-(*tert*-butoxycarbonyl)piperidin-4-yl)butanoic acid (CAS No: 142247-38-9, 2 g, 7 mmol) in DMF (20 mL) was added HATU (1.5 g, 3.8 mmol) followed by $NH_4Cl$ (4.0 g, 75 mmol) and *N,N*-Diisopropylethylamine (3 g, 20 mmol) at room temperature. The reaction mixture was stirred at room temperature for 12h. The reaction mixture was then diluted with water (100 mL) and extracted with EtOAc (3 × 100 mL). The organic layer was separated, dried over anhydrous $Na_2SO_4$ and concentrated *in vacuo.* The crude material was purified by combi-flash column chromatography, by eluting with 50% EtOAc in heptane, to afford *tert*-butyl 4-(4-amino-4-oxobutyl) piperidine-1-carboxylate (**Intermediate 190,** 1.5g, 51%) as Colorless Liquid. $^1H$ NMR (400 MHz, DMSO-$d_6$) δ ppm 0.87-0.97 (m, 2H), 1.13-1.19 (m, 2H), 1.22-1.27 (m, 1H), 1.38 (s, 9H), 1.45-1.54 (m, 2H), 1.59-1.62 (m, 2H) 1.99-2.02 (m, 2H) 2.66-2.70 (m, 2H), 3.88-3.92 (m, 2H), 6.66 (s, 1H), 7.20 (br s, 1H). Mass spec: m/z 171.2 [M-Boc]$^+$.

**Step 2**

**Intermediate 191: *tert*-butyl (R)-6-(4-(1-(tert-butoxycarbonyl)piperidin-4-yl)butanamido)-2-(1-methylpyrrolidin-2-yl)-1*H*-pyrrolo[3,2-c]pyridine-1-carboxylate**

To stirred a solution of *tert*-butyl (R)-6-bromo-2-(1-methylpyrrolidin-2-yl)-1*H*-pyrrolo[3,2-c]pyridine-1-carboxylate (**Intermediate 4, 0.4** g,1.0 mmol) in 1,4-dioxane (20.0 mL) was added *tert*-butyl 4-(4-amino-4-oxobutyl)piperidine-1-carboxylate (**Intermediate 190**, 0.2 g, 0.8 mmol) and cesium carbonate (1.0 g, 0.3 mmol) at room temperature. The reaction mixture was purged with argon for 10 min then $Pd_2(dba)_3$ (0.1 g, 0.1 mmol) and 4,5-bis(diphenylphosphino)-9,9-dimethylxanthene (0.2 g, 0.3 mmol) were added at room temperature and the reaction mixture was then stirred at 130°C for 2h. The reaction mixture was then concentrated *in vacuo* and the resulting residue was purified by combi-flash column chromatography, by eluting with 80% EtOAc in heptane, to afford *tert*-butyl (R)-6-(4-(1-(*tert*-butoxycarbonyl)piperidin-4-yl) butanamido)-2-(1-methylpyrrolidin-2-yl)-1*H*-pyrrolo[3,2-c]pyridine-1-carboxylate (**Intermediate 191**, 0.3 g, 50%) as a grey solid. $^1H$ NMR (400 MHz, DMSO-$d_6$) δ ppm 0.92-0.98 (m, 2H), 1.18-1.27 (m, 2H), 1.38 (s, 9H), 1.57-1.61 (m, 7H),1.66 (s, 9H), 1.69-1.79 (m, 3H), 2.27 (s, 3H) 2.30-2.42 (m, 3H), 2.59-2.74 (m, 2H), 3.08-3.16 (m, 1H), 3.89-3.90 (m, 2H), 6.70 (s, 1H), 8.49 (s, 1H), 8.79 (s, 1H), 10.32 (br s, 1H). Mass spec: m/z 570.5 [M+H]$^+$.

**Step 3**

**Intermediate 192: (R)-*N*-(2-(1-methylpyrrolidin-2-yl)-1*H*-pyrrolo[3,2-c]pyridin-6-yl)-4-(piperidin-4-yl) butanamide dihydrochloride**

To a solution of *tert*-butyl (R)-6-(4-(1-(tert-butoxycarbonyl)piperidin-4-yl)butanamido)-2-(1-methylpyrrolidin-2-yl)-1*H*-pyrrolo[3, 2-c]pyridine-1-carboxylate (**Intermediate 191**, 0.3 g, 0.52 mmol) in 1,4-dioxane (5.0 mL) was added 4M hydrochloric acid in 1,4-dioxane (5.0 mL) at 0°C. The reaction mixture was stirred at room temperature for 16h. The reaction mixture was then concentrated under reduced pressure to afford (R)-*N*-(2-(1-methylpyrrolidin-2-yl)-1*H*-pyrrolo [3,2-c]pyridin-6-yl)-4-(piperidin-4-yl)butanamide dihydrochloride (**Intermediate 192**, 0.25 g) as an off-white solid used for next step without purification. $^1$H NMR (400 MHz, DMSO-$d_6$) δ ppm 1.23-1.34 (m, 4H), 1.55-1.61 (m, 1H), 1.63-1.70 (m, 2H), 1.79-1.82 (m, 2H), 2.15-2.20 (m, 2H), 2.33-2.38 (m, 2H), 2.54 (s, 3H), 2.68-2.85 (m, 3H), 3.16-3.24 (m, 3H), 3.64-3.75 (m, 2H), 4.67-4.72 (m, 1H), 7.21 (br s, 1H), 7.84 (s, 1H), 8.93 (s, 1H), 11.40 (br s, 1H), 12.08 (br s, 1H), 13.04 (br s, 1H). Mass spec: m/z 370.3 [M+H]$^+$.

**Step 4**

**Example 41: 4-(1-(2-(2,6-dioxopiperidin-3-yl)-1,3-dioxoisoindolin-4-yl)piperidin-4-yl)-*N*-(2-((R)-1-methylpyrrolidin-2-yl)-1*H*-pyrrolo[3,2-c]pyridin-6-yl)butanamide**

To a solution of (R)-*N*-(2-(1-methylpyrrolidin-2-yl)-1*H*-pyrrolo[3,2-c]pyridin-6-yl)-4-(piperidin-4-yl)butanamide dihydrochloride (**Intermediate 192**, 0.24 g, 0.64 mmol) and 2-(2,6-dioxopiperidin-3-yl)-4-fluoroisoindoline-1,3-dione (CAS No: 835616-60-9, 0.179 g, 0.64 mmol) in dimethyl sulfoxide (1.0 mL) was added *N,N*-Diisopropylethylamine (0.428 g, 3.0 mmol) and the reaction mixture was heated at 130°C for 2h. The reaction mixture was then poured into ice cold water, a solid precipitated which was filtered and dried to obtain crude compound. The obtained crude material was purified by preparative HPLC (Method A) to afforded 4-(1-(2-(2,6-dioxopiperidin-3-yl)-1,3-dioxoisoindolin-4-yl)piperidin-4-yl)-*N*-(2-((R)-1-methylpyrrolidin-2-yl)-1*H*-pyrrolo[3,2-c]pyridin-6-yl)butanamide (**Example 41**, 0.052 g, 13%) as a yellow solid. $^1$H NMR (400 MHz, DMSO-$d_6$) δ ppm 1.26-1.38 (m, 4H), 1.42-1.49 (m, 1H), 1.61-1.71 (m, 2H), 1.75-1.83 (m, 3H), 1.84-1.93 (m, 2H), 1.98-2.07 (m, 1H), 2.08-2.13 (m, 1H), 2.14 (s, 3H), 2.21-2.28 (m, 1H), 2.38 (t, *J*=7.25 Hz, 2H), 2.53-2.62 (m, 2H), 2.82-2.90 (m, 2H), 3.09-3.15 (m, 1H), 3.25-3.29 (m, 2H), 3.69-3.70 (m, 2H), 5.06-5.10 (m, 1H), 6.33 (s, 1H), 7.30-7.32 (m, 2H), 7.64-7.68 (m, 1H), 8.08 (s, 1H), 8.40 (s, 1H), 10.12 (s, 1H), 11.06 (br s, 1H), 11.24 (br s, 1H). Mass spec: m/z 626.5 [M+H]$^+$.

**Example 42 was prepared following Scheme 40**

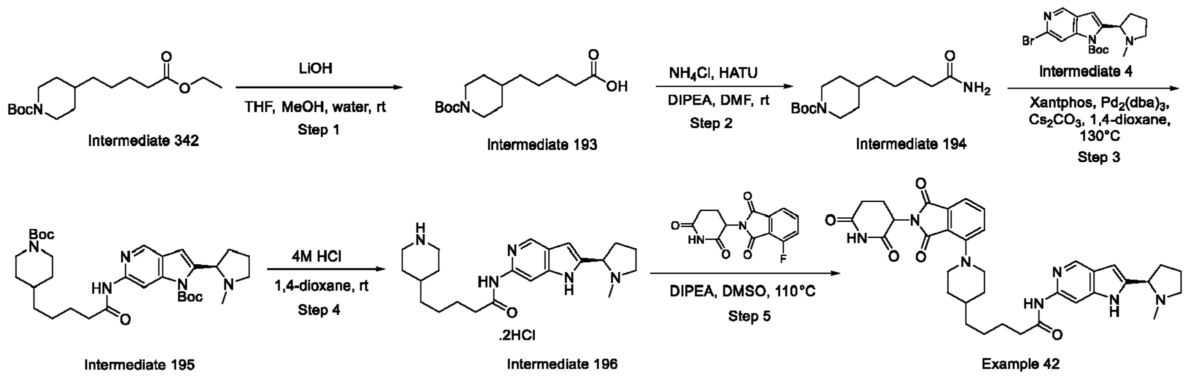


Scheme 40

**Step 1**

**Intermediate 193: 5-(1-*tert*-Butoxycarbonyl-4-piperidyl)pentanoic acid**

To a tert-butyl 4-(5-ethoxy-5-oxopentyl)piperidine-1-carboxylate (**Intermediate 342,** 2.50 g, 8.4 mmol) in methanol (8 mL) and THF (8 mL) was added lithium hydroxide (0.60 g, 25 mmol) in water (10 mL). The reaction mixture was stirred at room temperature for 6h. Progress of the reaction was monitored by TLC. After completion, the reaction mixture was concentrated *in vacuo.* The reaction mixture was diluted with water (50 mL), acidified to ~pH5 using aqueous 1 N HCl and extracted with EtOAc (2 × 100 mL). The combined organic layer was dried over $Na_2SO_4$ and concentrated *in vacuo* to afford 5-(1-*tert*-butoxycarbonyl-4-piperidyl)pentanoic acid (**Intermediate 193,** 2.30 g) as a liquid which was used for the next step without further purification. $^1$H NMR (400 MHz, DMSO-$d_6$) δ ppm 0.87-0.97 (m, 2H), 1.15-1.23 (m, 2H), 1.25-1.31 (m, 2H), 1.38 (s, 9H), 1.42-1.51 (m, 2H),1.53-1.65 (m, 2H), 2.19 (t, *J*=7.23 Hz, 2H), 2.65 (s, 2H), 3.31 (s, 1H),

3.90-3.91 (m, 2H), 11.97 (br s, 1H). Mass spec: m/z 286.2 $[M+H]^+$.

**Step 2**

**Intermediate 194: tert-Butyl 4-(5-amino-5-oxopentyl) piperidine-1-carboxylate**

To a solution of 5-(1-*tert*-butoxycarbonyl-4-piperidyl) pentanoic acid (**Intermediate 193**, 2.30 g, 8.06 mmol) in DMF (15 mL) was added HATU (4.74 g, 12.1 mmol) and *N*,*N*-diisopropylethylamine (3.19 g, 24.2 mmol) followed by ammonium chloride (1.73 g, 32.2 mmol) at room temperature. The reaction mixture was stirred at room temperature for 16h. Progress of the reaction was monitored by TLC. After completion, the reaction mixture was quenched with ice cold water (80 mL), extracted with EtOAc (2 × 100 mL). The organic layer was separated, washed with water (100 mL), brine solution (100 mL), dried over $Na_2SO_4$ and concentrated *in vacuo.* The crude material was purified by combi-flash column chromatography, by eluting with 90% EtOAc in heptane, to afford *tert*-butyl 4-(5-amino-5-oxopentyl) piperidine-1-carboxylate (**Intermediate 194,** 1.40 g, 61%) as a brownish-white solid. $^1$H NMR (400 MHz, DMSO-$d_6$) δ ppm 0.87-0.97(m, 2H), 1.14-1.30 (m, 4H), 1.38 (s, 9H), 1.41-1.50 (m, 2H), 1.53-1.65 (m, 2H), 2.02 (t, *J*=7.34 Hz, 2H), 2.55-2.73 (m, 3H), 3.90-3.91 (m, 2H), 6.66 (br s, 1H), 7.20 (br s, 1H). Mass spec: m/z 285.2 $[M+H]^+$.

**Step 3**

**Intermediate 195: *tert*-Butyl (*R*)-6-(5-(1-(*tert*-butoxycarbonyl) piperidin-4-yl) pentanamido)-2-(1-methylpyrrolidin-2-yl)-1*H*-pyrrolo [3, 2-*c*] pyridine-1-carboxylate**

To a solution of *tert*-butyl 4-(5-amino-5-oxopentyl) piperidine-1-carboxylate **(Intermediate 194,** 0.23 g, 0.84 mmol) and *tert*-butyl (*R*)-6-bromo-2-(1-methylpyrrolidin-2-yl)-1*H*-pyrrolo[3,2-*c*]pyridine-1-carboxylate (**Intermediate 4,** 0.40 g, 1.05 mmol) in 1,4-dioxane (5 mL) was added cesium carbonate (1.03 g, 3.15 mmol). The reaction mixture was purged with argon for 20 min. To the reaction mixture was added $Pd_2(dba)_3$, (0.12 g, 0.13 mmol) followed by 4,5-bis (diphenylphosphino)-9,9 dimethylxanthene (0.18 g, 0.31 mmol) and the reaction mixture was purged with argon for 10 min. The reaction mixture was heated at 130 °C for 5h. Progress of the reaction was monitored by TLC. After completion of the reaction, the reaction mixture was concentrated *in vacuo.* The crude material was purified by combi-flash column chromatography, by eluting with 85% EtOAc in heptane, to afford tert-butyl (R)-6-(5-(1-(tert-butoxycarbonyl) piperidin-4-yl) pentanamido)-2-(1-methylpyrrolidin-2-yl)-1*H*-pyrrolo [3, 2-*c*] pyridine-1-carboxylate (**Intermediate 195,** 0.39 g, 64%) as an off-white solid.

$^1$H NMR (400 MHz, DMSO-$d_6$) δ ppm 0.87-0.96 (m, 2H), 1.18-1.25 (m, 2H), 1.25-1.35 (m, 3H), 1.37 (s, 9H), 1.55-1.63 (m, 4H), 1.66 (s, 9H), 1.67-1.80 (m, 3H), 2.26 (s, 3H), 2.27-2.43 (m, 4H), 2.57-2.70 (m, 2H), 3.05-3.15 (m, 1H), 3.79-3.99 (m, 3H), 6.69 (s, 1H), 8.49 (s, 1H), 8.79 (s, 1H), 10.34 (s, 1H). Mass spec: m/z 583.7 $[M+H]^+$.

**Step 4**

**Intermediate 196: (*R*)-*N*-(2-(1-Methylpyrrolidin-2-yl)-1*H*-pyrrolo [3, 2-*c*] pyridin-6-yl)-5-(piperidin-4-yl) pentanamide dihydrochloride**

To a solution of *tert*-butyl (*R*)-6-(5-(1-(*tert*-butoxycarbonyl) piperidin-4-yl) pentanamido)-2-(1-methylpyrrolidin-2-yl)-1*H*-pyrrolo [3, 2-*c*] pyridine-1-carboxylate (**Intermediate 195,** 0.39 g, 0.66 mmol) in 1,4-dioxane (5 mL) was added 4 M HCl in 1,4-dioxane (2 mL) at room temperature and the reaction mixture was stirred at room temperature for 16h. Progress of the reaction was monitored by TLC. After completion, the reaction mixture was concentrated *in vacuo* and washed with diethyl ether (30 mL). The crude material was triturated with diethyl ether and dried to afford (*R*)-*N*-(2-(1-Methylpyrrolidin-2-yl)-1*H*-pyrrolo [3, 2-*c*] pyridin-6-yl)-5-(piperidin-4-yl) pentanamide dihydrochloride (**Intermediate 196,** 0.36 g) as a white solid. $^1$H NMR (400 MHz, DMSO-$d_6$) δ ppm 1.78-1.80 (m, 2H), 2.11-2.21 (m, 2H), 2.24-2.47 (m, 5H), 2.68 (s, 3H), 2.75-2.82 (m, 2H), 3.16-3.26 (m, 5H), 3.36-3.51 (m, 7H), 4.66-4.69 (m, 1H), 7.13 (s, 1H), 7.30 (s, 1H), 7.89 (s, 1H), 8.44-8.59 (m, 1H), 8.70-8.90 (m, 2H), 11.15 (s, 1H), 12.78 (s, 1H). Mass spec: m/z 383.6 $[M+H]^+$.

**Step 5**

**Example 42: 5-(1-(2-(2,6-Dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-*c*]pyridin-6-yl)pentanamide**

To a solution of (*R*)-*N*-(2-(1-Methylpyrrolidin-2-yl)-1*H*-pyrrolo[3, 2-*c*]pyridin-6-yl)-5-(piperidin-4-yl) pentanamide dihydrochloride (**Intermediate 196**, 0.36 g, 0.93 mmol) in DMSO (3 mL) was added 2-(2,6-dioxopiperidin-3-yl)-4-fluoroisoindoline-1,3-dione (CAS No: 835616-60-9, 0.27 g, 0.93 mmol) followed by *N*,*N*-diisopropylethylamine (0.61 g, 1.22 mmol). The reaction mixture was heated at 110 °C for 6h. Progress of the reaction was monitored by TLC. After completion, the reaction mixture was cooled to room temperature, treated with cold water (50 mL) to get a yellow precipitate, filtered, and then dried. The crude compound was purified by preparative HPLC (Method A) to afford 5-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo [3,2-*c*]pyridin-6-yl)pentanamide (**Example 42, 0.07** g, 12%) as a yellow solid. $^1$H NMR (400 MHz, DMSO-$d_6$) δ ppm 1.27-1.42 (m, 7H), 1.54-1.65 (m, 2H), 1.73-1.91 (m, 5H), 1.98-2.06 (m, 2H), 2.14 (s, 3H), 2.21-2.28 (m, 1H), 2.38 (t, *J*=7.27 Hz, 2H), 2.56-2.64 (m, 1H), 2.80-2.92 (m, 4H), 3.07-3.17 (m, 1H), 3.28 (s, 1H), 3.68-3.70 (m, 2H), 5.08-5.09 (m, 1H), 6.33-6.34 (m, 1H), 7.31-7.32 (m, 2H), 7.66-7.68 (m, 1H), 8.08 (s, 1H), 8.40 (s, 1H), 10.11 (s, 1H), 11.07 (s, 1H), 11.24 (s, 1H). Mass spec: m/z 640.3 [M+H]$^+$.

**Example 43 was synthesised following Scheme 41**

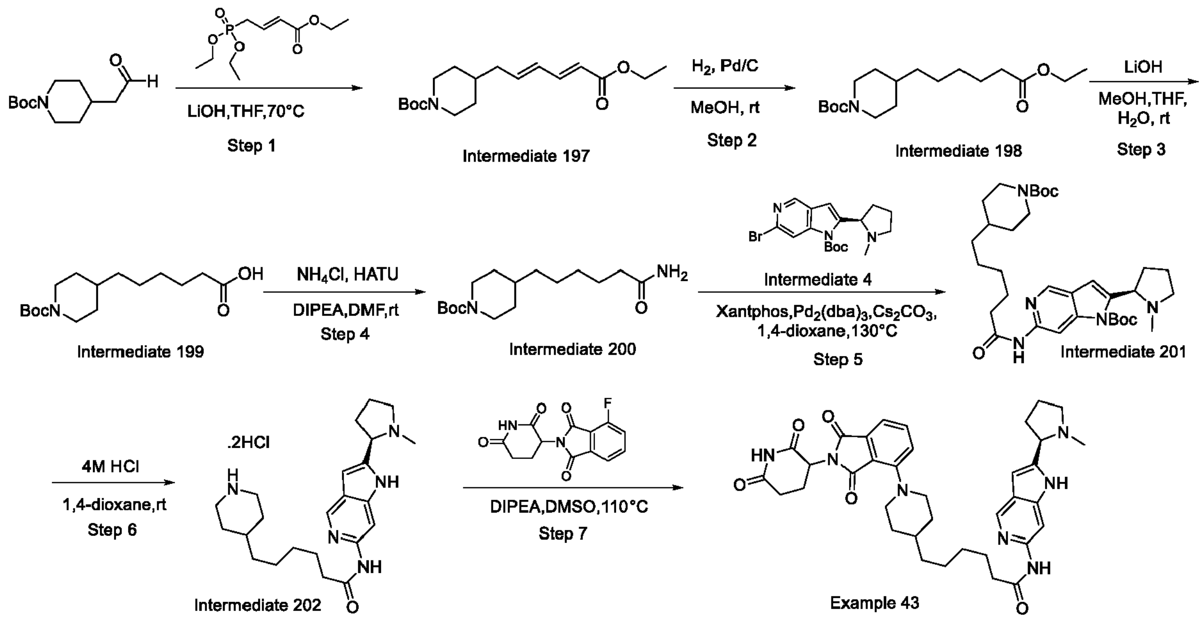


- **Scheme 41**

**Step 1**

**Intermediate 197: *tert*-butyl 4-((2E, 4E)-6-ethoxy-6-oxohexa-2, 4-dien-1-yl) piperidine-1-carboxylate**

To a solution of *tert*-butyl 4-(2-oxoethyl)piperidine-1-carboxylate (CAS No: 142374-19-4, 5.00 g, 21.99 mmol) and triethyl 4-phosphonocrotonate (6.12 g, 21.99 mmol) in tetrahydrofuran (50 mL) was added lithium hydroxide (1.07 g, 43.99 mmol) and the reaction mixture was heated at 70°C for 2h. The reaction mixture was then quenched with water (100 mL) and extracted with EtOAc (3 × 100 mL). The combined organic layers were separated and washed with water (100 mL) then brine solution (100 mL), dried over $Na_2SO_4$ and concentrated *in vacuo.* The crude material was purified by combi-flash column chromatography, by eluting with 90% EtOAc in heptane, to afford *tert*-butyl 4-((2E,4E)-6-ethoxy-6-oxohexa-2,4-dien-1-yl)piperidine-1-carboxylate (**Intermediate 197,** 3.80 g, 53%) as a colourless liquid. $^1$H NMR (400 MHz, DMSO-$d_6$) δ ppm 0.94-1.04 (m, 2H), 1.21 (t, *J*=7.02 Hz, 3H), 1.38 (s, 9H), 1.53-1.61 (m, 2H), 2.09 (t, *J*=5.48 Hz, 2H), 2.63-2.69 (m, 2H), 3.65-3.67 (m, 1H), 3.89-3.92 (m, 2H), 4.09-4.14 (m, 2H), 5.85-5.91 (m, 1H), 6.22-6.28 (m, 2H), 7.19-7.26 (m, 1H). Mass spec: m/z 267.6 [M+H-t-butyl]$^+$.

**Step 2**

**Intermediate 198: *tert*-butyl 4-(6-ethoxy-6-oxohexyl) piperidine-1-carboxylate**

To a solution of *tert*-butyl 4-((2E,4E)-6-ethoxy-6-oxohexa-2,4-dien-1-yl) piperidine-1-carboxylate (**Intermediate**

**197**, 3.80 g, 12 mmol) in methanol (60 mL) was added Pd/C (10% by wt., 1.20 g, 11 mmol) and the reaction mixture stirred at room temperature under 70 psi hydrogen pressure for 16h. The reaction mixture was then filtered through a celite bed and washing with methanol. The solvent was evaporated under reduced pressure to afford *tert*-butyl 4-(6-ethoxy-6-oxohexyl) piperidine-1-carboxylate **(Intermediate 198,** 3.40 g) as colourless oil which was used for the next step without further purification. $^1$H NMR (400 MHz, DMSO-$d_6$) δ ppm 0.90-0.95 (m, 3H), 1.15-1.21 (m, 3H), 1.23-1.29 (m, 4H), 1.39 (s, 9H), 1.48-1.55 (m, 2H), 1.59-1.62 (m, 2H), 2.26-2.29 (m, 2H), 2.63-2.66 (m, 2H), 3.56-3.59 (m, 2H), 3.89-3.92 (m, 2H), 4.02-4.07(m, 2H).

**Step 3**

**Intermediate 199: 6-(1-(*tert*-butoxycarbonyl) piperidin-4-yl) hexanoic acid**

To a solution of *tert*-butyl 4-(6-ethoxy-6-oxohexyl) piperidine-1-carboxylate (**Intermediate 198**, 3.40 g, 10 mmol) in MeOH (10 mL) and THF(10 mL) was added lithium hydroxide (0.50 g, 21 mmol) in $H_2O$ (10 mL). and the reaction mixture stirred at room temperature for 6h. The reaction mixture was then diluted with water (60 mL), acidified to pH~5 using aqueous 1N HCl and extracted with EtOAc (2 × 100 mL). The combined organic layer was separated, dried over $Na_2SO_4$ and concentrated *in vacuo* to afford 6-(1-(*tert*-butoxycarbonyl) piperidin-4-yl) hexanoic acid (**Intermediate 199,** 3.05 g) as a liquid which was used for the next step without further purification. $^1$H NMR (400 MHz, DMSO-$d_6$) δ ppm 0.90-0.94 (m, 2H), 1.16-1.25 (m, 6H), 1.32-1.34 (m, 1H), 1.38 (s, 9H), 1.45-1.54 (m, 2H), 1.59-1.62 (m, 2H), 2.18 (t, *J*=7.30 Hz, 2H), 2.63-2.65 (m, 2H), 3.89-3.92 (m, 2H), 11.95 (br s, 1H).Mass spec: m/z 300.3 $[M+H]^+$.

**Step 4**

**Intermediate 200: *tert*-butyl 4-(6-amino-6-oxohexyl) piperidine-1-carboxylate**

To a solution of 6-(1-(*tert*-butoxycarbonyl)piperidin-4-yl)hexanoic acid (**Intermediate 199**, 3.20 g, 10 mmol) in DMF (25 mL) was added HATU (6.0 g, 15 mmol) and diisopropylethylamine (5.3 mL, 30 mmol) and the reaction mixture was stirred at room temperature for 15 min. Ammonium chloride (2.2 g, 41 mmol) was then added and the reaction stirred at room temperature and stirred for 16h. The reaction mixture was then quenched with water (200 mL), extracted with EtOAc (3 × 100 mL). The combined organic layers were then washed with $H_2O$ (200 mL), brine solution (200 mL), dried over $Na_2SO_4$ and concentrated *in vacuo.* The crude material was purified by combi-flash column chromatography, by eluting with 95% EtOAc in heptane, to afford *tert*-butyl 4-(6-amino-6-oxohexyl) piperidine-1-carboxylate (**Intermediate 200, 2.50** g, 83%) as an off white solid. $^1$H NMR (400 MHz, DMSO-$d_6$) δ ppm 0.86-0.97 (m, 2H), 1.15-1.23 (m, 4H), 1.38 (s, 9H), 1.43-1.50 (m, 2H), 1.59-1.62 (m, 2H), 1.99-2.03 (m, 3H), 2.65-2.73 (m, 3H), 3.88-3.92 (m, 2H), 4.00-4.05 (m, 1H), 6.65 (br s, 1H) 7.19 (br s, 1H). Mass spec: m/z 299.3 $[M+H]^+$.

**Step 5**

**Intermediate 201: *tert*-butyl (R)-6-(6-(1-(*tert*-butoxycarbonyl)piperidin-4-yl)hexanamido)-2-(1-methylpyrrolidin-2-yl)-1H-pyrrolo[3,2-c]pyridine-1-carboxylate**

To a solution of *tert*-butyl 4-(6-amino-6-oxohexyl)piperidine-1-carboxylate **(Intermediate 200, 0.20** g, 0.67 mmol) and *tert*-butyl (R)-6-bromo-2-(1-methylpyrrolidin-2-yl)-1*H*-pyrrolo[3,2-c]pyridine-1-carboxylate (**Intermediate 4,** 0.25 g, 0.67 mmol) in 1,4-dioxane (5 mL) was added cesium carbonate (0.66 g, 2.01 mmol). The reaction mixture was purged with argon for 20 min then $Pd_2(dba)_3$ (0.08 g., 0.087 mmol) and Xantphos (0.12 g, 0.20 mmol) were added the reaction was purged further with argon for 10 min before heating at 130°C for 2h. The reaction mixture was then concentrated *in vacuo* and the crude material was purified by combi-flash column chromatography, by eluting with 85% EtOAc in heptane, to afford *tert*-butyl (R)-6-(6-(1-(*tert*-butoxycarbonyl)piperidin-4-yl)hexanamido)-2-(1-methylpyrrolidin-2-yl)-1H-pyrrolo[3,2-c]pyridine-1-carboxylate (**Intermediate 201**, 0.31 g, 93%) as an off white solid. $^1$H NMR (400 MHz, DMSO-$d_6$) δ ppm 0.90-0.94 (m, 2H), 1.17-1.21 (m, 2H), 1.25-1.29 (m, 5H), 1.37 (s, 9H), 1.58-1.66 (m, 6H), 1.70 (s, 9H), 1.73-1.75 (m, 2H), 2.31-2.39 (m, 5H), 2.69(s, 3H)., 3.08-3.15 (m, 1H), 3.87-3.90 (m, 3H), 6.69 (br s, 1H), 8.49 (s, 1H), 8.79 (s, 1H), 10.33 (s, 1H). Mass spec: m/z 598.3 $[M+H]^+$.

**Step 6**

**Intermediate 202: (R)-N-(2-(1-methylpyrrolidin-2-yl)-1*H*-pyrrolo[3,2-c]pyridin-6-yl)-6-(piperidin-4-yl) hexanamide dihydrochloride**

To a solution of *tert*-butyl (R)-6-(6-(1-(*tert*-butoxycarbonyl)piperidin-4-yl)hexanamido)-2-(1-methylpyrrolidin-2-yl)-1H-pyrrolo[3,2-c]pyridine-1-carboxylate (**Intermediate 201**, 0.31 g, 0.63 mmol) in 1,4-dioxane (5 mL) was added 4M HCl in 1,4-dioxane (6 mL) at 0°C and the reaction mixture was stirred at room temperature for 16h. The reaction mixture was then concentrated *in vacuo* to obtain crude material and purified by triturating with diethyl ether and dried to afford (R)-*N*-(2-(1-methylpyrrolidin-2-yl)-1*H*-pyrrolo[3,2-c]pyridin-6-yl)-6-(piperidin-4-yl)hexanamide dihydrochloride (**Intermediate 202,** 0.28 g) as an off-white solid which was used for the next step without further purification. $^1$H NMR (400 MHz, DMSO-$d_6$) δ ppm 1.17-1.37 (m, 8H), 1.62-1.78 (m, 4H), 2.31-2.47 (m, 4H) 2.70 (s, 3H), 2.73-2.89 (m, 5H), 3.18-3.28 (m, 2H) 3.56-3.58 (m, 1H), 3.60-3.78 (m, 2H), 4.62-4.63 (m,1H), 6.96 (br s, 1H), 7.32 (s, 2H), 8.01 (s, 1H), 8.28 (s, 1H), 8.54 (br s, 1H), 8.73 (br s, 1H), 10.59 (s, 1H). Mass spec: m/z 398.2 [M-H] $^+$.

**Step 7**

**Example 43: 6-(1-(2-(2,6-dioxopiperidin-3-yl)-1,3-dioxoisoindolin-4-yl)piperidin-4-yl)-*N*-(2-((R)-1-methylpyrrolidin-2-yl)-1*H*-pyrrolo[3,2-c]pyridin-6-yl)hexanamide**

To a solution of (R)-*N*-(2-(1-methylpyrrolidin-2-yl)-1*H*-pyrrolo[3,2-c]pyridin-6-yl)-6-(piperidin-4-yl)hexanamide dihydrochloride (**Intermediate 202**, 0.28 g, 0.70 mmol) in DMSO (5 mL) was added 2-(2,6-dioxopiperidin-3-yl)-4-fluoroisoindoline-1,3-dione (CAS No: 835616-60-9, 0.20 g, 0.70 mmol) followed by *N*,*N*-diisopropylethylamine (0.46 g, 3.52 mmol) and the reaction mixture was heated at 110°C for 6h. The reaction mixture was cooled to room temperature, and cold water (50 mL) was added to obtain yellow precipitation, which was filtered and dried under vacuum. The crude compound was purified by preparative HPLC (Method A) to afford 6-(1-(2-(2,6-dioxopiperidin-3-yl)-1,3-dioxoisoindolin-4-yl)piperidin-4-yl)-*N*-(2-((R)-1-methylpyrrolidin-2-yl)-1*H*-pyrrolo[3,2-c]pyridin-6-yl)hexanamide (**Example 43**, 0.06 g, 14%) as a yellow solid. $^1$H NMR (400 MHz, DMSO-$d_6$) δ ppm 1.28-1.34 (m, 9H), 1.60-1.63 (m, 2H), 1.75-1.89 (m, 5H), 2.02-2.05 (m, 1H), 2.08-2.13 (m, 1H), 2.14 (s, 3H), 2.22-2.28 (m, 1H), 2.32-2.39 (m, 2H), 2.54-2.63 (m, 2H), 2.77-2.91 (m, 3H), 3.10-3.16 (m, 1H), 3.26-3.29 (m, 1H), 3.65-3.69 (m, 2H), 5.06-5.11 (m, 1H), 6.34 (s, 1H), 7.31-7.32 (m, 2H), 7.64-7.69 (m, 1H), 8.09 (s, 1H), 8.41 (s, 1H), 10.11 (br s, 1H), 11.07 (br s, 1H), 11.25 (br s, 1H). Mass spec: m/z 654.3 [M+H]$^+$.

**Example 44 was synthesised following Scheme 42**

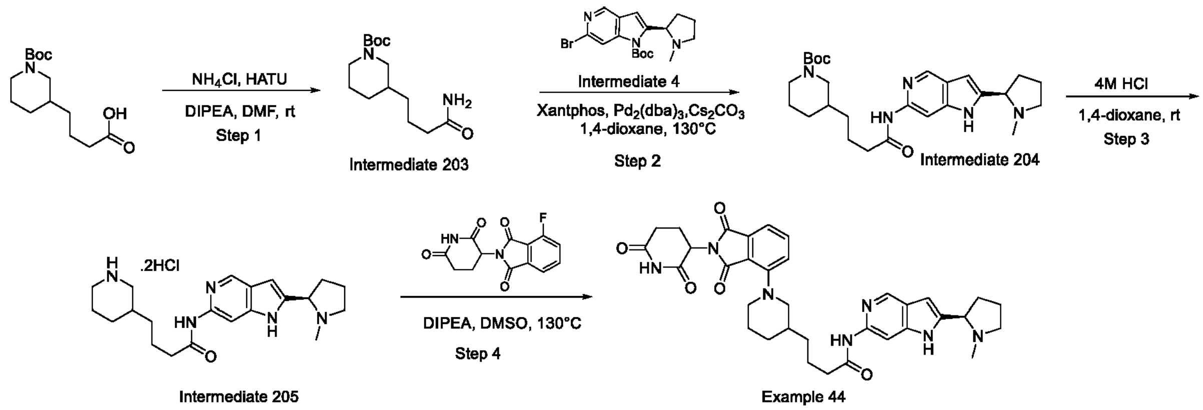


**Scheme 42**

**Step 1**

**Intermediate 203: *tert*-butyl 3-(4-amino-4-oxobutyl) piperidine-1-carboxylate**

To a solution of 4-(1(*tert*-butoxycarbonyl)piperidin-3-yl)butanoic acid (CAS No: 318536-95-7, 2 g, 7.369 mmol) in DMF (30 mL) was added HATU (4.33 g, 11.05 mmol). The resulting reaction mixture was stirred at 15 min then added ammonium chloride (1.97 g, 36.85 mmol) and N, N-diisopropylethylamine (3.89 g, 29.48 mmol) at room temperature. The

reaction mixture was stirred at room temperature for 2h. The reaction mixture was diluted with water (200 mL) and extracted with EtOAc (2 × 200 mL). The organic layer was dried over $Na_2SO_4$ and concentrated to give crude material which was purified by combi-flash chromatography, by eluting with 45% EtOAc in heptane, to afford *tert*-butyl 3-(4-amino-4-oxobutyl) piperidine-1-carboxylate (**Intermediate 203**, 1.20 g, 60%) as a yellow solid. $^1$H NMR (400 MHz, DMSO-$d_6$) δ ppm 1.00 - 1.19 (m, 3H) 1.39 (s, 9H) 1.45 - 1.59 (m, 3H) 1.65-1.75 (m, 1H) 2.00-2.03 (m, 2H) 2.23 - 2.42 (m, 1H) 2.53 - 2.65 (m, 2H) 2.73-2.79 (m, 1H) 3.56-3.59 (m, 2H), 6.67 (s, 1H) 7.21 (s, 1H). Mass spec: m/z 270.8 $[M+H]^+$.

**Step 2**

**Intermediate 204: *tert*-butyl 3-(4-((2-((R)-1-methylpyrrolidin-2-yl)-1H-pyrrolo[3, 2-c]pyridin-6-yl) amino)-4-oxobutyl)piperidine-1-carboxylate**

To a solution of *tert*-butyl 3-(4-amino-4-oxo-butyl)piperidine-1-carboxylate (**Intermediate 203,** 0.227 g, 0.84 mmol) and *tert*-butyl (R)-6-bromo-2-(1-methylpyrrolidin-2-yl)-1*H*-pyrrolo[3,2-c]pyridine-1-carboxylate (**Intermediate 4**, 0.40 g, 1.05 mmol) and Cesium carbonate (1.03 g, 3.15 mmol) in 1,4-dioxane (15 mL) was added. The reaction mixture was degassed with argon for 10 min, then $Pd_2(dba)_3$ (0.148 g, 0.15 mmol) and Xantphos (0.188 g, 0.31mmol) was added. The resulting reaction mixture was heated at 130°C for 2h. The reaction mixture was then concentrated *in vacuo* to obtain crude compound, which was purified by combi-flash column chromatography, by eluting with 50% EtOAC in heptane to afford tert-butyl 3-(4-((2-((R)-1-methylpyrrolidin-2-yl)-1H-pyrrolo[3,2-c]pyridin-6-yl) amino)-4-oxobutyl) piperidine-1-carboxylate (**Intermediate 204**, 0.22 g, 44%) as an off white solid. $^1$H NMR (400 MHz, DMSO-$d_6$) δ ppm 1.12 - 1.27 (m, 2H), 1.38 (s, 9H), 1.51 - 1.68 (m, 4H), 1.69 - 1.96 (m, 4H), 2.09- 2.12 (m, 2H) 2.14 (s, 3H) 2.19 - 2.30 (m, 2H), 2.35 (t, *J*=7.13 Hz, 2H), 2.70-2.77 (m, 1H), 3.10-3.14 (m, 1H), 3.26-3.30 (m, 3H) 3.64 - 3.81 (m, 1H), 6.33 (s, 1H), 8.07 (s, 1H), 8.40 (s, 1H), 10.11 (br,s, 1H), 11.24 (s, 1H). Mass spec: m/z 470.0 $[M+H]^+$.

**Step 3**

**Intermediate 205: N-(2-((R)-1-methylpyrrolidin-2-yl)-1H-pyrrolo[3,2-c]pyridin-6-yl)-4-(piperidin-3-yl) butanamide dihydrochloride**

To a solution of *tert*-butyl 3-(4-((2-((R)-1-methylpyrrolidin-2-yl)-1H-pyrrolo[3,2-c]pyridin-6-yl) amino)-4-oxobutyl) piperidine-1-carboxylate (**Intermediate 204**, 0.320 g, 0.32 mmol) in 1,4-dioxane (5.0 mL) was added 4M HCl in 1,4-dioxane (5.0 mL) at 0°C. The resulting reaction mixture was stirred at room temperature for 16h. The progress of the reaction was monitored by TLC. After completion, the reaction mixture was concentrated *in vacuo* to afford N-(2-((R)-1-methylpyrrolidin-2-yl)-1H-pyrrolo[3,2-c]pyridin-6-yl)-4-(piperidin-3-yl) butanamide dihydrochloride (**Intermediate 205**, 0.30 g) as an off-white solid used for next step without further purification.

**Step 4**

**Example 44: 4-(1-(2-(2,6-dioxopiperidin-3-yl)-1,3-dioxoisoindolin-4-yl)piperidin-3-yl)-N-(2-((R)-1-methylpyrrolidin-2-yl)-1H-pyrrolo[3,2-c]pyridin-6-yl)butanamide**

To a solution of N-(2-((R)-1-methylpyrrolidin-2-yl)-1H-pyrrolo[3,2-c]pyridin-6-yl)-4-(piperidin-3-yl) butanamide dihydrochloride (**Intermediate 205**, 0.30 g, 0.81 mmol) and 2-(2,6-dioxo-3 piperidyl)-4-fluoro-isoindoline-1,3-dione (CAS No: 835616-60-9, 0.22 g, 0.81 mmol) in DMSO (1.0 mL) was added DIPEA (0.53 g, 3.0 mmol). The resulting reaction mixture was heated at 130°C for 2h. The reaction mixture was then poured into ice cold water, a solid precipitated which was filtered and dried to obtain crude compound. The crude compound was purified by preparative HPLC (Method A) to afford 4-(1-(2-(2,6-dioxopiperidin-3-yl)-1,3-dioxoisoindolin-4-yl)piperidin-3-yl)-N-(2-((R)-1-methylpyrrolidin-2-yl)-1H-pyrrolo[3,2-c]pyridin-6-yl)butanamide (**Example 44**, 0.11 g, 22%) as a yellow solid. $^1$H NMR (400 MHz, DMSO-$d_6$) δ ppm 1.06-1.07 (m, 1H), 1.17-1.34 (m, 2H), 1.58-1.94 (m, 10H), 1.97-2.06 (m, 1H), 2.07-2.16 (m, 1H), 2.18 (s, 3H), 2.21-2.26 (m, 1H), 2.32 - 2.40 (m, 2H), 2.49-2.51 (m, 1H), 2.76-2.91 (m, 2H), 3.10-3.15 (m, 1H), 3.28-3.30 (m, 2H), 3.61-3.70 (m, 2H), 5.09-5.11 (m, 1H), 6.33 (s, 1H), 7.29-7.35 (m, 2H), 7.66 (t, *J*=7.70 Hz, 1H), 8.07 (s, 1H), 8.39 (s, 1H), 10.10 (s, 1H), 11.07 (s, 1H), 11.24 (s, 1H). Mass spec: m/z 626.3 $[M+H]^+$.

**Example 45 was synthesised following Scheme 43**

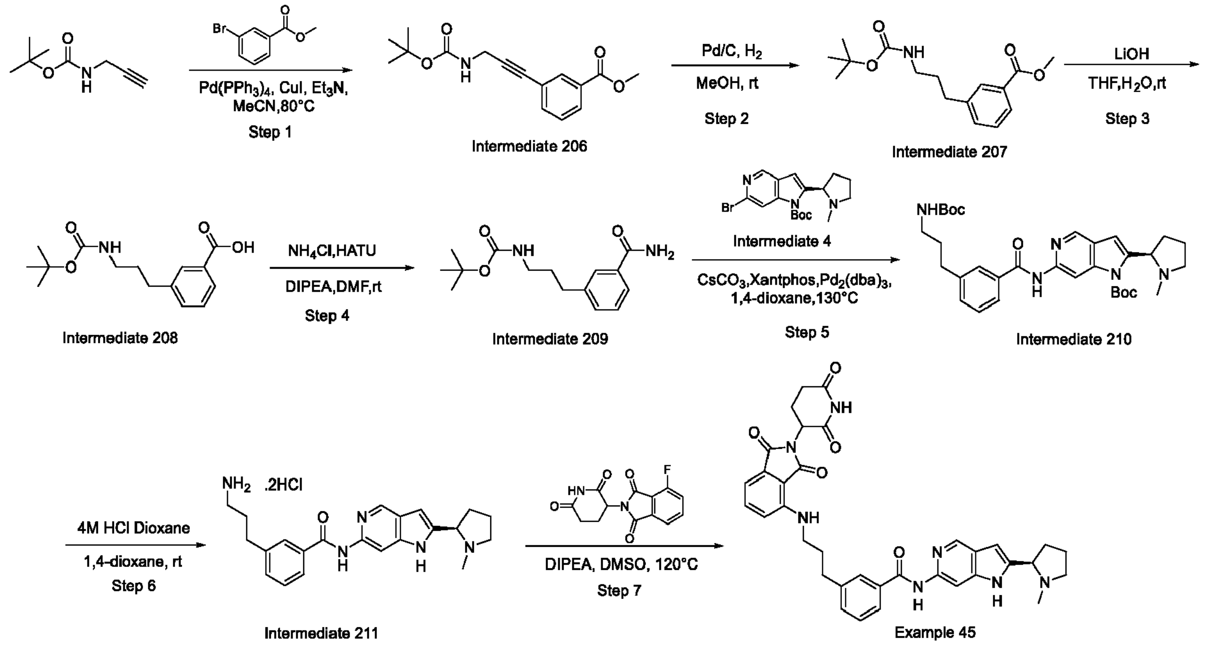


**Scheme 43**

**Step 1**

**Intermediate 206: methyl 3-(3-((*tert*-butoxycarbonyl)amino) prop-1-yn-1-yl)benzoate**

To a solution of methyl 3-bromobenzoate (CAS No: 618-89-3, 4.00 g, 18.60 mmol) and *tert*-butyl prop-2-yn-1-ylcarbamate (CAS No: 92136-39-5, 5.76 g, 37.16 mmol) in acetonitrile (40 mL) was added $Pd(PPh_3)_4$ (2.20 g, 1.90 mmol) followed by copper(I) iodide (0.71 g, 3.67 mmol) and triethylamine (9 mL, 64.2 mmol) at room temperature. The reaction mixture was degassed with argon gas for 10 min then heated at 80°C for 16h. Progress of the reaction was monitored by TLC. After completion, the reaction mixture was extracted with EtOAc (200 mL). The organic layer was separated, dried over anhydrous $Na_2SO_4$ and concentrated *in vacuo.* The crude material was purified by combi-flash column chromatography, by eluting with 20% EtOAc in heptane, to afford methyl 3-(3-((*tert*-butoxycarbonyl)amino)prop-1-yn-1-yl) benzoate (**Intermediate 206,** 2.0 g, 37%) as a yellow solid. $^1$H NMR (400 MHz, DMSO-$d_6$) δ ppm 1.40 (s, 9H), 3.86 (s, 3H), 3.99 (d, *J*=5.38 Hz, 2H), 7.37 (br s, 1H), 7.51-7.55 (m, 1H), 7.66-7.68 (m, 1H), 7.91-7.93 (m, 1H), 7.94 (s, 1H).

**Step 2**

**Intermediate 207: methyl 3-(3-((*tert*-butoxycarbonyl)amino)propyl)benzoate**

To a solution of methyl 3-(3-((*tert*-butoxycarbonyl)amino)prop-1-yn-1-yl)benzoate (**Intermediate 206,** 2.00 g, 6.91 mmol) in methanol (20 mL) was added 300 mg of 10% by wt. Pd/C at room temperature and the reaction mixture was stirred for 24h under $H_2$ atmosphere at 150 psi. Progress of the reaction was monitored by TLC. After completion, the reaction mixture was passed through celite bed and concentrated *in vacuo* to afford methyl 3-(3-((*tert*-butoxycarbonyl) amino)propyl)benzoate (**Intermediate 207,** 1.0 g) as an off-white solid which was used for the next step without further purification. $^1$H NMR (400 MHz, DMSO-$d_6$) δ ppm 1.37 (s, 9H), 1.65-1.69 (m, 2H), 2.61-2.65 (m, 2H), 2.91-2.93 (m, 2H), 3.84 (s, 3H), 6.87 (br s, 1H), 7.42-7.48 (m, 2H), 7.76-7.79 (m, 2H). Mass spec: m/z 193.8 [M+H-100] $^+$.

**Step 3**

**Intermediate 208: 3-(3-((*tert*-butoxycarbonyl)amino)propyl)benzoic acid**

To a solution of methyl 3-(3-((*tert*-butoxycarbonyl)amino)propyl)benzoate (**Intermediate 207,** 0.35 g, 1.19 mmol) in tetrahydrofuran (2 mL), methanol (20 mL) was added lithium hydroxide (0.075 g, 2.38 mmol) in water (2 mL) at room temperature. The reaction mixture was stirred at room temperature for 16h. Progress of the reaction was monitored by TLC. After completion, the reaction mixture was concentrated *in vacuo.* The reaction mixture was diluted with water (25

mL), acidified with 1N HCl to pH~4. The yellow solid precipitated, which was filtered and dried to obtain 3-(3-((*tert*-butoxycarbonyl)amino)propyl)benzoic acid (**Intermediate 208,** 0.21 g, 63%) as yellow solid which was used as such for the next step without further purification. $^1$H NMR (400 MHz, DMSO-$d_6$) δ ppm 1.37 (s, 9H), 1.66-1.70 (m, 2H), 2.60-2.66 (m, 2H), 2.90-2.93 (m, 2H), 6.85 (br s, 1H), 7.37-7.46 (m, 2H), 7.74-7.80 (m, 2H), 12.86 (br s, 1H).

**Step 4**

**Intermediate 209: *tert*-butyl (3-(3-carbamoylphenyl)propyl)carbamate**

To a solution of 3-(3-((*tert*-butoxycarbonyl)amino)propyl)benzoic acid (**Intermediate 208, 0.20** g, 0.72 mmol) in DMF (3 mL) was added HATU (0.42 g, 1.07 mmol) and diisopropylethylamine (0.27 g, 2.14 mmol) at RT. The reaction mixture was stirred at room temperature for 15 min then added ammonium chloride (0.15 g, 2.86 mmol). Reaction mixture was stirred at RT for 16h. Progress of the reaction was monitored by TLC. After completion, the reaction mixture was diluted with water (100 mL) and extracted with EtOAc (3 × 50 mL). The organic layer was washed with $NaHCO_3$ (2 × 40 mL) and brine solution (2 × 30 mL). The organic layer was separated, dried over $Na_2SO_4$ and concentrated *in vacuo* to obtain crude compound. The crude material was purified by combi-flash column chromatography by eluting with 15% EtOAc in heptane to afford tert-butyl (3-(3-carbamoylphenyl)propyl)carbamate (**Intermediate 209**, 0.15 g, 75%) as a colorless liquid. $^1$H NMR (400 MHz, DMSO-$d_6$) δ ppm 1.26 (s, 9H), 2.68-2.73 (m, 2H), 3.12-3.15 (m, 2H), 3.59-3.64 (m, 2H) 6.86 (br s, 1H), 7.19-7.34 (m, 2H), 7.67-7.70 (m, 1H), 7.90-7.95 (m, 1H), 8.18 (br s, 2H).

**Step 5**

**Intermediate 210: *tert*-butyl (R)-6-(3-(3-((*tert*-butoxycarbonyl)amino)propyl)benzamido)-2-(1-methylpyrrolidin-2-yl)-1*H*-pyrrolo [3, 2-c] pyridine-1-carboxylate**

To a solution of tert-butyl (3-(3-carbamoylphenyl)propyl)carbamate (**Intermediate 209**, 0.30 g, 1.07 mmol) in 1, 4-dioxane (5 mL) was added *tert*-butyl (R)-6-bromo-2-(1-methylpyrrolidin-2-yl)-1*H*-pyrrolo[3,2-c]pyridine-1-carboxylate (**Intermediate 4**, 0.14 g, 1.29 mmol) followed by cesium carbonate (1.06 g, 3.23 mmol). The reaction mixture was purged with argon for 20 min. To this reaction mixture $Pd_2(dba)_3$ (0.10 g, 0.11 mmol) and xantphos (0.31 g, 0.32 mmol) were added at room temperature and purged with argon for 10 min. The reaction mixture was heated at 130°C for 1h. Progress of the reaction was monitored by TLC. After completion, the reaction mixture diluted with water (50 mL) and extracted with EtOAc (3 × 50 mL). The organic layer was separated, dried over $Na_2SO_4$ and concentrated *in vacuo* to obtain crude compound. The crude material was purified by combi-flash column chromatography by eluting with 70% EtOAc in heptane to afford *tert*-butyl (R)-6-(3-(3-((*tert*-butoxycarbonyl)amino)propyl)benzamido)-2-(1-methylpyrrolidin-2-yl)-1*H*-pyrrolo[3,2-c]pyridine-1-carboxylate (**Intermediate 210,** 0.30 g, 51%) as an off-white solid. $^1$H NMR (400 MHz, DMSO-$d_6$) δ ppm 1.38 (s, 9H), 1.69 (s, 9H), 1.71-1.79 (m, 5H), 2.35(s, 3H), 2.36-2.39 (m, 2H), 2.64 (t, *J*=7.45 Hz, 2H), 2.93-2.96 (m, 2H), 3.10-3.12 (m, 1H), 3.89-3.95 (m, 1H), 6.75 (s, 1H), 6.87 (s, 1H), 7.39-7.41 (m, 2H), 7.84-7.89(m, 2H), 8.59 (s, 1H), 8.92 (s, 1H), 10.63 (s, 1H). Mass spec: m/z 576.2 $[M-H]^-$.

**Step 6**

**Intermediate 211: (R)-3-(3-aminopropyl)-*N*-(2-(1-methylpyrrolidin-2-yl)-1*H*-pyrrolo [3,2-c] pyridin-6-yl) benzamine dihydrochloride**

To a solution of tert-butyl (R)-6-(3-(3-((*tert*-butoxycarbonyl)amino)propyl)benzamido)-2-(1-methylpyrrolidin-2-yl)-1*H*-pyrrolo[3,2-c]pyridine-1-carboxylate (**Intermediate 210,** 0.30 g, 0.546 mmol) in 1,4-dioxane (2.5 mL) was added 4M HCl in 1,4-dioxane (5 mL) at 0°C. The reaction mixture was stirred at room temperature for 16h. Progress of the reaction was monitored by TLC. After completion, the reaction mixture was concentrated *in vacuo,* washed with DCM (2 × 20 mL) and dried. The crude material was washed with diethyl ether (2 × 20 mL) and dried to afford (R)-3-(3-aminopropyl)-*N*-(2-(1-methylpyrrolidin-2-yl)-1*H*-pyrrolo[3,2-c]pyridin-6-yl)benzamide dihydrochloride (**Intermediate 211,** 0.22 g crude) as an off white solid which was used for next step without further purification. $^1$H NMR (400 MHz, DMSO-$d_6$) δ ppm 1.93-2.00 (m, 2H), 2.15-2.22 (m, 2H), 2.37-2.45 (m, 2H), 2.53-2.56 (m, 1H), 2.73 (s, 3H), 2.75-2.84 (m, 3H), 3.16-3.25 (m, 1H), 3.73-3.75 (m, 1H), 4.72-4.74 (m, 1H), 7.26 (s, 1H), 7.53-7.57 (m, 2H), 8.02 (s, 3H),8.10 (s, 1H), 8.31 (s, 1H), 9.08 (s, 1H), 11.47 (br s, 1H), 11.98 (br s, 1H), 13.21 (br s, 1H).

**Step 7**

**Example 45: 3-(3-((2-(2,6-dioxopiperidin-3-yl)-1,3-dioxoisoindolin-4-yl)amino)propyl)-*N*-(2-((R)-1-methylpyrrolidin-2-yl)-1*H*-pyrrolo[3,2-c]pyridin-6-yl)benzamide**

To a solution of (R)-3-(3-aminopropyl)-*N*-(2-(1-methylpyrrolidin-2-yl)-1*H*-pyrrolo[3,2-c]pyridin-6-yl)benzamide dihydrochhloride (**Intermediate 211**, 0.24 g, 0.63 mmol) in DMSO (5 mL) was added 2-(2,6-dioxo-3 piperidyl)-4-fluoro-isoindoline-1,3-dione (CAS No: 835616-60-9, 0.17 g, 0.63 mmol) followed by diisopropylethylamine (0.33 mL 1.91 mmol) at room temperature. The reaction mixture was purged with argon for 15 min. The reaction mixture was heated at 100°C for 16h. Progress of the reaction was monitored by TLC. After completion, the reaction mixture was cooled to room temperature, treated with cold water (50 mL), filtered, dried, and washed with DCM/pentane (2 × 20 mL) and then dried. The crude material was purified by preparative HPLC (Method A) to afford 3-(3-((2-(2,6-dioxopiperidin-3-yl)-1,3-dioxoisoindolin-4-yl)amino)propyl)-*N*-(2-((R)-1-methylpyrrolidin-2-yl)-1*H*-pyrrolo[3,2-c]pyridin-6-yl)benzamide (**Example 45**, 0.8 g, 19.9%) as a yellow solid. $^1$H NMR (400 MHz, DMSO-$d_6$) δ ppm 1.86-2.16 (m, 7H), 2.16-2.24 (m, 1H), 2.25 (s, 3H), 2.47-2.49 (m, 1H), 2.53-2.60 (m, 2H), 2.74-2.78 (m, 2H), 2.83-2.92 (m, 1H), 3.12-3.16 (m, 1H), 3.34-3.39 (m, 2H), 5.03-5.07 (m, 1H), 6.39 (s, 1H), 6.64 (t, *J*=5.87 Hz, 1H), 7.02 (d, *J*=6.85 Hz, 1H), 7.09 (d, *J*=8.56 Hz, 1H), 7.38-7.44 (m, 2H), 7.56-7.60 (m, 1H), 7.83-7.86 (m, 1H), 7.94 (s, 1H), 8.19 (s, 1H), 8.49-8.50 (m, 1H), 10.42 (br s, 1H), 11.37 (br s, 1H). Mass spec: m/z 634.4 [M+H] $^+$.

**Example 46 was synthesised following Scheme 44**

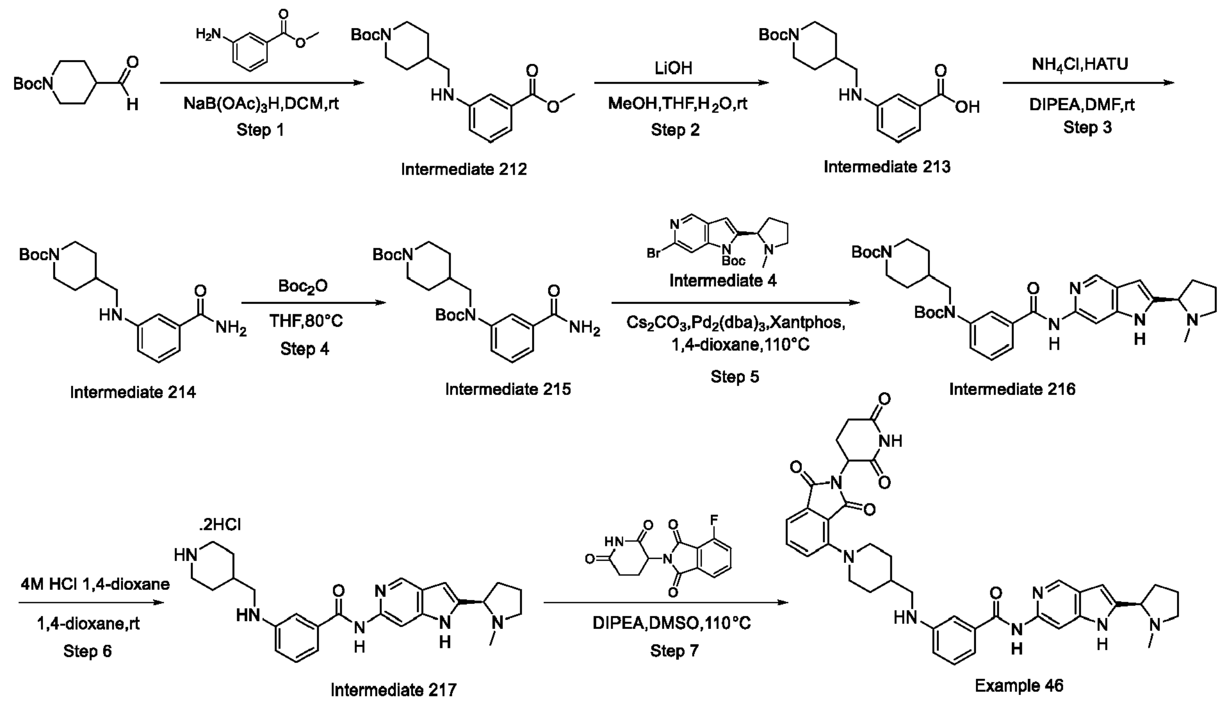


**Scheme 44**

**Step** 1

**Intermediate 212: *tert*-butyl 4-(((3-(methoxycarbonyl)phenyl)amino)methyl)piperidine-1-carboxylate**

To a solution of *tert*-butyl 4-formylpiperidine-1-carboxylate (CAS No: 137076-22-3, 5.0 g, 33.07 mmol) in dichloromethane (100 mL) was added methyl 3-aminobenzoate (CAS No: 4518-10-9, 7.75 g, 36.38 mmol). The reaction mixture was stirred at room temperature for 2h and then added sodium triacetoxyborohydride (21.68 g, 99.23 mmol) at room temperature and the reaction mixture was stirred at rt for 16h. Progress of the reaction was monitored by TLC. After completion, the reaction mixture was diluted with water (100 mL) and extracted with DCM (2 × 200 mL). The organic layer was seprated, dried over anhydrous $Na_2SO_4$ and concentrated *in vacuo.* The crude material was purified by combi-flash column chromatography by eluting with 60% EtOAc in heptane to afford *tert*-butyl 4-(((3-(methoxycarbonyl)phenyl)amino) methyl)piperidine-1-carboxylate (**Intermediate 212,** 10.0 g, 87%) as a yellow solid. $^1$H NMR (400 MHz, DMSO-$d_6$) δ ppm

1.01-1.09 (m, 2H), 1.39 (s, 9H), 1.71-1.74 (m, 3H), 2.64-2.67 (m, 2H), 2.91-2.94 (m, 2H), 3.80 (s, 3H), 3.93-3.96 (m, 2H), 5.99 (br s, 1H), 6.81 (d, *J*=7.89 Hz, 1H), 7.09 (d, *J*=7.20 Hz, 1H), 7.13-7.15 (m, 1H), 7.18 (s, 1H). Mass spec: m/z 347.1 $[M+H]^+$.

**Step 2**

**Intermediate 213: 3-(((1-(*tert*-butoxycarbonyl)piperidin-4-yl)methyl)amino)benzoic acid**

To a solution of *tert*-butyl 4-(((3-(methoxycarbonyl)phenyl)amino)methyl)piperidine-1-carboxylate (**Intermediate 212**, 3.0 g, 8.61 mmol) in tetrahydrofuran (5 mL) and methanol (5 mL) was added lithium hydroxide (0.631 g, 25.8 mmol) in water (10 mL) at 0°C. The reaction mixture was stirred at room temperature for 6h. Progress of the reaction was monitored by TLC. After completion, the reaction mixture was concentrated *in vacuo.* The reaction was diluted with water (20 mL) and the pH of solution was adjusted to 3-4 with 0.5 N HCl and extracted with EtOAc (2 × 100 mL). The organic layer was dried over $Na_2SO_4$ and concentrated *in vacuo.* to afford 3-(((1-(*tert*-butoxycarbonyl)piperidin-4-yl)methyl)amino) benzoic acid (**Intermediate 213**, 2.6 g, 93%) as a sticky liquid. $^1H$ NMR (400 MHz, DMSO-$d_6$) δ ppm 1.39 (s, 9H), 1.73-1.76 (m, 4H), 2.67-2.69 (m, 2H), 2.90-2.94 (m, 2H), 3.37-3.40 (m, 2H), 3.93-3.95 (m, 1H), 5.91 (br s, 1H), 6.78 (d, *J*=8.11 Hz, 1H), 7.07-7.09 (m, 1H), 7.13-7.15 (m, 1H), 7.17 (s, 1H). Mass spec: m/z 235.1 $[M+H]^+$.

**Step 3**

**Intermediate 214: *tert*-butyl 4-(((3-carbamoylphenyl)amino)methyl)piperidine-1-carboxylate**

To a solution of 3-(((1-(*tert*-butoxycarbonyl)piperidin-4-yl)methyl)amino)benzoic acid **(Intermediate 213**, 3.8 g, 11.0 mmol) in DMF (25 mL) was added diisopropylethylamine (6.0 mL, 34.0 mmol) and HATU (6.7 g, 17.0 mmol) followed by ammonium chloride (2.4 g, 45.0 mmol). The reaction mixture was stirred at room temperature for 16h. Progress of the reaction was monitored by TLC. After completion, the reaction mixture was diluted with water (200 mL) and extracted with EtOAc (3 × 100 mL). The organic layer was separated, dried over anhydrous $Na_2SO_4$ and concentrated *in vacuo* to afford *tert*-butyl 4-(((3-carbamoylphenyl)amino)methyl)piperidine-1-carboxylate (**Intermediate 214**, 3.4 g) as a yellow solid, which was used for the next step without further purification. $^1H$ NMR (400 MHz, DMSO-$d_6$) δ ppm 1.02-1.05 (m, 2H), 1.39 (s, 9H), 1.71-1.74 (m, 3H), 2.69-2.73 (m, 2H), 2.89-2.94 (m, 2H), 3.91-3.94 (m, 2H), 5.81 (br s, 1H), 6.69 (d, *J*=7.89 Hz, 1H), 6.93-7.04 (m, 2H), 7.07-7.11 (m, 1H), 7.16 (br s, 1H), 7.75 (br s, 1H). Mass spec: m/z 333.7 [M]+.

**Step 4**

**Intermediate 215: *tert*-butyl 4-(((*tert*-butoxycarbonyl)(3-carbamoylphenyl)amino)methyl)piperidine-1-carboxylate**

To a solution of *tert*-butyl 4-(((3-carbamoylphenyl)amino)methyl)piperidine-1-carboxylate (**Intermediate 214,** 3.2 g, 9.6 mmol) in tetrahydrofuran (30 mL) was added di-*tert*-butyl dicarbonate (6.3 g, 29.0 mmol). The reaction mixture was heated at 80°C for 16h. Progress of the reaction was monitored by TLC. After completion, the reaction mixture was concentrated *in vacuo.* The crude material was purified by combi-flash column chromatography by eluting with 95% EtOAc in heptane to afford *tert*-butyl 4-(((*tert*-butoxycarbonyl)(3-carbamoylphenyl)amino) methyl)piperidine-1-carboxylate (**Intermediate 215, 3.5** g, 84%) as a sticky liquid material. $^1H$ NMR (500 MHz, DMSO-$d_6$) δ ppm 1.36 (s, 9H), 1.37 (s, 9H), 1.53-1.59 (m, 4H), 2.61-2.63 (m, 1H),3.16-3.18 (m, 1H), 3.55-3.57 (m, 2H), 3.85-3.88 (m, 2H), 4.00-4.05 (m, 1H), 7.38-7.45 (m, 3H), 7.70-7.72 (m, 1H), 7.73 (br s, 1H), 8.00 (br s, 1H). Mass spec: m/z 433.8 $[M]^+$.

**Step 5**

**Intermediate 216: *tert*-butyl (R)-6-(3-((*tert*-butoxycarbonyl)((1-(tert-butoxycarbonyl)piperidin-4-yl)methyl)amino)benzamido)-2-(1-methylpyrrolidin-2-yl)-1H-pyrrolo[3,2-c]pyridine-1-carboxylate**

To a solution of tert-butyl 4-(((*tert*-butoxycarbonyl)(3-carbamoylphenyl)amino)methyl)piperidine-1-carboxylate (**Intermediate 215**, 0.4 g, 0.92 mmol) in 1,4-dioxane (6 mL) was added *tert*-butyl (R)-6-bromo-2-(1-methylpyrrolidin-2-yl)-1*H*-pyrrolo[3,2-c]pyridine-1-carboxylate (**Intermediate 4**, 0.38 g, 1.01 mmol) followed by caesium carbonate (0.90 g, 2.76 mmol). The reaction mixture was purged with argon for 20 min. To the reaction mixture $Pd_2(dba)_3$ (0.11 g, 0.12 mmol) and xantphos (0.16 g, 0.27 mmol) were added. The reaction mixture was purged with argon for 10 min and heated at 100°C for 2h. Progress of the reaction was monitored by TLC. After completion, the reaction mixture was passed through a celite pad and concentrated *in vacuo.* The crude material was purified by combi-flash column chromatography by eluting with

85% EtOAc in heptane to afford tert-butyl (R)-6-(3-((*tert*-butoxycarbonyl)((1-(*tert*-butoxycarbonyl)piperidin-4-yl)methyl) amino)benzamido)-2-(1-methylpyrrolidin-2-yl)-1H-pyrrolo[3,2-c]pyridine-1-carboxylate (**Intermediate 216**, 0.4 g, 85%) as an off white solid. $^1$H NMR (400 MHz, DMSO-$d_6$) δ ppm 1.37 (s, 9H), 1.38 (s, 9H), 1.59-1.62 (m, 6H), 1.69 (s, 9H), 1.73-1.75 (m, 2H), 2.35 (s, 3H), 2.37-2.42 (m, 1H), 2.62-2.67 (m, 2H), 3.11-3.13 (m, 1H), 3.37-3.39 (m, 1H), 3.62-3.64 (m, 2H), 3.87-3.92 (m, 3H), 6.76 (s, 1H), 7.45-3.47 (m, 2H), 7.85-7.87 (m, 1H), 7.95 (s, 1H), 8.60 (s, 1H), 8.93 (s, 1H), 10.79 (s, 1H). Mass spec: m/z 733.1 $[M+H]^+$.

**Step-6**

**Intermediate 217: (R)-N-(2-(1-methylpyrrolidin-2-yl)-1H-pyrrolo[3,2-c]pyridin-6-yl)-3-((piperidin-4-ylmethyl) amino) benzamide dihydrochloride**

To a solution of *tert*-butyl (R)-6-(3-((*tert*-butoxycarbonyl)((1-(*tert*-butoxycarbonyl)piperidin-4-yl)methyl)amino) benzamido)-2-(1-methylpyrrolidin-2-yl)-1H-pyrrolo[3,2-c]pyridine-1-carboxylate (**Intermediate 216,** 0.50 g, 0.79 mmol) in 1,4-dioxane (10 mL) was added 4M hydrochloric acid in 1,4-dioxane (12 mL) was added at 0°C. The reaction mixture was stirred at room temperature for 16h. Progress of the reaction was monitored by TLC. After completion, the reaction mixture was concentrated *in vacuo* to afford (R)-*N*-(2-(1-methylpyrrolidin-2-yl)-1*H*-pyrrolo[3,2-c]pyridin-6-yl)-3-((piperidin-4-ylmethyl)amino)benzamide dihydrochloride (**Intermediate 217**, 0.34 g) as a yellow solid which was used for the next step without further purification. $^1$H NMR (400 MHz, DMSO-$d_6$) δ ppm 1.32-1.39 (m, 2H), 1.91-1.95 (m, 3H), 2.17-2.21 (m, 2H), 2.40-2.43 (m, 2H), 2.53-2.57 (m, 1H), 2.81 (s, 3H), 2.86-2.89 (m, 1H), 3.05-3.08 (m, 2H), 3.26-3.29 (m, 3H), 3.73-3.75 (m, 1H), 4.72-4.74 (m, 1H), 6.88-6.91 (m, 1H), 7.26-7.31 (m, 4H), 8.23 (s, 1H), 8.53-8.55 (m, 1H), 8.78 (br s, 1H), 9.07 (s, 1H), 11.28 (br s, 1H), 11.75 (br s, 1H), 13.18 (br s, 1H). Mass spec: m/z 432.6 $[M+H]^+$.

**Step 7**

**Example 46: 3-(((1-(2-(2,6-dioxopiperidin-3-yl)-1,3-dioxoisoindolin-4-yl)piperidin-4-yl)niethyl)amino)-*N*-(2-((R)-1-methylpyrrolidin-2-yl)-1*H*-pyrrolo[3,2-c]pyridin-6-yl)benzamide**

To a solution of (R)-*N*-(2-(1-methylpyrrolidin-2-yl)-1*H*-pyrrolo[3,2-c]pyridin-6-yl)-3-((piperidin-4- ylmethyl)amino)benzamide dihydrochloride (**Intermediate 217,** 0.3 g, 0.90 mmol) in dimethyl sulfoxide (5 mL) was added 2-(2,6-dioxopiperidin-3-yl)-4-fluoroisoindoline-1,3-dione (CAS No: 835616-60-9, 0.26 g, 0.90 mmol) followed by *N*,*N*-diisopropylethylamine (0.78 mL, 4.50 mmol). The reaction mixture was heated at 110°C for 6h. Progress of the reaction was monitored by TLC. After completion, the reaction mixture was poured in ice cold water (50 mL) to obtain yellow solid, which was filtered and dried. The crude material was purified by preparative HPLC (Method A) to afford 3-(((1-(2-(2,6-dioxopiperidin-3-yl)-1,3-dioxoisoindolin-4-yl)piperidin-4-yl)methyl)amino)-*N*-(2-((R)-1-methylpyrrolidin-2-yl)-1*H*-pyrrolo [3,2-c]pyridin-6-yl)benzamide (**Example 46**, 0.1 g, 19%) as a yellow solid. $^1$H NMR (400 MHz, DMSO-$d_6$) δ ppm 1.42-1.50 (m, 2H), 1.78-1.92 (m, 6H), 1.99-2.05 (m, 1H), 2.11-2.14 (m, 1H), 2.17 (s, 3H), 2.23-2.29 (m, 1H), 2.54-2.61 (m, 2H), 2.83-2.92 (m, 3H), 3.06-3.09 (m, 2H), 3.12-3.16 (m, 1H), 3.32-3.34 (m, 1H), 3.72-3.76 (m, 2H), 5.07-5.11 (m, 1H), 5.91-5.92 (m, 1H), 6.38 (s, 1H), 6.78-6.81 (m, 1H), 7.15-7.22 (m, 3H), 7.32 (d, *J*=7.20 Hz, 1H), 7.35 (d, *J*=8.40 Hz, 1H), 7.65-7.70 (m, 1H), 8.17 (s, 1H), 8.48 (s, 1H), 10.13 (s, 1H), 11.07 (s, 1H), 11.34 (s, 1H). Mass spec: m/z: 688.0 $[M+H]^+$.

**Example 47 was synthesised following Scheme 45**

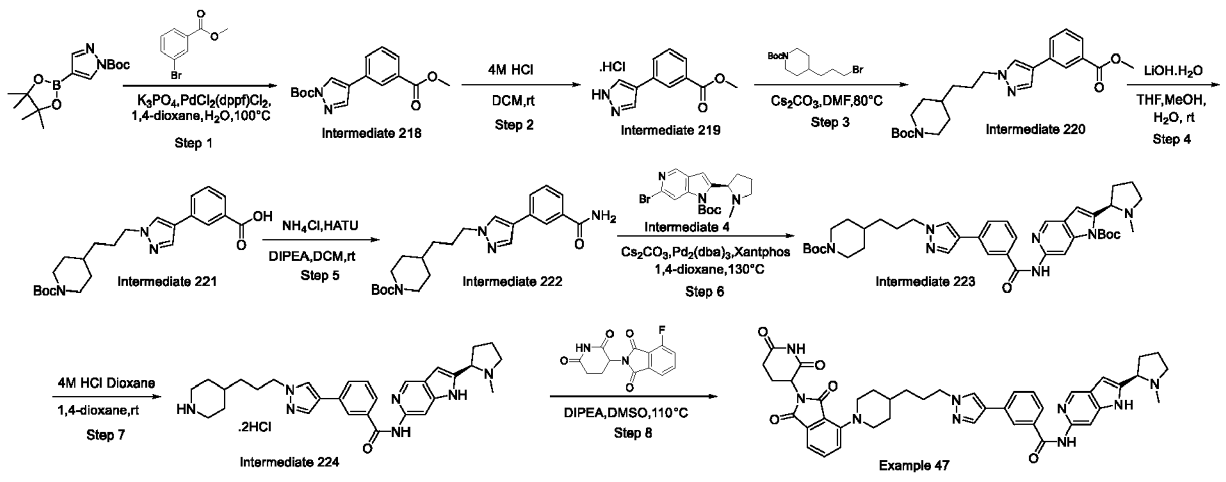


## Scheme 45

**Step 1**

**Intermediate 218: tert-butyl 4-(3-(methoxycarbonyl) phenyl)-1*H*-pyrazole-1-carboxylate**

To a solution of methyl-3-bromobenzoate (CAS No: 618-89-3, 2.5 g, 12 mmol) and *tert*-butyl-4-(4,4,5,5-tetramethyl-1,3,2-dioxaborolan-2-yl)pyrazole-1-carboxylate (CAS No: 552846-17-0, 3.8 g, 13 mmol) in 1,4-dioxane (10 mL) was added $K_3PO_4$ (6.2 g, 29 mmol) and water (2.0 mL) at room temperature. The reaction mixture was purged with argon for 10 min then Pd(dppf)$Cl_2$ (0.42 g, 0.58 mmol) was added, and the reaction mixture was further purged with argon for 5 min. The reaction mixture was then heated at 100°C for 16h. The reaction mixture was filtered through celite, water (50 mL) was added extracted with EtOAc (100 mL). The organic layer was dried over $Na_2SO_4$ and concentrated *in vacuo.* The crude material was purified by combi-flash column chromatography, eluting with 26% EtOAc in heptane, to afford tert-butyl 4-(3-(methoxycarbonyl) phenyl)-1*H*-pyrazole-1-carboxylate (**Intermediate 218,** 2.0 g, 57%) as an off white solid. $^1$H NMR (400 MHz, DMSO-$d_6$) δ ppm 1.61 (s, 9H), 3.89 (s, 3H), 7.57-7.58 (m, 1H), 7.88 (d, *J*=7.89 Hz, 1H), 8.05 (d, *J*=8.11 Hz, 1H), 8.27 (s, 1H), 8.39 (s, 1H), 8.86 (s, 1H). Mass spec: m/z: 303.2 $[M+H]^+$.

**Step 2**

**Intermediate 219: methyl-3-(1*H*-pyrazol-4-yl)benzoate hydrochloride**

To a solution of *tert*-butyl 4-(3-(methoxycarbonyl)phenyl)-1*H*-pyrazole-1-carboxylate **(Intermediate 218,** 2 g, 6.61 mmol) in dichloromethane (20 mL) was added 4M hydrochloric acid in 1,4-dioxane (10 mL) at 0°C under a $N_2$ atmosphere and the reaction mixture was stirred at room temperature for 16h. The reaction mixture was concentrated *in vacuo,* washed with EtOAc (100 mL) and pentane (100 mL) and dried *in vacuo* to afford methyl 3-(1*H*-pyrazol-4-yl) benzoate hydrochloride (**Intermediate 219**, 2.0 g) as a white solid, which was used in the next step without further purification. $^1$H NMR (500 MHz, DMSO-$d_6$) δ ppm 3.87 (s, 3H), 7.51-7.52 (m, 1H), 7.76-7.78 (m, 1H), 7.88-7.91 (m, 1H), 8.14 (d, *J*=1.60 Hz, 1H), 8.16 (s, 1H), 8.18 (s, 1H). Mass spec: m/z: 203.4 $[M+H]^+$.

**Step 3**

**Intermediate 220: *tert*-butyl 4-(3-(4-(3-(methoxycarbonyl)phenyl)-1*H*-pyrazol-1-yl)propyl)piperidine-1-carboxylate**

To a suspension of methyl-3-(1*H*-pyrazol-4-yl)benzoate hydrochloride (**Intermediate 219**, 2 g, 8.37 mmol) and tert-butyl 4-(3-bromopropyl)piperidine-1-carboxylate (CAS No: 164149-27-3, 2.56 g, 8.37 mmol) in DMF (10 mL) at 0°C under $N_2$ atmosphere was added cesium carbonate (6.82 g, 20.95 mmol) and the reaction mixture was heated at 80°C for 16h. The reaction mixture was poured into ice cold water (30 mL) and extracted with EtOAc (2 × 200 mL). The combined organic phases were dried over $Na_2SO_4$ and concentrated *in vacuo.* The crude material was purified by combi-flash column chromatography, eluting with 10% EtOAc in heptane, to afford *tert*-butyl 4-(3-(4-(3-(methoxycarbonyl)phenyl)-1*H*-pyrazol-1-yl)propyl)piperidine-1-carboxylate (**Intermediate 220**, 2.5 g, 70%) as yellow solid. Mass spec: m/z: 428.3 $[M+H]^+$.

**Step 4**

**Intermediate 221: 3-(1-(3-(1-(*tert*-butoxycarbonyl)piperidin-4-yl) propyl)-1*H*-pyrazol-4-yl)benzoic acid**

To a solution of *tert*-butyl 4-(3-(4-(3-(methoxycarbonyl)phenyl)-1*H*-pyrazol-1-yl)propyl)piperidine-1-carboxylate (**Intermediate 220**, 2.00 g, 4.67 mmol) in tetrahydrofuran (10 mL), methanol (10 mL) and water (5 mL) was added $LiOH.H_2O$ (0.98 g, 23.39 mmol) and the reaction mixture was stirred at rt for 4h. The reaction mixture was then concentrated *in vacuo,* water (10 mL) was added and the mixture acidified to pH~2 using aqueous citric acid. The resulting precipitate was filtered and dried under vacuum to afford 3-(1-(3-(1-(tert-butoxycarbonyl)piperidin-4-yl) propyl)-1*H*-pyrazol-4-yl)benzoic acid (**Intermediate 221**, 1.5 g, 78%) as an off white solid. $^1H$ NMR (400 MHz, DMSO-$d_6$) δ ppm 0.87-0.97 (m, 2H), 1.15-1.20 (m, 2H), 1.37 (s, 9H), 1.59-1.63 (m, 2H), 1.81-1.87 (m, 2H), 2.62-2.76 (m, 2H), 3.88-3.91 (m, 2H), 4.00-4.08 (m, 1H), 4.09-4.12 (m, 2H), 7.47 (t, J=7.75 Hz, 1H), 7.74-7.76 (m, 1H), 7.79-7.82 (m, 1H), 7.92 (s, 1H), 8.09 (s, 1H), 8.30 (s, 1H), 12.20 (br s, 1H). Mass spec: m/z: 414.2 $[M+H]^+$.

**Step 5**

**Intermediate 222: *tert*-butyl 4-(3-(4-(3-carbamoylphenyl)-1*H*-pyrazol-1-yl)propyl)piperidine-1-carboxylate**

To a solution of 3-(1-(3-(1-(*tert*-butoxycarbonyl)piperidin-4-yl)propyl)-1*H*-pyrazol-4-yl)benzoic acid (**Intermediate 221**, 2.5 g, 6.0 mmol) in DCM (25 mL) was added diisopropylethylamine (2.4 g, 18 mmol) and HATU (2.8 g, 7.3 mmol) followed by ammonium chloride (1.6 g, 30 mmol) and the reaction mixture was stirred at rt for 16h.

The reaction mixture was diluted with water (200 mL) and extracted with EtOAc (2 × 200 mL). The combined organic phases were washed with $NaHCO_3$ (2 × 40 mL) and brine (2 × 30 mL). The organic layer was dried over anhydrous $Na_2SO_4$ and concentrated and the crude material was purified by combi-flash column chromatography, by eluting with 12% EtOAc in heptane, to afford tert-butyl 4-(3-(4-(3-carbamoylphenyl)-1H-pyrazol-1-yl)propyl)piperidine-1-carboxylate (**Intermediate 222**, 1.5 g, 60%) as an off-white solid. $^1H$ NMR (400 MHz, DMSO-$d_6$) δ ppm 0.83-0.97 (m, 1H), 1.37 (s, 9H), 1.52-1.59 (m, 2H), 1.79-1.86 (m, 2H), 2.66-2.73 (m, 2H), 3.08-3.17 (m, 2H), 3.57-3.64 (m, 2H), 3.88-4.01 (m, 2H), 4.08-4.12 (m, 2H), 7.47 (t, J=7.78 Hz, 1H), 7.75 (d, J=7.67 Hz, 1H), 7.81 (d, J=7.89 Hz, 1H), 7.92 (s, 1H), 8.09 (s, 1H), 8.30 (s, 1H). Mass spec: m/z: 413.3 $[M+H]^+$.

**Step 6**

**Intermediate 223: *tert*-butyl (R)-6-(3-(1-(3-(1-(tert-butoxycarbonyl) piperidin-4-yl) propyl)-1*H*-pyrazol-4-yl) benzamido)-2-(1-methylpyrrolidin-2-yl)-1*H*-pyrrolo[3,2-c]pyridine-1-carboxylate**

To a solution of tert-butyl (R)-6-bromo-2-(1-methylpyrrolidin-2-yl)-1*H*-pyrrolo[3,2-c]pyridine-1-carboxylate (**Intermediate 4,** 0.400 g, 1.05 mmol) and tert-butyl 4-(3-(4-(3-carbamoylphenyl)-1H-pyrazol-1-yl)propyl)piperidine-1-carboxylate (**Intermediate 222**, 0.347 g, 0.84 mmol) in 1,4-dioxane (10 mL) was added cesium carbonate (1.03 g, 3.15 mmol). The reaction mixture was purged with argon for 10 min then tris(dibenzylideneacetone)dipalladium (0.148 g, 0.15 mmol) and Xantphos (0.188 g, 0.31mmol) were added. The reaction mixture was further degassed for 5 minutes with argon and then heated at 130°C for 2h. The reaction mixture was diluted with water (300 mL) and extracted with EtOAc (3 × 300 mL). The combined organic phases were dried over anhydrous $Na_2SO_4$ and concentrated *in vacuo.* The crude material was purified by combi-flash column chromatography, eluting with 70% EtOAc in heptane, to afford *tert*-butyl (R)-6-(3-(1-(3-(1-(tert-butoxycarbonyl)piperidin-4-yl)propyl)-1*H*-pyrazol-4-yl)benzamido)-2-(1-methylpyrrolidin-2-yl)-1*H*-pyrrolo[3,2-c]pyridine-1-carboxylate (**Intermediate 223**, 0.28 g, 30%) as a yellow solid. $^1H$ NMR (400 MHz, DMSO-$d_6$) δ ppm 1.15-1.22 (m, 2H), 1.17-1.20 (m, 2H) 1.37 (s, 9H), 1.60-1.65 (m, 5H), 1.70 (s, 9H), 1.74-1.86 (m, 2H), 2.31 (s, 3H) 2.32-2.36 (m, 4H), 2.62-2.66 (m, 2H), 3.10-3.13 (m, 2H), 3.92-3.94 (m, 1H), 4.10-4.14 (m, 2H), 6.76 (s, 1H), 7.46-7.50 (m, 1H), 7.75-7.85 (m, 2H), 8.00 (s, 1H), 8.30-8.32 (m, 2H), 8.61 (s, 1H), 8.96 (s, 1H), 10.74 (s, 1H). Mass spec: m/z: 712.4 $[M+H]^+$

**Step 7**

**Intermediate 224: (R)-*N*-(2-(1-methylpyrrolidin-2-yl)-1*H*-pyrrolo[3,2-c]pyridin-6-yl)-3-(1-(3-(piperidin-4-yl) propyl)-1*H*-pyrazol-4-yl) benzamide dihydrochloride**

To a solution of tert-butyl (R)-6-(3-(1-(3-(1-(tert-butoxycarbonyl)piperidin-4-yl)propyl)-1*H*-pyrazol-4-yl)benzamido)-2-(1-methylpyrrolidin-2-yl)-1*H*-pyrrolo[3,2-c]pyridine-1-carboxylate (**Intermediate 223**, 0.28 g, 0.39 mmol) in 1,4-dioxane (8 mL) was added 4M hydrochloric acid in 1,4-dioxane (8 mL) was at 0°C and the reaction mixture was stirred at

room temperature for 16h. The reaction mixture was concentrated *in vacuo* to afford (R)-*N*-(2-(1-methylpyrrolidin-2-yl)-1*H*-pyrrolo[3,2-c]pyridin-6-yl)-3-(1-(3-(piperidin-4-yl)propyl)-1*H*-pyrazol-4-yl) benzamide dihydrochloride (**Intermediate 224**, 0.29 g) as an off-white solid, which was used for next step without purification. $^1$H NMR (400 MHz, DMSO-$d_6$) δ ppm 1.18-1.30 (m, 5H), 1.76-1.87 (m, 4H), 2.21 (s, 3H), 2.76-2.85 (m, 6H), 3.20-3.27 (m, 5H), 4.13-4.16 (m, 2H), 7.27 (s, 1H), 7.59 (t, *J*=7.76 Hz, 1H), 7.89 (d, *J*=7.50 Hz, 1H), 7.94 (d, *J*= 8.16 Hz, 1H), 8.09 (s, 1H), 8.35 (s, 1H), 8.41 (s, 1H), 8.52 (s, 1H), 9.11 (s, 1H), 11.27 (s, 1H), 12.09 (br s, 1H), 13.18 (br s, 1H).

**Step 8**

**Example 47: 3-(1-(3-(1-(2-(2,6-dioxopiperidin-3-yl)-1,3-dioxoisoindolin-4-yl)piperidin-4-yl)propyl)-1*H*-pyrazol-4-yl)-*N*-(2-((R)-1-methylpyrrolidin-2-yl)-1*H*-pyrrolo[3,2-c]pyridin-6-yl)benzamide**

To a solution of (R)-*N*-(2-(1-methylpyrrolidin-2-yl)-1*H*-pyrrolo [3, 2-c] pyridin-6-yl)-3-(1-(3-(piperidin-4-yl) propyl)-1*H*-pyrazol-4-yl)benzamide dihydrochloride (**Intermediate 224**, 0.22 g, 0.42 mmol) in DMSO (4 mL) was added 2-(2,6-dioxopiperidin-3-yl)-4-fluoroisoindoline-1,3-dione (CAS No: 835616-60-9, 0.118 g, 0.42 mmol) and diisopropylethylamine (0.225 mL 1.29 mmol) and the reaction mixture was heated at 110°C for 6h. The reaction mixture was poured into ice cold $H_2O$ (40 mL) to get yellow precipitate, which was filtered and dried *in vacuo* to give crude material. The crude was purified by preparative HPLC (Method A) afford 3-(1-(3-(1-(2-(2,6-dioxopiperidin-3-yl)-1,3-dioxoisoindolin-4-yl) piperidin-4-yl)propyl)-1*H*-pyrazol-4-yl)-*N*-(2-((R)-1-methylpyrrolidin-2-yl)-1*H*-pyrrolo[3,2-c]pyridin-6-yl)benzamide (**Example 47**, 0.046 g, 14%) as a yellow solid. $^1$H NMR (400 MHz, DMSO-$d_6$) δ ppm 1.23-1.37 (m, 4H), 1.77-1.91 (m, 7H), 1.99-2.03 (m, 1H), 2.13-2.15 (m, 1H), 2.17 (s, 3H), 2.23-2.30 (m, 1H), 2.44-2.48 (m, 1H), 2.53-2.60 (m, 2H), 2.82-2.87 (m, 3H), 3.12-3.16 (m, 1H), 3.30-3.34 (m, 1H), 3.66-3.70 (m, 2H), 4.16-4.18 (m, 2H), 5.05-5.10 (m, 1H), 6.40 (s, 1H), 7.02 (s, 1H), 7.29-7.33 (m, 2H), 7.48 (t, *J*=7.75 Hz, 1H), 7.64-7.68 (m, 1H), 7.77 (d, J=7.88 Hz, 1H), 7.84 (d, J=8.00 Hz, 1H), 8.02 (s, 1H), 8.23 (s, 1H), 8.31 (s, 1H), 8.34 (s, 1H), 8.52 (s, 1H), 10.51 (s, 1H), 11.38 (s, 1H). Mass spec: m/z: 668.3 [M+H]$^+$.

**Example 48 was made following Scheme 46**

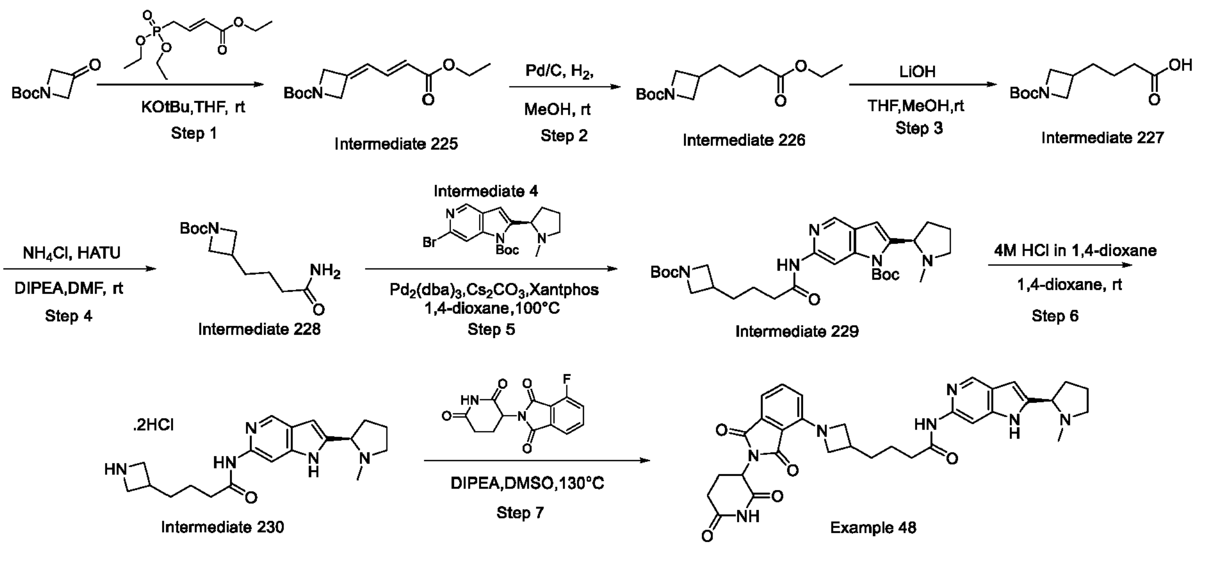


**Scheme 46**

**Step 1**

**Intermediate 225: *tert*-butyl (E)-3-(4-ethoxy-4-oxobut-2-en-1-ylidene) azetidine-1-carboxylate**

To a solution of tert-butyl-3-oxoazetidine-1-carboxylate (CAS No: 398489-26-4, 5.00 g, 28.62 mmol) in tetrahydrofuran (50 mL) was added potassium *tert*-butoxide (2.00 g, 20 mmol) at 0°C followed by triethyl 4-phosphonocrotonate (CAS No: 10236-14-3, 1.2 g, 4.30 mmol) and the reaction mixture was stirred at room temperature for 1h. The reaction mixture was then filtered through a celite bed and the filtrate was concentrated *in vacuo.* The crude material was purified by combi-flash column chromatography, eluting with 35% EtOAc in heptane, to afford tert-butyl (E)-3-(4-ethoxy-4-oxobut-2-en-1-ylidene)azetidine-1-carboxylate (**Intermediate 225**, 1.1 g, 14%) as an off-white solid. $^1$H NMR (400 MHz, DMSO-$d_6$) δ ppm 1.17 (t, J=6.8 Hz, 3H), 1.39 (s, 9H), 4.10-1.15 (m, 2H), 4.52 (s, 2H), 4.65 (s, 2H), 5.94 (d, J=15.34 Hz, 1H), 6.22 (d, *J*=11.20 Hz, 1H), 7.01-7.08 (m, 1H).

**Step 2**

**Intermediate 226: *tert*-butyl 3-(4-ethoxy-4-oxobutyl) azetidine-1-carboxylate**

To a solution of *tert*-butyl 3-[(E)-4-ethoxy-4-oxo-but-2-enylidene] azetidine-1-carboxylate (**Intermediate 225**, 1.1 g, 4.10 mmol) in methanol (45 mL) was added 10% palladium on carbon (0.60 g, 5.63 mmol). The reaction mixture was stirred at room temperature for 5h under $H_2$ atmosphere at 50 psi. The reaction mixture was filtered through a celite bed and filtrate was concentrated *in vacuo* to afford tert-butyl 3-(4-ethoxy-4-oxo-butyl)azetidine-1-carboxylate (**Intermediate 226**, 1.00 g, 90%) as a colorless liquid, which used in the next step without further purification. $^1$H NMR (400 MHz, DMSO-$d_6$) δ ppm 1.17 (t, *J*=7.09 Hz, 3H), 1.36 (s, 9H), 1.42-1.52 (m, 4H), 2.27 (t, *J*=7.1 Hz, 2H), 2.42-2.45 (m, 1H), 3.39-3.41 (m, 2H), 3.86-3.88 (m, 2H), 4.02-4.07 (m, 2H). Mass spec: m/z 272.1 $[M+H]^+$.

**Step 3**

**Intermediate 227: 4-(1-(*tert*-butoxycarbonyl)azetidin-3-yl)butanoic acid**

To a solution of tert-butyl 3-(4-ethoxy-4-oxo-butyl)azetidine-1-carboxylate (**Intermediate 226**, 1.4 g, 5.2 mmol) in tetrahydrofuran (10.0 mL) and methanol (5 mL) was added lithium hydroxide (0.25 g, 10 mmol) in water (2 mL) at room temperature and stirred for 2h. The reaction mixture was then acidified with aqueous citric acid and the pH was adjusted to ~5, upon which a solid precipitated. The solid was filtered and washed with water and dried under vacuum to afford 4-(1-(*tert*-butoxycarbonyl)azetidin-3-yl)butanoic acid (**Intermediate 227**, 1.20 g, 96%) as an off white solid. $^1$H NMR (400 MHz, DMSO-$d_6$) δ ppm 1.36 (s, 9H), 1.41-1.43 (m, 2H), 1.48-1.52 (m, 2H), 2.20 (t, J=6.85 Hz, 2H), 2.43-2.45 (m, 1H), 3.39-3.41 (m, 2H), 3.86-3.89 (m, 2H), 12.10 (br s, 1H). Mass spec: m/z 244.1 $[M+H]^+$.

**Step 4**

**Intermediate 228: tert-butyl 3-(4-amino-4-oxobutyl)azetidine-1-carboxylate**

To a solution of 4-(1-(tert-butoxycarbonyl)azetidin-3-yl)butanoic acid (**Intermediate 227,** 1.00 g, 4.0 mmol) in DMF (7 mL) was added HATU (2.3 g, 6.0 mmol) at room temperature and stirred for 15 min. Ammonium chloride (0.85 g, 16.0 mmol) was then added followed by diisopropylethylamine (2.1 mL, 12.0 mmol) and the reaction mixture was stirred at room temperature for 16h. The reaction mixture was then diluted with water (50 mL) and extracted with EtOAc (3 × 50 mL), dried over $Na_2SO_4$, and concentrated *in vacuo.* The crude material was purified by combi-flash column chromatography eluting with 80% EtOAc in heptane to afford tert-butyl 3-(4-amino-4-oxobutyl)azetidine-1-carboxylate (**Intermediate 228**, 0.25 g, 26%) as a colourless liquid. $^1$H NMR (400 MHz, DMSO-$d_6$) δ ppm 1.23-1.29 (m, 2H), 1.35 (s, 9H), 1.42-1.45 (m, 2H), 2.00 (t, J=7.34 Hz, 2H), 2.66-2.73 (m, 1H), 3.04-3.14 (m, 2H), 3.34-3.39 (m, 2H), 7.19 (br s, 2H). Mass spec: m/z 243.2 $[M+H]^+$.

**Step 5**

**Intermediate 229: *tert*-butyl (R)-6-(4-(1-(tert-butoxycarbonyl)azetidin-3-yl)butanamido)-2-(1-methylpyrrolidin-2-yl)-1H-pyrrolo[3,2-c]pyridine-1-carboxylate**

To a solution of tert-butyl 3-(4-amino-4-oxobutyl)azetidine-1-carboxylate (**Intermediate 228**, 0.20 g, 0.84 mmol) 1, 4-dioxane (5 mL) was added tert-butyl (R)-6-bromo-2-(1-methylpyrrolidin-2-yl)-1*H*-pyrrolo[3,2-c]pyridine-1-carboxylate (**Intermediate 4**, 0.15 g, 0.27 mmol) followed by cesium carbonate (1.03 g, 3.15 mmol) and the reaction mixture was purged with argon for 20 min. $Pd_2(dba)_3$ (0.148 g, 0.1578 mmol) followed by Xantphos (0.31 g, 0.188 mmol) was then added. The reaction mixture was further purged with argon gas for 10 minutes then heated at 100°C for 2h. The reaction mixture was then filtered through a celite bed and concentrated *in vacuo.* The crude material was purified by combi-flash column chromatography, by eluting with 85% EtOAc in heptane, to afford tert-butyl (R)-6-(4-(1-(tert-butoxycarbonyl) azetidin-3-yl)butanamido)-2-(1-methylpyrrolidin-2-yl)-1H-pyrrolo[3,2-c]pyridine-1-carboxylate (**Intermediate 229**, 0.15 g, 26%) as a yellow solid. $^1$H NMR (400 MHz, DMSO-$d_6$) δ ppm 1.36 (s, 9H), 1.60 (s, 9H), 1.64-1.71 (m, 6H), 2.33-2.37 (m, 8H) 2.73 (s, 3H), 3.97-3.90 (m, 4H), 6.70 (br s, 1H), 8.79 (s, 1H), 9.14 (s, 1H), 10.35 (br s, 1H). Mass spec: m/z 542.3 $[M+H]^+$.

**Step 6**

**Intermediate 230: (R)-4-(azetidin-3-yl)-N-(2-(1-methylpyrrolidin-2-yl)-1H-pyrrolo[3,2-c]pyridin-6-yl) butanamide dihydrochloride**

To a solution of tert-butyl (R)-6-(4-(1-(tert-butoxycarbonyl)azetidin-3-yl)butanamido)-2-(1-methylpyrrolidin-2-yl)-1H-pyrrolo[3,2-c]pyridine-1-carboxylate (**Intermediate 229**, 0.18 g, 0.40 mmol) in 1,4-dioxane (5.0 mL) was added 4M hydrochloric acid in 1,4-dioxane (5.0 mL) at 0°C and then stirred at room temperature for 16h. The reaction mixture was then concentrated *in vacuo* to afford (R)-4-(azetidin-3-yl)-N-(2-(1-methylpyrrolidin-2-yl)-1H-pyrrolo[3,2-c]pyridin-6-yl) butanamide dihydrochloride (**Intermediate 230**, 0.13 g) as a yellow solid, which was used in next step without further purification.

**Step 7**

**Example 48: 4-(1-(2-(2,6-dioxopiperidin-3-yl)-1,3-dioxoisoindolin-4-yl)azetidin-3-yl)-*N*-(2-((R)-1-methylpyrrolidin-2-yl)-1*H*-pyrrolo[3,2-c]pyridin-6-yl)butanamide**

To a solution of (R)-4-(azetidin-3-yl)-N-(2-(1-methylpyrrolidin-2-yl)-1*H*-pyrrolo[3,2-c]pyridin-6-yl) butanamide dihydrochloride (**Intermediate 230,** 0.15 g, 0.44 mmol) in dimethyl sulfoxide (1.0 mL) was added 2-(2,6-dioxopiperidin-3-yl)-4-fluoroisoindoline-1,3-dione (CAS No: 835616-60-9, 0.12 g, 0.43 mmol) followed by *N*,*N*-diisopropylethylamine (0.38 mL, 2.19 mmol) and the reaction mixture was heated at 130°C for 2h. The reaction mixture was then poured into ice cold water, upon which a solid precipitated, which was filtered and dried to obtain a crude product. The crude product was further purified by preparative HPLC (Method A) to afford 4-(1-(2-(2,6-dioxopiperidin-3-yl)-1,3-dioxoisoindolin-4-yl) azetidin-3-yl)-*N*-(2-((R)-1-methylpyrrolidin-2-yl)-1*H*-pyrrolo[3,2-c]pyridin-6-yl)butanamide (**Example 48**, 0.02 g, 8%) as a yellow solid. $^1$H NMR (400 MHz, DMSO-$d_6$) δ ppm 1.59-1.63 (m, 4H), 1.76-1.90 (m, 3H), 1.97-2.00 (m, 1H), 2.09-2.18 (m, 1H), 2.25 (s, 3H), 2.39 (t, J=6.79 Hz, 2H), 2.55-2.59 (m, 1H), 2.67-2.70 (m, 1H), 2.82-2.91 (m, 1H), 3.10-3.14 (m, 1H), 3.24-3.28 (m, 2H), 3.73-3.88 (m, 2H), 4.31-4.33 (m, 2H), 5.01-5.05 (m, 1H), 6.33 (s, 1H), 6.76-6.77 (m, 1H), 7.09 (d, J=6.85 Hz, 1H), 7.53-7.57 (m, 1H), 8.08 (s, 1H), 8.40 (s, 1H), 10.14 (s, 1H), 11.25 (s, 1H). Mass spec: m/z 598.1 [M+H]$^+$.

**Example 49 was made following Scheme 47**

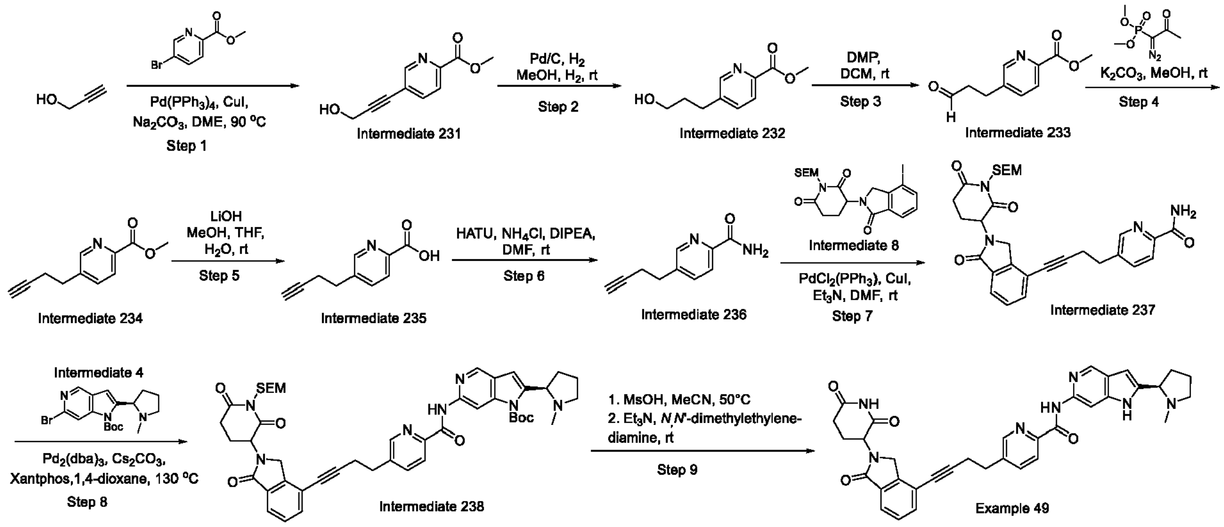


**Scheme 47**

**Step 1**

**Intermediate 231: methyl 5-(3-hydroxyprop-1-yn-1-yl)picolinate**

To a solution of methyl 5-bromopicolinate (CAS No: 29682-15-3, 10 g, 46.28 mmol) in 1,2-Dimethoxyethane (100

mL) was added propargyl alcohol (3.9 g, 69.40 mmol) and sodium carbonate (15.6 g, 185.12 mmol). The resulting reaction mixture was degassed for 10 min with argon gas and to this, $Pd(PPh_3)_4$ (5.34 g, 4.63 mmol) and copper iodide (0.88 g, 4.63 mmol) were added sequentially at room temperature and again degassed for 5 min with argon gas. The reaction mixture was heated at 90°C for 16 h. Progress of the reaction was monitored by TLC. After completion, the reaction mixture was filtered through celite, filtrate was diluted with water (50 mL) and extracted with EtOAc (200 mL). The organic layer was dried over $Na_2SO_4$ and concentrated *in vacuo.* The crude material was purified by combi-flash column chromatography, eluting with 30% EtOAc in heptane, to afford methyl 5-(3-hydroxyprop-1-ynyl)pyridine-2-carboxylate (**Intermediate 231**, 5.1 g, 59%) as a brown solid. $^1H$ NMR (400 MHz, DMSO-$d_6$) δ ppm 3.88 (s, 3H), 4.36 (d, J=5.6 Hz, 2H), 5.47 (t, J=6.0 Hz, 1H), 8.06-8.03 (m, 2H), 8.74 (s, 1H). Mass spec: m/z: 191.9 $[M+H]^+$.

**Step 2**

**Intermediate 232: methyl 5-(3-hydroxypropyl)picolinate**

To a solution of methyl 5-(3-hydroxyprop-1-yn-1-yl)picolinate (**Intermediate 231**, 5 g, 26.15 mmol) in methanol (80 mL) was added 10% by wt. Pd/C (900 mg, 8.45 mmol) under nitrogen atmosphere and the resulting solution was stirred under $H_2$ atmosphere (120 psi) at room temperature for 16h. The progress of reaction was monitored by TLC. After completion, the mixture was filtered through celite pad and filtrate was concentrated to give the crude mixture which was purified by combi-flash column chromatography, by eluting with 85% EtOAc in heptane, to afford methyl 5-(3-hydroxypropyl)picolinate (**Intermediate 232**, 2.4 g, 47%) as a brown liquid. $^1H$ NMR (400 MHz, DMSO-$d_6$) δ ppm 1.71-1.77 (m, 2H), 2.72 (t, J=8.0 Hz, 2H), 3.39-3.43 (m, 2H), 3.87 (s, 3H), 4.54 (t, J=5.2 Hz, 1H), 7.82 (d, J=7.2 Hz, 1H), 7.98 (d, J=7.2 Hz, 1H), 8.57 (s, 1H).

**Step 3**

**Intermediate 233: methyl 5-(3-oxopropyl)picolinate**

To a solution of methyl 5-(3-hydroxypropyl)picolinate (**Intermediate 232**, 1.0 g, 5.12 mmol) in dichloromethane (12 mL) was added Dess-Martin periodinane (4.34 g, 10.24 mmol) at 0°C and the reaction mixture was stirred at room temperature for 5h. The progress of the reaction was monitored by TLC. After completion of the reaction, the reaction mixture was diluted with water (60 mL) and extracted with EtOAc (2 × 60 mL). The organic layer was dried over $Na_2SO_4$ and concentrated to obtain crude methyl 5-(3-oxopropyl)picolinate (**Intermediate 233**, 1.0 g) as a white solid which was used for the next step without purification. $^1H$ NMR (400 MHz, DMSO-$d_6$) δ ppm 2.86-2.96 (m, 2H), 3.19-3.20 (m, 2H), 3.86 (s, 3H), 7.79-7.84 (d, *J*=8.4 Hz, 1H), 7.93-8.02 (m, 1H), 8.60 (s, 1H), 9.71 (s, 1H).

**Step 4**

**Intermediate 243: methyl 5-(but-3-yn-1-yl)picolinate**

To a solution of crude methyl 5-(3-oxopropyl)picolinate (**Intermediate 233**, 1.2 g, 6.21 mmol) in methanol (15 mL) was added potassium carbonate (1.3 g, 9.32 mmol) at 0°C. After stirring for 5 min (dimethyl (1-diazo-2-oxopropyl) phosphonate (1.8 g, 9.32 mmol) was added at the same temperature and stirred for 16h. The progress of reaction was monitored by TLC. After completion of the reaction, mixture was diluted with water (50 mL) and extracted with EtOAc (3 × 40 mL). The organic layer was washed with brine (40 mL), dried over $Na_2SO_4$ and concentrated to give the crude material which was purified by combi-flash column chromatography, by eluting with 25% EtOAc in heptane, to afford methyl 5-(but-3-yn-1-yl)picolinate (**Intermediate 234**, 450 mg, 38%) as a colorless liquid. $^1H$ NMR (400 MHz, DMSO-$d_6$) δ ppm 2.50-2.55 (m, 2H), 2.81 (br s, 1H), 2.87 (t, *J*=7.2 Hz, 2H), 3.87 (s, 3H), 7.88 (d, *J*=8.0 Hz, 1H), 8.50 (d, *J*=8.0 Hz, 1H), 8.61 (s, 1H). Mass spec: m/z 190.1 $[M+H]^+$.

**Step 5**

**Intermediate 235: 5-(but-3-yn-1-yl) picolinic acid**

To a solution of methyl 5-but-3-ynylpyridine-2-carboxylate (**Intermediate 234**, 450 mg, 2.39 mmol) in methanol (2 mL), and tetrahydrofuran (7 mL) was added lithium hydroxide (116 mg 4.76 mmol) in water (3 mL) at 0°C and the reaction mixture was stirred at room temperature for 5h. The progress of reaction mixture monitored by TLC. After completion of the reaction, solvent was evaporated under reduced pressure, water (20 mL) was added, acidified to pH 3-5 with 1 N HCl and the aqueous solution was extracted with 10% MeOH in DCM (3 × 40 mL). The organic layer was dried over $Na_2SO_4$ and

concentrated under vacuum to obtain crude 5-but-3-ynylpyridine-2-carboxylic acid (**Intermediate 235**, 410 mg, 98%) as a white solid. $^1$H NMR (400 MHz, DMSO-*d*$_6$) δ ppm 1.90 (s, 1H), 2.50-2.52 (m, 2H), 2.80-2.88 (m, 2H), 7.87 (d, J=7.60 Hz, 1H), 7.95 (d, *J*=7.60 Hz, 1H), 8.59 (s, 1H). Mass spec: m/z 176.1 [M+H]$^+$.

**Step 6**

**Intermediate 236: 5-(but-3-yn-1-yl)picolinamide**

To a solution of 5-but-3-ynylpyridine-2-carboxylic acid (**Intermediate 235**, 430 mg, 2.45 mmol) in DMF (7 mL) were added HATU (1.39 g, 3.68 mmol), *N, N*-Diisopropylethylamine (1.30 mL, 7.36 mmol) and ammonium chloride (0.52 g, 9.81 mmol) at room temperature and stirred reaction mixture for 16h. Progress of reaction was monitored by TLC. After completion of the reaction, mixture was diluted with water (60 mL) and extracted with 10% MeOH in DCM (3 × 40 mL). The organic layer was washed with ice cold water (60 ml) and brine (30 mL) and dried over $Na_2SO_4$ and concentrated *in vacuo*. The resulting crude residue was purified by combi-flash column chromatography, by eluting with 5% MeOH in DCM, to afford 5-(but-3-yn-1-yl)picolinamide (**Intermediate 236**, 400 mg, 94%) as a white solid. $^1$H NMR (400 MHz, DMSO-*d*$_6$) δ ppm 2.50-2.54 (m, 2H), 2.81 (s, 1H), 2.85 (t, *J*=6.8 Hz, 2H), 7.56 (s, 1H), 7.86 (d, *J*=7.6 Hz, 1H), 7.92 (d, *J*=8.0 Hz, 1H), 8.06 (s, 1H), 8.52 (s, 1H). Mass spec: m/z 174.9 [M+H]$^+$.

**Step 7**

**Intermediate 237: 5-(4-(2-(2,6-dioxo-1-((2-(trimethylsilyl)ethoxy)methyl)piperidin-3-yl)-1-oxoisoindolin-4-yl) but-3-yn-1-yl)picolinamide**

To a solution of 5-(but-3-yn-1-yl) picolinamide (**Intermediate 236**, 50 mg, 0.28 mmol) and 3-(4-iodo-1-oxo-isoindolin-2-yl)-1-(2-trimethylsilylethoxymethyl) piperidine-2,6-dione (**Intermediate 8**, 14 mg, 0.28 mmol) in DMF (2 mL) was added triethylamine (1.4 mL, 10.05 mmol) under argon. After purging for 10 min, copper iodide (5 mg 0.028 mmol) and Pd $(PPh_3)_4Cl_2$ (19 mg, 0.028 mmol) was added and stirred the reaction mixture at room temperature for 1h. After completion of reaction, the reaction mixture was diluted with water (30 mL) and extracted with EtOAc (3 × 30 mL). The organic layer was washed with brine (2 × 30 mL), dried over $Na_2SO_4$ and concentrated to give the crude residue which was purified by combi-flash column chromatography, by eluting with EtOAc, to afford 5-(4-(2-(2,6-dioxo-1-((2-(trimethylsilyl) ethoxy)methyl)piperidin-3-yl)-1-oxoisoindolin-4-yl)but-3-yn-1-yl)picolinamide (**Intermediate 237**, 60 mg, 38%) as a sticky liquid. $^1$H NMR (400 MHz, DMSO-*d*$_6$) δ ppm -0.04-0.05 (s, 9H), 0.81-0.85 (m, 2H), 2.72 (s, 2H), 2.80-2.83 (m, 2H), 2.84-2.86 (m, 2H), 2.89-2.97 (m, 2H), 2.98-3.07 (m, 1H), 3.51-3.55 (m, 2H), 4.10-4.25 (m, 2H), 5.02-5.10 (m, 2H), 7.49-7.58 (m, 4H), 7.70 (d, J=7.60 Hz, 1H), 7.92-8.05 (m, 3H). Mass spec: m/z 547.2 [M+H]$^+$.

**Step 8**

**Intermediate 238: *tert*-butyl 6-(5-(4-(2-(2,6-dioxo-1-((2-(trimethylsilyl)ethoxy)methyl)piperidin-3-yl)-1-oxoisoindolin-4-yl)but-3-yn-1-yl)picolinamido)-2-((R)-1-methylpyrrolidin-2-yl)-1H-pyrrolo[3,2-c]pyridine-1-carboxylate**

To a solution of tert-butyl (R)-6-bromo-2-(1-methylpyrrolidin-2-yl)-1H-pyrrolo[3,2-c]pyridine-1-carboxylate (**Intermediate 4**, 250 mg, 0.65 mmol) in 1,4-dioxane (10 mL) was added 5-(4-(2-(2,6-dioxo-1-((2-(trimethylsilyl)ethoxy) methyl)piperidin-3-yl)-1-oxoisoindolin-4-yl)but-3-yn-1-yl)picolinamide (**Intermediate 237**, 0.287 g, 0.52 mmol) and cesium carbonate (0.646 g, 1.97 mmol) and the resulting mixture was degassed with argon gas for 10-20 min, then $Pd_2(dba)_3$ (93 mg, 0.098 mmol) followed by 4,5-Bis(diphenylphosphino)-9,9-dimethylxanthene (117 mg, 0.19 mmol) were added. The resulting reaction mixture was heated at 130°C for 2h. After completion of the reaction, the mixture was concentrated under reduced pressure to obtain crude material which was purified by combi-flash column chromatography, by eluting with 80% EtOAc in heptane, to obtain tert-butyl 6-(5-(4-(2-(2,6-dioxo-1-((2-(trimethylsilyl)ethoxy)methyl)piperidin-3-yl)-1-oxoisoindolin-4-yl)but-3-yn-1-yl)picolinamido)-2-((R)-1-methylpyrrolidin-2-yl)-1H-pyrrolo[3,2-c]pyridine-1-carboxylate (**Intermediate 238**, 200 mg, 41%) as a green solid. $^1$H NMR (400 MHz, DMSO-*d*$_6$) δ ppm -0.070 (s, 9H), 0.71-0.74 (m, 2H), 1.62-1.65 (m, 2H), 1.62-1.66 (m, 2H), 1.72 (s, 9H), 2.27-2.39 (m, 6H), 2.40 (s, 3H), 2.89-2.95 (m, 2H), 3.06-3.13 (m, 4H), 3.34-3.36 (m, 1H), 3.62 (s, 2H), 3.91-3.94 (m, 1H), 4.31-4.39 (m, 2H), 6.76 (s, 1H), 7.46-7.51 (m, 1H), 7.62-7.62 (m, 1H), 7.65-7.67 (m, 1H), 8.09-8.11 (m, 1H), 8.20-8.23 (m, 1H), 8.58 (s, 1H), 8.75 (d, J=5.60 Hz, 1H), 8.98 (s, 1H), 10.44 (s, 1H).

**Step 9**

**Example 49: 5-(4-(2-(2,6-dioxopiperidin-3-yl)-1-oxoisoindolin-4-yl)but-3-yn-1-yl)-N-(2-((R)-1-methylpyrrolidin-2-yl)-1H-pyrrolo[3,2-c]pyridin-6-yl)picolinamide**

To a solution of tert-butyl 6-(5-(4-(2-(2,6-dioxo-1-((2-(trimethylsilyl)ethoxy)methyl)piperidin-3-yl)-1-oxoisoindolin-4-yl)but-3-yn-1-yl)picolinamido)-2-((R)-1-methylpyrrolidin-2-yl)-1H-pyrrolo[3,2-c]pyridine-1-carboxylate (**Intermediate 238,** 200 mg, 0.23 mmol) in MeCN (10 mL) was added methane sulfonic acid (347 mg, 3.54 mmol) at room temperature and heated at 50°C for 2h. After 2h the reaction was cooled to room temperature and then *N, N'*-dimethylethylenediamine (138 mg, 1.41 mmol) followed by triethylamine (481 mg, 4.72 mmol) were added at room temperature and stirred for 3h. The reaction mixture was concentrated under reduced pressure to obtain crude compound which was purified by preparative HPLC (Method A) to afford tert-butyl 6-(5-(4-(2-(2,6-dioxo-1-((2-(trimethylsilyl)ethoxy)methyl)piperidin-3-yl)-1-oxoisoindolin-4-yl)but-3-yn-1-yl)picolinamido)-2-((R)-1-methylpyrrolidin-2-yl)-1H-pyrrolo[3,2-c]pyridine-1-carboxylate (**Example 49**, 3 mg, 2%) as an off-white solid. $^1$H NMR (400 MHz, DMSO-$d_6$) δ ppm 1.81-189 (m, 5H), 2.17 (brs, 3H), 2.88-2.91 (m, 6H), 3.02-3.13 (m, 3H), 4.54-4.58 (m, 4H), 6.57 (s, 1H), 7.45-7.48 (m, 2H), 7.52 (d, J=6.8 Hz, 1H), 7.63 (d, J=6.8 Hz, 1H), 8.04-8.05 (m, 2H), 8.16 (d, J=8.0 Hz, 1H), 8.24-8.26 (m, 1H), 8.51-8.55 (m, 2H), 8.73 (s, 1H). Mass spec: m/z 616.3 $[M+H]^+$.

**Example 50 was synthesised following Scheme 48**

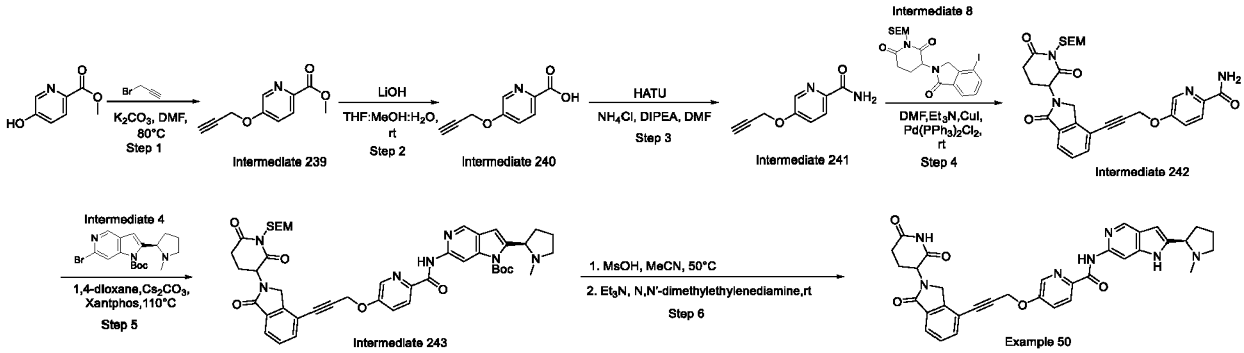


**Scheme 48**

**Step 1**

**Intermediate 239: methyl 5-(prop-2-yn-1-yloxy)picolinate**

To a solution of methyl 5-hydroxypyridine-2-carboxylate (CAS No: 30766-12-2, 4.0 g, 26.12 mmol) in DMF (30 mL) was added potassium carbonate (5.42 g, 39.18 mmol). The reaction mixture was stirred at room temperature for 15 min then 3-bromo-1-propyne (CAS No: 106-96-7, 4.66 g, 31.34 mmol) was added at room temperature and the reaction mixture was heated at 80°C for 16h. Progress of the reaction was monitored by TLC. After completion, reaction mixture was diluted with water (50 mL) and extracted with EtOAc (3 × 60 mL). The organic layer was washed with $NaHCO_3$ (2 × 50 mL) and brine (2 × 50 mL). The organic layers were separated and dried over $Na_2SO_4$ and concentrated to give crude product. The crude product was purified by column chromatography by eluting with 70% EtOAc in heptane to afford methyl 5-(prop-2-yn-1-yloxy)picolinate (**Intermediate 239**, 2.5 g, 50%) as an off-white solid. $^1$H NMR (400 MHz, DMSO-$d_6$) δ ppm 3.69 (s, 1H) 3.85 (s, 3H) 5.01 (d, *J*=2.49 Hz, 2H) 7.53-7.63 (m, 1H) 8.07 (d, *J*=8.71 Hz, 1H) 8.42 (d, *J*=2.90 Hz, 1H). Mass spec: m/z 191.9 $[M+H]^+$.

**Step 2**

**Intermediate 240: 5-(prop-2-yn-1-yloxy)picolinic acid**

To a solution of methyl 5-(prop-2-yn-1-yloxy)picolinate (**Intermediate 239**, 2 g, 10.46 mmol) in THF (7 mL) and methanol (7 mL) was added lithium hydroxide (1.02 g, 41.84 mmol) in water (7 mL) at room temperature and the reaction mixture was stirred at RT for 16h. Progress of the reaction was monitored by TLC. After completion of reaction solvent was evaporated under reduced pressure, water (15 mL) was added, acidified to pH 2-3 with 1N HCl at 0°C to obtain white precipitate, which was filtered and dried to afford 5-(prop-2-yn-1-yloxy)picolinic acid (**Intermediate 240,** 1.8 g, 91%) as an

off-white solid. $^1$H NMR (400 MHz, DMSO-$d_6$) δ ppm 3.68 (s, 1H) 4.99 (s, 2H) 7.54-7.57 (m, 1H) 8.04 (d, *J*=8.71 Hz, 1H) 8.38 (d, *J*=2.49 Hz, 1H) 12.20 (br s, 1H). Mass spec: m/z 178.0 [M+H]$^+$.

**Step 3**

**Intermediate 241: 5-(prop-2-yn-1-yloxy)picolinamide**

To a solution of 5-(prop-2-yn-1-yloxy)picolinic acid (**Intermediate 240**, 1.8 g, 10 mmol) in DMF (20 mL) was added HATU (8.0 g, 20 mmol) and N, N-Diisopropylethylamine (4.6 g, 36 mmol). Reaction mixture was stirred at room temperature for 15 min then added ammonium chloride (3.8 g, 71 mmol). Reaction mixture was stirred at room temperature for 16h. Progress of the reaction was monitored by TLC. The reaction mixture was diluted with water (20 mL), extracted with EtOAc (2 × 50 mL). The organic layer was washed with $NaHCO_3$ (2 × 30 mL) and brine (30 mL), dried over $Na_2SO_4$ and concentrated to give crude. The crude material was purified by combi-flash eluted with 2% MeOH in DCM to afford 5-(prop-2-yn-1-yloxy)picolinamide (**Intermediate 241**, 1.3 g, 73%) as a white solid. $^1$H NMR (400 MHz, DMSO-$d_6$) δ ppm 3.68 (s, 1H), 4.98 (s, 2H), 7.49 (br s, 1H), 7.56-7.58 (m, 1H), 7.94 (br s, 1H), 8.00-8.02 (m, 1H), 8.33 (s, 1H). Mass spec: m/z 177.1 [M+H]$^+$.

**Step 4**

**Intermediate 242: 5-((3-(2-(2,6-dioxo-1-((2-(trimethylsilyl)ethoxy)methyl)piperidin-3-yl)-1-oxoisoindolin-4-yl) prop-2-yn-1-yl)oxy)picolinamide**

To a solution of 5-(prop-2-yn-1-yloxy)picolinamide (**Intermediate 241**, 0.50 g, 2.83) and 3-(4-iodo-1-oxo-isoindolin-2-yl)-1-(2-trimethylsilylethoxymethyl)piperidine-2,6-dione (**Intermediate 8**, 1.42 g, 2.83 mmol) in DMF (20 mL) were added triethylamine (10.1 g, 99.33 mmol), dichlorobis(triphenylphosphine)palladium(II) (0.20 g, 0.28 mmol) and copper (I) iodide (0.055 g, 0.28 mmol) at rt. Reaction mixture was stirred at RT for 2h. Progress of the reaction was monitored by TLC. The reaction mixture was diluted with water (50 mL) and extracted with EtOAc (3 × 60 mL). The organic layer was washed with brine (2 × 50 mL), dried over $Na_2SO_4$ and concentrated to give crude. The crude product was purified by combi-flash column by eluting with 100% EtOAc to afford 5-((3-(2-(2,6-dioxo-1-((2-(trimethylsilyl)ethoxy)methyl)piperidin-3-yl)-1-oxoisoindolin-4-yl)prop-2-yn-1-yl)oxy)picolinamide (**Intermediate 242**, 0.65 g, 42%) as a yellow solid. $^1$H NMR (400 MHz, DMSO-$d_6$) δ ppm -0.09-0.05 (m, 9H), 0.75-0.92 (m, 2H), 1.98-2.09 (m, 1H), 2.27-2.37 (m, 1H), 2.81-2.82 (m, 1H), 3.00-3.14 (m, 1H), 3.52-3.55 (m, 2H), 4.10-4.22 (m, 1H), 4.26-4.40 (m, 1H), 5.13 (s, 2H), 5.21-5.34 (m, 3H), 7.50 (br s, 1H), 7.53-7.61 (m, 1H), 7.66-7.75 (m, 2H), 7.79 (d, *J*=7.67 Hz, 1H), 7.94 (br s, 1H), 7.99-8.09 (m, 1H), 8.40 (s, 1H). Mass spec: m/z 549.4 [M+H]$^+$.

**Step 5**

**Intermediate 243: *tert*-butyl 6-(5-((3-(2-(2,6-dioxo-1-((2-(trimethylsilyl)ethoxy)methyl)piperidin-3-yl)-1-oxoisoindolin-4-yl)prop-2-yn-1-yl)oxy)picolinamido)-2-((R)-1-methylpyrrolidin-2-yl)-1H-pyrrolo[3,2-c]pyridine-1-carboxylate**

To a solution 5-((3-(2-(2,6-dioxo-1-((2-(trimethylsilyl)ethoxy)methyl)piperidin-3-yl)-1-oxoisoindolin-4-yl)prop-2-yn-1-yl)oxy)picolinamide (**Intermediate 242**, 0.60 g, 1.09 mmol) in 1,4-dioxane (7 mL) was added tert-butyl 6-bromo-2-(1-methylpyrrolidin-2-yl)pyrrolo[3,2-c]pyridine-1-carboxylate (**Intermediate 4**, 0.49 g, 1.31 mmol) and cesium carbonate (1.07 g, 3.28 mmol) at room temperature and the reaction mixture was degassed with argon gas for 10 min then added xantphos (0.19 g, 0.32 mmol) and $Pd_2$ $(dba)_3$ (0.15 g, 0.16 mmol). The reaction mixture was heated at 110°C for 2h. Progress of the reaction was monitored by TLC. The reaction mixture was diluted with water (50 mL) and extracted with EtOAc (3 × 60 mL). The combined organic layer was washed with brine (2 × 50 mL), dried over $Na_2SO_4$ and concentrated to get crude. The crude product was purified by combi-flash column eluted with 2% MeOH in DCM to afford *tert*-butyl 6-[[5-[3-[2-[2,6-dioxo-1-(2-trimethylsilylethoxymethyl)-3-piperidyl]-1-oxo-isoindolin-4-yl]prop-2-ynoxy]pyridine-2-carbonyl]amino]-2-(1 methylpyrrolidin-2 yl)pyrrolo[3,2-c]pyridine-1-carboxylate (**Intermediate 243**, 0.32 g, 35%) as a yellow solid. $^1$H NMR (400 MHz, DMSO-$d_6$) δ ppm -0.07 (s, 9H), 0.71-0.86 (m, 2H), 1.62 (m, 3H), 1.72 (s, 9H), 2.06-2.13 (m, 1H), 2.30-2.33 (m, 4H), 2.35 (s, 3H), 2.74-2.78 (m, 1H), 3.01-3.12 (m, 2H), 3.41-3.53 (m, 3H), 3.89-3.92 (m, 1H), 4.08-4.36 (m, 2H), 4.93-5.03 (m, 2H), 5.21-5.23 (m,1H), 5.37 (s, 2H), 6.76 (s, 1H), 7.52-7.63 (m, 1H), 7.73-7.83 (m, 3H), 8.24 (d, J=8.71 Hz, 1H), 8.56-8.58 (m, 2H), 8.93-8.99 (m, 1H), 10.30 (s, 1H). Mass spec: m/z 848.4 [M+H]$^+$.

**Step 6**

**Example 50: 5-[3-[2-(2,6-dioxo-3-piperidyl)-1-oxo-isoindolin-4-yl]prop-2-ynoxy]-N-[2-(1-methylpyrrolidin-2-yl)-1H-pyrrolo[3,2-c]pyridin-6-yl]pyridine-2-carboxamide**

To a solution of tert-butyl 6-[[5-[3-[2-[2,6-dioxo-1-(2-trimethylsilylethoxymethyl)-3-piperidyl]-1-oxo-isoindolin-4-yl]prop-2-ynoxy]pyridine-2-carbonyl]amino]-2-(1-methylpyrrolidin-2-yl)pyrrolo[3,2-c]pyridine-1-carboxylate (**Intermediate 243**, 0.30 g, 0.353 mmol) in acetonitrile (8 mL) was added methane sulfonic acid (0.52 g, 5.30 mmol) at room temperature and the reaction mixture was heated at 50°C for 2h. To the reaction mixture triethylamine (0.72 g, 7.075 mmol) and *N,N'*-dimethylethylenediamine (0.20 g, 2.12 mmol) was added and the reaction mixture was stirred at RT for 2h. Progress of the reaction was monitored by TLC. The reaction mixture was concentrated to get crude mixture. The crude compound was purified by preparative HPLC (Method A) to afford 5-[3-[2-(2,6-dioxo-3-piperidyl)-1-oxo-isoindolin-4-yl]prop-2-ynoxy]-N-[2-(1-methylpyrrolidin-2-yl)-1H-pyrrolo[3,2-c]pyridin-6-yl]pyridine-2-carboxamide (**Example 50**, 0.28 g, 13%) as an off-white solid. $^1$H NMR (400 MHz, DMSO-$d_6$) δ ppm 1.78-1.95 (m, 3H), 1.97-2.06 (m, 1H), 2.18-2.20 (m, 3H), 2.30-2.44 (m, 1H), 2.54 (s, 3H), 2.61-2.69 (m, 1H), 2.86-2.96 (m, 1H), 3.08-3.20 (m, 1H), 4.25-4.48 (m, 2H), 5.10-5.18 (m, 1H), 5.36 (s, 2H), 6.42 (s, 1H), 7.48-7.62 (m, 1H), 7.69-7.84 (m, 3H), 8.17-8.30 (m, 2H), 8.49 (s, 1H), 8.57 (d, *J*=2.81 Hz, 1H), 10.17 (s, 1H), 11.14 (s, 1H), 11.46 (s, 1H). Mass spec: m/z 618.0 [M+H]$^+$.

**Example 51 was synthesised following Scheme 49**

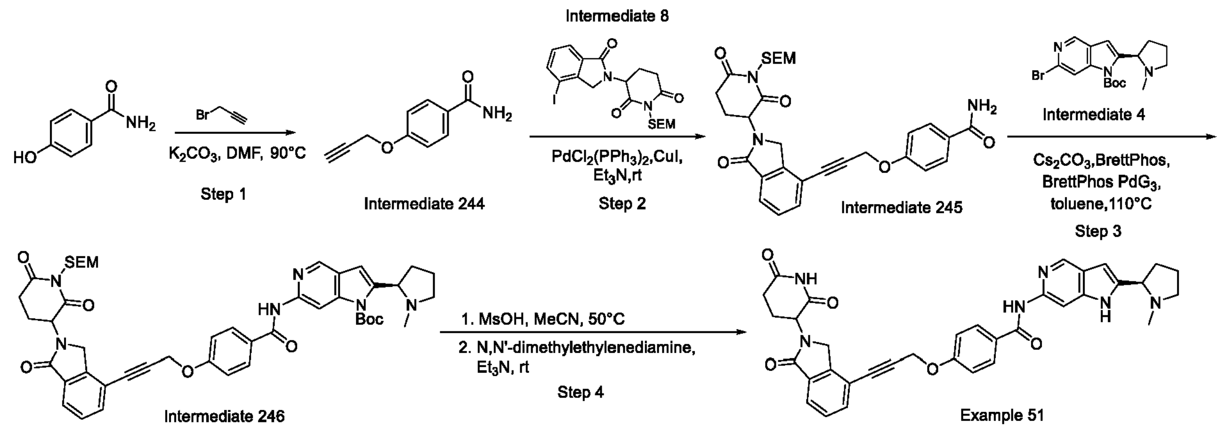


Scheme 49

**Step 1**

**Intermediate 244: 4-(prop-2-yn-1-yloxy)benzamide**

To a solution of 4-hydroxybenzamide (CAS No: 619-57-8, 4 g, 29.16 mmol) in DMF (40 mL) was added potassium carbonate (6.05 g, 43.75 mmol) and propargyl bromide (CAS No: 106-96-7, 5.20 g, 35.00 mmol). The reaction mixture was heated at 90°C for 12h. Progress of the reaction was monitored by TLC. After completion, the reaction mixture was diluted with water (80 mL) and extracted with EtOAc (2 × 80 mL). The organic layer was washed with brine (3 × 60 mL). The organic layer was separated and dried over $Na_2SO_4$ and concentrated *in vacuo*. The obtained crude material was purified by combi-flash column chromatography, by eluting with 2% MeOH in DCM, to afford 4-(prop-2-yn-1-yloxy)benzamide (**Intermediate 244**, 2.6 g, 51%) as an off solid. $^1$H NMR (400 MHz, DMSO-$d_6$) δ ppm 3.59 (s, 1H), 4.86 (s, 2H), 7.02 (d, *J* = 8.80 Hz, 2H), 7.19 (br s, 1H), 7.85 (d, J=7.83 Hz, 2H), 8.66 (br s, 1H). Mass spec: m/z 176.1 [M+H]$^+$.

**Step 2**

**Intermediate 245: 4-((3-(2-(2, 6-dioxo-1-((2-(trimethylsilyl) ethoxy) methyl) piperidin-3-yl)-1-oxoisoindolin-4-yl) prop-2-yn-1-yl) oxy)benzamide**

To a solution of 3-(4-iodo-1-oxoisoindolin-2-yl)-1-((2-(trimethylsilyl)ethoxy)methyl)piperidine-2,6-dione (**Intermediate 8**, 1.5 g, 3.0 mmol) and 4-(prop-2-yn-1-yloxy)benzamide (**Intermediate 244**, 0.53 g, 3.0 mmol) in DMF (20 mL)

was added triethylamine (15 mL, 100 mmol). The reaction mixture was purged with argon gas for 5 min then $PdCl_2(PPh_3)_2$ (0.21 g, 0.30 mmol) and Copper (I) iodide (0.05 g, 0.30 mmol) were added. The reaction mixture was stirred at room temperature for 2h. Progress of the reaction was monitored by TLC. After completion, the reaction mixture was diluted with water (50 mL) and extracted with EtOAc (3 × 60 mL). The organic layer was washed with brine solution (3 × 60 mL). The organic layer was dried over $Na_2SO_4$ and concentrated *in vacuo.* The obtained crude material was purified by combi-flash column chromatography, by eluting with 90% EtOAc in heptane, to afford 4-((3-(2-(2,6-dioxo-1-((2-(trimethylsilyl)ethoxy) methyl)piperidin-3-yl)-1-oxoisoindolin-4-yl)prop-2-yn-1-yl)oxy)benzamide (**Intermediate 245**, 1.0 g, 61%) as a yellow solid.

$^1$H NMR (400 MHz, DMSO-$d_6$) δ ppm -0.03 (s, 9H), 0.83-0.85 (m, 2H), 1.97-2.04 (m, 1H), 2.27-2.32 (m, 1H), 2.78-2.82 (m, 1H), 3.03-3.09 (m, 1H), 3.53 (s, 2H), 4.11-4.16 (m, 1H), 4.29-4.34 (m, 1H), 5.04-5.09 (m, 2H), 5.17 (s, 2H), 5.22-5.26 (m, 1H), 7.11 (d, *J*=7.88 Hz, 2H), 7.20 (br s, 1H), 7.54-7.58 (m, 1H), 7.71 (d, J=7.46 Hz, 1H), 7.78-7.79 (m, 1H), 7.83 (br s, 1H), 7.87 (d, J=8.29 Hz, 2H).

**Step 3**

**Intermediate 246: *tert*-butyl 6-(4-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((R)-1-methylpyrrolidin-2-yl)-1*H*-pyrrolo[3,2-c]pyridine-1-carboxylate**

To a solution of 4-((3-(2-(2,6-dioxo-1-((2-(trimethylsilyl)ethoxy)methyl)piperidin-3-yl)-1-oxoisoindolin-4-yl) prop-2-yn-1-yl)oxy)benzamide (**Intermediate 245**, 0.50 g, 0.9129 mmol) in toluene (10 mL) was added tert-butyl (R)-6-bromo-2-(1-methylpyrrolidin-2-yl)-1*H*-pyrrolo[3,2-c]pyridine-1-carboxylate (**Intermediate 4**, 0.41 g, 1.095 mmol) followed by caesium carbonate (0.89 g, 2.739 mmol) at room temperature. The reaction mixture was purged with argon for 10 min then BrettPhos (0.075 g, 0.1369 mmol) and BrettPhos PdG3 (0.12 g, 0.1369 mmol) were added, and the reaction mixture was again purged with argon gas for 5 min and heated at 110°C for 2h. Progress of the reaction was monitored by TLC. After completion, the reaction mixture was cooled to room temperature, treated with water (40 mL) and extracted with EtOAc (2 × 50 mL). The organic layer was separated and dried over $Na_2SO_4$ and concentrated *in vacuo.* The crude material was purified by combi-flash column chromatography, by eluting with 2% MeOH in DCM, to afford tert-butyl 6-(4-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((R)-1-methylpyrrolidin-2-yl)-1H-pyrrolo[3,2-c]pyridine-1-carboxylate (**Intermediate 246**, 0.24 g, 31%) as an off-white solid. $^1$H NMR (400 MHz, DMSO-$d_6$) δ ppm -0.07 (s, 9H), 0.76-0.80 (m, 2H), 1.17-1.19 (m, 1H), 1.70 (s, 9H), 1.98-2.05 (m, 2H), 2.13-2.15 (m, 1H), 2.28-2.31 (m, 3H), 2.35 (s, 3H), 2.74-2.78 (m, 1H), 2.99-3.09 (m, 1H), 3.36-3.48 (m, 2H), 3.90-3.94 (m, 1H), 3.99-4.07 (m, 1H), 4.09-4.18 (m, 1H), 4.29-4.37 (m, 1H), 4.95-5.02 (m, 2H), 5.19-5.26 (m, 3H), 6.75 (s, 1H), 7.17 (d, J=8.71 Hz, 2H), 7.54-7.60 (m, 1H), 7.73 (d, J=7.46 Hz, 1H), 7.78 (d, J=7.46 Hz, 1H), 8.09 (d, J=8.29 Hz, 2H), 8.58 (s, 1H), 8.93 (s, 1H), 10.54 (s, 1H). Mass spec: m/z 847.5 $[M+H]^+$.

**Step 4**

**Example 51: 4-((3-(2-(2,6-dioxopiperidin-3-yl)-1-oxoisoindolin-4-yl)prop-2-yn-1-yl)oxy)-*N*-(2-((R)-1-methylpyrrolidin-2-yl)-1*H*-pyrrolo[3,2-c]pyridin-6-yl)benzamide**

To a solution of tert-butyl 6-(4-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((R)-1-methylpyrrolidin-2-yl)-1*H*-pyrrolo[3,2-c]pyridine-1-carboxylate (**Intermediate 246**, 0.36 g, 0.42 mmol) in acetonitrile (10 mL) was added methanesulfonic acid (0.42 mL, 6.37 mmol) at room temperature and the reaction mixture was heated at 50°C for 2h. Reaction mixture was cooled to RT then triethylamine (1.19 mL, 8.50 mmol) added and N,N'-dimethylethylenediamine (0.30 mL, 2.55 mmol) and stirred at room temperature for 2h. Progress of the reaction was monitored by TLC. After completion, the reaction mixture was concentrated *in vacuo.* The crude compound was purified by preparative HPLC (Method A) to afford 4-((3-(2-(2,6-dioxopiperidin-3-yl)-1-oxoisoindolin-4-yl)prop-2-yn-1-yl)oxy)-N (2-((R)-1-methylpyrrolidin-2-yl)-1H-pyrrolo[3,2-c]pyridin-6-yl)benzamide (**Example 51**, 0.03 g, 11%) as an off white solid. $^1$H NMR (400 MHz, DMSO-$d_6$) δ ppm 1.75-1.94 (m, 3H), 1.97-2.04 (m, 1H), 2.11-2.15 (m, 1H), 2.17 (s, 3H), 2.20-2.30 (m, 1H), 2.38-2.44 (m, 1H), 2.59-2.63 (m, 2H), 2.86-2.96 (m, 1H), 3.10-3.18 (m, 1H), 4.26-4.33 (m, 1H), 4.37-4.45 (m, 1H), 5.13-5.18 (m, 1H), 5.22 (s, 2H), 6.40 (s, 1H), 7.18 (d, J=8.80 Hz, 2H), 7.54-7.59 (m, 1H), 7.72 (d, J=7.58, 1H), 7.78 (d, J=7.58, 1H), 8.07 (d, J=8.80 Hz, 2H), 8.19 (s, 1H), 8.49 (s, 1H), 10.28 (br s, 1H), 11.25 (br s, 1H), 11.37 (br s, 1H). Mass spec: m/z 617.2 $[M+H]^+$.

**Example 52 was synthesised following Scheme 50**

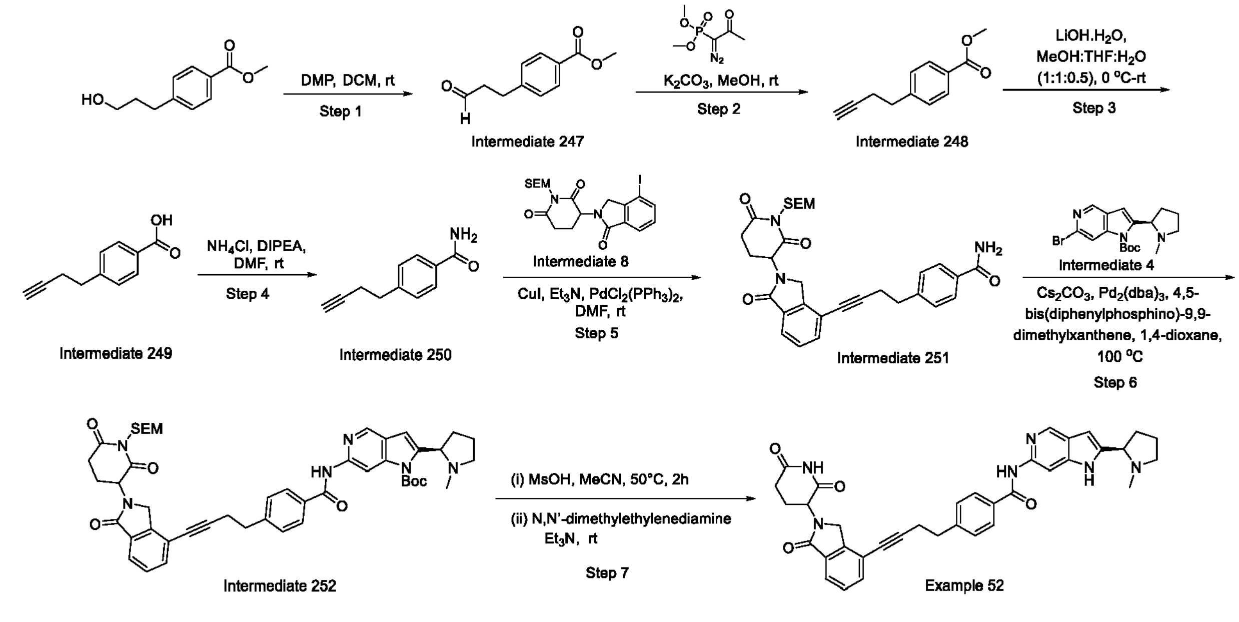


## Scheme 50

**Step** 1

**Intermediate 247: methyl 4-(3-oxopropyl)benzoate**

To a solution of methyl 4-(3-hydroxypropyl)benzoate (CAS No: 15403-22-2, 5 g, 25.74 mmol) in dichloromethane (40 mL) was added Dess-Martin periodinane (14.63 g, 33.47 mmol) at 0 °C and the reaction mixture was stirred at room temperature for 6h. The progress of reaction was monitored by TLC. After completion of the reaction, the mixture was diluted with saturated $NaHCO_3$ (100 mL) and saturated sodium thiosulphate (100 mL) and extracted with DCM (3 × 150 mL). The combined organic layer was separated, dried over anhydrous $Na_2SO_4$ and concentrated under reduced pressure to afford the crude methyl 4-(3-oxopropyl)benzoate (**Intermediate 247**, 7.0 g crude) as a yellow liquid which was used directly into next step without further purification. $^1$H NMR (400 MHz, DMSO-$d_6$) δ ppm 2.83 (d, *J*=6.80 Hz, 2H), 2.92 (d, *J*=7.2 HZ, 2H), 3.83 (s, 3H),7.37 (d, *J*=7.6 HZ, 2H), 7.87 (d, *J*=7.6 Hz, 2H), 9.71 (s, 1H). Mass spec: m/z 190.9 $[M+H]^+$.

**Step 2**

**Intermediate 248: methyl 4-(but-3-yn-1-yl)benzoate**

To a solution of methyl 4-(3-oxopropyl)benzoate (**Intermediate 247**, 3 g, 15.61 mmol) in methanol (30 mL) was added $K_2CO_3$ (4.3 g, 31.22 mmol) at room temperature followed by 1-diazo-1-dimethoxyphosphoryl-propan-2-one (3.30 g, 17.17 mmol) was added. The resulting reaction mixture was stirred for 4h. The progress of the reaction was monitored by TLC. After completion of the reaction, the mixture was diluted with water (500 mL) and extracted with diethyl ether (3 × 200 mL). The combined organic layer was separated, dried over anhydrous $Na_2SO_4$ and concentrated under reduced pressure to afford crude compound which was purified by combi-flash column chromatography, by eluting with 10% EtOAc in heptane, to afford methyl 4-(but-3-yn-1-yl)benzoate (**Intermediate 248**, 2.3 g, 78%) as a yellow liquid. $^1$H NMR (500 MHz, DMSO-$d_6$) δ ppm 2.48 (s, 1H), 2.50-2.52 (m, 1H), 2.77-2.81 (m, 1H), 2.84 (t, J=7.2 Hz, 2H), 3.84 (s, 3H), 7.40 (d, J=8.0 Hz, 2H), 7.88 (d, J=8.0 Hz, 2H). Mass spec: m/z 189.1 $[M+H]^+$.

**Step 3**

**Intermediate 249: 4-(but-3-yn-1-yl)benzoic acid**

To a solution of methyl 4-(but-3-yn-1-yl)benzoate (**Intermediate 248**, 2.3 g, 12 mmol) in tetrahydrofuran (10 mL) and methanol (10 mL) was added lithium hydroxide monohydrate (1.20 g, 49 mmol) in water (5 mL) at 0°C and the resulting

reaction mixture was stirred at room temperature for 6h. The progress of the reaction was monitored by TLC. After completion of reaction, solvent was evaporated under reduced pressure, water (20 mL) was added. The aqueous solution was acidified with 0.5 N HCl to a pH-3-4, then extracted with EtOAc (2 × 250 mL). The combined organic layer was dried over $Na_2SO_4$ and concentrated. The crude solid obtained was triturated with n-pentane to afford 4-(but-3-yn-1-yl)benzoic acid (**Intermediate 249**, 1.9 g, 89%) as an off-white solid.$^1$H NMR (500 MHz, DMSO-$d_6$) δ ppm 2.49-2.51 (m, 2H), 2.78 (s, 1H), 2.83 (t, *J*=6.8 Hz, 2H), 7.37 (d, *J*=7.6 Hz, 2H), 7.86 (d, *J*=6.8 Hz, 2H), 12.84 (br s, 1H). Mass spec: m/z 173.0 $[M+H]^+$.

**Step 4**

**Intermediate 250: 4-(but-3-yn-1-yl)benzamide**

To a solution of 4-(but-3-yn-1-yl)benzoic acid (**Intermediate 249**, 1.9 g, 11 mmol) in DMF (20 mL) was added HATU (6.50 g, 16 mmol) and *N*,*N*-Diisopropylethylamine (4.30 g, 33 mmol) at room temperature. After stirring for 10 min, $NH_4Cl$ (2.90 g, 55 mmol) was added and stirred for 16h at room temperature. The progress of the reaction was monitored by TLC. After completion of the reaction, mixture was diluted with water (500 mL) and extracted with EtOAc (3 × 250 mL). The combined organic layer was separated, dried over $Na_2SO_4$ and concentrated to afford 4-(but-3-yn-1-yl)benzamide (**Intermediate 250**, 1.5 g) as a white solid, which was used in the next step without further purification. $^1$H NMR (400 MHz, DMSO-$d_6$) δ ppm 2.48-2.50 (m, 2H), 2.77 (s, 1H), 2.80 (t, J=6.8 Hz, 2H), 7.27 (brs, 1H), 7.32 (d, J=8.0 Hz, 2H), 7.79 (d, J=7.6 Hz, 2H), 7.89 (brs, 1H). Mass spec: m/z 174.0 $[M+H]^+$.

**Step 5**

**Intermediate 251: 4-(4-(2-(2,6-dioxo-1-((2-(trimethylsilyl)ethoxy)methyl)piperidin-3-yl)-1-oxoisoindolin-4-yl) but-3-yn-1-yl)benzamide**

To a stirred solution of 4-(but-3-yn-1-yl)benzamide (**Intermediate 250**, 1 g, 5.77 mmol) and 3-(4-iodo-1-oxoisoindolin-2-yl)-1-((2-(trimethylsilyl)ethoxy)methyl)piperidine-2,6-dione (**Intermediate 8**, 2.89 g, 5.77 mmol) in DMF (10 mL) was added triethylamine (21.10 g, 207.84 mmol) at room temperature. The reaction mixture was purged with argon gas for 10 min then $PdCl_2(PPh_3)_2$ (410 mg, 0.58 mmol) and copper (I) iodide (112 mg, 0.58 mmol) was added under argon atmosphere and purging was continued for another 5 min. The reaction mixture was stirred at room temperature for 2h. The progress of the reaction was monitored by TLC. After completion of the reaction, solvent was evaporated under reduced pressure and the crude material was purified by combi-flash column chromatography, by eluting with 80% EtOAc in heptane, to afford 4-(4-(2-(2,6-dioxo-1-((2-(trimethylsilyl)ethoxy)methyl)piperidin-3-yl)-1-oxoisoindolin-4-yl)but-3-yn-1-yl)benzamide (**Intermediate 251**, 1.6 g, 51%) as a yellow solid. $^1$H NMR (400 MHz, DMSO-$d_6$) δ ppm -0.009 (s, 9H), 0.82-0.88 (m, 2H), 2.02-2.04 (m,1H), 2.33-2.37 (m, 1H), 2.81-2.89 (m, 3H), 2.91-3.03 (m, 3H), 3.03-3.11 (m, 1H), 3.51-3.56 (m, 2H), 4.07-4.09 (m, 1H), 4.19-4.21 (m, 1H), 5.04-5.08 (m, 1H), 5.19-5.24 (m, 1H), 7.27 (brs, 1H), 7.38 (d, J=8.0 Hz, 2H), 7.51-7.52 (m, 1H), 7.59 (d, J=7.6 Hz, 1H), 7.70 (d, J=7.2 Hz, 1H), 7.82 (d, J=8.4 Hz, 2H), 7.88 (brs, 1H). Mass spec: m/z 544.0 $[M-H]^-$.

**Step 6**

**Intermediate 252: *tert*-butyl 6-(4-(4-(2-(2,6-dioxo-1-((2-(trimethylsilyl)ethoxy)methyl)piperidin×-3-yl)-1-oxoisoindolin-4-yl)but-3-yn-1-yl)benzamido)-2-((*R*)-1-methylpyrrolidin-2-yl)-1H-pyrrolo[3,2-c]pyridine-1-carboxylate**

To a stirred solution of 4-(4-(2-(2,6-dioxo-1-((2-(trimethylsilyl)ethoxy)methyl)piperidin-3-yl)-1-oxoisoindolin-4-yl) but-3-yn-1-yl)benzamide (**Intermediate 251**, 401 mg, 0.73 mmol) and 1,4-dioxane (10 mL) in was added tert-butyl (R)-6-bromo-2-(1-methylpyrrolidin-2-yl)-1H-pyrrolo[3,2-c]pyridine-1-carboxylate (**Intermediate 4**, 224 mg, 0.58 mmol) and cesium carbonate (720 mg, 2.21 mmol). After degassing with argon for 15 min, $Pd_2(dba)_3$ (208 mg, 0.22 mmol) and 4,5-bis(diphenylphosphino)-9,9-dimethylxanthene (66 mg, 0.11 mmol) were added to the reaction mixture and degassed further with argon gas for 10 min. The resulting reaction mixture was heated at 100°C for 2h. The progress of the reaction was monitored by TLC. After completion of the reaction, the mixture was directly concentrated under reduced pressure to get crude compound which was purified by combi-flash column chromatography, by eluting with 90% EtOAc in heptane, to afford tert-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)benzamido)-2-((R)-1-methylpyrrolidin-2-yl)-1H-pyrrolo[3,2-c]pyridine-1-carboxylate (**Intermediate 252**, 390 mg, 63%) as a yellow solid.$^1$H NMR (400 MHz, DMSO-$d_6$) δ ppm -0.06 (s, 9H), 0.78-0.83 (m, 2H), 1.17-1.16 (m, 1H), 1.61-1.66 (m, 2H), 1.69 (s, 9H), 1.75-1.77 (m, 2H), 2.03-2.06 (m, 1H), 2.37 (s, 3H), 2.38-2.41(m, 1H), 2.76-2.77 (m, 1H), 2.85-2.88 (m, 2H), 2.97-3.06 (m, 3H), 3.13-3.20 (m, 1H), 3.45-3.47 (m, 2H), 3.92-3.94 (m, 1H), 4.07-4.09 (m, 1H),

4.23-4.25 (m, 1H), 4.95-5.01 (m, 2H), 5.17-5.21 (m, 1H), 6.75 (s, 1H), 7.46 (d, *J*=8.40 Hz, 2H), 7.51 (t, *J*=7.6 Hz, 1H), 7.61 (d, *J*=7.6 Hz, 1H), 7.69 (d, *J*=7.2 Hz, 1H), 8.01 (d, *J*=7.6 Hz, 2H), 8.58 (s, 1H), 8.93 (s, 1H), 10.59 (s, 1H). Mass spec: m/z 845.2 $[M+H]^+$.

**Step 7**

**Example 52: 4-(4-(2-(2,6-dioxopiperidin-3-yl)-1-oxoisoindolin-4-yl)but-3-yn-1-yl)-N-(2-((*R*)-1-methylpyrrolidin-2-yl)-1H-pyrrolo[3,2-c]pyridin-6-yl)benzamide**

To a solution of tert-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)benzamido)-2-((*R*)-1-methylpyrrolidin-2-yl)-1H-pyrrolo[3,2-c]pyridine-1-carboxylate (**Intermediate 252**, 390 mg, 0.46 mmol) in acetonitrile (8 mL) was added methanesulfonic acid (470 mg, 4.62 mmol) at room temperature and heated to 50°C for 2h. After completion of the reaction, mixture was cooled to room temperature and *N,N*-dimethylethylenediamine (214 mg, 2.31 mmol) and triethylamine (983 mg, 9.23 mmol) were added and stirred at room temperature for 3h. Progress of reaction was monitored by TLC. After completion of the reaction, solvent was evaporated under reduced pressure, diluted with water (20 mL) to obtain a white precipitate, which was filtered and dried to get the crude compound which was purified by preparative HPLC (Method A) to afford 4-(4-(2-(2,6-dioxopiperidin-3-yl)-1-oxoisoindolin-4-yl)but-3-yn-1-yl)-N-(2-((*R*)-1-methylpyrrolidin-2-yl)-1H-pyrrolo[3,2-c]pyridin-6-yl)benzamide (**Example 52**, 110 mg, 39%) as an off white solid. $^1$H NMR (400 MHz, DMSO-$d_6$) $\delta$ ppm 1.78-1.99 (m, 3H), 2.01-2.15 (m, 1H), 2.17-2.23 (m, 1H), 2.28 (s, 3H), 2.30-2.35 (m, 1H), 2.41-2.43 (m, 1H), 2.51-2.57 (m, 1H), 2.61-2.81 (m, 3H), 2.84-2.89 (m, 2H), 3.15-3.16 (m, 1H), 3.29-3.33 (m, 1H), 4.26-4.28 (m, 2H), 5.10-5.15 (m, 1H), 6.39 (s, 1H), 7.45-7.59 (m, 2H), 7.60 (d, J=6.80 Hz, 1H), 7.70 (d, J=6.4Hz, 1H), 7.50 (d, J=8.4 Hz, 2H), 8.18 (s, 1H), 8.19 (s, 1H), 8.49 (s, 1H), 10.33 (s, 1H), 11.12 (s, 1H), 11.36 (s, 1H). Mass spec: m/z 615.1 $[M+H]^+$.

**Example 53 was synthesised following Scheme 51**

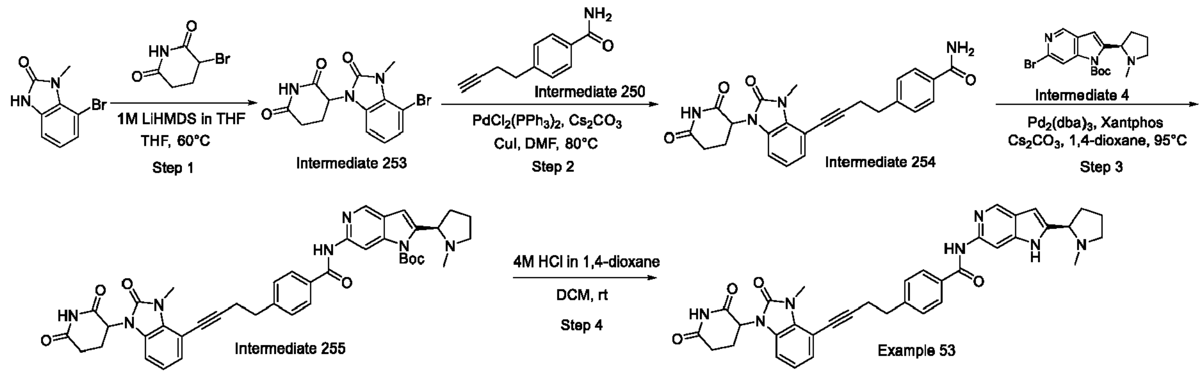


**Scheme 51**

**Step 1**

**Intermediate 253: 3-(4-bromo-3-methyl-2-oxo-2, 3-dihydro-1H-benzo[*d*]imidazol-1-yl)piperidine-2,6-dione**

To a solution of 7-bromo-1-methyl-1,3-dihydro-2*H*-benzo[*d*]imidazol-2-one (CAS No: 913297-44-6, 5.00 g, 22 mmol) in tetrahydrofuran (20 mL) was added 1M LiHMDS in THF (55 mL, 55 mmol). The resulting solution was then added dropwise to 3-bromopiperidine-2,6-dione (CAS No: 62595-74-8, 8.5 g, 44 mmol) in THF (55 mL) at room temperature and the reaction mixture was stirred at 60°C for 4h. The reaction mixture was poured into saturated aqueous $NH_4Cl$ solution (250 mL) and stirred for 10 min at room temperature. The resulting solid was collected by filtration, washed with ethyl acetate (20 mL) and dried under vacuum to afford 3-(4-bromo-3-methyl-2-oxo-2,3-dihydro-1H-benzo[d]imidazol-1-yl) piperidine-2,6-dione (**Intermediate 253**, 4.00 g, 54%) as a grey solid, which was used in the next step without further purification. $^1$H NMR (400 MHz, DMSO-$d_6$) $\delta$ ppm 1.98-2.08 (m, 1H), 2.62-2.73 (m, 2H), 2.83-2.95 (m, 1H), 3.64 (s, 3H), 5.39-5.43 (m, 1H), 6.94-7.02 (m, 1H), 7.17 (d, *J*=8.20 Hz, 1H), 7.24 (d, *J*=8.20 Hz, 1H), 11.12 (br s, 1H). Mass spec: m/z:

338.04 $[M+H]^+$.

**Step 2**

**Intermediate 254: 4-(4-(1-(2, 6-dioxopiperidin-3-yl)-3-methyl-2-oxo-2, 3-dihydro-1*H*-benzo[*d*]imidazol-4-yl) but-3-yn-1-yl)benzamide**

To a solution of 4-(but-3-yn-1-yl)benzamide (**Intermediate 250**, 0.49 g, 2.88 mmol) in dimethylformamide (15 mL) was added 3-(4-bromo-3-methyl-2-oxo-2,3-dihydro-1*H*-benzo[d]imidazol-1-yl)piperidine-2,6-dione (**Intermediate 253**, 0.65 g, 1.92 mmol) followed by cesium carbonate (1.98 g, 5.76 mmol) and the reaction mixture was purged with argon for 15 min. Copper(I) iodide (0.037 g, 0.19 mmol) and $PdCl_2(PPh_3)_2$ (0.14 g, 0.19 mmol) were then added and the reaction mixture was further purged with argon for 10 min and then stirred at 80°C for 3h. The reaction mixture was concentrated *in vacuo* to obtain crude compound. The crude material was purified by combi-flash column chromatography by eluting with 15% MeOH in DCM to afford 4-(4-(1-(2, 6-dioxopiperidin-3-yl)-3-methyl-2-oxo-2,3-dihydro-1H-benzo[d]imidazol-4-yl) but-3-yn-1-yl)benzamide (**Intermediate 254**, 0.40 g, 48%) as a brown solid. $^1$H NMR (400 MHz, DMSO-$d_6$) δ ppm 2.58-2.74 (m, 2H), 2.77-2.99 (m, 6H), 3.38 (s, 3H), 5.34-5.38 (m, 1H), 6.92-7.13 (m, 2H), 7.24-7.46 (m, 3H), 7.81-7.92 (m, 3H), 11.09 (br s, 1H). Mass spec: m/z: 429.26 $[M-H]^-$.

**Step 3**

**Intermediate 255: tert-butyl 6-(4-(4-(1-(2,6-dioxopiperidin-3-yl)-3-methyl-2-oxo-2,3-dihydro-1*H*-benzo[*d*]imidazol-4-yl)but-3-yn-1-yl)benzamido)-2-((*R*)-1-methylpyrrolidin-2-yl)-1*H*-pyrrolo[3,2-c]pyridine-1-carboxylate**

To a solution of 4-(4-(1-(2, 6-dioxopiperidin-3-yl)-3-methyl-2-oxo-2,3-dihydro-1H-benzo[d]imidazol-4-yl)but-3-yn-1-yl)benzamide (**Intermediate 254**, 0.38 g, 0.88 mmol) in 1,4-dioxane (9 mL) was added tert-butyl (*R*)-6-bromo-2-(1-methylpyrrolidin-2-yl)-1*H*-pyrrolo[3,2-*c*]pyridine-1-carboxylate (**Intermediate 4**, 0.40 g, 1.06 mmol) followed by cesium carbonate (0.87 g, 2.65 mmol) at room temperature. The reaction mixture was purged with argon for 15 min then $Pd_2(dba)_3$ (0.11 g, 0.13 mmol) and Xantphos (0.16 g, 0.26 mmol) were added and the reaction mixture was further purged with argon for 10 min and stirred at 95°C for 1h. The reaction mixture was filtered through celite, washed with dichloromethane (20 mL) and the filtrate was concentrated *in vacuo* to obtain crude compound. The crude material was purified by combi-flash column chromatography, by eluting with 10% MeOH in DCM, to afford *tert*-butyl 6-(4-(4-(1-(2,6-dioxopiperidin-3-yl)-3-methyl-2-oxo-2,3-dihydro-1*H*-benzo[*d*]imidazol-4-yl)but-3-yn-1-yl)benzamido)-2-((*R*)-1-methylpyrrolidin-2-yl)-1*H*-pyrrolo[3,2-*c*]pyridine-1-carboxylate (**Intermediate 255**, 0.40 g) as a yellow solid, which was used for the next step without further purification. Mass spec: m/z: 730.1 $[M+H]^+$.

**Step 4**

**Example 53: 4-(4-(1-(2,6-dioxopiperidin-3-yl)-3-methyl-2-oxo-2,3-dihydro-1*H*-benzo[*d*]imidazol-4-yl)but-3-yn-1-yl)-*N*-(2-((*R*)-1-methylpyrrolidin-2-yl)-1*H*-pyrrolo[3,2-*c*]pyridin-6-yl)benzamide**

To a solution of tert-butyl 6-(4-(4-(1-(2,6-dioxopiperidin-3-yl)-3-methyl-2-oxo-2,3-dihydro-1H-benzo[d]imidazol-4-yl)but-3-yn-1-yl)benzamido)-2-((R)-1-methylpyrrolidin-2-yl)-1*H*-pyrrolo[3,2-c]pyridine-1-carboxylate (**Intermediate 255**, 0.38 g, 0.52 mmol) in dichloromethane (3.0 mL) was added 4M hydrochloric acid in 1,4-dioxane (0.3 mL, 1.0 mmol) at 0°C. The reaction mixture was then stirred at room temperature for 2h. The reaction mixture was concentrated *in vacuo* and crude residue was washed with diethyl ether (10 mL) and dried under vacuum. This material was then purified by preparative HPLC (Method A) to afford 4-(4-(1-(2,6-dioxopiperidin-3-yl)-3-methyl-2-oxo-2,3-dihydro-1*H*-benzo[*d*]imidazol-4-yl)but-3-yn-1-yl)-*N*-(2-((*R*)-1-methylpyrrolidin-2-yl)-1*H*-pyrrolo[3,2-*c*]pyridin-6-yl)benzamide (**Example 53**, 0.01 g, 3%) as an off white solid. $^1$H NMR (400 MHz, DMSO-$d_6$) δ ppm 1.75-1.95 (m, 3H), 1.95-2.05 (m, 1H), 2.12-2.15 (m, 1H), 2.17 (s, 3H), 2.22-2.30 (m, 1H), 2.57-2.67 (m, 3H), 2.85-2.93 (m, 3H), 2.94-3.03 (m, 2H), 3.11-3.18 (m, 1H), 3.43 (s, 3H), 5.33-5.38 (m, 1H), 6.39 (s, 1H), 6.94-7.05 (m, 2H), 7.08-7.10 (m, 1H), 7.46 (d, J=8.25 Hz, 2H), 8.02 (d, J=8.25 Hz, 2H), 8.19. Mass spec: m/z: 630.0$[M+H]^+$.

**Example 54 was synthesised following Scheme 52**

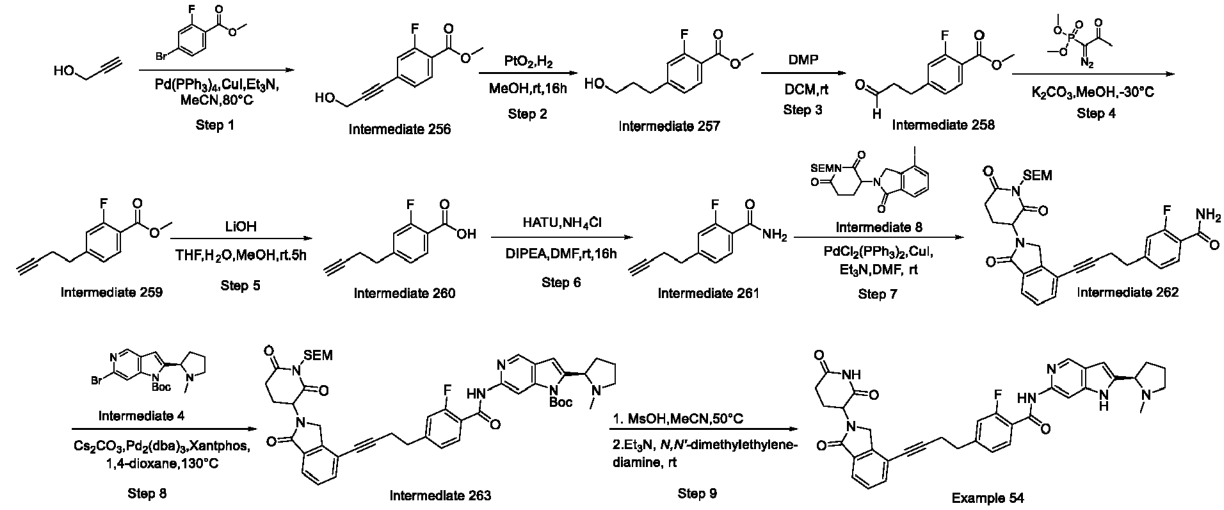


## Scheme 52

**Step** 1

**Intermediate 256: methyl 2-fluoro-4-(3-hydroxyprop-1-yn-1-yl)benzoate**

To a solution of methyl 4-bromo-2-fluorobenzoate (CAS No. 179232-29-2, 25.0 g, 107.28 mmol) in acetonitrile (250 mL) were added copper(I) iodide (2.08 g, 10.72 mmol), $PdCl_2(PPh_3)_2$ (7.60 g, 10.72 mmol), prop-2-yn-1-ol (CAS No. 107-19-7, 12.03 g, 214.56 mmol) and triethylamine (60.1 mL, 429.13 mmol). The reaction mixture was purged with argon for 15 min. The reaction mixture was heated at 80°C for 16h. Progress of the reaction was monitored by TLC. After completion, the reaction mixture was concentrated *in vacuo.* The reaction mixture was diluted with aqueous saturated sodium thiosulphate solution (200 mL) and extracted with EtOAc (2 × 200 mL). The organic layer was seperated, dried over anhydrous $Na_2SO_4$ and concentrated *in vacuo* to obtain crude material. The crude material was purified by combi-flash column chromatography, by eluting with 30% EtOAc in heptane, to afford methyl 2-fluoro-4-(3-hydroxyprop-1-yn-1-yl)benzoate (**Intermediate 256**, 18.0 g, 81%) as a yellow solid.

$^1$H NMR (400 MHz, DMSO-$d_6$) δ ppm 3.82 (s, 3H), 4.30 (d, *J*=6.14 Hz, 2H), 5.40 (t, *J*=5.92 Hz, 1H), 7.30-7.40 (m, 2H), 7.83-7.84 (m, 1H).

**Step 2**

**Intermediate 257: methyl 2-fluoro-4-(3-hydroxypropyl)benzoate**

To a solution methyl 2-fluoro-4-(3-hydroxyprop-1-yn-1-yl)benzoate (**Intermediate 256**, 17.0 g, 81.66 mmol) in methanol (400 mL) was added platinum oxide (1.85 g, 8.16 mmol). The reaction mixture was stirred at room temperature for 16h under $H_2$ atmosphere at 50 psi. Progress of the reaction was monitored by TLC. After completion of reaction, the reaction mixture was filtered through a celite bed and washed with MeOH (100 mL). The solvent was evaporated under reduced pressure to obtain the crude material. This material was purified by combi-flash column chromatography, by eluting with 30% EtOAc in heptane, to afford methyl 2-fluoro-4-(3-hydroxypropyl) benzoate (**Intermediate 257**, 10.50 g, 61%) as a yellow liquid. $^1$H NMR (400 MHz, DMSO-$d_6$) δ ppm 1.70-1.76 (m, 2H), 2.68 (t, *J*=7.45 Hz, 2H), 3.38-2.42 (m, 2H), 3.83 (s, 3H), 4.50 (t, *J*=4.60 Hz, 1H), 7.15-7.20 (m, 2H), 7.80-7.81 (m, 1H). Mass spec: m/z 213.0 $[M+H]^+$.

**Step 3**

**Intermediate 258: methyl 2-fluoro-4-(3-oxopropyl)benzoate**

To a solution of methyl 2-fluoro-4-(3-hydroxypropyl)benzoate (**Intermediate 257**, 10.0 g, 47.1 mmol) in dichloromethane (100 mL) was added Dess-Martin periodinane (30.0 g, 70.7 mmol) at 0°C. The reaction mixture was stirred at room temperature for 2h. Progress of the reaction was monitored by TLC. After completion, the reaction mixture filtered through a celite bed. The reaction mixture was quenched with $NaHCO_3$ solution (200 mL) and extracted with DCM (3 × 200 mL). The organic layer was separated, dried over anhydrous $Na_2SO_4$ and concentrated *in vacuo.* The crude material was purified by combi-flash column chromatography, by eluting with 30% EtOAc in heptane, to afford methyl 2-fluoro-4-(3-oxopropyl)benzoate (**Intermediate 258**, 8.5 g, 85%) as a yellow liquid. $^1$H NMR (400 MHz, DMSO-$d_6$) δ ppm 2.81-2.88 (m, 4H), 3.80 (s, 3H), 7.15-7.22 (m, 2H), 7.76-7.77 (m, 1H), 9.67 (s, 1H).

**Step 4**

**Intermediate 259: methyl 4-(but-3-yn-1-yl)-2-fluorobenzoate**

To a solution of methyl 2-fluoro-4-(3-oxopropyl) benzoate (**Intermediate 258**, 8.5 g, 35.7 mmol) in methanol (100 mL) was added potassium carbonate (6.7 g, 49 mmol) at - 30°C and the reaction mixture was stirred for 10 min, to this mixture dimethyl (1-diazo-2-oxopropyl) phosphonate (13.0 g, 61.0 mmol) was added dropwise at -30° and the reaction mixture was stirred at same temperature for 3h. Progress of the reaction was monitored by TLC. After completion, the reaction mixture was diluted with water (100 mL) and extracted with EtOAc (3 × 150 mL). The combined organic layer was separated, dried over anhydrous $Na_2SO_4$ and concentrated *in vacuo.* The crude material was purified by combi-flash column chromatography, by eluting with 10% EtOAc in heptane, to afford methyl 4-(but-3-yn-1-yl)-2-fluorobenzoate (**Intermediate 259**, 5.70 g, 68%) as a yellow liquid. $^1$H NMR (400 MHz, DMSO-$d_6$) δ ppm 2.49-2.53 (m, 2H), 2.79-2.83 (m, 2H), 2.85 (s, 1H), 3.84 (s, 3H), 7.21-7.28 (m, 2H), 7.81-7.82 (m, 1H).

**Step 5**

**Intermediate 260: 4-(but-3-yn-1-yl)-2-fluorobenzoic acid**

To a solution of methyl 4-(but-3-yn-1-yl)-2-fluorobenzoate (**Intermediate 259**, 5.70 g, 27.6 mmol) in tetrahydrofuran (30 mL) and methanol (30 mL) was added lithium hydroxide (2.03 g, 82.9 mmol) in water (15 mL) and the reaction mixture was stirred at room temperature for 5h. Progress of the reaction was monitored by TLC. After completion, the reaction mixture was concentrated *in vacuo.* The reaction mixture was diluted with water (10 mL), the reaction mixture was acidified to pH~2 by adding citric acid solution to obtain a white solid. This was filtered and dried to afford 4-(but-3-yn-1-yl)-2-fluorobenzoic acid (**Intermediate 260**, 4.7 g, 88%) as a white solid. $^1$H NMR (400 MHz, DMSO-$d_6$) δ ppm 2.51-2.54 (m, 2H), 2.78-2.82 (m, 2H), 2.84 (s, 1H), 7.17-7.23 (m, 2H), 7.78-7.79 (m, 1H), 13.07 (br s, 1H). Mass spec: m/z 190.9 [M-H]$^+$.

**Step 6**

**Intermediate 261: 4-(but-3-yn-1-yl)-2-fluorobenzamide**

To a solution of 4-(but-3-yn-1-yl)-2-fluorobenzoic acid (**Intermediate 260**, 4.7 g, 24 mmol) in DMF (50 mL) was added diisopropylethylamine (9.5 g, 73 mmol) and HATU (14.0 g, 37 mmol) followed by ammonium chloride (6.6 g, 120 mmol). The reaction mixture was stirred at room temperature for 16h. Progress of the reaction was monitored by TLC. After completion, the reaction mixture was poured in ice cold water (200 mL) and extracted with EtOAc (3 × 100 mL). The organic layer was separated, dried over anhydrous $Na_2SO_4$ and concentrated *in vacuo* to afford 4-(but-3-yn-1-yl)-2-fluorobenzamide (**Intermediate 261**, 3.5 g, 75%) as a white solid which was used for the next step without further purification. Mass spec: m/z 192.0 [M+H]$^+$.

**Step 7**

**Intermediate 262: 4-(4-(2-(2,6-dioxo-1-((2-(trimethylsilyl) ethoxy)methyl) piperidin-3-yl)-1-oxoisoindolin-4-yl) but-3-yn-1-yl)-2-fluorobenzamide**

To a solution of 4-(but-3-yn-1-yl)-2-fluorobenzamide (**Intermediate 261**, 1.0 g, 5.2 mmol) in DMF (10 mL) was added copper(I) iodide (0.10 g, 0.52 mmol), $PdCl_2(PPh_3)_2$ (0.37 g, 0.52 mmol), 3-(4-iodo-1-oxoisoindolin-2-yl)-1-((2-(trimethylsilyl)ethoxy)methyl)piperidine-2,6-dione (**Intermediate 8**, 2.6 g, 5.2 mmol) and triethylamine (26 mL, 180 mmol). The reaction mixture was purged with argon for 15 min. The reaction mixture was stirred at room temperature for 2h. Progress of the reaction was monitored by TLC. After completion of the reaction, aqueous saturated sodium thiosulphate solution (100 mL) was added and extracted with EtOAc (2 × 100 mL). The organic layer was separated, dried over anhydrous $Na_2SO_4$ and concentrated *in vacuo.* The crude material was purified by combi-flash column chromatography, by eluting with 70% EtOAc in heptane, to afford 4-(4-(2-(2, 6-dioxo-1-((2-(trimethylsilyl)ethoxy)methyl)piperidin-3-yl)-1-oxoisoindolin-4-yl)but-3-yn-1-yl)-2-fluorobenzamide (**Intermediate 262**, 1.20 g, 41%) as a yellow solid. $^1$H NMR (400 MHz, DMSO-$d_6$) δ ppm -0.03 (s, 9H), 0.84-0.88 (m, 2H), 1.98-2.06 (m, 1H), 2.36-2.40 (m, 1H), 2.77-2.87 (m, 4H), 2.87-2.96 (m, 2H), 3.50-3.56 (m, 2H), 4.10-4.15 (m, 1H), 4.25-4.30 (m, 1H), 5.03-5.10 (m, 2H), 5.21-5.26 (m, 1H), 7.20-7.30 (m, 3H),

7.48-7.61 (m, 3H), 7.71 (br s, 1H), 7.95 (br s, 1H). Mass spec: m/z 562.1 [M-H]+.

**Step 8**

**Intermediate 263: *tert*-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)-2-fluorobenzamido)-2-((R)-1-methylpyrrolidin-2-yl)-1*H*-pyrrolo[3,2-c]pyridine-1-carboxylate**

To a solution of 4-(4-(2-(2,6-dioxo-1-((2-(trimethylsilyl) ethoxy) methyl)piperidin-3-yl)-1-oxoisoindolin-4-yl) but-3-yn-1-yl)-2-fluorobenzamide (**Intermediate 262**, 0.6 g, 1.0 mmol) in 1,4-dioxane (20 mL) was added tert-butyl (R)-6-bromo-2-(1-methylpyrrolidin-2-yl)-1*H*-pyrrolo[3,2-c]pyridine-1-carboxylate (**Intermediate 4,** 0.5 g, 1.0 mmol) followed by cesium carbonate (1.0 g, 3.0 mmol). The reaction mixture was purged with argon for 10 min. then added $Pd_2(dba)_3$ (0.2 g, 0.2 mmol) and xantphos (0.2 g, 0.3 mmol) at room temperature. The reaction mixture was purged with argon for 10 min and heated at 130°C for 2h. Progress of the reaction was monitored by TLC. After completion, the reaction mixture was concentrated *in vacuo.* The crude material was purified by combi-flash column chromatography, by eluting with 80% EtOAc in heptane, to afford tert-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)-2-fluorobenzamido)-2-((R)-1-methylpyrrolidin-2-yl)-1*H*-pyrrolo[3,2-c]pyridine-1-carboxylate (**Intermediate 263**, 0.3 g, 30%) as a yellow solid. $^1$H NMR (400 MHz, DMSO-$d_6$) δ ppm -0.07 (s, 9H), 0.73-0.79 (m, 2H), 1.16-1.19 (m, 1H), 1.59-1.65 (m, 3H), 1.70 (s, 9H), 2.35 (s, 3H), 2.72-2.76 (m, 2H), 2.83-2.89 (m, 4H), 2.93-3.01 (m, 4H), 3.42-3.46 (m, 2H), 3.90-3.94 (m, 1H), 4.11-4.15 (m, 1H), 4.31-4.36 (m, 1H), 4.94-5.04 (m, 2H), 5.21-5.26 (m, 1H), 6.74 (s, 1H), 7.27-7.38 (m, 2H), 7.49-7.54 (m, 2H), 7.61-7.72 (m, 2H), 8.55 (s, 1H), 8.92 (s, 1H), 10.56 (br s, 1H). Mass spec: m/z 863.1 [M+H]+.

**Step 9**

**Example 54: 4-(4-(2-(2, 6-dioxopiperidin-3-yl)-1-oxoisoindolin-4-yl) but-3-yn-1-yl)-2-fluoro-*N*-(2-((R)-1-methylpyrrolidin-2-yl)-1*H*-pyrrolo [3, 2-c] pyridin-6-yl) benzamide**

To a solution of tert-butyl 6-(4-(4-(2-(2,6-dioxo-1-((2-(trimethylsilyl)ethoxy)methyl)piperidin-3-yl)-1-oxoisoindolin-4-yl)but-3-yn-1-yl)-2-fluorobenzamido)-2-((*R*)-1-methylpyrrolidin-2-yl)-1*H*-pyrrolo[3,2-c]pyridine-1-carboxylate (**Intermediate 263**, 0.5 g, 0.6 mmol) in acetonitrile (8 mL) was added methanesulfonic acid (0.6 mL, 9.0 mmol) at room temperature and the reaction mixture was heated at 50°C for 2h. To the reaction mixture triethylamine (2.0 mL, 10 mmol) and *N*, *N*-dimethylethylenediamine (0.4 mL, 3.0 mmol) were added at room temperature and stirred for 3h. Progress of the reaction was monitored by TLC. After completion, the reaction mixture was poured into ice cold water, a white solid precipitated, which was filtered and dried to obtain crude material. The crude was purified by preparative HPLC (Method A) to afford 4-(4-(2-(2,6-dioxopiperidin-3-yl)-1-oxoisoindolin-4-yl)but-3-yn-1-yl)-2-fluoro-N-(2-((R)-1-methylpyrrolidin-2-yl)-1H-pyrrolo[3,2-c]pyridin-6-yl)benzamide (**Example 54**, 0.1 g, 30%) as an off white solid. $^1$H NMR (400 MHz, DMSO-$d_6$) δ ppm 1.77-1.90 (m, 3H), 1.99-2.03 (m, 1H), 2.10-2.14 (m, 1H), 2.16 (s, 3H), 2.23-2.29 (m, 1H), 2.39-2.46 (m, 1H), 2.56-2.60 (m, 1H), 2.86-2.91 (m, 3H), 2.93-3.02 (m, 2H), 3.12-3.16 (m, 1H), 3.28-3.30 (m, 1H), 4.21-4.26 (m, 1H), 4.32-4.36 (m, 1H), 5.11-5.16 (m, 1H), 6.40 (s, 1H), 7.30-7.31 (m, 1H), 7.35-7.36 (m, 1H), 7.50-7.53 (m, 1H), 7.61-7.62 (m, 1H), 7.69-7.73 (m, 2H), 8.19 (s, 1H), 8.46 (s, 1H), 10.23 (s, 1H), 11.14 (s, 1H), 11.40 (s, 1H). Mass spec: m/z 633.2 [M+H]+.

**Example 55 was synthesised following Scheme 53**

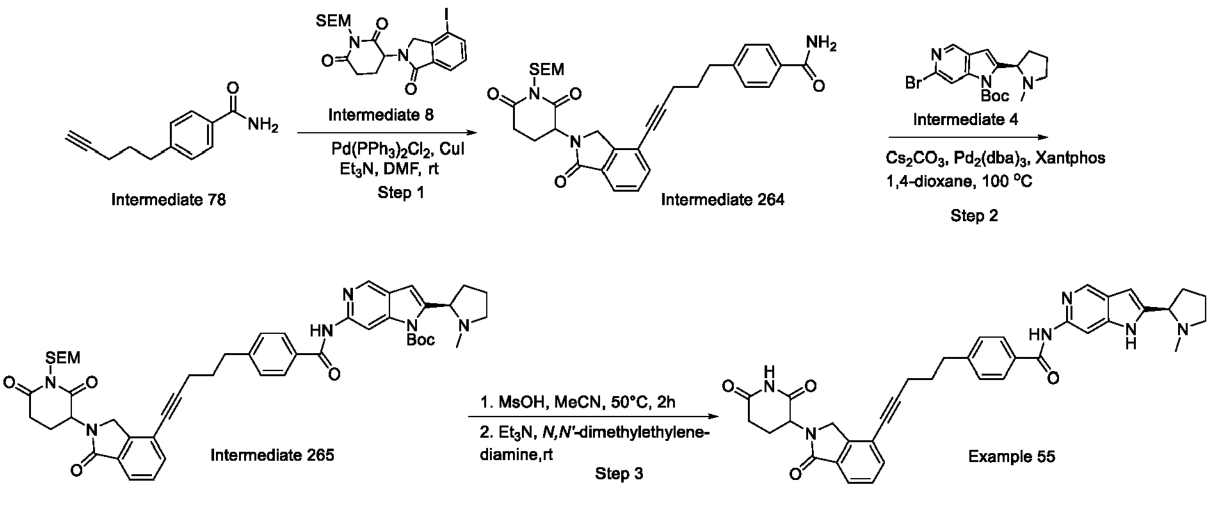


**Scheme 53**

**Step 1**

**Intermediate 264: 4-(5-(2-(2,6-dioxo-1-((2-(trimethylsilyl)ethoxy)methyl)piperidin-3-yl)-1-oxoisoindolin-4-yl) pent-4-yn-1-yl)benzamide**

To a solution of 4-(pent-4-yn-1-yl) benzamide (**Intermediate 78**, 1 g, 5.34 mmol) in DMF (15 mL), 3-(4-iodo-1-oxoisoindolin-2-yl)-1-((2-(trimethylsilyl)ethoxy)methyl)piperidine-2,6-dione (**Intermediate 8**, 2.67g, 5.37 mmol), triethylamine (20.1 g, 197.61 mmol), CuI (0.1 g, 0.534 mmol) and $PdCl_2(PPh_3)_2$ (0.38 g, 5.37 mmol) were added and the argon has was passed through the reaction mixture for 15 min. The reaction mixture was stirred at room temperature for 16h. Progress of reaction was monitored by TLC. After completion of reaction, the mixture was concentrated under reduced pressure to obtain the crude material which was purified by combi-flash column chromatography by eluting with 80% EtOAc in heptane to afford 4-(5-(2-(2,6-dioxo-1-((2-(trimethylsilyl)ethoxy)methyl)piperidin-3-yl)-1-oxoisoindolin-4-yl) pent-4-yn-1-yl)benzamide (**Intermediate 264**, 2.3 g, 77%) as green solid.$^1$H NMR (400 MHz, DMSO-$d_6$) δ ppm -0.08 (s, 9 H), 2.43-2.45 (m, 2H), 2.70 (s, 1H), 2.73-2.78 (m, 3H), 2.86 (s, 1H), 3.25-3.26 (m, 1H), 3.25-3.29 (m, 4H), 4.21-4.29 (m, 1H), 4.42-4.50 (m, 1H), 4.96-5.08 (m, 2H), 5.24 (dd, J=13.15, 4.38 Hz, 1H), 7.22 (br s, 1H), 7.22-7.27 (m, 2H), 7.47-7.54 (m, 1H), 7.60-7.72 (m, 2H), 7.77-7.80 (m, 2H), 7.84 (br s, 1H). Mass spec: m/z 558.1 $[M+H]^+$.

**Step 2**

**Intermediate 265: *tert*-butyl 6-(4-(5-(2-(2,6-dioxo-1-((2-(trimethylsilyl)ethoxy)methyl)piperidin-3-yl)-1-oxoisoindolin-4-yl)pent-4-yn-1-yl)benzamido)-2-((R)-1-methylpyrrolidin-2-yl)-1*H*-pyrrolo[3,2-c]pyridine-1-carboxylate**

To a solution of tert-butyl (R)-6-bromo-2-(1-methylpyrrolidin-2-yl)-1H-pyrrolo[3,2-c]pyridine-1-carboxylate (**Intermediate 4**, 1.0 g, 2.60 mmol) in 1,4-dioxane (20 mL) was added cesium carbonate (2.60 g, 7.90 mmol) and 4-(5-(2-(2,6-dioxo-1-((2-(trimethylsilyl)ethoxy)methyl)piperidin-3-yl)-1-oxoisoindolin-4-yl)pent-4-yn-1-yl)benzamide (**Intermediate 264**, 1.16 g, 2.08 mmol). The reaction mixture was degassed with argon gas for 10 min then added xantphos (0.47 g, 0.79 mmol) and $Pd_2(dba)_3$ (0.37 g, 0.39 mmol) and the reaction mixture was degassed with argon gas for 10 min and heated at 130°C for 2h. Progress of reaction was monitored by TLC. After completion of reaction, the reaction mixture was concentrated under reduced pressure to obtain the crude compound which was purified by combi-flash column chromatography by eluting with 80% EtOAc in heptane to afford *tert*-butyl 6-(4-(5-(2-(2,6-dioxo-1-((2-(trimethylsilyl) ethoxy)methyl)piperidin-3-yl)-1-oxoisoindolin-4-yl)pent-4-yn-1-yl)benzamido)-2-((R)-1-methylpyrrolidin-2-yl)-1H-pyrrolo[3,2-c]pyridine-1-carboxylate (**Intermediate 265**, 0.55 g, 24%) as a yellow solid. $^1$H NMR (400 MHz, DMSO-$d_6$) δ ppm -0.05 (s, 9H), 0.70-0.81 (m, 2H), 1.54-1.65 (m, 3H), 1.68 (s, 9H), 1.69-1.70 (m, 2H), 1.92-2.07 (m, 3H), 2.33 (s, 3H), 2.34-2.36 (m, 2H), 2.74-2.88 (m, 3H), 3.01-3.06 (m, 3H), 3.51 (m, 2H), 3.92 (m, 1H), 4.27-4.92 (m, 2H), 5.01-5.08 (m, 2H), 5.26-5.28 (m, 1H), 6.73-6.74(m, 1H), 7.36-7.38 (m, 2H), 7.52-7.54 (m, 1H), 7.67-7.73 (m, 2H), 7.98-8.00 (m, 2H), 8.58 (s, 1H), 8.92 (s,1H), 10.58 (s, 1H). Mass spec: m/z 859.2 $[M+H]^+$.

**Step 3**

**Example 55: 4-(5-(2-(2,6-dioxopiperidin-3-yl)-1-oxoisoindolin-4-yl)pent-4-yn-1-yl)-N-(2-((R)-1-methylpyrrolidin-2-yl)-1*H*-pyrrolo[3,2-c]pyridin-6-yl)benzamide**

To a stirred solution of tert-butyl 6-[[4-[5-[2-[2,6-dioxo-1-(2-trimethylsilylethoxymethyl)-3-piperidyl]-1-oxo-isoindolin-4-yl]pent-4-ynyl]benzoyl]amino]-2-[(2-(*R*))-1-methylpyrrolidin-2-yl]pyrrolo[3,2-c]pyridine-1-carboxylate (**Intermediate 265**, 0.5 g, 0.58 mmol) in acetonitrile (10 mL) was added methanesulfonic acid (0.85 g, 8.73 mmol) at room temperature. The reaction mixture was heated at 50°C for 2h then reaction mixture was allowed to cool to room and *N-N'*-dimethylethylenediamine (0.34 g, 3.49 mmol) and triethylamine (0.01 g, 11.64 mmol) were added. The reaction was stirred at room temperature for 3h and monitored by TLC. After completion of reaction, the mixture was concentrated under reduced pressure to get crude material which was purified by preparative HPLC (Method A) to afford 4-(5-(2-(2,6-dioxopiperidin-3-yl)-1-oxoisoindolin-4-yl)pent-4-yn-1-yl)-N-(2-((R)-1-methylpyrrolidin-2-yl)-1H-pyrrolo[3,2-c]pyridin-6-yl)benzamide (**Example 55**, 0.085 g, 23%) as off-white solid. $^1H$ NMR (400 MHz, DMSO-$d_6$) δ ppm 1.77-2.05 (m, 5H), 2.11-2.15 (m, 1H), 2.17 (s, 3H), 2.21-2.32 (m, 1H), 2.35-2.47 (m, 2H), 2.53-2.64 (m, 3H), 2.77-2.96 (m, 3H), 3.07-3.22 (m, 1H), 3.33-3.34 (m, 1H), 4.36-4.51 (m, 2H), 5.12-5.17 (m, 1H), 6.39 (s, 1H), 7.37-7.39 (m, 2H), 7.53-7.55 (m, 1H), 7.63-7.75 (m, 2H), 8.00-8.02 (m, 2H), 8.19 (s, 1H), 8.50 (s, 1H), 10.34 (s, 1H) 11.02 (s, 1H), 11.36 (s, 1H). Mass spec: m/z 629.0 $[M+H]^+$.

**Example 56 was synthesised following Scheme 54**

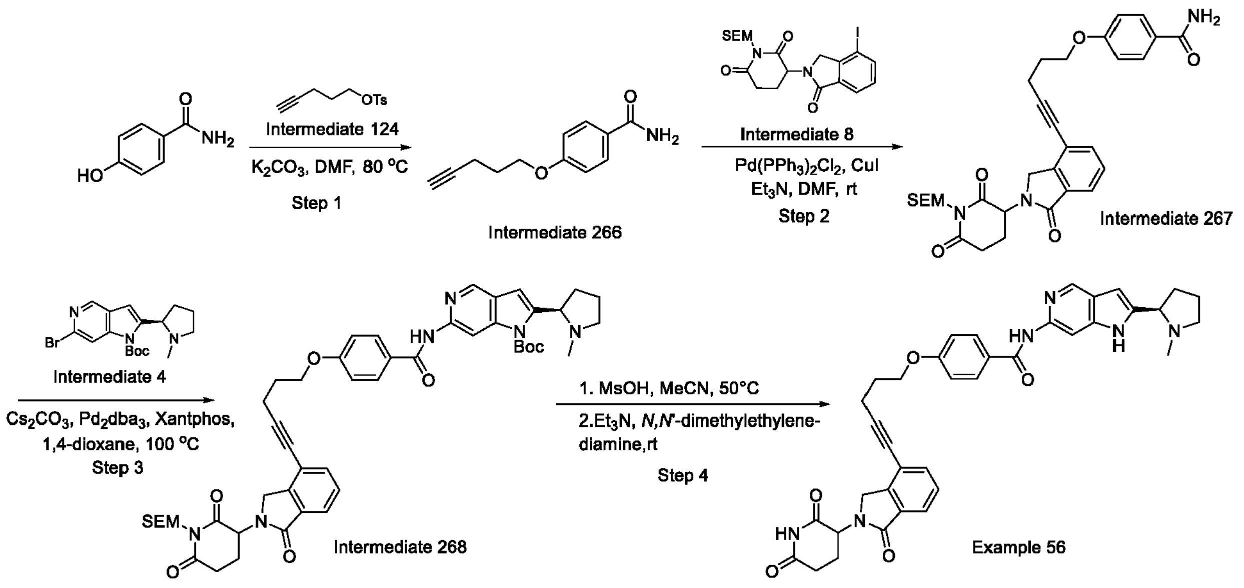


**Scheme 54**

**Step 1**

**Intermediate 266: 4-(pent-4-yn-1-yloxy)benzamide**

To a solution of 4-hydroxybenzamide (CAS No. 619-57-8, 1.2 g, 8.80 mmol) in DMF (10 mL) were added cesium carbonate (2.60 g, 7.90 mmol) and pent-4-ynyl 4-methylbenzenesulfonate (**Intermediate 124**, 2.50 g, 11.02 mmol) at room temperature. The reaction mixture was heated at 80°C for 16h. Progress of reaction was monitored by TLC. After completion of reaction, the mixture was poured into water (20 mL) and extracted with EtOAc (3 × 30 mL). The combined organic layer was separated and dried over $Na_2SO_4$, filtered and concentrated under reduced pressure to get the crude which was purified by combi-flash column chromatography eluted with 20% EtOAc in heptane to afford 4-(pent-4-yn-1-yloxy)benzamide (**Intermediate 266**, 1.1 g, 62%) as an off-white solid. $^1H$ NMR (400 MHz, DMSO-$d_6$) δ ppm 1.90-1.92 (m, 2H), 2.33-2.35 (m, 2H), 2.82 (s, 1H), 4.02-4.16 (m, 2H), 6.97 (d, J=8.13 Hz, 2H), 7.15 (br s, 1H), 7.83 (d, J=8.02 Hz, 2H), 7.84 (br s, 1H). Mass spec: m/z 204.1 $[M+H]^+$.

**Step 2**

**Intermediate 267: 4-((5-(2-(2,6-dioxo-1-((2-(trimethylsilyl)ethoxy)methyl)piperidin-3-yl)-1-oxoisoindolin-4-yl)pent-4-yn-1-yl)oxy)benzamide**

To a solution of 4-pent-4-ynoxybenzamide (**Intermediate 266**, 1 g, 4.90 mmol) and 3-(4-iodo-1-oxo-isoindolin-2-yl)-1-(2-trimethylsilylethoxymethyl)piperidine-2,6-dione (**Intermediate 8**, 2.50 g, 4.93 mmol) in DMF (10 mL) were added triethylamine (18 g, 180 mmol), $PdCl_2(PPh_3)_2$ (0.35 g, 0.49 mmol) and CuI (0.09 g, 0.49 mmol) and the reaction mixture was degassed with argon with 10 min. The reaction mixture was stirred at room temperature for 3h. Progress of reaction was monitored by TLC. After completion of reaction, the mixture was treated with water (60 mL) and extracted with EtOAc ($3 \times 60$ mL). The organic layer was separated and dried over $Na_2SO_4$, filtered and concentrated under reduced pressure to get the crude which was purified by combi-flash column chromatography by eluting with 80% EtOAc in heptane to afford 4-((5-(2-(2,6-dioxo-1-((2-(trimethylsilyl)ethoxy)methyl)piperidin-3-yl)-1-oxoisoindolin-4-yl)pent-4-yn-1-yl)oxy)benzamide (**Intermediate 267**, 1.50 g, 53%) as an off-white solid. $^1H$ NMR (400 MHz, DMSO-$d_6$) δ ppm -0.03 (s, 9H), 0.83-0.86 (m, 2H), 1.97-2.10 (m, 3H), 2.36-2.38 (m, 1H), 2.66-2.68 (m, 2H), 3.44-3.45 (m, 2H), 4.17 (s, 2H), 4.22-4.29 (m, 2H), 4.39-4.48 (m, 2H), 5.05-5.22 (m, 2H), 5.23-5.25 (m, 1H), 6.99-7.01 (m, 2H), 7.16 (br s, 1H), 7.53 (t, *J*=7.45 Hz, 1H), 7.65 (d, *J*=7.02 Hz, 1H), 7.72 (d, *J*=8.3 Hz, 2H), 7.79 (br s, 1H). Mass spec: m/z 574.4 $[M+H]^+$.

**Step 3**

**Intermediate 268: *tert*-butyl 6-(4-((5-(2-(2,6-dioxo-1-((2-(trimethylsilyl)ethoxy)methyl)piperidin-3-yl)-1-oxoisoindolin-4-yl)pent-4-yn-1-yl)oxy)benzamido)-2-((R)-1-methylpyrrolidin-2-yl)-1*H*-pyrrolo[3,2-c]pyridine-1-carboxylate**

To a solution of *tert*-butyl (R)-6-bromo-2-(1-methylpyrrolidin-2-yl)-1H-pyrrolo[3,2-c]pyridine-1-carboxylate (**Intermediate 4**, 0.80 g, 2.10 mmol) in 1,4-dioxane (15 mL) were added cesium carbonate (2 g, 6.30 mmol) and 4-((5-(2-(2,6-dioxo-1-((2-(trimethylsilyl)ethoxy)methyl)piperidin-3-yl)-1-oxoisoindolin-4-yl)pent-4-yn-1-yl)oxy)benzamide (**Intermediate 267**, 1 g, 2.01 mmol). The reaction was degassed with argon gas for 20 min followed by Xantphos (0.40 g, 0.60 mmol) and $Pd_2(dba)_3$ (0.30 g, 0.30 mmol) were added. The reaction mixture was again degassed with argon gas for 10 min. The resulting reaction mixture was heated at 100°C for 2h. Progress of reaction was monitored by TLC. After completion of reaction, the mixture was concentrated under reduced pressure to get crude which was purified by combi-flash column chromatography, by eluting with at 80% EtOAc in heptane, to obtain tert-butyl 6-(4-((5-(2-(2,6-dioxo-1-((2-(trimethylsilyl)ethoxy)methyl)piperidin-3-yl)-1-oxoisoindolin-4-yl)pent-4-yn-1-yl)oxy)benzamido)-2-((R)-1-methylpyrrolidin-2-yl)-1H-pyrrolo[3,2-c]pyridine-1-carboxylate (**Intermediate 268**, 0.52 g, 30%) as a green solid. Mass spec: m/z 875.5 $[M+H]^+$.

**Step 4**

**Example 56: 4-((5-(2-(2,6-dioxopiperidin-3-yl)-1-oxoisoindolin-4-yl)pent-4-yn-1-yl)oxy)-*N*-(2-((*R*)-1-methylpyrrolidin-2-yl)-1*H*-pyrrolo[3,2-c]pyridin-6-yl)benzamide**

To a solution of tert-butyl 6-(4-((5-(2-(2,6-dioxo-1-((2-(trimethylsilyl)ethoxy)methyl)piperidin-3-yl)-1-oxoisoindolin-4-yl)pent-4-yn-1-yl)oxy)benzamido)-2-((R)-1-methylpyrrolidin-2-yl)-1H-pyrrolo[3,2-c]pyridine-1-carboxylate (**Intermediate 268**, 0.50 g, 0.60 mmol) in acetonitrile (10 mL) was added methane sulfonic acid (0.80 g, 9 mmol) and heated the reaction mixture at 50°C for 2h. After 2h the reaction mixture was cooled to room temperature then added *N,N'*-dimethylethylenediamine (0.30 g, 3 mmol) and triethylamine (1.0 g, 10.04 mmol) at room temperature. The resulting reaction mixture was stirred for 3h at room temperature. Progress of reaction was monitored by TLC. After completion of reaction, the reaction mixture was concentrated under reduced pressure to obtain the crude product which was purified by preparative HPLC (Method A) to afford 4-((5-(2-(2,6-dioxopiperidin-3-yl)-1-oxoisoindolin-4-yl)pent-4-yn-1-yl)oxy)-N-(2-((*R*)-1-methylpyrrolidin-2-yl)-1H-pyrrolo[3,2-c]pyridin-6-yl)benzamide (**Example 56**, 0.11 g, 30%) as an off-white solid. $^1H$ NMR (400 MHz, DMSO-$d_6$) δ ppm 1.81-1.88 (m, 3H), 1.97-2.15 (m, 2H), 2.17 (s, 3H), 2.21- 2.46 (m, 3H), 2.58-2.62 (m, 2H), 2.64-2.73 (m, 2H), 2.81-3.00 (m, 1H), 3.08-3.28 (m, 2H), 4.22-4.24 (m, 2H), 4.32-4.44 (m, 2H), 5.10-5.13 (m, 1H), 6.38 (s, 1H), 7.06 (m, 2H), 7.49-7.56 (m, 1H), 7.66 (d, *J*=7.97 Hz, 1H), 7.71 (d, *J*=7.50 Hz, 1H), 8.04 (m, 2H), 8.17 (s, 1H), 8.49 (s, 1H), 10.26 (br s, 1H), 11.00 (br s, 1H), 11.34 (br s, 1H). Mass spec: m/z 645.1 $[M+H]^+$.

**Example 57 was synthesised following Scheme 55**

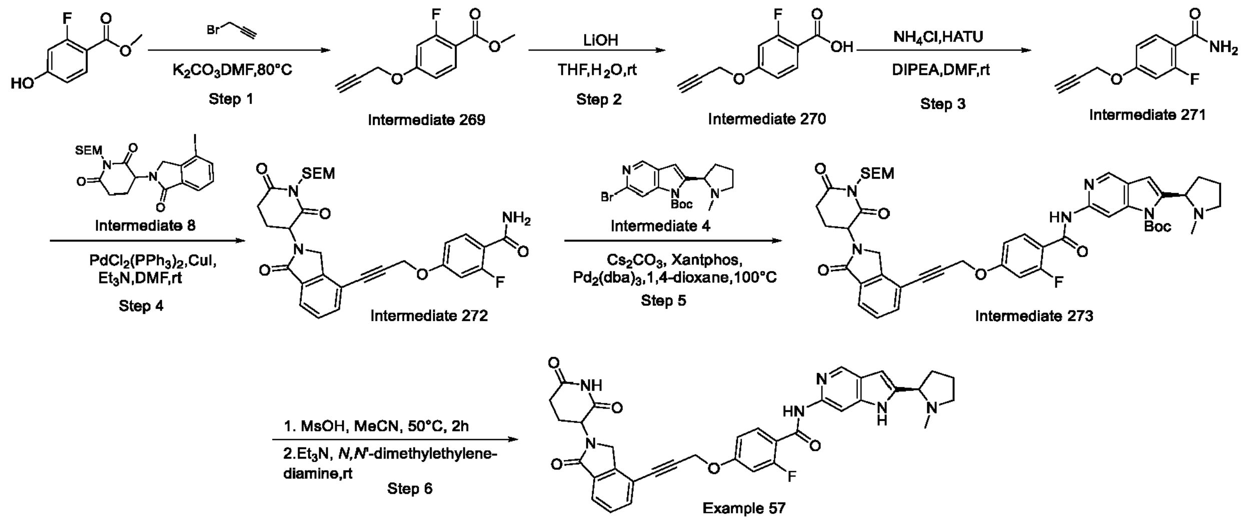


**Scheme 55**

**Step 1**

**Intermediate 269: methyl 2-fluoro-4-(prop-2-yn-1-yloxy)benzoate**

To a solution of methyl 2-fluoro-4-hydroxybenzoate (CAS No. 197507-22-5, 10.0 g, 58.77 mmol) in DMF (100 mL) was added potassium carbonate(12.3 g, 88.16 mmol) and 3-bromoprop-1-yne (10.48 g, 88.16 mmol). The reaction mixture was heated at 80°C for 12h. Progress of the reaction was monitored by TLC. After completion, the reaction mixture was diluted with water (100 mL) and extracted with EtOAc (2 × 100 mL). The organic layer was seprated, dried over anhydrous $Na_2SO_4$ and concentrated *in vacuo.* The crude material was purified by combi-flash column chromatography, by eluting with 50% EtOAc in heptane, to afford methyl 2-fluoro-4-(prop-2-yn-1-yloxy)benzoate (**Intermediate 269**, 8.0 g, 65%) as an off white solid. $^1$H NMR (400 MHz, $CDCl_3$) δ ppm 2.57 (s, 1H), 3.90 (s, 3H), 4.73 (s, 2H), 6.69-6.84 (m, 2H), 7.92-7.93 (m, 1H).

**Step 2**

**Intermediate 270: 2-fluoro-4-(prop-2-yn-1-yloxy) benzoic acid**

To a solution of methyl 2-fluoro-4-(prop-2-yn-1-yloxy) benzoate (**Intermediate 269**, 8.0 g, 3 8.42 mmol) in tetrahydrofuran (10 mL) and methanol (10 mL) was added lithium hydroxide (1.87 g, 76.85 mmol) in water (3 mL). Reaction mixture was stirred at room temperature for 12h. Progress of the reaction was monitored by TLC. The reaction mixture was concentrated *in vacuo.* The reaction mixture was diluted with water (25 mL). The pH of the aqueous layer was adjusted to 3-4 with 0.5 N HCl and extracted with EtOAc (2 × 100 mL). The organic layer was dried over $Na_2SO_4$ and concentrated *in vacuo.* The crude material was purified by combi-flash column chromatography, by eluting with 30% EtOAc in heptane, to to afford 2-fluoro-4-(prop-2-yn-1-yloxy) benzoic acid (**Intermediate 270**, 6.0 g, 80%) as an off white solid. $^1$H NMR (400 MHz, DMSO-$d_6$) δ ppm 3.65 (s, 1H), 4.92 (s, 2H), 6.89-6.97 (m, 2H), 7.85-7.86 (m, 1H), 12.94 (br s, 1H). Mass spec: m/z 194.9 $[M+H]^+$.

**Step 3**

**Intermediate 271: 2-fluoro-4-(prop-2-yn-1-yloxy)benzamide**

To a solution of 4-(but-3-yn-1-yl)-2-fluorobenzoic acid (**Intermediate 270**, 6.0 g, 0.03 mmol) in DMF (80 mL) was added diisopropylethylamine (22.0 mL, 0.12 mmol) and HATU (17.0 g, 0.046 mmol) followed by ammonium chloride (6.6 g, 6.12 mmol). The reaction mixture was stirred at room temperature for 12h. Progress of the reaction was monitored by TLC. After completion, the reaction mixture was diluted with water (200 mL) and extracted with EtOAc (3 × 100 mL). The organic layer was separated, dried over anhydrous $Na_2SO_4$ and concentrated *in vacuo.* The crude material was purified by combi-flash column chromatography, by eluting with 50% EtOAc in heptane, to afford 2-fluoro-4-(prop-2-yn-1-yloxy) benzamide (**Intermediate 271**, 5.0 g, 62%) as an off white solid. $^1$H NMR (400 MHz, DMSO-$d_6$) δ ppm 3.63 (s, 1H), 4.89 (s, 2H),

6.86-6.97 (m, 2H), 7.48 (br s, 2H), 7.67-7.68 (m, 1H). Mass spec: m/z 194.0 $[M+H]^+$.

**Step 4**

**Intermediate 272: 4-((3-(2-(2, 6-dioxo-1-((2-(trimethylsilyl) ethoxy) methyl) piperidin-3-yl)-1-oxoisoindolin-4-yl) prop-2-yn-1-yl) oxy)-2-fluorobenzamide**

To a solution of 2-fluoro-4-(prop-2-yn-1-yloxy) benzamide (**Intermediate 271**, 1.0 g, 4.78 mmol) in DMF (10 mL) was added copper(I) iodide (0.09 g, 0.478 mmol) followed by $PdCl_2(PPh_3)_2$ (0.34 g, 0.47 mmol), 3-(4-iodo-1-oxoisoindolin-2-yl)-1-((2-(trimethylsilyl)ethoxy)methyl)piperidine-2,6-dione (**Intermediate 8**, 2.39 g, 4.78 mmol) and triethylamine (23.4 mL, 167.00 mmol). The reaction mixture was purged with argon for 20 min. The reaction mixture was stirred at room temperature for 16h. Progress of the reaction was monitored by TLC. After completion, the reaction mixture was diluted with water (30 mL) and extracted with EtOAc (3 × 30 mL). The organic layer was separated, dried over anhydrous $Na_2SO_4$ and concentrated *in vacuo.* The crude material was purified by combi-flash column chromatography, by eluting with 95% EtOAc in heptane, to afford 4-((3-(2-(2, 6-dioxo-1-((2-(trimethylsilyl) ethoxy) methyl) piperidin-3-yl)-1-oxoisoindolin-4-yl) prop-2-yn-1-yl) oxy)-2-fluorobenzamide (**Intermediate 272**, 1.5 g, 55%) as a white solid. $^1H$ NMR (400 MHz, DMSO-$d_6$) δ ppm -0.03 (s, 9H), 0.81-0.86 (m, 2H), 2.03-2.08 (m, 1H), 2.29-2.39 (m, 2H), 2.78-2.82 (m, 1H), 3.03-3.12 (m, 1H), 3.50-3.57 (m, 2H), 4.18-4.39 (m, 2H), 5.19 (s, 2H), 5.19-5.21 (m, 2H), 6.96-7.04 (m, 2H), 7.44 (br s, 1H), 7.49 (br s, 1H), 7.57 (t, *J*=7.67 Hz, 1H), 7.69-7.73 (m, 2H), 7.79 (d, *J*=7.46 Hz, 1H).

**Step 5**

**Intermediate 273: *tert*-butyl-6-(4-((3-(2-(2,6-dioxo-1-((2-(trimethylsilyl)ethoxy)methyl)piperidin-3-yl)-1-oxoisoindolin-4-yl)prop-2-yn-1-yl)oxy)-2-fluorobenzamido)-2-((*R*)-1-methylpyrrolidin-2-yl)-1*H*-pyrrolo[3,2-c]pyridine-1-carboxylate**

To a solution of 4-((3-(2-(2, 6-dioxo-1-((2-(trimethylsilyl) ethoxy) methyl) piperidin-3-yl)-1-oxoisoindolin-4-yl) prop-2-yn-1-yl) oxy)-2-fluorobenzamide (**Intermediate 272**, 0.7 g, 1.23 mmol) in 1, 4-dioxane (10 mL) was added tert-butyl (R)-6-bromo-2-(1-methylpyrrolidin-2-yl)-1*H*-pyrrolo[3,2-c]pyridine-1-carboxylate (**Intermediate 4**, 0.47 g, 1.23 mmol) followed by cesium carbonate (1.21 g, 3.71 mmol). The reaction mixture was purged with argon for 20 min then added $Pd_2(dba)_3$ (0.25 g, 0.27 mmol) and Xantphos (0.22 g, 0.37 mmol). The reaction mixture was again purged with argon for 10 min and heated at 100°C for 2h. Progress of the reaction was monitored by TLC. After completion, the reaction mixture was diluted with water (100 mL), extracted with EtOAc (3 × 100 mL). The organic layer was separated, dried over anhydrous $Na_2SO_4$ and concentrated *in vacuo.* The crude material was purified by combi-flash column chromatography, by eluting with 50% EtOAc in heptane, to afford tert-butyl 6-(4-((3-(2-(2,6-dioxo-1-((2-(trimethylsilyl)ethoxy)methyl)piperidin-3-yl)-1-oxoisoindolin-4-yl)prop-2-yn-1-yl)oxy)-2-fluorobenzamido)-2-((*R*)-1-methylpyrrolidin-2-yl)-1*H*-pyrrolo[3,2-c]pyridine-1-carboxylate (**Intermediate 273**, 0.5 g, 47%) as an off white solid. $^1H$ NMR (400 MHz, DMSO-$d_6$) δ ppm -0.07 (s, 9H), 0.74-0.80 (m, 2H), 1.15-1.19 (m, 1H), 1.69 (s, 9H), 1.72-1.79 (m, 2H), 2.03-2.11 (m, 1H), 2.30-2.33 (m, 1H), 2.35 (s, 3H), 2.36-2.43 (m, 2H), 2.72-2.80 (m, 1H), 3.04-3.16 (m, 2H), 3.42-3.48 (m, 2H), 3.90-3.94 (m, 1H), 4.18-4.22 (m, 1H), 4.40-4.44 (m, 1H), 4.96-5.04 (m, 2H), 5.24 (s, 2H), 5.28-5.29 (m, 1H), 6.74 (s, 1H), 7.03 (d, J=8.68, 1H), 7.11-7.12 (m, 1H), 7.52-7.64 (m, 1H), 7.72-7.81 (m, 3H), 8.55 (s, 1H), 8.92 (s, 1H), 10.36 (s, 1H). Mass spec: m/z 865.1 $[M]^+$.

**Step 6**

**Example 57: 4-((3-(2-(2,6-dioxopiperidin-3-yl)-1-oxoisoindolin-4-yl)prop-2-yn-1-yl)oxy)-2-fluoro-*N*-(2-((R)-1-methylpyrrolidin-2-yl)-1*H*-pyrrolo[3,2-c]pyridin-6-yl)benzamide**

To a solution of *tert*-butyl 6-(4-((3-(2-(2,6-dioxo-1-((2-(trimethylsilyl)ethoxy)methyl)piperidin-3-yl)-1-oxoisoindolin-4-yl)prop-2-yn-1-yl)oxy)-2-fluorobenzamido)-2-((*R*)-1-methylpyrrolidin-2-yl)-1*H*-pyrrolo[3,2-c]pyridine-1-carboxylate (**Intermediate 273**, 0.5 g, 0.57 mmol) in acetonitrile (10 mL) was added methane sulfonic acid (0.57 mL, 8.67 mmol) at room temperature and the reaction mixture was heated at 50°C for 2h. To the reaction mixture was added triethylamine (0.88 g, 8.67 mmol) followed by *N, N*-dimethylethylenediamine (0.41 mL, 3.46 mmol) and the reaction mixture was stirred at room temperature for 3h. Progress of the reaction was monitored by TLC. After completion, the reaction mixture was poured into ice cold water, a soli precipitated and this was filtered, and dried to obtain the crude compound. The crude material was purified by preparative HPLC (Method A) to afford 4-((3-(2-(2,6-dioxopiperidin-3-yl)-1-oxoisoindolin-4-yl) prop-2-yn-1-yl)oxy)-2-fluoro-*N*-(2-((*R*)-1-methylpyrrolidin-2-yl)-1*H*-pyrrolo[3,2-c]pyridin-6-yl)benzamide (**Example 57**, 0.05 g, 14%) as an off white solid. $^1H$ NMR (400 MHz, DMSO-$d_6$) δ ppm 1.77-1.94 (m, 3H), 2.02-2.05 (m, 1H), 2.12-2.14 (m, 1H), 2.16 (s, 3H), 2.22-2.29 (m, 1H), 2.39-2.43 (m, 1H), 2.59-2.63 (m, 1H), 2.88-2.97 (m, 1H),

3.12-3.16 (m, 1H), 3.28-3.30 (m, 1H), 4.29-4.33 (m, 1H), 4.43-4.48 (m, 1H), 5.16-5.20 (m, 1H), 5.25 (s, 2H), 6.40 (s, 1H), 7.04-7.05 (m, 1H), 7.14-7.15 (m, 1H), 7.57-7.58 (m, 1H), 7.74 (d, *J*=7.30 Hz, 1H), 7.77-7.82 (m, 2H), 8.20 (s, 1H), 8.47 (s, 1H), 10.03 (br s, 1H), 11.28 (br s, 1H), 11.41 (br s, 1H). Mass spec: m/z 633.0 $[M-H]^+$.

**Example 58 was synthesised following Scheme 56**

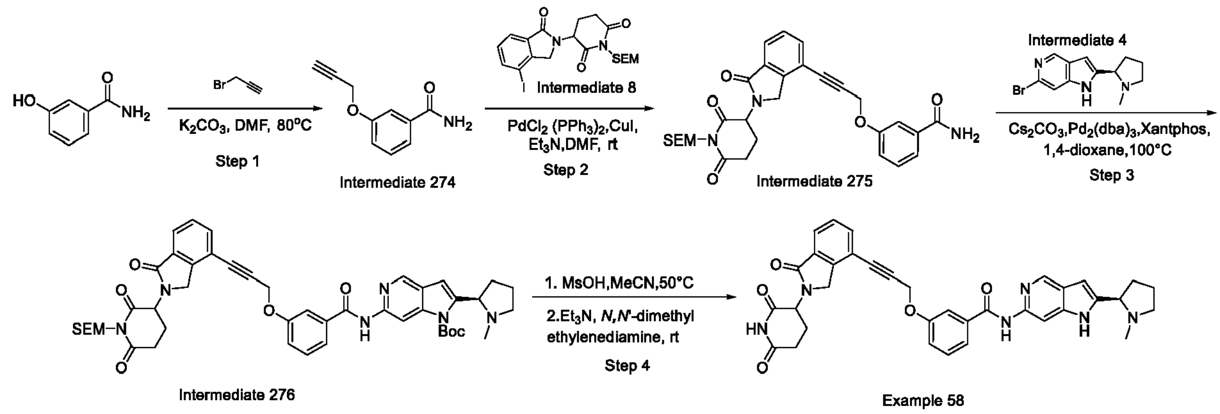


**Scheme 56**

**Step 1**

**Intermediate 274: 3-(prop-2-yn-1-yloxy)benzamide** To a solution of 3-hydroxybenzamide (CAS No: 618-49-5, 5 g, 36.12 mmol) in DMF (10 mL) was added potassium carbonate (7.60 g, 55 mmol) and bromoprop-1-yne (CAS No: 106-96-7, 4.3 g, 36.10 mmol) at room temperature. The resulting reaction mixture was heated at 80°C for 16h. The reaction mixture was then treated with water (100 mL) and extracted with EtOAc (2 × 100 mL). The organic layer was separated and dried over $Na_2SO_4$, filtered and concentrated under reduced pressure to afford the crude compound which was purified by using combi-flash column chromatography, eluting with 60% EtOAc in heptane, to afford 3-(prop-2-yn-1-yloxy)benzamide (**Intermediate 274**, 4.80 g, 75%) as an off-white solid. $^1H$ NMR (400 MHz, DMSO-$d_6$) δ ppm 7.13 (d, *J*=8 Hz, 1H), 7.35-7.39 (m, 2H), 7.48 (br s, 1H), 7.49 (d, J=8.10 Hz, 1H), 7.93 (s, 1H). Mass spec: m/z 175.1 $[M+H]^+$.

**Step 2**

**Intermediate 275: 3-((3-(2-(2, 6-dioxo-1-((2-(trimethylsilyl)ethoxy)methyl)piperidin-3-yl)-1-oxoisoindolin-4-yl) prop-2-yn-1-yl)oxy) benzamide**

To a solution of 3-(prop-2-yn-1-yloxy)benzamide (**Intermediate 274**, 1.2 g, 6.90 mmol) and 3-(4-iodo-1-oxoisoindolin-2-yl)-1-((2-(trimethylsilyl)ethoxy)methyl)piperidine-2,6-dione (**Intermediate 8**, 3.40 g, 6.90 mmol) in DMF (10 mL) was added triethylamine (25 g, 250 mmol) at room temperature followed by the addition of CuI (0.13 g, 0.69 mmol) an$PdCl_2(PPh_3)_2$ (0.49 g, 0.69 mmol). The reaction mixture was degassed for 5 minutes with argon. The resulting reaction mixture was then stirred at room temperature for 3h. The reaction was then treated with water (50 mL) and extracted with EtOAc (3 × 60 mL). The organic layers were separated and dried over $Na_2SO_4$, filtered and concentrated under reduced pressure to afford the crude compound which was purified by combi-flash column chromatography by eluting with 80% EtOAc in heptane to afford 3-((3-(2-(2, 6-dioxo-1-((2-(trimethylsilyl)ethoxy)methyl) piperidin-3-yl)-1-oxoisoindolin-4-yl)prop-2-yn-1-yl)oxy)benzamide (**Intermediate 275**, 1.30 g, 35%) as a green solid. $^1H$ NMR (400 MHz, DMSO-$d_6$) δ ppm -0.14-0.10 (m, 9H), 0.82-0.86 (m, 2H), 2.30-2.35 (m, 1H), 2.80-2.84 (m, 1H), 3.04-3.07 (m, 1H), 3.51-3.56 (m, 2H), 4.02-4.03 (m, 1H), 4.10-4.14 (m, 1H), 4.28-4.33 (m, 1H), 5.05-5.12 (m, 2H), 5.16 (s, 2H), 5.21-5.26 (m, 1H), 7.20 (d, *J*=8.10 Hz, 1H), 7.32 (s, 1H), 7.37-7.40 (m, 1H), 7.48-7.62 (m, 3H), 7.71 (d, *J*=8.12 Hz, 1H), 7.78 (d, *J*=7.46Hz, 1H), 7.97 (s, 1H). Mass spec: m/z 546 $[M+H]^+$.

**Step 3**

**Intermediate 276: *tert*-butyl 6-(3-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((R)-1-methylpyrrolidin-2-yl)-1*H*-pyrrolo[3,2-c]pyridine-1-carboxylate**

To a solution of tert-butyl (R)-6-bromo-2-(1-methylpyrrolidin-2-yl)-1H-pyrrolo[3,2-c]pyridine-1-carboxylate (**Intermediate 4,** 1 g, 2.6 mmol) and 3-((3-(2-(2, 6-dioxo-1-((2-(trimethylsilyl) ethoxy) methyl)piperidin-3-yl)-1-oxoisoindolin-4-yl) prop-2-yn-1-yl) oxy)benzamide (**Intermediate 275**, 0.80 g, 1 mmol) in 1,4-dioxane (20 mL) was added caesium carbonate (2 g, 6 mmol) and the resulting reaction mixture was degassed with argon for 20 min followed. Xanthphos (0.30 g, 0.60 mmol) and $Pd_2(dba)_3$ (0.60 g, 0.60 mmol) were added and the reaction mixture was again degassed with argon for 10 min then heated at 100°C for 2h. The reaction mixture was concentrated under reduced pressure to get the crude material which was purified by combi-flash column chromatography by eluting with 80% EtOAc and heptane to afford tert-butyl 6-(3-((3-(2-(2,6-dioxo-1-((2-(trimethylsilyl)ethoxy)methyl)piperidin-3-yl)-1-oxoisoindolin-4-yl)prop-2-yn-1-yl)oxy) benzamido)-2-((R)-1-methylpyrrolidin-2-yl)-1*H*-pyrrolo[3,2-c]pyridine-1-carboxylate (**Intermediate 276**, 0.85 g, 50%) as a yellow solid. $^1$H NMR (400 MHz, DMSO-$d_6$) δ ppm -0.14-0.01 (m, 9H), 0.75-0.82 (m, 2H), 1.61-1.93 (m, 11H), 1.95-1.98 (m, 1H), 2.17-2.30 (m, 1H), 2.30-2.49 (m, 5H), 2.58-2.64 (m, 1H), 2.95-2.96 (m, 1H), 3.13-3.30 (m, 2H), 3.40-3.46 (m, 2H), 3.90-3.93 (m, 1H), 4.01-4.06 (m, 1H), 4.26-4.30 (m, 1H), 4.83-4.94 (m, 2H), 5.12-5.16 (m, 1H), 5.24 (s, 2H), 6.75 (s, 1H), 7.24-7.26 (m, 1H), 7.38-7.43 (m, 1H), 7.36-7.43 (m, 1H), 7.43-7.58 (m, 1H), 7.58-7.78 (m, 2H), 7.84 (s, 1H), 8.59 (s, 1H), 8.92 (s, 1H), 10.78 (s, 1H). Mass spec: m/z 847.1 $[M+H]^+$.

**Step 4**

**Example 58: 3-((3-(2-(2,6-dioxopiperidin-3-yl)-1-oxoisoindolin-4-yl)prop-2-yn-1-yl)oxy)-*N*-(2-((R)-1-methylpyrrolidin-2-yl)-1*H*-pyrrolo[3,2-c]pyridin-6-yl)benzamide**

To a solution of tert-butyl 6-(3-((3-(2-(2,6-dioxo-1-((2-(trimethylsilyl)ethoxy)methyl)piperidin-3-yl)-1-oxoisoindolin-4-yl)prop-2-yn-1-yl)oxy)benzamido)-2-((R)-1-methylpyrrolidin-2-yl)-1H-pyrrolo[3,2-c]pyridine-1-carboxylate (**Intermediate 276**, 0.60 g, 0.70 mmol) in acetonitrile (15 mL) was added methanesulfonic acid (0.69 g, 7.08 mmol) at room temperature. The reaction mixture was then heated to 50°C for 2h before cooling to room temperature. *N,N'*-dimethylethylenediamine (0.34 g, 3.54 mmol) and triethylamine (1.44 g, 14.20 mmol) were added and the resulting reaction mixture was stirred at room temperature for 3h. The reaction mixture was concentrated under reduced pressure to afford the crude compound which was purified by preparative HPLC (Method A) to afford 3-((3-(2-(2,6-dioxopiperidin-3-yl)-1-oxoisoindolin-4-yl)prop-2-yn-1-yl)oxy)-N-(2-((R)-1-methylpyrrolidin-2-yl)-1H-pyrrolo[3,2-c]pyridin-6-yl)benzamide (**Example 58**, 0.51 g, 19%) as an off-white solid. $^1$H NMR (400 MHz, DMSO-$d_6$) δ ppm 1.78-1.95 (m, 4H), 2.12-2.14 (m, 2H), 2.15 (s, 3H), 2.23-2.30 (m, 2H), 2.78-2.86 (m, 1H), 3.12-3.16 (m, 1H), 4.14-4.19 (m, 1H), 4.32-4.36 (m, 1H), 5.03-5.08 (m, 1H), 5.24 (s, 2H), 6.39 (s, 1H), 7.24-7.27 (m, 1H), 7.44 (t, *J*=8.10 Hz, 1H), 7.55 (t, *J*=8.10, 1H), 7.67-7.79 (m, 4H), 8.17 (s, 1H), 8.50 (s, 1H), 10.40 (s, 1H), 11.05 (s, 1H), 11.40 (s, 1H). Mass spec: m/z 617 $[M+H]^+$.

**Example 59 was synthesised following Scheme 57**

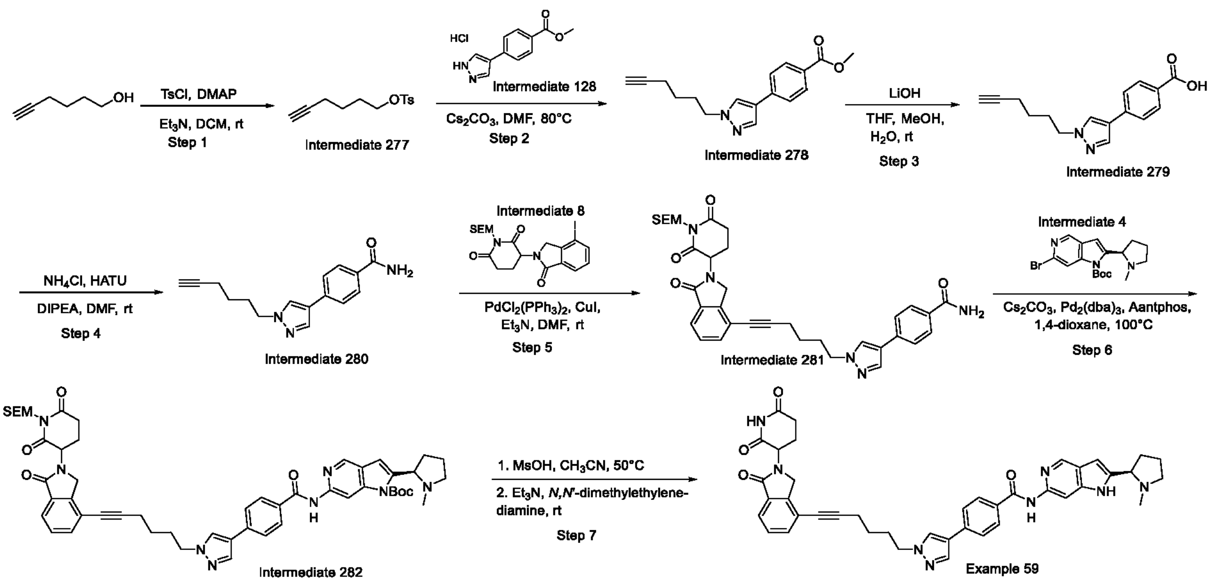


## Scheme 57

**Step 1**

**Intermediate 277: hex-5-yn-1-yl 4-methylbenzenesulfonate**

To a cooled (0°C) solution of hex-5-yn-1-ol (CAS No: 928-90-5, 5.00 g, 51 mmol) in dichloromethane (50 mL) was added p-toluenesulfonyl chloride (15.0 g, 76.0 mmol) followed by triethylamine (21 mL, 150 mmol) and 4-dimethylaminopyridine (0.66 g, 5.1 mmol). The reaction mixture was stirred at room temperature for 16h then diluted with water (25 mL) and extracted with dichloromethane (3 × 25 mL). The combined organic layers were separated, dried over anhydrous $Na_2SO_4$, and concentrated *in vacuo.* The crude material was purified by combi-flash chromatography, by eluting with 55% ethyl acetate in heptane, to afford hex-5-yn-1-yl 4-methylbenzenesulfonate (**Intermediate 277**, 7.00 g, 54%) as a yellow oil. $^1$H NMR (400 MHz, DMSO-$d_6$) δ ppm 1.39-1.44 (m, 2H), 1.61-1.66 (m, 2H), 2.08-2.13 (m, 2H), 2.42 (s, 3H), 2.74 (s, 1H), 4.04 (t, J=6.40 Hz, 2H), 7.48 (d, J=8.40 Hz, 2H), 7.78 (d, J=8.40 Hz, 2H).

Step 2

**Intermediate 278: methyl-4-(1-(hex-5-yn-1-yl)-1*H*-pyrazol-4-yl) benzoate**

To a cooled (0°C) solution of methyl 4-(1*H*-pyrazol-4-yl)benzoate hydrochloride (**Intermediate 128**, 4.00 g, 16.76 mmol) in dimethylformamide (50 mL) was added hex-5-yn-1-yl 4-methylbenzenesulfonate (**Intermediate 277**, 6.34 g, 25.14 mmol) followed by cesium carbonate (16.4 g, 50.28 mmol) at 0°C. The reaction mixture was then heated at 80°C for 16h. The reaction mixture was then concentrated *in vacuo* and the obtained residue was poured into water (100 mL). The resultant precipitate was collected by filtration and dried *in vacuo* to afford methyl-4-(1-(hex-5-yn-1-yl)-1*H*-pyrazol-4-yl) benzoate (**Intermediate 278**, 2.70 g, 57%) as an off white solid, which was used for the next step without further purification. $^1$H NMR (400 MHz, DMSO-$d_6$) δ ppm 1.38-1.46 (m, 2H), 1.85-1.93 (m, 2H), 2.17-2.21 (m, 2H), 2.77 (s, 1H), 3.84 (s, 3H), 4.15 (t, *J*=6.80 Hz, 2H), 7.72 (d, *J*=8.40 Hz, 2H), 7.93 (d, *J*=8.40 Hz, 2H), 7.99 (s, 1H), 8.33 (s, 1H). Mass spec m/z 282.9 $[M+H]^+$.

**Step 3**

**Intermediate 279: 4-(1-(hex-5-yn-1-yl)-1*H*-pyrazol-4-yl) benzoic acid**

To a solution of methyl-4-(1-(hex-5-yn-1-yl)-1*H*-pyrazol-4-yl) benzoate (**Intermediate 278**, 2.70 g, 9.56 mmol) in tetrahydrofuran (25 mL), methanol (15 mL) and water (15 mL) was added lithium hydroxide (0.70 g, 28.7 mmol)and the reaction mixture was stirred at room temperature for 16h then concentrated *in vacuo.* The obtained residue was diluted with water (10 mL) and acidified to pH~2 with aqueous citric acid (20 mL). The resultant precipitate was collected by filtration and dried *in vacuo* to afford 4-(1-(hex-5-yn-1-yl)-1*H*-pyrazol-4-yl)benzoic acid (**Intermediate 279**, 2.30 g) as a

white solid, which was used for the next step without further purification. $^1$H NMR (400 MHz, DMSO-$d_6$) δ ppm 1.35-1.43 (m, 2H), 1.82-1.89 (m, 2H), 2.14-2.18 (m, 2H), 2.73 (s, 1H), 4.12 (t, J=6.80 Hz, 2H), 7.66 (d, J=8.40 Hz, 2H), 7.88 (d, J=8.40 Hz, 2H), 7.95 (s, 1H), 8.28 (s, 1H), 12.76 (br s, 1H). Mass spec m/z 268.9 [M+H]$^+$.

**Step 4**

**Intermediate 280: 4-(1-(hex-5-yn-1-yl)-1*H*-pyrazol-4-yl) benzamide**

To a solution of 4-(1-(hex-5-yn-1-yl)-1*H*-pyrazol-4-yl)benzoic acid (**Intermediate 279**, 2.30 g, 8.6 mmol) in dimethylformamide (25 mL) was added *N, N*-diisopropylethylamine (7.5 mL, 43 mmol) followed by HATU (4.0 g, 10 mmol) and the reaction mixture was stirred at room temperature for 10 min. Ammonium chloride (2.3 g, 43 mmol) was added and the reaction mixture was stirred at room temperature for 16h then concentrated *in vacuo.* The obtained residue was poured into water (100 mL), the resultant precipitate was collected by filtration and dried *in vacuo* to afford 4-(1-(hex-5-yn-1-yl)-1*H*-pyrazol-4-yl) benzamide (**Intermediate 280**, 1.50 g, 65%) as an off-white solid which was used for the next step without further purification. $^1$H NMR (400 MHz, DMSO-$d_6$) δ ppm 1.39-1.46 (m, 2H), 1.85-1.92 (m, 2H), 2.17-2.22 (m, 2H), 2.77 (s, 1H), 4.14 (t, J=6.80 Hz, 2H), 7.27 (br s, 1H), 7.64 (d, J=8.40 Hz, 2H), 7.86 (d, J=8.40 Hz, 2H), 7.91 (br s, 1H), 7.96 (s, 1H), 8.28 (s, 1H). Mass spec m/z 267.9 [M+H]+.

**Step 5**

**Intermediate 281: 4-(1-(6-(2-(2,6-dioxo-1-((2-(trimethylsilyl)ethoxy)methyl)piperidin-3-yl)-1-oxoisoindolin-4-yl) hex-5-yn-1-yl)-1*H*-pyrazol-4-yl) benzamide**

To a solution of 4-(1-(hex-5-yn-1-yl)-1*H*-pyrazol-4-yl) benzamide (**Intermediate 280**, 1.00 g, 3.7 mmol) in dimethylformamide (5 mL) was added 3-(4-iodo-1-oxoisoindolin-2-yl)-1-((2-(trimethylsilyl)ethoxy)methyl)piperidine-2,6-dione (**Intermediate 8**, 1.9 g, 3.7 mmol), followed by triethylamine (18 mL, 130 mmol). The reaction mixture was purged with argon for 15 min, then $PdCl_2(PPh_3)_2$ (0.27 g, 0.37 mmol) and copper (I) iodide (0.073 g, 0.37 mmol) were added. The reaction mixture was further purged with argon for 10 min and stirred at room temperature for 2h. The reaction mixture was diluted with water (100 mL) and extracted with ethyl acetate (3 × 200 mL). The combined organic layers were separated, dried over anhydrous $Na_2SO_4$, and concentrated *in vacuo.* The crude material was purified by combi-flash chromatography, by eluting with 100% ethyl acetate in heptane, to afford 4-(1-(6-(2-(2,6-dioxo-1-((2-(trimethylsilyl)ethoxy) methyl) piperidin-3-yl)-1-oxoisoindolin-4-yl) hex-5-yn-1-yl)-1*H*-pyrazol-4-yl) benzamide (**Intermediate 281**, 1.50 g, 63%) as a yellow solid. $^1$H NMR (400 MHz, DMSO-$d_6$) δ ppm -0.07 (s, 9H), 0.76-0.83 (m, 2H), 1.49-1.58 (m, 2H), 1.90-2.05 (m, 3H), 2.36-2.39 (m, 1H), 2.70-2.76 (m, 2H), 2.84-2.86 (m, 1H), 3.98-3.05 (m, 1H), 3.46-3.50 (m, 2H), 4.11-4.26 (m, 3H), 4.42-4.48 (m, 1H), 4.98-5.05 (m, 2H), 5.20-5.25 (m, 1H), 7.25 (br s, 1H), 7.47-7.70 (m, 5H), 7.80-7.84 (m, 2H), 7.88 (br s, 1H), 7.91 (s, 1H), 8.27 (s, 1H). Mass spec m/z 637.9 [M-H]$^-$.

**Step 5**

**Intermediate 282: *tert*-butyl 6-(4-(1-(6-(2-(2,6-dioxo-1-((2-(trimethylsilyl)ethoxy)methyl)piperidin-3-yl)-1-oxoisoindolin-4-yl)hex-5-yn-1-yl)-1*H*-pyrazol-4-yl)benzamido)-2-((*R*)-1-methylpyrrolidin-2-yl)-1*H*-pyrrolo[3,2-c]pyridine-1-carboxylate**

To a solution of 4-(1-(6-(2-(2, 6-dioxo-1-((2-(trimethylsilyl)ethoxy)methyl)piperidin-3-yl)-1-oxoisoindolin-4-yl) hex-5-yn-1-yl)-1*H*-pyrazol-4-yl) benzamide (**Intermediate 281**, 0.8 g, 1 mmol) in 1, 4-dioxane (15 mL) was added *tert*-butyl-(R)-6-bromo-2-(1-methylpyrrolidin-2-yl)-1*H*-pyrrolo[3,2-c]pyridine-1-carboxylate (**Intermediate 4**, 0.6 g, 2 mmol) followed by cesium carbonate (2 g, 5 mmol). The reaction mixture was purged with argon for 15 min, then $Pd_2(dba)_3$ (0.2 g, 0.2 mmol) followed by Xantphos (0.3 g, 0.5 mmol) were added. The reaction mixture was purged with argon for 10 min and then heated at 100°C for 2h. The reaction mixture was filtered through a celite bed and the filter pad was washed with ethyl acetate (50 mL). The filtrate was concentrated *in vacuo* and the crude material purified by combi-flash chromatography, by eluting with 100% ethyl acetate in heptane, to afford tert-butyl 6-(4-(1-(6-(2-(2,6-dioxo-1-((2-(trimethylsilyl)ethoxy)methyl)piperidin-3-yl)-1-oxoisoindolin-4-yl)hex-5-yn-1-yl)-1*H*-pyrazol-4-yl)benzamido)-2-((*R*)-1-methylpyrrolidin-2-yl)-1*H*-pyrrolo[3,2-c]pyridine-1-carboxylate (**Intermediate 282**, 0.40 g, 30%) as a yellow solid. $^1$H NMR (400 MHz, DMSO-$d_6$) δ ppm -0.04 (s, 9H), 0.79-0.82 (m, 2H), 1.55-1.62 (m, 5H), 1.70 (s, 9H), 1.73-1.75 (m, 2H), 1.98-2.04 (m, 4H), 2.36 (s, 3H), 2.42-2.45 (m, 2H), 2.74-2.78 (m, 1H), 3.01-3.14 (m, 2H), 3.46-3.56 (m, 2H), 4.20 (t, J=6.80 Hz, 2H), 4.26-4.30 (m, 2H), 4.46-4.50 (m, 1H), 5.00-5.08 (m, 2H), 5.23-528 (m, 1H), 6.76 (s, 1H), 7.53 (t, J=8.0 Hz, 1H), 7.64-7.73 (m, 4H), 7.99 (s, 1H), 8.05 (d, J=8.0 Hz, 2H), 8.35 (s, 1H), 8.60 (s, 1H), 8.94 (s, 1H), 10.61 (br s, 1H). Mass spec m/z 939.0 [M+H]$^+$.

**Step 7**

**Example 59: 4-(1-(6-(2-(2,6-dioxopiperidin-3-yl)-1-oxoisoindolin-4-yl)hex-5-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide**

To a solution of tert-butyl 6-(4-(1-(6-(2-(2,6-dioxo-1-((2-(trimethylsilyl)ethoxy)methyl)piperidin-3-yl)-1-oxoisoindolin-4-yl)hex-5-yn-1-yl)-1*H*-pyrazol-4-yl)benzamido)-2-((*R*)-1-methylpyrrolidin-2-yl)-1*H*-pyrrolo[3,2-c]pyridine-1-carboxylate (**Intermediate 282**, 0.40 g, 0.43 mmol) in acetonitrile (8 mL) was added methanesulfonic acid (0.42 mL, 6.4 mmol) and the reaction mixture was heated at 50°C for 2h. The reaction mixture was cooled to room temperature, then *N,N'*-dimethylethylenediamine (0.31 mL, 2.6 mmol) followed by triethylamine (1.2 mL, 8.5 mmol) were added and the mixture stirred at room temperature for 3h. The reaction mixture was concentrated *in vacuo* and the resultant residue was diluted with water (50 mL). The resultant precipitate was collected by filtration and dried *in vacuo.* The crude solid was purified by preparative HPLC (Method A) to afford 4-(1-(6-(2-(2,6-dioxopiperidin-3-yl)-1-oxoisoindolin-4-yl)hex-5-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide (**Example 59**, 0.19 g, 63%) as an off white solid. $^1$H NMR (400 MHz, DMSO-$d_6$) δ ppm 1.54-1.61 (m, 2H), 1.78-1.94 (m, 3H), 1.99-2.06 (m, 3H), 2.13-2.15 (m, 1H), 2.17 (s, 3H), 2.23-2.30 (m, 1H), 2.39-2.46 (m, 2H), 2.52-2.59 (m, 3H), 2.86-2.95 (m, 1H), 3.12-3.19 (m, 1H), 4.21 (t, *J*=6.82 Hz, 2H), 4.30-4.34 (m, 1H), 4.44-4.49 (m, 1H), 5.11-5.16 (m, 1H), 6.39 (s, 1H), 7.50-7.54 (m, 1H), 7.63-7.65 (m, 1H), 7.68-7.73 (m, 3H), 7.99 (s, 1H), 8.00-8.05 (m, 2H), 8.20 (s, 1H), 8.35 (s, 1H), 8.50 (s, 1H), 10.38 (s, 1H), 11.00 (s, 1H), 11.36 (s, 1H). Mass spec m/z 709.3 [M+H]$^+$.

**Example 60 was synthesised following Scheme 58**

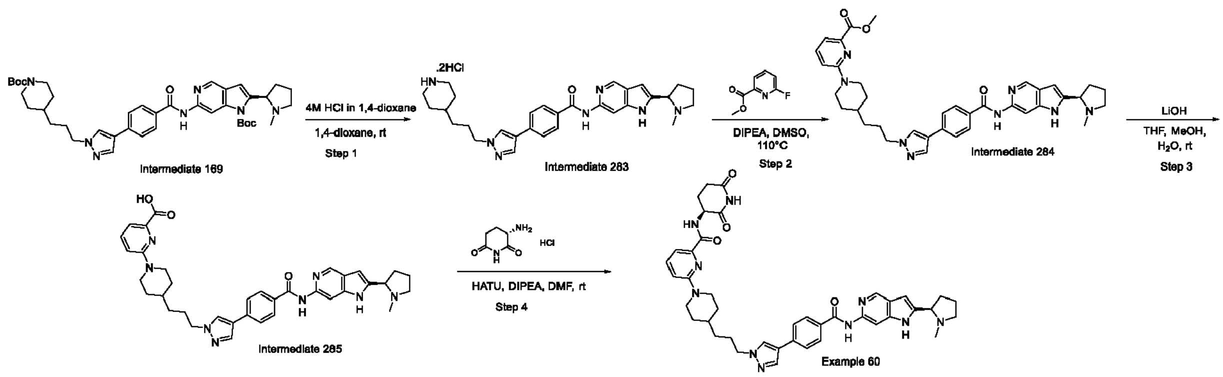


## Scheme 58

**Step 1**

**Intermediate 283: (*R*)-*N*-(2-(1-methylpyrrolidin-2-yl)-1*H*-pyrrolo[3,2-c]pyridin-6-yl)-4-(1-(3-(piperidin-4-yl) propyl)-1*H*-pyrazol-4-yl)benzamide dihydrochloride**

To a cooled (0°C) solution of *tert*-butyl-(R)-6-(4-(1-(3-(1-(*tert*-butoxycarbonyl)piperidin-4-yl)propyl)-1*H*-pyrazol-4-yl)benzamido)-2-(1-methylpyrrolidin-2-yl)-1*H*-pyrrolo[3,2-c]pyridine-1-carboxylate (**Intermediate 169**, 1.40 g, 2.0 mmol) in 1,4-dioxane (10 mL) was added 4M hydrochloric acid in 1,4-dioxane (15 mL) and the reaction mixture was stirred at room temperature for 16h. The reaction mixture was concentrated *in vacuo* to afford (R)-N-(2-(1-methylpyrrolidin-2-yl)-1*H*-pyrrolo[3,2-c]pyridin-6-yl)-4-(1-(3-(piperidin-4-yl) propyl)-1*H*-pyrazol-4-yl)benzamide dihydrochloride (**Intermediate 283**, 1.20 g) as an off-white solid which was used for the next step without further purification. $^1$H NMR (400 MHz, DMSO-$d_6$) δ ppm 1.12-1.34 (m, 4H), 1.45-1.61 (m, 1H), 1.70-1.89 (m, 4H), 2.12-2.20 (m, 2H), 2.32-2.40 (m, 2H), 2.49 (s, 3H), 2.78-2.81 (m, 2H), 3.20-3.24 (m, 2H), 3.69-3.71 (m, 1H), 3.72-3.75 (m, 1H), 4.12 (t, *J*=6.40 Hz, 2H), 4.70-4.80 (m, 1H), 7.10 (s, 1H), 7.82 (d, *J*=8.0 Hz, 2H), 8.06 (s, 1H), 8.21 (d, *J*=8.0 Hz, 2H), 8.27 (s, 1H), 8.40 (s, 1H), 8.60 (br s, 1H), 8.83 (br s, 1H), 9.10 (s, 1H), 11.42 (br s, 1H), 12.0 (br s, 1H), 13.29 (br s, 1H). Mass spec m/z 512.1 [M+H]$^+$.

**Step 2**

**Intermediate 284: methyl-(*R*)-6-(4-(3-(4-(4-((2-(1-methylpyrrolidin-2-yl)-1*H*-pyrrolo[3,2-c]pyridin-6-yl)carbamoyl) phenyl)-1*H*-pyrazol-1-yl)propyl)piperidin-1-yl)picolinate**

To a solution of (R)-N (2-(1-methylpyrrolidin-2-yl)-1H-pyrrolo[3,2-c]pyridin-6-yl)-4-(1-(3-(piperidin-4-yl) propyl)-1*H*-pyrazol-4-yl)benzamide dihydrochloride (**Intermediate 283**, 0.80 g, 1.56 mmol) in dimethylsulfoxide (10 mL) was added methyl-6-fluoropicolinate (CAS No: 455-71-0, 0.31 g, 2.03 mmol) followed by *N*,*N*-diisopropylethylamine (1.37 mL, 7.82 mmol). The reaction mixture was heated at 110°C for 16h then poured into water (100 mL). The resultant precipitate was collected by filtration and dried *in vacuo* to afford methyl-(R)-6-(4-(3-(4-(4-((2-(1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinate (**Intermediate 284**, 0.35 g, 35%) as a brown solid, which was used for the next step without further purification. $^1$H NMR (400 MHz, DMSO-$d_6$) δ ppm 1.01-1.15 (m, 3H), 1.18-1.24 (m, 2H), 1.47-1.58 (m, 1H), 1.69-1.97 (m, 6H), 2.13-2.27 (m, 3H), 2.54 (s, 3H), 2.76-2.80 (m, 2H), 3.12-3.16 (m, 1H), 3.81 (s, 3H), 4.13 (t, *J*=6.80 Hz, 2H), 4.32-4.36 (m, 2H), 6.40 (s, 1H), 7.05 (d, *J*=8.80 Hz, 1H), 7.24 (d, *J*=7.60 Hz, 1H), 7.63 (t, J=8.0 Hz, 1H), 7.70 (d, *J*=8.0 Hz, 2H), 8.0 (s, 1H), 8.06 (d, *J*=7.60 Hz, 2H), 8.20 (s, 1H), 8.34 (s, 1H), 8.51 (s, 1H), 10.39 (br s, 1H), 11.37 (br s, 1H). Mass spec m/z 647.3 $[M+H]^+$.

**Step 3**

**Intermediate 285: (*R*)-6-(4-(3-(4-(4-((2-(1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinic acid**

To a cooled (0°C) solution of methyl-(*R*)-6-(4-(3-(4-(4-((2-(1-methylpyrrolidin-2-yl)-1*H*-pyrrolo[3,2-c]pyridin-6-yl) carbamoyl)phenyl)-1*H*-pyrazol-1-yl)propyl)piperidin-1-yl)picolinate **(Intermediate 284,** 0.35 g, 0.54 mmol) in tetrahydrofuran (5 mL), methanol (2 mL) and water (3 mL) was added lithium hydroxide (0.039 g, 1.62 mmol) and the reaction mixture was stirred at room temperature for 16h. The reaction mixture was concentrated *in vacuo* and the obtained residue was diluted in water (10 mL) and acidified to ~pH4 by the addition of 1N aqueous HCl solution. The resultant precipitate was collected by filtration and dried *in vacuo* to afford (*R*)-6-(4-(3-(4-(4-((2-(1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinic acid **(Intermediate 285,** 0.20 g, 58%) as an off brown solid, which was used for the next step without further purification. $^1$H NMR (400 MHz, DMSO-$d_6$) δ ppm 1.01-1.13 (m, 2H), 1.18-1.30 (m, 2H), 1.47-1.58 (m, 1H), 1.66-2.01 (m, 6H), 2.08-2.25 (m, 3H), 2.30-2.42 (m, 2H), 2.54 (s, 3H), 3.12-3.20 (m, 1H), 4.13 (t, *J*=6.40 Hz, 2H), 4.35-4.45 (m, 2H), 4.68-4.63 (m, 1H), 6.15 (s, 1H), 7.02 (d, *J*=8.0 Hz, 1H), 7.23 (d, *J*=6.80 Hz, 1H), 7.62 (t, *J*=8.0 Hz, 1H), 7.74 (d, *J*=8.0 Hz, 2H), 8.01 (s, 1H), 8.14 (d, *J*=8.0 Hz, 2H), 8.34 (s, 1H), 8.38 (s, 1H), 8.81 (s, 1H), 11.07 (br s, 1H), 11.56 (br s, 1H), 12.63 (br s, 1H). Mass spec m/z 633.1 $[M+H]^+$.

**Step 4**

**Example 60: *N*-((S)-2,6-dioxopiperidin-3-yl)-6-(4-(3-(4-(4-((2-((*R*)-1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinamide**

To a cooled (0°C) solution of (*R*)-6-(4-(3-(4-(4-((2-(1-methylpyrrolidin-2-yl)-1H-pyrrolo[3,2-c]pyridin-6-yl)carbamoyl)phenyl)-1*H*-pyrazol-1-yl)propyl)piperidin-1-yl)picolinic acid **(Intermediate 285,** 0.20 g, 0.31 mmol) in dimethylformamide (10 mL) was added HATU (0.18 g, 0.47 mmol) and *N*,*N*-diisopropylethylamine (0.22 mL, 1.26 mmol). The reaction mixture was stirred for 10 min, then (*S*)-3-aminopiperidine-2,6-dione hydrochloride (CAS No: 25181-50-4, 0.062 g, 0.38 mmol) was added at 0°C and the reaction mixture was stirred at room temperature for 16h. The reaction mixture was poured into water (100 mL), the resultant precipitate was collected by filtration and dried *in vacuo.* The crude solid was purified by preparative HPLC (Method A) to afford *N*-((*S*)-2,6-dioxopiperidin-3-yl)-6-(4-(3-(4-(4-((2-((*R*)-1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinamide **(Example 60, 0.02** g, 9%) as an off white solid. $^1$H NMR (400 MHz, DMSO-$d_6$) δ ppm 1.08-1.18 (m, 2H), 1.20-1.30 (m, 2H), 1.48-1.58 (m, 1H), 1.71-1.82 (m, 3H), 1.83-1.95 (m, 4H), 1.97-2.05 (m, 2H), 2.17 (s, 3H), 2.20-2.30 (m, 3H), 2.52-2.56 (m, 2H), 2.73-2.82 (m, 2H), 3.11-3.19 (m, 1H), 4.14 (t, *J*=6.0 Hz, 2H), 4.38-4.42 (m, 2H), 4.67-4.76 (m, 1H), 6.39 (s, 1H), 7.02 (d, *J*=8.80 Hz, 1H), 7.26 (d, *J*=7.60 Hz, 1H), 7.64 (t, *J*=7.60 Hz, 1H), 7.71 (d, *J*=8.80 Hz, 2H), 8.0 (s, 1H), 8.06 (d, *J*=7.60 Hz, 2H), 8.20 (s, 1H), 8.34 (s, 1H), 8.50 (s, 1H), 8.73 (d, *J*=8.80 Hz, 1H), 10.38 (br s, 1H), 10.87 (br s, 1H), 11.36 (br s, 1H). Mass spec m/z 743.2 $[M+H]^+$.

**Example 61 was synthesised following Scheme 59**

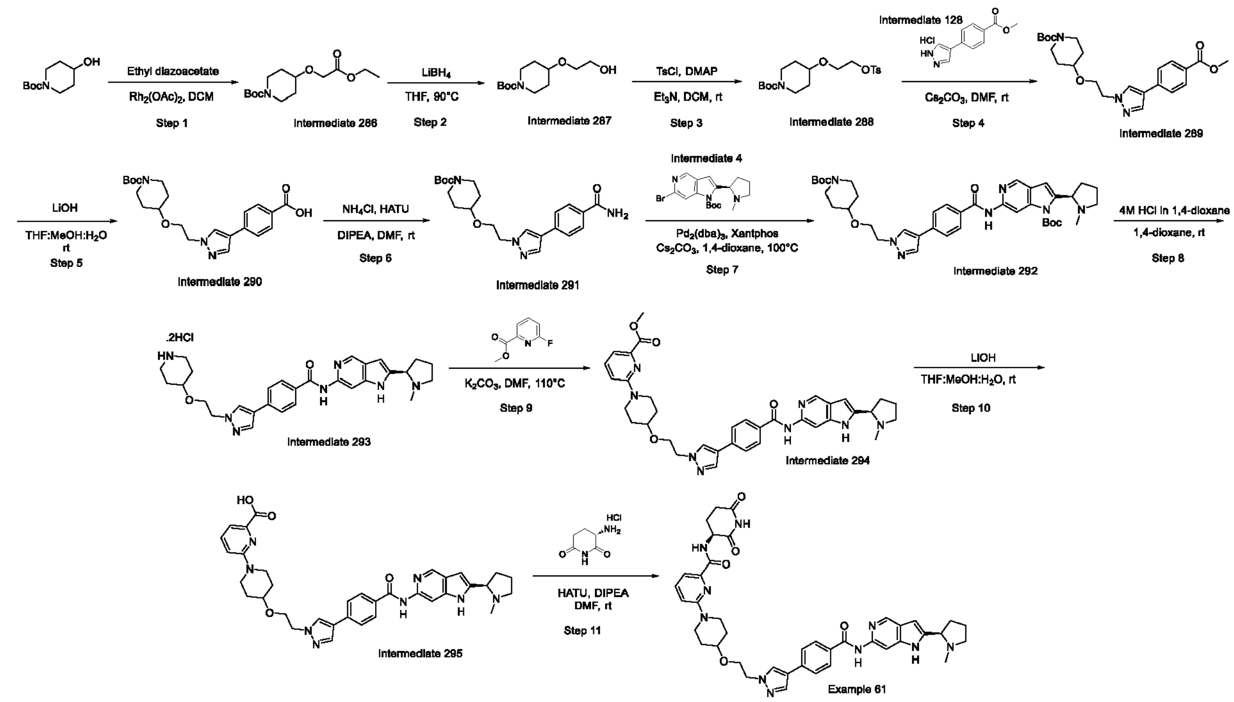


## Scheme 59

### Step 1

**Intermediate 286: *tert*-butyl-4-(2-ethoxy-2-oxoethoxy)piperidine-1-carboxylate**

To a solution of *tert*-butyl-4-hydroxypiperidine-1-carboxylate (CAS No: 109384-19-2, 5.00 g, 25 mmol) in dichloromethane (100 mL) was added rhodium (II) acetate dimer (0.55 g, 1.2 mmol). The reaction mixture was stirred at room temperature for 10 min, then ethyl-2-diazoacetate (8.5 g, 75 mmol) was added and the reaction stirred at room temperature for 48h. The reaction mixture was poured into water (200 mL) and extracted with dichloromethane (3 × 100 mL). The combined organic layers were separated, dried over anhydrous $Na_2SO_4$, filtered, and concentrated *in vacuo.* The crude material was purified by combi-flash column chromatography, by eluting with 40% ethyl acetate in heptane, to afford *tert*-butyl-4-(2-ethoxy-2-oxoethoxy) piperidine-1-carboxylate **(Intermediate 286,** 4.50 g, 63%) as a colorless oil. $^1H$ NMR (400 MHz, DMSO-$d_6$) δ ppm 1.18 (t, *J*=7.02 Hz, 3H), 1.27-1.34 (m, 2H), 1.37 (s, 9H), 1.70-1.82 (m, 2H), 2.94-3.00 (m, 2H), 3.46-3.54 (m, 1H), 3.56-3.64 (m, 2H), 4.05-4.13 (m, 4H). Mass spec m/z 232.2 $[M+H]^+$.

### Step 2

**Intermediate 287: *tert*-butyl-4-(2-hydroxyethoxy)piperidine-1-carboxylate**

To a solution of *tert*-butyl 4-(2-ethoxy-2-oxoethoxy)piperidine-1-carboxylate **(Intermediate 286,** 4.50 g, 16.0 mmol) in tetrahydrofuran (100 mL) was added lithium borohydride (1.1 g, 47.0 mmol) and the reaction mixture was stirred at 90°C for 16h. The reaction mixture was then poured into water (250 mL) and extracted with ethyl acetate (3 × 250 mL). The combined organic layers were dried over anhydrous $Na_2SO_4$, filtered, and concentrated *in vacuo* to afford *tert*-butyl 4-(2-hydroxyethoxy)piperidine-1-carboxylate **(Intermediate 287**, 3.00 g, 78%) as a colorless oil, which was used for the next step without further purification. $^1H$ NMR (400 MHz, DMSO-$d_6$) δ ppm 1.23-1.32 (m, 2H), 1.36 (s, 9H), 1.72-1.78 (m, 2H), 2.92-3.01 (m, 2H), 3.37-3.49 (m, 5H), 3.57-3.66 (m, 2H), 4.52-4.56 (m, 1H).

### Step 3

**Intermediate 288: *tert*-butyl 4-(2-(tosyloxy)ethoxy)piperidine-1-carboxylate**

To a cooled (0°C) solution of *tert*-butyl 4-(2-hydroxyethoxy) piperidine-1-carboxylate **(Intermediate 287,** 3.00 g, 12.0 mmol) in dichloromethane (200 mL) was added triethylamine (5.2 mL, 37 mmol) followed by *p*-toluenesulfonyl chloride (2.9 g, 15.0 mmol) and 4-dimethylaminopyridine (0.15 g, 1.2 mmol) and the reaction mixture was stirred at room

temperature for 16h. The reaction mixture was poured in water (200 mL) and extracted with dichloromethane (3 × 250 mL). The combined organic layers were dried over anhydrous $Na_2SO_4$, filtered, and concentrated *in vacuo.* The crude material was purified by combi-flash column chromatography, by eluting with 10% ethyl acetate in heptane, to afford *tert*-butyl-4-(2-(tosyloxy)ethoxy)piperidine-1-carboxylate **(Intermediate 288,** 4.00 g, 82%) as a colorless liquid. $^1$H NMR (400 MHz, DMSO-$d_6$) δ ppm 1.18-1.24 (m, 2H), 1.39 (s, 9H), 1.62-1.68 (m, 2H), 2.42 (s, 3H), 2.92-2.98 (m, 2H), 3.36-3.42 (m, 1H), 3.46-3.63 (m, 4H), 4.08-4.16 (m, 2H), 7.47 (d, *J*=7.88 Hz, 2H), 7.78 (d, *J*=7.88 Hz, 2H). Mass spec m/z 399.9 $[M+H]^+$.

**Step 4**

**Intermediate 289: *tert*-butyl-4-(2-(4-(4-(methoxycarbonyl)phenyl)-1*H*-pyrazol-1-yl)ethoxy)piperidine-1-carboxylate**

To a solution of *tert*-butyl 4-(2-(tosyloxy)ethoxy)piperidine-1-carboxylate **(Intermediate 288,** 4.00 g, 11.0 mmol) in dimethylformamide (25 mL) was added cesium carbonate (7.40 g, 23.0 mmol) followed by methyl 4-(1*H*-pyrazol-4-yl) benzoate hydrochloride **(Intermediate 128,** 1.80 g, 7.5 mmol) and the reaction mixture was stirred at room temperature for 12h. The reaction mixture was poured in ice cold water (200 mL) and extracted with dichloromethane (3 × 100 mL). The combined organic layers were separated, dried over anhydrous $Na_2SO_4$, filtered, and concentrated *in vacuo.* The crude material was purified by combi-flash column chromatography, by eluting with 40% ethyl acetate in heptane, to afford *tert*-butyl 4-(2-(4-(4-(methoxycarbonyl) phenyl)-1*H*-pyrazol-1-yl) ethoxy) piperidine-1-carboxylate **(Intermediate 289,** 2.80 g, 86%) as a colorless oil. $^1$H NMR (400 MHz, DMSO-$d_6$) δ ppm 1.24-1.30 (m, 2H), 1.35 (s, 9H), 1.64-1.72 (m, 2H), 2.94-3.04 (m, 2H), 3.42-3.52 (m, 3H), 3.78-3.82 (m, 2H), 3.84 (s, 3H), 4.24-4.28 (m, 2H), 7.72 (d, *J*=8.40 Hz, 2H), 7.92 (d, J=8.40 Hz, 2H), 7.95 (s, 1H), 8.31 (s, 1H). Mass spec m/z 430.0 $[M+H]^+$.

**Step 5**

**Intermediate 290: 4-(1-(2-((1-(tert-butoxycarbonyl)piperidin-4-yl)oxy)ethyl)-1*H*-pyrazol-4-yl) benzoic acid**

To a solution of *tert*-butyl-4-(2-(4-(4-(methoxycarbonyl)phenyl)-1H-pyrazol-1-yl)ethoxy)piperidine-1-carboxylate **(Intermediate 289,** 2.80 g, 6.5 mmol) in tetrahydrofuran (25 mL), methanol (25 mL) and water (25 mL) was added lithium hydroxide (0.48 g, 20.0 mmol) and the reaction mixture was stirred at room temperature for 3h then concentrated *in vacuo.* The obtained residue was taken up in water (10 mL) and acidified to pH~5 with 1N hydrochloric acid, then extracted with dichloromethane (3 × 100 mL). The combined organic layers were separated, dried over anhydrous $Na_2SO_4$, filtered, and concentrated *in vacuo* to afford 4-(1-(2-((1-(*tert*-butoxycarbonyl) piperidin-4-yl)oxy)ethyl)-1*H*-pyrazol-4-yl)benzoic acid **(Intermediate 290, 2.30** g) as a colorless oil, which was used for the next step without further purification. $^1$H NMR (400 MHz, DMSO-$d_6$) δ ppm 1.22-1.31 (m, 2H), 1.35 (s, 9H), 1.65-1.72 (m, 2H), 2.96-3.02 (m, 2H), 3.42-3.54 (m, 3H), 3.78-3.82 (m, 2H), 4.24-4.28 (m, 2H), 7.69 (d, J=7.89 Hz, 2H), 7.91 (d, *J*=7.89 Hz, 2H), 7.99 (s, 1H), 8.28 (s, 1H), 12.65 (br s, 1H). Mass spec m/z 415.9 $[M+H]^+$.

**Step 6**

**Intermediate 291: *tert*-butyl-4-(2-(4-(4-carbamoylphenyl)-1*H*-pyrazol-1-yl)ethoxy)piperidine-1-carboxylate**

To a solution of 4-(1-(2-((1-(*tert*-butoxycarbonyl)piperidin-4-yl)oxy)ethyl)-1*H*-pyrazol-4-yl)benzoic acid **(Intermediate 290,** 2.30 g, 5.5 mmol) in dimethylformamide (25 mL) was added HATU (5.4 g, 14.0 mmol) and the reaction mixture stirred at room temperature for 15 min. Then ammonium chloride (1.5 g, 28.0 mmol) and *N, N*-diisopropylethylamine (2.9 mL, 17.0 mmol) were added and the reaction mixture was stirred at room temperature for 16h. The reaction mixture was quenched with ice cold water (200 mL) and extracted with dichloromethane (2 × 200 mL). The combined organic layers were separated, washed with brine solution (100 mL), dried over anhydrous $Na_2SO_4$, filtered, and concentrated *in vacuo.* The crude material was purified by combi-flash column chromatography, by eluting with 5% MeOH in dichloromethane, to afford *tert*-butyl 4-(2-(4-(4-carbamoylphenyl)-1*H*-pyrazol-1-yl)ethoxy)piperidine-1-carboxylate **(Intermediate 291,** 2.0 g, 87%) as an off white solid. $^1$H NMR (400 MHz, DMSO-$d_6$) δ ppm 1.35 (s, 9H), 1.66-1.74 (m, 2H), 3.11-3.18 (m, 2H), 3.42-3.53 (m, 3H), 3.58-3.64 (m, 2H), 3.78-3.82 (m, 2H), 4.24-4.28 (m, 2H), 7.27 (br s, 1H), 7.64 (d, *J*=8.00 Hz, 2H), 7.86 (d, *J*=8.00 Hz, 2H), 7.91 (br s, 1H), 7.97 (s, 1H), 8.25 (s, 1H). Mass spec m/z 415.0 $[M+H]^+$.

**Step 7**

**Intermediate 292: *tert*-butyl-(*R*)-6-(4-(1-(2-((1-(tert-butoxycarbonyl) piperidin-4-yl) oxy)ethyl)-1*H*-pyrazol-4-yl) benzamido)-2-(1-methylpyrrolidin-2-yl)-1*H*-pyrrolo[3,2-*c*] pyridine-1-carboxylate**

To a solution of *tert*-butyl-4-(2-(4-(4-carbamoylphenyl)-1*H*-pyrazol-1-yl)ethoxy)piperidine-1-carboxylate **(Intermediate 291,** 1.50 g, 3.6 mmol) in 1,4-dioxane (20 mL) was added *tert*-butyl-(R)-6-bromo-2-(1-methylpyrrolidin-2-yl)-1H-pyrrolo[3,2-c]pyridine-1-carboxylate (**Intermediate 4,** 1.70 g, 4.47 mmol) followed by cesium carbonate (4.40 g, 13.5 mmol). The reaction mixture was purged with argon for 15 min then $Pd_2(dba)_3$ (0.63 g, 0.67 mmol) and Xantphos (0. 80 g, 1.34 mmol) were added. The reaction mixture was purged with argon for another 10 min and the reaction mixture then stirred at 100°C for 2h. The reaction mixture was filtered through celite bed, the filter pad was washed with ethyl acetate (50 mL) and the filtrate was concentrated *in vacuo.* The crude material was purified by combi-flash column chromatography, by eluting with 90% ethyl acetate in heptane, to afford *tert*-butyl-(R)-6-(4-(1-(2-((1-(*tert*-butoxycarbonyl)piperidin-4-yl)oxy) ethyl)-1*H*-pyrazol-4-yl)benzamido)-2-(1-methylpyrrolidin-2-yl)-1*H*-pyrrolo[3,2-*c*]pyridine-1-carboxylate (**Intermediate 292**, 1.2 g, 38%) as a yellow solid. $^1$H NMR (400 MHz, DMSO-$d_6$) $\delta$ ppm 1.22-1.32 (m, 2H), 1.36 (s, 9H), 1.54-1.64 (2H), 1.70 (s, 9H), 1.72-1.82 (m, 2H), 2.28-2.32 (m, 2H), 2.36 (s, 3H), 2.39-2.42 (m, 1H), 2.94-3.05 (m, 2H), 3.11-3.18 (m, 1H), 3.42-3.56 (m, 3H), 3.78-3.84 (m, 2H), 3.89-3.95 (m, 1H), 4.24-4.32 (m, 2H), 6.75 (s, 1H), 7.70 (d, *J*=8.0 Hz, 2H), 8.01 (s, 1H), 8.05 (d, *J*=8.0 Hz, 2H), 8.31 (s, 1H), 8.59 (s, 1H), 8.94 (s, 1H), 10.62 (br s, 1H). Mass spec m/z 714.0 $[M+H]^+$.

**Step 8**

**Intermediate 293: (*R*)-*N*-(2-(1-methylpyrrolidin-2-yl)-1*H*-pyrrolo[3,2-c]pyridin-6-yl)-4-(1-(2-(piperidin-4-yloxy) ethyl)-1*H*-pyrazol-4-yl)benzamide dihydrochloride**

To a cooled (0°C) solution of *tert*-butyl-(*R*)-6-(4-(1-(2-((1-(*tert*-butoxycarbonyl)piperidin-4-yl)oxy)ethyl)-1*H*-pyrazol-4-yl)benzamido)-2-(1-methylpyrrolidin-2-yl)-1*H*-pyrrolo[3,2-*c*]pyridine-1-carboxylate (**Intermediate 292,** 1.1 g, 1.5 mmol) in 1,4-dioxane (10 mL) was added 4M hydrochloric acid in 1,4-dioxane (20 mL) and the reaction mixture was stirred at room temperature for 3h. The reaction mixture was concentrated *in vacuo,* the resultant solid was washed with ether (50 mL) and dried *in vacuo* to afford (*R*)-*N*-(2-(1-methylpyrrolidin-2-yl)-1*H*-pyrrolo [3,2-*c*]pyridin-6-yl)-4-(1-(2-(piperidin-4-yloxy)ethyl)-1*H*-pyrazol-4-yl)benzamide dihydrochloride **(Intermediate 293,** 0.80 g, 98%) as an off white solid which was used for the next step without further purification. $^1$H NMR (400 MHz, DMSO-$d_6$) $\delta$ ppm 1.60-1.69 (m, 2H), 1.87-1.96 (m, 2H), 2.15-2.22 (m, 2H), 2.33-2.43 (m, 1H), 2.46 (s, 3H), 2.78-2.82 (m, 3H), 2.84-2.93 (m, 2H), 2.95-3.06 (m, 2H), 3.20-3.32 (m, 1H), 3.70-3.78 (m, 1H), 3.82-3.84 (m, 2H), 4.28-4.32 (m, 2H), 7.32 (s, 1H), 7.81-7.88 (m, 2H), 8.08 (s, 1H), 8.24-8.28 (m, 2H), 8.36-8.40 (m, 2H), 8.89 (br s, 1H), 9.04 (br s, 1H), 9.12 (s, 1H), 11.67 (br s, 1H), 12.19 (s, 1H), 13.46 (s, 1H).

**Step 9**

**Intermediate 294: methyl-(*R*)-6-(4-(2-(4-(4-((2-(1-methylpyrrolidin-2-yl)-1*H*-pyrrolo[3,2-*c*]pyridin-6-yl)carbamoyl) phenyl)-1*H*-pyrazol-1-yl)ethoxy)piperidin-1-yl)picolinate**

To a solution of of (*R*)-*N*-(2-(1-methylpyrrolidin-2-yl)-1*H*-pyrrolo [3, 2-*c*] pyridin-6-yl)-4-(1-(2-(piperidin-4-yloxy) ethyl)-1*H*-pyrazol-4-yl) benzamide dihydrochoride **(Intermediate 293,** 0.80 g, 1.55 mmol) and methyl 6-fluoropicolinate (CAS No: 455-71-0, 0.24 g, 1.55 mmol) in dimethylformamide (10 mL) was added potassium carbonate (0.64 g, 4.63 mmol) and the reaction mixture was stirred at 110°C for 16h.The reaction mixture was then poured into water (100 mL), the resultant precipitate was collected by filtration and dried *in vacuo* to afford methyl-(*R*)-6-(4-(2-(4-(4-((2-(1-methylpyrrolidin-2-yl)-1*H*-pyrrolo[3,2-*c*]pyridin-6-yl)carbamoyl)phenyl)-1*H*-pyrazol-1-yl)ethoxy)piperidin-1-yl)picolinate (**Intermediate 294**, 0.51 g) as a brown solid, which was used for the next step without further purification. $^1$H NMR (400 MHz, DMSO-$d_6$) $\delta$ ppm 1.33-1.47 (m, 2H), 1.72-1.96 (m, 5H), 2.05-2.12 (m, 1H), 2.17 (s, 3H), 2.22-2.32 (m, 1H), 2.52-2.56 (m, 1H), 3.10-3.23 (m, 3H), 3.35-3.43 (m, 1H), 3.50-3.60 (m, 1H), 3.81 (s, 3H), 3.85-3.94 (m, 3H), 4.26-4.32 (m, 2H), 6.39 (s, 1H), 7.02-7.05 (m, 1H), 7.24 (d, *J*=7.40 Hz, 1H), 7.62 (t, *J*=7.40 Hz, 1H), 7.69 (d, *J*=7.88 Hz, 2H), 8.01 (s, 1H), 8.04 (d, *J*=7.88 Hz, 2H), 8.20 (s, 1H), 8.31 (s, 1H), 8.50 (s, 1H), 10.36 (br s, 1H), 11.35 (br s, 1H). Mass spec m/z 648.9 $[M+H]^+$.

**Step 10**

**Intermediate 295: (*R*)-6-(4-(2-(4-(4-((2-(1-methylpyrrolidin-2-yl)-1*H*-pyrrolo[3,2-*c*]pyridin-6-yl) carbamoyl)phenyl)-1*H*-pyrazol-1-yl)ethoxy)piperidin-1-yl)picolinic acid**

To a solution of methyl-(*R*)-6-(4-(2-(4-(4-((2-(1-methylpyrrolidin-2-yl)-1*H*-pyrrolo[3,2-*c*]pyridin-6-yl)carbamoyl) phenyl)-1*H*-pyrazol-1-yl)ethoxy)piperidin-1-yl)picolinate **(Intermediate 294,** 0.50 g, 0.77 mmol) in tetrahydrofuran (5 mL), methanol (5 mL) and water (2 mL) was added lithium hydroxide (0.05 g, 2.31 mmol) and the reaction mixture was stirred at

room temperature for 16h. The reaction mixture was concentrated *in vacuo.* The resultant residue was poured into ice cold water (100 mL), acidified with 1N hydrochloric acid to pH~5 and extracted with dichloromethane (3 × 100 mL). The combined organic layers were separated, dried over anhydrous $Na_2SO_4$, filtered, and concentrated *in vacuo* to afford (*R*)-6-(4-(2-(4-(4-((2-(1-methylpyrrolidin-2-yl)-1*H*-pyrrolo [3, 2-*c*] pyridin-6-yl) carbamoyl) phenyl)-1*H*-pyrazol-1-yl) ethoxy) piperidin-1-yl) picolinic acid **(Intermediate 295,** 0.30 g, 61%) as a colorless oil, which was used for the next step without further purification. $^1$H NMR (400 MHz, DMSO-$d_6$) δ ppm 1.37-1.50 (m, 2H), 1.77-1.90 (m, 5H), 2.09-2.14 (m, 1H), 2.17 (s, 3H), 2.19-2.29 (m, 1H), 3.11-3.25 (m, 2H), 3.34-3.36 (m, 2H), 3.49-3.54 (m, 1H), 3.86-3.88 (m, 2H), 3.91-4.01 (m, 2H), 4.28-4.32 (m, 2H), 6.39 (s, 1H), 6.88-6.94 (m, 1H), 7.18-7.20 (m, 1H), 7.56-7.71 (m, 3H), 7.99-8.07 (m, 3H), 8.20 (s, 1H), 8.32 (s, 1H), 8.51 (s, 1H), 10.39 (s, 1H), 11.37 (br s, 1H). Mass spec m/z 634.9 [M+H]$^+$.

**Step 11**

**Example 61: *N*-((*S*)-2,6-dioxopiperidin-3-yl)-6-(4-(2-(4-(4-((2-((*R*)-1-methylpyrrolidin-2-yl)-1*H*-pyrrolo[3,2-*c*]pyridin-6-yl)carbamoyl)phenyl)-1*H*-pyrazol-1-yl)ethoxy)piperidin-1-yl)picolinamide**

To a solution of (*R*)-6-(4-(2-(4-(4-((2-(1-methylpyrrolidin-2-yl)-1H-pyrrolo [3, 2-c] pyridin-6-yl) carbamoyl) phenyl)-1*H*-pyrazol-1-yl) ethoxy) piperidin-1-yl) picolinic acid **(Intermediate 295,** 0.30 g, 0.47 mmol) in dimethylformamide (5.0 mL) was added HATU (0.27 g, 0.70 mmol) and the mixture was stirred at room temperature for 15 min. (3*S*)-3-aminopiperidine-2,6-dione hydrochloride (CAS No: 25181-50-4, 0.15 g, 0.94 mmol) and *N, N*-diisopropylethylamine (0.30 g, 0.23 mmol) were added and the reaction was stirred at room temperature for a further 16h. The reaction mixture was quenched with water (50 mL), the resultant precipitate was collected by filtration, washed with *n*-pentane (50 mL) and dried *in vacuo.* The crude material was purified by preparative HPLC (Method A) to afford *N*-((*S*)-2,6-dioxopiperidin-3-yl)-6-(4-(2-(4-(4-((2-((*R*)-1-methylpyrrolidin-2-yl)-1*H*-pyrrolo[3,2-*c*]pyridin-6-yl)carbamoyl)phenyl)-1*H*-pyrazol-1-yl) ethoxy)piperidin-1-yl)picolinamide **(Example 61, 0.12** g, 29%) as an off white solid. $^1$H NMR (400 MHz, DMSO-$d_6$) δ ppm 1.37-1.50 (m, 2H), 1.77-2.03 (m, 6H), 2.09-2.14 (m, 1H), 2.17 (s, 3H), 2.19-2.29 (m, 2H), 2.74-2.84 (m, 1H), 3.11-3.25 (m, 3H), 3.34-3.36 (m, 2H), 3.50-3.59 (m, 1H), 3.86-3.88 (m, 2H), 3.91-4.01 (m, 2H), 4.28-4.32 (m, 2H), 4.65-4.77 (m, 1H), 6.39 (s, 1H), 7.00-7.02 (m, 1H), 7.25-7.27 (m, 1H), 7.62-7.71 (m, 3H), 8.01 (s, 1H), 8.03-8.07 (m, 2H), 8.20 (s, 1H), 8.32 (s, 1H), 8.50 (s, 1H), 8.73 (d, *J*=8.38 Hz, 1H), 10.37 (s, 1H), 10.88 (s, 1H), 11.36 (s, 1H). Mass spec m/z 745.1 [M+H]$^+$.

**Example 62 was synthesised following Scheme 60**

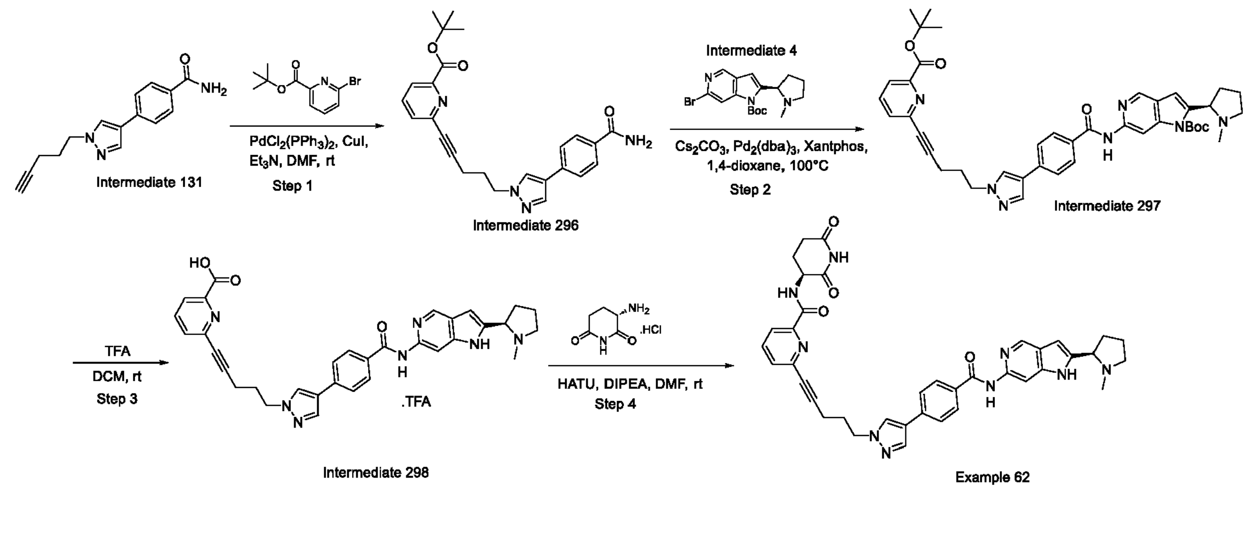


# Scheme 60

**Step 1**

**Intermediate 296: *tert*-butyl-6-(5-(4-(4-carbamoylphenyl)-1H pyrazol-1-yl)pent-1-yn-1-yl) picolinate**

To a solution of 4-(1-(pent-4-yn-1-yl)-1*H*-pyrazol-4-yl)benzamide **(Intermediate 131,** 1.50 g, 5.9 mmol) in

dimethylformamide (2 mL) was added *tert*-butyl-6-bromopicolinate (CAS No: 910044-07-4, 1.50 g, 5.9 mmol) followed by triethylamine (31mL, 220 mmol). The reaction mixture was purged with argon for 15 min, then copper (I) iodide (0.11 g, 0.59 mmol) and $PdCl_2(PPh_3)_2$ (0.43 g, 0.59 mmol) were added. The reaction mixture was further purged with argon for 10 min and stirred at room temperature for 2h. The reaction mixture was then diluted with water (100 mL) and extracted with ethyl acetate (3 × 100 mL). The combined organic layers were separated, dried over anhydrous $Na_2SO_4$, and concentrated *in vacuo.* The crude material was purified by combi-flash chromatography, by eluting with 100% ethyl acetate in heptane, to afford *tert*-butyl-6-(5-(4-(4-carbamoylphenyl)-1*H*-pyrazol-1-yl)pent-1-yn-1-yl) picolinate **(Intermediate 296,** 1.80 g, 71%) as a white solid. $^1$H NMR (400 MHz, DMSO-$d_6$) δ ppm 1.55 (s, 9H), 2.10-2.16 (m, 2H), 3.16 (t, *J*=6.40 Hz, 2H), 4.28 (t, *J*=6.40 Hz, 2H), 7.28 (br s, 1H), 7.63-7.67 (m, 3H), 7.85 (d, *J*=7.60 Hz, 2H), 7.90-7.95 (m, 3H), 7.99 (s, 1H), 8.34 (s, 1H).

**Step 2**

**Intermediate 297: *tert*-butyl-(*R*)-6-(4-(1-(5-(6-(*tert*-butoxycarbonyl)pyridin-2-yl)pent-4-yn-1-yl)-1*H*-pyrazol-4-yl) benzamido)-2-(1-methylpyrrolidin-2-yl)-1*H*-pyrrolo[3,2-c]pyridine-1-carboxylate**

To a solution of *tert*-butyl-6-(5-(4-(4-carbamoylphenyl)-1*H*-pyrazol-1-yl)pent-1-yn-1-yl) picolinate **(Intermediate 296,** 1.50 g, 3.5 mmol) in 1,4-dioxane (10 mL) was added *tert*-butyl-(*R*)-6-bromo-2-(1-methylpyrrolidin-2-yl)-1*H*-pyrrolo [3,2-c]pyridine-1-carboxylate **(Intermediate 4,** 1.30 g, 3.5 mmol) followed by cesium carbonate (3.40 g, 10 mmol). The reaction mixture was purged with argon for 15 min, then $Pd_2(dba)_3$ (0.49 g, 0.52 mmol) and Xantphos (0.62 g, 1.0 mmol) were added. The reaction mixture was purged with argon for another 10 min and then heated at 100°C for 2h. The reaction mixture was concentrated *in vacuo* and the crude material was purified by combi-flash chromatography, by eluting with 80% ethyl acetate in heptane, to afford *tert*-butyl-(*R*)-6-(4-(1-(5-(6-(tert-butoxycarbonyl)pyridin-2-yl)pent-4-yn-1-yl)-1*H*-pyrazol-4-yl)benzamido)-2-(1-methylpyrrolidin-2-yl)-1*H*-pyrrolo[3,2-c]pyridine-1-carboxylate **(Intermediate 297,** 1.00 g, 40%) as a white solid. $^1$H NMR (400 MHz, DMSO-$d_6$) δ ppm 1.56 (s, 9H), 1.70 (s, 9H), 1.74-1.79 (m, 3H), 2.12-2.19 (m, 2H), 2.29-2.33 (m, 1H), 2.36 (s, 3H), 2.37-2.39 (m, 1H), 2.66-2.68 (m, 2H), 3.11-3.15 (m, 1H), 3.90-3.96 (m, 1H), 4.30 (t, *J*=6.40 Hz, 2H), 6.75 (s, 1H), 7.66-7.72 (m, 3H), 7.90-7.96 (m, 2H), 8.04-8.08 (m, 3H), 8.40 (s, 1H), 8.59 (s, 1H), 8.94 (s, 1H), 10.62 (br s, 1H). Mass spec m/z 730.56 $[M+H]^+$.

**Step 3**

**Intermediate 298: (*R*)-6-(5-(4-(4-((2-(1-methylpyrrolidin-2-yl)-1*H*-pyrrolo[3, 2-c]pyridin-6-yl)carbamoyl) phenyl)-1*H*-pyrazol-1-yl)pent-1-yn-1-yl) picolinic acid trifluoroacetate**

To a cooled (0°C) solution of *tert*-butyl-(*R*)-6-(4-(1-(5-(6-(*tert*-butoxycarbonyl) pyridin-2-yl)pent-4-yn-1-yl)-1*H*-pyrazol-4-yl) benzamido)-2-(1-methylpyrrolidin-2-yl)-1*H*-pyrrolo[3,2-c]pyridine-1-carboxylate **(Intermediate 297,** 0.70 g, 0.96 mmol) in dichloromethane (5 mL) was added trifluoroacetic acid (5 mL) and the reaction mixture was stirred at room temperature for 4h. The reaction mixture was concentrated *in vacuo* to afford (*R*)-6-(5-(4-(4-((2-(1-methylpyrrolidin-2-yl)-1*H*-pyrrolo[3,2-c]pyridin-6-yl)carbamoyl)phenyl)-1*H*-pyrazol-1-yl)pent-1-yn-1-yl) picolinic acid trifluoroacetate **(Intermediate 298,** 0.80 g) as a white solid which was used for the next step without further purification. Mass spec m/z 574.30 $[M+H]^+$.

**Step 4**

**Example 62: *N*-((S)-2,6-dioxopiperidin-3-yl)-6-(5-(4-(4-((2-((R)-1-methylpyrrolidin-2-yl)-1*H*-pyrrolo[3,2-c]pyridin-6-yl)carbamoyl)phenyl)-1*H*-pyrazol-1-yl)pent-1-yn-1-yl) picolinamide**

To a solution of (*R*)-6-(5-(4-(4-((2-(1-methylpyrrolidin-2-yl)-1*H*-pyrrolo[3,2-c]pyridin-6-yl)carbamoyl)phenyl)-1*H*-pyrazol-1-yl)pent-1-yn-1-yl) picolinic acid trifluoroacetate **(Intermediate 298,** 0.60 g, 0.98 mmol) in dimethylformamide (5 mL) was added HATU (0.56 g, 0.15 mmol) and the reaction mixture was stirred at room temperature for 15 min. (*S*)-3-aminopiperidine-2, 6-dione hydrochloride (CAS No: 25181-50-4, 0.16 g, 0.98 mmol) and *N, N*-diisopropylethylamine (0.3 mL, 1.96 mmol) were then added and the reaction mixture was stirred at room temperature for 16h. The reaction mixture was then quenched with ice cold water (50 mL), the resultant precipitate was collected by filtration and dried *in vacuo.* The crude material was purified by preparative HPLC (Method A) to afford *N*-((S)-2,6-dioxopiperidin-3-yl)-6-(5-(4-(4-((2-((R)-1-methylpyrrolidin-2-yl)-1*H*-pyrrolo[3,2-c]pyridin-6-yl)carbamoyl)phenyl)-1*H*-pyrazol-1-yl)pent-1-yn-1-yl) picolinamide **(Example 62, 0.50** g, 74%) as an off white solid. $^1$H NMR (400 MHz, DMSO-$d_6$) δ ppm 1.76-2.01 (m, 5H), 2.12-2.16 (m, 2H), 2.17 (s, 3H), 2.19-2.21 (m, 1H), 2.23-2.30 (m, 2H), 2.53-2.57 (m, 2H), 2.75-2.85 (m, 1H), 3.12-3.16 (m, 1H), 3.32-3.35 (m, 1H), 4.30 (t, *J*=6.80 Hz, 2H), 4.77-4.84 (m, 1H), 6.39 (s, 1H), 7.69-7.73 (m, 3H), 7.98 (s, 1H), 7.99-8.02 (m, 1H), 8.04-8.06 (m, 2H), 8.07(s, 1H), 8.20 (s, 1H), 8.39 (s, 1H), 8.50 (s, 1H), 8.97 (d, *J*=8.40 Hz, 1H), 10.39 (br

s, 1H), 10.87 (br s, 1H), 11.36 (br s, 1H). Mass spec m/z 684.2 $[M+H]^+$.

**Example 63 was synthesised following Scheme 61**

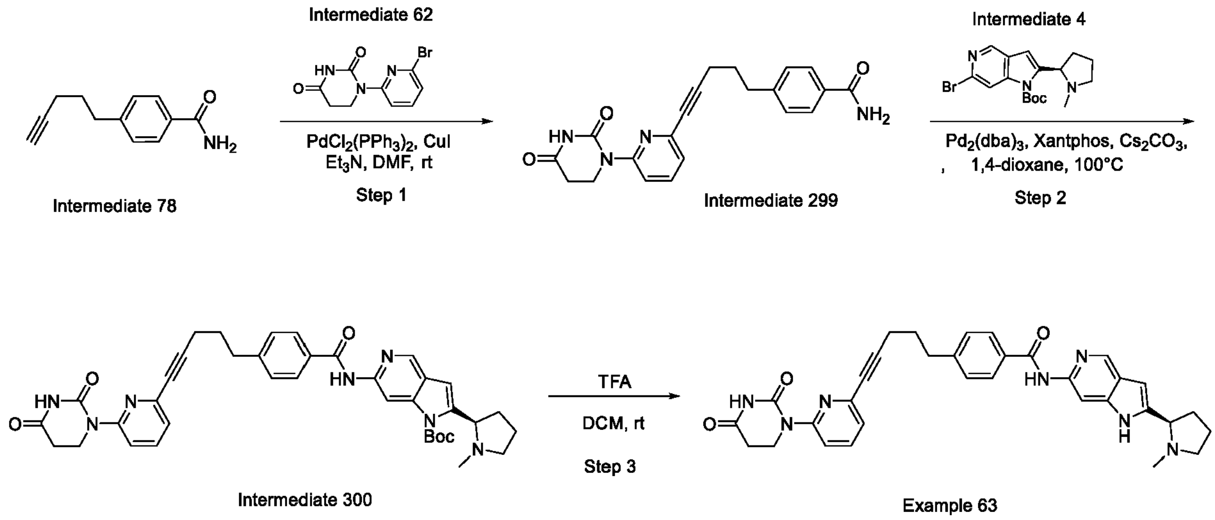


**Scheme 61**

**Step 1**

**Intermediate 299: 4-(5-(6-(2,4-dioxotetrahydropyrimidin-1(2*H*)-yl)pyridin-2-yl)pent-4-yn-1-yl)benzamide**

To a solution of 4-(pent-4-yn-1-yl)benzamide **(Intermediate 78,** 1.00 g, 3.70 mmol) in dimethylformamide (15 mL) was added 1-(6-bromopyridin-2-yl)dihydropyrimidine-2,4(1*H*,3*H*)-dione **(Intermediate 62,** 0.55 g, 2.96 mmol) followed by triethylamine (18.7 mL, 133.29 mmol). The reaction mixture was purged with argon for 15 min , then copper (I) iodide (0.072 g, 0.37 mmol) and $PdCl_2(PPh_3)_2$ (0.26 g, 0.37 mmol) were added. The reaction mixture was purged with argon for 10 min and then stirred at room temperature for 16h. The reaction mixture was diluted with water (200 mL) and extracted with ethyl acetate (3 × 90 mL). The combined organic layers were dried over anhydrous $Na_2SO_4$, and concentrated *in vacuo.* The crude material was purified by combi-flash column chromatography, by eluting with 80% ethyl acetate in heptane, to afford 4-(5-(6-(2,4-dioxotetrahydropyrimidin-1(2*H*)-yl)pyridin-2-yl)pent-4-yn-1-yl)benzamide **(Intermediate 299,** 0.85 g, 61%) as a yellow solid. $^1H$ NMR (400 MHz, DMSO-$d_6$) δ ppm 1.84-1.93 (m, 2H), 2.44-2.48 (m, 2H), 2.66-2.70 (m, 2H), 2.76-2.80 (m, 2H), 4.01-4.04 (m, 2H), 7.25 (br s, 1H), 7.27-7.35 (m, 3H), 7.72-7.84 (m, 4H), 7.89 (br s, 1H), 10.55 (s, 1H). Mass spec m/z 377.2 $[M+H]^+$.

**Step 2**

**Intermediate 300: *tert*-butyl-(R)-6-(4-(5-(6-(2, 4-dioxotetrahydropyrimidin-1(2*H*)-yl) pyridin-2-yl) pent-4-yn-1-yl) benzamido)-2-(1-methylpyrrolidin-2-yl)-1*H*-pyrrolo [3, 2-c] pyridine-1-carboxylate**

To a solution of 4-(5-(6-(2,4-dioxotetrahydropyrimidin-1(2*H*)-yl)pyridin-2-yl)pent-4-yn-1-yl)benzamide **(Intermediate 299,** 0.39 g, 1.05 mmol) in 1,4-dioxane (15 mL) was added tert-butyl-(R)-6-bromo-2-(1-methylpyrrolidin-2-yl)-1*H*-pyrrolo[3,2-c]pyridine-1-carboxylate **(Intermediate 4,** 0.5 g, 1.31 mmol) followed by cesium carbonate (1.28 g, 3.94 mmol). The reaction mixture was purged with argon for 15 min, then $Pd_2(dba)_3$ (0.18 g, 0.19 mmol) and Xantphos (0.23 g, 0.39 mmol) were added. The reaction mixture was purged with argon for another 10 min and heated at 100°C for 2h. The reaction mixture was filtered through celite bed and the filter pad was washed with ethyl acetate (100 mL). The filtrate was concentrated *in vacuo* and the crude material was purified by combi-flash chromatography, by eluting with 90% ethyl acetate in heptane, to afford *tert*-butyl-(*R*)-6-(4-(5-(6-(2,4-dioxotetrahydropyrimidin-1(2*H*)-yl)pyridin-2-yl)pent-4-yn-1-yl) benzamido)-2-(1methylpyrrolidin-2-yl)-1*H*-pyrrolo[3,2-c]pyridine-1-carboxylate **(Intermediate 300,** 0.50 g, 56%) as a yellow solid. $^1H$ NMR (400 MHz, DMSO-$d_6$) δ ppm 1.70 (s, 9H), 1.74-1.80 (m, 3H), 1.85-1.96 (m, 2H), 2.28-2.32 (m, 1H), 2.35 (s, 3H), 2.39-2.42 (m, 1H), 2.52-2.56 (m, 2H), 2.66-2.70 (m, 2H), 2.76-2.86 (m, 2H), 3.12-3.16 (m, 1H), 3.90-3.94 (m,

1H), 4.02-4.10 (m, 2H), 6.75 (s, 1H), 7.27-7.33 (m, 2H), 7.38 (d, *J*=8.0 Hz, 1H), 7.67-7.84 (m, 3H), 7.99 (d, *J*=8.0 Hz, 1H), 8.59 (s, 1H), 8.93 (s, 1H), 10.55 (br s, 1H), 10.62 (br s, 1H). Mass spec m/z 576.3 $[M-100+H]^+$.

**Step 3**

**Example 63: (*R*)-4-(5-(6-(2,4-dioxotetrahydropyrimidin-1(2*H*)-yl)pyridin-2-yl)pent-4-yn-1-yl)-*N*-(2-(1-methylpyrrolidin-2-yl)-1*H*-pyrrolo[3,2-c]pyridin-6-yl)benzamide**

To a cooled (0°C) solution of *tert*-butyl-(*R*)-6-(4-(5-(6-(2, 4-dioxotetrahydropyrimidin-1(2*H*)-yl) pyridin-2-yl) pent-4-yn-1-yl) benzamido)-2-(1-methylpyrrolidin-2-yl)-1*H*-pyrrolo[3,2-c]pyridine-1-carboxylate **(Intermediate 300,** 0.50 g, 0.74 mmol) in dichloromethane (10 mL) was added trifluoroacetic acid (5 mL) and the reaction mixture was stirred at room temperature for 1h. The reaction mixture was concentrated *in vacuo* and the crude material was purified by preparative HPLC (Method A) to afford (*R*)-4-(5-(6-(2,4-dioxotetrahydropyrimidin-1(2*H*)-yl)pyridin-2-yl)pent-4-yn-1-yl)-*N*-(2-(1-methylpyrrolidin-2-yl)-1*H*-pyrrolo[3,2-c]pyridin-6-yl)benzamide **(Example 63,** 0.067 g, 16%) as a pale yellow solid. $^1H$ NMR (400 MHz, DMSO-$d_6$) δ ppm 1.75-1.98 (m, 6H), 2.17 (s, 3H), 2.21-2.24 (m, 1H), 2.25-2.30 (m, 1H), 2.45-2.47 (m, 2H), 2.64-2.72 (m, 2H), 2.79-2.84 (m, 2H), 3.11-3.21 (m, 1H), 4.02-4.06 (m, 2H), 6.40 (s, 1H), 7.26-7.30 (m, 1H), 7.35 (d, *J*=8.40 Hz, 2H), 7.70-7.74 (m, 1H), 7.76-7.80 (m, 1H), 8.00 (d, *J*=8.40 Hz, 2H), 8.19 (s, 1H), 8.50 (s, 1H), 10.38 (br s, 1H), 10.55 (br s, 1H), 11.37 (br s, 1H). Mass spec m/z 576.2 $[M+H]^+$.

**Example 64 was synthesised following Scheme 62**

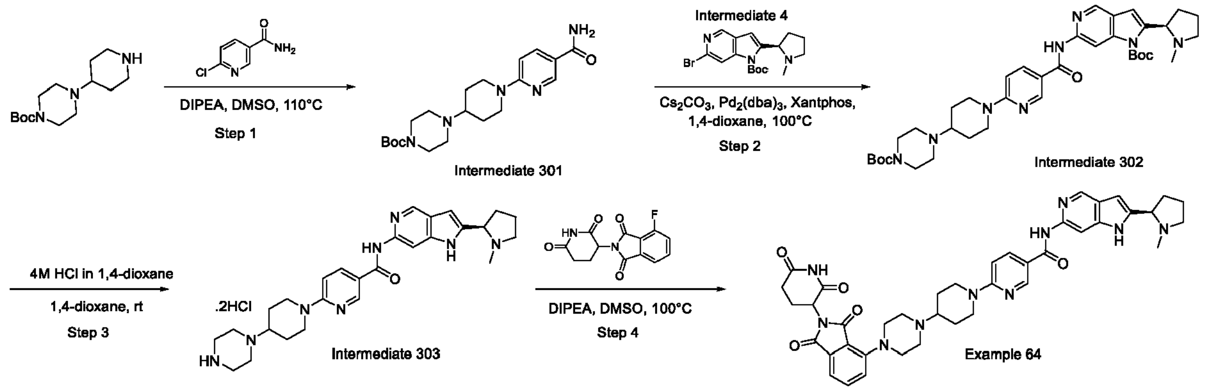


## Scheme 62

**Step** 1

**Intermediate 301: *tert*-butyl-4-(1-(5-carbamoylpyridin-2-yl) piperidin-4-yl)piperazine-1-carboxylate**

To a solution of *tert*-butyl 4-(piperidin-4-yl)piperazine-1-carboxylate (CAS No: 205059-24-1, 2.8 g, 11 mmol) in dimethyl sulfoxide (10 mL) was added 6-chloronicotinamide (CAS No: 6271-78-9, 1.5 g, 9.6 mmol) and the reaction mixture was heated at 110°C for 2h. The reaction mixture was poured into water (200 mL), the resultant precipitate was collected by filtration and dried *in vacuo* to afford *tert*-butyl 4-(1-(5-carbamoylpyridin-2-yl) piperidin-4-yl) piperazine-1-carboxylate **(Intermediate 301**, 1.00 g, 27%) as an off white solid, which was used for the next step without further purification. $^1H$ NMR (401 MHz, DMSO-$d_6$) δ ppm 1.30-1.36 (m, 2H), 1.39 (s, 9H), 1.76-1.85 (m, 2H), 2.40-2.46 (m, 4H), 2.54-2.58 (m, 1H), 2.82-2.88 (m, 2H), 3.22-3.32 (m, 4H), 4.38-4.48 (m, 2H), 6.84 (d, *J*=9.20 Hz, 1H), 7.12 (br s, 1H), 7.75 (br s, 1H), 7.93 (dd, *J*=9.20, 2.40 Hz, 1H), 8.59 (d, *J*=2.40 Hz, 1H). Mass spec m/z 390.27 $[M+H]^+$.

**Step 2**

**Intermediate 302: *tert*-butyl-(R)-6-(6-(4-(4-(tert-butoxycarbonyl)piperazin-1-yl)piperidin-1-yl) nicotinamido)-2-(1-methylpyrrolidin-2-yl)-1*H*-pyrrolo[3,2-c]pyridine-1-carboxylate**

To a solution of *tert*-butyl-4-(1-(5-carbamoylpyridin-2-yl) piperidin-4-yl)piperazine-1-carboxylate **(Intermediate**

**301**, 0.41 g, 1.05 mmol) in 1,4-dioxane (15 mL) was added *tert*-butyl-(*R*)-6-bromo-2-(1-methylpyrrolidin-2-yl)-1*H*-pyrrolo [3,2-c]pyridine-1-carboxylate **(Intermediate 4**, 0.50 g, 1.31 mmol) followed by cesium carbonate (0.86 g, 2.63 mmol). The reaction mixture was purged with argon for 15 min then $Pd_2(dba)_3$ (0.18 g, 0.19 mmol) and Xantphos (0.23 g, 0.39 mmol) were added. The reaction mixture was purged with argon for another 10 min and then stirred at 100°C for 2h. The reaction mixture was concentrated *in vacuo* and the crude material was purified by combi-flash chromatography, by eluting with 80% ethyl acetate in heptane, to afford *tert*-butyl-(*R*)-6-(6-(4-(4-(*tert*-butoxycarbonylpiperazin-1-yl)piperidin-1-yl)nicotinamido)-2-(1-methylpyrrolidin-2-yl)-1*H*-pyrrolo[3,2-c]pyridine-1-carboxylate **(Intermediate 302,** 0.45 g, 50%) as a yellow solid. $^1$H NMR (400 MHz, DMSO-$d_6$) δ ppm 1.39 (s, 9H), 1.54-1.62 (m, 2H), 1.69 (s, 9H), 1.73-1.87 (m, 4H), 2.28-2.33 (m, 1H), 2.35 (s, 3H), 2.38-2.47 (m, 6H), 2.86-2.94 (m, 2H), 3.11-318 (m, 1H), 3.23-3.27 (m, 3H), 3.32-3.36 (m, 2H), 3.88-3.95 (m, 1H), 4.44-4.48 (m, 2H), 6.74 (s, 1H), 6.88 (d, *J*=9.20 Hz, 1H), 8.13 (d, *J*=9.20 Hz, 1H), 8.57 (s, 1H), 8.78 (br s, 1H), 8.90 (s, 1H), 10.46 (br s, 1H). Mass spec m/z 689.57 $[M+H]^+$.

**Step 3**

**Intermediate 303: (*R*)-*N*-(2-(1-methylpyrrolidin-2-yl)-1*H*-pyrrolo[3,2-c]pyridin-6-yl)-6-(4-(piperazin-1-yl) piperidin-1-yl) nicotinamide dihydrochloride**

To a cooled (0°C) solution of *tert*-butyl-(*R*)-6-(6-(4-(4-(*tert*-butoxycarbonyl)piperazin-1-yl)piperidin-1-yl) nicotinamido)-2-(1-methylpyrrolidin-2-yl)-1*H*-pyrrolo[3,2-c]pyridine-1-carboxylate **(Intermediate 302,** 0.40 g, 0.58 mmol) in 1,4-dioxane (4 mL) was added 4M hydrochloric acid in 1,4-dioxane (6 mL) and the reaction mixture was stirred at room temperature for 16h. The reaction mixture was concentrated *in vacuo* to afford (*R*)-*N*-(2-(1-methylpyrrolidin-2-yl)-1*H*-pyrrolo[3,2-c]pyridin-6-yl)-6-(4-(piperazin-1-yl)piperidin-1-yl)nicotinamide dihydrochloride **(Intermediate 303,** 0.40 g) as a brown solid, which was used for the next step without further purification. $^1$H NMR (400 MHz, DMSO-$d_6$) δ ppm 1.66-1.78 (m, 3H), 2.16-2.28 (m, 5H), 2.49 (s, 3H), 2.96-3.04 (m, 2H), 3.23-3.27 (m, 3H), 3.52-3.56 (m, 2H), 3.62-3.76 (m, 7H), 4.66-4.76 (m, 2H), 7.12 (d, *J*=9.20 Hz, 1H), 7.28 (s, 1H), 8.23 (s, 1H), 8.36 (dd, *J*=2.40, 9.20 Hz, 1H), 8.99 (d, *J*=2.40 Hz, 1H), 9.08 (s, 1H), 9.83 (br s, 1H), 11.40 (br s, 1H), 12.01 (s, 1H), 12.16 (br s, 1H), 13.29 (br s, 1H). Mass spec m/z 489.37 $[M+H]^+$.

**Step 4**

**Example 64: 6-(4-(4-(2-(2,6-dioxopiperidin-3-yl)-1,3-dioxoisoindolin-4-yl)piperazin-1-yl)piperidin-1-yl)-*N*-(2-((*R*)-1-methylpyrrolidin-2-yl)-1*H*-pyrrolo[3,2-c]pyridin-6-yl)nicotinamide**

To a solution of (*R*)-*N*-(2-(1-methylpyrrolidin-2-yl)-1*H*-pyrrolo[3,2-c] pyridin-6-yl)-6-(4-(piperazin-1-yl) piperidin-1-yl) nicotinamide dihydrochloride **(Intermediate 303,** 0.40 g, 0.8 mmol) in dimethyl sulfoxide (10 mL) was added 2-(2,6-dioxopiperidin-3-yl)-4-fluoroisoindoline-1,3-dione (CAS No: 835616-60-9, 0.20 g, 0.8 mmol) followed by *N, N*-diisopropylethylamine (0.7 mL, 4 mmol) and the reaction mixture was heated at 100°C for 3h. The reaction mixture was concentrated *in vacuo.* The obtained residue was poured into ice cold water (100 mL), the resultant precipitate was collected by filtration and dried *in vacuo.* The crude material was purified by preparative HPLC (Method A) to afford 6-(4-(4-(2-(2,6-dioxopiperidin-3-yl)-1,3-dioxoisoindolin-4-yl)piperazin-1-yl)piperidin-1-yl)-*N*-(2-((*R*)-1-methylpyrrolidin-2-yl)-1*H*-pyrrolo[3,2-c]pyridin-6-yl)nicotinamide (**Example 64**, 0.11 g, 20%) as an off-yellow solid. $^1$H NMR (400 MHz, DMSO-$d_6$) δ ppm 1.38-1.51 (m, 2H), 1.76-1.96 (m, 5H), 1.99-2.06 (m, 1H), 2.09-2.14 (m, 1H), 2.16 (s, 3H), 2.22-2.30 (m, 1H), 2.39-2.44 (m, 2H), 2.55-2.63 (m, 3H), 2.66-2.73 (m, 4H), 2.82-2.99 (m, 4H), 3.11-3.17 (m, 1H), 3.25-3.29 (m, 2H), 4.44-4.52 (m, 2H), 5.06-5.12 (m, 1H), 6.37 (s, 1H), 6.90 (d, *J*=9.20 Hz, 1H), 7.30-7.37 (m, 2H), 7.68-7.72 (m, 1H), 8.13-8.17 (m, 2H), 8.48 (s, 1H), 8.79 (d, J=2.40 Hz, 1H), 10.26 (s, 1H), 11.08 (s, 1H), 11.33 (s, 1H). Mass spec m/z 745.3 $[M+H]^+$.

**Example 65 was synthesised following Scheme 63**

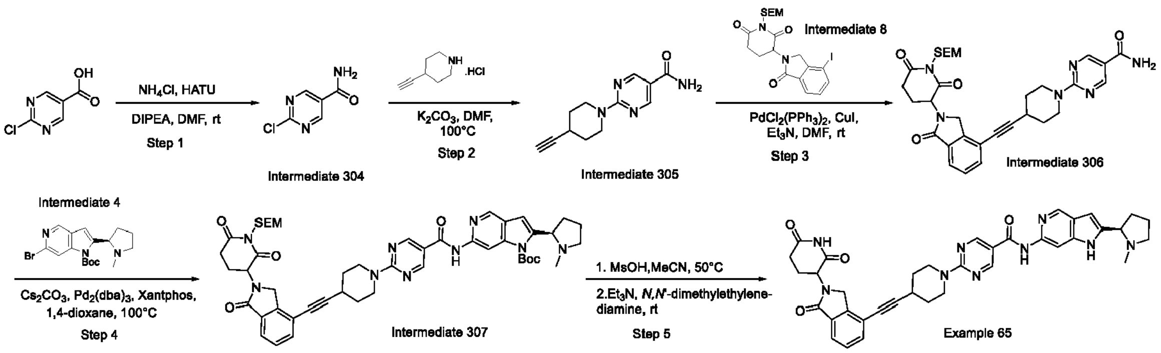


# Scheme 63

### Step 1

**Intermediate 304: 2-chloropyrimidine-5-carboxamide**

To a solution of 2-chloropyrimidine-5-carboxylic acid (CAS No: 374068-01-6, 5.00 g, 31.54 mmol) in dimethylformamide (70 mL) was added HATU (18.93 g, 47.31 mmol) and *N, N*-diisopropylethylamine (16.5 mL, 94.61 mmol) and the reaction mixture was stirred at room temperature for 10 min. Ammonium chloride (6.75 g, 126.15 mmol) was added and the reaction mixture was stirred at room temperature for a further 16h. The reaction mixture was quenched with ice cold water (200 mL), the resultant precipitate was collected by filtration and dried *in vacuo* to afford 2-chloropyrimidine-5-carboxamide **(Intermediate 304,** 5.10 g, 85%) as a white solid, which was used for the next step without further purification. $^1$H NMR (400 MHz, DMSO-$d_6$) δ ppm 7.84 (br s, 1H), 8.26 (br s, 1H), 9.10 (s, 2H). Mass spec m/z 157.1 [M+H]$^+$.

### Step 2

**Intermediate 305: 2-(4-ethynylpiperidin-1-yl)pyrimidine-5-carboxamide**

To a solution of 4-ethynylpiperidine hydrochloride (CAS No: 550378-30-8, 1.80 g, 13 mmol) in dimethylformamide (20 mL) was added 2-chloropyrimidine-5-carboxamide **(Intermediate 304,** 2.00 g, 13 mmol) followed by potassium carbonate (7.0 g, 51 mmol) and the reaction mixture was heated at 100°C for 2h. The reaction mixture was quenched with water (50 mL) and extracted with ethyl acetate (2 × 50 mL). The combined organic layers were dried over anhydrous $Na_2SO_4$, filtered, and concentrated *in vacuo.* The crude material was purified by combi-flash chromatography, by eluting with 50% ethyl acetate in heptane, to afford 2-(4-ethynylpiperidin-1-yl) pyrimidine-5-carboxamide **(Intermediate 305,** 1.30 g, 43%) as an off-white solid. $^1$H NMR (400 MHz, DMSO-$d_6$) δ ppm 1.44-1.55 (m, 2H), 1.78-1.87 (m, 2H), 2.70-2.79 (m, 1H), 3.01 (d, *J*=2.40 Hz, 1H), 3.45-3.56 (m, 2H), 4.14-4.26 (m, 2H), 7.29 (br s, 1H), 7.84 (br s, 1H), 8.77 (s, 2H). Mass spec m/z 231.5 [M+H]$^+$.

### Step 3

**Intermediate 306: 2-(4-((2-(2, 6-dioxo-1-((2-(trimethylsilyl)ethoxy)methyl)piperidin-3-yl)-1-oxoisoindolin-4-yl) ethynyl)piperidin-1-yl)pyrimidine-5-carboxamide**

To a solution of 2-(4-ethynylpiperidin-1-yl) pyrimidine-5-carboxamide **(Intermediate 305,** 0.50 g, 2.17 mmol) in dimethylformamide (15 mL) was added 3-(4-iodo-1-oxoisoindolin-2-yl)-1-((2-(trimethylsilyl)ethoxy)methyl) piperidine-2,6-dione **(Intermediate 8,** 1.08 g, 2.17 mmol) followed by triethylamine (11.0 mL, 78.17 mmol). The reaction mixture was purged with argon for 15 min then copper (I) iodide (0.042 g, 0.22 mmol) and $PdCl_2(PPh_3)_2$ (0.15 g, 0.22 mmol) were added. The reaction mixture was further purged with argon for 10 min and stirred at room temperature for 16h. The reaction mixture was diluted with water (200 mL) and extracted with ethyl acetate (3 × 90 mL). The combined organic layers were dried over anhydrous $Na_2SO_4$, filtered, and concentrated *in vacuo.* The crude material was purified by combi-flash chromatography, by eluting with 80% ethyl acetate in heptane, to afford 2-(4-((2-(2,6-dioxo-1-((2-(trimethylsilylethoxy)methylpiperidin-3-yl)-1-oxoisoindolin-4-yl) ethynyl)piperidin-1-yl)pyrimidine-5-carboxamide **(Intermediate 306,** 0.90 g, 69%) as a yellow solid. $^1$H NMR (400 MHz, DMSO-$d_6$) δ ppm -0.05 (s, 9H), 0.77-0.88 (m, 2H), 1.58-1.70 (m, 2H), 1.88-2.00 (m, 2H), 2.02-2.10 (m, 1H), 2.39-2.47 (m, 1H), 2.76-2.82 (m, 1H), 3.01-3.12 (m, 2H), 3.48-3.64 (m, 4H),

4.22-4.31 (m, 3H), 4.46-4.52 (m, 1H), 5.00-5.10 (m, 2H), 5.24-5.30 (m, 1H), 7.27 (br s, 1H), 7.53 (t, *J*=8.0 Hz, 1H), 7.67 (d, *J*=7.20 Hz, 1H), 7.73 (d, *J*=7.20 Hz, 1H), 7.83 (br s, 1H), 8.77 (s, 2H). Mass spec m/z 603.1 $[M+H]^+$.

**Step 4**

**Intermediate 307: *tert*-butyl-6-(2-(4-((2-(2,6-dioxo-1-((2-(trimethylsilyl)ethoxy)methyl)piperidin-3-yl)-1-oxoisoindolin-4-yl)ethynyl)piperidin-1-yl)pyrimidine-5-carboxamido)-2-((*R*)-1-methylpyrrolidin-2-yl)-1*H*-pyrrolo[3,2-c]pyridine-1-carboxylate**

To a solution of 2-(4-((2-(2, 6-dioxo-1-((2-(trimethylsilyl) ethoxy) methyl) piperidin-3-yl)-1-oxoisoindolin-4-yl) ethynyl) piperidin-1-yl) pyrimidine-5-carboxamide **(Intermediate 306,** 0.79 g, 1.31 mmol) in 1,4-dioxane (10 mL) was added *tert*-butyl-(*R*)-6-bromo-2-(1-methylpyrrolidin-2-yl)-1*H*-pyrrolo[3,2-c]pyridine-1-carboxylate **(Intermediate 4,** 0.50 g, 1.31 mmol) followed by cesium carbonate (0.63 g, 3.29 mmol). The reaction mixture was purged with argon for 15 min then $Pd_2(dba)_3$ (0.12 g, 0.13 mmol) and Xantphos (0.23 g, 0.39 mmol) were added. The reaction mixture was further purged with argon for 10 min and heated at 100°C for 2h. The reaction mixture was concentrated *in vacuo* to obtain the crude compound. The crude material was purified by combi-flash chromatography, by eluting with 70% ethyl acetate in heptane, to afford *tert*-butyl-6-(2-(4-((2-(2,6-dioxo-1-((2-(trimethylsilyl)ethoxy)methyl)piperidin-3-yl)-1-oxoisoindolin-4-yl)ethynyl)piperidin-1-yl)pyrimidine-5-carboxamido)-2-((*R*)-1-methylpyrrolidin-2-yl)-1*H*-pyrrolo[3,2-c]pyridine-1-carboxylate **(Intermediate 307,** 0.80 g, 67%) as a yellow solid. $^1$H NMR (400 MHz, DMSO-$d_6$) δ ppm -0.04 (s, 9H), 0.78-0.88 (m, 2H), 1.20-1.30 (m, 2H), 1.54-1.64 (m, 3H), 1.68 (s, 9H), 1.74-1.80 (m, 2H), 1.97-2.11 (m, 3H), 2.28-2.32 (m, 1H), 2.35 (s, 3H), 2.38-2.42 (m, 1H), 2.75-2.82 (m, 1H), 3.00-3.18 (m, 2H), 3.46-3.66 (m, 4H), 3.86-3.94 (m, 1H), 4.23-4.39 (m, 3H), 4.48-4.52 (m, 1H), 5.00-5.10 (m, 2H), 5.25-5.30 (m, 1H), 6.74 (s, 1H), 7.52-7.58 (m, 1H), 7.68 (d, *J*=7.60 Hz, 1H), 7.73 (d, *J*=7.60 Hz, 1H), 8.58 (s, 1H), 8.90 (s, 1H), 8.95 (s, 2H), 10.71 (br s, 1H). Mass spec m/z 901.9 $[M+H]^+$.

**Step 5**

**Example 65: 2-(4-((2-(2,6-dioxopiperidin-3-yl)-1-oxoisoindolin-4-yl)ethynyl)piperidin-1-yl)-*N*-(2-((*R*)-1-methylpyrrolidin-2-yl)-1*H*-pyrrolo[3,2-c]pyridin-6-yl)pyrimidine-5-carboxamide**

To a solution of *tert*-butyl 6-(2-(4-((2-(2,6-dioxo-1-((2-(trimethylsilyl)ethoxy)methyl)piperidin-3-yl)-1-oxoisoindolin-4-yl)ethynyl)piperidin-1-yl)pyrimidine-5-carboxamido)-2-((*R*)-1-methylpyrrolidin-2-yl)-1*H*-pyrrolo[3,2-c]pyridine-1-carboxylate **(Intermediate 307,** 0.80 g, 0.88 mmol) in acetonitrile (10 mL) was added methanesulfonic acid (0.59 mL, 8.87 mmol) and the reaction mixture was heated at 50°C for 2h. The reaction mixture was cooled to room temperature, then *N,N'*-dimethylethylenediamine (0.53 mL, 4.43 mmol) followed by triethylamine (2.48 mL, 17.74 mmol) were added and the mixture stirred at room temperature for 3h. The reaction mixture was concentrated *in vacuo* and the resultant residue was diluted with water (50 mL). The resultant precipitate was collected by filtration and dried *in vacuo.* The crude solid was purified by preparative HPLC (Method A) to afford 2-(4-((2-(2,6-dioxopiperidin-3-yl)-1-oxoisoindolin-4-yl)ethynyl)piperidin-1-yl)-*N*-(2-((*R*)-1-methylpyrrolidin-2-yl)-1*H*-pyrrolo[3,2-c]pyridin-6-yl)pyrimidine-5-carboxamide **(Example 65,** 0.18 g, 30%) as an off-white solid. $^1$H NMR (400 MHz, DMSO-$d_6$) δ ppm 1.64-1.74 (m, 2H), 1.76-2.04 (m, 6H), 2.09-2.14 (m, 1H), 2.16 (s, 3H), 2.23-2.29 (m, 1H), 2.56-2.63 (m, 1H), 2.86-2.96 (m, 1H), 3.08-3.17 (m, 2H), 3.28-3.30 (m, 2H), 3.62-3.71 (m, 2H), 4.24-4.39 (m, 3H), 4.42-4.51 (m, 1H), 5.10-5.15 (m, 1H), 6.38 (s, 1H), 7.53 (t, *J*=7.60 Hz, 1H), 7.67 (dd, *J*=7.60, 1.20 Hz, 1H), 7.72 (dd, *J*=7.60, 0.80 Hz, 1H), 8.16 (s, 1H), 8.49 (s, 1H), 8.96 (s, 2H), 10.49 (s, 1H), 10.99 (br s, 1H), 11.37 (s, 1H). Mass spec m/z 672.1 $[M+H]^+$.

**Example 66 was synthesised following Scheme 64**

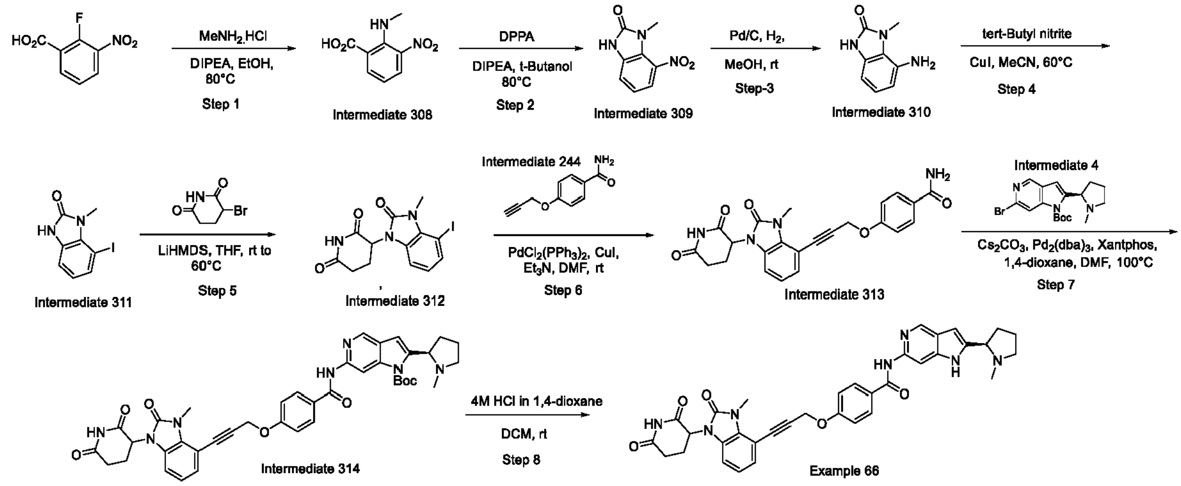


## Scheme 64

### Step 1

**Intermediate 308: 2-(methylamino)-3-nitrobenzoic acid**

To a solution of 2-fluoro-3-nitro-benzoic acid (CAS No: 317-46-4, 20.0 g, 108.04 mmol) in ethanol (300 mL) was added methylamine hydrochloride (37.21 g, 540.22 mmol) followed by *N,N*-diisopropylethylamine (169.0 g, 1296.5 mmol). The reaction mixture was heated at 80°C for 2h then concentrated *in vacuo.* The obtained residue was diluted with water (100 mL) and acidified to pH~3 with 1N aqueous HCl. The resultant precipitate was collected by filtration and dried *in vacuo* to afford 2-(methylamino)-3-nitrobenzoic acid **(Intermediate 308,** 20.0 g, 94%) as a yellow solid, which was used for the next step without further purification. $^1$H NMR (400 MHz, DMSO-$d_6$) δ ppm 2.69 (s, 3H), 6.72 (t, *J*=7.60 Hz, 1H), 7.98 (d, *J*=8.0 Hz, 1H), 8.04 (d, *J*=7.60 Hz, 1H), 8.61 (br s, 1H), 13.46 (br s, 1H). Mass spec m/z 197.0 $[M+H]^+$.

### Step 2

**Intermediate 309: 1-methyl-7-nitro-1,3-dihydro-2*H*-benzo[d]imidazol-2-one**

To a solution of 2-(methylamino)-3-nitrobenzoic acid **(Intermediate 308,** 20.0 g, 100.92 mmol) in *tert*-butanol (250 mL) was added *N,N*-diisopropylethylamine (26.3 g, 201.84 mmol) and diphenylphosphoryl azide (34.35 g, 121.10 mmol). The reaction mixture was heated at 80°C for 2h, then concentrated *in vacuo.* The obtained residue was diluted with water (100 mL), the resultant precipitate was collected by filtration and dried *in vacuo* to afford 1-methyl-7-nitro-1,3-dihydro-2*H*-benzo[d]imidazol-2-one (**Intermediate 309**, 15.0 g, 78%) as a yellow solid, which was used for the next step without further purification. $^1$H NMR (400 MHz, DMSO-$d_6$) δ ppm 3.35 (s, 3H), 7.14 (t, *J*=8.0 Hz, 1H), 7.32 (d, *J*=7.60 Hz, 1H), 7.60 (d, *J*=8.0 Hz, 1H), 11.62 (br s, 1H). Mass spec m/z 192.0 $[M-H]^-$.

### Step 3

**Intermediate 310: 7-amino-1-methyl-1,3-dihydro-2*H*-benzo[d]imidazol-2-one**

To a solution of 1-methyl-7-nitro-1,3-dihydro-2*H*-benzo[d]imidazol-2-one **(Intermediate 309,** 8.0 g, 41.41 mmol) in methanol (350 mL) was added 10% Pd/C (3.00 g) and the reaction mixture was stirred under an atmosphere of hydrogen at 100 psi at room temperature for 16h. The reaction mixture was filtered through celite bed and the filter pad was washed with methanol (100 mL). The filtrate was concentrated *in vacuo* to afford 7-amino-1-methyl-1,3-dihydro-2H-benzo[d] imidazol-2-one **(Intermediate 310,** 6.50 g, 96%) as a brown solid, which was used for the next step without further purification. $^1$H NMR (400 MHz, DMSO-$d_6$) δ ppm 3.51 (s, 3H), 4.85 (br s, 2H), 6.30 (d, *J*=7.60 Hz, 1H), 6.35 (d, *J*=8.0 Hz, 1H), 6.68 (t, *J*=8.0 Hz, 1H), 10.54 (br s, 1H).

### Step 4

**Intermediate 311: 7-iodo-1-methyl-1,3-dihydro-2*H*-benzo[d]imidazol-2-one**

To a solution of 7-amino-1-methyl-1, 3-dihydro-2*H*-benzo[d]imidazol-2-one **(Intermediate 310,** 6.50 g, 40 mmol) in acetonitrile (120 mL) was added copper (I) iodide (12.0 g, 60.0 mmol). The reaction mixture was purged with argon for 20 min then tert-butyl nitrite (6.8 g, 60.0 mmol) was added and the reaction heated at 60°C for 16h. The reaction mixture was concentrated *in vacuo* and the crude material was purified by combi-flash chromatography, by eluting with 12% MeOH in dichloromethane, to afford 7-iodo-1-methyl-1,3-dihydro-2*H*-benzo[d]imidazol-2-one **(Intermediate 311,** 2.50 g, 23%) as a yellow solid. $^1$H NMR (400 MHz, DMSO-$d_6$) δ ppm 3.56 (s, 3H), 6.75 (t, *J*=7.60 Hz, 1H), 6.98 (d, *J*=7.60 Hz, 1H), 7.38 (d, *J*=8.0 Hz, 1H), 11.07 (br s, 1H). Mass spec m/z 275.0 [M+H]$^+$.

**Step 5**

**Intermediate 312: 3-(4-iodo-3-methyl-2-oxo-2,3-dihydro-1*H*-benzo[d]imidazol-1-yl)piperidine-2,6-dione**

To a solution of 7-iodo-1-methyl-1,3-dihydro-2*H*-benzo[d]imidazol-2-one **(Intermediate 311,** 2.50 g, 9.1 mmol) in tetrahydrofuran (20 mL) was added 1.0 M LiHMDS in tetrahydrofuran (23 mL, 23 mmol) dropwise and the reaction mixture was stirred at room temperature for 30 min. The above reaction mixture was then added to a solution of 3-bromopiperidine-2, 6-dione (CAS No: 62595-74-8, 3.50 g, 18.0 mmol) in tetrahydrofuran (30 mL) and the reaction heated at 60°C for 4h. The reaction mixture was quenched with saturated aqueous ammonium chloride solution (50 mL) and the mixture stirred at room temperature for 30 min. The resultant precipitate was collected by filtration, washed with water (50 mL) and dried *in vacuo* to afford 3-(4-iodo-3-methyl-2-oxo-2,3-dihydro-1*H*-benzo[d]imidazol-1-yl)piperidine-2,6-dione **(Intermediate 312,** 0.51 g) as an off-white solid, which was used for the next step without further purification. $^1$H NMR (400 MHz, DMSO-$d_6$) δ ppm 1.98-2.04 (m, 1H), 2.58-2.77 (m, 2H), 2.82-2.94 (m, 1H), 3.64 (s, 3H), 5.34-5.45 (m, 1H), 6.82 (t, *J*=7.60 Hz, 1H), 7.16 (d, *J*=7.60 Hz, 1H), 7.48 (d, *J*=7.60 Hz, 1H), 11.11 (br s, 1H). Mass spec m/z 386.06 [M+H]$^+$.

**Step 6**

**Intermediate 313: 4-((3-(1-(2,6-dioxopiperidin-3-yl)-3-methyl-2-oxo-2,3-dihydro-1*H*-benzo[d]imidazol-4-yl) prop-2-yn-1-yl)oxy)benzamide**

To a solution of 3-(4-iodo-3-methyl-2-oxo-2,3-dihydro-1*H*-benzo[d]imidazol-1-yl)piperidine-2,6-dione **(Intermediate 312,** 0.45 g, 1.16 mmol) in dimethylformamide (5 mL) was added 4-(prop-2-yn-1-yloxy)benzamide **(Intermediate 244,** 0.30 g, 1.75 mmol) followed by triethylamine (6.1 mL, 43.22 mmol). The reaction mixture was purged with argon for 15 min, then copper (I) iodide (0.04 g, 0.23 mmol) and $PdCl_2(PPh_3)_2$ (0.08 g, 0.11 mmol) were added. The reaction mixture was purged with argon for another 10 min and then stirred at room temperature for 1h. The reaction mixture was concentrated *in vacuo* and the crude material was purified by combi-flash chromatography, by eluting with 10% MeOH in dichloromethane, to afford 4-((3-(1-(2,6-dioxopiperidin-3-yl)-3-methyl-2-oxo-2,3-dihydro-1*H*-benzo[d]imidazol-4-yl) prop-2-yn-1-yl) oxy) benzamide **(Intermediate 313**, 0.21 g, 42%) as an off-white solid. $^1$H NMR (400 MHz, DMSO-$d_6$) δ ppm 1.94-2.06 (m, 1H), 2.58-2.71 (m, 2H), 2.81-2.93 (m, 1H), 3.48 (s, 3H), 5.20 (s, 2H), 5.34-5.42 (m, 1H), 6.98-7.05 (m, 1H), 7.08-7.14 (m, 3H), 7.15-7.22 (m, 2H), 7.82-7.90 (m, 3H), 11.10 (s, 1H). Mass spec m/z 433.20 [M+H]$^+$.

**Step 7**

**Intermediate 314: *tert*-butyl-6-(4-((3-(1-(2,6-dioxopiperidin-3-yl)-3-methyl-2-oxo-2,3-dihydro-1*H*-benzo[d]imidazol-4-yl)prop-2-yn-1-yl)oxy)benzamido)-2-((*R*)-1-methylpyrrolidin-2-yl)-1*H*-pyrrolo[3,2-c]pyridine-1-carboxylate**

To a solution of 4-((3-(1-(2, 6-dioxopiperidin-3-yl)-3-methyl-2-oxo-2,3-dihydro-1*H*-benzo[d]imidazol-4-yl) prop-2-yn-1-yl) oxy)benzamide **(Intermediate 313,** 0.50 g, 1.15 mmol) in 1,4-dioxane (30 mL) and dimethylformamide (3 mL) was added *tert*-butyl-(*R*)-6-bromo-2-(1-methylpyrrolidin-2-yl)-1*H*-pyrrolo[3,2-c]pyridine-1-carboxylate **(Intermediate 4,** 0.52 g, 1.38 mmol) followed by cesium carbonate (1.14 g, 3.46 mmol). The reaction mixture was purged with argon for 15 min, then $Pd_2(dba)_3$ (0.16 g, 0.17 mmol) and Xantphos (0.20 g, 0.34 mmol) were added. The reaction mixture was further purged with argon for another 10 min and then heated at 100°C for 2h. The reaction mixture was concentrated *in vacuo* and the crude material was purified by combi-flash chromatography, by eluting with 11% MeOH in dichloromethane, to afford *tert*-butyl-6-(4-((3-(1-(2,6-dioxopiperidin-3-yl)-3-methyl-2-oxo-2,3-dihydro-1*H*-benzo[d]imidazol-4-yl)prop-2-yn-1-yl)oxy)benzamido)-2-((*R*)-1-methylpyrrolidin-2-yl)-1*H*-pyrrolo[3,2-c]pyridine-1-carboxylate **(Intermediate 314,** 0.40 g, 47%) as an off-white solid. $^1$H NMR (400 MHz, DMSO-$d_6$) δ ppm 1.56-1.65 (m, 2H), 1.69 (s, 9H), 1.72-1.80 (m, 2H), 1.96-2.05 (m, 2H), 2.28-2.33 (m, 1H), 2.35 (s, 3H), 2.38-2.40 (m, 1H), 2.64-2.68 (m, 1H), 2.81-2.92 (m, 1H), 3.13-3.18 (m, 1H), 3.51 (s, 3H), 5.25 (s, 2H), 5.36-5.40 (m, 1H), 6.74 (s, 1H), 6.99-7.05 (m, 1H), 7.17 (d, *J*=8.80 Hz,

2H), 7.87 (d, *J*=8.80 Hz, 2H), 8.10 (d, *J*=8.80 Hz, 2H), (s, 1H), 8.92 (s, 1H), 10.60 (s, 1H), 11.11 (s, 1H). Mass spec m/z 732.45 $[M+H]^+$.

**Step 8**

**Example 66: 4-((3-(1-(2,6-dioxopiperidin-3-yl)-3-methyl-2-oxo-2,3-dihydro-1*H*-benzo[d]imidazol-4-yl)prop-2-yn-1-yl)oxy)-*N*-(2-((*R*)-1-methylpyrrolidin-2-yl)-1*H*-pyrrolo[3,2-c]pyridin-6-yl)benzamide**

To a cooled (0°C) solution of *tert*-butyl-6-(4-((3-(1-(2,6-dioxopiperidin-3-yl)-3-methyl-2-oxo-2,3-dihydro-1*H*-benzo[d]imidazol-4-yl)prop-2-yn-1-yl)oxy)benzamido)-2-((*R*)-1-methylpyrrolidin-2-yl)-1*H*-pyrrolo[3,2-c]pyridine-1-carboxylate **(Intermediate 314,** 0.40 g, 0.54 mmol) in dichloromethane (4 mL) was added 4M hydrochloric acid in 1,4-dioxane (2 mL) and the reaction mixture was stirred at room temperature for 4h. The reaction mixture was concentrated *in vacuo* and the crude material was purified by preparative HPLC (Method A) to afford 4-((3-(1-(2,6-dioxopiperidin-3-yl)-3-methyl-2-oxo-2,3-dihydro-1*H*-benzo[d]imidazol-4-yl)prop-2-yn-1-yl)oxy)-*N*-(2-((R)-1-methylpyrrolidin-2-yl)-1*H*-pyrrolo[3,2-c]pyridin-6-yl)benzamide **(Example 66,** 0.10 g, 31%) as an off white solid. $^1$H NMR (400 MHz, DMSO-$d_6$) δ ppm 1.75-1.95 (m, 3H), 1.98-2.04 (m, 1H), 2.09-2.14 (m, 1H), 2.17 (s, 3H), 2.23-2.30 (m, 1H), 2.58-2.71 (m, 3H), 2.81-2.92 (m, 1H), 3.12-3.16 (m, 1H), 3.51 (s, 3H), 5.25 (s, 2H), 5.36-5.40 (m, 1H), 6.38 (s, 1H), 7.03 (t, *J*=8.0 Hz, 1H), 7.11-7.14 (m, 1H), 7.15-7.20 (m, 3H), 8.06-8.11 (m, 2H), 8.18 (s, 1H), 8.49 (s, 1H), 10.35 (s, 1H), 11.10 (s, 1H), 11.34 (s, 1H). Mass spec m/z 632.2 $[M+H]^+$.

**Example 67 was synthesised following Scheme 65**

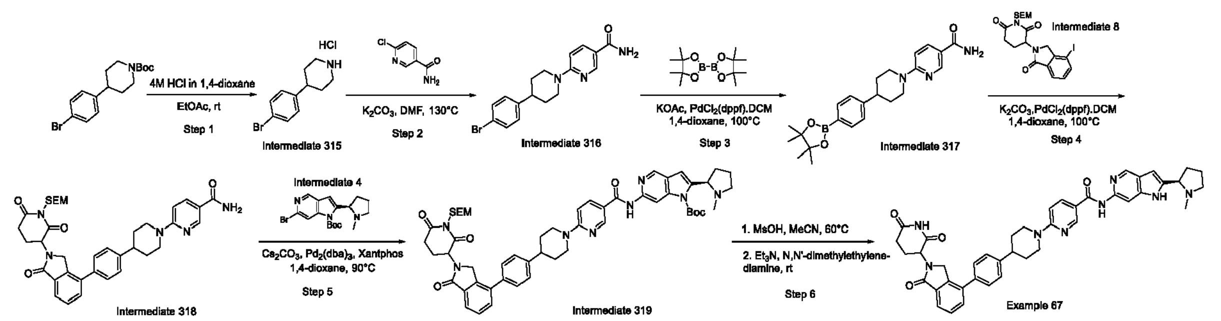


**Scheme 65**

**Step 1**

**Intermediate 315: 4-(4-bromophenyl)piperidine hydrochloride**

To a solution of *tert*-butyl-4-(4-bromophenyl)piperidine-1-carboxylate (CAS No: 352437-09-3, 6.00 g, 17.6) in ethyl acetate (10 mL) was added 4M hydrochloric acid in 1,4-dioxane (20 mL) at 0°C. The reaction mixture was stirred at room temperature for 16h. Progress of the reaction was monitored by TLC. After completion, the reaction mixture was concentrated *in vacuo* and washed with *n*-pentane (20 ml) to afford 4-(4-bromophenyl)piperidine hydrochloride **(Intermediate 315,** 4.60 g, 94%) as an off white solid, which was used for the next step without further purification. $^1$H NMR (400 MHz, DMSO-$d_6$) δ ppm 1.75-1.94 (m, 4H), 2.77-2.88 (m, 1H), 2.90-3.02 (m, 2H), 3.28-3.34 (m, 2H), 7.13-7.25 (m, 2H), 7.45-7.59 (m, 2H), 8.87 (br s, 1H), 8.97 (br s, 1H). Mass spec m/z 240.3 $[M+H]^+$.

**Step 2**

**Intermediate 316: 6-(4-(4-bromophenyl) piperidin-1-yl)nicotinamide**

To a solution of 4-(4-bromophenyl)piperidine hydrochloride **(Intermediate 315,** 4.00 g, 14.0 mmol) in dimethylformamide (30 mL) was added 6-chloronicotinamide (CAS No: 6271-78-9, 2.00 g, 13.0 mmol) followed by potassium carbonate (8.00 g, 58.0 mmol) and the reaction mixture was stirred at 130°C for 16h. The reaction mixture was poured into ice cold water (300 mL). The resultant precipitate was collected by filtration, washed with water (50 mL and dried *in vacuo* to afford 6-(4-(4-bromophenyl) piperidin-1-yl) nicotinamide **(Intermediate 316,** 4.50 g, 86%) as an off white solid, which was

used for the next step without further purification. $^1$H NMR (400 MHz, DMSO-$d_6$) δ ppm 1.51-1.63 (m, 2H), 1.75-1.88 (m, 2H), 2.78-2.86 (m, 1H), 2.91-3.00 (m, 2H), 4.50-4.62 (m, 2H), 6.86-6.88 (m, 1H), 7.08 (br s, 1H), 7.22 (d, *J*=8.0 Hz, 2H), 7.47 (d, *J*=8.0 Hz, 2H), 7.72 (br s, 1H), 7.92-7.96 (m, 1H), 8.62 (s, 1H). Mass spec m/z 360.1 [M+H]$^+$.

**Step 3**

**Intermediate 317: 6-(4-(4-(4,4,5,5-tetramethyl-1,3,2-dioxaborolan-2-ylphenyl) piperidin-1-yl) nicotinamide**

To a solution of 6-(4-(4-bromophenyl)piperidin-1-yl)nicotinamide **(Intermediate 316,** 1.50 g, 4.2 mmol) 1,4-dioxane (30 mL) was added bis(pinacolato)diboron (CAS No: 73183-34-3, 1.40 g, 5.4 mmol) followed by potassium acetate (1.2 g, 12.0 mmol). The reaction mixture was purged with argon for 15 min then $PdCl_2$(dppf).DCM (0.34 g, 0.42 mmol) was added. The reaction mixture was purged with argon for another 10 min and then stirred at 100°C for 4h. The reaction mixture was then concentrated *in vacuo.* The obtained solid was washed with diethyl ether (2 × 50 mL) and dried *in vacuo* to afford 6-(4-(4-(4,4,5,5-tetramethyl-1,3,2-dioxaborolan-2-yl)phenyl) piperidin-1-yl)nicotinamide **(Intermediate 317,** 3.60 g) as a grey solid, which was used for the next step without further purification. $^1$H NMR (400 MHz, DMSO-$d_6$) δ ppm 1.15 (s, 6H), 1.27 (s, 6H), 1.50-1.65 (m, 2H), 1.81-1.86 (m, 2H), 2.83-3.00 (m, 3H), 4.50-4.58 (m, 2H), 6.86-6.88 (m, 1H), 7.10 (br s, 1H), 7.26 (d, *J*=7.60 Hz, 2H), 7.60 (d, *J*=7.60 Hz, 2H), 7.76 (br s, 1H), 7.92-7.96 (m, 1H), 8.62 (s, 1H). Mass spec m/z 408.2 [M+H]$^+$.

**Step 4**

**Intermediate 318: 6-(4-(4-(2-(2,6-dioxo-1-((2-(trimethylsilyl)ethoxy)methyl)piperidin-3-yl)-1-oxoisoindolin-4-yl) phenyl)piperidin-1-yl)nicotinamide**

To a solution of 6-(4-(4-(4,4,5,5-tetramethyl-1,3,2-dioxaborolan-2-yl)phenyl)piperidin-1-yl)nicotinamide **(Intermediate 317,** 3.09 g, 7.59 mmol) in 1,4-dioxane (30 mL) was added 3-(4-iodo-1-oxoisoindolin-2-yl)-1-((2-(trimethylsilyl) ethoxy)methyl)piperidine-2,6-dione **(Intermediate 8,** 1.90 g, 3.80 mmol) followed by potassium carbonate (1.57 g, 11.4 mmol). The reaction mixture was purged with argon for 15 min then $PdCl_2$(dppf).DCM (0.31 g, 0.38 mmol) was added. The reaction mixture was purged with argon for another 10 min and then stirred at 100°C for 4h. The mixture was concentrated *in vacuo* and the crude material was purified by combi-flash column chromatography, by eluting with 3% MeOH in dichloromethane, to afford 6-(4-(4-(2-(2, 6-dioxo-1-((2-(trimethylsilyl)ethoxy)methylpiperidin-3-yl)-1-oxoisoindolin-4-yl) phenyl)piperidin-1-yl)nicotinamide **(Intermediate 318,** 1.20 g, 48%) as a yellow solid. $^1$H NMR (400 MHz, DMSO-$d_6$) δ ppm -0.10 (s, 9H), 0.71-0.82 (m, 2H), 1.54-1.68 (m, 2H), 1.80-1.90 (m, 2H), 1.96-2.04 (m, 1H), 2.36-2.40 (m, 1H), 2.70-2.78 (m, 1H), 2.86-3.04 (m, 4H), 3.42-3.52 (m, 2H), 4.28-4.32 (m, 1H), 4.52-4.63 (m, 3H), 4.95-5.05 (m, 2H), 5.23-5.27 (m, 1H), 6.84-6.86 (m, 1H), 7.05 (br s, 1H), 7.35 (d, *J*=8.20 Hz, 2H), 7.49 (d, *J*=8.20 Hz, 2H), 7.57-7.74 (m, 4H), 7.93 (dd, *J*=9.12, 2.07 Hz, 1H), 8.60 (d, *J*=2.07 Hz, 1H). Mass spec m/z 654.1 [M+H]$^+$.

**Step 5**

**Intermediate 319: tert-butyl-6-(6-(4-(4-(2-(2,6-dioxo-1-((2-(trimethylsilyl)ethoxy)methyl)piperidin-3-yl)-1-oxoisoindolin-4-yl)phenyl)piperidin-1-yl)nicotinamido)-2-((*R*)-1-methylpyrrolidin-2-yl)-1*H*-pyrrolo[3,2-c]pyridine-1-carboxylate**

To a solution of 6-(4-(4-(2-(2,6-dioxo-1-((2-(trimethylsilyl)ethoxy)methyl)piperidin-3-yl)-1-oxoisoindolin-4-yl) phenyl)piperidin-1-yl)nicotinamide **(Intermediate 318,** 1.10 g, 1.7 mmol) in 1,4-dioxane (18 mL) was added *tert*-butyl-(*R*)-6-bromo-2-(1-methylpyrrolidin-2-yl)-1*H*-pyrrolo[3,2-*c*]pyridine-1-carboxylate **(Intermediate 4,** 0.70 g, 1.9 mmol) followed by cesium carbonate (1.5 g, 4.5 mmol). The reaction mixture was purged with argon for 15 min then $Pd_2(dba)_3$ (0.24 g, 0.25 mmol) and Xantphos (0.30 g, 0.50 mmol) were added. The reaction mixture was purged with argon for another 10 min and then stirred at 90°C for 3h. The reaction mixture was filtered through celite bed and the filter pad was washed with ethyl acetate (100 mL), and the filtrate concentrated *in vacuo.* The obtained solid residue was washed with diethyl ether (2 × 50 mL) dried *in vacuo* to afford *tert*-butyl-6-(6-(4-(4-(2-(2,6-dioxo-1-((2-(trimethylsilyl)ethoxy)methyl) piperidin-3-yl)-1-oxoisoindolin-4-yl)phenyl)piperidin-1-yl)nicotinamido)-2-((*R*)-1-methylpyrrolidin-2-yl)-1*H*-pyrrolo[3,2-c] pyridine-1-carboxylate **(Intermediate 319,** 0.80 g) as a yellow solid, which was used for the next step without further purification. $^1$H NMR (400 MHz, DMSO-$d_6$) δ ppm -0.08 (s, 9H), 0.76-0.84 (m, 2H), 1.06-1.11 (m, 2H), 1.56-1.62 (m, 2H), 1.69 (s, 9H), 1.72-1.80 (m, 2H), 1.86-1.96 (m, 2H), 1.98-2.07 (m, 3H), 2.29-2.33 (m, 2H), 2.35 (s, 3H) 2.38-2.42 (m, 1H), 2.76-2.80 (m, 1H), 3.13-3.25 (m, 1H), 3.35-3.41 (m, 2H), 3.48-3.52 (m, 2H), 4.40-4.44 (m, 2H), 4.58-4.69 (m, 2H), 4.99-5.08 (m, 2H), 5.12-5.16 (m, 1H), 6.74 (s, 1H), 6.93-6.95 (m, 1H), 7.34-7.40 (m, 1H), 7.50-7.56 (m, 2H), 7.62-7.66 (m, 1H), 7.67-7.76 (m, 2H), 8.15-8.21 (m, 2H), 8.57 (s, 1H), 8.83 (s, 1H), 8.92 (s, 1H), 10.50 (br s, 1H). Mass spec m/z 952.8

[M+H]+.

**Step 6**

**Example 67: 6-(4-(4-(2-(2,6-dioxopiperidin-3-yl)-1-oxoisoindolin-4-yl)phenyl)piperidin-1-yl)-*N*-(2-((*R*)-1-methylpyrrolidin-2-yl)-1*H*-pyrrolo[3,2-*c*]pyridin-6-yl)nicotinamide**

To a solution of *tert*-butyl-6-(6-(4-(4-(2-(2,6-dioxo-1-((2-(trimethylsilyl)ethoxy)methyl)piperidin-3-yl)-1-oxoisoindolin-4-yl)phenyl)piperidin-1-yl)nicotinamido)-2-((*R*)-1-methylpyrrolidin-2-yl)-1*H*-pyrrolo[3,2-*c*]pyridine-1-carboxylate **(Intermediate 319,** 0.75 g, 0.78 mmol) in acetonitrile (8.0 mL) was added methanesulfonic acid (0.52 mL, 7.86 mmol) and the reaction mixture was stirred at 60°C for 2h. After cooling to room temperature, *N*, *N'*-dimethylethylenediamine (0.47 mL, 3.93 mmol) followed by triethylamine (2.20 mL, 15.74 mmol) were added the reaction mixture was stirred at room temperature for 3h. The reaction mixture was concentrated *in vacuo* and the obtained residue was diluted with water (50 mL). The resultant precipitate was collected by filtration and dried *in vacuo.* The crude solid was purified by preparative HPLC (Method B) to afford 6-(4-(4-(2-(2,6-dioxopiperidin-3-yl)-1-oxoisoindolin-4-yl)phenyl)piperidin-1-yl)-*N*-(2-((*R*)-1-methylpyrrolidin-2-yl)-1*H*-pyrrolo[3,2-*c*]pyridin-6-yl)nicotinamide **(Example 67,** 0.03 g, 7%) as an off white solid. $^1$H NMR (400 MHz, DMSO-$d_6$) δ ppm 1.60-1.74 (m, 2H), 1.80-2.05 (m, 6H), 2.22 (s, 3H), 2.42-2.46 (m, 2H), 2.56-2.58 (m, 2H), 2.83-3.08 (m, 5H), 3.13-3.25 (m, 1H), 4.40-4.44 (m, 1H), 4.58-4.69 (m, 3H), 5.12-5.16 (m, 1H), 6.45 (s, 1H), 6.93-6.95 (m, 1H), 7.40 (d, *J*=8.40 Hz, 2H), 7.54 (d, *J*=8.40 Hz, 2H), 7.60-7.66 (m, 1H), 7.67-7.76 (m, 2H), 8.15-8.21 (m, 2H), 8.52 (s, 1H), 8.81-8.82 (m, 1H), 10.32 (br s, 1H), 10.97 (s, 1H), 11.41 (br s, 1H). Mass spec m/z 723.0 [M+H]+.

**Example 68 was synthesised following Scheme 66**

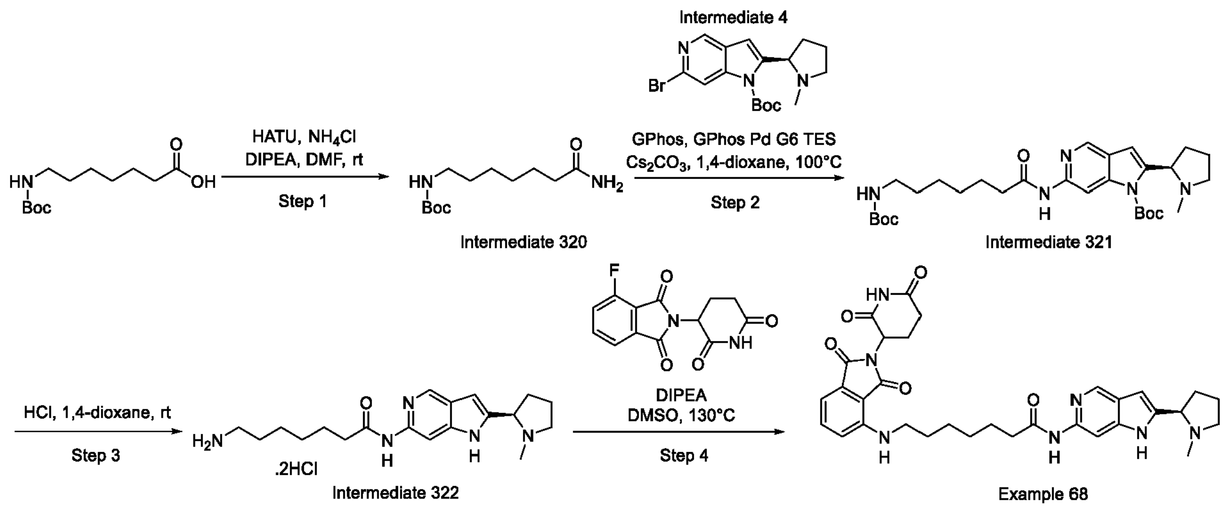


**Scheme 66**

**Step 1**

**Intermediate 320: *tert*-butyl N-(6-carbamoylhexyl)carbamate**

To a solution of 7-[(tert-butoxycarbonyl)amino]heptanoic acid (CAS No: 60142-89-4, 500 mg, 2.04 mmol) in DMF (5 mL) were added DIPEA (790 mg, 6.12 mmol), HATU (1.16 g, 3.06 mmol) and $NH_4Cl$ (545 mg, 10.2 mmol). The reaction mixture was stirred for 2h at rt. The reaction mixture was diluted with water and extracted three times with EtOAc. The combined organic layers were washed with brine, dried over anhydrous $Na_2SO_4$, filtered and concentrated under reduced pressure. The crude material was purified by flash chromatography on silica gel using petroleum ether/EtOAc (9/1) as eluent to afford *tert*-butyl N-(6-carbamoylhexyl)carbamate **(Intermediate 320,** 363 mg, 73%) as a white solid. Mass spec: m/z: 315 [M+H]+.

**Step 2**

**Intermediate 321: *tert*-butyl 6-{7-[(tert-butoxycarbonyl)amino]heptanamido}-2-[(2R)-1-methylpyrrolidin-2-yl]pyrrolo[3,2-c]pyridine-1-carboxylate**

To a solution of *tert*-butyl N-(6-carbamoylhexyl)carbamate **(Intermediate 320,** 200 mg, 0.737 mmol), tert-butyl 6-bromo-2-[(rel-2R)-1-methylpyrrolidin-2-yl]pyrrolo[3,2-c]pyridine-1-carboxylate **(Intermediate 4,** 364 mg, 0.958 mmol) and $Cs_2CO_3$ (720 mg, 2.21 mmol in 1,4-dioxane (4 mL) and) were added GPhos (80 mg, 0.147 mmol) and GPhos Pd G6 TES (70 mg, 0.0740 mmol). The reaction mixture was stirred at 100°C overnight under nitrogen atmosphere, then concentrated under reduced pressure. The residue was chromatographed by reverse-phase flash chromatography (C18) using MeCN/$H_2O$ (36/64) as eluent to afford *tert*-butyl 6-{7-[(tert-butoxycarbonyl)amino]heptanamido}-2-[(2R)-1-methylpyrrolidin-2-yl]pyrrolo[3,2-c]pyridine-1-carboxylate **(Intermediate 321,** 310 mg, 69%) as a yellow solid. Mass spec: m/z: 544 $[M+H]^+$.

**Step 3**

**Intermediate 322: 7-amino-N-{2-[(2R)-1-methylpyrrolidin-2-yl]-1H-pyrrolo[3,2-c]pyridin-6-yl}heptanamide dihydrochloride**

To a solution of *tert*-butyl 6-{7-[(tert-butoxycarbonyl)amino]heptanamido}-2-[(rel-2R)-1-methylpyrrolidin-2-yl]pyrrolo[3,2-c]pyridine-1-carboxylate **(Intermediate 321,** 310 mg, 0.507 mmol) in 1,4-dioxane (10 mL) was added a solution of HCl (2 mL, 12N in $H_2O$). The reaction mixture was stirred at rt overnight, then concentrated under reduced pressure to afford 7-amino-N-{2-[(2R)-1-methylpyrrolidin-2-yl]-1H-pyrrolo[3,2-c]pyridin-6-yl}heptanamide dihydrochloride **(Intermediate 322,** 210 mg, 49%) as a yellow solid which was used in Step 4 without further purification. Mass spec: m/z: 344 $[M+H]^+$.

**Step 4**

**Example 68: 7-{[2-(2,6-dioxopiperidin-3-yl)-1,3-dioxoisoindol-4-yl]amino}-N-12-[(2R)-1-methylpyrrolidin-2-yl]-1H-pyrrolo[3,2-c]pyridin-6-yl}heptanamide**

A solution of 7-amino-N- { 2-[(2R)-1-methylpyrrolidin-2-yl]-1H-pyrrolo[3,2-c]pyridin-6-yl}heptanamide dihydrochloride **(Intermediate 322,** 200 mg, 0.262 mmol) in DMSO (4 mL) were added 2-(2,6-dioxopiperidin-3-yl)-4-fluoroisoindole-1,3-dione (CAS: 835616-60-9, 145 mg, 0.524 mmol) and DPIEA (339 mg, 2.62 mmol). The reaction mixture was stirred at 130°C for 2h, then quenched with water and concentrated under reduced pressure. The residue was purified by preparative HPLC (Column: Xselect CSH OBD, 30 × 150 mm, 5μm; Eluent: Water (+0.1% formic acid)/MeCN from 98/2 to 70/30; Flow rate: 60 mL/min mL/min) to afford 7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}heptanamide **(Example 68,** 46.8 mg, 27%) as a yellow solid. Mass spec: m/z: 600 $[M+H]^+$; $^1H$ NMR (400 MHz, DMSO-$d_6$) δ ppm 11.25 (s, 1H), 11.10 (s, 1H), 10.10 (s, 1H), 8.40 (s, 1H), 8.15 (s, 1H), 8.07 (s, 1H), 7.54 - 7.58 (m, 1H), 7.07 - 7.10 (m, 1H), 7.00 - 7.02 (s, 1H), 6.52 - 6.55 (m, 1H), 6.33 (s, 1H), 5.02 - 5.07 (m, 1H), 3.28 - 3.31 (m, 3H), 3.12 - 3.26 (m, 1H), 2.82 - 2.94 (m, 1H), 2.50 - 2.60 (m, 2H), 2.34 - 2.38 (m, 2H), 2.24 - 2.26 (m, 1H), 2.11 - 2.14 (m, 4H), 2.01 - 2.04 (m, 1H), 1.79 -1.81 (m, 3H), 1.43 - 1.65 (m, 4H), 1.31 - 1.42 (m, 4H).

**Example 69 was synthesised following Scheme 67**

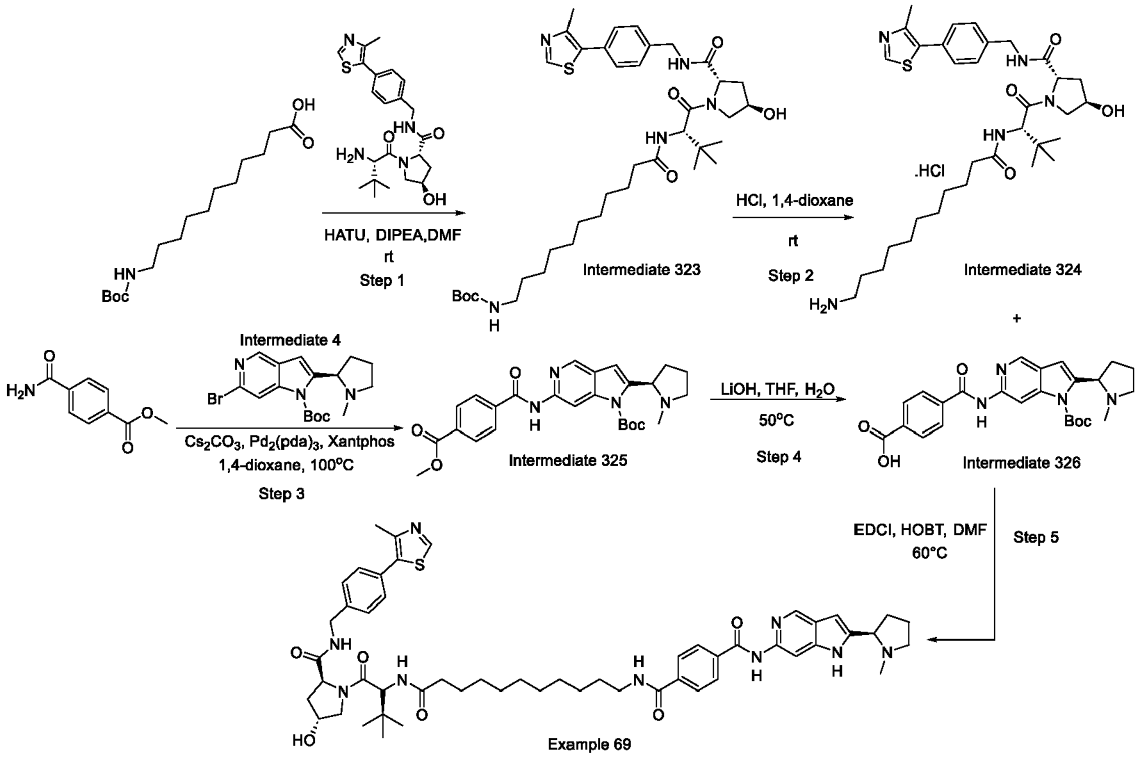


**Scheme 67**

### Step 1

**Intermediate 323: tert-butyl N-(10-{[(2S)-1-[(2S,4R)-4-hydroxy-2-({[4-(4-methyl-1,3-thiazol-5-yl)phenyl]methyl} carbamoyl)pyrrolidin-1-yl]-3,3-dimethyl-1-oxobutan-2-yl]carbamoyl}decyl)carbamate**

To a solution of 11-[(tert-butoxycarbonyl)amino]undecanoic acid (CAS No: 10436-25-6, 5 g, 16.5 mmol) in DMF (100 mL) was added (2S,4R)-1-[(2S)-2-amino-3,3-dimethylbutanoyl]-4-hydroxy-N-{[4-(4-methyl-1,3-thiazol-5-yl)phenyl] methyl}pyrrolidine-2-carboxamide hydrochloride (CAS No: 1448189-80-7, 7.14 g, 16.5 mmol), DIPEA (8.58 g, 66.3 mmol) and HATU (8.20 g, 21.5 mmol) under a nitrogen atmoshphere. The reaction mixture was stirred at rt for 4h. Water (200 mL) was added and the resulting mixture was extracted with EtOAc (3 × 500 mL) and the organic layers were combined, washed with brine (200 mL), dried over anhydrous sodium sulfate, filtered and concentrated under reduced pressure. The residue was chromatographed on a silica gel column, eluting with DCM/MeOH (20/1), to afford tert-butyl N-(10-{ [(2S)-1-[(2S,4R)-4-hydroxy-2-({ [4-(4-methyl-1,3-thiazol-5-yl)phenyl]methyl}carbamoyl)pyrrolidin-1-yl]-3,3-dimethyl-1-oxobutan-2-yl]carbamoyl}decyl)carbamate **(Intermediate 323,** 8 g, 67.5%) as a yellow solid. Mass spec: m/z:714.7 $[M+H]^+$.

### Step 2

**Intermediate 324: (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide hydrochloride**

To a solution of tert-butyl N-(10-{[(2S)-1-[(2S,4R)-4-hydroxy-2-({[4-(4-methyl-1,3-thiazol-5-yl)phenyl]methyl} carbamoyl)pyrrolidin-1-yl]-3,3-dimethyl-1-oxobutan-2-yl]carbamoyl}decyl)carbamate (**Intermediate 323,** 700 mg, 0.980 mmol) in 1,4-dioxane (3 mL) was added HCl (3 mL, 4M in 1,4-dioxane) and the reaction mixture was stirred at room temperature for 1h. The reaction was then concentrated under reduced pressure to afford (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide hydrochloride (**Intermediate 324,** 600 mg) as a yellow solid, which was used in the next step without further purification. Mass spec: m/z: 614.4 $[M+H]^+$.

**Step 3**

**Intermediate 325: tert-butyl 6-[4-(methoxycarbonyl)benzamido]-2-[(2R)-1-methylpyrrolidin-2-yl]pyrrolo[3,2-c]pyridine-1-carboxylate**

To a solution of tert-butyl 6-bromo-2-[(2R)-1-methylpyrrolidin-2-yl]pyrrolo[3,2-c]pyridine-1-carboxylate (**Intermediate 4,** 500 mg, 1.31 mmol) in 1,4-dioxane (10 mL) was added methyl 4-carbamoylbenzoate (CAS No: 6757-31-9, 235 mg, 1.31 mmol), $Cs_2CO_3$ (1.29 g, 3.94 mmol), Xantphos (152 mg, 0.263 mmoL) and $Pd_2(dba)_3$ (120 mg, 0.131 mmol) and the reaction was stirred at 100°C overnight under $N_2$. The mixture was quenched with water (100 mL) and extracted with EtOAc (3 × 100 mL). The combined organic layers were washed with brine, dried over anhydrous $Na_2SO_4$, filtered and concentrated under reduced pressure. The residue was chromatographed on a silica gel column, eluting with DCM / MeOH (95/5), to afford tert-butyl6-[4-(methoxycarbonyl)benzamido]-2-[(2R)-1-methylpyrrolidin-2-yl]pyrrolo[3,2-c]pyridine-1-carboxylate **(Intermediate 325,** 300 mg, 41%) as a yellow solid. Mass spec: m/z: 479.3 $[M+H]^+$.

**Step 4**

**Intermediate 326: 4-({2-[(2R)-1-methylpyrrolidin-2-yl]-1H-pyrrolo[3,2-c]pyridin-6-yl}carbamoyl)benzoic acid**

To a solution of tert-butyl 6-[4-(methoxycarbonyl)benzamido]-2-[(2R)-1-methylpyrrolidin-2-yl]pyrrolo[3,2-c]pyridine-1-carboxylate **(Intermediate 325,** 260 mg, 0.543 mmol) in THF (5 mL) was added $H_2O$ (5 mL) and LiOH (104 mg, 4.34 mmol) and the reaction was stirred at 50°C for 2h. Water (50 mL) was added the mixture was adjusted to pH 4-6 by dropwise addition of concentrated HCl. The resulting solution was extracted with EtOAc (3 × 100 mL) and the combined organic phases were dried over anhydrous sodium sulfate and concentrated under vacuum to afford 4-({2-[(2R)-1-methylpyrrolidin-2-yl]-1H-pyrrolo[3,2-c]pyridin-6-yl}carbamoyl)benzoic acid **(Intermediate 326,** 190 mg, 86%) as a yellow solid, which was used in the next step without further purification. Mass spec: m/z: 365.3 $[M+H]^+$.

**Step 5**

**Example 69: (2S,4R)-1-[(2S)-3,3-dimethyl-2-(11-{[4-({2-[(2R)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide**

To a solution of 4-({2-[(2R)-1-methylpyrrolidin-2-yl]-1H-pyrrolo[3,2-c]pyridin-6-yl}carbamoyl)benzoic acid **(Intermediate 326,** 50.0 mg, 0.134 mmol) in DMF (1.5 mL) was added (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide hydrochloride **(Intermediate 324,** 84.2 mg, 0.137 mmol), EDCI (31.5 mg, 0.164 mmol) and HOBT (24.1 mg, 0.178 mmol) and the resulting mixture was stirred at 60°C overnight. The crude product was purified by preparative HPLC (Column: XBridge Prep Shield RP C18 Column, 19 * 250 mm, 5μm; Mobile Phase A: Water (+0.1% formic acid), Mobile Phase B: MeOH; Flow rate: 25 mL/min; Gradient: 38% B to 68% B in 10 min) to afford (2S,4R)-1-[(2S)-3,3-dimethyl-2-(11-{[4-({2-[(2R)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide **(Example 69,** 14.2 mg, 10.7%) as a white solid. $^1H$ NMR (400 MHz, MeOH-d4) δ ppm 8.91 (s, 1H), 8.55 (s, 1H), 8.21 (s, 1H), 8.08 (d, *J* = 8.4 Hz, 2H), 7.97 (d, *J* = 8.8 Hz, 2H), 7.40 - 7.48 (m, 4H), 6.56 (s, 1H), 4.65 (s, 1H), 4.59 - 4.64 (m, 2H), 4.50 - 4.56 (m, 1H), 4.34 - 4.38 (m, 1H), 3.88 - 3.90 (m, 1H), 3.82 - 3.83 (m, 1H), 3.41 - 3.49 (m, 1H), 3.33 - 3.39 (m, 2H), 2.48 - 2.49 (m, 4H), 2.34 - 2.36 (m, 3H), 2.26 - 2.29 (m, 4H), 2.06 - 2.10 (m, 3H), 1.90 - 1.93 (m, 1H), 1.61 - 1.67 (m, 4H), 1.34 - 1.39 (m, 13H), 1.04 (s, 9H). Mass spec: m/z: 962.65.

**Example 70 was synthesised following Scheme 68**

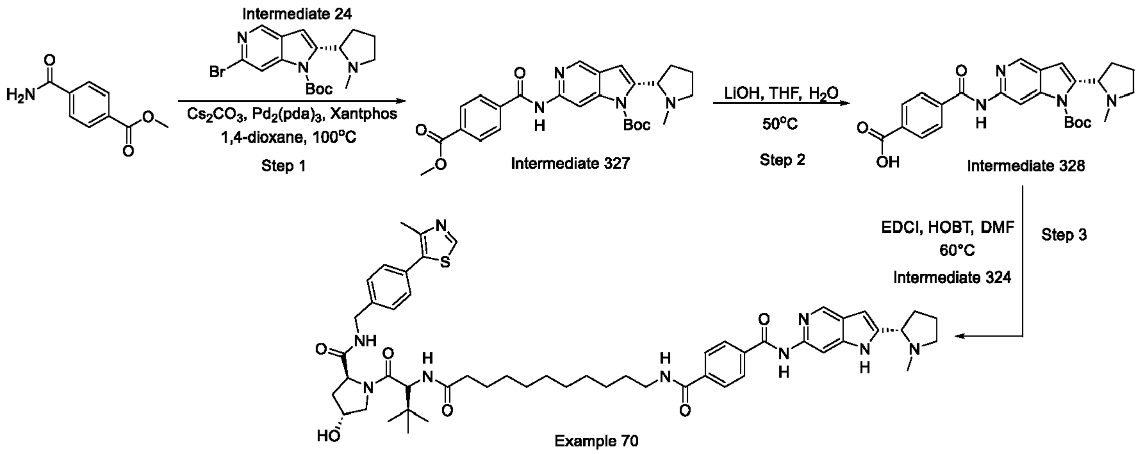


## Scheme 68

**Step** 1

**Intermediate 327: tert-butyl 6-[4-(methoxycarbonyl)benzamido]-2-[(2S)-1-methylpyrrolidin-2-yl]pyrrolo[3,2-c] pyridine-1-carboxylate**

**Intermediate 327** was synthesised following an analogous procedure used for **Intermediate 325** shown in **Step 3** of **Scheme 67,** using methyl 4-carbamoylbenzoate (CAS No: 6757-31-9) as the amide and tert-butyl 6-bromo-2-[(2S)-1-methylpyrrolidin-2-yl]pyrrolo[3,2-c]pyridine-1-carboxylate **(Intermediate 24)** to afford tert-butyl 6-[4-(methoxycarbonyl) benzamido]-2-[(2S)-1-methylpyrrolidin-2-yl]pyrrolo[3,2-c]pyridine-1-carboxylate **(Intermediate 327).** Mass spec: m/z: 479.3 $[M+H]^+$.

**Step 2**

**Intermediate 328: 4-({2-[(2S)-1-methylpyrrolidin-2-yl]-1H-pyrrolo[3,2-c]pyridin-6-yl}carbamoyl)benzoic acid**

**Intermediate 328** was synthesised following an analogous procedure used for **Intermediate 325** shown in **Step 4** of **Scheme 67,** using tert-butyl 6-[4-(methoxycarbonyl)benzamido]-2-[(2S)-1-methylpyrrolidin-2-yl]pyrrolo[3,2-c]pyridine-1-carboxylate **(Intermediate 327)** as the ester to afford 4-({2-[(2S)-1-methylpyrrolidin-2-yl]-1H-pyrrolo[3,2-c]pyridin-6-yl}carbamoyl)benzoic acid **(Intermediate 328).** Mass spec: 365.3 $[M+H]^+$.

**Step 3**

**Example 70: (2S,4R)-1-[(2S)-3,3-dimethyl-2-(11-{[4-({2-[(2S)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl] methyl}pyrrolidine-2-carboxamide**

**Example 70** was synthesised following an analogous procedure used for **Example 69,** shown in **Step 5** of **Scheme 67,** using 4-({2-[(2S)-1-methylpyrrolidin-2-yl]-1H-pyrrolo[3,2-c]pyridin-6-yl}carbamoyl)benzoic acid **(Intermediate 328)** as the acid and (2S,4R)-1-[(2S)-2-(11-aminoundecanamido)-3,3-dimethylbutanoyl]-4-hydroxy-N-{[4-(4-methyl-1,3-thiazol-5-yl)phenyl]methyl}pyrrolidine-2-carboxamide hydrochloride **(Intermediate 324)** as the amine, to afford (2S,4R)-1-[(2S)-3,3-dimethyl-2-(11-{[4-({2-[(2S)-1-methylpyrrolidin-2-yl]-1H-pyrrolo[3,2-c]pyridin-6-yl}carbamoyl)phenyl]formamido}undecanamido)butanoyl]-4-hydroxy-N-{ [4-(4-methyl-1,3-thiazol-5-yl)phenyl]methyl}pyrrolidine-2-carboxamide **(Example 70).** $^1H$ NMR (400 MHz, DMSO-d6) δ ppm 11.41 (s, 1H), 10.63 (s, 1H), 8.98 (s, 1H), 8.51-8.59 (m, 3H), 8.21 (s, 1H), 8.11 (d, *J* = 8.4 Hz, 2H), 7.94 (d, *J* = 8.4 Hz, 2H), 7.83 (d, *J* = 9.2 Hz, 1H), 7.37 - 7.43 (m, 4H), 6.40 (s, 1H), 4.54 (d, *J* = 9.6 Hz, 1H), 4.43 - 4.50 (m, 2H), 4.35 - 4.37 (m, 1H), 4.23 - 4.25 (m, 1H), 3.67 - 3.70 (m, 1H), 3.31 - 3.35 (m, 1H), 3.24 - 3.28 (m, 2H), 3.15 - 3.22 (m, 1H), 2.44 (s, 3H), 2.24 - 2.28 (m, 2H), 2.17 (s, 3H), 2.11 - 2.14 (m, 3H), 1.88 - 1.92 (m, 4H), 1.49 - 1.53 (m, 4H), 1.25 - 1.29 (m, 14H), 0.93 (s, 9H). Mass spec: m/z: 960.75 $[M+H]^+$.

**Example 71 was synthesized following Scheme 69**

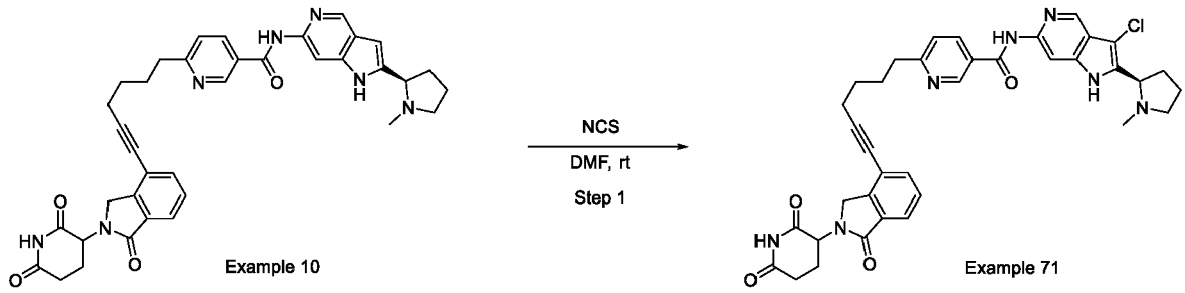


## Scheme 69

**Step 1**

**Example 71: *N*-(3-chloro-2-((*R*)-1-methylpyrrolidin-2-yl)-1*H*-pyrrolo[3, 2-c]pyridin-6-yl)-6-(6-(2-(2, 6-dioxopiperidin-3-yl)-1-oxoisoindolin-4-yl) hex-5-yn-1-yl) nicotinamide**

To a solution of 6-(6-(2-(2,6-dioxopiperidin-3-yl)-1-oxoisoindolin-4-yl)hex-5-yn-1-yl)-*N*-(2-((*R*)-1-methylpyrrolidin-2-yl)-1*H*-pyrrolo[3,2-c]pyridin-6-yl)nicotinamide **(Example 10,** 0.050 g, 0.077 mmol) in dimethylformamide (1 mL) was added *N*-chlorosuccinimide (0.016 g, 0.11 mmol) and the reaction mixture was stirred at room temperature for 30h. The reaction mixture was quenched with water (20 mL) and extracted with ethyl acetate (2 $\times$ 20 mL). The combined organic layers were washed with saturated brine solution (20 mL), dried over anhydrous $Na_2SO_4$, and concentrated *in vacuo.* The crude material was purified by preparative HPLC (Method A) to afford *N*-(3-chloro-2-((*R*)-1-methylpyrrolidin-2-yl)-1*H*-pyrrolo[3,2-c]pyridin-6-yl)-6-(6-(2-(2,6-dioxopiperidin-3-yl)-1-oxoisoindolin-4-yl)hex-5-yn-1-yl) nicotinamide **(Example 71,** 0.006 g, 11% yield) as a white solid. $^1$H NMR (400 MHz, DMSO-$d_6$) $\delta$ 1.59-1.68 (m, 2H), 1.81-1.92 (m, 4H), 1.94-2.06 (m, 2H), 2.16 (s, 3H), 2.18-2.21 (m, 1H), 2.31-2.34 (m, 1H), 2.40-2.45 (m, 1H), 2.56-2.62 (m, 3H), 2.84-2.92 (m, 3H), 3.12-3.21 (m, 1H), 3.50-3.54 (m, 1H), 4.29-4.35 (m, 1H), 4.42-4.49 (m, 1H), 5.11-5.16 (m, 1H), 7.41 (d, *J*=8.0 Hz, 1H), 7.50 (t, *J*=7.60 Hz, 1H), 7.64 (d, *J*=7.60 Hz, 1H), 7.70 (d, *J*=7.60 Hz, 1H), 8.26 (s, 1H), 8.29 (m, 1H), 8.51 (s, 1H), 9.08 (s, 1H), 10.83 (s, 1H), 10.99 (s, 1H), 11.71 (s, 1H). Mass spec m/z 678.0 $[M+H]^+$.

**Example 72 was synthesized following Scheme70**

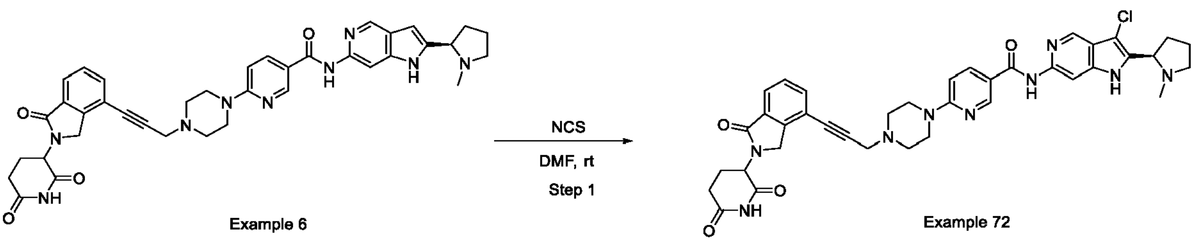


## Scheme 70

**Step 1**

**Example 72: *N*-(3-chloro-2-((*R*)-1-methylpyrrolidin-2-yl)-1*H*-pyrrolo[3,2-c]pyridin-6-yl)-6-(4-(3-(2-(2,6-dioxopiperidin-3-yl)-1-oxoisoindolin-4-yl)prop-2-yn-1-yl)piperazin-1-yl)nicotinamide**

To a solution of 6-(4-(3-(2-(2,6-dioxopiperidin-3-yl)-1-oxoisoindolin-4-yl)prop-2-yn-1-yl)piperazin-1-yl)-*N*-(2-((*R*)-1-methylpyrrolidin-2-yl)-1*H*-pyrrolo[3,2-c]pyridin-6-yl)nicotinamide **(Example 6,** 0.09 g, 0.13 mmol) in dimethylformamide (0.5 mL) was added *N*-chlorosuccinimide (0.027 g, 0.19 mmol) and the reaction mixture was stirred at room temperature for 30h. The reaction mixture was quenched with water (20 mL), the resultant precipitate was collected by filtration and dried *in vacuo.* The crude material was purified by preparative HPLC (Method A) to afford *N*-(3-chloro-2-((*R*)-1-methylpyrrolidin-2-yl)-1*H*-pyrrolo[3,2-c]pyridin-6-yl)-6-(4-(3-(2-(2,6-dioxopiperidin-3-yl)-1-oxoisoindolin-4-yl) prop-2-yn-1-yl)piperazin-1-yl)nicotinamide **(Example 72,** 0.009 g, 9% yield) as an off-white solid. $^1$H NMR (400 MHz, DMSO-$d_6$) δ 1.80-1.90 (m, 2H), 1.94-2.05 (m, 2H), 2.16 (s, 3H), 2.18-2.21 (m, 1H), 2.29-2.35 (m, 1H), 2.40-2.46 (m, 1H), 2.55-2.61 (m, 1H), 2.62-2.69 (m, 4H), 2.85-2.95 (m, 1H), 3.15-3.20 (m, 1H), 3.48-3.54 (m, 1H), 3.66-3.73 (m, 6H), 4.29-4.37 (m, 1H), 4.43-4.50 (m, 1H), 5.10-5.14 (m, 1H), 6.89 (d, *J*=9.20 Hz, 1H), 7.54 (t, *J*=7.60 Hz, 1H), 7.68-7.76 (m, 2H), 8.16 (dd, *J*=2.80, 9.20 Hz, 1H), 8.23 (s, 1H), 8.48 (s, 1H), 8.80 (s, 1H), 10.43 (s, 1H), 10.98 (s, 1H), 11.65 (br s, 1H). Mass spec m/z 719.80 [M+H]$^+$.

**Example 73 was synthesized following Scheme 71**

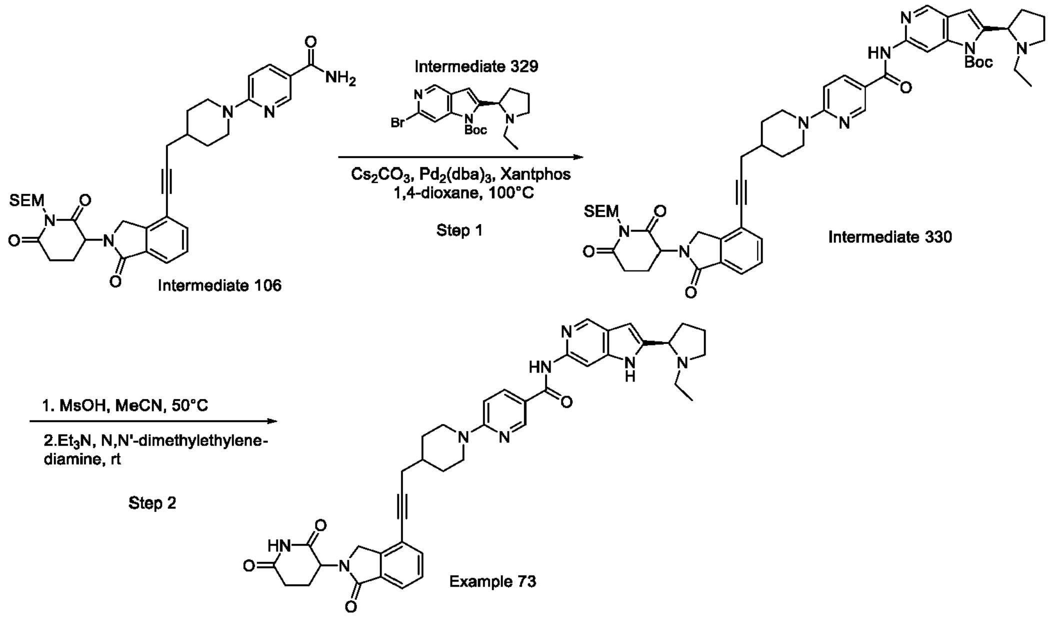


**Scheme 71**

**Step** 1

**Intermediate 330: tert-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperidin-1-yl)nicotinamido)-2-((*R*)-1-ethylpyrrolidin-2-yl)-1*H*-pyrrolo[3,2-*c*]pyridine-1-carboxylate**

To a solution of 6-(4-(3-(2-(2,6-dioxo-1-((2-(trimethylsilyl)ethoxy)methyl)piperidin-3-yl)-1-oxoisoindolin-4-yl) prop-2-yn-1-yl)piperidin-1-yl)nicotinamide **(Intermediate 106, 0.37** g, 0.61 mmol) in 1,4-dioxane (10 mL) was added *tert*-butyl (*R*)-6-bromo-2-(1-ethylpyrrolidin-2-yl)-1*H*-pyrrolo[3,2-*c*]pyridine-1-carboxylate **(Intermediate 329,** 0.30 g, 0.76 mmol) followed by cesium carbonate (0.74 g, 2.28 mmol) at room temperature. The reaction mixture was purged with argon for 15 min then $Pd_2(dba)_3$ (0.11 g, 0.11 mmol) and Xantphos (0.13 g, 0.23 mmol) were added. The reaction mixture was further purged with argon for another 10 min and then stirred at 100°C for 2h. The reaction mixture was concentrated *in vacuo* to obtain crude compound. The crude material was purified by combi-flash chromatography by eluting with 90% ethyl acetate in heptane to afford *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperidin-1-yl)nicotinamido)-2-((*R*)-1-ethylpyrrolidin-2-yl)-1*H*-pyrrolo[3,2-c]pyridine-1-carboxylate **(Intermediate 330,** 0.32 g, 45%) as a brown solid. $^1$H NMR (400 MHz, DMSO-$d_6$) δ -0.04 (s, 9H), 0.77-0.87 (m, 2H), 1.24 (t, J=6.80 Hz, 3H), 1.73 (s, 9H), 1.84-1.97 (m, 3H), 2.01-2.22 (m, 5H), 2.38-2.43 (m, 1H), 2.58-2.65 (m, 1H), 2.77-2.82 (m, 1H), 2.93-3.01 (m, 2H), 3.28-3.36 (m, 2H), 3.45-3.59 (m, 8H), 4.25-4.30 (m, 1H), 4.42-4.55 (m, 3H), 5.01-5.08 (m, 2H), 5.21-5.31 (m, 2H), 6.92-6.94 (m,1H), 7.50-7.57 (m, 2H), 7.65 (d, *J*=7.60 Hz, 1H), 7.72 (d, *J*=7.60 Hz, 1H), 8.13-8.20 (m, 1H), 8.74 (s, 1H), 8.77-8.81 (m, 1H), 8.94 (s, 1H), 10.72 (br s, 1H). Mass spec: m/z: 929.7 [M+H]$^+$.

**Step 2**

**Example 73: 6-(4-(3-(2-(2,6-dioxopiperidin-3-yl)-1-oxoisoindolin-4-yl)prop-2-yn-1-yl)piperidin-1-yl)-*N*-(2-((*R*)-1-ethylpyrrolidin-2-yl)-1*H*-pyrrolo[3,2-*c*]pyridin-6-yl)nicotinamide**

To a solution of *tert*-butyl 6-(6-(4-(3-(2-(2,6-dioxo-1-((2-(trimethylsilyl)ethoxy)methyl)piperidin-3-yl)-1-oxoisoindolin-4-yl)prop-2-yn-1-yl)piperidin-1-yl)nicotinamido)-2-((*R*)-1-ethylpyrrolidin-2-yl)-1*H*-pyrrolo[3,2-*c*]pyridine-1-carboxylate **(Intermediate 330,** 0.28 g, 0.30 mmol) in acetonitrile (10 mL) was added methanesulfonic acid (0.29 mL, 4.52 mmol) at room temperature and the reaction mixture was stirred at 50°C for 2h. *N*, *N'*-dimethylethylenediamine (0.21 mL, 1.81 mmol) followed by triethylamine (0.61 g, 6.03 mmol) and the reaction stirred at room temperature for 3h. The reaction mixture was diluted with water (100 mL), resulting in a precipitate which was filtered and dried to obtain crude compound. The crude material was purified by preparative HPLC (Method A) to afford 6-(4-(3-(2-(2,6-dioxopiperidin-3-yl)-1-oxoisoindolin-4-yl)prop-2-yn-1-yl)piperidin-1-yl)-*N*-(2-((*R*)-1-ethylpyrrolidin-2-yl)-1*H*-pyrrolo[3,2-*c*]pyridin-6-yl)nicotinamide **(Example 73,** 0.14 g, 66%) as an off white solid. $^1$H NMR (400 MHz, DMSO-$d_6$) δ 0.98 (t, *J*=7.19 Hz, 3H), 1.24-1.36 (m, 2H), 1.78-1.94 (m, 6H), 1.97-2.05 (m, 1H), 2.10-2.24 (m, 3H), 2.39-2.45 (m, 1H), 2.54-2.64 (m, 4H), 2.89-2.98 (m, 3H), 3.20-3.26 (m, 1H), 3.42-3.50 (m, 1H), 4.26-4.37 (m, 1H), 4.40-4.55 (m, 3H), 5.10-5.16 (m, 1H), 6.36 (s, 1H), 6.86-6.89 (m, 1H), 7.49-7.53 (m, 1H), 7.64 (d, *J*=7.20 Hz, 1H), 7.71 (d, *J*=7.20 Hz, 1H), 8.12-8.17 (m, 2H), 8.47 (s, 1H), 8.78-8.79 (m, 1H), 10.22 (s, 1H), 11.00 (br s, 1H), 11.28 (br s, 1H). Mass spec: m/z: 699.0 [M+H]$^+$.

**Intermediate 329 was synthesized following Scheme 72**

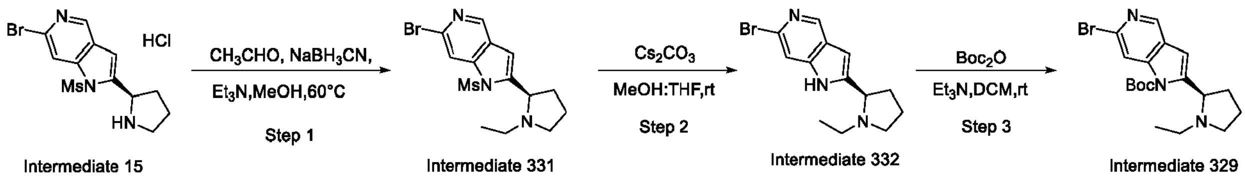


Intermediate 15 Intermediate 331 Intermediate 332 Intermediate 329

Scheme 72

**Step 1**

**Intermediate 331: (R)-6-bromo-2-(1-ethylpyrrolidin-2-yl)-1-(methylsulfonyl)-1*H*-pyrrolo [3, 2-c] pyridine**

To a cooled (0°C) solution of (R)-6-bromo-1-(methylsulfonyl)-2-(pyrrolidin-2-yl)-1*H*-pyrrolo[3,2-c]pyridine hydrochloride **(Intermediate 15,** 10.0 g, 26.27 mmol) in methanol (50 mL) was added triethylamine (29.4 mL, 210.1 mmol) and acetaldehyde (4.21 mL, 78.80 mmol) followed by sodium cyanoborohydride (3.88 g, 52.53 mmol). The reaction mixture was then heated at 60°C for 16h. The reaction mixture was diluted with water (200 mL) and extracted with ethyl acetate (3 × 200 mL). The combined organic layers were dried over anhydrous $Na_2SO_4$, filtered, and concentrated *in vacuo.* The crude material was purified by combi-flash chromatography, by eluting with 60% ethyl acetate in heptane, to afford (R)-6-bromo-2-(1-ethylpyrrolidin-2-yl)-1-(methylsulfonyl)-1*H*-pyrrolo[3,2-c]pyridine **(Intermediate 331,** 5.6 g, 57%) as a yellow solid. $^1$H NMR (400 MHz, DMSO-$d_6$) δ ppm 0.97-1.17 (m, 3H), 1.64-1.82 (m, 3H), 2.20-2.38 (m, 3H), 2.72-2.84 (m, 1H), 3.22-3.31 (m, 1H), 3.55 (s, 3H), 3.95-4.04 (m, 1H), 6.92 (s, 1H), 7.96 (s, 1H), 8.67 (s, 1H). Mass spec m/z 373.1 [M+2H]$^+$.

**Step 2**

**Intermediate 332: (*R*)-6-bromo-2-(1-ethylpyrrolidin-2-yl)-1*H*-pyrrolo [3, 2-c] pyridine**

To a cooled (0°C) solution of (R)-6-bromo-2-(1-ethylpyrrolidin-2-yl)-1-(methylsulfonyl)-1*H*-pyrrolo [3, 2-c] pyridine **(Intermediate 331,** 5.60 g, 15.04 mmol) in tetrahydrofuran (25 mL) and methanol (25 mL) was added cesium carbonate (9.81 g, 30.08 mmol). The reaction mixture was stirred at room temperature for 4h. The reaction mixture was diluted with water (200 mL) and extracted with ethyl acetate (3 × 200 mL). The combined organic layers were dried over anhydrous $Na_2SO_4$, filtered, and concentrated *in vacuo* to afford crude (R)-6-bromo-2-(1-ethylpyrrolidin-2-yl)-1*H*-pyrrolo [3,2-c]pyridine **(Intermediate 332,** 4.1 g, 93%) as an off-white solid which was used for the next step without further purification. $^1$H NMR (400 MHz, DMSO-$d_6$) δ ppm 0.91-0.97 (m, 3H), 1.69-1.88 (m, 3H), 2.06-2.20 (m, 3H), 2.49-2.55 (m, 1H), 3.17-3.21 (m, 1H), 3.44-3.48 (m, 1H), 6.40 (s, 1H), 7.40 (s, 1H), 8.44 (s, 1H), 11.48 (br s, 1H). Mass spec m/z 295.9 [M+2H]$^+$.

**Step 3**

**Intermediate 329: *tert*-butyl-(*R*)-6-bromo-2-(1-ethylpyrrolidin-2-yl)-1*H*-pyrrolo [3, 2-c] pyridine-1-carboxylate**

To a solution of (*R*)-6-bromo-2-(1-ethylpyrrolidin-2-yl)-1*H*-pyrrolo [3, 2-c] pyridine **(Intermediate 332,** 4.0 g, 14 mmol) in dichloromethane (40 mL) was added triethylamine (5.7 mL, 41 mmol) followed by di-*tert*-butyl dicarbonate (6.0 g, 27 mmol) and 4-dimethylaminopyridine (0.34 g, 2.7 mmol) and the reaction mixture was stirred at room temperature for 3h. The reaction mixture was diluted with water (500 mL) and extracted with DCM (3 × 500 mL). The combined organic layers were dried over anhydrous $Na_2SO_4$, filtered, and concentrated *in vacuo.* The crude material was purified by combi-flash chromatography, by eluting with 45% ethyl acetate in heptane, to afford *tert*-butyl (R)-6-bromo-2-(1-ethylpyrrolidin-2-yl)-1*H*-pyrrolo[3, 2-c]pyridine-1-carboxylate which was further purified by SFC chiral purification (Column: Chiralpak IK (250 * 30 mm) 5 um. Isocratic elution with 25% Mobile Phase (A): $CO_2$ and mobile Phase (B): MeCN + isopropyl alcohol with 0.1% isopropylamine) to afford desired isomer *tert*-butyl-(*R*)-6-bromo-2-(1-ethylpyrrolidin-2-yl)-1*H*-pyrrolo[3,2-c] pyridine-1-carboxylate **(Intermediate 329,** 2.2 g, 41%) as an off white solid. $^1$H NMR (400 MHz, DMSO-$d_6$) δ ppm 1.06 (t, *J*=7.13 Hz, 3H), 1.65 (s, 9H), 1.70-1.80 (m, 2H), 2.24-2.38 (m, 4H), 2.72-2.82 (m, 1H), 3.22-3.25 (m, 1H), 4.06-4.10 (m, 1H), 6.83 (s, 1H), 8.05 (s, 1H), 8.61 (s, 1H). Mass spec m/z 395.8 $[M+H]^+$.

**Example 74 was synthesized following Scheme 73**

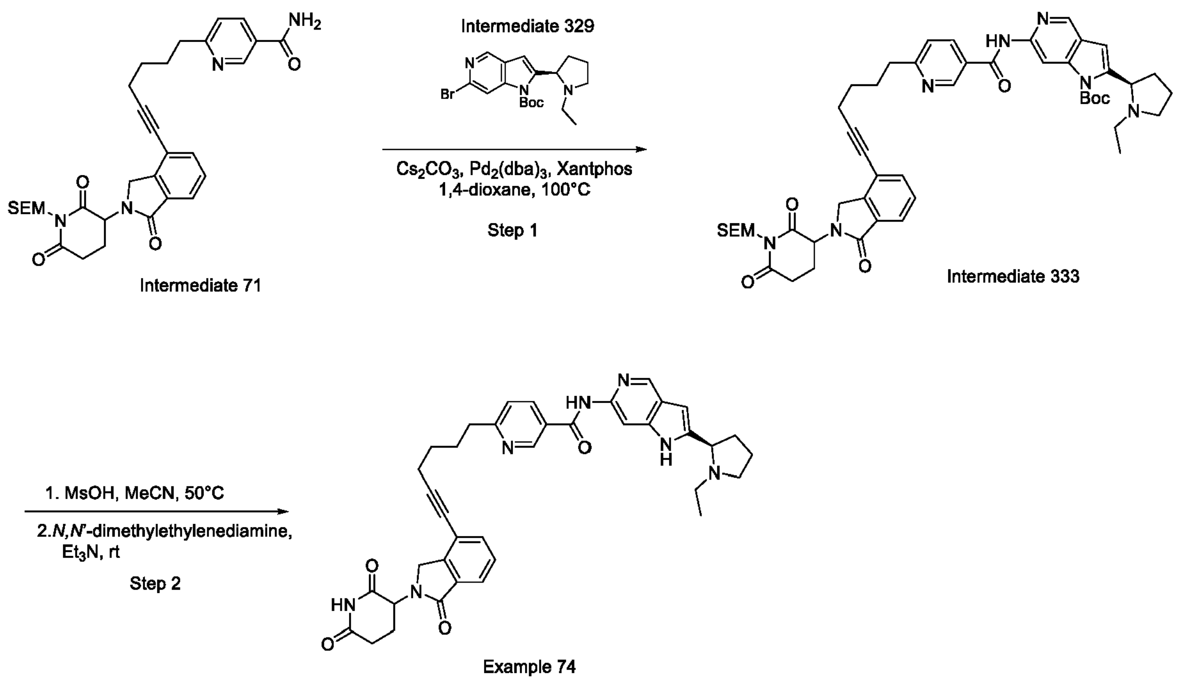


Intermediate 71

Intermediate 333

Example 74

Scheme 73

**Step 1**

**Intermediate 333: *tert*-butyl-6-(6-(6-(2-(2,6-dioxo-1-((2-(trimethylsilyl)ethoxy)methyl)piperidin-3-yl)-1-oxoisoindolin-4-yl)hex-5-yn-1-yl)nicotinamido)-2-((*R*)-1-ethylpyrrolidin-2-yl)-1*H*-pyrrolo[3,2-*c*]pyridine-1-carboxylate**

To a solution of 6-(6-(2-(2,6-dioxo-1-((2-(trimethylsilyl)ethoxy)methyl)piperidin-3-yl)-1-oxoisoindolin-4-yl)hex-5-yn-1-yl)nicotinamide **(Intermediate 71**, 0.35 g, 0.61 mmol) in 1,4-dioxane (10 mL) was added *tert*-butyl-(*R*)-6-bromo-2-(1-ethylpyrrolidin-2-yl)-1*H*-pyrrolo[3,2-*c*]pyridine-1-carboxylate **(Intermediate 329, 0.29** g, 0.73 mmol) followed by cesium carbonate (0.59 g, 1.83 mmol). The reaction mixture was purged with argon for 15 min, then $Pd_2(dba)_3$ (0.086 g, 0.091 mmol) and Xantphos (0.11 g, 0.18 mmol) were added. The reaction mixture was purged with argon for another 10 min and then stirred at 100°C for 2h. The reaction mixture was filtered through celite bed, the filter pad was washed with ethyl

acetate (100 mL) and the filtrate was concentrated *in vacuo.* The crude material was purified by combi-flash chromatography, by eluting with 4% MeOH in DCM, to afford *tert*-butyl-6-(6-(6-(2-(2,6-dioxo-1-((2-(trimethylsilyl)ethoxy)methyl)piperidin-3-yl)-1-oxoisoindolin-4-yl)hex-5-yn-1-yl)nicotinamido)-2-((*R*)-1-ethylpyrrolidin-2-yl)-1*H*-pyrrolo[3,2-*c*]pyridine-1-carboxylate **(Intermediate 333,** 0.33 g, 61% yield) as a pale yellow solid. $^1$H NMR (400 MHz, DMSO-$d_6$) δ -0.05 (s, 9H), 0.75-0.85 (m, 2H), 1.07 (t, *J*=6.84 Hz, 3H), 1.50-1.64 (m, 4H), 1.69 (s, 9H), 1.74-1.80 (m, 2H), 1.82-1.95 (m, 2H), 1.99-2.11 (m, 1H), 2.21-2.43 (m, 4H), 2.53-2.58 (m, 1H), 2.72-2.92 (m, 4H), 2.99-3.12 (m, 1H), 3.21-3.26 (m, 1H), 3.44-3.56 (m, 2H), 4.08-4.15 (m, 1H), 4.25-4.30 (m, 1H), 4.45-4.50 (m 1H), 4.96-5.09 (m, 2H), 5.24-5.30 (m, 1H), 6.79 (s, 1H), 7.41 (d, *J*=7.60 Hz, 1H), 7.51-7.56 (m, 1H), 7.65 (d, *J*=7.40 Hz, 1H), 7.72 (d, *J*=7.40 Hz, 1H), 8.27 (d, *J*=7.60 Hz, 1H), 8.60 (s, 1H), 8.92 (s, 1H), 9.06 (br s, 1H), 10.90 (br s, 1H). Mass spec m/z 888.6 [M+H]$^+$.

**Step 2**

**Example 74: 6-(6-(2-(2, 6-dioxopiperidin-3-yl)-1-oxoisoindolin-4-yl) hex-5-yn-1-yl)-*N*-(2-((*R*)-1-ethylpyrrolidin-2-yl)-1*H*-pyrrolo [3, 2-c] pyridin-6-yl) nicotinamide**

To a solution of *tert*-butyl-6-(6-(6-(2-(2,6-dioxo-1-((2-(trimethylsilyl)ethoxy)methyl)piperidin-3-yl)-1-oxoisoindolin-4-yl)hex-5-yn-1-yl)nicotinamido)-2-((*R*)-1-ethylpyrrolidin-2-yl)-1*H*-pyrrolo[3,2-*c*]pyridine-1-carboxylate **(Intermediate 333,** 0.30 g, 0.34 mmol) in acetonitrile (10 mL) was added methanesulfonic acid (0.22 mL, 3.38 mmol) and the reaction mixture was stirred at 50°C for 2h. After cooling to room temperature, *N,N'*-dimethylethylenediamine (0.40 mL, 3.38 mmol) followed by triethylamine (1.66 mL, 11.82 mmol) were added the reaction mixture was stirred at room temperature for 2h. The reaction mixture was concentrated *in vacuo* and the residue was diluted with ice cold water (80 mL). The resultant precipitate was collected by filtration, washed with pentane (2 × 40 mL) and dried *in vacuo.* The crude material was purified by preparative HPLC (Method A) to afford 6-(6-(2-(2,6-dioxopiperidin-3-yl)-1-oxoisoindolin-4-yl)hex-5-yn-1-yl)-*N*-(2-((*R*)-1-ethylpyrrolidin-2-yl)-1*H*-pyrrolo[3,2-*c*]pyridin-6-yl)nicotinamide **(Example 74,** 0.12 g, 53% yield) as an off white solid. $^1$H NMR (400 MHz, DMSO-$d_6$) δ 0.98 (t, *J*=7.20 Hz, 3H), 1.58-1.68 (m, 2H), 1.78-1.95 (m, 5H), 1.97-2.06 (m, 1H), 2.10-2.24 (m, 3H), 2.37-2.43 (m, 2H), 2.56-2.62 (m, 3H), 2.85-2.90 (m, 3H), 3.21-3.27 (m, 1H), 3.44-3.50 (m, 1H), 4.28-4.36 (m, 1H), 4.42-4.50 (m, 1H), 5.11-5.16 (m, 1H), 6.38 (s, 1H), 7.40 (d, *J*=8.00 Hz, 1H), 7.49-7.55 (m, 1H), 7.64 (d, *J*=7.40 Hz, 1H), 7.71 (d, *J*=7.40 Hz, 1H), 8.19 (s, 1H), 8.29 (dd, *J*=8.00, 2.00 Hz, 1H), 8.50 (s, 1H), 9.08 (d, *J*=2.00 Hz, 1H), 10.66 (s, 1H), 10.99 (br s, 1H), 11.34 (br s, 1H). Mass spec m/z 658.1 [M+H]$^+$.

**Example 75 was synthesized following Scheme 74**

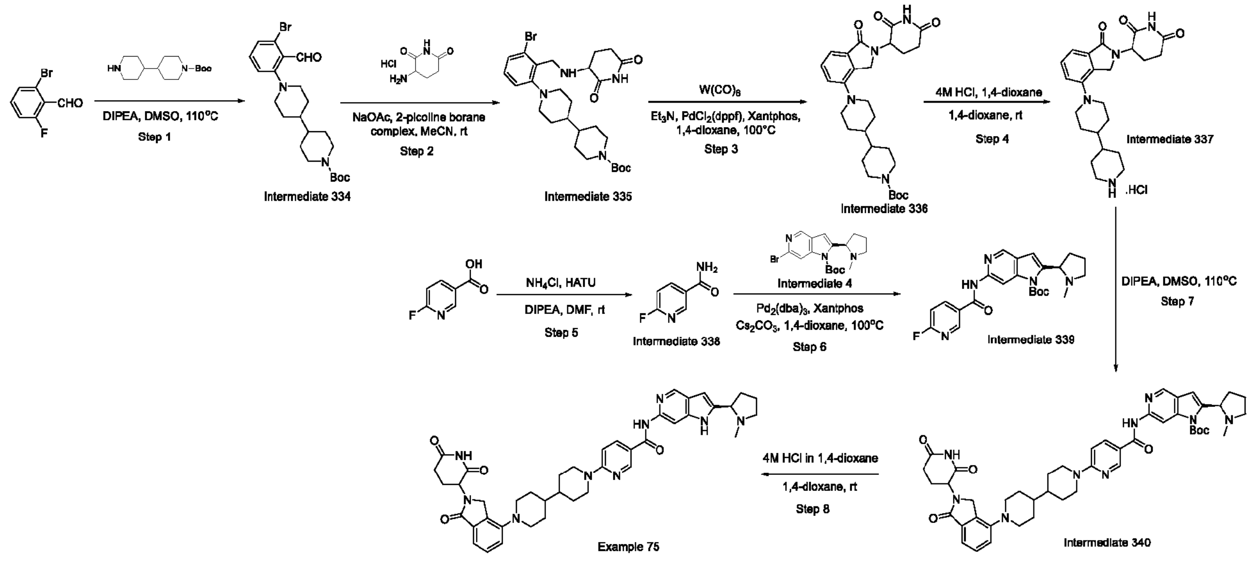


**Scheme 74**

**Step 1**

**Intermediate 334: *tert*-butyl 1'-(3-bromo-2-formylphenyl)-[4,4'-bipiperidine]-1-carboxylate**

To a solution of 2-bromo-6-fluorobenzaldehyde (CAS No: 360575-28-6, 4.0 g, 20 mmol) in DMSO (10 mL) was added *N*,*N*-diisopropylethylamine (13.0 g, 99 mmol) followed by *tert*-butyl [4,4'-bipiperidine]-1-carboxylate (CAS No: 171049-35-7, 6.30 g, 24.0 mmol) at room temperature and the reaction mixture was heated to 110°C for 16h. The reaction mixture was quenched with water (50 mL), resulting in a precipitate, which was filtered and dried *in vacuo* to afford *tert*-butyl 1'-(3-bromo-2-formylphenyl)-[4,4'-bipiperidine]-1-carboxylate **(Intermediate 334, 4.0** g, 45%) as a yellow solid which was used for the next step without further purification. $^1$H NMR (400 MHz, DMSO-$d_6$) δ 0.94-1.09 (m, 2H), 1.15-1.24 (m, 2H), 1.38 (s, 9H), 1.60-1.76 (m, 4H), 2.60-2.68 (m, 2H), 2.76-2.84 (m, 2H), 3.12-3.20 (m, 2H), 3.86-4.04 (m, 4H), 7.21 (d, *J*=8.40 Hz, 1H), 7.31 (d, *J*=8.40 Hz, 1H), 7.38 (t, *J*=8.40 Hz, 1H), 10.02 (s, 1H). Mass spec: m/z: 451.3 [M+H]$^+$.

**Step 2**

**Intermediate 335: *tert*-butyl 1'-(3-bromo-2-(((2,6-dioxopiperidin-3-yl)amino)methyl)phenyl)-[4,4'-bipiperidine]-1-carboxylate**

To a solution of *tert*-butyl 1'-(3-bromo-2-formylphenyl)-[4,4'-bipiperidine]-1-carboxylate **(Intermediate 334,** 4.0 g, 8.86 mmol) and 3-aminopiperidine-2,6-dione hydrochloride (CAS No: 24666-56-6, 2.20 g, 13.30 mmol) in acetonitrile (60 mL) was added sodium acetate (1.09 g, 13.30 mmol) followed by 2-picoline borane complex (CAS No: 3999-38-0, 1.90 g, 17.72 mmol) and the reaction mixture was stirred at room temperature for 12h. The reaction mixture was quenched with water (100 mL) and extracted with ethyl acetate (3 × 100 mL). The combined organic phases were dried over $Na_2SO_4$, filtered, and concentrated *in vacuo* to obtain the crude compound. The crude material was purified by combi-flash chromatography by eluting with EtOAc to afford *tert*-butyl 1'-(3-bromo-2-(((2,6-dioxopiperidin-3-yl)amino)methyl)phenyl)-[4,4'-bipiperidine]-1-carboxylate **(Intermediate 335,** 2.0 g, 40%) as a solid. $^1$H NMR (400 MHz, DMSO-$d_6$) δ 0.84-0.88 (m, 1H), 0.97-1.11 (m, 2H), 1.16-1.31 (m, 4H), 1.39 (s, 9H), 1.65-1.81 (m, 4H), 2.24-2.34 (m, 2H), 2.42-2.47 (m, 2H), 2.53-2.58 (m, 1H), 2.64-2.76 (m, 2H), 2.92-2.98 (m, 1H), 3.08-3.16 (m, 1H), 3.20-3.26 (m, 1H), 3.85-4.04 (m, 4H), 7.09-7.20 (m, 2H), 7.32 (d, *J*=7.60 Hz, 1H), 10.78 (s, 1H). Mass spec: m/z: 563.3 [M+H]$^+$.

**Step 3**

**Intermediate 336: *tert*-butyl 1'-(2-(2,6-dioxopiperidin-3-yl)-1-oxoisoindolin-4-yl)-[4,4'-bipiperidine]-1-carboxylate**

To a solution of *tert*-butyl 1'-(3-bromo-2-(((2,6-dioxopiperidin-3-yl)amino)methyl)phenyl)-[4,4'-bipiperidine]-1-carboxylate **(Intermediate 335,** 2.0 g, 3.60 mmol) in 1,4-dioxane (20 mL) was added triethylamine (1.10 g, 11.0 mmol) and tungsten hexacarbonyl (0.64 g, 1.80 mmol). The reaction mixture was purged with argon gas for 15 min then Xantphos (0.42 g, 0.71 mmol) followed by $PdCl_2$(dppf) (0.55 g, 0.71 mmol) were added at room temperature. The reaction mixture was further purged with argon gas for 10 min and then heated at 100°C for 16h. The reaction mixture was quenched with water (30 mL) and extracted with ethyl acetate (3 × 100 mL). The combined organic phases were dried over $Na_2SO_4$, filtered, and concentrated *in vacuo* to obtain the crude compound. The crude material was purified by combi-flash chromatography by eluting with 70% EtOAc in heptane to afford *tert*-butyl 1'-(2-(2,6-dioxopiperidin-3-yl)-1-oxoisoindolin-4-yl)-[4,4'-bipiperidine]-1-carboxylate **(Intermediate 336,** 1.10 g, 61%) as a brown solid. $^1$H NMR (400 MHz, DMSO-$d_6$) δ 1.00-1.10 (m, 3H), 1.27-1.32 (m, 1H), 1.39 (s, 9H), 1.64-1.79 (m, 4H), 1.95-2.04 (m, 2H), 2.60-2.73 (m, 4H), 2.87-2.98 (m, 2H), 3.11-3.18 (m, 1H), 3.36-3.45 (m, 3H), 3.92-4.04 (m, 2H), 4.24-4.33 (m, 1H), 4.41-4.46 (m, 1H), 5.09-5.14 (m, 1H), 7.15 (d, *J*=8.0 Hz, 1H), 7.29 (d, *J*=8.0Hz, 1H), 7.40 (t, *J*=8.0 Hz, 1H), 10.98 (s, 1H). Mass spec: m/z: 511.5 [M+H]$^+$.

**Step 4**

**Intermediate 337: 3-(4-([4,4'-bipiperidin]-1-yl)-1-oxoisoindolin-2-yl)piperidine-2,6-dione hydrochloride**

To a solution of *tert*-butyl 1'-(2-(2,6-dioxopiperidin-3-yl)-1-oxoisoindolin-4-yl)-[4,4'-bipiperidine]-1-carboxylate **(Intermediate 336,** 1.0 g, 2.0 mmol) in 1,4-dioxane (10 mL) was added 4M HCl in dioxane (20 mL) at 0°C. The reaction mixture was then stirred at room temperature for 16h under a nitrogen atmosphere. The reaction mixture was concentrated under reduced pressure to afford 3-(4-([4,4'-bipiperidin]-1-yl)-1-oxoisoindolin-2-yl)piperidine-2,6-dione hydrochloride **(Intermediate 337,** 0.7 g, 80%) as a brown solid, which was used for the next step without further purification. $^1$H NMR (400 MHz, DMSO-$d_6$) δ 1.00-1.10 (m, 3H), 1.27-1.32 (m, 1H), 1.64-1.79 (m, 4H), 1.95-2.04 (m, 2H), 2.60-2.73 (m, 4H), 2.87-2.98 (m, 2H), 3.11-3.18 (m, 1H), 3.36-3.45 (m, 3H), 3.92-4.04 (m, 2H), 4.24-4.33 (m, 1H), 4.41-4.46 (m, 1H), 5.09-5.14 (m, 1H), 7.30 (d, *J*=8.0 Hz, 1H), 8.71 (t, *J*=8.0 Hz, 1H), 8.91 (d, *J*=8.0 Hz, 1H), 10.09 (br s, 2H), 10.99 (s, 1H).

Mass spec: m/z: 411.2 [M+H]+.

**Step 5**

**Intermediate 338: 6-fluoronicotinamide**

To a solution of 6-fluoronicotinic acid (CAS No: 403-45-2, 2.0 g, 14.0 mmol) in dimethylformamide (10 mL) was added HATU (8.30 g, 21.0 mmol) and *N, N*-diisopropylethylamine (5.60 g, 42.0 mmol) at room temperature. After cooling to 0°C, ammonium chloride (2.24 g, 42.0 mmol) was added and the reaction stirred at room temperature for 16h. The reaction mixture was quenched with water (50 mL), resulting in a precipitated, which was filtered and dried *in vacuo* to afford 6-fluoronicotinamide **(Intermediate 338,** 0.85 g, 43%) as a white solid, which was used in the next step without further purification. $^1$H NMR (400 MHz, DMSO-$d_6$) δ 7.27-7.30 (m, 1H), 7.64 (br s, 1H), 8.16 (br s, 1H), 8.36-8.42 (m, 1H), 8.54 (d, *J*=8.00 Hz, 1H). Mass spec: m/z: 141 [M+H]+.

**Step 6**

**Intermediate 339: *tert*-butyl (*R*)-6-(6-fluoronicotinamido)-2-(1-methylpyrrolidin-2-yl)-1*H*-pyrrolo[3,2-c]pyridine-1-carboxylate**

To a solution of 6-fluoronicotinamide **(Intermediate 338,** 0.29 g, 2.10 mmol) in 1,4-dioxane (20 mL) was added *tert*-butyl (*R*)-6-bromo-2-(1-methylpyrrolidin-2-yl)-1*H*-pyrrolo[3,2-c]pyridine-1-carboxylate **(Intermediate 4,** 1.0 g, 2.60 mmol) followed by cesium carbonate (2.60 g, 7.90 mmol) at room temperature. The reaction mixture was purged with argon for 15 min followed by the addition of $Pd_2(dba)_3$ (0.37g, 0.39 mmol) and Xantphos (0.47 g, 0.79 mmol). The reaction mixture was further purged with argon for 10 min and then heated at 100°C for 2h. The reaction mixture was concentrated *in vacuo* and the resulting crude material was purified by combi-flash chromatography by eluting with 80% ethyl acetate in heptane to afford *tert*-butyl (*R*)-6-(6-fluoronicotinamido)-2-(1-methylpyrrolidin-2-yl)-1*H*-pyrrolo[3,2-c]pyridine-1-carboxylate **(Intermediate 339,** 0.8 g, 69%) as a yellow solid. $^1$H NMR (400 MHz, DMSO-$d_6$) δ 1.59-1.65 (m, 2H), 1.69 (s, 9H), 1.72-1.78 (m, 2H), 2.35 (s, 3H), 2.38-2.42 (m, 1H), 3.11-3.18 (m, 1H), 3.88-3.96 (m, 1H), 6.76 (s, 1H), 7.30-7.34 (m, 1H), 8.52-8.58 (m, 1H), 8.61 (s, 1H), 8.86 (s, 1H), 8.92 (s, 1H), 11.07 (s, 1H). Mass spec: m/z: 440.1 [M+H]+.

**Step 7**

**Intermediate 340: *tert*-butyl 6-(6-(1'-(2-(2,6-dioxopiperidin-3-yl)-1-oxoisoindolin-4-yl)-[4,4'-bipiperidin]-1-yl)nicotinamido)-2-((*R*)-1-methylpyrrolidin-2-yl)-1*H*-pyrrolo[3,2-c]pyridine-1-carboxylate**

To a solution of *tert*-butyl (*R*)-6-(6-fluoronicotinamido)-2-(1-methylpyrrolidin-2-yl)-1*H*-pyrrolo[3,2-c]pyridine-1-carboxylate **(Intermediate 339,** 0.5 g, 1.14 mmol) in DMSO (5.0 mL) was added 3-(4-([4,4'-bipiperidin]-1-yl)-1-oxoisoindolin-2-yl)piperidine-2,6-dione hydrochloride **(Intermediate 337,** 0.61 g, 1.37 mmol) followed by *N, N*-diisopropylethylamine (0.75 g, 5.70 mmol) and the reaction mixture was heated at 110°C for 6h. The reaction mixture was quenched with water (50 mL), resulting in a precipitate, which was filtered and dried *in vacuo* to afford *tert*-butyl 6-(6-(1'-(2-(2,6-dioxopiperidin-3-yl)-1-oxoisoindolin-4-yl)-[4,4'-bipiperidin]-1-yl)nicotinamido)-2-((*R*)-1-methylpyrrolidin-2-yl)-1*H*-pyrrolo [3,2-c]pyridine-1-carboxylate **(Intermediate 340,** 0.53 g, 56% yield, crude) as a yellow solid, which was used in the next step without further purification. $^1$H NMR (400 MHz, DMSO-$d_6$) δ 1.15-1.24 (m, 2H), 1.39 (s, 9H), 1.60-1.70 (m, 2H), 1.72-1.90 (m, 7H), 1.85-1.93 (m, 2H), 1.96-2.03 (m, 1H), 2.09-2.14 (m, 1H), 2.17 (s, 3H), 2.23-2.30 (m, 1H), 2.56-2.63 (m, 1H), 2.65-2.74 (m, 2H), 2.81-2.94 (m, 3H), 3.11-3.18 (m, 1H), 3.26-3.30 (m, 2H), 3.38-3.46 (m, 2H), 4.26-4.34 (m, 1H), 4.40-4.56 (m, 3H), 5.09-5.14 (m, 1H), 6.37 (s, 1H), 6.87 (d, *J*=8.0 Hz, 1H), 7.15 (d, *J*=8.0 Hz, 1H), 7.29-7.33 (m, 1H), 7.40-7.45 (m, 1H), 8.13-8.19 (m, 2H), 8.48 (s, 1H), 8.79 (s, 1H), 10.23 (s, 1H), 10.97 (br s, 1H). Mass spec: m/z: 730.44 [M-100+H]+.

**Step 8**

**Example 75: 6-(1'-(2-(2,6-dioxopiperidin-3-yl)-1-oxoisoindolin-4-yl)-[4,4'-bipiperidin]-1-yl)-*N*-(2-((*R*)-1-methylpyrrolidin-2-yl)-1*H*-pyrrolo[3,2-c]pyridin-6-yl)nicotinamide**

To a solution of *tert*-butyl 6-(6-(1'-(2-(2,6-dioxopiperidin-3-yl)-1-oxoisoindolin-4-yl)-[4,4'-bipiperidin]-l-yl)nicotinamido)-2-((*R*)-1-methylpyrrolidin-2-yl)-1*H*-pyrrolo[3,2-c]pyridine-1-carboxylate **(Intermediate 340,** 0.5 g, 0.60 mmol) in 1,4-dioxane (10 mL) was added 4M HCl in dioxane (15 mL) at 0°C. The reaction mixture was then stirred at room temperature for 16h under a nitrogen atmosphere. The reaction mixture was concentrated under reduced pressure to

obtain the crude residue which was purified by preparative HPLC (Method A) to afford 6-(1'-(2-(2,6-dioxopiperidin-3-yl)-1-oxoisoindolin-4-yl)-[4,4'-bipiperidin]-1-yl)-*N*-(2-((*R*)-1-methylpyrrolidin-2-yl)-1*H*-pyrrolo[3,2-c]pyridin-6-yl)nicotinamide **(Example 75,** 0.075 g, 17%) as a an off-white solid. $^1$H NMR (400 MHz, DMSO-$d_6$) δ 1.13-1.52 (m, 7H), 1.76-1.84 (m, 4H), 1.85-1.93 (m, 2H), 1.96-2.03 (m, 1H), 2.09-2.14 (m, 1H), 2.16 (s, 3H), 2.23-2.30 (m, 1H), 2.56-2.63 (m, 1H), 2.65-2.74 (m, 2H), 2.81-2.94 (m, 3H), 3.11-3.18 (m, 1H), 3.26-3.30 (m, 2H), 3.38-3.46 (m, 2H), 4.26-4.34 (m, 1H), 4.40-4.56 (m, 3H), 5.09-5.14 (m, 1H), 6.37 (s, 1H), 6.87 (d, *J*=9.20 Hz, 1H), 7.15 (d, *J*=8.0 Hz, 1H), 7.29 (d, *J*=8.0 Hz, 1H), 7.42 (d, *J*=8.0 Hz, 1H), 8.11 (d, *J*=9.20 Hz, 1H), 8.16 (s, 1H), 8.48 (s, 1H), 8.79 (d, *J*=2.25 Hz, 1H), 10.23 (s, 1H), 10.97 (br s, 1H), 11.32 (s, 1H). Mass spec: m/z: 730.1 [M+H]$^+$.

**Intermediate 342 was synthesised following Scheme 75**

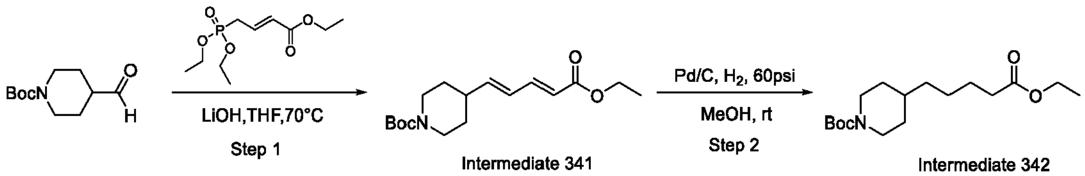


**Scheme 75**

**Step 1**

**Intermediate 341: tert-butyl 4-((1E, 3E)-5-ethoxy-5-oxopenta-1, 3-dien-1-ylpiperidine-1-carboxylate**

To a solution of tert-butyl 4-formylpiperidine-1-carboxylate (CAS No: 137076-22-3, 10.0 g, 46.9 mmol) and ethyl (E)-4-(diethoxyphosphoryl)but-2-enoate (CAS No: 42516-28-9, 11.7 g, 46.9 mmol) in tetrahydrofuran (100 mL) was added lithium hydroxide (1.35 g, 56.3 mmol) and the reaction mixture was heated at 80°C for 2h. The reaction mixture was diluted with water (50 mL), resulting in a precipitate, which was filtered and dried to obtain crude compound. The crude material was purified by combi-flash column chromatography by eluting with 30% EtOAc in heptane to afford tert-butyl 4-((1E,3E)-5 -ethoxy-5-oxopenta-1,3 -dien-1 -yl)piperidine-1 -carboxylate **(Intermediate 341,** 10.0 g, 70%) as a yellow liquid. Mass spec: m/z: 209.9 [M+H-100]$^+$.

**Step 2**

**Intermediate 342: tert-butyl 4-(5-ethoxy-5-oxopentyl)piperidine-1-carboxylate**

To a solution of tert-butyl 4-((1E,3E)-5-ethoxy-5-oxopenta-1,3-dien-1-yl)piperidine-1-carboxylate **(Intermediate 341,** 12.00 g, 40.62 mmol) in methanol (120 mL) was added 10% Pd/C (3.2 g, 30 mmol) at room temperature and the reaction mixture was stirred for 6h under $H_2$ atmosphere at 60 psi. The reaction mixture was filtered through celite and concentrated in *vacuo.* The crude material was purified by combi-flash column chromatography by eluting with 30% EtOAc in heptane to afford tert-butyl 4-(5-ethoxy-5-oxopentyl)piperidine-1-carboxylate **(Intermediate 342,** 9.6 g, 79%) as a colorless liquid. $^1$H NMR (400 MHz, DMSO-$d_6$) δ ppm 0.87-0.90 (m, 2H), 1.15-1.20 (m, 6H), 1.23-1.31 (m, 1H), 1.38 (s, 9H), 1.46-1.53 (m, 2H), 1.58-1.61 (m, 2H), 2.27 (t, *J*=7.23 Hz, 2H), 2.62-2.65 (m, 2H), 3.89-3.91 (m, 2H), 4.01-4.06 (m, 2H). Mass spec: m/z: 214.5 [M+H-100]$^+$.

**Intermediate 345 was synthesised following Scheme 76**

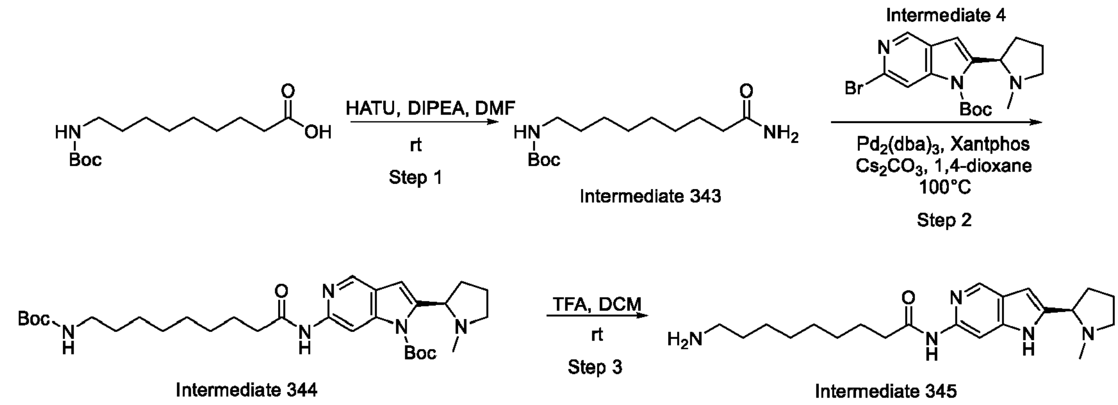


**Scheme 76**

**Step** 1

**Intermediate 343: tert-butyl N-(8-carbamoyloctyl)carbamate**

To a stirred solution of 9-[(tert-butoxycarbonyl)amino]nonanoic acid (CAS No: 173435-78-4, 300 mg, 1.09 mmol), HATU (537 mg, 1.41 mmol), DIPEA (702 mg, 5.43 mmol) in anhydrous DMF (6 mL) was added $NH_4Cl$ (174 mg, 3.26 mmo). The reaction was stirred at rt for 2h and poured into water (50mL) and filtered. The filter cake was washed with water and dried to get tert-butyl N-(8-carbamoyloctyl)carbamate **(Intermediate 343, 240** mg, 73.0%) as a white solid, which was used in the next step without further purification. Mass spec: m/z/: 295.20 $[M+Na]^+$.

**Step 2**

**Intermediate 344: N-[8-({2-[(2R)-1-methylpyrrolidin-2-yl]-1H-pyrrolo[3,2-c]pyridin-6-yl}carbamoyl)octyl]carbamate**

To a solution of tert-butyl N-(8-carbamoyloctyl)carbamate **(Intermediate 343,** 100 mg, 0.360 mmol) and tert-butyl 6-bromo-2-[(2R)-1-methylpyrrolidin-2-yl]pyrrolo[3,2-c]pyridine-1-carboxylate **(Intermediate 4,** 137 mg, 0.327 mmol) in 1,4-dioxane(4 mL) was added $Cs_2CO_3$ (293 mg, 0.900 mmol), Xantphos (41.6 mg, 0.0720 mmol) and $Pd_2(dba)_3$ (33.0 mg, 0.0360 mmol) and the reaction was stirred for 3h at 100°C under a nitrogen atmosphere. The volatiles were removed under reduced pressure and the crude residue was purified by a C18 silica column, eluting with MeCN/$H_2O$ (37/63) to afford tert-butyl N-[8-({2-[(2R)-1-methylpyrrolidin-2-yl]-1H-pyrrolo[3,2-c]pyridin-6-yl}carbamoyl)octyl]carbamate **(Intermediate 344,** 120 mg, 70%) as a yellow solid. Mass spec: m/z: 572.25 $[M+H]^+$.

**Step 3**

**Intermediate 345: 9-amino-N-{2-[(2R)-1-methylpyrrolidin-2-yl]-1H-pyrrolo[3,2-c]pyridin-6-yl}nonanamide ditrifluoroacetate**

To a solution of tert-butyl 6-{9-[(tert-butoxycarbonyl)amino]nonanamido}-2-[(2R)-1-methylpyrrolidin-2-yl]pyrrolo[3,2-c]pyridine-1-carboxylate **(Intermediate 344,** 120 mg, 0.184 mmol) in DCM (2 mL) was added TFA (1 mL) and the reaction was stirred at rt for 2h. The volatiles were removed under reduced pressure to afford 9-amino-N-{2-[(2R)-1-methylpyrrolidin-2-yl]-1H-pyrrolo[3,2-c]pyridin-6-yl}nonanamide ditrifluoroacetate **(Intermediate 345,** 80.0 mg, 99.7%) as a brown oil. Mass spec: m/z: 372.25 $[M+H]^+$.

**Example 76: Efficacy *in vitro***

**MLLT1 Degradation Assay:**

MLLT1 degradation efficacy of compounds were tested in an automated chemiluminescent detection system (JESS, Cat# 004-650). MV4-11 (#CRL-9591, ATCC) cells were seeded at a density of 1.2 million cells/well in a non-treated 6-well cell culture plate in 2.4 mL of complete medium (IMDM + 10% FBS + 1X Penicillin-Streptomycin). 10 mM DMSO stocks were prepared, and cells were treated with three concentrations of test compounds viz. 1000, 100 and 10 nM at a

final DMSO concentration of 0.1% and incubated for 24h at 37°C, 5% $CO_2$. Post incubation, cells were centrifuged, washed with HBSS and lysed in 70 uL of RIPA buffer supplemented with Protease inhibitors (sc-24948; Santa Cruz). Subsequently, protein estimation was done using BCA protein assay kit (23225; Pierce). 4 ug protein lysate (4 uL of 1ug/uL lysate) was loaded into each well of the JESS cartridge (SM-WO01; Bio-techne) and the JESS run was performed using 1:900 dilution of anti-human MLLT1 antibody (14893; Cell signaling technology) and 1:1200 dilution of anti-human GAPDH antibody (2118; Cell signaling technology) with anti-rabbit secondary detection module (DM-001; Bio-Techne). Post run, data were analysed using "Compass for SW" software for chemiluminescent detection. The peak area for each band was used for the calculation of MLLT1 degradation with respect to DMSO control. Data is shown as "MLLT1 % degradation".

**Category A:** A ≥ 75% degradation
**Category B:** 50% ≤ B < 75% degradation
**Category C:** 25% ≤ C < 50% degradation
**Category D:** D < 25% degradation

| Example Number | MLLT 1 % Degradation @ 1 μM | MLLT1 % Degradation @ 0.1 μM | MLLT1 % Degradation @ 0.01 μM |
|---|---|---|---|
| 1 | C | C | C |
| 2 | A | A | A |
| 3 | A | A | C |
| 4 | A | A | B |
| 5 | A | A | B |
| 6 | B | A | A |
| 7 | A | A | A |
| 8 | A | A | A |
| 9 | A | A | A |
| 10 | B | A | A |
| 11 | B | C | D |
| 12 | A | A | B |
| 13 | A | A | A |
| 14 | A | A | B |
| 15 | B | A | B |
| 16 | B | A | A |
| 17 | A | A | A |
| 18 | A | A | B |
| 19 | A | A | B |
| 20 | C | B | B |
| 21 | B | A | A |
| 22 | A | A | A |
| 23 | C | C | C |
| 24 | A | A | A |
| 25 | A | A | A |
| 26 | C | D | D |
| 27 | B | C | C |
| 29 | A | C | D |
| 30 | A | B | C |
| 33 | A | A | B |

(continued)

| Example Number | MLLT 1 % Degradation @ 1 μM | MLLT1 % Degradation @ 0.1 μM | MLLT1 % Degradation @ 0.01 μM |
|---|---|---|---|
| 34 | A | A | ND |
| 35 | A | A | A |
| 36 | A | A | A |
| 37 | A | A | B |
| 38 | C | D | D |
| 40 | B | C | ND |
| 41 | A | B | B |
| 42 | B | B | ND |
| 43 | B | A | ND |
| 44 | C | C | ND |
| 45 | A | A | A |
| 46 | B | B | ND |
| 47 | A | B | C |
| 49 | C | D | D |
| 50 | B | B | B |
| 51 | A | A | A |
| 52 | B | A | A |
| 53 | B | A | A |
| 54 | B | A | A |
| 55 | A | A | A |
| 56 | A | A | A |
| 57 | A | A | A |
| 58 | C | A | B |
| 59 | B | A | A |
| 60 | A | A | A |
| 61 | B | A | A |
| 62 | B | A | B |
| 63 | C | D | D |
| 64 | A | B | B |
| 65 | B | B | B |
| 66 | A | A | A |
| 67 | A | A | A |
| 69 | C | C | C |
| 71 | B | A | A |
| 72 | C | A | A |
| 73 | B | A | B |
| 74 | B | B | B |

(continued)

| Example Number | MLLT 1 % Degradation @ 1 μM | MLLT1 % Degradation @ 0.1 μM | MLLT1 % Degradation @ 0.01 μM |
|---|---|---|---|
| 75 | A | A | A |
| ND = not determined | | | |

**Table 6**

**MV4-11 Cellular Proliferative Assays**

**Assay Format A**

An MV4-11 proliferation assay was used to test the ability of compounds to reduce cell viability. MV4-11 cells (ATCC #CRL9591) were grown in IMDM medium (11510596, Gibco) supplemented with 10% fetal bovine serum (F7524, Gibco) and 1% Penicillin/Streptomycin (15140-122, Gibco) at 37°C with 5% $CO_2$. Cells were seeded in 384-well Elplasia plates (4447, Corning) at a density of 50 cells/well and treated with vehicle (DMSO) or compounds (10 serial semi-log dilutions, 30 μM as top concentration).

After 120 hours of treatment, cells were incubated for 10 min at RT with the CellTiter-Glo (CTG) reagent (G7571, Promega). Measurement of the luminescence signal was performed on a microplate reader (Ensight, PerkinElmer). CTG reagent measures the amount of ATP, which is directly proportional to the number of cells, to determine the number of viable cells in culture.

The data were analysed as follows:

$$\text{Data normalization, } N(x) = \frac{x - \langle cr \rangle}{\langle sr \rangle - \langle cr \rangle} \times 100$$

where x is the measured raw signal value of a well, (cr) is the median of the measured signal values for the Central Reference (DMSO) wells on a plate, (sr) is the median of the measured signal values for the Scale Reference (reference compound) wells on a plate.

$$\text{Curve fitting, } S_0 + \frac{S_{\text{inf}} - S_0}{1 + \left(\frac{AC_{50}}{10^C}\right)^n}$$

where the experimental set-up assumes the Hill parameters in certain ranges: $S_0$ ~ Central Reference (CR) from normalization (= Bottom), $S_{inf}$ ~ Scale Reference (SR) from normalization (= Top), nHill ~ 1, $AC_{50}$ ~ within the measured concentration range.

**Assay Format B**

MV4-11 cells at a confluency of -50-80% was used for cell growth assay. 600 cells/well in a 384-well opaque cell culture plate (6007680; Perkin Elmer) were seeded in 40 uL of complete medium (IMDM + 10% FBS + 1X Penicillin-Streptomycin). Cells were incubated overnight at 37°C, 5% $CO_2$. 10 mM DMSO stocks were prepared, cells were treated with 10 concentrations of test compounds starting from 1 uM or 100 nM, 3-fold dilution in 10 uL (5x concentration) of complete medium. Final DMSO concentration was 0.3%. Culture plates were centrifuged at 40 x g, 30s. Cells were incubated with compounds for 168 h (7 days). After 7 days, 20 uL CTG 2.0 reagent (G9242; Promega) was added per well and luminescence reading was taken in Envision Xcite 2105 multimode plate reader. Data was analyzed in GraphPad Prism 10.0 and data was shown as % viability with respect to DMSO control and dose-response curves were generated using four parameter logistic regression. Assay quality was determined by z-factor calculation using positive and negative controls. $IC_{50}$ was ≤ 100 nM for each of the Examples set out in the Table below:

**Table 7**

| Example Number | Assay format |
|---|---|
| 2 | B |

(continued)

| Example Number | Assay format |
|---|---|
| 3 | A |
| 4 | A |
| 6 | B |
| 7 | B |
| 8 | B |
| 9 | B |
| 10 | B |
| 12 | B |
| 13 | B |
| 14 | B |
| 15 | B |
| 16 | B |
| 17 | B |
| 19 | A |
| 20 | A |
| 21 | A |
| 22 | A |
| 24 | A |
| 25 | A |
| 29 | A |
| 33 | A |
| 34 | A |
| 35 | A |
| 36 | A |
| 37 | A |
| 40 | A |
| 41 | A |
| 42 | A |
| 43 | A |
| 45 | A |
| 47 | A |
| 48 | A |
| 51 | A |
| 52 | A |
| 53 | A |
| 54 | A |
| 55 | A |
| 56 | A |
| 57 | A |

(continued)

| Example Number | Assay format |
|---|---|
| 58 | A |
| 59 | B |
| 60 | B |
| 61 | B |
| 62 | B |
| 65 | B |
| 66 | B |
| 67 | B |
| 68 | A |
| 75 | B |

In one embodiment, the invention provides compounds according to the following aspects.

1. A compound which is a Proteolysis Targeting Chimera (PROTAC) or a pharmaceutically acceptable salt thereof, wherein the PROTAC has the structure:

**M-LINK-U**

wherein U is an E3 ubiquitin ligase binding moiety, LINK is a moiety that covalently links M and U, and M is an MLLT1 and/or MLLT3 binder of formula (I):

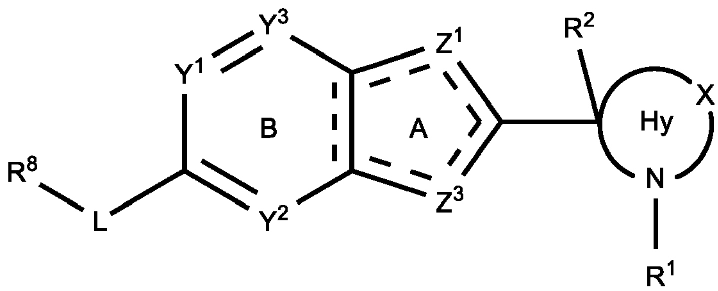

**(I)**

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;

Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;

L is -C(O)N(H)-, wherein the C atom of L is bonded to $R^8$, and the N atom of L is bonded to ring B;

$R^1$ is H, $C_{1-4}$ cycloalkyl, or $C_{1-4}$ alkyl which is itself unsubstituted or substituted with one $C_{1-4}$ alkoxy or one, two or three halo;

$R^2$ is H or methyl;

each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii)

two $R^3$ linked to the same C atom in ring Hy form, together with the C atom to which they are attached, a $C_{3-6}$

cycloalkyl ring or a $C_{3-6}$ heterocycloalkyl ring;

$R^4$ and $R^6$ are independently selected from H, halo, CN, $C_{1-4}$ alkoxy, and $C_{1-4}$ alkyl which is itself unsubstituted or substituted by one, two or three halo;

$R^5$ is selected from H, halo, $C_{1-4}$ alkoxy, $C_{3-5}$ cycloalkyl and $C_{1-4}$ alkyl which is itself unsubstituted or substituted by one, two or three halo;

$R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two R9;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and

each $R^{11}$ is independently selected from H, $C_{1-4}$ alkyl, and $C_{1-4}$ cycloalkyl;

wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

2. A compound according to aspect 1, wherein LINK is (a) a single bond, or (b) a chemical linker group represented by formula (L):

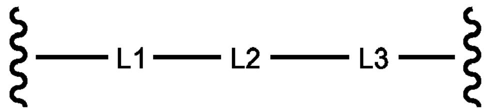

(L)

wherein:

LINK is attached to M via L1;

LINK is attached to U via L3;

L1 and L3 are each independently selected from a single bond, -N(R')-, -C(O)N(R')-, -O-, -N(R')C(O)-, -C(O)-, -$S(O_2)$N(R')-, -N(R')$S(O_2)$-, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;

L2 is represented by the formula $-(L4)_m-$;

each L4 is independently selected from a divalent 3- to 6-membered cycloalkyl ring, a divalent 4- to 7-membered heterocyclyl ring and a unit of formula:

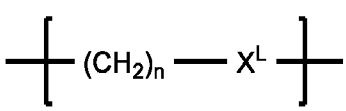 or 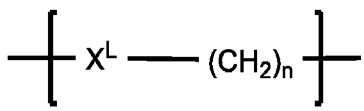 ;

each $X^L$ is independently selected from a single bond, -N(R')-, -O-, -C(O)-, -S-, -SO-, - $SO_2$-, -C(H)=C(H)- and -C(H)=C(H)-;

n is selected from 1 to 4;

m is selected from 1 to 30; and

each R' is independently selected from H and $C_{1-4}$ alkyl.

3. A compound according to aspect 2, wherein each L4 is independently selected from a divalent 4- to 7-membered heterocyclyl ring and a unit of formula

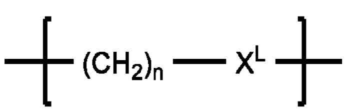 or 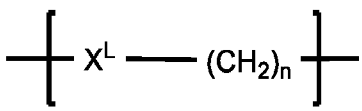 ;

wherein each $X^L$ is independently selected from a single bond, -O-, or -S-; and

n is selected from 1 or 2.

4. A compound according to aspect 2 or 3, wherein each divalent 4- to 7-membered heterocyclyl ring of L1, L2 and L3 when present contains at least one N atom.

5. A compound according to aspect 4, wherein each divalent 4- to 7-membered heterocyclyl ring of L1, L2 and L3 when present contains one or two N atoms.

6. A compound according to aspect 5, wherein each divalent 4- to 7-membered heterocyclyl ring of L1, L2 and L3 when present is independently selected from piperidinyl and diazinanyl.

7. A compound according to any one of aspects 2 to 6, wherein L2 is selected from $-(CH_2)_m-$, $-(CH_2CH_2O)_m-$, $-(OCH_2CH_2)_m-$, $-(CH_2CH_2S)_m-$, $-(SCH_2CH_2)_m-$, and a divalent 4- to 7-membered heterocyclyl ring, preferably wherein L2 is selected from $-(CH_2)_m$ and a divalent 4- to 7-membered heterocyclyl ring.

8. A compound according to any one of aspects 2 to 7, wherein m is selected from 1 to 10.

9. A compound according to any one of aspects 2 to 8, wherein L1 and L3 are each independently selected from a single bond, $-(C_{1-6}$ alkylene)-, -N(R')-, -C(O)N(R')-, -O-, -N(R')C(O)-, -C(O)-, $-S(O_2)N(R')-$, $-N(R')S(O_2)-$, ethynyl, and a divalent 4- to 7-membered heterocyclyl ring.

10. A compound according to any one of aspects 2 to 9, wherein L1 and L3 are each independently selected from a single bond, $-(C_{1-6}$ alkylene)-, -N(R')-, -C(O)N(R')-, - N(R')C(O)-, -O-, ethynyl, and a divalent 4- to 7-membered heterocyclyl ring.

11. A compound according to any one of aspects 2 to 10, wherein L1 and L3 are each independently selected from a single bond, $-(C_{1-6}$ alkylene)-, -N(R')-, -C(O)N(R')-, -N(R')C(O)-, -O-, ethynyl, piperidinyl and diazinanyl, preferably wherein L1 and L3 are each independently selected from a single bond, $-(C_{1-6}$ alkylene)-, -N(R')-, - C(O)N(R')-, -N(R') C(O)-, ethynyl, piperidinyl and diazinanyl.

12. A compound according to any one of aspects 1 to 11, wherein U is a CRBN or VHL E3 ubiquitin ligase binding moiety.

13. A compound according to aspect 12, wherein U is a CRBN E3 ubiquitin ligase binding moiety.

14. A compound according to aspect 12, wherein U is a VHL E3 ubiquitin ligase binding moiety.

15. A compound according to any one of aspects 1 to 12, wherein U is:

(a) a CRBN E3 ubiquitin ligase binding moiety of formula (U1):

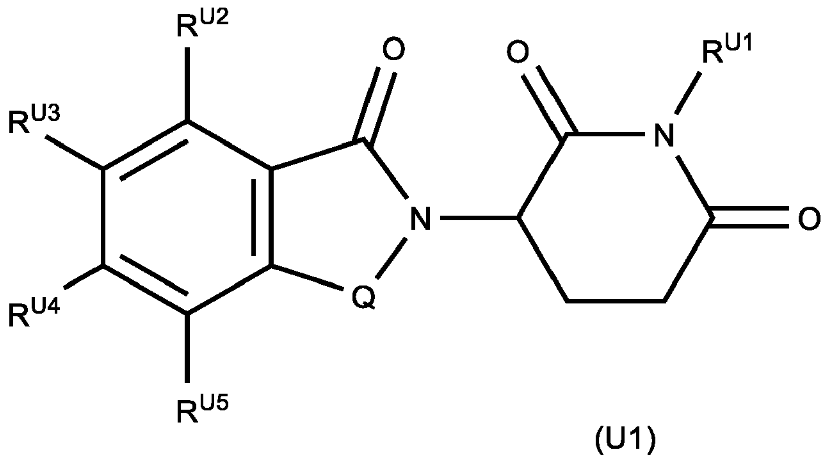

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

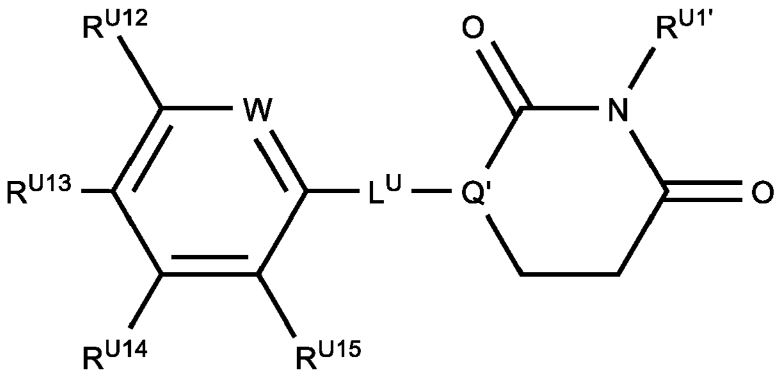

(U4)

wherein:

$R^{U1'}$ is H;
W is N or $CR^{U16}$;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

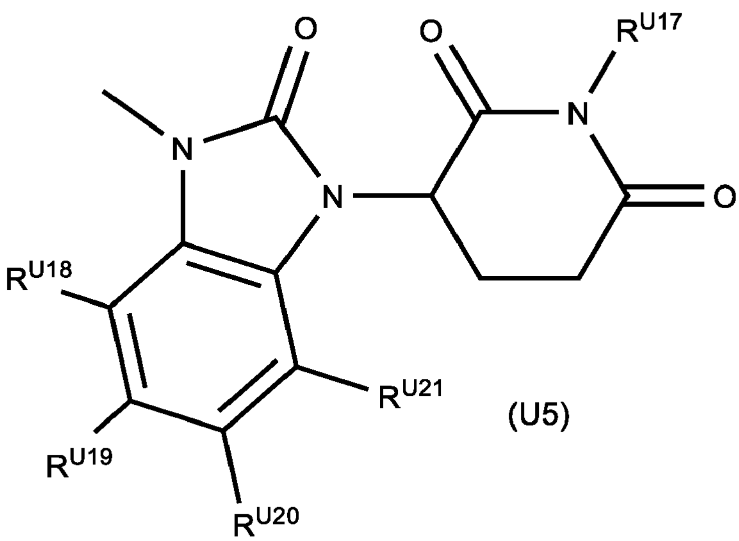

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;
wherein one and only one of $R^{U18}$, $R^{U19}$, $R^{U20}$ and $R^{U21}$ is a single bond to LINK;
or (d) a VHL E3 ubiquitin ligase binding moiety of formula (U2):

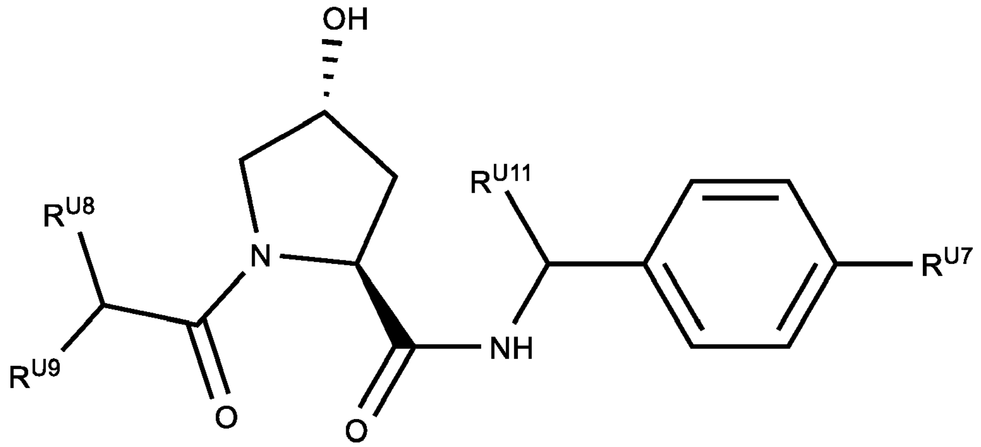

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

16. A compound according to aspect 15, wherein U is a VHL E3 ubiquitin ligase binding moiety of formula (U3):

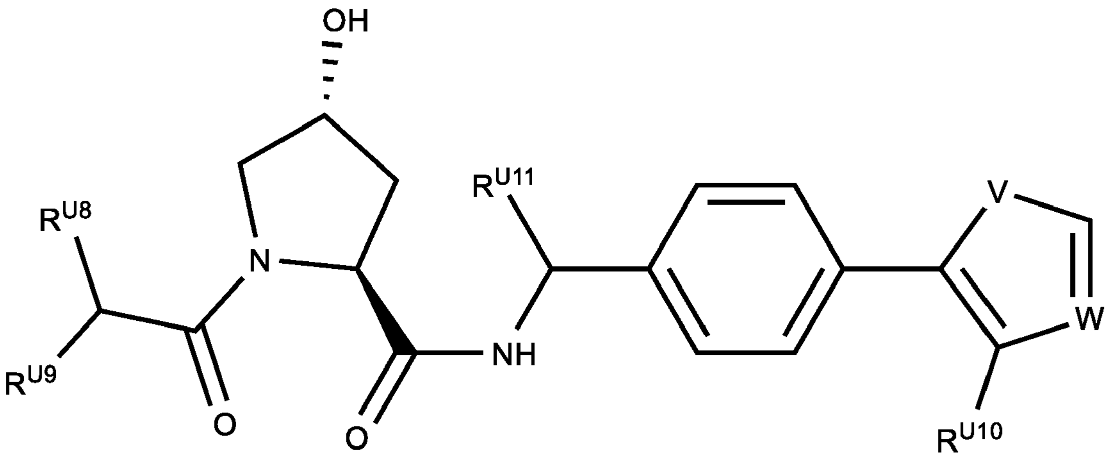

(U3)

wherein:

V is S or O;

W is N or CH;
$R^{U10}$ is H or $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

17. A compound according to aspect 16, wherein $R^{U10}$ is H or methyl.

18. A compound according to any one of aspects 15 to 17, wherein $R^{U8}$ is t-butyl.

19. A compound according to aspect 15, wherein U is a CRBN ubiquitin ligase binding moiety of formula (U1), wherein $R^{U6}$ is H or methyl.

20. A compound according to aspect 15 or 19, wherein U is a CRBN ubiquitin ligase binding moiety of formula (U1), wherein Q is selected from -C(O)- and $-CH_2-$.

21. A compound according to any one of aspects 15, 19 and 20, wherein U is a CRBN ubiquitin ligase binding moiety of formula (U1), wherein three of $R^{U2}$, $R^{U3}$, $R^{U4}$ and $R^{U5}$ are H.

22. A compound according to aspect 15, wherein U is a CRBN ubiquitin ligase binding moiety of formula (U4), wherein Q' is N.

23. A compound according to aspect 15 or 22, wherein U is a CRBN ubiquitin ligase binding moiety of formula (U4), wherein Q' is CH.

24. A compound according to aspect 23, wherein $L^U$ is a single bond.

25. A compound according to aspect 23, wherein $L^U$ is -C(O)N(H)-, wherein the C atom of $L^U$ is bonded to phenyl, and the N atom of $L^U$ is bonded to Q'.

26. A compound according to any one of aspects 15 and 22 to 25, wherein U is a CRBN ubiquitin ligase binding moiety of formula (U4), wherein $R^{U15}$ and $R^{U16}$ are H, and two of $R^{U12}$, $R^{U13}$ and $R^{U14}$ are H.

27. A compound according to any one of aspects 15 and 22 to 25, wherein U is a CRBN ubiquitin ligase binding moiety of formula (U4), wherein $R^{U13}$, $R^{U14}$ and $R^{U15}$ are H, $R^{U12}$ is a single bond to LINK, and $R^{U16}$ is selected from H, F and Me.

28. A compound according to aspect 15, wherein U is a CRBN ubiquitin ligase binding moiety of formula (U5), wherein three of $R^{U18}$, $R^{U19}$, $R^{U20}$ and $R^{U21}$ are H.

29. A compound according to any one of the preceding aspects, wherein the N atom of ring Hy which is substituted by $R^1$ is adjacent to the C atom of ring Hy that is bonded to ring A.

30. A compound according to any one of the preceding aspects, wherein no more than two of $Y^1$, $Y^2$ and $Y^3$ are N.

31. A compound according to any one of the preceding aspects, wherein $Z^1$ is - $C(R^4)$- and $Z^3$ is -N(H)-.

32. A compound according to any one of the preceding aspects, wherein $Z^1$ is - $C(R^4)$-, $Z^3$ is -N(H)-, $Y^1$ is N, $Y^2$ is $-C(R^6)$- and $Y^3$ is $-C(R^5)$-.

33. A compound according to any one of the preceding aspects, wherein X is a bond, -N(Me)-, O or $-CH_2-$.

34. A compound according to any one of the preceding aspects, wherein X is a bond.

35. A compound according to any one of the preceding aspects, wherein M is of formula (II):

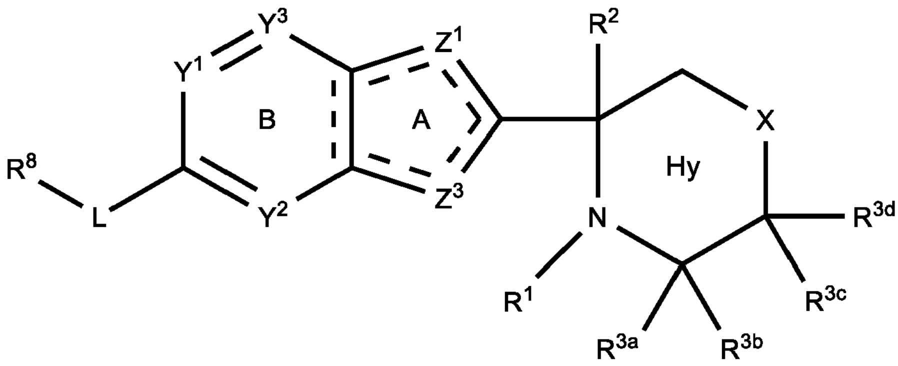

(II)

wherein:

$Z^1$, $Z^3$, $Y^1$, $Y^2$, $Y^3$, $R^1$, $R^2$, $R^8$, X and L are as defined in any one of the preceding aspects;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is $-N(R^{11})-$, O, S, $-S(O)_2-$ or $-S(O)(NR^{11})-$, then neither $R^{3c}$ nor $R^{3d}$ are halo;
wherein at least two of $R^{3a}$, $R^{3b}$, $R^{3c}$ and $R^{3d}$ are H; and
wherein either (i) $R^8$ is a bond to LINK, or (ii) $R^8$ is a group and M is bonded to LINK via a C or N atom within group $R^8$ such that a hydrogen atom on the C or N atom within group $R^8$ is replaced with a bond to LINK.

36. A compound according to any one of the preceding aspects, wherein $R^4$, $R^5$ and $R^6$ are H.

37. A compound according to any one of aspects 1 to 35, wherein one of $R^4$, $R^5$ and $R^6$ is not H.

38. A compound according to aspect 37, wherein one of $R^4$, $R^5$ and $R^6$ is selected from fluoro, chloro, methoxy, methyl and trifluoromethyl, and the rest are H.

39. A compound according to any one of aspects 1 to 28, wherein M is of formula (III):

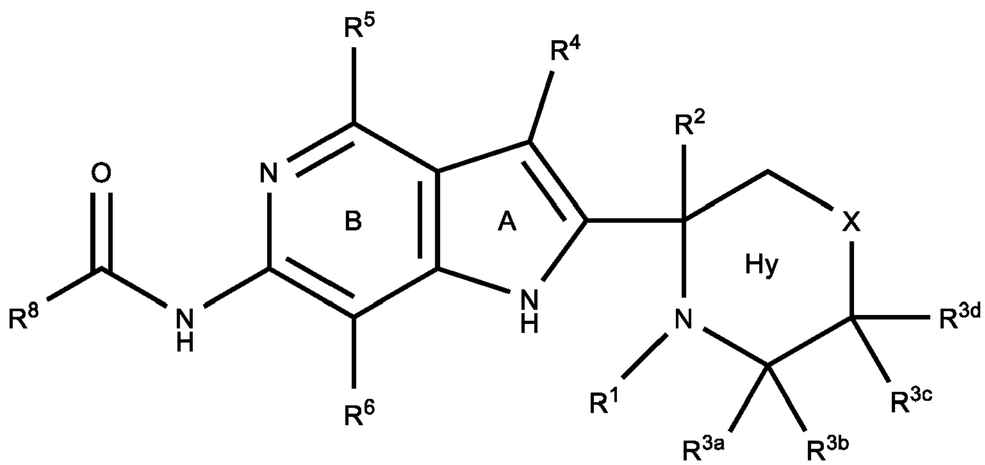

(III)

wherein:

$R^1$ is H or $C_{1-4}$ alkyl which is itself unsubstituted or substituted with one $C_{1-4}$ alkoxy or

one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring,
with the proviso that when X is -N(Me)- or O, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from H, CN, halo, $C_{1-4}$ alkoxy, and $C_{1-4}$ alkyl which is itself unsubstituted or substituted by one, two or three halo;
$R^5$ and $R^6$ are independently selected from H, halo, $C_{1-4}$ alkoxy, and $C_{1-4}$ alkyl which is itself unsubstituted or substituted by one, two or three halo;
$R^8$ is (i) a bond to LINK or (ii) a group selected from a 6-membered aryl ring, a 5- to 6-membered heteroaryl ring, and a 5- to 6-membered heterocyclyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

40. A compound according to aspect 39, wherein the C or N atom within group $R^8$ that is bonded to LINK is a C or N atom of the aryl, heteroaryl, or heterocyclyl, ring of group $R^8$, or a C or N atom of the heteroaryl, cycloalkyl, heterocyclyl or phenyl ring of $R^{10}$.

41. A compound according to any one of aspects 1 to 40, wherein $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl and cyclopropyl.

42. A compound according to aspect 41, wherein $R^8$ is a 5- to 6-membered heteroaryl ring or phenyl, the group $R^8$ being unsubstituted or substituted by one or two $C_{1-4}$ alkyl.

43. A compound according to any one of the preceding aspects, wherein Hy is an unsubstituted pyrrolidine ring, $R^1$ is methyl, and $R^2$ is H.

44. A compound according to aspect 1, wherein the PROTAC structure is selected from:

4-(4-(2-(2,6-dioxopiperidin-3-yl)-1-oxoisoindolin-4-yl)but-3-yn-1-yl)-*N*-(2-((*R*)-1-methylpyrrolidin-2-yl)-1*H*-pyrrolo[3,2-*c*]pyridin-6-yl)piperidine-1-carboxamide;
6-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)nicotinamide;
4-((1-(2-(2,6-dioxopiperidin-3-yl)-1,3-dioxoisoindolin-4-yl)piperidin-4-yl)methyl)-*N*-(2-((*R*)-1-methylpyrrolidin-2-yl)-1*H*-pyrrolo[3,2-*c*]pyridin-6-yl)benzamide;
4-(2-(1-(2-(2,6-dioxopiperidin-3-yl)-1,3-dioxoisoindolin-4-yl)piperidin-4-yl)ethyl)-*N*-(2-((*R*)-1-methylpyrrolidin-2-yl)-1*H*-pyrrolo[3,2-c]pyridin-6-yl)benzamide;
6-(4-((1-(2-(2,6-dioxopiperidin-3-yl)-1,3-dioxoisoindolin-4-yl)piperidin-4-yl)methyl)piperazin-1-yl)-*N*-(2-((*R*)-1-methylpyrrolidin-2-yl)-1*H*-pyrrolo[3,2-c]pyridin-6-yl)nicotinamide;
6-(4-(3-(2-(2,6-dioxopiperidin-3-yl)-1-oxoisoindolin-4-yl)prop-2-yn-1-yl)piperazin-1-yl)-*N*-(2-((*R*)-1-methylpyrrolidin-2-yl)-1*H*-pyrrolo[3,2-*c*]pyridin-6-yl)nicotinamide;
*N*-((S)-2,6-dioxopiperidin-3-yl)-6-(6-(4-((2-((*R*)-1-methylpyrrolidin-2-yl)-1*H*-pyrrolo[3,2-c]pyridin-6-yl)carbamoyl)phenyl)hex-1-yn-1-yl)picolinamide;
4-(6-(2-(2, 6-dioxopiperidin-3-yl)-1-oxoisoindolin-4-yl)hex-5-yn-1-yl)-*N*-(2-((*R*)-1-methylpyrrolidin-2-yl)-1*H*-pyrrolo[3,2-c]pyridin-6-yl)benzamide;

(*R*)-4-(6-(6-(2,4-dioxotetrahydropyrimidin-1(2*H*)-yl)pyridin-2-yl)hex-5-yn-1-yl)-*N*-(2-(1-methylpyrrolidin-2-yl)-1*H*-pyrrolo[3,2-c]pyridin-6-yl)benzamide;
6-(6-(2-(2, 6-dioxopiperidin-3-yl)-1-oxoisoindolin-4-yl) hex-5-yn-1-yl)-*N*-(2-((*R*)-1-methylpyrrolidin-2-yl)-1*H*-pyrrolo [3, 2-*c*] pyridin-6-yl) nicotinamide;
*N*-((*S*)-2,6-dioxopiperidin-3-yl)-5-(5-(4-((2-((*R*)-1-methylpyrrolidin-2-yl)-1*H*-pyrrolo[3,2-c]pyridin-6-yl)carbamoyl)phenyl)pent-1-yn-1-yl)picolinamide;
6-(5-(2-(2,6-dioxopiperidin-3-yl)-1-oxoisoindolin-4-yl)pent-4-yn-1-yl)-*N*-(2-((*R*)-1-methylpyrrolidin-2-yl)-1*H*-pyrrolo[3,2-c]pyridin-6-yl)nicotinamide;
*N*-(3-chloro-2-((*R*)-1-methylpyrrolidin-2-yl)-1*H*-pyrrolo [3, 2-c] pyridin-6-yl)-9-(2-(2, 6-dioxopiperidin-3-yl)-1-oxoisoindolin-4-yl) non-8-ynamide;
3-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide;
4-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide;
6-(4-(3-(2-(2,6-dioxopiperidin-3-yl)-1-oxoisoindolin-4-yl)prop-2-yn-1-yl)piperidin-1-yl)-*N*-(2-((*R*)-1-methylpyrrolidin-2-yl)-1*H*-pyrrolo[3,2-*c*]pyridin-6-yl)nicotinamide;
6-(4-((2-(2,6-dioxopiperidin-3-yl)-1-oxoisoindolin-4-yl)ethynyl)piperidin-1-yl)-*N*-(2-((R)-1-methylpyrrolidin-2-yl)-1*H*-pyrrolo[3,2-c]pyridin-6-yl)nicotinamide;
6-(4-((1-(2-(2,6-dioxopiperidin-3-yl)-1,3-dioxoisoindolin-4-yl)piperidin-4-yl)methoxy)piperidin-1-yl)-*N*-(2-((*R*)-1-methylpyrrolidin-2-yl)-1*H*-pyrrolo[3,2-c]pyridin-6-yl)nicotinamide;
4-((4-(2-(2,6-dioxopiperidin-3-yl)-1-oxoisoindolin-4-yl)but-3-yn-1-yl)oxy)-*N*-(2-((*R*)-1-methylpyrrolidin-2-yl)-1*H*-pyrrolo[3,2-c]pyridin-6-yl)benzamide;
4-(1-(5-(2-(2,6-dioxopiperidin-3-yl)-1-oxoisoindolin-4-yl)pent-4-yn-1-yl)-1*H*-pyrazol-3-yl)-*N*-(2-((*R*)-1-methylpyrrolidin-2-yl)-1*H*-pyrrolo[3,2-c]pyridin-6-yl)benzamide;
4-(1-(5-(2-(2,6-dioxopiperidin-3-yl)-1-oxoisoindolin-4-yl)pent-4-yn-1-yl)-1H-pyrazol-4-yl)-N-(2-((R)-1-methylpyrrolidin-2-yl)-1H-pyrrolo[3,2-c]pyridin-6-yl)benzamide;
4-(1-(5-(2-(2,6-dioxopiperidin-3-yl)-1-oxoisoindolin-5-yl)pent-4-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide;
3-chloro-4-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)benzamide;
4-(((2S)-4-(2-(2,6-dioxopiperidin-3-yl)-1-oxoisoindolin-4-yl)but-3-yn-2-yl)oxy)-N-(2-((R)-1-methylpyrrolidin-2-yl)-1H-pyrrolo[3,2-c]pyridin-6-yl)benzamide;
4-(((2R)-4-(2-(2,6-dioxopiperidin-3-yl)-l-oxoisoindolin-4-yl)but-3-yn-2-yl)oxy)-N-(2-((R)-1-methylpyrrolidin-2-yl)-1H-pyrrolo[3,2-c]pyridin-6-yl)benzamide;
3-((9-(2-(2,6-dioxopiperidin-3-yl)-1,3-dioxoisoindolin-4-yl)non-8-yn-1-yl)oxy)-N (2-((*R*)-1-methylpyrrolidin-2-yl)-1*H*-pyrrolo[3,2-c]pyridin-6-yl)benzamide;
7-{[2-(2,6-dioxopiperidin-3-yl)-1,3-dioxoisoindol-5-yl]amino}-N-{2-[(2R)-1-methylpyrrolidin-2-yl]-1H-pyrrolo [3,2-c] pyridin-6-yl}heptanamide;
7-{[2-(2,6-dioxopiperidin-3-yl)-1,3-dioxoisoindol-5-yl]amino}-N-{2-[(2S)-1-methylpyrrolidin-2-yl]-1H-pyrrolo [3,2-c]pyridin-6-yl}heptanamide;
7-{[2-(2,6-dioxopiperidin-3-yl)-1,3-dioxoisoindol-4-yl]amino}-N-{2-[(2R)-1-methylpyrrolidin-2-yl]-1H-pyrrolo [3,2-c] pyridin-6-yl}heptanamide;
9-{[2-(2,6-dioxopiperidin-3-yl)-1,3-dioxoisoindol-4-yl]amino}-N-{2-[(2R)-1-methylpyrrolidin-2-yl]-1H-pyrrolo [3,2-c]pyridin-6-yl}nonanamide;
12-{[2-(2,6-dioxopiperidin-3-yl)-1,3-dioxoisoindol-4-yl]amino}-N-{2-[(2R)-1-methylpyrrolidin-2-yl]-1H-pyrrolo [3,2-c]pyridin-6-yl}dodecanamide;
12-{[2-(2,6-dioxopiperidin-3-yl)-1,3-dioxoisoindol-4-yl]amino}-N-{2-[(2S)-1-methylpyrrolidin-2-yl]-1H-pyrrolo [3,2-c]pyridin-6-yl}dodecanamide;
N-{3-chloro-2-[(2R)-1-methylpyrrolidin-2-yl]-1H-pyrrolo[3,2-c]pyridin-6-yl}-7-{[2-(2,6-dioxopiperidin-3-yl)-1,3-dioxoisoindol-4-yl]amino}heptanamide;
4-[1-(3-{1-[2-(2,6-dioxopiperidin-3-yl)-1,3-dioxoisoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide;
4-[1-(3-{1-[2-(2,6-dioxopiperidin-3-yl)-1-oxo-3H-isoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide;
4-(3-{[2-(2,6-dioxopiperidin-3-yl)-1,3-dioxoisoindol-4-yl]amino}propyl)-N-{2-[(2R)-1-methylpyrrolidin-2-yl]-1H-pyrrolo[3,2-c]pyridin-6-yl}benzamide;
4-(3-{[2-(2,6-dioxopiperidin-3-yl)-1,3-dioxoisoindol-4-yl]amino}propyl)-N-{2-[(2S)-1-methylpyrrolidin-2-yl]-1H-pyrrolo[3,2-c]pyridin-6-yl}benzamide;

1-(3-{1-[2-(2,6-dioxopiperidin-3-yl)-1,3-dioxoisoindol-4-yl]piperidin-4-yl}propyl)-N-{2-[(2R)-1-methylpyrrolidin-2-yl]-1H-pyrrolo[3,2-c]pyridin-6-yl}pyrazole-4-carboxamide;
2-(((1r,4R)-4-((2-(2,6-dioxopiperidin-3-yl)-1,3-dioxoisoindolin-4-yl)amino)cyclohexyl)oxy)-*N*-(2-((R)-1-methylpyrrolidin-2-yl)-1*H*-pyrrolo[3,2-c]pyridin-6-yl)acetamide;
2-(4-((2-(2,6-dioxopiperidin-3-yl)-1,3-dioxoisoindolin-4-yl)amino)butoxy)-*N*-(2-((*R*)-1-methylpyrrolidin-2-yl)-1*H*-pyrrolo[3,2-c]pyridin-6-yl)acetamide;
4-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)butanamide;
5-(1-(2-(2,6-Dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-*c*]pyridin-6-yl)pentanamide;
6-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)hexanamide;
4-(1-(2-(2,6-dioxopiperidin-3-yl)-1,3-dioxoisoindolin-4-yl)piperidin-3-yl)-*N*-(2-((*R*)-1-methylpyrrolidin-2-yl)-1H-pyrrolo[3,2-c]pyridin-6-yl)butanamide;
3-(3-((2-(2,6-dioxopiperidin-3-yl)-1,3-dioxoisoindolin-4-yl)amino)propyl)-*N*-(2-((*R*)-1-methylpyrrolidin-2-yl)-1*H*-pyrrolo[3,2-c]pyridin-6-yl)benzamide;
3-(((1-(2-(2,6-dioxopiperidin-3-yl)-1,3-dioxoisoindolin-4-yl)piperidin-4-yl)methyl)amino)-*N*-(2-((*R*)-1-methylpyrrolidin-2-yl)-1*H*-pyrrolo[3,2-c]pyridin-6-yl)benzamide;
3-(1-(3-(1-(2-(2,6-dioxopiperidin-3-yl)-1,3-dioxoisoindolin-4-yl)piperidin-4-yl)propyl)-1*H*-pyrazol-4-yl)-*N*-(2-((*R*)-1-methylpyrrolidin-2-yl)-1*H*-pyrrolo[3,2-c]pyridin-6-yl)benzamide;
4-(1-(2-(2,6-dioxopiperidin-3-yl)-1,3-dioxoisoindolin-4-yl)azetidin-3-yl)-*N*-(2-((R)-1-methylpyrrolidin-2-yl)-1*H*-pyrrolo[3,2-c]pyridin-6-yl)butanamide;
5-(4-(2-(2,6-dioxopiperidin-3-yl)-1-oxoisoindolin-4-yl)but-3-yn-1-yl)-N-(2-((R)-1 - methylpyrrolidin-2-yl)-1H-pyrrolo[3,2-c]pyridin-6-yl)picolinamide;
5-[3-[2-(2,6-dioxo-3-piperidyl)-1-oxo-isoindolin-4-yl]prop-2-ynoxy]-N-[2-(1-methylpyrrolidin-2-yl)-1H-pyrrolo [3,2-c]pyridin-6-yl]pyridine-2-carboxamide;
4-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)benzamide;
4-(4-(2-(2,6-dioxopiperidin-3-yl)-1-oxoisoindolin-4-yl)but-3-yn-1-yl)-N-(2-((*R*)-1-methylpyrrolidin-2-yl)-1H-pyrrolo[3,2-c]pyridin-6-yl)benzamide;
4-(4-(1-(2,6-dioxopiperidin-3-yl)-3-methyl-2-oxo-2,3-dihydro-1*H*-benzo[*d*]imidazol-4-yl)but-3-yn-1-yl)-*N*-(2-((*R*)-1-methylpyrrolidin-2-yl)-1*H*-pyrrolo[3,2-*c*]pyridin-6-yl)benzamide;
4-(4-(2-(2, 6-dioxopiperidin-3-yl)-1-oxoisoindolin-4-yl) but-3-yn-1-yl)-2-fluoro-*N*-(2-((R)-1-methylpyrrolidin-2-yl)-1*H*-pyrrolo [3, 2-c] pyridin-6-yl) benzamide;
4-(5-(2-(2,6-dioxopiperidin-3-yl)-1-oxoisoindolin-4-yl)pent-4-yn-1-yl)-N-(2-((R)-1-methylpyrrolidin-2-yl)-1*H*-pyrrolo[3,2-c]pyridin-6-yl)benzamide;
4-((5-(2-(2,6-dioxopiperidin-3-yl)-1-oxoisoindolin-4-yl)pent-4-yn-1-yl)oxy)-*N*-(2-((*R*)-1-methylpyrrolidin-2-yl)-1*H*-pyrrolo[3,2-c]pyridin-6-yl)benzamide;
4-((3-(2-(2,6-dioxopiperidin-3-yl)-1-oxoisoindolin-4-yl)prop-2-yn-1-yl)oxy)-2-fluoro-*N*-(2-((R)-1-methylpyrrolidin-2-yl)-1*H*-pyrrolo[3,2-c]pyridin-6-yl)benzamide;
3-((3-(2-(2,6-dioxopiperidin-3-yl)-1-oxoisoindolin-4-yl)prop-2-yn-1-yl)oxy)-*N*-(2-((R)-1-methylpyrrolidin-2-yl)-1*H*-pyrrolo[3,2-c]pyridin-6-yl)benzamide;
4-(1-(6-(2-(2,6-dioxopiperidin-3-yl)-1-oxoisoindolin-4-yl)hex-5-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide;
*N*-((*S*)-2,6-dioxopiperidin-3-yl)-6-(4-(3-(4-(4-((2-((*R*)-1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinamide;
*N*-((*S*)-2,6-dioxopiperidin-3-yl)-6-(4-(2-(4-(4-((2-((*R*)-1-methylpyrrolidin-2-yl)-1*H*-pyrrolo[3,2-*c*]pyridin-6-yl)carbamoyl)phenyl)-1*H*-pyrazol-1-yl)ethoxy)piperidin-1- yl)picolinamide;
*N*-((*S*)-2,6-dioxopiperidin-3-yl)-6-(5-(4-(4-((2-((R)-1-methylpyrrolidin-2-yl)-1*H*-pyrrolo[3,2-c]pyridin-6-yl)carbamoyl)phenyl)-1*H*-pyrazol-1-yl)pent-1-yn-1-yl) picolinamide;
(*R*)-4-(5-(6-(2,4-dioxotetrahydropyrimidin-1(2*H*)-yl)pyridin-2-yl)pent-4-yn-1-yl)-*N*-(2-(1-methylpyrrolidin-2-yl)-1*H*-pyrrolo[3,2-c]pyridin-6-yl)benzamide;
6-(4-(4-(2-(2,6-dioxopiperidin-3-yl)-1,3-dioxoisoindolin-4-yl)piperazin-1-yl)piperidin-1-yl)-*N*-(2-((*R*)-1-methylpyrrolidin-2-yl)-1*H*-pyrrolo[3,2-c]pyridin-6-yl)nicotinamide;
2-(4-((2-(2,6-dioxopiperidin-3-yl)-1-oxoisoindolin-4-yl)ethynyl)piperidin-1-yl)-*N*-(2-((*R*)-1-methylpyrrolidin-2-yl)-1*H*-pyrrolo[3,2-c]pyridin-6-yl)pyrimidine-5-carboxamide;
4-((3-(1-(2,6-dioxopiperidin-3-yl)-3-methyl-2-oxo-2,3-dihydro-1*H*-benzo[d]imidazol-4-yl)prop-2-yn-1-yl)oxy)-*N*-(2-((*R*)-1-methylpyrrolidin-2-yl)-1H-pyrrolo[3,2-c]pyridin-6-yl)benzamide;

6-(4-(4-(2-(2,6-dioxopiperidin-3-yl)-1-oxoisoindolin-4-yl)phenyl)piperidin-1-yl)-*N*-(2-((*R*)-1-methylpyrrolidin-2-yl)-1*H*-pyrrolo[3,2-*c*]pyridin-6-yl)nicotinamide;
7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}heptanamide;
(2S,4R)-1-[(2S)-3,3-dimethyl-2-(11-{[4-({2-[(2R)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
(2S,4R)-1-[(2S)-3,3-dimethyl-2-(11-{[4-({2-[(2S)-2-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
N-(3-chloro-2-((R)-1-methylpyrrolidin-2-yl)-1H-pyrrolo[3,2-c]pyridin-6-yl)-6-(6-(2-(2,6-dioxopiperidin-3-yl)-1-oxoisoindolin-4-yl)hex-5-yn-1-yl)nicotinamide;
N-(3-chloro-2-((R)-1-methylpyrrolidin-2-yl)-1H-pyrrolo[3,2-c]pyridin-6-yl)-6-(4-(3-(2-(2,6-dioxopiperidin-3-yl)-1-oxoisoindolin-4-yl)prop-2-yn-1-yl)piperazin-1-yl)nicotinamide;
6-(4-(3-(2-(2,6-dioxopiperidin-3-yl)-1-oxoisoindolin-4-yl)prop-2-yn-1-yl)piperidin-1-yl)-N-(2-((R)-1-ethylpyrrolidin-2-yl)-1H-pyrrolo[3,2-c]pyridin-6-yl)nicotinamide;
6-(6-(2-(2,6-dioxopiperidin-3-yl)-1-oxoisoindolin-4-yl)hex-5-yn-1-yl)-N-(2-((R)-1-ethylpyrrolidin-2-yl)-1H-pyrrolo[3,2-c]pyridin-6-yl)nicotinamide; and
6-(1'-(2-(2,6-dioxopiperidin-3-yl)-1-oxoisoindolin-4-yl)-[4,4'-bipiperidin]-1-yl)-N-(2-((R)-1-methylpyrrolidin-2-yl)-1H-pyrrolo[3,2-c]pyridin-6-yl)nicotinamide.

45. A compound according to any one of aspects 1 to 28, wherein M is of formula (IV):

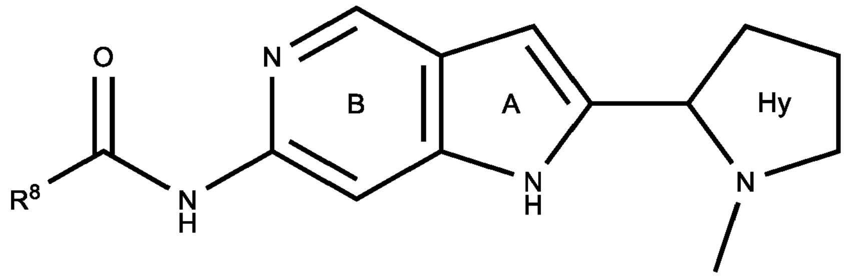

(IV)

wherein $R^8$ is (i) a bond to LINK or (ii) a group selected from a 6-membered aryl ring and a 5- to 6-membered heteroaryl ring, the group $R^8$ being unsubstituted or substituted by one, two or three substituents independently selected from halo, $C_{1-4}$ alkyl and $R^{10}$; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl and cyclopropyl;
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

46. A compound according to any one of aspects 1 to 45, wherein $R^8$ is phenyl, pyrimidinyl or pyridinyl.

47. A compound according to any one of the preceding aspects, wherein the group $R^8$ is unsubstituted or substituted by one fluoro or chloro group and/or by one group $R^{10}$.

48. A compound according to any one of the preceding aspects, wherein the group $R^8$ is phenyl, pyrimidinyl or pyridinyl which is unsubstituted or substituted by one fluoro or chloro group and/or by one group $R^{10}$, wherein $R^{10}$ is pyrazolyl, or wherein phenyl, pyrimidinyl or pyridinyl which is unsubstituted or substituted by one fluoro or chloro group.

49. A compound according to any one of aspects 45 to 48, wherein $R^8$ is selected from one of the following structures:

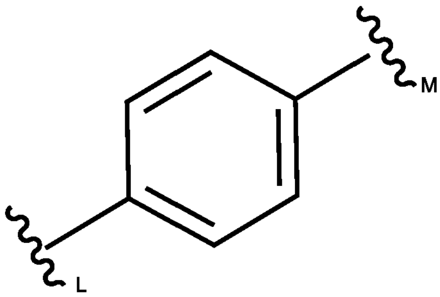

A

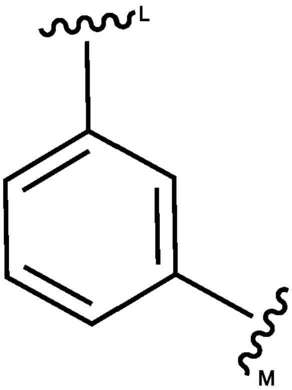

B

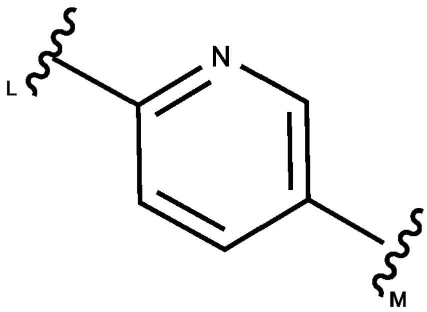

C

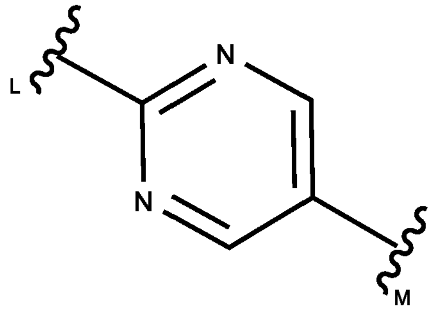

D

wherein:

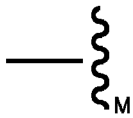

represents the point of attachment of $R^8$ to the rest of moiety M;

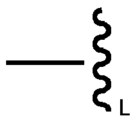

represents the point of attachment of $R^8$ to LINK or $R^{10}$.

50. A compound according to any one of aspects 1 to 35 and 39 to 43, wherein at least one of $R^4$, $R^5$ and $R^6$ is not H.

51. A compound according to any one of aspects 1 to 39, wherein $R^8$ is a bond to link.

52. A compound according to any one of aspects 1 to 39, wherein $R^8$ is a group (ii) and M is bonded to LINK via a C or N atom within group $R^8$.

53. A compound according to any one of the preceding aspects, wherein the bond from ring Hy to ring A is in the "up" position and the bond from ring Hy to $R^2$ is in the "down" position relative to ring Hy, as depicted below:

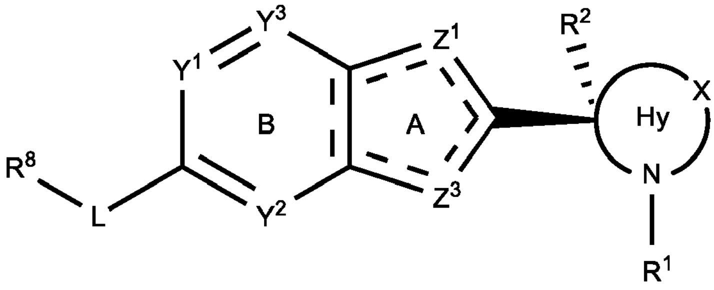
.

54. A pharmaceutical composition which comprises a compound as defined in any one of aspects 1 to 53 and a pharmaceutically acceptable carrier or diluent.

55. A compound as defined in any one of aspects 1 to 53 for use in the treatment of the human or animal body by therapy.

56. A compound as defined in any one of aspects 1 to 53 for use in the treatment of cancer.

57. The compound for use according to aspect 56, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma, neuroblastoma, midline glioma or sarcoma, preferably neuroblastoma or midline glioma.

58. The compound for use according to aspect 56, wherein the cancer is acute myeloid leukaemia (AML), myelodysplastic syndrome (MDS), myeloproliferative neoplasm (MPN) or acute lymphoblastic leukaemia (ALL).

59. The compound for use according to aspect 58, wherein the cancer is mixed-lineage leukaemia (MLL) or rearranged acute leukemia (AML and ALL).

60. The compound for use according to aspect 58, wherein the cancer is NPM1 mutant leukemia.

## Claims

**1.** A compound which is a Proteolysis Targeting Chimera (PROTAC) or a pharmaceutically acceptable salt thereof, wherein the PROTAC has the structure:

**M-LINK-U**

wherein U is an E3 ubiquitin ligase binding moiety, LINK is a moiety that covalently links M and U, and M is an MLLT1 and/or MLLT3 binder of formula (I):

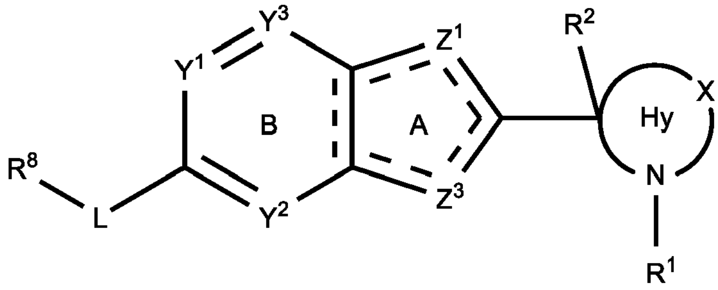

(I)

wherein:

one of $Z^1$ and $Z^3$ is -N(H)- and the other is N or $-C(R^4)-$, $Y^1$ is N, $Y^2$ is N or $-C(R^6)-$, and $Y^3$ is N or $-C(R^5)-$;
Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, $-N(R^{11})-$, O, S, $-S(O)_2-$, $-S(O)(NR^{11})-$, or $-C(R^{11})_2-$; and the rest of ring Hy is unsubstituted or substituted by one or two $R^3$;
L is -C(O)N(H)-, wherein the C atom of L is bonded to $R^8$, and the N atom of L is bonded to ring B;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$ and $R^6$ are independently selected from H, halo, CN, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
$R^8$ is (i) a bond to LINK or (ii) a group selected from a 6-membered aryl ring, a 5- to 6-membered heteroaryl ring, a 5- to 6-membered heterocyclyl ring, and a 5- to 6-membered cycloalkyl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two R9;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
each $R^{11}$ is independently selected from H, $C_{1-4}$ alkyl, and $C_{1-4}$ cycloalkyl;
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

**2.** A compound according to claim 1, wherein LINK is (a) a single bond, or (b) a chemical linker group represented by formula (L):

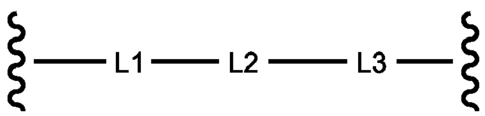

(L)

wherein:

LINK is attached to M via L1;
LINK is attached to U via L3;

L1 and L3 are each independently selected from a single bond, -N(R')-, -C(O)N(R')-, -O-, -N(R')C(O)-, -C(O)-, -$S(O_2)$N(R')-, -N(R')$S(O_2)$-, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;
L2 is represented by the formula -$(L4)_m$-;
each L4 is independently selected from a divalent 3- to 6-membered cycloalkyl ring, a divalent 4- to 7-membered heterocyclyl ring and a unit of formula:

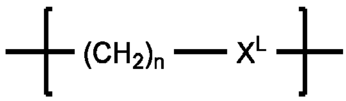 or 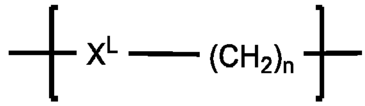 ;

each $X^L$ is independently selected from a single bond, -N(R')-, -O-, -C(O)-, -S-, -SO-, - $SO_2$-, -C(H)=C(H)- and -C(H)≡C(H)-;
n is selected from 1 to 4;
m is selected from 1 to 30; and
each R' is independently selected from H and $C_{1-4}$ alkyl.

**3.** A compound according to claim 1 or 2, wherein U is:

(a) a CRBN E3 ubiquitin ligase binding moiety of formula (U1):

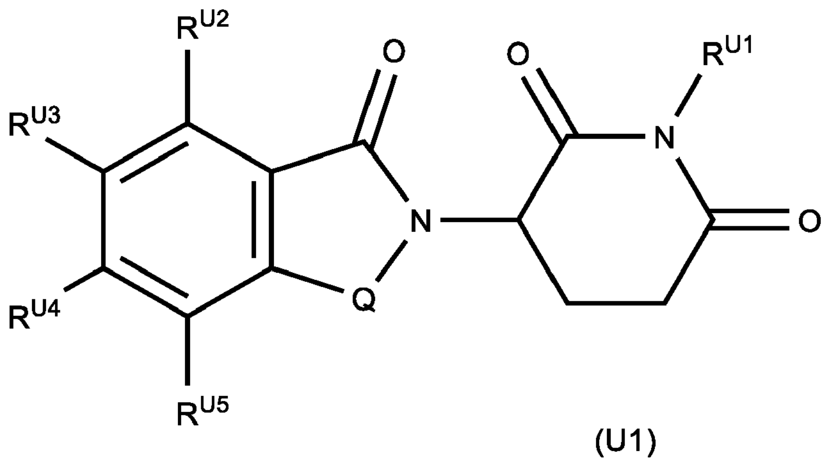

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

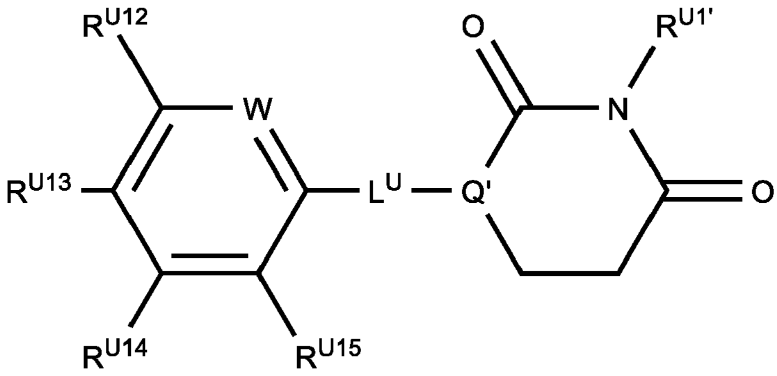

(U4)

wherein:

$R^{U1'}$ is H;
W is N or $CR^{U16}$;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

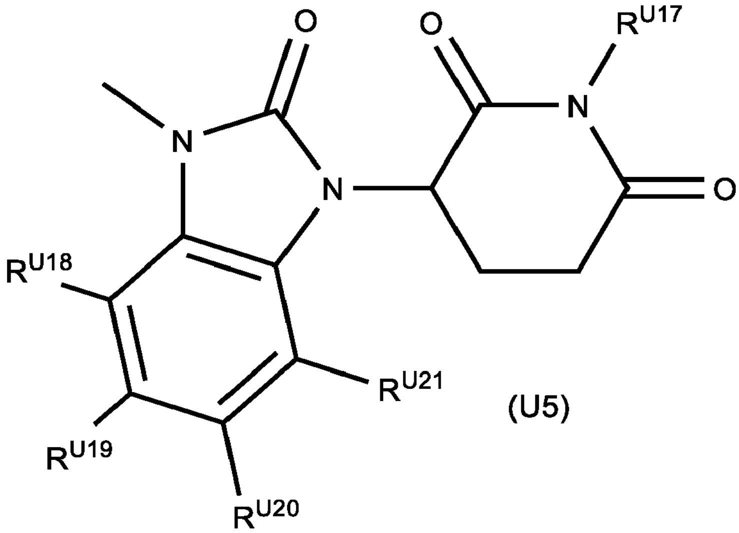

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;
wherein one and only one of $R^{U18}$, $R^{U19}$, $R^{U20}$ and $R^{U21}$ is a single bond to LINK;

or (d) a VHL E3 ubiquitin ligase binding moiety of formula (U2):

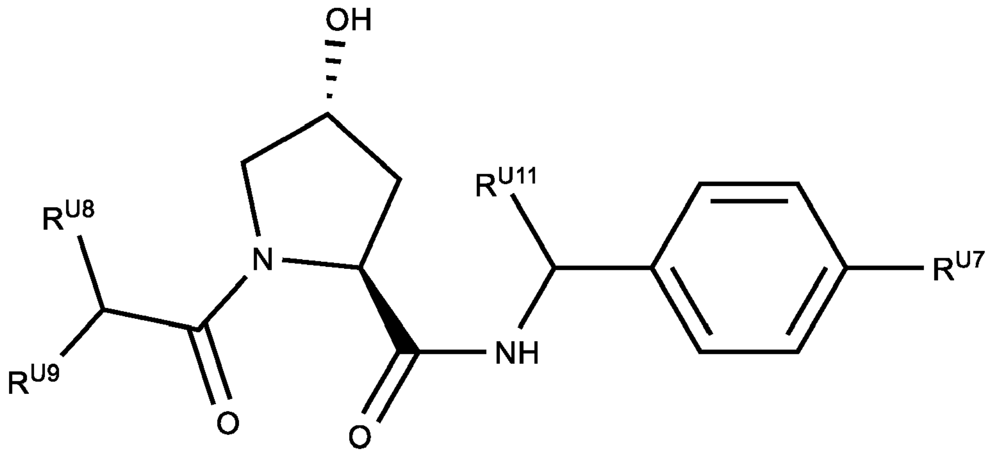

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

**4.** A compound according to any one of the preceding claims, wherein the N atom of ring Hy which is substituted by $R^1$ is adjacent to the C atom of ring Hy that is bonded to ring A.

**5.** A compound according to any one of the preceding claims, wherein $Z^1$ is -$C(R^4)$-, $Z^3$ is -N(H)-, $Y^1$ is N, $Y^2$ is -$C(R^6)$- and $Y^3$ is -$C(R^5)$-.

**6.** A compound according to any one of the preceding claims, wherein M is of formula (II):

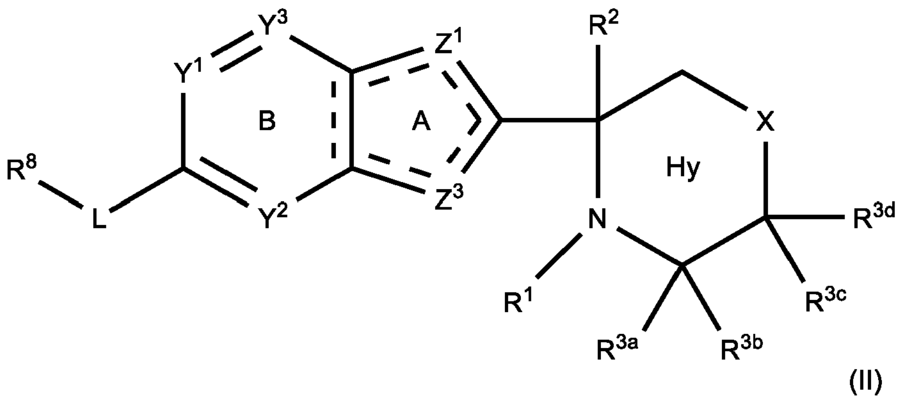

(II)

wherein:

$Z^1$, $Z^3$, $Y^1$, $Y^2$, $Y^3$, $R^1$, $R^2$, $R^8$, X and L are as defined in any one of the preceding claims;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl

ring, with the proviso that when X is -N($R^{11}$)-, O, S, -S(O)$_2$- or -S(O)($NR^{11}$)-, then neither $R^{3c}$ nor $R^{3d}$ are halo; wherein at least two of $R^{3a}$, $R^{3b}$, $R^{3c}$ and $R^{3d}$ are H; and
wherein either (i) $R^8$ is a bond to LINK, or (ii) $R^8$ is a group and M is bonded to LINK via a C or N atom within group $R^8$ such that a hydrogen atom on the C or N atom within group $R^8$ is replaced with a bond to LINK.

**7.** A compound according to any one of the preceding claims, wherein M is of formula (III):

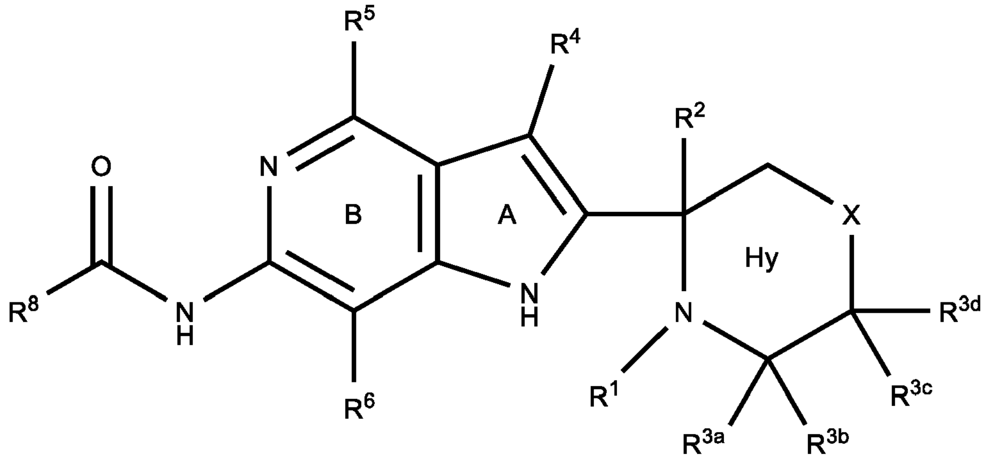

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring,
with the proviso that when X is -N(Me)- or O, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or -$CH_2$-;
$R^4$ is selected from H, CN, halo, $C_{1-4}$ alkoxy, and $C_{1-4}$ alkyl which is itself unsubstituted or substituted by one, two or three halo;
$R^5$ and $R^6$ are independently selected from H, halo, $C_{1-4}$ alkoxy, and $C_{1-4}$ alkyl which is itself unsubstituted or substituted by one, two or three halo;
$R^8$ is (i) a bond to LINK or (ii) a group selected from a 6-membered aryl ring, a 5- to 6-membered heteroaryl ring, and a 5- to 6-membered heterocyclyl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

**8.** A compound according to any one of the preceding claims, wherein Hy is an unsubstituted pyrrolidine ring, $R^1$ is methyl, and $R^2$ is H.

**9.** A compound according to claim 1, wherein the PROTAC structure is selected from:

4-(4-(2-(2,6-dioxopiperidin-3-yl)-1-oxoisoindolin-4-yl)but-3-yn-1-yl)-*N*-(2-((*R*)-1-methylpyrrolidin-2-yl)-1*H*-pyrrolo[3, 2-*c*]pyridin-6-yl)piperidine-1-carboxamide;
6-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)nicotinamide;
4-((1-(2-(2,6-dioxopiperidin-3-yl)-1,3-dioxoisoindolin-4-yl)piperidin-4-yl)methyl)-*N*-(2-((*R*)-1-methylpyrrolidin-2-yl)-1*H*-pyrrolo[3,2-c]pyridin-6-yl)benzamide;
4-(2-(1-(2-(2,6-dioxopiperidin-3-yl)-1,3-dioxoisoindolin-4-yl)piperidin-4-yl)ethyl)-*N*-(2-((*R*)-1-methylpyrrolidin-2-yl)-1*H*-pyrrolo[3,2-c]pyridin-6-yl)benzamide;
6-(4-((1-(2-(2,6-dioxopiperidin-3-yl)-1,3-dioxoisoindolin-4-yl)piperidin-4-yl)methyl)piperazin-1-yl)-*N*-(2-((*R*)-1-methylpyrrolidin-2-yl)-1*H*-pyrrolo[3,2-c]pyridin-6-yl)nicotinamide;
6-(4-(3-(2-(2,6-dioxopiperidin-3-yl)-1-oxoisoindolin-4-yl)prop-2-yn-1-yl)piperazin-1-yl)-*N*-(2-((*R*)-1-methylpyrrolidin-2-yl)-1*H*-pyrrolo[3,2-*c*]pyridin-6-yl)nicotinamide;
*N*-((S)-2,6-dioxopiperidin-3-yl)-6-(6-(4-((2-((*R*)-1-methylpyrrolidin-2-yl)-1*H*-pyrrolo[3,2-c]pyridin-6-yl)carbamoyl)phenyl)hex-1-yn-1-yl)picolinamide;
4-(6-(2-(2,6-dioxopiperidin-3-yl)-1-oxoisoindolin-4-yl)hex-5-yn-1-yl)-*N*-(2-((*R*)-1-methylpyrrolidin-2-yl)-1*H*-pyrrolo[3,2-c]pyridin-6-yl)benzamide;
(*R*)-4-(6-(6-(2,4-dioxotetrahydropyrimidin-1(2*H*)-yl)pyridin-2-yl)hex-5-yn-1-yl)-*N*-(2-(1-methylpyrrolidin-2-yl)-1*H*-pyrrolo[3,2-c]pyridin-6-yl)benzamide;
6-(6-(2-(2, 6-dioxopiperidin-3-yl)-1-oxoisoindolin-4-yl) hex-5-yn-1-yl)-*N*-(2-((*R*)-1-methylpyrrolidin-2-yl)-1*H*-pyrrolo [3, 2-*c*] pyridin-6-yl) nicotinamide;
*N*-((*S*)-2,6-dioxopiperidin-3-yl)-5-(5-(4-((2-((*R*)-1-methylpyrrolidin-2-yl)-1*H*-pyrrolo[3,2-c]pyridin-6-yl)carbamoyl)phenyl)pent-1-yn-1-yl)picolinamide;
6-(5-(2-(2,6-dioxopiperidin-3-yl)-1-oxoisoindolin-4-yl)pent-4-yn-1-yl)-*N*-(2-((*R*)-1-methylpyrrolidin-2-yl)-1*H*-pyrrolo[3,2-c]pyridin-6-yl)nicotinamide;
*N*-(3-chloro-2-((R)-1-methylpyrrolidin-2-yl)-1*H*-pyrrolo [3, 2-c] pyridin-6-yl)-9-(2-(2, 6-dioxopiperidin-3-yl)-1-oxoisoindolin-4-yl) non-8-ynamide;
3-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide;
4-(1-(2-(2,6-dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-c]pyridin-6-yl)benzamide;
6-(4-(3-(2-(2,6-dioxopiperidin-3-yl)-1-oxoisoindolin-4-yl)prop-2-yn-1-yl)piperidin-1-yl)-*N*-(2-((*R*)-1-methylpyrrolidin-2-yl)-1*H*-pyrrolo[3,2-*c*]pyridin-6-yl)nicotinamide;
6-(4-((2-(2,6-dioxopiperidin-3-yl)-1-oxoisoindolin-4-yl)ethynyl)piperidin-1-yl)-*N*-(2-((R)-1-methylpyrrolidin-2-yl)-1*H*-pyrrolo[3,2-c]pyridin-6-yl)nicotinamide;
6-(4-((1-(2-(2,6-dioxopiperidin-3-yl)-1,3-dioxoisoindolin-4-yl)piperidin-4-yl)methoxy)piperidin-1-yl)-*N*-(2-((*R*)-1-methylpyrrolidin-2-yl)-1*H*-pyrrolo[3,2-c]pyridin-6-yl)nicotinamide;
4-((4-(2-(2,6-dioxopiperidin-3-yl)-1-oxoisoindolin-4-yl)but-3-yn-1-yl)oxy)-*N*-(2-((*R*)-1-methylpyrrolidin-2-yl)-1*H*-pyrrolo[3,2-c]pyridin-6-yl)benzamide;
4-(1-(5-(2-(2,6-dioxopiperidin-3-yl)-1-oxoisoindolin-4-yl)pent-4-yn-1-yl)-1*H*-pyrazol-3-yl)-*N*-(2-((*R*)-1-methylpyrrolidin-2-yl)-1*H*-pyrrolo[3,2-c]pyridin-6-yl)benzamide;
4-(1-(5-(2-(2,6-dioxopiperidin-3-yl)-1-oxoisoindolin-4-yl)pent-4-yn-1-yl)-1H-pyrazol-4-yl)-N-(2-((R)-1-methylpyrrolidin-2-yl)-1H-pyrrolo[3,2-c]pyridin-6-yl)benzamide;
4-(1-(5-(2-(2,6-dioxopiperidin-3-yl)-1-oxoisoindolin-5-yl)pent-4-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide;
3 -chloro-4-((3 -(2-(2,6 - di ox opiperi din-3 -yl)-1 -oxoi soindolin-4-yl)prop-2-yn-1 - yl)oxy)-*N*-(2-((*R*)-1-methylpyrrolidin-2-yl)-1H-pyrrolo[3,2-c]pyridin-6-yl)benzamide;
4-(((2S)-4-(2-(2,6-dioxopiperidin-3-yl)-1-oxoisoindolin-4-yl)but-3-yn-2-yl)oxy)-N-(2-((R)-1-methylpyrrolidin-2-yl)-1H-pyrrolo[3,2-c]pyridin-6-yl)benzamide;
4-(((2R)-4-(2-(2,6-dioxopiperidin-3-yl)-l-oxoisoindolin-4-yl)but-3-yn-2-yl)oxy)-N-(2-((R)-1-methylpyrrolidin-2-yl)-1H-pyrrolo[3,2-c]pyridin-6-yl)benzamide;
3-((9-(2-(2,6-dioxopiperidin-3-yl)-1,3-dioxoisoindolin-4-yl)non-8-yn-1-yl)oxy)-*N*-(2-((*R*)-1-methylpyrrolidin-2-yl)-1*H*-pyrrolo[3,2-c]pyridin-6-yl)benzamide;
7-{[2-(2,6-dioxopiperidin-3-yl)-1,3-dioxoisoindol-5-yl]amino}-N-{2-[(2R)-1-methylpyrrolidin-2-yl]-1H-pyrrolo [3,2-c] pyridin-6-yl}heptanamide;
7-{[2-(2,6-dioxopiperidin-3-yl)-1,3-dioxoisoindol-5-yl]amino}-N-{2-[(2S)-1-methylpyrrolidin-2-yl]-1H-pyrrolo [3,2-c]pyridin-6-yl}heptanamide;
7-{[2-(2,6-dioxopiperidin-3-yl)-1,3-dioxoisoindol-4-yl]amino}-N-{2-[(2R)-1-methylpyrrolidin-2-yl]-1H-pyrrolo [3,2-c] pyridin-6-yl}heptanamide;

9-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}nonanamide;
12-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}dodecanamide;
12-{[2-(2,6-dioxopiperidin-3-yl)-1,3-dioxoisoindol-4-yl]amino}-N-{2-[(2S)-1-methylpyrrolidin-2-yl]-1H-pyrrolo[3,2-c]pyridin-6-yl}dodecanamide;
N-{3-chloro-2-[(2R)-1-methylpyrrolidin-2-yl]-1H-pyrrolo[3,2-c]pyridin-6-yl}-7-{[2-(2,6-dioxopiperidin-3-yl)-1,3-dioxoisoindol-4-yl]amino}heptanamide;
4-[1-(3-{ 1-[2-(2,6-dioxopiperidin-3-yl)-1,3-dioxoisoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide;
4-[1-(3-{1-[2-(2,6-dioxopiperidin-3-yl)-1-oxo-3H-isoindol-4-yl]piperidin-4-yl}propyl)pyrazol-4-yl]-N-{2-[(2R)-1-methylpyrrolidin-2-yl]-1H-pyrrolo[3,2-c]pyridin-6-yl}benzamide;
4-(3-{[2-(2,6-dioxopiperidin-3-yl)-1,3-dioxoisoindol-4-yl]amino}propyl)-N-{2-[(2R)-1-methylpyrrolidin-2-yl]-1H-pyrrolo[3,2-c]pyridin-6-yl}benzamide;
4-(3-{[2-(2,6-dioxopiperidin-3-yl)-1,3-dioxoisoindol-4-yl]amino}propyl)-N-{2-[(2S)-1-methylpyrrolidin-2-yl]-1H-pyrrolo[3,2-c]pyridin-6-yl}benzamide;
1-(3-{1-[2-(2,6-dioxopiperidin-3-yl)-1,3-dioxoisoindol-4-yl]piperidin-4-yl}propyl)-N-{2-[(2R)-1-methylpyrrolidin-2-yl]-1H-pyrrolo[3,2-c]pyridin-6-yl}pyrazole-4-carboxamide;
2-(((1r,4R)-4-((2-(2,6-dioxopiperidin-3-yl)-1,3-dioxoisoindolin-4-yl)amino)cyclohexyl)oxy)-*N*-(2-((R)-1-methylpyrrolidin-2-yl)-1*H*-pyrrolo[3,2-c]pyridin-6-yl)acetamide;
2-(4-((2-(2,6-dioxopiperidin-3-yl)-1,3-dioxoisoindolin-4-yl)amino)butoxy)-*N*-(2-((R)-1-methylpyrrolidin-2-yl)-1*H*-pyrrolo[3,2-c]pyridin-6-yl)acetamide;
4-(1-(2-(2,6-dioxopiperidin-3-yl)-1,3-dioxoisoindolin-4-yl)piperidin-4-yl)-*N*-(2-((R)-1-methylpyrrolidin-2-yl)-1*H*-pyrrolo[3,2-c]pyridin-6-yl)butanamide;
5-(1-(2-(2,6-Dioxopiperidin-3-yl)-1,3-dioxoisoindolin-4-yl)piperidin-4-yl)-*N*-(2-((*R*)-1-methylpyrrolidin-2-yl)-1*H*-pyrrolo[3,2-*c*]pyridin-6-yl)pentanamide;
6-(1-(2-(2,6-dioxopiperidin-3-yl)-1,3-dioxoisoindolin-4-yl)piperidin-4-yl)-*N*-(2-((R)-1-methylpyrrolidin-2-yl)-1*H*-pyrrolo[3,2-c]pyridin-6-yl)hexanamide;
4-(1-(2-(2,6-dioxopiperidin-3-yl)-1,3-dioxoisoindolin-4-yl)piperidin-3-yl)-N-(2-((R)-1-methylpyrrolidin-2-yl)-1H-pyrrolo[3,2-c]pyridin-6-yl)butanamide;
3-(3-((2-(2,6-dioxopiperidin-3-yl)-1,3-dioxoisoindolin-4-yl)amino)propyl)-*N*-(2-((R)-1-methylpyrrolidin-2-yl)-1*H*-pyrrolo[3,2-c]pyridin-6-yl)benzamide;
3-(((1-(2-(2,6-dioxopiperidin-3-yl)-1,3-dioxoisoindolin-4-yl)piperidin-4-yl)methyl)amino)-*N*-(2-((R)-1-methylpyrrolidin-2-yl)-1*H*-pyrrolo[3,2-c]pyridin-6-yl)benzamide;
3-(1-(3-(1-(2-(2,6-dioxopiperidin-3-yl)-1,3-dioxoisoindolin-4-yl)piperidin-4-yl)propyl)-1*H*-pyrazol-4-yl)-*N*-(2-((R)-1-methylpyrrolidin-2-yl)-1*H*-pyrrolo[3,2-c]pyridin-6-yl)benzamide;
4-(1-(2-(2,6-dioxopiperidin-3-yl)-1,3-dioxoisoindolin-4-yl)azetidin-3-yl)-*N*-(2-((R)-1-methylpyrrolidin-2-yl)-1*H*-pyrrolo[3,2-c]pyridin-6-yl)butanamide;
5-(4-(2-(2,6-dioxopiperidin-3-yl)-1-oxoisoindolin-4-yl)but-3-yn-1-yl)-N-(2-((R)-1 - methylpyrrolidin-2-yl)-1H-pyrrolo[3,2-c]pyridin-6-yl)picolinamide;
5-[3-[2-(2,6-dioxo-3-piperidyl)-1-oxo-isoindolin-4-yl]prop-2-ynoxy]-N-[2-(1-methylpyrrolidin-2-yl)-1H-pyrrolo[3,2-c]pyridin-6-yl]pyridine-2-carboxamide;
4-((3-(2-(2,6-dioxopiperidin-3-yl)-1-oxoisoindolin-4-yl)prop-2-yn-1-yl)oxy)-*N*-(2-((*R*)-1-methylpyrrolidin-2-yl)-1*H*-pyrrolo[3,2-c]pyridin-6-yl)benzamide;
4-(4-(2-(2,6-dioxopiperidin-3-yl)-1-oxoisoindolin-4-yl)but-3-yn-1-yl)-N-(2-((*R*)-1-methylpyrrolidin-2-yl)-1H-pyrrolo[3,2-c]pyridin-6-yl)benzamide;
4-(4-(1-(2,6-dioxopiperidin-3-yl)-3-methyl-2-oxo-2,3-dihydro-1*H*-benzo[*d*]imidazol-4-yl)but-3-yn-1-yl)-*N*-(2-((*R*)-1-methylpyrrolidin-2-yl)-1*H*-pyrrolo[3,2-*c*]pyridin-6-yl)benzamide;
4-(4-(2-(2, 6-dioxopiperidin-3-yl)-1-oxoisoindolin-4-yl) but-3-yn-1-yl)-2-fluoro-*N*-(2-((R)-1-methylpyrrolidin-2-yl)-1*H*-pyrrolo [3, 2-c] pyridin-6-yl) benzamide;
4-(5-(2-(2,6-dioxopiperidin-3-yl)-1-oxoisoindolin-4-yl)pent-4-yn-1-yl)-N-(2-((R)-1-methylpyrrolidin-2-yl)-1*H*-pyrrolo[3,2-c]pyridin-6-yl)benzamide;
4-((5-(2-(2,6-dioxopiperidin-3-yl)-1-oxoisoindolin-4-yl)pent-4-yn-1-yl)oxy)-*N*-(2-((*R*)-1-methylpyrrolidin-2-yl)-1*H*-pyrrolo[3,2-c]pyridin-6-yl)benzamide;
4-((3-(2-(2,6-dioxopiperidin-3-yl)-1-oxoisoindolin-4-yl)prop-2-yn-1-yl)oxy)-2-fluoro-*N*-(2-((R)-1-methylpyrrolidin-2-yl)-1*H*-pyrrolo[3,2-c]pyridin-6-yl)benzamide;
3-((3-(2-(2,6-dioxopiperidin-3-yl)-1-oxoisoindolin-4-yl)prop-2-yn-1-yl)oxy)-*N*-(2-((R)-1-methylpyrrolidin-2-yl)-1*H*-pyrrolo[3,2-c]pyridin-6-yl)benzamide;

4-(1-(6-(2-(2,6-dioxopiperidin-3-yl)-1-oxoisoindolin-4-yl)hex-5-yn-1-yl)-1*H*-pyrazol-4-yl)-*N*-(2-((*R*)-1-methylpyrrolidin-2-yl)-1*H*-pyrrolo[3,2-c]pyridin-6-yl)benzamide;
*N*-((S)-2,6-dioxopiperidin-3-yl)-6-(4-(3-(4-(4-((2-((*R*)-1-methylpyrrolidin-2-yl)-1*H*-pyrrolo[3,2-c]pyridin-6-yl)carbamoyl)phenyl)-1*H*-pyrazol-1-yl)propyl)piperidin-1-yl)picolinamide;
*N*-((*S*)-2,6-dioxopiperidin-3-yl)-6-(4-(2-(4-(4-((2-((*R*)-1-methylpyrrolidin-2-yl)-1*H*-pyrrolo[3,2-*c*]pyridin-6-yl)carbamoyl)phenyl)-1*H*-pyrazol-1-yl)ethoxy)piperidin-1-yl)picolinamide;
*N*-((S)-2,6-dioxopiperidin-3-yl)-6-(5-(4-(4-((2-((R)-1-methylpyrrolidin-2-yl)-1H-pyrrolo[3,2-c]pyridin-6-yl)carbamoyl)phenyl)-1*H*-pyrazol-1-yl)pent-1-yn-1-yl) picolinamide;
(*R*)-4-(5-(6-(2,4-dioxotetrahydropyrimidin-1(2*H*)-yl)pyridin-2-yl)pent-4-yn-1-yl)-*N*-(2-(1-methylpyrrolidin-2-yl)-1*H*-pyrrolo[3,2-c]pyridin-6-yl)benzamide;
6-(4-(4-(2-(2,6-dioxopiperidin-3-yl)-1,3-dioxoisoindolin-4-yl)piperazin-1-yl)piperidin-1-yl)-*N*-(2-((*R*)-1-methylpyrrolidin-2-yl)-1*H*-pyrrolo[3,2-c]pyridin-6-yl)nicotinamide;
2-(4-((2-(2,6-dioxopiperidin-3-yl)-1-oxoisoindolin-4-yl)ethynyl)piperidin-1-yl)-*N*-(2-((*R*)-1-methylpyrrolidin-2-yl)-1*H*-pyrrolo[3,2-c]pyridin-6-yl)pyrimidine-5-carboxamide;
4-((3-(1-(2,6-dioxopiperidin-3-yl)-3-methyl-2-oxo-2,3-dihydro-1*H*-benzo[d]imidazol-4-yl)prop-2-yn-1-yl)oxy)-*N*-(2-((*R*)-1-methylpyrrolidin-2-yl)-1*H*-pyrrolo[3,2-c]pyridin-6-yl)benzamide;
6-(4-(4-(2-(2,6-dioxopiperidin-3-yl)-1-oxoisoindolin-4-yl)phenyl)piperidin-1-yl)-*N*-(2-((*R*)-1-methylpyrrolidin-2-yl)-1*H*-pyrrolo[3,2-*c*]pyridin-6-yl)nicotinamide;
7-{[2-(2,6-dioxopiperidin-3-yl)-1,3-dioxoisoindol-4-yl]amino}-N-{2-[(2R)-1-methylpyrrolidin-2-yl]-1H-pyrrolo[3,2-c]pyridin-6-yl}heptanamide;
(2S,4R)-1-[(2S)-3,3-dimethyl-2-(11-{[4-({2-[(2R)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
(2S,4R)-1-[(2S)-3,3-dimethyl-2-(11-{[4-({2-[(2S)-1-methylpyrrolidin-2-yl]-1H-pyrrolo[3,2-c]pyridin-6-yl}carbamoyl)phenyl]formamido}undecanamido)butanoyl]-4-hydroxy-N-{[4-(4-methyl-1,3-thiazol-5-yl)phenyl]methyl}pyrrolidine-2-carboxamide;
N-(3-chloro-2-((R)-1-methylpyrrolidin-2-yl)-1H-pyrrolo[3,2-c]pyridin-6-yl)-6-(6-(2-(2,6-dioxopiperidin-3-yl)-1-oxoisoindolin-4-yl)hex-5-yn-1-yl)nicotinamide;
N-(3-chloro-2-((R)-1-methylpyrrolidin-2-yl)-1H-pyrrolo[3,2-c]pyridin-6-yl)-6-(4-(3 - (2-(2,6-dioxopiperidin-3-yl)-1-oxoisoindolin-4-yl)prop-2-yn-1-yl)piperazin-1-yl)nicotinamide;
6-(4-(3-(2-(2,6-dioxopiperidin-3-yl)-1-oxoisoindolin-4-yl)prop-2-yn-1-yl)piperidin-1-yl)-N-(2-((R)-1-ethylpyrrolidin-2-yl)-1H-pyrrolo[3,2-c]pyridin-6-yl)nicotinamide;
6-(6-(2-(2,6-dioxopiperidin-3-yl)-1-oxoisoindolin-4-yl)hex-5-yn-1-yl)-N-(2-((R)-1-ethylpyrrolidin-2-yl)-1H-pyrrolo[3,2-c]pyridin-6-yl)nicotinamide; and
6-(1'-(2-(2,6-dioxopiperidin-3-yl)-1-oxoisoindolin-4-yl)-[4,4'-bipiperidin]-1-yl)-N-(2-((R)-1-methylpyrrolidin-2-yl)-1H-pyrrolo[3,2-c]pyridin-6-yl)nicotinamide.

**10.** A compound according to any one of the preceding claims, wherein M is of formula (IV):

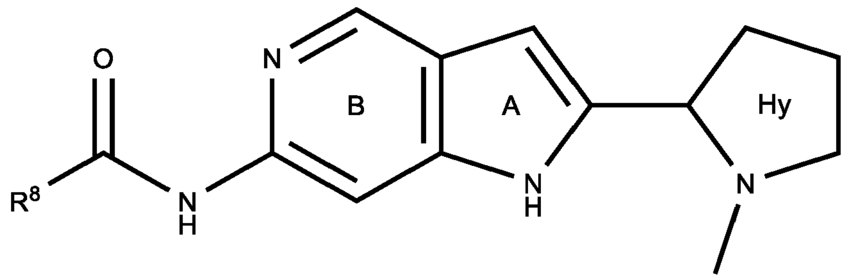

(IV)

wherein $R^8$ is (i) a bond to LINK or (ii) a group selected from a 6-membered aryl ring and a 5- to 6-membered heteroaryl ring, the group $R^8$ being unsubstituted or substituted by one, two or three substituents independently selected from halo, $C_{1-4}$ alkyl and $R^{10}$; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl and cyclopropyl;
wherein either $R^8$ is (i) a bond to LINK, or $R^8$ is a group (ii) and M is bonded to LINK via a C or N atom within group $R^8$ such that a hydrogen atom on the C or N atom within group $R^8$ is replaced with a bond to LINK.

**11.** A pharmaceutical composition which comprises a compound as defined in any one of claims 1 to 10 and a

pharmaceutically acceptable carrier or diluent.

**12.** A compound as defined in any one of claims 1 to 10 for use in the treatment of the human or animal body by therapy.

**13.** A compound as defined in any one of claims 1 to 10 for use in the treatment of cancer.

**14.** The compound for use according to claim 13, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma, neuroblastoma, midline glioma or sarcoma, preferably neuroblastoma or midline glioma.

**15.** The compound for use according to claim 13, wherein the cancer is acute myeloid leukaemia (AML), myelodysplastic syndrome (MDS), myeloproliferative neoplasm (MPN) or acute lymphoblastic leukaemia (ALL), for instance wherein the cancer is mixed-lineage leukaemia (MLL), rearranged acute leukemia (AML and ALL) or NPM1 mutant leukemia.

Europäisches Patentamt
European Patent Office
Office européen des brevets

# EUROPEAN SEARCH REPORT

Application Number
EP 24 18 3693

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2024/054749 A2 (BRIDGE MEDICINES [US]) 14 March 2024 (2024-03-14)<br>* claims; examples; tables 1-3 *<br>----- | 1-15 | INV.<br>C07D471/04<br>A61P35/00<br>A61K47/55 |
| Y | WO 2022/086937 A1 (ICAHN SCHOOL MED MOUNT SINAI [US]; VAN ANDEL RES INSTITUTE [US]) 28 April 2022 (2022-04-28)<br>* page 375 - page 377; claims; examples *<br>----- | 1-15 | |
| Y | WO 2023/183412 A1 (BAYLOR COLLEGE MEDICINE [US]) 28 September 2023 (2023-09-28)<br>* page 23, line 25 - page 27, line 15; claims; figures; examples *<br>----- | 1-15 | |
| Y | GARNAR-WORTZEL LEOPOLD ET AL: "Chemical Inhibition of ENL/AF9 YEATS Domains in Acute Leukemia",<br>ACS CENTRAL SCIENCE,<br>vol. 7, no. 5, 30 April 2021 (2021-04-30), pages 815-830, XP055935704,<br>ISSN: 2374-7943, DOI: 10.1021/acscentsci.0c01550<br>Retrieved from the Internet:<br>URL:https://pubs.acs.org/doi/pdf/10.1021/acscentsci.0c01550><br>* the whole document *<br>----- | 1-15 | TECHNICAL FIELDS SEARCHED (IPC)<br>C07D |
| A | TROUP ROBERT I. ET AL: "Current strategies for the design of PROTAC linkers: a critical review",<br>EXPLORATION OF TARGETED ANTI-TUMOR THERAPY,<br>vol. 1, no. 5,<br>30 October 2020 (2020-10-30), XP055828975,<br>DOI: 10.37349/etat.2020.00018<br>Retrieved from the Internet:<br>URL:https://www.explorationpub.com/uploads/Article/A100218/100218.pdf><br>* the whole document *<br>----- | 1-15 | |

The present search report has been drawn up for all claims

| Place of search | Date of completion of the search | Examiner |
|---|---|---|
| Munich | 9 December 2024 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2
EPO FORM 1503 03.82 (P04C01)

## ANNEX TO THE EUROPEAN SEARCH REPORT ON EUROPEAN PATENT APPLICATION NO.

EP 24 18 3693

This annex lists the patent family members relating to the patent documents cited in the above-mentioned European search report. The members are as contained in the European Patent Office EDP file on
The European Patent Office is in no way liable for these particulars which are merely given for the purpose of information.

09-12-2024

| Patent document cited in search report | Publication date | Patent family member(s) | Publication date |
|---|---|---|---|
| WO 2024054749 A2 | 14-03-2024 | NONE | |
| WO 2022086937 A1 | 28-04-2022 | US 2023391765 A1 | 07-12-2023 |
| | | WO 2022086937 A1 | 28-04-2022 |
| WO 2023183412 A1 | 28-09-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020041331 A
- WO 2019140003 A
- WO 2013106643 A
- US 20160045607 A
- WO 2014187777 A
- US 20140356322 A
- US 9249 A
- US 153 A
- US 20160058872 A
- US 20150291562 A


### Non-patent literature cited in the description


- *Cell*, 2013, vol. 153, 17
- *Cell*, 2017, vol. 168, 629
- *Cell Mol Life Sci*, 2018, vol. 75, 3931
- *Nat Rev Mol Cell Biol*, 2012, vol. 13, 543
- *Nature*, 2020, vol. 577, 121
- *Nature*, 2017, vol. 543, 270
- *New Engl J Med*, 2016, vol. 374, 2209
- *Cancer Disc*, 2022, vol. 12, 2684
- *Cell Rep*, 16 February 2021, vol. 34 (7), 108749
- *ACS Cent Sci*, 2021, vol. 7, 815
- *Angew. Chem. Int. Ed.*, 2018, vol. 57, 16302
- **GREG T. HERMANSON**. Bioconjugate Techniques. Academic Press Inc, 1996
- **GU et al.** *BioEssays*, 2018, vol. 40, 1700247
- **SUN et al.** *Signal Transduction and Targeted Therapy*, 2019, vol. 4, 64
- **WINTER et al.** *Science*, 19 June 2015, 1376
- **EISENHAUER et al.** *European Journal of Cancer*, 2009, vol. 45, 228-247
- *Blood*, 2007, vol. 109, 1815
- *Leukemia Res*, 2018, vol. 68, 32
- *CHEMICAL ABSTRACTS*, 156185-63-6
- *CHEMICAL ABSTRACTS*, 7598-35-8
- *CHEMICAL ABSTRACTS*, 316141-37-4
- *CHEMICAL ABSTRACTS*, 73365-02-3
- *CHEMICAL ABSTRACTS*, 90965-06-3
- *CHEMICAL ABSTRACTS*, 191732-72-6
- *CHEMICAL ABSTRACTS*, 66171-50-4
- *CHEMICAL ABSTRACTS*, 106-96-7
- *CHEMICAL ABSTRACTS*, 619-42-1
- *CHEMICAL ABSTRACTS*, 159635-49-1
- *CHEMICAL ABSTRACTS*, 835616-60-9
- *CHEMICAL ABSTRACTS*, 180307-56-6
- *CHEMICAL ABSTRACTS*, 57260-71-6
- *CHEMICAL ABSTRACTS*, 6271-78-9
- *CHEMICAL ABSTRACTS*, 158407-04-6
- *CHEMICAL ABSTRACTS*, 52070-67-4
- *CHEMICAL ABSTRACTS*, 619-44-3
- *CHEMICAL ABSTRACTS*, 5390-04-5
- *CHEMICAL ABSTRACTS*, 910044-07-4
- *CHEMICAL ABSTRACTS*, 504-07-4, 5.00
- *CHEMICAL ABSTRACTS*, 626-05-1
- *CHEMICAL ABSTRACTS*, 26218-78-0
- *CHEMICAL ABSTRACTS*, 927-74-2
- *CHEMICAL ABSTRACTS*, 845306-08-3
- *CHEMICAL ABSTRACTS*, 25181-50-4
- *CHEMICAL ABSTRACTS*, 30515-28-7
- *CHEMICAL ABSTRACTS*, 286961-14-6
- *CHEMICAL ABSTRACTS*, 22726-00-7
- *CHEMICAL ABSTRACTS*, 149353-75-3
- *CHEMICAL ABSTRACTS*, 89151-44-0
- *CHEMICAL ABSTRACTS*, 188646-83-5
- *CHEMICAL ABSTRACTS*, 95798-23-5
- *CHEMICAL ABSTRACTS*, 619-57-8
- *CHEMICAL ABSTRACTS*, 698-67-9
- *CHEMICAL ABSTRACTS*, 914672-66-5
- *CHEMICAL ABSTRACTS*, 552846-17-0
- *CHEMICAL ABSTRACTS*, 103440-53-5
- *CHEMICAL ABSTRACTS*, 24666-56-6
- *CHEMICAL ABSTRACTS*, 3964-57-6
- *CHEMICAL ABSTRACTS*, 99-76-3
- *CHEMICAL ABSTRACTS*, 42969-65-3
- *CHEMICAL ABSTRACTS*, 2914-69-4
- *CHEMICAL ABSTRACTS*, 618-49-5
- *CHEMICAL ABSTRACTS*, 87462-64-4

- *CHEMICAL ABSTRACTS*, 51857-17-1
- *CHEMICAL ABSTRACTS*, 835616-61-0
- *CHEMICAL ABSTRACTS*, 18934-81-1
- *CHEMICAL ABSTRACTS*, 164149-27-3
- *CHEMICAL ABSTRACTS*, 2075-45-8
- *CHEMICAL ABSTRACTS*, 123088-59-5
- *CHEMICAL ABSTRACTS*, 2093387-36-9
- *CHEMICAL ABSTRACTS*, 1814936-54-3
- *CHEMICAL ABSTRACTS*, 3956-07-8
- *CHEMICAL ABSTRACTS*, 92136-39-5
- *CHEMICAL ABSTRACTS*, 437701-80-9
- *CHEMICAL ABSTRACTS*, 111300-06-2
- *CHEMICAL ABSTRACTS*, 142247-38-9
- *CHEMICAL ABSTRACTS*, 142374-19-4
- *CHEMICAL ABSTRACTS*, 318536-95-7
- *CHEMICAL ABSTRACTS*, 618-89-3
- *CHEMICAL ABSTRACTS*, 137076-22-3
- *CHEMICAL ABSTRACTS*, 4518-10-9
- *CHEMICAL ABSTRACTS*, 398489-26-4
- *CHEMICAL ABSTRACTS*, 10236-14-3
- *CHEMICAL ABSTRACTS*, 29682-15-3
- *CHEMICAL ABSTRACTS*, 30766-12-2
- *CHEMICAL ABSTRACTS*, 15403-22-2
- *CHEMICAL ABSTRACTS*, 913297-44-6
- *CHEMICAL ABSTRACTS*, 62595-74-8
- *CHEMICAL ABSTRACTS*, 179232-29-2
- *CHEMICAL ABSTRACTS*, 107-19-7
- *CHEMICAL ABSTRACTS*, 197507-22-5
- *CHEMICAL ABSTRACTS*, 455-71-0
- *CHEMICAL ABSTRACTS*, 205059-24-1
- *CHEMICAL ABSTRACTS*, 374068-01-6
- *CHEMICAL ABSTRACTS*, 550378-30-8
- *CHEMICAL ABSTRACTS*, 317-46-4
- *CHEMICAL ABSTRACTS*, 352437-09-3
- *CHEMICAL ABSTRACTS*, 73183-34-3
- *CHEMICAL ABSTRACTS*, 10436-25-6
- *CHEMICAL ABSTRACTS*, 1448189-80-7
- *CHEMICAL ABSTRACTS*, 6757-31-9
- *CHEMICAL ABSTRACTS*, 360575-28-6
- *CHEMICAL ABSTRACTS*, 171049-35-7
- *CHEMICAL ABSTRACTS*, 3999-38-0
- *CHEMICAL ABSTRACTS*, 42516-28-9
- *CHEMICAL ABSTRACTS*, 173435-78-4